# EUROPEAN PATENT SPECIFICATION

(11) **EP 3 423 482 B1**
(45) Date of publication and mention of the grant of the patent: **09.09.2026**
(21) Application number: 17711774.4
(22) Date of filing: 03.03.2017
(51) Int. Cl.: C07K 14/725, C07K 16/30, C12N 5/0783, C12N 15/62, A61K 40/31, A61K 40/42, A61K 40/11, A61P 35/00

(54) **CELLS EXPRESSING MULTIPLE CHIMERIC ANTIGEN RECEPTOR (CAR) MOLECULES AND USES THEREFORE**
ZELLEN MIT EXPRESSION VON MEHREREN CHIMÄREN ANTIGENREZEPTOR (CAR)-MOLEKÜLEN UND VERWENDUNGEN DAFÜR
CELLULES EXPRIMANT DE MULTIPLES MOLÉCULES DE RÉCEPTEUR D'ANTIGÈNE CHIMÈRE (CAR) ET LEURS UTILISATIONS

(30) Priority: 04.03.2016 US 201662303466 P
(43) Date of publication of application: 09.01.2019
(73) Proprietor: Novartis AG, 4056 Basel (CH); The Trustees of The University of Pennsylvania, Philadelphia, PA 19104 (US)
(72) Inventor: DRANOFF, Glenn, Cambridge, Massachusetts 02139 (US)
(74) Representative: Wilding, James Roger
(86) International application number: PCT/IB2017/051267
(87) International publication number: WO 2017/149515

(56) References cited:
- WO-A1-2014/130657
- WO-A1-2015/075468
- WO-A1-2015/157252
- WO-A1-2016/028896
- WO-A1-2017/027291
- WO-A2-2016/014565
- WO-A2-2016/090034
- WO-A9-2015/188141
- ANONYMOUS: "Pilot Study of Autologous T-cells Redirected to Mesothelin and CD19 With a Chimeric Antigen Receptor in Patients With Metastatic Pancreatic Cancer (NCT02465983 on 2016_01_20)", 20 January 2016 (2016-01-20), XP002770264, Retrieved from the Internet <URL:https://clinicaltrials.gov/archive/NCT02465983/2016_01_20> [retrieved on 20150517]
- HERMANSON DAVID L ET AL: "Functional Chimeric Antigen Receptor-Expressing Natural Killer Cells Derived From Human Pluripotent Stem Cells", BLOOD, vol. 122, no. 21, 10 December 2013 (2013-12-10), THE AMERICAN SOCIETY OF HEMATOLOGY, US, pages 896, XP009180118, ISSN: 0006-4971

## Description

### FIELD OF THE INVENTION

The present disclosure relates generally to the use of immune effector cells (e.g., T cells, NK cells) engineered to express a Chimeric Antigen Receptor (CAR) that targets B cells and engineered to express a CAR that targets cells expressing a tumor antigen other than a B-Cell antigen, e.g., cells expressing a solid tumor antigen, myeloid tumor antigen, or cells expressing an antigen of a hematological tumor not of B-Cell origin, to treat a disease associated with expression of the tumor antigen.

### BACKGROUND OF THE INVENTION

Immunotherapy is a promising approach for the treatment of tumors. Immunotherapy with cells expressing chimeric antigen receptors (CARs) that target antigens expressed by the tumor has the advantage of targeted therapies that can invoke a rapid and sustained immune response against a tumor. CAR therapy has shown promising results in the clinic in treating some hematological cancers, such as B cell malignancies (see, e.g., Sadelain et al., Cancer Discovery 3:388-398 (2013)). The clinical results of the murine derived CART19 (i.e., "CTL019") have shown promise in establishing complete remissions in patients suffering with CLL, as well as in childhood ALL (see, e.g., Kalos et al., Sci Transl Med 3:95ra73 (2011), Porter et al., NEJM 365:725-733 (2011), Grupp et al., NEJM 368:1509-1518 (2013)). However, studies exploring CAR therapy for treating other cancers have demonstrated variable efficacy, in part due to the limited persistence and proliferation of the CAR-expressing cells *in vivo*.

Thus, there exists a need for CAR cell therapies with enhanced efficacy, e.g., enhanced proliferation or prolonged persistence in a patient.

WO 2015/188141 relates to mesothelin-targeted chimeric antigen receptors and uses thereof.

### SUMMARY OF THE INVENTION

The present invention provides a cell comprising a first chimeric antigen receptor (CAR) and a second CAR, each of which comprises an antigen binding domain, a transmembrane domain, and an intracellular signaling domain, wherein the antigen binding domain of said first CAR binds to a B-Cell antigen and the antigen binding domain of said second CAR binds to a tumor antigen other than a B-Cell antigen, wherein the tumor antigen other than a B-Cell antigen is a solid tumor antigen, further wherein
(i) the intracellular signaling domain of said first CAR comprises a costimulatory signaling domain, but not a primary signaling domain; and
(ii) the intracellular signaling domain of said second CAR comprises a costimulatory signaling domain and a primary signaling domain.

The invention also provides the cell of the invention for use in a method of treating cancer. The invention also provides the cell of the invention for use in a method of treating a disease associated with expression of a solid tumor antigen, wherein the disease is a tumor characterized as glioblastoma, ovarian cancer, lung cancer, prostate cancer, colorectal cancer, pancreatic cancer, breast carcinoma, adenocarcinoma or mesothelioma.

The invention further provides a nucleic acid encoding a first CAR and a second CAR, each of which comprises an antigen binding domain, a transmembrane domain, and an intracellular signalling domain, wherein the antigen binding domain of said first CAR binds to a B-Cell antigen and the antigen binding domain of said second CAR binds to a tumor antigen other than a B-Cell antigen, wherein the tumor antigen other than a B-Cell antigen is a solid tumor antigen, further wherein
(i) the intracellular signaling domain of said first CAR comprises a costimulatory signaling domain, but not a primary signaling domain; and
(ii) the intracellular signaling domain of said second CAR comprises a costimulatory signaling domain and a primary signaling domain.

This and other embodiments of the present invention are set out in the appended claims.

### DETAILED DESCRIPTION

The technical information set out below may in some respects go beyond the scope of the invention, which is defined by the appended claims. The additional technical information is provided to place the actual invention in a broader technical context and to illustrate possible related technical developments.

Any incidental references to methods for treatment of the human or animal body by surgery or therapy and diagnostic methods practiced on the human or animal body are not to be construed as claiming protection for such methods as such, but are instead to be construed as referring to products, in particular substances or compositions, for use in any of these methods.

The present disclosure features, at least in part, methods and compositions for treating a disease associated with expression of a tumor antigen, *e.g.,* a cancer, in a subject using an immune effector cell (e.g., T cell) engineered to expresss a first chimeric antigen receptor (CAR) and a second CAR, wherein the antigen binding domain of said first CAR binds to a B-Cell antigen and the antigen binding domain of said second CAR binds to a tumor antigen other than a B-Cell antigen, e.g., to enhance the efficacy (e.g., the persistence and/or proliferation of the tumor antigen-targeting CAR-expressing immune effector cell in a patient) of the CAR-expressing immune effector cell therapy. Without wishing to be bound by theory, treatment with an immune effector cell expressing a CAR targeting a B-Cell antigen and a CAR targeting a tumor antigen enhances the anti tumor efficacy of the tumor antigen-targeting CAR-expressing immune effector cell in a subject, *e.g*., by one or more of: increasing the proliferation of said CAR-expressing immune effector cells and/or increasing the in vivo persistence of said CAR expressing immune effector cells, *e.g.,* as compared to administering an immune effector cell expressing only the tumor-targeting CAR (e.g., not expressing the CAR tareting a B-Cell antigen). In aspects, the B-Cell antigen and the tumor antigen other than a B-Cell antigen are not expressed on the same cell (e.g., the B-Cell antigen is not expressed on the cell, e.g., tumor cell, which expresses the tumor antigen).

In an aspect, the invention relates to a cell that includes a first chimeric antigen receptor (CAR) and a second CAR, each of which includes an antigen binding domain, a transmembrane domain, and an intracellular signaling domain, wherein the antigen binding domain of said first CAR binds to a B-Cell antigen and the antigen binding domain of said second CAR binds to a tumor antigen other than a B-Cell antigen. In aspects of the invention, the B-Cell antigen targeted by the first CAR and the tumor antigen other than a B-Cell antigen targeted by the second CAR are not expressed on the same cell.

In instances disclosed herein, the second CAR binds: (a) a solid tumor antigen; (b) a myeloid tumor antigen; or (c) an antigen of a hematological tumor not of B-cell lineage. In the invention, the antigen binding domain of the second CAR binds to a tumor antigen other than a B-Cell antigen, wherein the tumor antigen other than a B-Cell antigen is a solid tumor antigen.

Also in the invention, (i) the intracellular signaling domain of the first CAR comprises a costimulatory signaling domain, but not a primary signaling domain; and (ii) the intracellular signaling domain of the second CAR comprises a costimulatory signaling domain and a primary signaling domain.

In embodiments, the B-Cell antigen is selected from the group consisting of CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, 5, 7, IL-7/3R, IL7/4/3R, and TL4R.

In embodiments, the B-Cell antigen is selected from the group consisting of CD19, CD20, CD22, FcRn5, FcRn2, BCMA, CS-1, and CD138

In one aspect, the cell includes a first chimeric antigen receptor that includes an antigen binding domain that binds a B-Cell antigen that is BCMA. In embodiments, the antigen binding domain of said first CAR includes a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 12 or 13. In embodiments, the antigen binding domain of said first CAR further includes a light chain complementary determining region 1 (LC CDR1), a light chain complementary determining region 2 (LC CDR2), and a light chain complementary determining region 3 (LC CDR3) of any light chain binding domain amino acid sequence listed in Table 12 or 13. In embodiments, the antigen binding domain of said first CAR includes: (i) the amino acid sequence of any light chain variable region listed in Table 12 or 13; (ii) an amino acid sequence having at least one, two or three modifications but not more than 20 or 10 modifications of the amino acid sequence of any of the light chain variable regions provided in Table 12 or 13; or (iii) an amino acid sequence with 95-99% identity to the amino acid sequence of any of the light chain variable regions provided in Table 12 or 13. In embodiments, the antigen binding domain of said first CAR includes: (i) the amino acid sequence of any heavy chain variable region listed in Table 12 or 13; (ii) an amino acid sequence having at least one, two or three modifications but not more than 20 or 10 modifications of the amino acid sequence of any of the heavy chain variable regions provided in Table 12 or 13; or (iii) an amino acid sequence with 95-99% identity to the amino acid sequence of any of the heavy chain variable regions provided in Table 12 or 13. In embodiments, the antigen binding domain of said first CAR includes a polypeptide having the amino acid sequence of any light chain variable region listed in Table 12 or 13, and the amino acid sequence of any heavy chain variable region listed in Table 12 or 13. In embodiments, the antigen binding domain of said first CAR includes a polypeptide having a sequence of SEQ ID NO: 349; SEQ ID NO: 339, SEQ ID NO: 340; SEQ ID NO: 341; SEQ ID NO: 342; SEQ ID NO: 343; SEQ ID NO: 344, SEQ ID NO: 345, SEQ ID NO: 346, SEQ ID NO: 347, SEQ ID NO: 348, SEQ ID NO: 350, SEQ ID NO: 351, SEQ ID NO: 352, SEQ ID NO: 353, SEQ ID NO: 429, SEQ ID NO: 430, SEQ ID NO: 431, SEQ ID NO: 432, SEQ ID NO: 433, SEQ ID NO: 434, SEQ ID NO: 435, SEQ ID NO: 436, SEQ ID NO: 437, SEQ ID NO: 438, SEQ ID NO: 439, SEQ ID NO: 440, SEQ ID NO: 441, SEQ ID NO: 442, SEQ ID NO: 443, SEQ ID NO: 444, SEQ ID NO: 445, SEQ ID NO: 446, SEQ ID NO: 447, SEQ ID NO: 448, SEQ ID NO: 449, SEQ ID NO: 563, SEQ ID NO: 564, SEQ ID NO: 565 or SEQ ID NO: 566.

In another aspect, the cell includes a first chimeric antigen receptor that includes an antigen binding domain that binds a B-Cell antigen that is CD19. In embodiments, the antigen binding domain of said first CAR includes a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 6, Table 7 or Table 9. In embodiments, the antigen binding domain of said first CAR further includes a light chain complementary determining region 1 (LLC CDR1), a light chain complementary determining region 2 (LC CDR2), and a light chain complementary determining region 3 (LC CDR3) of any light chain binding domain amino acid sequence listed in Table 6, Table 8 or Table 9. In embodiments, the antigen binding domain of said first CAR includes: (i) the amino acid sequence of any light chain variable region listed in Table 6 or Table 9; (ii) an amino acid sequence having at least one, two or three modifications but not more than 20 or 10 modifications of the amino acid sequence of any of the light chain variable regions provided in Table 6 or Table 9; or (iii) an amino acid sequence with 95-99% identity to the amino acid sequence of any of the light chain variable regions provided in Table 6 or Table 9. In embodiments, the antigen binding domain of said first CAR includes: (i) the amino acid sequence of any heavy chain variable region listed in Table 6 or Table 9; (ii) an amino acid sequence having at least one, two or three modifications but not more than 20 or 10 modifications of the amino acid sequence of any of the heavy chain variable regions provided in Table 6 or Table 9; or (iii) an amino acid sequence with 95-99% identity to the amino acid sequence of any of the heavy chain variable regions provided in Table 6 or Table 9. In embodiments, the antigen binding domain of said first CAR includes a polypeptide having the amino acid sequence of any light chain variable region listed in Table 6 or Table 9, and the amino acid sequence of any heavy chain variable region listed in Table 6 or Table 9. In embodiments, the antigen binding domain of said first CAR includes a polypeptide having a sequence of SEQ ID NO: 83; SEQ ID NO: 84, SEQ ID NO: 85; SEQ ID NO: 86; SEQ ID NO: 87; SEQ ID NO: 88; SEQ ID NO: 89, SEQ ID NO: 90, SEQ ID NO: 91, SEQ ID NO: 92, SEQ ID NO: 93, SEQ ID NO: 94, SEQ ID NO: 95, or SEQ ID NO: 112.

In an instance disclosed herein, the cell includes a second CAR that includes an antigen binding domain that binds a myeloid tumor antigen, and wherein said myeloid tumor antigen is selected from the group consisting of CD123, CD33 and CLL-1.

In an instance disclosed herein, the cell includes a second CAR that includes an antigen binding domain that binds a T cell lymphoma antigen.

In the invention, the cell includes a second CAR that includes an antigen binding domain that binds a solid tumor antigen, e.g., wherein said solid tumor antigen is selected from the group consisting of EGFRvIII, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, CD24, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, GFRα4, and a peptide of any of these antigens presented on MHC. In embodiments, the solid tumor antigen is selected from the group consisting of CLDN6, mesothelin and EGFRvIII.

In one aspect, the cell includes a second chimeric antigen receptor that includes an antigen binding domain that binds EGFRvIII. In embodiments, the antigen binding domain of said second CAR includes a heavy chain complementary determining region 1 (HC CDR1), a heavy chain complementary determining region 2 (HC CDR2), and a heavy chain complementary determining region 3 (HC CDR3) of any anti-EGFRvIII heavy chain binding domain amino acid sequence listed in Table 5. In embodiments, the antigen binding domain of said second CAR further includes a light chain complementary determining region 1 (LC CDR1), a light chain complementary determining region 2 (LC CDR2), and a light chain complementary determining region 3 (LC CDR3) of any anti-EGFRvIII light chain binding domain amino acid sequence listed in Table 5. In embodiments, the antigen binding domain of said second CAR includes: (i) the amino acid sequence of any anti-EGFRvIII light chain variable region listed in Table 5; (ii) an amino acid sequence having at least one, two or three modifications but not more than 20 or 10 modifications of the amino acid sequence of any of the anti-EGFRvIII light chain variable regions provided in Table 5; or (iii) an amino acid sequence with 95-99% identity to the amino acid sequence of any of the anti-EGFRvIII light chain variable regions provided in Table 5. In embodiments, the antigen binding domain of said second CAR includes: (i) the amino acid sequence of any anti-EGFRvIII heavy chain variable region listed in Table 5; (ii) an amino acid sequence having at least one, two or three modifications but not more than 20 or 10 modifications of the amino acid sequence of any of the anti-EGFRvIII heavy chain variable regions provided in Table 5; or (iii) an amino acid sequence with 95-99% identity to the amino acid sequence of any of the anti-EGFRvIII heavy chain variable regions provided in Table 5. In embodiments, the antigen binding domain of said second CAR includes a polypeptide having the amino acid sequence of any anti-EGFRvIII light chain variable region listed in Table 5, and the amino acid sequence of any anti-EGFRvIII heavy chain variable region listed in Table 5. In embodiments, the antigen binding domain of said second CAR includes a polypeptide having a sequence of any of SEQ ID NOS: 71-79.

In one aspect, the cell includes a second chimeric antigen receptor that includes an antigen binding domain that binds mesothelin. In embodiments, the antigen binding domain of said second CAR includes a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 2 or 3. In embodiments, the antigen binding domain of said second CAR further includes a light chain complementary determining region 1 (LC CDR1), a light chain complementary determining region 2 (LC CDR2), and a light chain complementary determining region 3 (LC CDR3) of any light chain binding domain amino acid sequence listed in Table 2 or 4. In embodiments, the antigen binding domain of said second CAR includes: (i) the amino acid sequence of any light chain variable region listed in Table 2; (ii) an amino acid sequence having at least one, two or three modifications but not more than 20 or 10 modifications of the amino acid sequence of any of the light chain variable regions provided in Table 2; or (iii) an amino acid sequence with 95-99% identity to the amino acid sequence of any of the light chain variable regions provided in Table 2. In embodiments, the antigen binding domain of said second CAR includes: (i) the amino acid sequence of any heavy chain variable region listed in Table 2; (ii) an amino acid sequence having at least one, two or three modifications but not more than 20 or 10 modifications of the amino acid sequence of any of the heavy chain variable regions provided in Table 2; or (iii) an amino acid sequence with 95-99% identity to the amino acid sequence of any of the heavy chain variable regions provided in Table 2. In embodiments, the antigen binding domain of said second CAR includes a polypeptide having the amino acid sequence of any light chain variable region listed in Table 2, and the amino acid sequence of any heavy chain variable region listed in Table 2. In embodiments, the antigen binding domain of said second CAR includes a polypeptide having a sequence of any one of SEQ ID NOS: 46-70.

In embodiments, including in any of the aforementioned aspects and embodiments, the antigen binding domain of said first CAR is in the format of an scFv.

In embodiments, including in any of the aforementioned aspects and embodiments, the antigen binding domain of said second CAR is in the format of an scFv.

In some instances disclosed herein, the intracellular signaling domain of said first or said second CAR includes one or more primary signaling domains , e.g., as described herein.

In some instances disclosed herein, the intracellular signaling domains of said first CAR and said second CAR include a primary signaling domain , e.g., as described herein.

In some instances disclosed herein, the intracellular signaling domain of said first or said second CAR includes one or more costimulatory signaling domains, e.g., as described herein.

In embodiments, including in any of the aforementioned aspects and embodiments, the intracellular signaling domains of said first CAR and said second CAR include one or more costimulatory signaling domains, e.g., as described herein.

In the invention, (i) the intracellular signaling domain of said first CAR comprises a costimulatory signaling domain, but not a primary signaling domain; and (ii) the intracellular signaling domain of said second CAR comprises a costimulatory signaling domain and a primary signaling domain.

In embodiments, including in any of the aforementioned aspects and embodiments, the primary signaling domains include a CD3-zeta stimulatory domain, e.g., as described herein.

In embodiments, including in any of the aforementioned aspects and embodiments, the costimulatory signaling domain is an intracellular domain of a costimulatory protein selected from the group consisting of CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, and a ligand that specifically binds with CD83.

In one instance or embodiment of any of the methods and compositions described herein, the transmembrane domain of the first CAR molecule, the second CAR molecule, or both the first CAR molecule and second CAR moleucle comprises a transmembrane domain from a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some embodiments, the transmembrane domain of the first CAR, the second CAR, or both the first CAR and second CAR comprises the amino acid sequence of SEQ ID NO: 12, an amino acid sequence comprises at least one, two or three modifications but not more than 20, 10 or 5 modifications of the amino acid sequence of SEQ ID NO: 12, or a sequence with 95-99% identity to the amino acid sequence of SEQ ID NO:12.

In embodiments, the transmembrane domains of the first CAR molecule and second CAR molecule are the same. In other embodiments, the transmembrane domains of the first CAR molecule and second CAR molecule are different.

In one instance or embodiment of any of the methods and compositions described herein, the antigen binding domain of the first CAR molecule, the antigen binding domain of the second CAR molecule, or the antigen binding domain of both the first CAR molecule and the second CAR molecule is connected to a transmembrane domain by a hinge region. In some embodiments, the hinge region comprises SEQ ID NO:4, or a sequence with 95-99% identity thereof.

In one instance or embodiment of any of the methods and compositions described herein, the intracellular signaling domain of the first CAR molecule, the second CAR molecule or both the first CAR molecule and second CAR molecule comprises a costimulatory signaling domain comprising a functional signaling domain obtained from a protein selected from the group consisting of a MHC class I molecule, a TNF receptor protein, an Immunoglobulin-like protein, a cytokine receptor, an integrin, a signaling lymphocytic activation molecule (SLAM protein), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMFS), SELPLG (CD162), LTBR, LAT, GADS, SLP-76, PAG/Cbp, CD19a, and a ligand that specifically binds with CD83. In some embodiments, the costimulatory domain comprises the amino acid sequence of SEQ ID NO:14, or an amino acid sequence having at least one, two or three modifications but not more than 20, 10 or 5 modifications of the amino acid sequence of SEQ ID NO:14, or an amino acid sequence with 95-99% identity to the amino acid sequence of SEQ ID NO:14. In some embodiments, the intracellular signaling domain comprises a functional signaling domain of 4-1BB and/or a functional signaling domain of CD3 zeta. In some embodiments, the intracellular signaling domain comprises the amino acid sequence of SEQ ID NO: 14 and/or the amino acid sequence of SEQ ID NO:18 or SEQ ID NO:20; or an amino acid sequence having at least one, two or three modifications but not more than 20, 10 or 5 modifications of the amino acid sequence of SEQ ID NO:14 and/or the amino acid sequence of SEQ ID NO:18 or SEQ ID NO:20; or an amino acid sequence with 95-99% identity to the amino acid sequence of SEQ ID NO:14 and/or the amino acid sequence of SEQ ID NO:18 or SEQ ID NO:20. In some embodiments, the intracellular signaling domain comprises the amino acid sequence of SEQ ID NO:14 and the amino acid sequence of SEQ ID NO:18 or SEQ ID NO:20, wherein the amino acid sequences comprising the intracellular signaling domain are expressed in the same frame and as a single polypeptide chain.

In the invention, the first CAR molecule ( the B-Cell antigen-targeting CAR molecule) comprises an intracellular signaling domain that comprises a costimulatory signaling domain, e.g., as described herein, but does not comprise a primary signaling domain. In other instances disclosed herein, the first CAR molecule (e.g., the B-Cell antigen-targeting CAR molecule) comprises an intracellular signaling domain that comprises a costimulatory signaling domain, e.g., as described herein, and a primary signaling domain, e.g., as described herein. In some instances disclosed herein, the second CAR molecule (e.g., the tumor antigen-targeting CAR molecule) comprises an intracellular signaling domain that comprises a costimulatory signaling domain, e.g., as described herein, but does not comprise a primary signaling domain. In some instances disclosed herein, the second CAR molecule (e.g., the tumor antigen-targeting CAR molecule) comprises an intracellular signaling domain that comprises a primary signaling domain, e.g., as described herein, but does not comprise a costimulatory signaling domain. In the invention, the second CAR molecule ( the tumor antigen-targeting CAR molecule) comprises an intracellular signaling domain that comprises a costimulatory signaling domain, e.g., as described herein, and a primary signaling domain, e.g., as described herein.

In the invention, the first CAR molecule ( the B-Cell antigen-targeting CAR molecule) comprises an intracellular signaling domain that comprises a costimulatory signaling domain, e.g., as described herein, but does not comprise a primary signaling domain, and the second CAR molecule ( the tumor antigen-targeting CAR molecule), comprises an intracellular signaling domain that comprises a costimulatory signaling domain, e.g., as described herein, and a primary signaling domain, e.g., as described herein.

In another instance disclosed herein, the first CAR molecule (e.g., the B-Cell antigen-targeting CAR molecule) comprises an intracellular signaling domain that comprises a costimulatory signaling domain, e.g., as described herein, and a primary signaling domain, e.g., as described herein, and the second CAR molecule (e.g., the tumor antigen-targeting CAR molecule), comprises an intracellular signaling domain that comprises a costimulatory signaling domain, e.g., as described herein, and a primary signaling domain, e.g., as described herein.

In an instance or embodiment of any of the methods and compositions described herein, the first CAR molecule, the second CAR molecule, or both the first CAR molecule and the second CAR molecule further comprises a leader sequence comprising the amino acid sequence of SEQ ID NO:2.

In instances and embodiments, including in any of the aforementioned aspects and embodiments, the costimulatory domain of both said first and said second CAR include an intracellular domain of 4-1BB, e.g., as described herein.

In instances and embodiments, including in any of the aforementioned aspects and embodiments, the one or more of said costimulatory domains includes an intracellular domain of CD28, e.g., as described herein.

In instances and embodiments, including in any of the aforementioned aspects and embodiments, the first or second CAR includes two costimulatory domains: (1) a 4-1BB costimulatory domain, e.g., as described herein; and (2) a CD28 costimulatory domain, e.g., as described herein.

In an aspect (including in any of the aforementioned aspects and embodiments that include a BCMA CAR) the antigen binding domain of said first CAR binds BCMA and the first CAR includes a polypeptide having an amino acid sequence selected from the group consisting of SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, and SEQ ID NO: 999.

In an aspect (including in any of the aforementioned aspects and embodiments that include a CD19 CAR) the antigen binding domain of said first CAR binds CD19 and the first CAR includes a polypeptide having an amino acid sequence selected from the group consisting of SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, and SEQ ID NO: 281.

In an aspect (including in any of the aforementioned aspects and embodiments that include a EGFRvIII CAR) the antigen binding domain of said second CAR binds EGFRvIII and the second CAR includes a polypeptide having an amino acid sequence selected from the group consisting of SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090, and SEQ ID NO: 1096.

In an aspect (including in any of the aforementioned aspects and embodiments that include a mesothelin CAR) the antigen binding domain of said second CAR binds mesothelin and the second CAR includes a polypeptide having an amino acid sequence selected from the group consisting of SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, and SEQ ID NO: 306.

In another aspect, the disclosure provides a cell which includes a CAR, e.g., a bispecific CAR (e.g., as described herein), which includes a first antigen binding domain that binds a B-Cell antigen, e.g., as described herein, a second antigen binding domain that binds a tumor antigen, e.g., as described herein, a transmembrane domain, e.g., as described herein, and an intracellular signaling domain, e.g., as described herein. In instances, the first antigen binding domain binds CD19, e.g., includes a CD19 binding domain described herein. In instances, the first antigen binding domain binds BCMA, e.g., includes a BCMA binding domain described herein. In instances, the second antigen biding domain binds a solid tumor antigen, a myeloid tumor antigen, or an antigen of a hematological tumor not of B-Cell lineage. In instances, the second antigen binding domain binds a solid tumor antigen, e.g., as described herein. In instances, the second antigen binding domain binds EGFRvIII (e.g., includes a EGFRvIII binding domain described herein). In other instances, the second antigen binding domain binds mesothelin (e.g., includes a mesothelin binding domain described herein). In instances, the CAR includes a first antigen binding domain to CD19, e.g., as described herein, and a second antigen binding domain to EGFRvIII, e.g., as described herein. In instances, the CAR includes a first antigen binding domain to BCMA, e.g., as described herein, and a second antigen binding domain to EGFRvIII, e.g., as described herein. In instances, the CAR includes a first antigen binding domain to CD19, e.g., as described herein, and a second antigen binding domain to mesothelin, e.g., as described herein. In instances, the CAR includes a first antigen binding domain to BCMA, e.g., as described herein, and a second antigen binding domain to mesothelin, e.g., as described herein. In instances, the CAR includes an intracellular signaling domain that includes a CD3z primary signaling domain, e.g., as described herein, and a 4-1BB costimulatory signaling domain, e.g., as described herein. In instances, the CAR includes an intracellular signaling domain that includes a CD3z primary signaling domain, e.g., as described herein, and a CD28 costimulatory signaling domain, e.g., as described herein.

In an aspect (including in any of the aforementioned aspects and embodiments), the cell is derived from a patient diagnosed with a myeloid tumor, or a hematological tumor not of B-Cell lineage.

In an aspect (including in any of the aforementioned aspects and embodiments), the patient is diagnosed with a myeloid tumor expressing an antigen selected from the group consisting of CD123, CD33 and CLL-1.

In an aspect (including in any of the aforementioned aspects and embodiments), the cell is derived from a patient diagnosed with a solid tumor. In embodiments, the patient is diagnosed with a solid tumor expressing an antigen selected from the group consisting of: EGFRvIII, mesothelin, GD2, Tn Ag, PSMA, TAG72, CD44v6, CEA, EPCAM, KIT, IL-13Ra2, GD3, CD171, IL-11Ra, PSCA, VEGFR2, LewisY, CD24, PDGFR-beta, SSEA-4, folate receptor alpha, ERBB2, Her2/neu, MUC1, EGFR, NCAM, Ephrin B2, CAIX, LMP2, sLe, HMWMAA, o-acetyl-GD2, folate receptor beta, TEMI/CD248, TEM7R, FAP, Legumain, HPV E6 or E7, CLDN6, TSHR, GPRC5D, ALK, Plysialic acid, PLAC1, globoH, NY-BR-1, UPK2, HAVCR1, ADRB3, PANX3, GPR20, Ly6k, OR51E2, TARP, and GFRα4.

In an aspect (including in any of the aforementioned aspects and embodiments), the cell is a human cell and is not derived from a patient diagnosed with a tumor.

In an aspect (including in any of the aforementioned aspects and embodiments), the cell is a T cell, a natural killer (NK) cell, a cytotoxic T lymphocyte (CTL), a tumor infiltrating lymphocyte (TIL), or a regulatory T cell.

In another aspect, disclosed is a cell for use in a method of stimulating a T cell-mediated immune response to a myeloid tumor cell in a mammal, the method including administering to a mammal an effective amount of a cell as described herein, e.g., a cell of any of the aforementioned aspects and embodiments.

In another aspect, disclosed is a cell for use in a method of providing an anti-myeloid tumor, immunity in a mammal, including administering to the mammal an effective amount of a cell as described herein, e.g., in any of the aforementioned aspects and embodiments.

In another aspect, disclosed is a cell for use in a method of treating a mammal having a disease associated with expression of a myeloid tumor antigen, said method including administering an effective amount of a cell as described herein, e.g., in any of the aforementioned aspects and embodiments.

The invention provides a cell of the invention for use in a method of treating cancer, optionally wherein the cancer is a solid tumor.

In embodiments or instances of the aspects involving a method for stimulating a T cell-mediated immune response to a myeloid tumor cell in a mammal, a method of providing an anti-myeloid tumor, immunity in a mammal and/or a method of treating a mammal having a disease associated with expression of a myeloid tumor antigen, the myeloid tumor expresses an antigen selected from the group consisting of CD123, CD33 and CLL-1. In embodiments, the mammal has a tumor characterized as acute myeloid leukemia (AML), acute lymphoblastic B-cell leukemia (B-cell acute lymphoid leukemia, BALL), acute lymphoblastic T-cell leukemia (T cell acute lymphoid leukemia (TALL)), B-cell prolymphocytic leukemia, chronic lymphocytic leukemia, chronic myeloid leukemia (CML), myelodysplastic syndrome, plasma cell myeloma, or a combination thereof.

In another aspect, disclosed is a cell for use in a method of stimulating a T cell-mediated immune response to a hematological tumor not of B-cell lineage, e.g., a T cell lymphoma tumor cell in a mammal, the method including administering to a mammal an effective amount of a cell as described herein, e.g., a cell of any of the aforementioned aspects and embodiments.

In another aspect, disclosed is a cell for use in a method of providing immunity to an anti-hematological tumor not of B-cell lineage, e.g., an anti-T cell lymphoma tumor immunity, in a mammal, including administering to the mammal an effective amount of a cell as described herein, e.g., in any of the aforementioned aspects and embodiments.

In another aspect, disclosed is a cell for use in a method of treating a mammal having a disease associated with expression of an antigen of a hematological tumor not of B-cell lineage, e.g., a T cell lymphoma tumor antigen, said method including administering an effective amount of a cell as described herein, e.g., in any of the aforementioned aspects and embodiments.

In another aspect, disclosed is a cell for use in a method of stimulating a T cell-mediated immune response to a solid tumor cell in a mammal, the method including administering to a mammal an effective amount of a cell as described herein, e.g., a cell of any of the aforementioned aspects and embodiments.

In another aspect, disclosed is a cell for use in a method of providing an anti-solid tumor, immunity in a mammal, including administering to the mammal an effective amount of a cell as described herein, e.g., in any of the aforementioned aspects and embodiments.

In another aspect, disclosed is a cell for use in a method of treating a mammal having a disease associated with expression of a solid tumor antigen, said method including administering an effective amount of a cell as described herein, e.g., in any of the aforementioned aspects and embodiments.

The invention provides a cell of the invention for use in a method of treating cancer, optionally wherein the cancer is a solid tumor.

In embodiments or instances of the aspects involving a method for stimulating a T cell-mediated immune response to a solid tumor cell in a mammal, a method of providing an anti-solid tumor, immunity in a mammal and/or a method of treating a mammal having a disease associated with expression of a solid tumor antigen, the solid tumor cell expresses an antigen selected from the group consisting of: EGFRvIII, mesothelin, CS-1, GD2, Tn Ag, PSMA, TAG72, CD44v6, CEA, EPCAM, KIT, IL-13Ra2, GD3, CD171, IL-11Ra, PSCA, VEGFR2, LewisY, CD24, PDGFR-beta, SSEA-4, folate receptor alpha, ERBB2, Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, CLDN6, TSHR, GPRC5D, ALK, Plysialic acid, PLAC1, globoH, NY-BR-1, UPK2, HAVCR1, ADRB3, PANX3, GPR20, Ly6k, OR51E2, TARP, and GFRα4, e.g., EGFRvIII or mesothelin. In embodiments, the mammal has a tumor characterized as glioblastoma, ovarian cancer, lung cancer, prostate cancer, colorectal cancer, pancreatic cancer, breast carcinoma, adenocarcinoma or mesothelioma.

In some embodiments, the solid tumor antigen is present in/on a mesothelioma (*e.g.,* a malignant pleural mesothelioma), a lung cancer (*e.g.,* non-small cell lung cancer, small cell lung cancer, squamous cell lung cancer, or large cell lung cancer), a pancreatic cancer (*e.g.,* pancreatic ductal adenocarcinoma), an esophageal adenocarcinoma, an ovarian cancer, a breast cancer, a colorectal cancer, a bladder cancer or any combination thereof, or a metastasis of any of the aforementioned cancers. In one embodiment or instance of any of the methods and compositions described herein, the disease associated with expression of the tumor antigen is a pancreatic cancer, *e.g.,* a metastatic pancreatic ductal adenocarcinoma (PDA). In one embodiment, the pancreatic cancer is in a subject who has progressed on at least one prior standard therapy. In one embodiment, the disease is mesothelioma (*e.g.,* malignant pleural mesothelioma), *e.g.,* in a subject who has progressed on at least one prior standard therapy. In one embodiment, the disease is ovarian cancer, *e.g.,* serous epithelial ovarian cancer, *e.g.,* in a subject who has progressed after at least one prior regimen of standard therapy. In one embodiment, the disease is mesothelioma, malignant pleural mesothelioma, non-small cell lung cancer, small cell lung cancer, squamous cell lung cancer, or large cell lung cancer, pancreatic cancer, pancreatic ductal adenocarcinoma, pancreatic metastatic, esophageal adenocarcinoma, breast cancer, ovarian cancer, colorectal cancer and bladder cancer, or any combination thereof.

In embodiments, the cells are autologous to the treated mammal.

In embodiments, the cells are allogeneic to the treated mammal.

In embodiments, the mammal is a human.

In another aspect, the invention relates to a method, including any of the aforementioned methods, wherein the administering of the cells of the invention, e.g., as described herein, results in partial or complete elimination of said tumor cells and, thereafter, continue to persist in said subject at a level greater than, or for a length of time longer than, otherwise identical cells that lack the first CAR.

In embodiments or instances of the methods described herein, the mammal is administered a lymphodepleting therapy prior to, concurrently with, or after administration of said cells.

In embodiments or instances of the methods described herein, mammal is not administered a lympodepleting therapy prior to or concurrently with administration of said cells.

In embodiments or instances of any of the methods and compositions described herein, the method can further comprise administering a lymphodepleting agent. In one embodiment, the lymphodepleting agent reduces the level of T cells, e.g., regulatory T cells, and/or regulatory B cells, as compared to the level prior to administration of the lymphodepleting agent. In one embodiment, the lymphodepleting agent comprises fludarabine, cyclophosphamide, corticosteroids, alemtuzumab, or total body irradiation (TBI), or a combination thereof.

Any of the methods and compositions described herein can further comprise administering an additional therapeutic agent that treats the disease associated with a tumor antigen. In one embodiment, the additional therapeutic agent is an anti-cancer therapeutic agent.

In another aspect, the disclosure provides a nucleic acid encoding the first CAR and the second CAR of any one of the aforementioned aspects and embodiments, e.g., as described herein. The invention provides a nucleic acid encoding a first CAR and a second CAR, each of which comprises an antigen binding domain, a transmembrane domain, and an intracellular signalling domain, wherein the antigen binding domain of said first CAR binds to a B-Cell antigen and the antigen binding domain of said second CAR binds to a tumor antigen other than a B-Cell antigen, wherein the tumor antigen other than a B-Cell antigen is a solid tumor antigen, further wherein (i) the intracellular signaling domain of said first CAR comprises a costimulatory signaling domain, but not a primary signaling domain; and (ii) the intracellular signaling domain of said second CAR comprises a costimulatory signaling domain and a primary signaling domain.

In embodiments, the sequence of said first CAR and said second CAR are separated by an independent ribosomal entry site, a promoter element, or a sequence encoding a T2A, P2A, E2A, or F2A element.

In another aspect, the disclosure provides a vector including the nucleic acid of the aforementioned aspect and embodiments, e.g., as described herein. In instances, the vector is a lentiviral vector.

In another aspect, the disclosure provides a composition including a first nucleic acid encoding the first CAR (e.g., a CAR comprising a binding domain to a B-Cell antigen, e.g., as described herein) of any one of the preceding aspects and embodiments, and a second nucleic acid encoding the second CAR (e.g., a CAR comprising a binding domain to a tumor antigen, e.g., as described herein) of any one of the preceding aspects and embodiments. In instances, the first and the second nucleic acids are included within separate vectors. In instances, the vectors are lentiviral vectors.

In another aspect, the disclosure provides a method of generating the cell of any one of the aforementioned aspects and embodiments, e.g., a cell as described herein, including introducing into said cell the nucleic acid of any one of the preceding nucleic acid aspects and embodiments, e.g., as described herein, the vector of any one of the preceding vector aspects and instances, e.g., as described herein, or the composition of any one of the preceding composition aspects and instances, e.g., as described herein,.

In another aspect, the disclosure provides a method of generating the cell of any one of the preceding aspects and embodiments, including introducing into said cell a first vector including nucleic acid encoding the first CAR of any one of the aforementioned aspects and embodiments, e.g., as described herein, and introducing into said cells a second vector including nucleic acid encoding the second CAR of any one of the aforementioned aspects and instances, e.g., as described herein. In instances, the introduction of said first vector and said second vector is simultaneous. In instances, the introduction of said first vector and said second vector is sequential.

In another aspect, the disclosure provides a cell including nucleic acid encoding the first CAR of any one of the aforementioned aspects and embodiments, e.g., as described herein, and the second CAR of any one of the aforementioned aspects and embodiments, e.g., as described herein.

In another aspect, the disclosure provides a cell described herein, e.g., a cell expressing a CAR which binds a B-Cell antigen, e.g., described herein, and expressing a CAR which binds a tumor antigen other than a B-Cell antigen, e.g., described herein, for use as a medicament. In another aspect, the disclosure provides a cell described herein, e.g., a cell expressing a CAR which binds a B-Cell antigen, e.g., described herein, and expressing a CAR which binds a tumor antigen other than a B-Cell antigen, e.g., described herein, for use as a medicament for the treatment of a disease associated with the expression of the tumor antigen other than a B-Cell antigen. In another aspect, the disclosure provides a cell described herein, e.g., a cell expressing a CAR which binds a B-Cell antigen, e.g., described herein, and expressing a CAR which binds a tumor antigen other than a B-Cell antigen, e.g., described herein, for use as a medicament for the treatment of cancer, e.g., a cancer expressing the tumor antigen other than a B-Cell antigen. In another aspect, the disclosure provides a cell described herein, e.g., a cell expressing a CAR which binds a B-Cell antigen, e.g., described herein, and expressing a CAR which binds a tumor antigen other than a B-Cell antigen, e.g., described herein; a nucleic acid described herein, or a composition described herein; for use in the manufacture of a medicament.

The invention provides a cell of the invention for use in a method of treating cancer. In some embodiments, the cancer is a sold tumor. The invention provides a cell of the invention for use in a method of treating a disease associated with expression of a solid tumor antigen, wherein the disease is a tumor characterized as glioblastoma, ovarian cancer, lung cancer, prostate cancer, colorectal cancer, pancreatic cancer, breast carcinoma, adenocarcinoma or mesothelioma.

Unless otherwise defined, all technical and scientific terms used herein have the same meaning as commonly understood by one of ordinary skill in the art to which this invention belongs. Although methods and materials similar or equivalent to those described herein can be used in the practice or testing of the present disclosure, suitable methods and materials are described below.

In addition, the materials, methods, and examples are illustrative only and not intended to be limiting. Headings, sub-headings or numbered or lettered elements, *e.g.,* (a), (b), (i) etc, are presented merely for ease of reading. The use of headings or numbered or lettered elements in this document does not require the steps or elements be performed in alphabetical order or that the steps or elements are necessarily discrete from one another. Other features, objects, and advantages of the invention will be apparent from the description and drawings, and from the claims.

### BRIEF DESCRIPTION OF THE DRAWINGS

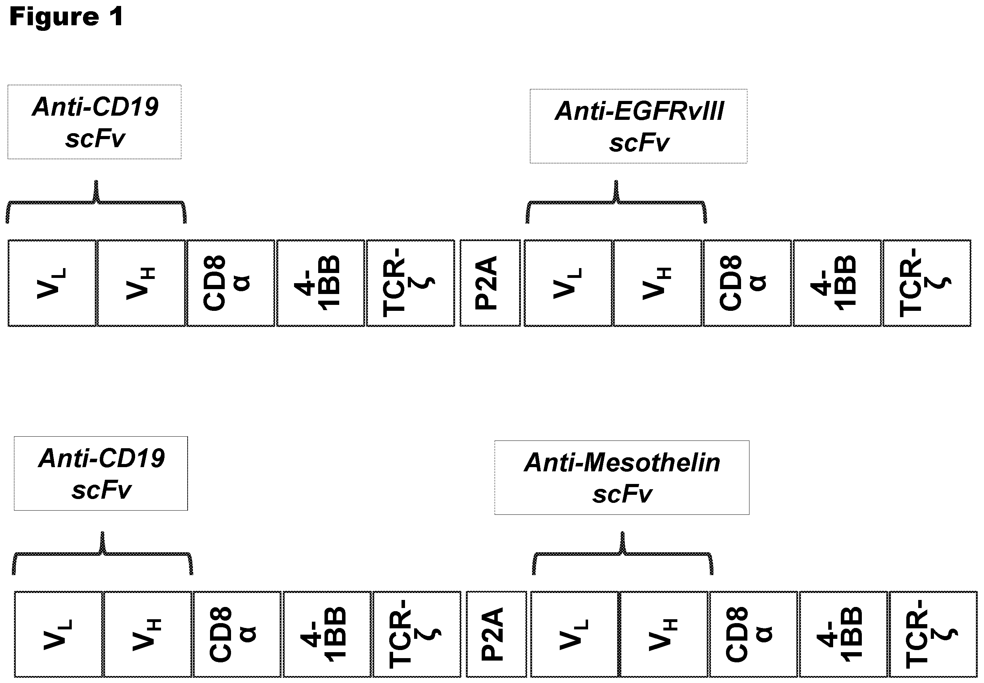

**Figure 1** shows a diagram of a nucleic acic construct for bicistronic expression of a B-cell antigen CAR and a solid tumor antigen CAR. The top construct encodes a CD19 CAR (B-cell antigen CAR) and an EGFRvIII CAR (solid tumor CAR), separated by a P2A protease cleavage site. The bottom construct encodes a CD19 CAR (B-cell antigen CAR) and a Mesothelin CAR (solid tumor CAR), separated by a P2A protease cleavage site.

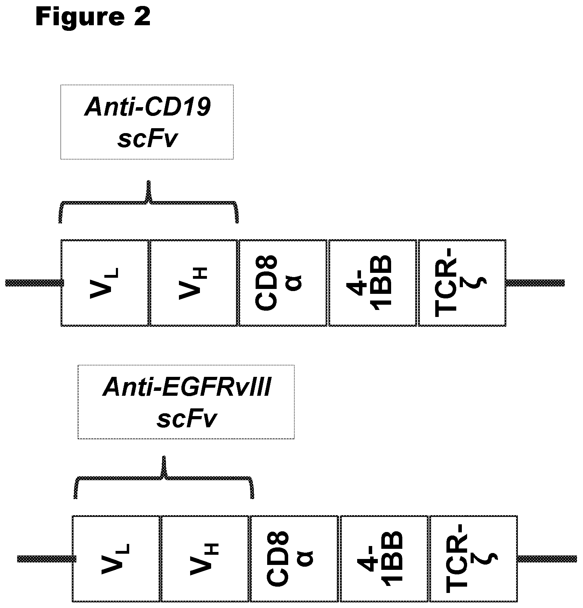

**Figure 2****,** shows a diagram of a set of nucleic acic constructs for expression of a B-cell antigen CAR and a solid tumor antigen CAR. A first construct encodes a CD19 CAR (B-cell antigen CAR) and a second construct encodes a EGFRvIII CAR (solid tumor CAR). The constructs may be provided in separate vectors, e.g., separate lentiviral vectors. Cells are transfected with the set of constructs to express both the B-cell antigen CAR and the solid tumor antigen CAR.

### FURTHER DETAILED DESCRIPTION

Disclosed herein are methods and compositions for treating a disease associated with expression of a tumor antigen, *e.g.,* a cancer, in a subject using an immune effector cell (e.g., T cell) engineered to expresss a first chimeric antigen receptor (CAR) and a second CAR, wherein the antigen binding domain of said first CAR binds to a B-Cell antigen and the antigen binding domain of said second CAR binds to a tumor antigen other than a B-Cell antigen, e.g., to enhance the efficacy (e.g., the persistence and/or proliferation of the CAR-expressing immune effector cell in a patient) of the CAR-expressing immune effector cell therapy. Without wishing to be bound by theory, treatment with an immune effector cell expressing a CAR targeting a B-Cell antigen and a CAR targeting a tumor antigen enhances the anti tumor efficacy of the CAR-expressing immune effector cell in a subject, *e.g*., by one or more of: increasing the proliferation of said CAR-expressing immune effector cells and/or increasing the in vivo persistence of said CAR expressing immune effector cells, *e.g.,* as compared to administering an immune effector cell expressing only the tumor-targeting CAR (e.g., not expressing the CAR tareting a B-Cell antigen).

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule as defined below. In some embodiments, the domains in the CAR polypeptide construct are in the same polypeptide chain, e.g., comprise a chimeric fusion protein. In some embodiments, the domains in the CAR polypeptide construct are not contiguous with each other, e.g., are in different polypeptide chains, e.g., as provided in an RCAR as described herein.

In one aspect, the stimulatory molecule is the zeta chain associated with the T cell receptor complex. In one aspect, the cytoplasmic signaling domain comprises a primary signaling domain (e.g., a primary signaling domain of CD3-zeta). In one aspect, the cytoplasmic signaling domain further comprises one or more functional signaling domains derived from at least one costimulatory molecule as defined below. In one aspect, the costimulatory molecule is chosen from 4-1BB (i.e., CD137), CD27, ICOS, and/or CD28. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a co-stimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more co-stimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more co-stimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen recognition domain (e.g., a scFv) during cellular processing and localization of the CAR to the cellular membrane.

A CAR that comprises an antigen binding domain (e.g., a scFv, or TCR) that targets, e.g., binds to, a specific antigen X, such as those described herein, is also referred to as XCAR, X-CAR or X-targeing CAR. For example, a CAR that comprises an antigen binding domain that targets CD19 is referred to as CD19CAR. A CAR that comprises an antigen binding domain (e.g., a scFv or TCR) that targets a specific tumor antigen (TA), such as those described herein, is also referred to as TA CAR. A CAR that comprises an antigen binding domain (e.g., a scFv or TCR) that targets a specific B cell antigen (BCA), such as those described herein (e.g. in connection with the first CAR molecule of the compositions of the disclosure), is also referred to as BCA CAR.

The term "signaling domain" refers to the functional portion of a protein which acts by transmitting information within the cell to regulate cellular activity via defined signaling pathways by generating second messengers or functioning as effectors by responding to such messengers. In some aspects, the signaling domain of the CAR described herein is derived from a stimulatory molecule or co-stimulatory molecule described herein, or is a synthesized or engineered signaling domain.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an intact antibody, or recombinant variants thereof, and refers to the antigen binding domain, e.g., an antigenic determining variable region of an intact antibody, that is sufficient to confer recognition and specific binding of the antibody fragment to a target, such as an antigen. Examples of antibody fragments include, but are not limited to, Fab, Fab', F(ab')₂, and Fv fragments, scFv antibody fragments, linear antibodies, single domain antibodies such as sdAb (either VL or VH), camelid VHH domains, and multi-specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The term "complementarity determining region" or "CDR," as used herein, refers to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a VH, e.g., a mammalian VH, e.g., a human VH; and amino acid residues 24-34 (LCDR1), 50-56 (LCDR2), and 89-97 (LCDR3) in a VL, e.g., a mammalian VL, e.g., a human VL.

The portion of the CAR disclosed herein comprising an antibody or antibody fragment thereof may exist in a variety of forms where the antigen binding domain is expressed as part of a contiguous polypeptide chain including, for example, scFv antibody fragments, linear antibodies, single domain antibodies such as sdAb (either VL or VH), camelid VHH domains ,a humanized antibody, a bispecific antibody, an antibody conjugate (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR disclosed herein comprises an antibody fragment. In a further aspect, the CAR comprises an antibody fragment that comprises a scFv.

As used herein, the term "binding domain" or "antibody molecule" (also referred to herein as "anti-target (e.g., CD19) binding domain") refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present disclosure includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated or can be derived from a biological sample, or might be macromolecule besides a polypeptide. Such a biological sample can include, but is not limited to a tissue sample, a tumor sample, a cell or a fluid with other biological components.

The term "anti-tumor effect" or "anti-tumor activity" refers to a biological effect which can be manifested by various means, including but not limited to, e.g., a decrease in tumor volume, a decrease in the number of tumor cells, a decrease in the number of metastases, an increase in life expectancy, decrease in tumor cell proliferation, decrease in tumor cell survival, or amelioration of various physiological symptoms associated with the cancerous condition. An "anti-tumor effect" can also be manifested by the ability of the peptides, polynucleotides, cells and antibodies of the disclosure in prevention of the occurrence of tumor in the first place.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically

The term "xenogeneic" refers to a graft derived from an animal of a different species.

The term "apheresis" as used herein refers to an extracorporeal process by which the blood of a donor of patient is removed from the donor or patient and passed through an apparatus that separates out selected particular constituent(s) and returns the remainder to the circulation of the donor or patient, *e.g.,* by retransfusion. Thus, in the context of "an apheresis sample" refers to a sample obtained using apheresis.

The term "cancer" refers to a disease characterized by the uncontrolled growth of aberrant cells. Cancer includes all types of cancerous growths or oncogenic processes, metastatic tissues or malignantly transformed cells, tissues or organs irrespective of the histopathologic type or stage of invasiveness. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor antigen" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR disclosed herein can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules, such as downregulation of TGF-β, and/or reorganization of cytoskeletal structures, and the like.

The term "stimulatory molecule," refers to a molecule expressed by an immune effector cell (e.g., a T cell, NK cell, B cell) that provides the cytoplasmic signaling sequence(s) that regulate activation of the immune effector cell in a stimulatory way for at least some aspect of the immune effector cell signaling pathway, e.g., the T cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing primary cytoplasmic signaling sequence that is of particular use in the invention includes, but is not limited to, those derived from CD3 zeta, common FcR gamma (FCER1G), Fc gamma RIIa, FcR beta (Fc epsilon R1b), CD3 gamma, CD3 delta , CD3 epsilon, CD5, CD22, CD79a, CD79b, CD278 (also known as "ICOS"), FcεRI, DAP10, DAP12, and CD66d. In a specific CAR disclosed herein,
the intracellular signaling domain in any one or more CARs disclosed herein comprises an intracellular signaling sequence, e.g., a primary signaling sequence of CD3-zeta. In a specific CAR disclosed herein, the primary signaling sequence of CD3-zeta is the sequence provided as SEQ ID NO: 18, or the equivalent residues from a non-human species, e.g., mouse, rodent, monkey, ape and the like. In a specific CAR disclosed herein, the primary signaling sequence of CD3-zeta is the sequence as provided in SEQ ID NO:20, or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain generates a signal that promotes an immune effector function of the CAR-expressingcell, e.g., a CART cell or CAR-expressing NK cell. Examples of immune effector function, e.g., in a CART cell or CAR-expressing NK cell, include cytolytic activity and helper activity, including the secretion of cytokines. While the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

An intracellular signaling domain can comprise a primary intracellular signaling domain. Exemplary primary intracellular signaling domains include those derived from the molecules responsible for primary stimulation, or antigen dependent simulation. An intracellular signaling domain can comprise a costimulatory intracellular domain. Exemplary costimulatory intracellular signaling domains include those derived from molecules responsible for costimulatory signals, or antigen independent stimulation. In an embodiment, the intracellular signaling domain is synthesized or engineered. For example, in the case of a CAR-expressing immune effector cell, e.g., CART cell or CAR-expressing NK cell, a primary intracellular signaling domain can comprise a cytoplasmic sequence of a T cell receptor, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, common FcR gamma (FCERl G), Fc gamma RIIa, FcR beta, CD3 gamma, CD3 delta, CD3 epsilon, CD5, CD22, CD79a, CD79b, CD278 ("ICOS"), FcεRI CD66d, DAP10 and DAP12.

The term "zeta" or alternatively "zeta chain", "CD3-zeta" or "TCR-zeta" is defined as the protein provided as GenBan Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO:18. In one aspect, the "zeta stimulatory domain" or a "CD3-zeta stimulatory domain" is the sequence provided as SEQ ID NO:20. Also encompassed herein are CD3 zeta domains comprising one or more mutations to the amino acid sequences described herein, e.g., SEQ ID NO: 20.

The term "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to an MHC class I molecule, a TNF receptor protein, an Immunoglobulin-like protein, a cytokine receptor, an integrin, a signaling lymphocytic activation molecule (SLAM protein), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B-4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain or costimulatory signaling domain can be the intracellular portion of a costimulatory molecule. The intracellular signaling domain can comprise the entire intracellular portion, or the entire native intracellular signaling domain, of the molecule from which it is derived, or a functional fragment thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the sequence provided as SEQ ID NO:14 or the equivalent residues from a non-human species, e.g., mouse, rodent, monkey, ape and the like.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloid-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and co-stimulation are examples of immune effector function or response.

The term "effector function" refers to a specialized function of a cell. Effector function of a T cell, for example, may be cytolytic activity or helper activity including the secretion of cytokines.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the noncoding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTOR^{®} gene delivery technology from Oxford BioMedica, the LENTIMAX^{™} vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or antibody fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present disclosure, the following abbreviations for the commonly occurring nucleic acid bases are used. "A" refers to adenosine, "C" refers to cytosine, "G" refers to guanosine, "T" refers to thymidine, and "U" refers to uridine.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The terms "B cell antigen" or "B-Cell antigen" are used interchangeably, and refer to a molecule (typically a protein, carbohydrate or lipid) that is preferentially and specifically expressed on the surface of a B cell which can be targeted with an agent which binds thereto. The B cell antigen of particular interest is preferentially expressed on B cells compared to other non-B cell tissues of a mammal. The B cell antigen may be expressed on one particular B cell population, e.g., B cell precursors or mature B cells, or on more than one particular B cell population, e.g., both precursor B cells and mature B cells. Exemplary B cell surface markers include: CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, 5, 7, IL-7/3R, IL7/4/3R, and IL4R. Particularly preferred B-Cell antigens include: CD19, CD20, CD22, FcRn5, FcRn2, BCMA, CS-1 and CD138. In embodiments, the B-Cell antigen is CD19. In embodiments, the B-Cell antigen is CD20. In embodiments, the B-Cell antigen is CD22. In embodiments, the B-Cell antigen is BCMA. In embodiments, the B-Cell antigen is FcRn5. In embodiments, the B-Cell antigen is FcRn2. In embodiments, the B-Cell antigen is CS-1. In embodiments, the B-Cell antigen is CD138.

The terms "cancer associated antigen" or "tumor antigen" interchangeably refers to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the CARs of the present disclosure includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library. Accordingly, the present disclosure provides CARs that comprise an antigen binding domain that binds to a MHC presented peptide of a molecule selected from the group of WT1, NY-ESO-1, LAGE-1a, MAGE-A1 and RAGE-1.

The terms "solid tumor antigen" or "solid tumor cell antigen" refer to a molecule (typically a protein, carbohydrate or lipid) that is preferentially and specifically expressed on the surface of a solid tumor cell which can be targeted with an agent which binds thereto. The solid tumor antigen of particular interest is preferentially expressed on a solid tumor cell compared to other non-tumor tissues of a mammal. The solid tumor antigen may be expressed on one particular solid tumor cell population, e.g., on mesothelioma tumor cells, or on more than one particular solid tumor cell population, e.g., both mesothelioma tumor cells and ovarian cancer cells. Exemplary solid tumor antigens include: EGFRvIII, mesothelin, GD2, Tn Ag, PSMA, TAG72, CD44v6, CEA, EPCAM, KIT, IL-13Ra2, leguman , GD3, CD171, IL-11Ra, PSCA, MAD-CT-1, MAD-CT-2, VEGFR2, LewisY, CD24, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, GPR20, Ly6k, OR51E2, TARP, GFRα4, and a peptide of any of these antigens presented on MHC. Particularly preferred solid tumor antigens include: CLDN6, mesothelin and EGFRvIII.

The terms "myeloid tumor antigen" or "myeloid tumor cell antigen" refer to a molecule (typically a protein, carbohydrate or lipid) that is preferentially and specifically expressed on the surface of a myeloid tumor cell which can be targeted with an agent which binds thereto. The myeloid tumor antigen of particular interest is preferentially expressed on a myeloid tumor cell compared to other non-tumor tissues of a mammal. The myeloid tumor antigen may be expressed on one particular myeloid tumor cell population, e.g., on acute myeloid leukemia (AML) tumor cells, or on more than one particular myeloid tumor cell population. Exemplary myeloid tumor antigens include: CD123, CD33 and CLL-1.

The term "antigen of a hematological tumor not of B-Cell lineage" refers to a molecule (typically a protein, carbohydrate or lipid) that is preferentially and specifically expressed on the surface of a tumor or cancer of hematopoietic or lymphoid tissue origin, other than of B-Cell origin. These include tumors of myeloid lineage origin, e.g., tumors derived from granulocyte, erythrocyte, thrombocyte, macrophage and/or mast cell origin, or any of their precursor cell populations, and tumors of lymphoid origin other than B-Cell origin, e.g., T cell, NK cell and/or plasma cell origin, or any of their precursor cell populations.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)ₙ, where n is a positive integer equal to or greater than 1. For example, n=1, n=2, n=3, n=4, n=5 and n=6, n=7, n=8, n=9 and n=10 (SEQ ID NO:28). In one embodiment, the flexible polypeptide linkers include, but are not limited to, (Gly₄ Ser)₄ (SEQ ID NO:29) or (Gly₄ Ser)₃ (SEQ ID NO:30). In another embodiment, the linkers include multiple repeats of (Gly₂Ser), (GlySer) or (Gly₃Ser) (SEQ ID NO:31). Also included within the scope of the disclosure are linkers described in WO2012/138475).

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In the preferred embodiment of a construct for transient expression, the polyA is between 50 and 5000 (SEQ ID NO: 34), preferably greater than 64, more preferably greater than 100, most preferably greater than 300 or 400. Poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a CAR disclosed herein ). In specific embodiments, the terms "treat," "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "therapeutic" as used herein means a treatment. A therapeutic effect is obtained by reduction, suppression, remission, or eradication of a disease state.

The term "tolerance" or "immune tolerance" as used herein refers to a state in which a subject has a reduced or absent immune response to a specific antigen or group of antigens to which the subject is normally responsive to. Tolerance is achieved under conditions that suppress the immune reaction and is not just the absence of an immune response. In an embodiment, tolerance in a subject can be characterized by one or more of the following: a decreased level of a specific immunological response (e.g., mediated by antigen-specific effector T lymphocytes, B lymphocytes, or antibody); a delay in the onset or progression of a specific immunological response; or a reduced risk of the onset or progression of a specific immunological response, as compared to untreated subjects.

The term "prophylaxis" as used herein means the prevention of or protective treatment for a disease or disease state.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a cognate binding partner (e.g., a stimulatory and/or costimulatory molecule present on a T cell) protein present in a sample, but which antibody or ligand, does not substantially recognize or bind other molecules in the sample.

"Regulatable chimeric antigen receptor (RCAR),"as used herein, refers to a set of polypeptides, typically two in the simplest embodiments, which when in an immune effector cell, provides the cell with specificity for a target cell, typically a cancer cell, and with regulatable intracellular signal generation. In some embodiments, an RCAR comprises at least an extracellular antigen binding domain, a transmembrane and a cytoplasmic signaling domain (also referred to herein as "an intracellular signaling domain") comprising a functional signaling domain derived from a stimulatory molecule and/or costimulatory molecule as defined herein in the context of a CAR molecule. In some embodiments, the set of polypeptides in the RCAR are not contiguous with each other, e.g., are in different polypeptide chains. In some embodiments, the RCAR includes a dimerization switch that, upon the presence of a dimerization molecule, can couple the polypeptides to one another, e.g., can couple an antigen binding domain to an intracellular signaling domain. In some embodiments, the RCAR is expressed in a cell (e.g., an immune effector cell) as described herein, e.g., an RCAR-expressing cell (also referred to herein as "RCARX cell"). In an embodiment the RCARX cell is a T cell, and is referred to as a RCART cell. In an embodiment the RCARX cell is an NK cell, and is referred to as a RCARN cell. The RCAR can provide the RCAR-expressing cell with specificity for a target cell, typically a cancer cell, and with regulatable intracellular signal generation or proliferation, which can optimize an immune effector property of the RCAR-expressing cell. In embodiments, an RCAR cell relies at least in part, on an antigen binding domain to provide specificity to a target cell that comprises the antigen bound by the antigen binding domain.

"Membrane anchor" or "membrane tethering domain", as that term is used herein, refers to a polypeptide or moiety, e.g., a myristoyl group, sufficient to anchor an extracellular or intracellular domain to the plasma membrane.

"Switch domain," as that term is used herein, e.g., when referring to an RCAR, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequences, and are referred to collectively as a heterodimerization switch. In embodiments, the switch is intracellular. In embodiments, the switch is extracellular. In embodiments, the switch domain is a polypeptide-based entity, e.g., FKBP or FRB-based, and the dimerization molecule is small molecule, e.g., a rapalogue. In embodiments, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In embodiments, the switch domain is a polypeptide-based entity, e.g., myc receptor, and the dimerization molecule is an antibody or fragments thereof, e.g., myc antibody.

"Dimerization molecule," as that term is used herein, e.g., when referring to an RCAR, refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001.

The term "bioequivalent" refers to an amount of an agent other than the reference compound (e.g., RAD001), required to produce an effect equivalent to the effect produced by the reference dose or reference amount of the reference compound ( e.g., RAD001). In an embodiment the effect is the level of mTOR inhibition, e.g., as measured by P70 S6 kinase inhibition, e.g., as evaluated in an in vivo or in vitro assay, e.g., as measured by an assay described herein, e.g., the Boulay assay, or measurement of phosphorylated S6 levels by western blot. In an embodiment, the effect is alteration of the ratio of PD-1 positive/PD-1 negative T cells, as measured by cell sorting. In an embodiment a bioequivalent amount or dose of an mTOR inhibitor is the amount or dose that achieves the same level of P70 S6 kinase inhibition as does the reference dose or reference amount of a reference compound. In an embodiment, a bioequivalent amount or dose of an mTOR inhibitor is the amount or dose that achieves the same level of alteration in the ratio of PD-1 positive/PD-1 negative T cells as does the reference dose or reference amount of a reference compound.

The term "low, immune enhancing, dose" when used in conjuction with an mTOR inhibitor, e.g., an allosteric mTOR inhibitor, e.g., RAD001 or rapamycin, or a catalytic mTOR inhibitor, refers to a dose of mTOR inhibitor that partially, but not fully, inhibits mTOR activity, e.g., as measured by the inhibition of P70 S6 kinase activity. Methods for evaluating mTOR activity, e.g., by inhibition of P70 S6 kinase, are discussed herein. The dose is insufficient to result in complete immune suppression but is sufficient to enhance the immune response. In an embodiment, the low, immune enhancing, dose of mTOR inhibitor results in a decrease in the number of PD-1 positive T cells and/or an increase in the number of PD-1 negative T cells, or an increase in the ratio of PD-1 negative T cells/PD-1 positive T cells. In an embodiment, the low, immune enhancing, dose of mTOR inhibitor results in an increase in the number of naive T cells. In an embodiment, the low, immune enhancing, dose of mTOR inhibitor results in one or more of the following:
an increase in the expression of one or more of the following markers: CD62L^{high}, CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
wherein any of the changes described above occurs, e.g., at least transiently, e.g., as compared to a non-treated subject.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

"Relapsed" or "relapse" as used herein refers to the return or reappearance of a disease (e.g., cancer) or the signs and symptoms of a disease such as cancer after a period of improvement or responsiveness, e.g., after prior treatment of a therapy, e.g., cancer therapy. The initial period of responsiveness may involve the level of cancer cells falling below a certain threshold, e.g., below 20%, 1%, 10%, 5%, 4%, 3%, 2%, or 1%. The reappearance may involve the level of cancer cells rising above a certain threshold, e.g., above 20%, 1%, 10%, 5%, 4%, 3%, 2%, or 1%. For example, e.g., in the context of B-ALL, the reappearance may involve, e.g., a reappearance of blasts in the blood, bone marrow (> 5%), or any extramedullary site, after a complete response. A complete response, in this context, may involve < 5% BM blast. More generally, in an embodiment, a response (e.g., complete response or partial response) can involve the absence of detectable MRD (minimal residual disease). In an embodiment, the initial period of responsiveness lasts at least 1, 2, 3, 4, 5, or 6 days; at least 1, 2, 3, or 4 weeks; at least 1, 2, 3, 4, 6, 8, 10, or 12 months; or at least 1, 2, 3, 4, or 5 years.

Ranges: throughout this disclosure, various aspects can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

### Description

Provided herein are compositions and methods of use for the treatment of a disease, such as cancer, comprising the use of a cell, e.g., an immune effector cell (e.g., an NK cell or T cell) engineered to express a first CAR molecule that targets a B-Cell antigen (e.g., a BCA CAR) and a second CAR molecule that targets a tumor antigen (e.g., a TA CAR). The invention provides a cell as defined in the claims. The invention also provides a cell as defined in the claims for use in a method of treating cancer. The invention also provides a cell as defined in the claims for use in for use in a method of treating a disease associated with expression of a solid tumor antigen, wherein the disease is a tumor characterized as glioblastoma, ovarian cancer, lung cancer, prostate cancer, colorectal cancer, pancreatic cancer, breast carcinoma, adenocarcinoma or mesothelioma.

In an embodiment, the cancer is a solid tumor, myeloid tumor or hematological tumor not of B-Cell lineage. In an embodiment, the tumor is a solid tumor. In an embodiment, the tumor is a myeloid tumor. In an embodiment, the tumor is a hematological tumor not of B-Cell lineage.

In instances, the compositions and methods described herein result in a tumor-targeting CAR-expressing immune effector cell with enchanced proliferation and/or with increased or prolonged in vivo persistence, relative to the same cell which does not express the BCA CAR.

Without wishing to be bound by theory, treatment with a cell, e.g., an immune effector cell expressing a CAR targeting a B-Cell antigen (BCA CAR) on an immune effector cell expressing a CAR targeting a tumor antigen (TA CAR) enhances the anti tumor efficacy of the TA CAR-expressing immune effector cell in a subject, *e.g.,* by one or more of: increasing the proliferation of said CAR-expressing immune effector cells and/or increasing the in vivo persistence of said CAR expressing immune effector cells, *e.g.,* as compared to administering an immune effector cell expressing only the TA CAR (e.g., not expressing the BCA CAR). Without being bound by theory, CAR cell therapies targeting, e.g., solid tumors, may suffer from lack of persistence in vivo as cells expressing the tumor antigen targeted by the CAR become inaccessible, or drop in number due to the effect of the CAR expressing cell. In contrast, CAR-T cell therapy targeting B-Cell antigens such as, for example, CD19, exhibit rapid and significant expansion in vivo, followed by long-term persistence. Without being bound by theory, these beneficial effects observed for CAR-T cell therapy targeting B-Cell antigens may be mediated by the widespread (i.e., circulating) availability of B-Cells which allows cells expressing a B-Cell antigen-targeting CAR to be exposed to, and be stimulated by, this readily available cell population and may further be enhanced by the natural immune-stimulatory effects of B-cell/T-cell interaction. Thus, without being bound by theory, inclusion of a CAR targeting a B-cell antigen is beneficial in that it mediates rapid expansion and persistence of the CART cell expressing said CAR, and that when the CAR cell further expresses a tumor antigen, such cell benefits from those effects relative to a CAR T cell which only expresses the tumor antigen-targeting CAReven when populations of cells expressing the tumor antigen are low, inaccessible or non-existent, thereby allowing the CART cells be primed for mediating improved cytotoxicity against the tumor antigen-expressing cell, e.g., cancer, and to persist through periods of remission and can then become effective in periods of relapse, without having to readminister cells. Thus, administering cells, e.g., immune effector cells, expressing both a BCA CAR and a TA CAR can enhance the efficacy of a TA CAR-expressing cell for treating a disease, e.g., cancer.

The cells of the present disclosure are genetically engineered to express a first CAR molecule, wherein the first CAR molecule comprises an antigen binding domain specific for a B-Cell antigen, and genetically engineered to express a second CAR molecule, wherein the second CAR molecule comprises an antigen binding domain specific for a tumor antigen .

In the cells of the invention, the antigen binding domain of the second CAR binds to a tumor antigen other than a B-Cell antigen, wherein the tumor antigen other than a B-Cell antigen is a solid tumor antigen.

In embodiments, the B-cell antigen is not expressed on the cell which expresses the tumor antigen.

The antigen binding domain binds to a B cell antigen described herein or a tumor antigen described herein. A CAR molecule that binds to a B cell antigen is also referred to herein as "BCA CAR". A CAR molecule that binds to a tumor antigen other than a B-Cell antigen, e.g., a solid tumor antigen, a myeloid tumor antigen, or an antigen of a hematological tumor not of B-Cell origin, is also referred to herein as "TA CAR". The CAR may further comprise a transmembrane domain and an intracellular signaling domain comprising a costimulatory domain and/or a primary signaling domain, e.g., as described herein. In an embodiment, the intracellular signaling domain of the BCA CAR and/or TA CAR includes, but is not limited to, one or more of a CD3-zeta chain, 4-1BB, CD27, ICOS, and CD28 signaling modules and combinations thereof.

In one aspect, the disclosure provides an immune effector cell (e.g., T cell, NK cell) engineered to express a TA CAR and engineered to express a BCA CAR, wherein the engineered immune effector cell exhibits an antitumor property, e.g., reduces tumor volume, stimulates tumor regression, decreases tumor burden, or increases overall survival; while at the same time having increased persistence in vivo, or increased proliferation, relative to the same cell which does not express the BCA CAR.

Also described herein are methods of using said cells engineered to express a BCA CAR and a TA CAR.

Also described herein are methods of making or selecting a cell engineered to express a BCA CAR and a TA CAR, methods for administering the cells for treating a disease associated with a tumor antigen, and additional combination therapies for use with the cells of the invention.

### Chimeric Antigen Receptor (CAR)

The present disclosure encompasses immune effector cells (e.g., T cells or NK cells) comprising one or more recombinant nucleic acid constructs comprising sequences encoding a CAR molecule that binds to a tumor antigen (e.g., a TA CAR) and a CAR molecule that binds to a B cell antigen (e.g., a BCA CAR), wherein the TA CAR comprises an antigen binding domain (e.g., antibody or antibody fragment, TCR or TCR fragment) that binds specifically to a tumor antigen described herein and the BCA CAR comprises an antigen binding domain (e.g., antibody or antibody fragment, TCR or TCR fragment) that binds specifically to a B cell antigen described herein, e.g., wherein the sequence of the antigen binding domain is contiguous with and in the same reading frame as a nucleic acid sequence encoding an intracellular signaling domain. The intracellular signaling domain can comprise a costimulatory signaling domain and/or a primary signaling domain, e.g., a zeta chain. The costimulatory signaling domain refers to a portion of the CAR comprising at least a portion of the intracellular domain of a costimulatory molecule. The cells of the invention are defined in the appended claims.

In one aspect, the CARs disclosed herein comprise at least one intracellular signaling domain selected from the group of a CD137 (4-1 BB) signaling domain, a CD28 signaling domain, a CD27 signaling domain, an ICOS signaling domain, a CD3zeta signal domain, and any combination thereof. In one aspect, the CARs disclosed herein comprise at least one intracellular signaling domain is from one or more costimulatory molecule(s) selected from CD137(4-1BB),CD28, CD27, or ICOS.

Sequences of non-limiting examples of various components that can be part of a CAR molecule, e.g., a TA CAR or a BCA CAR described herein, are listed in Table 1, where "aa" stands for amino acids, and "na" stands for nucleic acids that encode the corresponding peptide.

**Table 1. Sequences of various components of CAR (aa amino acids, na nucleic acids that encodes the corresponding protein)**

| SEQ ID NO | Description | Sequence |
|---|---|---|
| 1 | EF-1 promoter (na) | CGTGAGGCTCCGGTGCCCGTCAGTGGGCAGAGCGCACATCG CCCACAGTCCCCGAGAAGTTGGGGGGAGGGGTCGGCAATT GAACCGGTGCCTAGAGAAGGTGGCGCGGGGTAAACTGGGA AAGTGATGTCGTGTACTGGCTCCGCCTTTTTCCCGAGGGTGG GGGAGAACCGTATATAAGTGCAGTAGTCGCCGTGAACGTTC TTTTTCGCAACGGGTTTGCCGCCAGAACACAGGTAAGTGCC GTGTGTGGTTCCCGCGGGCCTGGCCTCTTTACGGGTTATGGC CCTTGCGTGCCTTGAATTACTTCCACCTGGCTGCAGTACGTG ATTCTTGATCCCGAGCTTCGGGTTGGAAGTGGGTGGGAGAG TTCGAGGCCTTGCGCTTAAGGAGCCCCTTCGCCTCGTGCTTG AGTTGAGGCCTGGCCTGGGCGCTGGGGCCGCCGCGTGCGAA TCTGGTGGCACCTTCGCGCCTGTCTCGCTGCTTTCGATAAGT CTCTAGCCATTTAAAATTTTTGATGACCTGCTGCGACGCTTT TTTTCTGGCAAGATAGTCTTGTAAATGCGGGCCAAGATCTG CACACTGGTATTTCGGTTTTTGGGGCCGCGGGCGGCGACGG GGCCCGTGCGTCCCAGCGCACATGTTCGGCGAGGCGGGGCC TGCGAGCGCGGCCACCGAGAATCGGACGGGGGTAGTCTCA |
| | | AGCTGGCCGGCCTGCTCTGGTGCCTGGCCTCGCGCCGCCGT GTATCGCCCCGCCCTGGGCGGCAAGGCTGGCCCGGTCGGCA CCAGTTGCGTGAGCGGAAAGATGGCCGCTTCCCGGCCCTGC TGCAGGGAGCTCAAAATGGAGGACGCGGCGCTCGGGAGAG CGGGCGGGTGAGTCACCCACACAAAGGAAAAGGGCCTTTC CGTCCTCAGCCGTCGCTTCATGTGACTCCACGGAGTACCGG GCGCCGTCCAGGCACCTCGATTAGTTCTCGAGCTTTTGGAGT ACGTCGTCTTTAGGTTGGGGGGAGGGGTTTTATGCGATGGA GTTTCCCCACACTGAGTGGGTGGAGACTGAAGTTAGGCCAG CTTGGCACTTGATGTAATTCTCCTTGGAATTTGCCCTTTTTG AGTTTGGATCTTGGTTCATTCTCAAGCCTCAGACAGTGGTTC AAAGTTTTTTTCTTCCATTTCAGGTGTCGTGA |
| 2 | Leader (aa) | MALPVTALLLPLALLLHAARP |
| 3 | Leader (na) | ATGGCCCTGCCTGTGACAGCCCTGCTGCTGCCTCTGGCTCTG CTGCTGCATGCCGCTAGACCC |
| 307 | Leader (na-v2) | ATGGCCCTCCCTGTCACCGCCCTGCTGCTTCCGCTGGCTCTT CTGCTCCACGCCGCTCGGCCC |
| 4 | CD 8 hinge (aa) | TTTPAPRPPTPAPTIASQPLSLRPEACRPAAGGAVHTRGLDFAC D |
| 5 | CD8 hinge (na) | ACCACGACGCCAGCGCCGCGACCACCAACACCGGCGCCCA CCATCGCGTCGCAGCCCCTGTCCCTGCGCCCAGAGGCGTGC CGGCCAGCGGCGGGGGCGCAGTGCACACGAGGGGGCTGG ACTTCGCCTGTGAT |
| 6 | Ig4 hinge (aa) | ESKYGPPCPPCPAPEFLGGPSVFLFPPKPKDTLMISRTPEVTCVV VDVSQEDPEVQFNWYVDGVEVHNAKTKPREEQFNSTYRVVS VLTVLHQDWLNGKEYKCKVSNKGLPSSIEKTISKAKGQPREPQ VYTLPPSQEEMTKNQVSLTCLVKGFYPSDIAVEWESNGQPENN YKTTPPVLDSDGSFFLYSRLTVDKSRWQEGNVFSCSVMHEAL HNHYTQKSLSLSLGKM |
| 7 | Ig4 hinge (na) | GAGAGCAAGTACGGCCCTCCCTGCCCCCCTTGCCCTGCCCC CGAGTTCCTGGGCGGACCCAGCGTGTTCCTGTTCCCCCCA AGCCCAAGGACACCCTGATGATCAGCCGGACCCCCGAGGTG ACCTGTGTGGTGGTGGACGTGTCCCAGGAGGACCCCGAGGT CCAGTTCAACTGGTACGTGGACGGCGTGGAGGTGCACAACG CCAAGACCAAGCCCCGGGAGGAGCAGTTCAATAGCACCTA CCGGGTGGTGTCCGTGCTGACCGTGCTGCACCAGGACTGGC TGAACGGCAAGGAATACAAGTGTAAGGTGTCCAACAAGGG CCTGCCCAGCAGCATCGAGAAAACCATCAGCAAGGCCAAG GGCCAGCCTCGGGAGCCCCAGGTGTACACCCTGCCCCCTAG CCAAGAGGAGATGACCAAGAACCAGGTGTCCCTGACCTGCC TGGTGAAGGGCTTCTACCCCAGCGACATCGCCGTGGAGTGG GAGAGCAACGGCCAGCCCGAGAACAACTACAAGACCACCC CCCCTGTGCTGGACAGCGACGGCAGCTTCTTCCTGTACAGC CGGCTGACCGTGGACAAGAGCCGGTGGCAGGAGGGCAACG TCTTTAGCTGCTCCGTGATGCACGAGGCCCTGCACAACCAC TACACCCAGAAGAGCCTGAGCCTGTCCCTGGGCAAGATG |
| 8 | IgD hinge (aa) | RWPESPKAQASSVPTAQPQAEGSLAKATTAPATTRNTGRGGE |
| | | EKKKEKEKEEQEERETKTPECPSHTQPLGVYLLTPAVQDLWLR DKATFTCFVVGSDLKDAHLTWEVAGKVPTGGVEEGLLERHSN GSQSQHSRLTLPRSLWNAGTSVTCTLNHPSLPPQRLMALREPA AQAPVKLSLNLLASSDPPEAASWLLCEVSGFSPPNILLMWLED QREVNTSGFAPARPPPQPGSTTFWAWSVLRVPAPPSPQPATYT CVVSHEDSRTLLNASRSLEVSYVTDH |
| 9 | IgD hinge (na) | AGGTGGCCCGAAAGTCCCAAGGCCCAGGCATCTAGTGTTCC TACTGCACAGCCCCAGGCAGAAGGCAGCCTAGCCAAAGCT ACTACTGCACCTGCCACTACGCGCAATACTGGCCGTGGCGG GGAGGAGAAGAAAAAGGAGAAAGAGAAAGAAGAACAGGA AGAGAGGGAGACCAAGACCCCTGAATGTCCATCCCATACCC AGCCGCTGGGCGTCTATCTCTTGACTCCCGCAGTACAGGAC TTGTGGCTTAGAGATAAGGCCACCTTTACATGTTTCGTCGTG GGCTCTGACCTGAAGGATGCCCATTTGACTTGGGAGGTTGC CGGAAAGGTACCCACAGGGGGGGTTGAGGAAGGGTTGCTG GAGCGCCATTCCAATGGCTCTCAGAGCCAGCACTCAAGACT CACCCTTCCGAGATCCCTGTGGAACGCCGGGACCTCTGTCA CATGTACTCTAAATCATCCTAGCCTGCCCCCACAGCGTCTGA TGGCCCTTAGAGAGCCAGCCGCCCAGGCACCAGTTAAGCTT AGCCTGAATCTGCTCGCCAGTAGTGATCCCCCAGAGGCCGC CAGCTGGCTCTTATGCGAAGTGTCCGGCTTTAGCCCGCCCA ACATCTTGCTCATGTGGCTGGAGGACCAGCGAGAAGTGAAC ACCAGCGGCTTCGCTCCAGCCCGGCCCCCACCCCAGCCGGG TTCTACCACATTCTGGGCCTGGAGTGTCTTAAGGGTCCCAGC ACCACCTAGCCCCCAGCCAGCCACATACACCTGTGTTGTGT CCCATGAAGATAGCAGGACCCTGCTAAATGCTTCTAGGAGT CTGGAGGTTTCCTACGTGACTGACCATT |
| 10 | GS hinge/linker (aa) | GGGGSGGGGS |
| 11 | GS hinge/linker (na) | GGTGGCGGAGGTTCTGGAGGTGGAGGTTCC |
| 12 | CD8TM (aa) | IYIWAPLAGTCGVLLLSLVITLYC |
| 13 | CD8 TM (na) | ATCTACATCTGGGCGCCCTTGGCCGGGACTTGTGGGGTCCTT CTCCTGTCACTGGTTATCACCCTTTACTGC |
| 308 | CD8 TM (na-v2) | ATCTACATTTGGGCCCCTCTGGCTGGTACTTGCGGGGTCCTG CTGCTTTCACTCGTGATCACTCTTTACTGT |
| 14 | 4-1BB intracellular domain (aa) | KRGRKKLLYIFKQPFMRPVQTTQEEDGCSCRFPEEEEGGCEL |
| 15 | 4-1BB intracellular domain (na) | AAACGGGGCAGAAAGAAACTCCTGTATATATTCAAACAACC ATTTATGAGACCAGTACAAACTACTCAAGAGGAAGATGGCT GTAGCTGCCGATTTCCAGAAGAAGAAGAAGGAGGATGTGA ACTG |
| 309 | 4-1BB intracellular domain (na-v2) | AAGCGCGGTCGGAAGAAGCTGCTGTACATCTTTAAGCAACC CTTCATGAGGCCTGTGCAGACTACTCAAGAGGAGGACGGCT GTTCATGCCGGTTCCCAGAGGAGGAGGAAGGCGGCTGCGA ACTG |
| 16 | CD27 intracellular domain (aa) | QRRKYRSNKGESPVEPAEPCRYSCPREEEGSTIPIQEDYRKPEP ACSP |
| 17 | CD27 intracellular domain (na) | AGGAGTAAGAGGAGCAGGCTCCTGCACAGTGACTACATGA ACATGACTCCCCGCCGCCCCGGGCCCACCCGCAAGCATTAC CAGCCCTATGCCCCACCACGCGACTTCGCAGCCTATCGCTC C |
| 18 | CD3-zeta (aa) | RVKFSRSADAPAYKQGQNQLYNELNLGRREEYDVLDKRRGR DPEMGGKPRRKNPQEGLYNELQKDKMAEAYSEIGMKGERRR GKGHDGLYQGLSTATKDTYDALHMQALPPR |
| 19 | CD3-zeta (na) | AGAGTGAAGTTCAGCAGGAGCGCAGACGCCCCCGCGTACA AGCAGGGCCAGAACCAGCTCTATAACGAGCTCAATCTAGGA CGAAGAGAGGAGTACGATGTTTTGGACAAGAGACGTGGCC GGGACCCTGAGATGGGGGGAAAGCCGAGAAGGAAGAACCC TCAGGAAGGCCTGTACAATGAACTGCAGAAAGATAAGATG GCGGAGGCCTACAGTGAGATTGGGATGAAAGGCGAGCGCC GGAGGGGCAAGGGGCACGATGGCCTTTACCAGGGTCTCAGT ACAGCCACCAAGGACACCTACGACGCCCTTCACATGCAGGC CCTGCCCCCTCGC |
| 310 | CD3-zeta (na-v2) | CGCGTGAAATTCAGCCGCAGCGCAGATGCTCCAGCCTACAA GCAGGGGCAGAACCAGCTCTACAACGAACTCAATCTTGGTC GGAGAGAGGAGTACGACGTGCTGGACAAGCGGAGAGGACG GGACCCAGAAATGGGCGGGAAGCCGCGCAGAAAGAATCCC CAAGAGGGCCTGTACAACGAGCTCCAAAAGGATAAGATGG CAGAAGCCTATAGCGAGATTGGTATGAAAGGGGAACGCAG AAGAGGCAAAGGCCACGACGGACTGTACCAGGGACTCAGC ACCGCCACCAAGGACACCTATGACGCTCTTCACATGCAGGC CCTGCCGCCTCGG |
| 20 | CD3-zeta (aa) | RVKFSRSADAPAYQQGQNQLYNELNLGRREEYDVLDKRRGR DPEMGGKPRRKNPQEGLYNELQKDKMAEAYSEIGMKGERRR GKGHDGLYQGLSTATKDTYDALHMQALPPR |
| 21 | CD3-zeta (na) | AGAGTGAAGTTCAGCAGGAGCGCAGACGCCCCCGCGTACC AGCAGGGCCAG AACCAGCTCTATAACGAGCTCAATCTAGGACGAAGAGAGG AGTACGATGTTT TGGACAAGAGACGTGGCCGGGACCCTGAGATGGGGGGAAA GCCGAGAAGGA AGAACCCTCAGGAAGGCCTGTACAATGAACTGCAGAAAGA TAAGATGGCGG AGGCCTACAGTGAGATTGGGATGAAAGGCGAGCGCCGGAG GGGCAAGGGGC ACGATGGCCTTTACCAGGGTCTCAGTACAGCCACCAAGGAC |
| | | ACCTACGACGC CCTTCACATGCAGGCCCTGCCCCCTCGC |
| 22 | linker | GGGGS |
| 23 | linker | GGTGGCGGAGGTTCTGGAGGTGGAGGTTCC |
| 24 | PD-1 extracellular domain (aa) | |
| 25 | PD-1 extracellular domain (na) | Cccggatggtttctggactctccggatcgcccgtggaatcccccaaccttctcaccggcactcttgg ttgtgactgagggcgataatgcgaccttcacgtgctcgttctccaacacctccgaatcattcgtgctg aactggtaccgcatgagcccgtcaaaccagaccgacaagctcgccgcgtttccggaagatcggtc gcaaccgggacaggattgtcggttccgcgtgactcaactgccgaatggcagagacttccacatga gcgtggtccgcgctaggcgaaacgactccgggacctacctgtgcggagccatctcgctggcgcct aaggcccaaatcaaagagagcttgagggccgaactgagagtgaccgagcgcagagctgaggtg ccaactgcacatccatccccatcgcctcggcctgcggggcagtttcagaccctggtc |
| 26 | PD-1 CAR (aa) with signal | Malpvtalllplalllhaarppgwfldspdrpwnpptfspallvvtegdnatftcsfsntsesfvln wyrmspsnqtdklaafpedrsqpgqdcrfrvtqlpngrdfhmsvvrarrndsgtylcgaislap kaqikeslraelrvterraevptahpspsprpagqfqtlvtttpaprpptpaptiasqplslrpeacrp aaggavhtrgldfacdiyiwaplagtcgvlllslvitlyckrgrkkllyifkqpfmrpvqttqeedg cscrfpeeeeggcelrvkfsrsadapaykqgqnqlynelnlgrreeydvldkrrgrdpemggkp rrknpqeglynelqkdkmaeayseigmkgerrrgkghdglyqglstatkdtydalhmqalppr |
| 27 | PD-1 CAR (na) | Atggccctccctgtcactgccctgcttctcccccctcgcactcctgctccacgccgctagaccacccg gatggtttctggactctccggatcgcccgtggaatcccccaaccttctcaccggcactcttggttgtg actgagggcgataatgcgaccttcacgtgctcgttctccaacacctccgaatcattcgtgctgaactg gtaccgcatgagcccgtcaaaccagaccgacaagctcgccgcgtttccggaagatcggtcgcaac cgggacaggattgtcggttccgcgtgactcaactgccgaatggcagagacttccacatgagcgtg gtccgcgctaggcgaaacgactccgggacctacctgtgcggagccatctcgctggcgcctaagg cccaaatcaaagagagcttgagggccgaactgagagtgaccgagcgcagagctgaggtgccaa ctgcacatccatccccatcgcctcggcctgcggggcagtttcagaccctggtcacgaccactccgg cgccgcgcccaccgactccggccccaactatcgcgagccagcccctgtcgctgaggccggaag catgccgccctgccgccggaggtgctgtgcatacccggggattggacttcgcatgcgacatctaca tttgggctcctctcgccggaacttgtggcgtgctccttctgtccctggtcatcaccctgtactgcaagc ggggtcggaaaaagcttctgtacattttcaagcagcccttcatgaggcccgtgcaaaccacccagg aggaggacggttgctcctgccggttccccgaagaggaagaaggaggttgcgagctgcgcgtgaa gttctcccggagcgccgacgcccccgcctataagcagggccagaaccagctgtacaacgaactg aacctgggacggcgggaagagtacgatgtgctggacaagcggcgcggccgggaccccgaaat gggcgggaagcctagaagaaagaaccctcaggaaggcctgtataacgagctgcagaaggacaa gatggccgaggcctactccgaaattgggatgaagggagagcggcggaggggaaaggggcacg acggcctgtaccaaggactgtccaccgccaccaaggacacatacgatgccctgcacatgcaggc ccttccccctcgc |
| 28 | linker | (Gly-Gly-Gly-Ser)ₙ, where n = 1-10 |
| 29 | linker | (Gly₄ Ser)₄ |
| 30 | linker | (Gly₄ Ser)3 |
| 31 | linker | (Gly₃Ser) |
| 32 | polyA (2000 A's) | [a]₂₀₀₀ |
| 33 | polyA (150 A's) | [a]₁₅₀ |
| 34 | polyA (5000 A's) | [a]₅₀₀₀ |
| 35 | polyA (100 T's) | [t]₁₀₀ |
| 36 | polyA (500 T's) | [t]₅₀₀ |
| 37 | polyA (64 A's) | [a]₆₄ |
| 38 | polyA (400 A's) | [a]₄₀₀ |
| 39 | PD1 CAR (aa) | Pgwfldspdrpwnpptfspallvvtegdnatftcsfsntsesfvlnwyrmspsnqtdklaafpe drsqpgqdcrfrvtqlpngrdfhmsvvrarrndsgtylcgaislapkaqikeslraelrvterraev ptahpspsprpagqfqtlvtttpaprpptpaptiasqplslrpeacrpaaggavhtrgldfacdiyi waplagtcgvllllslvitlyckrgrkkllyifkqpfmrpvqttqeedgcscrfpeeeeggcelrvkf srsadapaykqgqnqlynelnlgrreeydvldkrrgrdpemggkprrknpqeglynelqkdk maeayseigmkgerrrgkghdglyqglstatkdtydalhmqalppr |
| 40 | ICOS intracellular domain (aa) | T K K K Y S S S V H D P N G E Y M F M R A V N T A K K S R L T D V T L |
| 41 | ICOS intracellular domain (na) | ACAAAAAAGAAGTATTCATCCAGTGTGCACGACCCTAACGGTGAATACATGTTCAT GAGAGCAGTGAACACAGCCAAAAAATCCAGACTCACAGATGTGACCCTA |
| 42 | ICOS TM domain (aa) | T T T P A P P P P T P A P T I A S Q P L S L R P E A C R P A A G G A V H T R G L D F A C D F W L P I G C A A F V V V C I L G C I L I C W L |
| 43 | ICOS TM domain (na) | ACCACGACGCCAGCGCCGCGACCACCAACACCGGCGCCCACCATCGCGTCGCAGCC CCTGTCCCTGCGCCCAGAGGCGTGCCGGCCAGCGGCGGGGGGCGCAGTGCACACGA GGGGGCTGGACTTCGCCTGTGATTTCTGGTTACCCATAGGATGTGCAGCCTTTGTT GTAGTCTGCATTTTGGGATGCATACTTATTTGTTGGCTT |
| 44 | CD28 intracellular domain (aa) | RSKRSRLLHSDYMNMTPRRPGPTRKHYQPYAPPRDFAAYRS |
| 45 | CD28 intracellular domain (na) | AGGAGTAAGAGGAGCAGGCTCCTGCACAGTGACTACATGAACATGACTCCCCGCCG CCCCGGGCCCACCCGCAAGCATTACCAGCCCTATGCCCCACCACGCGACTTCGCAG CCTATCGCTCC |

In specific aspects, a CAR construct as disclosed herein (a CAR that binds to a B cell antigen or a CAR that binds to a tumor antigen) comprises a scFv domain, wherein the scFv may be preceded by an optional leader sequence such as provided in SEQ ID NO: 2, and followed by an optional hinge sequence such as provided in SEQ ID NO:4 or SEQ ID NO:6 or SEQ ID NO:8 or SEQ ID NO:10, a transmembrane region such as provided in SEQ ID NO:12, an intracellular signalling domain that includes SEQ ID NO:14, SEQ ID NO:16, SEQ ID NO: 42, or SEQ ID NO:44 and a CD3 zeta sequence that includes SEQ ID NO: 18 or SEQ ID NO:20, e.g., wherein the domains are contiguous with and in the same reading frame to form a single fusion protein.

In one aspect, an exemplary CAR constructs comprise an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), and an intracellular stimulatory domain (e.g., an intracellular stimulatory domain decribed herein). In one aspect, an exemplary CAR construct comprises an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), an intracellular costimulatory signaling domain (e.g., a costimulatory signaling domain described herein) and/or an intracellular primary signaling domain (e.g., a primary signaling domain described herein).

An exemplary leader sequence is provided as SEQ ID NO: 2. An exemplary hinge/spacer sequence is provided as SEQ ID NO: 4 or SEQ ID NO:6 or SEQ ID NO:8 or SEQ ID NO: 10. An exemplary transmembrane domain sequence is provided as SEQ ID NO: 12. An exemplary sequence of the intracellular signaling domain of the 4-1BB protein is provided as SEQ ID NO: 14. An exemplary sequence of the intracellular signaling domain of CD27 is provided as SEQ ID NO: 16. An exemplary sequence of the intracellular signaling domain of CD28 is provided as SEQ ID NO:42. An exemplary sequence of the intracellular signaling domain of CD28 is provided as SEQ ID NO:44. An exemplary CD3zeta domain sequence is provided as SEQ ID NO: 18 or SEQ ID NO:20.

The nucleic acid sequences coding for the desired molecules can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

The present disclosure includes retroviral and lentiviral vector constructs expressing a CAR that can be directly transduced into a cell. Methods for viral transduction are described herein, and are well known in the art.

The present disclosure also includes an RNA construct that can be directly transfected into a cell. A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (SEQ ID NO:32). RNA so produced can efficiently transfect different kinds of cells. In one embodiment, the template includes sequences for the CAR. In an embodiment, an RNA CAR vector is transfected into a cell, e.g., a T cell or a NK cell, by electroporation.

### Antigen binding domain

The CAR-expressing cells of the invention comprise a target-specific binding element otherwise referred to as an antigen binding domain. The choice of moiety depends upon the type and number of ligands that define the surface of a target cell. For example, the antigen binding domain may be chosen or engineered to recognize a ligand that acts as a cell surface marker on target cells associated with a particular disease state, e.g., a tumor antigen associated with a particular cancer (e.g., an antigen binding domain that binds to a tumor antigen). In other embodiments, the antigen binding domain is chosen or engineered to recognize normal B cells, or a subpopulation of B cells, for depleting normal B cells or a target B cell population (e.g., an antigen binding domain that binds to a B cell antigen).

The antigen binding domain can be any domain that binds to the antigen including but not limited to a monoclonal antibody, a polyclonal antibody, a recombinant antibody, a bispecific antibody, a conjugated antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, and to an alternative scaffold known in the art to function as antigen binding domain, such as a recombinant fibronectin domain, a T cell receptor (TCR), a recombinant TCR with enhanced affinity, or a fragment there of, e.g., single chain TCR, and the like. In some instances, it is beneficial for the antigen binding domain to be derived from the same species in which the CAR will ultimately be used in. For example, for use in humans, it may be beneficial for the antigen binding domain of the CAR to comprise human or humanized residues for the antigen binding domain of an antibody or antibody fragment.

### Tumor antigens

The present disclosure provides immune effector cells (e.g., T cells, NK cells) that are engineered to contain one or more CARs that direct the immune effector cells to cancer cell. This is achieved through an antigen binding domain on the CAR that is specific for a tumor antigen There are two classes of tumor antigens (tumor antigens) that can be targeted by the CARs of the instant invention: (1) a tumor antigen that is expressed on the surface of cancer cells; and (2) a tumor antigen that itself is intracellar, however, a fragment of such antigen (peptide) is presented on the surface of the cancer cells by MHC (major histocompatibility complex). The cells of the invention comprise a second CAR, wherein the antigen binding domain of said second CAR binds to a tumor antigen other than a B-Cell antigen, wherein the tumor antigen other than a B-Cell antigen is a solid tumor antigen.

In one embodiment, the tumor antigen is expressed on both normal cells and cancer cells, but is expressed at lower levels on normal cells. In one embodiment, the method further comprises selecting a TA CAR that binds a tumor antigen with an affinity that allows the cell engineered to express the TA CAR to bind and kill the cancer cells expressing a tumor antigen but less than 30%, 25%, 20%, 15%, 10%, 5% or less of the normal cells expressing a tumor antigen are killed, e.g., as determined by an assay described herein. For example, a killing assay such as flow cytometry based on Cr51 CTL can be used. In one embodiment, the selected TA CAR has an antigen binding domain that has a binding affinity K_{D} of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the selected antigen binding domain has a binding affinity that is at least five-fold, 10-fold, 20-fold, 30-fold, 50-fold, 100-fold or 1,000-fold less than a reference antibody, e.g., an antibody described herein.

Accordingly, cells can be engineered to express, e.g., express, a TA CAR comprising an antigen binding domain that can target, e.g., bind to, any one of the exemplary tumor antigens (tumor antigens): CD123, CD30, CD171, CS-1, CLL-1 (CLECL1), CD33, EGFRvIII, GD2, GD3, Tn Ag , sTn Ag, Tn-O-Glycopeptides, Stn-O-Glycopeptides, PSMA, FLT3, FAP, TAG72, CD44v6, CEA, EPCAM, B7H3, KIT, IL-13Ra2, Mesothelin, IL-11Ra, PSCA, VEGFR2, LewisY, PDGFR-beta, PRSS21, SSEA-4, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, LAIR1, FCAR, LILRA2, CD300LF, CLEC12A, BST2, EMR2, LY75, GPC3, FCRL5, IGLL1, and peptides of these antigens presented on MHC.

The antigen binding domain of a TA CAR expressed by a cell of the invention, targets a tumor antigen that is associated with a solid tumor, e.g., expressed by a solid tumor cell, referred to herein as a solid tumor associated antigen, e.g., an antigen associated with mesothelioma (e.g., malignant pleural mesothelioma), lung cancer (e.g., non-small cell lung cancer, small cell lung cancer, squamous cell lung cancer, or large cell lung cancer), pancreatic cancer (e.g., pancreatic ductal adenocarcinoma), esophageal adenocarcinoma, ovarian cancer, breast cancer, colorectal cancer and bladder cancer or any combination thereof. In one embodiment, the disease is pancreatic cancer, e.g., metastatic pancreatic ductal adenocarcinoma (PDA), e.g., in a subject who has progressed on at least one prior standard therapy. In one embodiment, the disease is mesothelioma (e.g., malignant pleural mesothelioma), e.g., in a subject who has progressed on at least one prior standard therapy. In one embodiment, the disease is ovarian cancer, e.g., serous epithelial ovarian cancer, e.g., in a subject who has progressed after at least one prior regimen of standard therapy.

Examples of solid tumor associated antigens (i.e., solid tumor antigens) include, without limitation: EGFRvIII, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, CD24, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, GFRα4, and a peptide of any of these antigens presented on MHC.

In an embodiment, the antigen binding domain of a TA CAR, e.g., a TA CAR expressed by a cell of the invention, binds to human mesothelin. In an embodiment, the antigen binding domain is a murine scFv domain that binds to human mesothelin, e.g., SS1 or SEQ ID NO: 46. In an embodiment, the antigen binding domain is a humanized antibody or antibody fragment, e.g., scFv domain, derived from the murine SS1 scFv. In an embodiment, the antigen binding domain is a human antibody or antibody fragment that binds to human mesothelin. Exemplary human scFv domains (and their sequences) and the murine SS1 scFv that bind to mesothelin are provided in Table 2. CDR sequences are underlined. The scFv domain sequences provided in Table 2 include a light chain variable region (VL) and a heavy chain variable region (VH). The VL and VH are attached by a linker comprising the sequence GGGGSGGGGSGGGGS (SEQ ID NO: 30) (e.g., as shown in SS1 scFv domains) or GGGGSGGGGSGGGGSGGGGS (SEQ ID NO: 29) (e.g., as shown in M1, M2, M3, M4, M5, M6, M7, M8, M9, M10, M11, M12, M13, M14, M15, M16, M17, M18, M19, M20, M21, M22, M23, or M24 scFv domains). The scFv domains listed in Table 2 are in the following orientation: VL-linker-VH.

**Table 2. Antigen binding domains that bind to mesothelin**

| **Tumor antigen** | **Name** | **Amino acid sequence** | **SEQ ID NO:** |
|---|---|---|---|
| mesothelin | M5 (human) | QVQLVQSGAEVEKPGASVKVSCKASGYTFTDYYMHWVRQAPGQGLEWMGW INPNSGGTNYAQKFQGRVTMTRDTSISTAYMELSRLRSDDTAVYYCASGW DFDYWGQGTLVTVSSGGGGSGGGGSGGGGSGGGGSDIVMTQSPSSLSASV GDRVTITCRASQSIRYYLSWYQQKPGKAPKLLIYTASILQNGVPSRFSGS GSGTDFTLTISSLQPEDFATYYCLQTYTTPDFGPGTKVEIK | 51 |
| mesothelin | M11 (human) | QVQLQQSGAEVKKPGASVKVSCKASGYTFTGYYMHWVRQAPGQGLEWMGW INPNSGGTNYAQNFQGRVTMTRDTSISTAYMELRRLRSDDTAVYYCASGW DFDYWGQGTLVTVSSGGGGSGGGGSGGGGSGGGGSDIRMTQSPSSLSASV GDRVTITCRASQSIRYYLSWYQQKPGKAPKLLIYTASILQNGVPSRFSGS GSGTDFTLTISSLQPEDFATYYCLQTYTTPDFGPGTKVEIK | 57 |
| mesothelin | ss1 (murine) | Q V Q L Q Q S G P E L E K P G A S V K I S C K A S G Y S F T G Y T M N W V K Q S H G K S L E W I G L I T P Y N G A S S Y N Q K F R G K A T L T V D K S S S T A Y M D L L S L T S E D S A V Y F C A R G G Y D G R G F D Y W G Q G T T V T V S S G G G G S G G G G S G G G G S D I E L T Q S P A I M S A S P G E K V T M T C S A S S S V S Y M H W Y Q Q K S G T S P K R W I Y D T S K L A S G V P G R F S G S G S G N S Y S L T I S S V E A E D D A T Y Y C Q Q W S G Y P L T F G A G T K L E I | 46 |
| mesothelin | M1 (human) | QVQLQQSGAEVKKPGASVKVSCKASGYTFTGYYMHWVRQAPGQGLEWMGR INPNSGGTNYAQKFQGRVTMTRDTSISTAYMELSRLRSEDTAVYYCARGR YYGMDVWGQGTMVTVSSGGGGSGGGGSGGGGSGGGGSEIVLTQSPATLSL SPGERATISCRASQSVSSNFAWYQQRPGQAPRLLIYDASNRATGIPPRFS GSGSGTDFTLTISSLEPEDFAAYYCHQRSNWLYTFGQGTKVDIK | 47 |
| mesothelin | M2 (human) | QVQLVQSGAEVKKPGASVKVSCKASGYTFTGYYMHWVRQAPGQGLEWMGW INPNSGGTNYAQKFQGRVTMTRDTSISTAYMELSRLRSDDTAVYYCARDL RRTVVTPRAYYGMDVWGQGTTVTVSSGGGGSGGGGSGGGGSGGGGSDIQL TQSPSTLSASVGDRVTITCQASQDISNSLNWYQQKAGKAPKLLIYDASTL ETGVPSRFSGSGSGTDFSFTISSLQPEDIATYYCQQHDNLPLTFGQGTKV EIK | 48 |
| mesothelin | M3 (human) | QVQLVQSGAEVKKPGAPVKVSCKASGYTFTGYYMHWVRQAPGQGLEWMGW INPNSGGTNYAQKFQGRVTMTRDTSISTAYMELSRLRSDDTAVYYCARGE WDGSYYYDYWGQGTLVTVSSGGGGSGGGGSGGGGSGGGGSDIVLTQTPSS LSASVGDRVTITCRASQSINTYLNWYQHKPGKAPKLLIYAASSLQSGVPS RFSGSGSGTDFTLTISSLQPEDFATYYCQQSFSPLTFGGGTKLEIK | 49 |
| mesothelin | M4 (human) | QVQLVESGGGLVQPGGSLRLSCAASGFTFSSYWMHWVRQVPGKGLVWVSR INTDGSTTTYADSVEGRFTISRDNAKNTLYLQMNSLRDDDTAVYYCVGGH WAVWGQGTTVTVSSGGGGSGGGGSGGGGSGGGGSDIQMTQSPSTLSASVG DRVTITCRASQSISDRLAWYQQKPGKAPKLLIYKASSLESGVPSRFSGSG SGTEFTLTISSLQPDDFAVYYCQQYGHLPMYTFGQGTKVEIK | 50 |
| mesothelin | M6 (human) | QVQLVQSGAEVKKPGASVKVSCKASGYTFTSYYMHWVRQAPGQGLEWMGI INPSGGSTSYAQKFQGRVTMTRDTSTSTVYMELSSLRSEDTAVYYCARYR LIAVAGDYYYYGMDVWGQGTMVTVSSGGGGSGGGGSGGGGSGGGGSDIQM TQSPSSVASVGDRVTITCRASQGVGRWLAWYQQKPGTAPKLLIYAASTLQ SGVPSRFSGSGSGTDFTLTINNLQPEDFATYYCQQANSFPLTFGGGTRLE IK | 52 |
| mesothelin | M7 (human) | QVQLVQSGGGVVQPGRSLRLSCAASGFTFSSYAMHWVRQAPGKGLEWVAV ISYDGSNKYYADSVKGRFTISRDNSKNTLYLQMNSLRAEDTAVYYCARWK VSSSSPAFDYWGQGTLVTVSSGGGGSGGGGSGGGGSGGGGSEIVLTQSPA TLSLSPGERAILSCRASQSVYTKYLGWYQQKPGQAPRLLIYDASTRATGI PDRFSGSGSGTDFTLTINRLEPEDFAVYYCQHYGGSPLITFGQGTRLEIK | 53 |
| mesothelin | M8 (human) | QVQLQQSGAEVKKPGASVKVSCKTSGYPFTGYSLHWVRQAPGQGLEWMGW INPNSGGTNYAQKFQGRVTMTRDTSISTAYMELSRLRSDDTAVYYCARDH YGGNSLFYWGQGTLVTVSSGGGGSGGGGSGGGGSGGGGSDIQLTQSPSSI SASVGDTVSITCRASQDSGTWLAWYQQKPGKAPNLLMYDASTLEDGVPSR FSGSASGTEFTLTVNRLQPEDSATYYCQQYNSYPLTFGGGTKVDIK | 54 |
| mesothelin | M9 (human) | QVQLVQSGAEVKKPGASVEVSCKASGYTFTSYYMHWVRQAPGQGLEWMGI INPSGGSTGYAQKFQGRVTMTRDTSTSTVHMELSSLRSEDTAVYYCARGG YSSSSDAFDIWGQGTMVTVSSGGGGSGGGGSGGGGSGGGGSDIQMTQSPP SLSASVGDRVTITCRASQDISSALAWYQQKPGTPPKLLIYDASSLESGVP SRFSGSGSGTDFTLTISSLQPEDFATYYCQQFSSYPLTFGGGTRLEIK | 55 |
| mesothelin | M10 (human) | QVQLVQSGAEVKKPGASVKVSCKASGYTFTSYGISWVRQAPGQGLEWMGW ISAYNGNTNYAQKLQGRVTMTTDTSTSTAYMELRSLRSDDTAVYYCARVA GGIYYYYGMDVWGQGTTITVSSGGGGSGGGGSGGGGSGGGGSDIVMTQTP DSLAVSLGERATISCKSSHSVLYNRNNKNYLAWYQQKPGQPPKLLFYWAS | 56 |
| | | TRKSGVPDRFSGSGSGTDFTLTISSLQPEDFATYFCQQTQTFPLTFGQGT RLEIN | |
| mesothelin | M12 (human) | QVQLVQSGAEVKKPGASVKVSCKASGYTFTGYYMHWVRQAPGQGLEWMGR INPNSGGTNYAQKFQGRVTMTTDTSTSTAYMELRSLRSDDTAVYYCARTT TSYAFDIWGQGTMVTVSSGGGGSGGGGSGGGGSGGGGSDIQLTQSPSTLS ASVGDRVTITCRASQSISTWLAWYQQKPGKAPNLLIYKASTLESGVPSRF SGSGSGTEFTLTISSLQPDDFATYYCQQYNTYSPYTFGQGTKLEIK | 58 |
| mesothelin | M13 (human) | QVQLVQSGGGLVKPGGSLRLSCEASGFIFSDYYMGWIRQAPGKGLEWVSY IGRSGSSMYYADSVKGRFTFSRDNAKNSLYLQMNSLRAEDTAVYYCAASP VVAATEDFQHWGQGTLVTVSSGGGGSGGGGSGGGGSGGGGSDIVMTQTPA TLSLSPGERATLSCRASQSVTSNYLAWYQQKPGQAPRLLLFGASTRATGI PDRFSGSGSGTDFTLTINRLEPEDFAMYYCQQYGSAPVTFGQGTKLEIK | 59 |
| mesothelin | M14 (human) | QVQLVQSGAEVRAPGASVKISCKASGFTFRGYYIHWVRQAPGQGLEWMGI INPSGGSRAYAQKFQGRVTMTRDTSTSTVYMELSSLRSDDTAMYYCARTA SCGGDCYYLDYWGQGTLVTVSSGGGGSGGGGSGGGGSGGGGSDIQMTQSP PTLSASVGDRVTITCRASENVNIWLAWYQQKPGKAPKLLIYKSSSLASGV PSRFSGSGSGAEFTLTISSLQPDDFATYYCQQYQSYPLTFGGGTKVDIK | 60 |
| mesothelin | M15 (human) | QVQLVQSGGGLVQPGRSLRLSCAASGFTFDDYAMHWVRQAPGKGLEWVSG ISWNSGSIGYADSVKGRFTISRDNAKNSLYLQMNSLRAEDTAVYYCAKDG SSSWSWGYFDYWGQGTLVTVSSGGGGSGGGGSGGGGSSELTQDPAVSVA LGQTVRTTCQGDALRSYYASWYQQKPGQAPMLVIYGKNNRPSGIPDRFSG SDSGDTASLTITGAQAEDEADYYCNSRDSSGYPVFGTGTKVTVL | 61 |
| mesothelin | M16 (human) | EVQLVESGGGLVQPGRSLRLSCAASGFTFDDYAMHWVRQAPGKGLEWVSG ISWNSGSTGYADSVKGRFTISRDNAKNSLYLQMNSLRAEDTALYYCAKDS SSWYGGGSAFDIWGQGTMVTVSSGGGGSGGGGSGGGGSSELTQEPAVSV ALGQTVRITCQGDSLRSYYASWYQQKPGQAPVLVIFGRSRRPSGIPDRFS GSSSGNTASLIITGAQAEDEADYYCNSRDNTANHYVFGTGTKLTVL | 62 |
| mesothelin | M17 (human) | EVQLVESGGGLVQPGRSLRLSCAASGFTFDDYAMHWVRQAPGKGLEWVSG ISWNSGSTGYADSVKGRFTISRDNAKNSLYLQMNSLRAEDTALYYCAKDS SSWYGGGSAFDIWGQGTMVTVSSGGGGSGGGGSGGGGSSSELTQDPAVSV ALGQTVRITCQGDSLRSYYASWYQQKPGQAPVLVIYGKNNRPSGIPDRFS GSSSGNTASLTITGAQAEDEADYYCNSRGSSGNHYVFGTGTKVTVL | 63 |
| mesothelin | M18 (human) | QVQLVQSGGGLVQPGGSLRLSCAASGFTFSSYWMHWVRQAPGKGLVWVSR INSDGSSTSYADSVKGRFTISRDNAKNTLYLQMNSLRAEDTAVYYCVRTG WVGSYYYYMDVWGKGTTVTVSSGGGGSGGGGSGGGGSGGGGSEIVLTQSP GTLSLSPGERATLSCRASQSVSSNYLAWYQQKPGQPPRLLIYDVSTRATG IPARFSGGGSGTDFTLTISSLEPEDFAVYYCQQRSNWPPWTFGQGTKVEI K | 64 |
| mesothelin | M19 (human) | QVQLVQSGGGVVQPGRSLRLSCAASGFTFSSYGMHWVRQAPGKGLEWVAV ISYDGSNKYYADSVKGRFTISRDNSKNTLYLQMNSLRAEDTAVYYCAKGY SRYYYYGMDVWGQGTTVTVSSGGGGSGGGGSGGGGSGGGGSEIVMTQSPA TLSLSPGERAILSCRASQSVYTKYLGWYQQKPGQAPRLLIYDASTRATGI PDRFSGSGSGTDFTLTINRLEPEDFAVYYCQHYGGSPLITFGQGTKVDIK | 65 |
| mesothelin | M20 (human) | QVQLVQSGGGLVQPGGSLRLSCAASGFTFSSYAMSWVRQAPGKGLEWVSA ISGSGGSTYYADSVKGRFTISRDNSKNTLYLQMNSLRAEDTAVYYCAKRE AAAGHDWYFDLWGRGTLVTVSSGGGGSGGGGSGGGGSGGGGSDIRVTQSP SSLSASVGDRVTITCRASQSISSYLNWYQQKPGKAPKLLIYAASSLQSGV PSRFSGSGSGTDFTLTISSLQPEDFATYYCQQSYSIPLTFGQGTKVEIK | 66 |
| mesothelin | M21 (human) | QVQLVQSWAEVKKPGASVKVSCKASGYTFTSYYMHWVRQAPGQGLEWMGI INPSGGSTSYAQKFQGRVTMTRDTSTSTVYMELSNLRSEDTAVYYCARSP RVTTGYFDYWGQGTLVTVSSGGGGSGGGGSGGGGSGGGGSDIQLTQSPST LSASVGDRVTITCRASQSISSWLAWYQQKPGKAPKLLIYKASSLESGVPS RFSGSGSGTEFTLTISSLQPDDFATYYCQQYSSYPLTFGGGTRLEIK | 67 |
| mesothelin | M22 (human) | QVQLVQSGAEVRRPGASVKISCRASGDTSTRHYIHWLRQAPGQGPEWMGV INPTTGPATGSPAYAQMLQGRVTMTRDTSTRTVYMELRSLRFEDTAVYYC ARSVVGRSAPYYFDYWGQGTLVTVSSGGGGSGGGGSGGGGSGGGGSDIQM TQSPSSLSASVGDRVTITCRASQGISDYSAWYQQKPGKAPKLLIYAASTL | 68 |
| | | QSGVPSRFSGSGSGTDFTLTISYLQSEDFATYYCQQYYSYPLTFGGGTKV DIK | |
| mesothelin | M23 (human) | QVQLQQSGAEVKKPGASVKVSCKASGYTFTNYYMHWVRQAPGQGLEWMGI INPSGGYTTYAQKFQGRLTMTRDTSTSTVYMELSSLRSEDTAVYYCARIR SCGGDCYYFDNWGQGTLVTVSSGGGGSGGGGSGGGGSGGGGSDIQLTQSP STLSASVGDRVTITCRASENVNIWLAWYQQKPGKAPKLLIYKSSSLASGV PSRFSGSGSGAEFTLTISSLQPDDFATYYCQQYQSYPLTFGGGTKVDIK | 69 |
| mesothelin | M24 (human) | QITLKESGPALVKPTQTLTLTCTFSGFSLSTAGVHVGWIRQPPGKALEWL ALISWADDKRYRPSLRSRLDITRVTSKDQVVLSMTNMQPEDTATYYCALQ GFDGYEANWGPGTLVTVSSGGGGSGGGGSGGGGSGGGGSDIVMTQSPSSL SASAGDRVTITCRASRGISSALAWYQQKPGKPPKLLIYDASSLESGVPSR FSGSGSGTDFTLTIDSLEPEDFATYYCQQSYSTPWTFGQGTKVDIK | 70 |

The sequences of the CDR sequences of the scFv domains of the mesothelin antigen binding domains provided in Table 2 are shown in Table 3 for the heavy chain variable domains and in Table 4 for the light chain variable domains.

**Table 3. Amino acid sequences for the heavy chain (HC) CDR1, CDR2, and CDR3 regions of human anti-mesothelin scFvs**

| **Descrip.** | **HC-CDR1** | **SEQ ID NO:** | **HC-CDR2** | **SEQ ID NO:** | **HC-CDR3** | **SEQ ID NO:** |
|---|---|---|---|---|---|---|
| M5 | GYTFTDYYMH | 115 | WINPNSGGTNYAQKFQG | 34 | GWDFDY | 159 |
| M11 | GYTFTGYYMH | 121 | WINPNSGGTNYAQNFQG | 141 | GWDFDY | 165 |
| Ss1 | GYSFTGYTMN | 132 | LITPYNGASSYNQKFRG | 154 | GGYDGRGFDY | 179 |
| M1 | GYTFTGYYMH | 113 | RTNPNSGGTNYAQKFQG | 133 | GRYYGMDV | 155 |
| M2 | GYTFTGYYMH | 113 | WINPNSGGTNYAQKFQG | 134 | DLRRTVVTPRAYYG MDV | 156 |
| M3 | GYTFTGYYMH | 113 | WINPNSGGTNYAQKFQG | 134 | GEWDGSYYYDY | 157 |
| M4 | GFTFSSYWMH | 114 | RINTDGSTTTYADSVEG | 135 | GHWAV | 158 |
| M6 | GYTFTSYYMH | 116 | IINPSGGSTSYAQKFQ | 136 | YRLIAVAGDYYYYG MDV | 160 |
| M7 | GFTFSSYAMH | 117 | VISYDGSNKYYADSVKG | 137 | WKVSSSSPAFDY | 161 |
| M8 | GYPFTGYSLH | 118 | WINPNSGGTNYAQKFQG | 138 | DHYGGNSLFY | 162 |
| M9 | GYTFTSYYMH | 119 | IINPSGGSTGYAQKFQG | 139 | GGYSSSSDAFDI | 163 |
| M10 | GYTFTSYGIS | 120 | WISAYNGNTNYAQKLQ | 140 | VAGGIYYYYGMDV | 164 |
| M12 | GYTFTGYYMH | 121 | RINPNSGGTNYAQKFQG | 142 | TTTSYAFDI | 166 |
| M13 | GFIFSDYYMG | 122 | YIGRSGSSMYYADSVKG | 143 | SPVVAATEDFQH | 167 |
| M14 | GFTFRGYYIH | 123 | IINPSGGSRAYAQKFQG | 144 | TASCGGDCYYLDY | 168 |
| M15 | GFTFDDYAMH | 124 | GISWNSGSIGYADSVK | 145 | DGSSSWSWGYFDY | 169 |
| M16 | GFTFDDYAMH | 124 | GISWNSGSTGYADSVKG | 140 | DSSSWYGGGSAFDI | 170 |
| M17 | GFTFDDYAMH | 124 | GISWNSGSTGYADSVKG | 146 | DSSSWYGGGSAFDI | 171 |
| M18 | GFTFSSYWMH | 125 | RINSDGSSTSYADSVKG | 147 | TGWVGSYYYYMDV | 172 |
| M19 | GFTFSSYGMH | 126 | VISYDGSNKYYADSVKG | 148 | GYSRYYYYGMDV | 173 |
| M20 | GFTFSSYAMS | 127 | AISGSGGSTYYADSVKG | 149 | REAAAGHDWYFDL | 174 |
| M21 | GYTFTSYYMH | 128 | IINPSGGSTSYAQKFQG | 150 | SPRVTTGYFDY | 175 |
| M22 | GDTSTRHYIH | 129 | VINPTTGPATGSPAYAQMLQ G | 151 | SVVGRSAPYYFDY | 176 |
| M23 | GYTFTNYYMH | 130 | IINPSGGYTTYAQKFQG | 152 | IRSCGGDCYYFDN | 177 |
| M24 | GFSTSTAGVHVG | 131 | LISWADDKRYRPSLRS | 153 | QGFDGYEAN | 178 |

**Table 4. Amino acid sequences for the light chain (LC) CDR1, CDR2, and CDR3 regions of human anti-mesothelin scFvs**

| **Description** | **LC-CDR1** | **SEQ ID NO:** | **LC-CDR2** | **SEQ ID NO:** | **LC-CDR3** | **SEQ ID NO:** |
|---|---|---|---|---|---|---|
| M5 | RASQSIRYYLS | 184 | TASILQN | 209 | LQTYTTPD | 234 |
| M11 | RASQSIRYYLS | 190 | TASILQN | 215 | LQTYTTPD | 240 |
| Ss1 | SASSSVSYMH | 204 | DTSKLAS | 229 | QQWSGYPLT | 254 |
| M1 | RASQSVSSNFA | 180 | DASNRAT | 205 | HQRSNWLⱼYT | 230 |
| M2 | QASQDISNSLN | 181 | DASTLET | 206 | QQHDNLPLT | 231 |
| M3 | RASQSINTYLN | 182 | AASSLQS | 207 | QQSFSPLT | 232 |
| M4 | RASQSISDRLA | 183 | KASSLES | 208 | QQYGHLPMYT | 233 |
| M6 | RASQGVGRWLA | 185 | AASTLQS | 210 | QQANSFPLT | 235 |
| M7 | RASQSVYTKYLG | 186 | DASTRAT | 211 | QHYGGSPLIT | 236 |
| M8 | RASQDSGTWLA | 187 | DASTLED | 212 | QQYNSYPLT | 237 |
| M9 | RASQDISSALA | 188 | DASSLES | 213 | QQFSSYPLT | 238 |
| M10 | KSSHSVLYNRNNKNYLA | 189 | WASTRKS | 214 | QQTQTFPLT | 239 |
| M12 | RASQSISTWLA | 191 | KASTLES | 216 | QQYNTYSPYT | 241 |
| M13 | RASQSVTSNYLA | 192 | GAS TRAT | 217 | QQYGSAPVT | 242 |
| M14 | RASENVNIWLA | 193 | KSSSLAS | 218 | QQYQSYPLT | 243 |
| M15 | QGDALRSYYAS | 194 | GKNNRPS | 219 | NSRDSSGYPV | 244 |
| M16 | QGDSLRSYYAS | 195 | GRSRRPS | 220 | NSRDNTANHYV | 245 |
| M17 | QGDSLRSYYAS | 196 | GKNNRPS | 221 | NSRGSSGNHYV | 246 |
| M18 | RASQSVSSNYLA | 197 | DVSTRAT | 222 | QQRSNWPPWT | 247 |
| M19 | RASQSVYTKYLG | 198 | DASTRAT | 223 | QHYGGSPLIT | 248 |
| M20 | RASQSISSYLN | 199 | AASSLQS | 224 | QQSYSIPLT | 249 |
| M21 | RASQSISSWLA | 200 | KASSLES | 225 | QQYSSYPLT | 250 |
| M22 | RASQGISDYS | 201 | AASTLQS | 226 | QQYYSYPLT | 251 |
| M23 | RASENVNIWLA | 202 | KSSSLAS | 227 | QQYQSYPLT | 252 |
| M24 | RASRGISSALA | 203 | DASSLES | 228 | QQSYSTPWT | 253 |

Any known anti-mesothelin binding domain, from, for example, a known antibody, bispecific molecule or CAR, may be suitable for use in the TA CAR of the present invention. For example, the antigen binding domain against mesothelin is or may be derived from an antigen binding, e.g., CDRs or VH and VL, of an antibody, antigen-binding fragment or CAR described in, e.g., PCT publication WO2015/090230. In embodiments, the antigen binding domain against mesothelin is or is derived from an antigen binding portion, e.g., CDRs or VH and VL, of an antibody, antigen-binding fragment, or CAR described in, e.g., PCT publication WO1997/025068, WO1999/028471, WO2005/014652, WO2006/099141, WO2009/045957, WO2009/068204, WO2013/142034, WO2013/040557, or WO2013/063419.

In one embodiment, the mesothelin binding domain comprises one or more (e.g., all three) light chain complementary determining region 1 (LC CDR1), light chain complementary determining region 2 (LC CDR2), and light chain complementary determining region 3 (LC CDR3) of a mesothelin binding domain described herein, e.g., provided in Table 2 or 4, and/or one or more (e.g., all three) heavy chain complementary determining region 1 (HC CDR1), heavy chain complementary determining region 2 (HC CDR2), and heavy chain complementary determining region 3 (HC CDR3) of a mesothelin binding domain described herein, e.g., provided in Table 2 or 3. In one embodiment, the mesothelin binding domain comprises one, two, or all of LC CDR1, LC CDR2, and LC CDR3 of any amino acid sequences as provided in Table 4; and one, two or three of all of HC CDR1, HC CDR2 and HC CDR3, of any amino acid acid sequences as provided in Table 3.

In one embodiment, the mesothelin antigen binding domain comprises:
(i)
   (a) a LC CDR1 amino acid sequence of SEQ ID NO: 184, a LC CDR2 amino acid sequence of SEQ ID NO: 209, and a LC CDR3 amino acid sequence of SEQ ID NO: 234; and
   (b) a HC CDR1 amino acid sequence of SEQ ID NO: 115, a HC CDR2 amino acid sequence of SEQ ID NO: 134, and a HC CDR3 amino acid sequence of SEQ ID NO: 159;
(ii)
   (a) a LC CDR1 amino acid sequence of SEQ ID NO: 190, a LC CDR2 amino acid sequence of SEQ ID NO: 215, and a LC CDR3 amino acid sequence of SEQ ID NO: 240; and
   (b) a HC CDR1 amino acid sequence of SEQ ID NO: 121, a HC CDR2 amino acid sequence of SEQ ID NO: 141, and a HC CDR3 amino acid sequence of SEQ ID NO: 165;
(iii)
   (a) a LC CDR1 amino acid sequence of SEQ ID NO: 204, a LC CDR2 amino acid sequence of SEQ ID NO: 229, and a LC CDR3 amino acid sequence of SEQ ID NO: 254; and
   (b) a HC CDR1 amino acid sequence of SEQ ID NO: 132, a HC CDR2 amino acid sequence of SEQ ID NO: 154, and a HC CDR3 amino acid sequence of SEQ ID NO: 179;
(iv)
   (a) a LC CDR1 amino acid sequence of SEQ ID NO: 180, a LC CDR2 amino acid sequence of SEQ ID NO: 205, and a LC CDR3 amino acid sequence of SEQ ID NO: 230; and
   (b) a HC CDR1 amino acid sequence of SEQ ID NO: 113, a HC CDR2 amino acid sequence of SEQ ID NO: 133, and a HC CDR3 amino acid sequence of SEQ ID NO: 155;
(v)
   (a) a LC CDR1 amino acid sequence of SEQ ID NO: 181, a LC CDR2 amino acid sequence of SEQ ID NO: 206, and a LC CDR3 amino acid sequence of SEQ ID NO: 231; and
   (b) a HC CDR1 amino acid sequence of SEQ ID NO: 113, a HC CDR2 amino acid sequence of SEQ ID NO: 134, and a HC CDR3 amino acid sequence of SEQ ID NO: 156;
(vi)
   (a) a LC CDR1 amino acid sequence of SEQ ID NO: 182, a LC CDR2 amino acid sequence of SEQ ID NO: 207, and a LC CDR3 amino acid sequence of SEQ ID NO: 232; and
   (b) a HC CDR1 amino acid sequence of SEQ ID NO: 113, a HC CDR2 amino acid sequence of SEQ ID NO: 134, and a HC CDR3 amino acid sequence of SEQ ID NO: 157;
(vii)
   (a) a LC CDR1 amino acid sequence of SEQ ID NO: 183, a LC CDR2 amino acid sequence of SEQ ID NO: 208, and a LC CDR3 amino acid sequence of SEQ ID NO: 233; and
   (b) a HC CDR1 amino acid sequence of SEQ ID NO: 114, a HC CDR2 amino acid sequence of SEQ ID NO: 135, and a HC CDR3 amino acid sequence of SEQ ID NO: 158;
(viii)
   (a) a LC CDR1 amino acid sequence of SEQ ID NO: 186, a LC CDR2 amino acid sequence of SEQ ID NO: 210, and a LC CDR3 amino acid sequence of SEQ ID NO: 235; and
   (b) a HC CDR1 amino acid sequence of SEQ ID NO: 116, a HC CDR2 amino acid sequence of SEQ ID NO: 136, and a HC CDR3 amino acid sequence of SEQ ID NO: 160;
(ix)
   (a) a LC CDR1 amino acid sequence of SEQ ID NO: 186, a LC CDR2 amino acid sequence of SEQ ID NO: 211, and a LC CDR3 amino acid sequence of SEQ ID NO: 236; and
   (b) a HC CDR1 amino acid sequence of SEQ ID NO: 117, a HC CDR2 amino acid sequence of SEQ ID NO: 137, and a HC CDR3 amino acid sequence of SEQ ID NO: 161;
(x)
   (a) a LC CDR1 amino acid sequence of SEQ ID NO: 187, a LC CDR2 amino acid sequence of SEQ ID NO: 212, and a LC CDR3 amino acid sequence of SEQ ID NO: 237; and
   (b) a HC CDR1 amino acid sequence of SEQ ID NO: 118, a HC CDR2 amino acid sequence of SEQ ID NO: 138, and a HC CDR3 amino acid sequence of SEQ ID NO: 162;
(xi)
   (a) a LC CDR1 amino acid sequence of SEQ ID NO: 188, a LC CDR2 amino acid sequence of SEQ ID NO: 213, and a LC CDR3 amino acid sequence of SEQ ID NO: 238; and
   (b) a HC CDR1 amino acid sequence of SEQ ID NO: 119, a HC CDR2 amino acid sequence of SEQ ID NO: 139, and a HC CDR3 amino acid sequence of SEQ ID NO: 163;
(xii)
   (a) a LC CDR1 amino acid sequence of SEQ ID NO: 189, a LC CDR2 amino acid sequence of SEQ ID NO: 214, and a LC CDR3 amino acid sequence of SEQ ID NO: 239; and
   (b) a HC CDR1 amino acid sequence of SEQ ID NO: 120, a HC CDR2 amino acid sequence of SEQ ID NO: 140, and a HC CDR3 amino acid sequence of SEQ ID NO: 164;
(xiii)
   (a) a LC CDR1 amino acid sequence of SEQ ID NO: 191, a LC CDR2 amino acid sequence of SEQ ID NO: 216, and a LC CDR3 amino acid sequence of SEQ ID NO: 241; and
   (b) a HC CDR1 amino acid sequence of SEQ ID NO: 121, a HC CDR2 amino acid sequence of SEQ ID NO: 142, and a HC CDR3 amino acid sequence of SEQ ID NO: 166;
(xiv)
   (a) a LC CDR1 amino acid sequence of SEQ ID NO: 192, a LC CDR2 amino acid sequence of SEQ ID NO: 217, and a LC CDR3 amino acid sequence of SEQ ID NO: 242; and
   (b) a HC CDR1 amino acid sequence of SEQ ID NO: 122, a HC CDR2 amino acid sequence of SEQ ID NO: 143, and a HC CDR3 amino acid sequence of SEQ ID NO: 167;
(xv)
   (a) a LC CDR1 amino acid sequence of SEQ ID NO: 193, a LC CDR2 amino acid sequence of SEQ ID NO: 218, and a LC CDR3 amino acid sequence of SEQ ID NO: 243; and
   (b) a HC CDR1 amino acid sequence of SEQ ID NO: 123, a HC CDR2 amino acid sequence of SEQ ID NO: 144, and a HC CDR3 amino acid sequence of SEQ ID NO: 168;
(xvi)
   (a) a LC CDR1 amino acid sequence of SEQ ID NO: 194, a LC CDR2 amino acid sequence of SEQ ID NO: 219, and a LC CDR3 amino acid sequence of SEQ ID NO: 244; and
   (b) a HC CDR1 amino acid sequence of SEQ ID NO: 124, a HC CDR2 amino acid sequence of SEQ ID NO: 145, and a HC CDR3 amino acid sequence of SEQ ID NO: 169;
(xvii)
   (a) a LC CDR1 amino acid sequence of SEQ ID NO: 195, a LC CDR2 amino acid sequence of SEQ ID NO: 220, and a LC CDR3 amino acid sequence of SEQ ID NO: 245; and
   (b) a HC CDR1 amino acid sequence of SEQ ID NO: 124, a HC CDR2 amino acid sequence of SEQ ID NO: 146, and a HC CDR3 amino acid sequence of SEQ ID NO: 170;
(xviii)
   (a) a LC CDR1 amino acid sequence of SEQ ID NO: 196, a LC CDR2 amino acid sequence of SEQ ID NO: 221, and a LC CDR3 amino acid sequence of SEQ ID NO: 246; and
   (b) a HC CDR1 amino acid sequence of SEQ ID NO: 124, a HC CDR2 amino acid sequence of SEQ ID NO: 146, and a HC CDR3 amino acid sequence of SEQ ID NO: 171;
(xix)
   (a) a LC CDR1 amino acid sequence of SEQ ID NO: 197, a LC CDR2 amino acid sequence of SEQ ID NO: 222, and a LC CDR3 amino acid sequence of SEQ ID NO: 247; and
   (b) a HC CDR1 amino acid sequence of SEQ ID NO: 125, a HC CDR2 amino acid sequence of SEQ ID NO: 147, and a HC CDR3 amino acid sequence of SEQ ID NO: 172;
(xx)
   (a) a LC CDR1 amino acid sequence of SEQ ID NO: 198, a LC CDR2 amino acid sequence of SEQ ID NO: 223, and a LC CDR3 amino acid sequence of SEQ ID NO: 248; and
   (b) a HC CDR1 amino acid sequence of SEQ ID NO: 126, a HC CDR2 amino acid sequence of SEQ ID NO: 148, and a HC CDR3 amino acid sequence of SEQ ID NO: 173;
(xxi)
   (a) a LC CDR1 amino acid sequence of SEQ ID NO: 199, a LC CDR2 amino acid sequence of SEQ ID NO: 224, and a LC CDR3 amino acid sequence of SEQ ID NO: 249; and
   (b) a HC CDR1 amino acid sequence of SEQ ID NO: 127, a HC CDR2 amino acid sequence of SEQ ID NO: 149, and a HC CDR3 amino acid sequence of SEQ ID NO: 174;
(xxii)
   (a) a LC CDR1 amino acid sequence of SEQ ID NO: 200, a LC CDR2 amino acid sequence of SEQ ID NO: 225, and a LC CDR3 amino acid sequence of SEQ ID NO: 250; and
   (b) a HC CDR1 amino acid sequence of SEQ ID NO: 128, a HC CDR2 amino acid sequence of SEQ ID NO: 150, and a HC CDR3 amino acid sequence of SEQ ID NO: 175;
(xxiii)
   (a) a LC CDR1 amino acid sequence of SEQ ID NO: 201, a LC CDR2 amino acid sequence of SEQ ID NO: 226, and a LC CDR3 amino acid sequence of SEQ ID NO: 251; and
   (b) a HC CDR1 amino acid sequence of SEQ ID NO: 129, a HC CDR2 amino acid sequence of SEQ ID NO: 151, and a HC CDR3 amino acid sequence of SEQ ID NO: 176;
(xxiv)
   (a) a LC CDR1 amino acid sequence of SEQ ID NO: 202, a LC CDR2 amino acid sequence of SEQ ID NO: 227, and a LC CDR3 amino acid sequence of SEQ ID NO: 252; and
   (b) a HC CDR1 amino acid sequence of SEQ ID NO: 130, a HC CDR2 amino acid sequence of SEQ ID NO: 152, and a HC CDR3 amino acid sequence of SEQ ID NO: 177; or
(xxv)
   (a) a LC CDR1 amino acid sequence of SEQ ID NO: 203, a LC CDR2 amino acid sequence of SEQ ID NO: 228, and a LC CDR3 amino acid sequence of SEQ ID NO: 253; and
   (b) a HC CDR1 amino acid sequence of SEQ ID NO: 131, a HC CDR2 amino acid sequence of SEQ ID NO: 153, and a HC CDR3 amino acid sequence of SEQ ID NO: 178.

In one embodiment, the mesothelin binding domain comprises a light chain variable region described herein (e.g., in Table 2) and/or a heavy chain variable region described herein (e.g., in Table 2). In one embodiment, the mesothelin binding domain is a scFv comprising a light chain and a heavy chain of an amino acid sequence listed in Table 2. In an embodiment, the mesothelin binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 2, or a sequence with 95-99% identity with an amino acid sequence provided in Table 2; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 2, or a sequence with 95-99% identity to an amino acid sequence provided in Table 2.

In one embodiment, the mesothelin binding domain comprises an amino acid sequence selected from a group consisting of SEQ ID NO: 46; SEQ ID NO: 47; SEQ ID NO: 48; SEQ ID NO: 49; SEQ ID NO: 50; SEQ ID NO: 51; SEQ ID NO: 52; SEQ ID NO: 53; SEQ ID NO: 54; SEQ ID NO: 55; SEQ ID NO: 56; SEQ ID NO: 57; SEQ ID NO: 58; SEQ ID NO: 59; SEQ ID NO: 60; SEQ ID NO: 61; SEQ ID NO: 62; SEQ ID NO: 63; SEQ ID NO: 64; SEQ ID NO: 65; SEQ ID NO: 66; SEQ ID NO: 67, SEQ ID NO: 68; SEQ ID NO: 69; and SEQ ID NO: 70; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the mesothelin binding domain is a scFv, and a light chain variable region comprising an amino acid sequence described herein, e.g., in Table 2, is attached to a heavy chain variable region comprising an amino acid sequence described herein, e.g., in Table 2, via a linker, e.g., a linker described herein. In one embodiment, the mesothelin binding domain includes a (Gly4-Ser)n linker, wherein n is 1, 2, 3, 4, 5, or 6, preferably 4 (SEQ ID NO: 80). The light chain variable region and heavy chain variable region of a scFv can be, e.g., in any of the following orientations: light chain variable region-linker-heavy chain variable region or heavy chain variable region-linker-light chain variable region.

In an embodiment, the antigen binding domain of a TA CAR, e.g., a TA CAR expressed by a cell of the invention, binds to human EGFRvIII. In an embodiment, the antigen binding domain is a murine scFv domain that binds to human EGFRvIII such as, e.g., mu310C. In an embodiment, the antigen binding domain is a humanized antibody or antibody fragment, e.g., scFv domain, derived from the murine mu310C scFv. Exemplary humanized scFv domains (and their sequences) and murine SS1 scFv that bind to EGFRvIII are provided in Table 5.

In an embodiment, the antigen binding domain of a TA CAR, e.g., a TA CAR expressed by a cell of the inveniton, binds to human claudin 6 (CLDN6). In an embodiment, the antigen binding domain is a murine scFv domain that binds to human CLDN6. In an embodiment, the antigen binding domain is a humanized antibody or antibody fragment. Exemplary scFv domains (and their sequences) that bind to CLDN6 are provided in Table 5. The scFv domain sequences provided in Table 5 include a light chain variable region (VL) and a heavy chain variable region (VH). The VL and VH are attached by a linker comprising the sequence GGGGSGGGGSGGGGSGGGGS (SEQ ID NO: 29), e.g., in the following orientation: VL-linker-VH.

**Table 5. Antigen binding domains that bind to the tumor antigen EGFRvIII**

| **Tumor antigen** | **Name** | **Amino acid sequence** | **SEQ ID NO:** |
|---|---|---|---|
| EGFR vIII | huscFv1 | Eiqlvqsgaevkkpgatvkisckgsgfniedyyihwvqqapgkglewmgridpendetkygpif qgrvtitadtstntvymelsslrsedtavyycafrggvywgqgttvtvssggggsggggsggggsgg ggsdvvmtqspdslavslgeratinckssqslldsdgktylnwlqqkpgqppkrlislvskldsgvp drfsgsgsgtdftltisslqaedvavyycwqgthfpgtfgggtkveik | 71 |
| EGFR vIII | huscFv2 | Dvvmtqspdslavslgeratinckssqslldsdgktylnwlqqkpgqppkrlislvskldsgvpdrfs gsgsgtdftltisslqaedvavyycwqgthfpgtfgggtkveikggggsggggsggggsggggsei qlvqsgaevkkpgatvkisckgsgfniedyyihwvqqapgkglewmgridpendetkygpifqg rvtitadtstntvymelsslrsedtavyycafrggvywgqgttvtvss | 72 |
| EGFR vIII | huscFv3 | Eiqlvqsgaevkkpgeslrisckgsgfniedyyihwvrqmpgkglewmgridpendetkygpif qghvtisadtsintvylqwsslkasdtamyycafrggvywgqgttvtvssggggsggggsggggs ggggsdvvmtqsplslpvtlgqpasisckssqslldsdgktylnwlqqrpgqsprrlislvskldsgv pdrfsgsgsgtdftlkisrveaedvgvyycwqgthfpgtfgggtkveik | 73 |
| EGFR vIII | huscFv4 | Dvvmtqsplslpvtlgqpasisckssqslldsdgktylnwlqqrpgqsprrlislvskldsgvpdrfsg sgsgtdftlkisrveaedvgvyycwqgthfpgtfgggtkveikggggsggggsggggsggggseiq lvqsgaevkkpgeslrisckgsgfniedyyihwvrqmpgkglewmgridpendetkygpifqgh vtisadtsintvylqwsslkasdtamyycafrggvywgqgttvtvss | 74 |
| EGFR vIII | huscFv5 | Eiqlvqsgaevkkpgatvkisckgsgfniedyyihwvqqapgkglewmgridpendetkygpif qgrvtitadtstntvymelsslrsedtavyycafrggvywgqgttvtvssggggsggggsggggsgg ggsdvvmtqsplslpvtlgqpasisckssqslldsdgkty lnwlqqrpgqsprrlislvskldsgvpdr fsgsgsgtdftlkisrveaedvgvyycwqgthfpgtfgggtkveik | 75 |
| EGFR vIII | huscFv6 | Eiqlvqsgaevkkpgeslrisckgsgfniedyyihwvrqmpgkglewmgridpendetkygpif qghvtisadtsintvylqwsslkasdtamyycafrggvywgqgttvtvssggggsggggsggggs ggggsdvvmtqspdslavslgeratinckssqsllldsdgktylnwlqqkpgqppkrlislvskldsg vpdrfsgsgsgtdftltisslqaedvavyycwqgthfpgtfgggtkveik | 76 |
| EGFR vIII | huscFv7 | Dvvmtqspdslavslgeratinckssqslldsdgktylnwlqqkpgqppkrlislvskldsgvpdrfs gsgsgtdftltisslqaedvavyycwqgthfpgtfgggtkveikggggsggggsggggsggggsei qlvqsgaevkkpgeslrisckgsgfniedyyihwvrqmpgkglewmgridpendetkygpifqg hvtisadtsintvylqwsslkasdtamyycafrggvywgqgttvtvss | 77 |
| EGFR vIII | huscFv8 | Dvvmtqsplslpvtlgqpasisckssqslldsdgktylnwlqqrpgqsprrlislvskldsgvpdrfsg sgsgtdftlkisrveaedvgvyycwqgthfpgtfgggtkveikggggsggggsggggsggggseiq lvqsgaevkkpgatvkisckgsgfniedyyihwvqqapgkglewmgridpendetkygpifqgr vtitadtstntvymelsslrsedtavyycafrggvywgqgttvtvss | 78 |
| EGFR vIII | Mu310C | eiqlqqsgaelvkpgasvklsctgsgfniedyyihwvkqrteqglewigridpendetkygpifqgr atitadtssntvylqlssltsedtavyycafrggvywgpgttltvssggggsggggsggggshmdvv mtqspltlsvaigqsasisckssqslldsdgktylnwllqrpgqspkrlislvskldsgvpdrftgsgsgt dftlrisrveaedlgiyycwqgthfpgtfgggtkleik | 79 |
| Claudin6 | muMAB 64A | EVQLQQSGPELVKPGASMKISCKASGYSFTGYTMNWVKQSHGK NLEWIGLINPYNGGTIYNQKFKGKATLTVDKSSSTAYMELLSLTS EDSAVYYCARDYGFVLDYWGQGTTLTVSSGGGGSGGGGSGGGG SGGGGSQIVLTQSPSIMSVSPGEKVTITCSASSSVSYMHWFQQKPG | 98 |
| | | TSPKLCIYSTSNLASGVPARFSGRGSGTSYSLTISRVAAEDAATYY CQQRSNYPPWTFGGGTKLEIK | |
| Claudin6 | mAb206 -LCC | EVQLQQSGPELVKPGASMKISCKASGYSFTGYTMNWVKQSHGK NLEWIGLINPYNGGTIYNQKFKGKATLTVDKSSSTAYMELLSLTS EDSAVYYCARDYGFVLDYWGQGTTLTVSSGGGGSGGGGSGGGG SGGGGSQIVLTQSPAIMSASPGEKVTITCSASSSVSYLHWFQQKPG TSPKLWVYSTSNLPSGVPARFGGSGSGTSYSLTISRMEAEDAATY YCQQRSIYPPWTFGGGTKLEIK | 99 |
| Claudin6 | mAb206 -SUBG | EVQLQQSGPELVKPGASMKISCKASGYSFTGYTMNWVKQSHGK NLEWIGLINPYNGGTIYNQKFKGKATLTVDKSSSTAYMELLSLTS EDSAVYYCARDYGFVLDYWGQGTTLTVSSGGGGSGGGGSGGGG SGGGGSQIVLTQSPSIMSVSPGEKVTITCSASSSVSYMHWFQQKPG TSPKLGIYSTSNLASGVPARFSGRGSGTSYSLTISRVAAEDAATYY CQQRSNYPPWTFGGGTKLEIK | 100 |

In one embodiment, the EGFRvIII binding domain comprises one or more (e.g., all three) light chain complementary determining region 1 (LC CDR1), light chain complementary determining region 2 (LC CDR2), and light chain complementary determining region 3 (LC CDR3) of an EGFRvIII binding domain described herein, e.g., provided in Table 5, and/or one or more (e.g., all three) heavy chain complementary determining region 1 (HC CDR1), heavy chain complementary determining region 2 (HC CDR2), and heavy chain complementary determining region 3 (HC CDR3) of an EGFRvIII binding domain described herein, e.g., provided in Table 5.

In one embodiment, the EGFRvIII binding domain comprises a light chain variable region described herein (e.g., in Table 5) and/or a heavy chain variable region described herein (e.g., in Table 5). In one embodiment, the EGFRvIII binding domain is a scFv comprising a light chain and a heavy chain of an amino acid sequence listed in Table 5. In an embodiment, the EGFRvIII binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 5, or a sequence with 95-99% identity with an amino acid sequence provided in Table 5; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 5, or a sequence with 95-99% identity to an amino acid sequence provided in Table 5.

In one embodiment, the EGFRvIII binding domain comprises an amino acid sequence selected from a group consisting of SEQ ID NO: 71; SEQ ID NO: 72; SEQ ID NO: 73; SEQ ID NO: 74; SEQ ID NO: 75; SEQ ID NO: 76; SEQ ID NO: 77; SEQ ID NO: 78; and SEQ ID NO: 79; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the EGFRvIII binding domain is a scFv, and a light chain variable region comprising an amino acid sequence described herein, e.g., in Table 5, is attached to a heavy chain variable region comprising an amino acid sequence described herein, e.g., in Table 5, via a linker, e.g., a linker described herein. In one embodiment, the EGFRvIII binding domain includes a (Gly4-Ser)n linker, wherein n is 1, 2, 3, 4, 5, or 6, preferably 4 (SEQ ID NO: 80). The light chain variable region and heavy chain variable region of a scFv can be, e.g., in any of the following orientations: light chain variable region-linker-heavy chain variable region or heavy chain variable region-linker-light chain variable region.

In one embodiment, the claudin-6 binding domain comprises one or more (e.g., all three) light chain complementary determining region 1 (LC CDR1), light chain complementary determining region 2 (LC CDR2), and light chain complementary determining region 3 (LC CDR3) of an EGFRvIII binding domain described herein, e.g., provided in Table 5, and/or one or more (e.g., all three) heavy chain complementary determining region 1 (HC CDR1), heavy chain complementary determining region 2 (HC CDR2), and heavy chain complementary determining region 3 (HC CDR3) of an claudin-6 binding domain described herein, e.g., provided in Table 5.

In one embodiment, the claudin-6 binding domain comprises a light chain variable region described herein (e.g., in Table 5) and/or a heavy chain variable region described herein (e.g., in Table 5). In one embodiment, the claudin-6 binding domain is a scFv comprising a light chain and a heavy chain of an amino acid sequence listed in Table 5. In an embodiment, the claudin-6 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in **Table** 5, or a sequence with 95-99% identity with an amino acid sequence provided in Table 5; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 5, or a sequence with 95-99% identity to an amino acid sequence provided in Table 5.

In one embodiment, the claudin-6 binding domain comprises an amino acid sequence selected from a group consisting of SEQ ID NO: 98; SEQ ID NO: 99; and SEQ ID NO: 100; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the claudin-6 binding domain is a scFv, and a light chain variable region comprising an amino acid sequence described herein, e.g., in Table 5, is attached to a heavy chain variable region comprising an amino acid sequence described herein, e.g., in Table 5, via a linker, e.g., a linker described herein. In one embodiment, the claudin-6 binding domain includes a (Gly4-Ser)n linker, wherein n is 1, 2, 3, 4, 5, or 6, preferably 4 (SEQ ID NO: 80). The light chain variable region and heavy chain variable region of a scFv can be, e.g., in any of the following orientations: light chain variable region-linker-heavy chain variable region or heavy chain variable region-linker-light chain variable region.

In one embodiment, an antigen binding domain against GD2 is an antigen binding portion, e.g., CDRs, of an antibody described in, e.g., Mujoo et al., Cancer Res. 4-7(4):1098-1104 (1987); Cheung et al., Cancer Res 45(6):2642-2649 (1985), Cheung et al., J Clin Oncol 5(9):1430-1440 (1987), Cheung et al., J Clin Oncol 16(9):3053-3060 (1998), Handgretinger et al., Cancer Immunol Immunother 35(3):199-204 (1992). In some embodiments, an antigen binding domain against GD2 is an antigen binding portion of an antibody selected from mAb 14.18, 14G2a, ch14.18, hu14.18, 3F8, hu3F8, 3G6, 8B6, 60C3, 10B8, ME36.1, and 8H9, see e.g., WO2012033885, WO2013040371, WO2013192294, WO2013061273, WO2013123061, WO2013074916, and WO201385552. In some embodiments, an antigen binding domain against GD2 is an antigen binding portion of an antibody described in US Publication No.: 20100150910 or PCT Publication No.: WO 2011160119.

In one embodiment, an antigen binding domain against the Tn antigen, the sTn antigen, a Tn-O-glycopeptide antigen, or a sTn-O-glycopeptide antigen is an antigen binding portion, e.g., CDRs, of an antibody described in, e.g., US 2014/0178365, WO2015/120180, US8,440,798, EP 2083868 A2, Brooks et al., PNAS 107(22):10056-10061 (2010), and Stone et al., Oncolmmunology 1(6):863-873(2012).

In one embodiment, an antigen binding domain against PSMA is an antigen binding portion, e.g., CDRs, of an antibody described in, e.g., Parker et al., Protein Expr Purif 89(2):136-145 (2013), US 20110268656 (J591 ScFv); Frigerio et al, European J Cancer 49(9):2223-2232 (2013) (scFvD2B); WO 2006125481 (mAbs 3/A12, 3/E7 and 3/F11) and single chain antibody fragments (scFv A5 and D7).

In one embodiment, an antigen binding domain against CD97 is an antigen binding portion, e.g., CDRs, of an antibody described in, e.g., US6,846,911;de Groot et al., J Immunol 183(6):4127-4134 (2009); or an antibody from R&D: MAB3734.

In one embodiment, an antigen binding domain against TAG72 is an antigen binding portion, e.g., CDRs, of an antibody described in, e.g., Hombach et al., Gastroenterology 113(4):1163-1170 (1997); and Abcam ab691.

In one embodiment, an antigen binding domain against CD44v6 is an antigen binding portion, e.g., CDRs, of an antibody described in, e.g., Casucci et al., Blood 122(20):3461-3472 (2013).

In one embodiment, an antigen binding domain against CEA is an antigen binding portion, e.g., CDRs, of an antibody described in, e.g., Chmielewski et al., Gastoenterology 143(4): 1095-1107 (2012).

In one embodiment, an antigen binding domain against EPCAM is an antigen binding portion, e.g., CDRS, of an antibody selected from MT110, EpCAM-CD3 bispecific Ab (see, e.g., clinicaltrials.gov/ct2/show/NCT00635596); Edrecolomab; 3622W94; ING-1; and adecatumumab (MT201).

In one embodiment, an antigen binding domain against KIT is an antigen binding portion, e.g., CDRs, of an antibody described in, e.g., US7915391, US20120288506 , and several commercial catalog antibodies.

In one embodiment, an antigen binding domain against IL-13Ra2 is an antigen binding portion, e.g., CDRs, of an antibody described in, e.g., WO2008/146911, WO2004087758, several commercial catalog antibodies, and WO2004087758.

In one embodiment, an antigen binding domain against CD171 is an antigen binding portion, e.g., CDRs, of an antibody described in, e.g., Hong et al., J Immunother 37(2):93-104 (2014).

In one embodiment, an antigen binding domain against PSCA is an antigen binding portion, e.g., CDRs, of an antibody described in, e.g., Morgenroth et al., Prostate 67(10):1121-1131 (2007) (scFv 7F5); Nejatollahi et al., J of Oncology 2013(2013), article ID 839831 (scFv C5-II); and US Pat Publication No. 20090311181.

In one embodiment, an antigen binding domain against MAD-CT-2 is an antigen binding portion, e.g., CDRs, of an antibody described in, e.g., PMID: 2450952; US7635753.

In one embodiment, an antigen binding domain against Folate receptor alpha is an antigen binding portion, e.g., CDRs, of the antibody IMGN853, or an antibody described in US20120009181; US4851332, LK26: US5952484.

In one embodiment, an antigen binding domain against ERBB2 (Her2/neu) is an antigen binding portion, e.g., CDRs, of the antibody trastuzumab, or pertuzumab.

In one embodiment, an antigen binding domain against MUC1 is an antigen binding portion, e.g., CDRs, of the antibody SAR566658.

In one embodiment, the antigen binding domain against EGFR is antigen binding portion, e.g., CDRs, of the antibody cetuximab, panitumumab, zalutumumab, nimotuzumab, or matuzumab.

In one embodiment, an antigen binding domain against NCAM is an antigen binding portion, e.g., CDRs, of the antibody clone 2-2B: MAB5324 (EMD Millipore)

In one embodiment, an antigen binding domain against CAIX is an antigen binding portion, e.g., CDRs, of the antibody clone 303123 (R&D Systems).

In one embodiment, an antigen binding domain against Fos-related antigen 1 is an antigen binding portion, e.g., CDRs, of the antibody 12F9 (Novus Biologicals).

In one embodiment, an antigen binding domain against SSEA-4 is an antigen binding portion, e.g., CDRs, of antibody MC813 (Cell Signaling), or other commercially available antibodies.

In one embodiment, an antigen binding domain against PDGFR-beta is an antigen binding portion, e.g., CDRs, of an antibody Abcam ab32570.

In one embodiment, an antigen binding domain against ALK is an antigen binding portion, e.g., CDRs, of an antibody described in, e.g., Mino-Kenudson et al., Clin Cancer Res 16(5):1561-1571 (2010).

In one embodiment, an antigen binding domain against plysialic acid is an antigen binding portion, e.g., CDRs, of an antibody described in, e.g., Nagae et al., J Biol Chem 288(47):33784-33796 (2013).

In one embodiment, an antigen binding domain against PLAC1 is an antigen binding portion, e.g., CDRs, of an antibody described in, e.g., Ghods et al., Biotechnol Appl Biochem 2013 doi:10.1002/bab.1177.

In one embodiment, an antigen binding domain against GloboH is an antigen binding portion of the antibody VK9; or an antibody described in, e.g., Kudryashov V et al, Glycoconj J.15(3):243-9 ( 1998), Lou et al., Proc Natl Acad Sci USA 111(7):2482-2487 (2014) ; MBr1: Bremer E-G et al. J Biol Chem 259:14773-14777 (1984).

In one embodiment, an antigen binding domain against NY-BR-1 is an antigen binding portion, e.g., CDRs of an antibody described in, e.g., Jager et al., Appl Immunohistochem Mol Morphol 15(1):77-83 (2007).

In one embodiment, an antigen binding domain against sperm protein 17 is an antigen binding portion, e.g., CDRs, of an antibody described in, e.g., Song et al., Target Oncol 2013 Aug 14 (PMID: 23943313); Song et al., Med Oncol 29(4):2923-2931 (2012).

In one embodiment, an antigen binding domain against TRP-2 is an antigen binding portion, e.g., CDRs, of an antibody described in, e.g., Wang et al, J Exp Med. 184(6):2207-16 (1996).

In one embodiment, an antigen binding domain against CYP1B1 is an antigen binding portion, e.g., CDRs, of an antibody described in, e.g., Maecker et al, Blood 102 (9): 3287-3294 (2003).

In one embodiment, an antigen binding domain against RAGE-1 is an antigen binding portion, e.g., CDRs, of the antibody MAB5328 (EMD Millipore).

In one embodiment, an antigen binding domain against human telomerase reverse transcriptase is an antigen binding portion, e.g., CDRs, of the antibody cat no: LS-B95-100 (Lifespan Biosciences)

In one embodiment, an antigen binding domain against intestinal carboxyl esterase is an antigen binding portion, e.g., CDRs, of the antibody 4F12: cat no: LS-B6190-50 (Lifespan Biosciences).

In one embodiment, an antigen binding domain against mut hsp70-2 is an antigen binding portion, e.g., CDRs, of the antibody Lifespan Biosciences: monoclonal: cat no: LS-C133261-100 (Lifespan Biosciences).

In one embodiment, an antigen binding domain against MAD-CT-2 is an antigen binding portion, e.g., CDRs, of an antibody described in, e.g., PMID: 2450952; US7635753.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed above.

### Myeloid Tumor Antigens

As set out in the claims, the cell of the invention comprises a CAR having an antigen binding domain that binds to a tumor antigen other than a B-Cell antigen, wherein the tumor antigen is a solid tumor antigen.

The present disclosure also provides immune effector cells (e.g., T cells, NK cells) that are engineered to contain (in addition to one or more BCA CAR molecules) one or more CAR molecules that target a tumor antigen. In one aspect the tumor antigen is an antigen expressed on a myeloid tumor (either a surface antigen or as a comples with MHC), and the cells comprise a CAR that recognizes a myeloid tumor antigen.

In an instance, the myeloid tumor antigen is an antigen that is preferentially or specifically expressed on the surface of a myeloid tumor cell.

The present disclosure provides CARs that can target the following myeloid tumor antigens: CD123, CD34, Flt3, CD33 and CLL-1. In instances, the myeloid tumor antigen is selected from CD123, CD33 and CLL-1. In instances, the myeloid tumor antigen is CD123. In instances, the myeloid tumor antigen is CD33. In instances, the myeloid tumor antigen is CD34. In instances, the myeloid tumor antigen is Flt3. In instances, the myeloid tumor antigen is CLL-1. In instances, the antigen binding domain targets the human antigen.

In one instance, the antigen-binding domain of a TA CAR, e.g., the TA CAR expressed by a cell of the disclosure (e.g., a cell that also expresses a BCA CAR), can be chosen such that a myeloid tumor population is targeted. Alternatively, when targeting of more than one type of myeloid tumor is desired, an antigen binding domain that targets a myeloid tumor antigen that is expressed by more than one, e.g., all, of the myeloid tumors to be targeted can be selected.

In one aspect, the antigen-binding domain of a TA CAR, e.g., the TA CAR expressed by a cell of the disclosure, binds to CD123, e.g., human CD123. Any known CD123 binding domain may be used in the disclosure. In one instance, an antigen binding domain against CD123 is an antigen binding portion, e.g., CDRs or VH and VL, of an antibody, antigen-binding fragment or CAR described in, e.g., PCT publication WO2014/130635. In one instance, an antigen binding domain against CD123 is an antigen binding portion, e.g., CDRs or VH and VL, of an antibody, antigen-binding fragment or CAR described in, e.g., PCT publication WO/2016/028896. In one instance, an antigen binding domain against CD123 is an antigen binding portion, e.g., CDRs, of an antibody, antigen-binding fragment, or CAR described in, e.g., PCT publication WO1997/024373, WO2008/127735 (e.g., a CD123 binding domain of 26292, 32701, 37716 or 32703), WO2014/138805 (e.g., a CD123 binding domain of CSL362), WO2014/138819, WO2013/173820, WO2014/144622, WO2001/66139, WO2010/126066 (e.g., the CD123 binding domain of any of Old4, Old5, Old17, Old19, New102, or Old6), WO2014/144622, or US2009/0252742. In instances, the antigen binding domain is or is derived from a murine anti-human CD123 binding domain. In instances, the antigen binding domain is a humanized antibody or antibody fragment, e.g., scFv domain. In an instance, the antigen binding domain is a human antibody or antibody fragment that binds to human CD123. In instances, the antigen binding domain is an scFv domain which includes a light chain variable region (VL) and a heavy chain variable region (VH). The VL and VH may attached by a linker described herein, e.g., comprising the sequence GGGGSGGGGSGGGGS (SEQ ID NO: 30), and may be in any orientation, e.g., VL-linker-VH, or VH-linker-VL.

In one aspect, the antigen-binding domain of a TA CAR, e.g., the TA CAR expressed by a cell of the disclosure, binds to CD33, e.g., human CD33. Any known CD33 binding domain may be used . In one instance, an antigen binding domain against CD33 is an antigen binding portion, e.g., CDRs or VH and VL, of an antibody, antigen-binding fragment or CAR described in, e.g., PCT publication WO2016/014576.

In one instance, an antigen binding domain against CD33 is an antigen binding portion of or derived from Gemtuzumab ozogamicin (e.g., comprising an antigen binding domain comprising one or more, e.g., one, two, or three, CDRs of the heavy chain variable domain and/or one or more, e.g., one, two, or three, CDRs of the light chain variable domain, or the VH or VL, or the scFv sequence, of the scFv sequence of Gemtuzumab ozogamicin) (previously marketed as Mylotarg), e.g., Bross et al., Clin Cancer Res 7(6):1490-1496 (2001) (Gemtuzumab Ozogamicin, hP67.6). In one instance, an antigen binding domain against CD33 is an antigen binding portion of or derived from (e.g., comprising an antigen binding domain comprising one or more, e.g., one, two, or three, CDRs of the heavy chain variable domain and/or one or more, e.g., one, two, or three, CDRs of the light chain variable domain, or the VH or VL, or the scFv sequence) of the scFv sequence encoded by GenBank reference no. AM402974.1 (See, Wang et al., Mol. Ther., vol. 23:1, pp. 184-191 (2015). In one instance, an antigen binding domain against CD33 is an antigen binding portion, e.g., CDRs, of an antibody described in,, e.g., Caron et al., Cancer Res 52(24):6761-6767 (1992) (Lintuzumab, HuM195), Lapusan et al., Invest New Drugs 30(3):1121-1131 (2012) (AVE9633), Aigner et al., Leukemia 27(5): 1107-1115 (2013) (AMG330, CD33 BiTE), Dutour et al., Adv hematol 2012:683065 (2012), and Pizzitola et al., Leukemia doi:10.1038/Lue.2014.62 (2014). In instances, the antigen binding domain is or is derived from a murine anti-human CD33 binding domain. In instances, the antigen binding domain is a humanized antibody or antibody fragment, e.g., scFv domain. In an instance, the antigen binding domain is a human antibody or antibody fragment that binds to human CD33. In instances, the antigen binding domain is an scFv domain which includes a light chain variable region (VL) and a heavy chain variable region (VH). The VL and VH may attached by a linker described herein, e.g., comprising the sequence GGGGSGGGGSGGGGS (SEQ ID NO: 30), and may be in any orientation, e.g., VL-linker-VH, or VH-linker-VL.

In one aspect, the antigen-binding domain of a TA CAR, e.g., the TA CAR expressed by a cell of the disclosure, binds to CLL-1, e.g., human CLL-1. Any known CLL-1 binding domain may be used . In one instance, an antigen binding domain against CLL-1 is an antigen binding portion, e.g., CDRs or VH and VL, of an antibody, antigen-binding fragment or CAR described in, e.g., PCT publication WO2016/014535 .

In one instance, an antigen binding domain against CLL-1 is an antigen binding portion, e.g., CDRs, of an antibody available from R&D, ebiosciences, Abcam, for example, PE-CLL1-hu Cat# 353604 (BioLegend); and PE-CLL1 (CLEC12A) Cat# 562566 (BD). In instances, the antigen binding domain is or is derived from a murine anti-human CLL-1 binding domain. In instances, the antigen binding domain is a humanized antibody or antibody fragment, e.g., scFv domain. In an instance, the antigen binding domain is a human antibody or antibody fragment that binds to human CLL-1. In instances, the antigen binding domain is an scFv domain which includes a light chain variable region (VL) and a heavy chain variable region (VH). The VL and VH may attached by a linker described herein, e.g., comprising the sequence GGGGSGGGGSGGGGS (SEQ ID NO: 30), and may be in any orientation, e.g., VL-linker-VH, or VH-linker-VL.

### B Cell Antigens

The present disclosure provides immune effector cells (e.g., T cells, NK cells) that are engineered to contain (in addition to one or more TA CAR molecules) one or more CAR molecules that target a B-Cell antigen. This is achieved through an antigen binding domain on the CAR that is specific for a B cell antigen. Such a CAR may be referred to herein as a BCA CAR. The cells of the invention comprise a first CAR, wherein the antigen binding domain of said first CAR binds to a B-Cell antigen.

In an embodiment, the B cell antigen is an antigen that is preferentially or specifically expressed on the surface of the B cell. The antigen can be expressed on the surface of any one of the following types of B cells: progenitor B cells (e.g., pre-B cells or pro-B cells), early pro-B cells, late pro-B cells, large pre-B cells, small pre-B cells, immature B cells, e.g., naive B cells, mature B cells, plama B cells, plasmablasts, memory B cells, B-1 cells, B-2 cells, marginal-zone B cells, follicular B cells, germinal center B cells, or regulatory B cells (Bregs).

The present disclosure provides CARs that can target the following B cell antigens: CD10, CD19, CD20, CD21, CD22, CD23, CD24, CD25, CD37, CD38, CD53, CD72, CD73, CD74, CD75, CD77, CD79a, CD79b, CD80, CD81, CD82, CD83, CD84, CD85, ROR1, BCMA, CD86, and CD179b. Other B cell antigens that can be targeted by a CAR described herein include: CD1a, CD1b, CD1c, CD1d, CD2, CD5, CD6, CD9, CD11a, CD11b, CD11c, CD17, CD18, CD26, CD27, CD29, CD30, CD31, CD32a, CD32b, CD35, CD38, CD39, CD40, CD44, CD45, CD45RA, CD45RB, CD45RC, CD45RO, CD46, CD47, CD48, CD49b, CD49c, CD49d, CD50, CD52, CD54, CD55, CD58, CD60a, CD62L, CD63, CD63, CD68 CD69, CD70, CD85E, CD85I, CD85J, CD92, CD95, CD97, CD98, CD99, CD100, CD102, CD108, CD119, CD120a, CD120b, CD121b, CD122, CD124, CD125, CD126, CD130, CD132, CD137, CD138, CD139, CD147, CD148, CD150, CD152, CD162, CD164, CD166, CD167a, CD170, CD175, CD175s, CD180, CD184, CD185, CD192, CD196, CD197, CD200, CD205, CD210a, CDw210b, CD212, CD213a1, CD213a2, CD215, CD217, CD218a, CD218b, CD220, CD221, CD224, CD225, CD226, CD227, CD229, CD230, CD232, CD252, CD253, CD257, CD258, CD261, CD262, CD263, CD264, CD267, CD268, CD269, CD270, CD272, CD274, CD275, CD277, CD279, CD283, CD289, CD290, CD295, CD298, CD300a, CD300c, CD305, CD306, CD307a, CD307b, CD307c, CD307d, CD307e, CD314, CD315, CD316, CD317, CD319, CD321, CD327, CD328, CD329, CD338, CD351, CD352, CD353, CD354, CD355, CD357, CD358, CD360, CD361, CD362, and CD363.

In another embodiment, the B cell antigen targeted by the BCA CAR is chosen from CD19, BCMA, CD20, CD22, FcRn5, FcRn2, CS-1 and CD138. In an embodiment, the B-Cell antigen targeted by the BCA CAR is CD19. In an embodiment, the B-Cell antigen targeted by the BCA CAR is CD20. In an embodiment, the B-Cell antigen targeted by the BCA CAR is CD22. In an embodiment, the B-Cell antigen targeted by the BCA CAR is BCMA. In an embodiment, the B-Cell antigen targeted by the BCA CAR is FcRn5. In an embodiment, the B-Cell antigen targeted by the BCA CAR is FcRn2. In an embodiment, the B-Cell antigen targeted by the BCA CAR is CS-1. In an embodiment, the B-Cell antigen targeted by the BCA CAR is CD138.

In one embodiment, the antigen-binding domain of a BCA CAR, e.g., the BCA CAR expressed by a cell of the invention (e.g., a cell that also expresses a TA CAR), can be chosen such that a preferred B cell population is targeted. For example, in an embodiment where targeting of B regulatory cells is desired, an antigen binding domain is selected that targets a B cell antigen that is expressed on regulatory B cells and not on other B cell populations, e.g., plasma B cells and memory B cells. Cell surface markers expressed on regulatory B cells include: CD19, CD24, CD25, CD38, or CD86, or markers described in He et al., 2014, J Immunology Research, Article ID 215471. When targeting of more than one type of B cells is desired, an antigen binding domain that targets a B cell antigen that is expressed by all of the B cells to be targeted can be selected.

In an embodiment, the antigen-binding domain of a BCA CAR, e.g., the BCA CAR expressed by a cell of the invention, binds to CD19. CD19 is found on B cells throughout differentiation of the lineage from the pro/pre-B cell stage through the terminally differentiated plasma cell stage. In an embodiment, the antigen binding domain is a murine scFv domain that binds to human CD19, e.g., CTL019 (e.g., SEQ ID NO: 95). In an embodiment, the antigen binding domain is a humanized antibody or antibody fragment, e.g., scFv domain, derived from the murine CTL019 scFv. In an embodiment, the antigen binding domain is a human antibody or antibody fragment that binds to human CD19. Exemplary scFv domains (and their sequences, e.g., CDRs, VL and VH sequences) that bind to CD19 are provided in Table 6. The scFv domain sequences provided in Table 6 include a light chain variable region (VL) and a heavy chain variable region (VH). The VL and VH are attached by a linker comprising the sequence GGGGSGGGGSGGGGS (SEQ ID NO: 30), e.g., in the following orientation: VL-linker-VH.

**Table 6. Antigen Binding domains that bind B cell antigen CD19**

| **B cell antige n** | **Name** | **Amino Acid Sequence** | **SEQ ID NO:** |
|---|---|---|---|
| CD19 | muCTL 019 | DIQMTQTTSSLSASLGDRVTISCRASQDISKYLNWYQQKPDGTVK LLIYHTSRLHSGVPSRFSGSGSGTDYSLTISNLEQEDIATYFCQQ GNTLPYTFGGGTKLEITGGGGSGGGGSGGGGSEVKLQESGPGLVA PSQSLSVTCTVSGVSLPDYGVSWIRQPPRKGLEWLGVIWGSETTY YNSALKSRLTIIKDNSKSQVFLKMNSLQTDDTAIYYCAKHYYYGG SYAMDYWGQGTSVTVSS | **95** |
| CD19 | huscFv1 | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIKGGGGSGGGGSGGGGSQVQLQESGPGLVK PSETLSLTCTVSGVSLPDYGVSWIRQPPGKGLEWIGVIWGSETTY YSSSLKSRVTISKDNSKNQVSLKLSSVTAADTAVYYCAKHYYYGG SYAMDYWGQGTLVTVSS | **83** |
| CD19 | huscFv2 | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIKGGGGSGGGGSGGGGSQVQLQESGPGLVK PSETLSLTCTVSGVSLPDYGVSWIRQPPGKGLEWIGVIWGSETTY YQSSLKSRVTISKDNSKNQVSLKLSSVTAADTAVYYCAKHYYYGG SYAMDYWGQGTLVTVSS | **84** |
| CD19 | huscFv3 | QVQLQESGPGLVKPSETLSLTCTVSGVSLPDYGVSWIRQPPGKGL EWIGVIWGSETTYYSSSLKSRVTISKDNSKNQVSLKLSSVTAADT AVYYCAKHYYYGGSYAMDYWGQGTLVTVSSGGGGSGGGGSGGGGS EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIK | **85** |
| CD19 | huscFv4 | QVQLQESGPGLVKPSETLSLTCTVSGVSLPDYGVSWIRQPPGKGL EWIGVIWGSETTYYQSSLKSRVTISKDNSKNQVSLKLSSVTAADT AVYYCAKHYYYGGSYAMDYWGQGTLVTVSSGGGGSGGGGSGGGGS EIVMTQSPATLSLSPGERATLSCRASQDISKYLNWYQQKPGQAPR | **86** |
| | | LLIYHTSRLHSGIPARFSGSGSGTDYTLTISSLQPEDFAVYFCQQ GNTLPYTFGQGTKLEIK | |
| CD19 | huscFv5 | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIKGGGGSGGGGSGGGGSGGGGSQVQLQESG PGLVKPSETLSLTCTVSGVSLPDYGVSWIRQPPGKGLEWIGVIWG SETTYYSSSLKSRVTISKDNSKNQVSLKLSSVTAADTAVYYCAKH YYYGGSYAMDYWGQGTLVTVSS | **87** |
| CD19 | huscFv6 | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIKGGGGSGGGGSGGGGSGGGGSQVQLQESG PGLVKPSETLSLTCTVSGVSLPDYGVSWIRQPPGKGLEWIGVIWG SETTYYQSSLKSRVTISKDNSKNQVSLKLSSVTAADTAVYYCAKH YYYGGSYAMDYWGQGTLVTVSS | **88** |
| CD19 | huscFv7 | QVQLQESGPGLVKPSETLSLTCTVSGVSLPDYGVSWIRQPPGKGL EWIGVIWGSETTYYSSSLKSRVTISKDNSKNQVSLKLSSVTAADT AVYYCAKHYYYGGSYAMDYWGQGTLVTVSSGGGGSGGGGSGGGGS GGGGSEIVMTQSPATLSLSPGERATLSCRASQDISKYLNWYQQKP GQAPRLLIYHTSRLHSGIPARFSGSGSGTDYTLTISSLQPEDFAV YFCQQGNTLPYTFGQGTKLEIK | **89** |
| CD19 | huscFv8 | QVQLQESGPGLVKPSETLSLTCTVSGVSLPDYGVSWIRQPPGKGL EWIGVIWGSETTYYQSSLKSRVTISKDNSKNQVSLKLSSVTAADT AVYYCAKHYYYGGSYAMDYWGQGTLVTVSSGGGGSGGGGSGGGGS GGGGSEIVMTQSPATLSLSPGERATLSCRASQDISKYLNWYQQKP GQAPRLLIYHTSRLHSGIPARFSGSGSGTDYTLTISSLQPEDFAV YFCQQGNTLPYTFGQGTKLEIK | **90** |
| CD19 | huscFv9 | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIKGGGGSGGGGSGGGGSGGGGSQVQLQESG PGLVKPSETLSLTCTVSGVSLPDYGVSWIRQPPGKGLEWIGVIWG SETTYYNSSLKSRVTISKDNSKNQVSLKLSSVTAADTAVYYCAKH YYYGGSYAMDYWGQGTLVTVSS | **91** |
| CD19 | Hu scFv10 | QVQLQESGPGLVKPSETLSLTCTVSGVSLPDYGVSWIRQPPGKGL EWIGVIWGSETTYYNSSLKSRVTISKDNSKNQVSLKLSSVTAADT AVYYCAKHYYYGGSYAMDYWGQGTLVTVSSGGGGSGGGGSGGGGS GGGGSEIVMTQSPATLSLSPGERATLSCRASQDISKYLNWYQQKP GQAPRLLIYHTSRLHSGIPARFSGSGSGTDYTLTISSLQPEDFAV YFCQQGNTLPYTFGQGTKLEIK | **92** |
| CD19 | Hu scFv11 | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIKGGGGSGGGGSGGGGSQVQLQESGPGLVK PSETLSLTCTVSGVSLPDYGVSWIRQPPGKGLEWIGVIWGSETTY YNSSLKSRVTISKDNSKNQVSLKLSSVTAADTAVYYCAKHYYYGG SYAMDYWGQGTLVTVSS | **93** |
| CD19 | Hu scFv12 | QVQLQESGPGLVKPSETLSLTCTVSGVSLPDYGVSWIRQPPGKGL EWIGVIWGSETTYYNSSLKSRVTISKDNSKNQVSLKLSSVTAADT AVYYCAKHYYYGGSYAMDYWGQGTLVTVSSGGGGSGGGGSGGGGS | **94** |
| | | EIVMTQSPATLSLSPGERATLSCRASQDISKYLNWYQQKPGQAPR LLIYHTSRLHSGIPARFSGSGSGTDYTLTISSLQPEDFAVYFCQQ GNTLPYTFGQGTKLEIK | |

The sequences of the CDR sequences of the scFv domains of the CD19 antigen binding domains provided in Table 6 are shown in Table 7 for the heavy chain variable domains and in Table 8 for the light chain variable domains. "ID" stands for the respective SEQ ID NO for each CDR.

**Table 7. Heavy Chain Variable Domain CDRs**

| Description | FW | HCDR1 | ID | HCDR2 | ID | HCDR3 | ID |
|---|---|---|---|---|---|---|---|
| murine_CART19 | | GVSLPDYGVS | 255 | VIWGSETTYYNSALKS | 256 | HYYYGGSYAMDY | 260 |
| humanized_CART19 a | VH4 | GVSLPDYGVS | | VIWGSETTYY***S***S***S***LKS | 257 | HYYYGGSYAMDY | 260 |
| humanized_CART19 b | VH4 | GVSLPDYGVS | 255 | VIWGSETTYY***Q***S***S***LKS | 258 | HYYYGGSYAMDY | 260 |
| humanized_CART19 c | VH4 | GVSLPDYGVS | 255 | VIWGSETTYYNS***S***LKS | 259 | | 260 |

**Table 8. Light Chain Variable Domain CDRs**

| Description | FW | LCDR1 | ID | LCDR2 | ID | LCDR3 | ID |
|---|---|---|---|---|---|---|---|
| murine_CART19 | | RASQDISKYLN | 261 | HTSRLHS | 262 | QQGNTLPYT | 263 |
| humanized_CART19 a | VK3 | RASQDISKYLN | 261 | HTSRLHS HTSRLHS | 262 | QQGNTLPYT | 263 |
| humanized_CART19 b | VK3 | RASQDISKYLN | 261 | HTSRLHS | 262 | QQGNTLPYT | 263 |
| humanized_CART19 c | VK3 | RASQDISKYLN | 261 | HTSRLHS | 262 | QQGNTLPYT | 263 |

In an embodiment, the antigen binding domain comprises an anti-CD19 antibody, or fragment thereof, e.g., an scFv. For example, the antigen binding domain comprises a variable heavy chain and a variable light chain listed in Table 9. The linker sequence joining the variable heavy and variable light chains can be any of the linker sequences described herein, or alternatively, can be GSTSGSGKPGSGEGSTKG (SEQ ID NO: 81). The light chain variable region and heavy chain variable region of a scFv can be, e.g., in any of the following orientations: light chain variable region-linker-heavy chain variable region or heavy chain variable region-linker-light chain variable region.

**Table 9. Additional Anti-CD19 antibody binding domains**

| **Ab Name** | **VH Sequence** | **VL Sequence** |
|---|---|---|
| S125-C1 | QVQLLESGAELVRPGSSVKISCKA SGYAFSSYWMNWVKQRPGQGLEWI GQIYPGDGDTNYNGKFKGQATLTA DKSSSTAYMQLSGLTSEDSAVYSC ARKTISSVVDFYFDYWGQGTTVT (SEQ ID NO: 96) | ELVLTQSPKFMSTSVGDRVSVTCKAS QNVGTNVAWYQQKPGQSPKPLIYSAT YRNSGVPDRFTGSGSGTDFTLTITNV QSKDLADYFYFCQYNRYPYTSGGGTK LEIKRRS (SEQ ID NO: 97) |

| | **ScFv Sequence** | |
|---|---|---|
| SJ25-C1 scFv | QVQLLESGAELVRPGSSVKISCKASGYAFSSYWMNWVKQRPGQGLEWIGQIY PGDGDTNYNGKFKGQATLTADKSSSTAYMQLSGLTSEDSAVYSCARKTISSV VDFYFDYWGQGTTVTGSTSGSGKPGSGEGSTKGELVLTQSPKFMSTSVGDRV SVTCKASQNVGTNVAWYQQKPGQSPKPLIYSATYRNSGVPDRFTGSGSGTDF TLTITNVQSKDLADYFYFCQYNRYPYTSGGGTKLEIKRRS (SEQ ID NO: 112) | |

In one embodiment, the CD19 binding domain comprises one or more (e.g., all three) light chain complementary determining region 1 (LC CDR1), light chain complementary determining region 2 (LC CDR2), and light chain complementary determining region 3 (LC CDR3) of a CD19 binding domain described herein, e.g., provided in Table 6 or 7, and/or one or more (e.g., all three) heavy chain complementary determining region 1 (HC CDR1), heavy chain complementary determining region 2 (HC CDR2), and heavy chain complementary determining region 3 (HC CDR3) of a CD19 binding domain described herein, e.g., provided in Table 6 or 8. In one embodiment, the mesothelin binding domain comprises one, two, or all of LC CDR1, LC CDR2, and LC CDR3 of any amino acid sequences as provided in Table 8 ; and one, two or all of HC CDR1, HC CDR2, and HC CDR3 of any amino acid sequences as provided in Table 7.

In one embodiment, the CD19 antigen binding domain comprises:
(i)
   (a) a LLC CDR1 amino acid sequence of SEQ ID NO: 261, a LC CDR2 amino acid sequence of SEQ ID NO: 262, and a LC CDR3 amino acid sequence of SEQ ID NO: 263; and
   (b) a HC CDR1 amino acid sequence of SEQ ID NO: 255, a HC CDR2 amino acid sequence of SEQ ID NO: 256, and a HC CDR3 amino acid sequence of SEQ ID NO: 260
(ii)
   (a) a LC CDR1 amino acid sequence of SEQ ID NO: 261, a LC CDR2 amino acid sequence of SEQ ID NO: 262, and a LC CDR3 amino acid sequence of SEQ ID NO: 263; and
   (b) a HC CDR1 amino acid sequence of SEQ ID NO: 255, a HC CDR2 amino acid sequence of SEQ ID NO: 257, and a HC CDR3 amino acid sequence of SEQ ID NO: 260;
(iii)
   (a) a LC CDR1 amino acid sequence of SEQ ID NO: 261, a LC CDR2 amino acid sequence of SEQ ID NO: 262, and a LLC CDR3 amino acid sequence of SEQ ID NO: 263; and
   (b) a HC CDR1 amino acid sequence of SEQ ID NO: 255, a HC CDR2 amino acid sequence of SEQ ID NO: 258, and a HC CDR3 amino acid sequence of SEQ ID NO: 260; or
(iv)
   (a) a LC CDR1 amino acid sequence of SEQ ID NO: 261, a LC CDR2 amino acid sequence of SEQ ID NO: 262, and a LC CDR3 amino acid sequence of SEQ ID NO: 263; and
   (b) a HC CDR1 amino acid sequence of SEQ ID NO: 255, a HC CDR2 amino acid sequence of SEQ ID NO: 259, and a HC CDR3 amino acid sequence of SEQ ID NO: 260.

In one embodiment, the CD19 binding domain comprises a light chain variable region described herein (e.g., in Table 6 or 9) and/or a heavy chain variable region described herein (e.g., in Table 6 or 9). In one embodiment, the mesothelin binding domain is a scFv comprising a light chain and a heavy chain of an amino acid sequence listed in Table 3 or 4. In an embodiment, the CD19 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 6 or 9, or a sequence with 95-99% identity with an amino acid sequence provided in Table 6 or 9; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 6 or 9, or a sequence with 95-99% identity to an amino acid sequence provided in Table 6 or 9.

In one embodiment, the CD19 binding domain comprises an amino acid sequence selected from a group consisting of SEQ ID NO: 83; SEQ ID NO: 84, SEQ ID NO: 85; SEQ ID NO: 86; SEQ ID NO: 87; SEQ ID NO: 88; SEQ ID NO: 89, SEQ ID NO: 90, SEQ ID NO: 91, SEQ ID NO: 92, SEQ ID NO: 93, SEQ ID NO: 94, SEQ ID NO: 95, and SEQ ID NO: 112; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the CD19 binding domain is a scFv, and a light chain variable region comprising an amino acid sequence described herein, e.g., in Table 6 or 9, is attached to a heavy chain variable region comprising an amino acid sequence described herein, e.g., in Table 6 or 9, via a linker, e.g., a linker described herein. In one embodiment, the CD19 binding domain includes a (Gly4-Ser)n linker, wherein n is 1, 2, 3, 4, 5, or 6, preferably 4 (SEQ ID NO: 80). The light chain variable region and heavy chain variable region of a scFv can be, e.g., in any of the following orientations: light chain variable region-linker-heavy chain variable region or heavy chain variable region-linker-light chain variable region.

Any known CD19 CAR, e.g., the CD19 antigen binding domain of any known CD19 CAR, in the art can be used in accordance with the instant invention to construct a CAR. For example, LG-740; CD19 CAR described in the US Pat. No. 8,399,645; US Pat. No. 7,446,190; Xu et al., Leuk Lymphoma. 2013 54(2):255-260(2012); Cruz et al., Blood 122(17):2965-2973 (2013); Brentjens et al., Blood, 118(18):4817-4828 (2011); Kochenderfer et al., Blood 116(20):4099-102 (2010); Kochenderfer et al., Blood 122 (25):4129-39(2013); and 16th Annu Meet Am Soc Gen Cell Ther (ASGCT) (May 15-18, Salt Lake City) 2013, Abst 10. In one embodiment, an antigen binding domain against CD19 is an antigen binding portion, e.g., CDRs, of a CAR, antibody or antigen-binding fragment thereof described in, e.g., PCT publication WO2012/079000; PCT publication \\702014/153270; Kochenderfer, J.N. et al., J. Immunother. 32 (7), 689-702 (2009); Kochenderfer, J.N., et al., Blood, 116 (20), 4099-4102 (2010); PCT publication WO2014/031687; Bejcek, Cancer Research, 55, 2346-2351, 1995; or U.S. Patent No. 7,446,190.

In an embodiment, the antigen-binding domain of a BCA CAR, e.g., the BCA CAR expressed by a cell of the invention, binds to BCMA. BCMA is found preferentially expressed in mature B lymphocytes. In an embodiment, the antigen binding domain is a murine scFv domain that binds to human BCMA. In an embodiment, the antigen binding domain is a humanized antibody or antibody fragment, e.g., scFv domain, that binds human BCMA. In an embodiment, the antigen binding domain is a human antibody or antibody fragment that binds to human BCMA. Exemplary scFv domains (and their sequences, e.g., CDRs, VL and VH sequences) that bind to BCMA are provided in Table 12, Table 13, Table 14 and Table 15. The scFv domain sequences provided in Table 12 and Table 13 include a light chain variable region (VL) and a heavy chain variable region (VH). The VL and VH are attached by a linker, e.g., in the following orientation: VH-linker-VL.

**Table 12. Antigen Binding domains that bind the B-Cell antigen BCMA**

| The amino acid sequences variable heavy chain and variable light chain sequences for each scFv is also provided. | | |
|---|---|---|
| **Name/ Description** | **SEQ ID NO:** | **Sequence** |
| **139109** | | |
| **139109- aa ScFv domain** | 349 | EVQLVESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSDIQ LTQSPSSLSASVGDRVTITCRASQSISSYLNWYQQKPGKAPKLLI YAASSLQSGVPSRFSGSGSGTDFTLTISSLQPEDFATYYCQQSYS TPYTFGQGTKVEIK |
| **139109- nt ScFv domain** | 364 | GAAGTGCAATTGGTGGAATCAGGGGGAGGACTTGTGCAGCCTGGA GGATCGCTGAGACTGTCATGTGCCGTGTCCGGCTTTGCCCTGTCC AACCACGGATGTCCTGGGTCCGCCGCGCCTGGAAAGGGCCTC GAATGGGTGTCGGGTATTGTGTACAGCGGTAGCACCTACTATGCC GCATCCGTGAAGGGGAGATTCACCATCAGCCGGGACAACTCCAGG AACACTCTGTACCTCCAAATGAATTCGCTGAGGCCAGAGGACACT GCCATCTACTACTGCTCCGCGCATGGCGGAGAGTCCGACGTCTGG GGACAGGGGACCACCGTGACCGTGTCTAGCGCGTCCGGCGGAGGC GGCAGCGGGGGTCGGGCATCAGGGGGCGGCGGATCGGACATCCAG CTCACCCAGTCCCCGAGCTCGCTGTCCGCCTCCGTGGGAGATCGG GTCACCATCACGTGCCGCGCCAGCCAGTCGATTTCCTCCTACCTG AACTGGTACCAACAGAAGCCCGGAAAAGCCCCGAAGCTTCTCATC TACGCCGCCTCGAGCCTGCAGTCAGGAGTGCCCTCACGGTTCTCC GGCTCCGGTTCCGGTACTGATTTCACCCTGACCATTTCCTCCCTG CAACCGGAGGACTTCGCTACTTACTACTGCCAGCAGTCGTACTCC |
| | | ACCCCCTACACTTTCGGACAAGGCACCAAGGTCGAAATCAAG |
| **139109- aa VH** | 379 | EVQLVESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139109- aa VL** | 394 | DIQLTQSPSSLSASVGDRVTITCRASQSISSYLNWYQQKPGKAPK LLIYAASSLQSGVPSRFSGSGSGTDFTLTISSLQPEDFATYYCQQ SYSTPYTFGQGTKVEIK |

| **139103** | | |
|---|---|---|
| **139103- aa ScFv domain** | 339 | QVQLVESGGGLVQPGRSLRLSCAASGFTFSNYAMSWVRQAPGKGL GWVSGISRSGENTYYADSVKGRFTISRDNSKNTLYLQMNSLRDED TAVYYCARSPAHYYGGMDVWGQGTTVTVSSASGGGGSGGRASGGG GSDIVLTQSPGTLSLSPGERATLSCRASQSISSSFLAWYQQKPGQ APRLLIYGASRRATGIPDRFSGSGSGTDFTLTISRLEPEDSAVYY CQQYHSSPSWTFGQGTKLEIK |
| **139103- nt ScFv domain** | 354 | CAAGTGCAACTCGTGGAATCTGGTGGAGGACTCGTGCAACCCGGA AGATCGCTTAGACTGTCGTGTGCCGCCAGCGGGTTCACTTTCTCG AACTACGCGATGTCCTGGGTCCGCCAGGCACCCGGAAAGGGACTC GGTTGGGTGTCCGGCATTTCCCGGTCCGGCGAAAATACCTACTAC GCCGACTCCGTGAAGGGCCGCTTCACCATCTCAAGGGACAACAGC AAAAACACCCTGTACTTGCAAATGAACTCCCTGCGGGATGAAGAT ACAGCCGTGTACTATTGCGCCCGGTCGCCTGCCCATTACTACGGC GGAATGGACGTCTGGGGACAGGGAACCACTGTGACTGTCAGCAGC GCGTCGGGTGGCGGCGGCTCAGGGGGTCGGGCCTCCGGGGGGGGA GGGTCCGACATCGTGCTGACCCAGTCCCCGGGAACCCTGAGCCTG AGCCCGGGAGAGCGCGCGACCCTGTCATGCCGGGCATCCCAGAGC ATTAGCTCCTCCTTTCTCGCCTGGTATCAGCAGAAGCCCGGACAG GCCCCGAGGCTGCTGATCTACGGCGCTAGCAGAAGGGCTACCGGA ATCCCAGACCGGTTCTCCGGCTCCGGTTCCGGGACCGATTTCACC CTTACTATCTCGCGCCTGGAACCTGAGGACTCCGCCGTCTACTAC TGCCAGCAGTACCACTCATCCCCGTCGTGGACGTTCGGACAGGGC ACCAAGCTGGAGATTAAG |
| **139103- aa VH** | 369 | QVQLVESGGGLVQPGRSLRLSCAASGFTFSNYAMSWVRQAPGKGL GWVSGISRSGENTYYADSVKGRFTISRDNSKNTLYLQMNSLRDED TAVYYCARSPAHYYGGMDVWGQGTTVTVSS |
| **139103- aa VL** | 384 | DIVLTQSPGTLSLSPGERATLSCRASQSISSSFLAWYQQKPGQAP RLLIYGASRRATGIPDRFSGSGSGTDFTLTISRLEPEDSAVYYCQ QYHSSPSWTFGQGTKLEIK |

| **139105** | | |
|---|---|---|
| **139105- aa ScFv domain** | 340 | QVQLVESGGGLVQPGRSLRLSCAASGFTFDDYAMHWVRQAPGKGL EWVSGISWNSGSIGYADSVKGRFTISRDNAKNSLYLQMNSLRAED TALYYCSVHSFLAYWGQGTLVTVSSASGGGGSGGRASGGGGSDIV MTQTPLSLPVTPGEPASISCRSSQSLLHSNGYNYLDWYLQKPGQS PQLLIYLGSNRASGVPDRFSGSGSGTDFTLKISRVEAEDVGVYYC MQALQTPYTFGQGTKVEIK |
| **139105- nt ScFv domain** | 355 | CAAGTGCAACTCGTCGAATCCGGTGGAGGTCTGGTCCAACCTGGT AGAAGCCTGAGACTGTCGTGTGCGGCCAGCGGATTCACCTTTGAT GACTATGCTATGCACTGGGTGCGGCAGGCCCCAGGAAAGGGCCTG |
| | | GAATGGGTGTCGGGAATTAGCTGGAACTCCGGGTCCATTGGCTAC GCCGACTCCGTGAAGGGCCGCTTCACCATCTCCCGCGACAACGCA AAGAACTCCCTGTACTTGCAAATGAACTCGCTCAGGGCTGAGGAT ACCGCGCTGTACTACTGCTCCGTGCATTCCTTCCTGGCCTACTGG GGACAGGGAACTCTGGTCACCGTGTCGAGCGCCTCCGGCGGCGGG GGCTCGGGTGGACGGGCCTCGGGCGGAGGGGGGTCCGACATCGTG ATGACCCAGACCCCGCTGAGCTTGCCCGTGACTCCCGGAGAGCCT GCATCCATCTCCTGCCGGTCATCCCAGTCCCTTCTCCACTCCAAC GGATACAACTACCTCGACTGGTACCTCCAGAAGCCGGGACAGAGC CCTCAGCTTCTGATCTACCTGGGGTCAAATAGAGCCTCAGGAGTG CCGGATCGGTTCAGCGGATCTGGTTCGGGAACTGATTTCACTCTG AAGATTTCCCGCGTGGAAGCCGAGGACGTGGGCGTCTACTACTGT ATGCAGGCGCTGCAGACCCCCTATACCTTCGGCCAAGGACGAAA GTGGAGATCAAG |
| **139105- aa VH** | 370 | QVQLVESGGGLVQPGRSLRLSCAASGFTFDDYAMHWVRQAPGKGL EWVSGISWNSGSIGYADSVKGRFTISRDNAKNSLYLQMNSLRAED TALYYCSVHSFLAYWGQGTLVTVSS |
| **139105- aa VL** | 385 | DIVMTQTPLSLPVTPGEPASISCRSSQSLLHSNGYNYLDWYLQKP GQSPQLLIYLGSNRASGVPDRFSGSGSGTDFTLKISRVEAEDVGV YYCMQALQTPYTFGQGTKVEIK |

| **139111** | | |
|---|---|---|
| **139111- aa ScFv domain** | 341 | EVQLLESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSDIV MTQTPLSLSVTPGQPASISCKSSQSLLRNDGKTPLYWYLQKAGQP PQLLIYEVSNRFSGVPDRFSGSGSGTDFTLKISRVEAEDVGAYYC MQNIQFPSFGGGTKLEIK |
| **139111- nt ScFv domain** | 356 | GAAGTGCAATTGTTGGAATCTGGAGGAGGACTTGTGCAGCCTGGA GGATCACTGAGACTTTCGTGTGCGGTGTCAGGCTTCGCCCTGAGC AACCACGGCATGAGCTGGGTGCGGAGAGCCCCGGGAAGGGTCTG GAATGGGTGTCCGGGATCGTCTACTCCGGTTCAACTTACTACGCC GCAAGCGTGAAGGGTCGCTTCACCATTTCCCGCGATAACTCCCGG AACACCCTGTACCTCCAAATGAACTCCCTGCGGCCCGAGGACACC GCCATCTACTACTGTTCCGCGCATGGAGGAGAGTCCGATGTCTGG GGACAGGGCACTACCGTGACCGTGTCGAGCGCCTCGGGGGAGGA GGCTCCGGCGGTCGCGCCTCCGGGGGGGTGGCAGCGACATTGTG ATGACGCAGACTCCACTCTCGCTGTCCGTGACCCCGGGACAGCCC GCGTCCATCTCGTGCAAGAGCTCCCAGAGCCTGCTGAGGAACGAC GGAAAGACTCCTCTGTATTGGTACCTCCAGAAGGCTGGACAGCCC CCGCAACTGCTCATCTACGAAGTGTCAAATCGCTTCTCCGGGGTG CCGGATCGGTTTTCCGGCTCGGGATCGGGCACCGACTTCACCCTG AAAATCTCCAGGGTCGAGGCCGAGGACGTGGGAGCCTACTACTGC ATGCAAAACATCCAGTTCCCTTCCTTCGGCGGCGGCACAAAGCTG GAGATTAAG |
| **139111- aa VH** | 371 | EVQLLESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139111- aa VL** | 386 | DIVMTQTPLSLSVTPGQPASISCKSSQSLLRNDGKTPLYWYLQKA GQPPQLLIYEVSNRFSGVPDRFSGSGSGTDFTLKISRVEAEDVGA YYCMQNIQFPSFGGGTKLEIK |

| **139100** | | |
|---|---|---|
| **139100- aa ScFv domain** | 342 | QVQLVQSGAEVRKTGASVKVSCKASGYIFDNFGINWVRQAPGQGL EWMGWINPKNNNTNYAQKFQGRVTITADESTNTAYMEVSSLRSED TAVYYCARGPYYYQSYMDVWGQGTMVTVSSASGGGGSGGRASGGG GSDIVMTQTPLSLPVTPGEPASISCRSSQSLLHSNGYNYLNWYLQ KPGQSPQLLIYLGSKRASGVPDRFSGSGSGTDFTLHITRVGAEDV GVYYCMQALQTPYTFGQGTKLEIK |
| **139100- nt ScFv domain** | 357 | CAAGTCCAACTCGTCCAGTCCGGCGCAGAAGTCAGAAAAACCGGT GCTAGCGTGAAAGTGTCCTGCAAGGCCTCCGGCTACATTTTCGAT AACTTCGGAATCAACTGGGTCAGACAGGCCCCGGGCCAGGGGCTG GAATGGATGGGATGGATCAACCCCAAGAACAACAACACCAACTAC GCACAGAAGTTCCAGGGCCGCGTGACTATCACCGCCGATGAATCG ACCAATACCGCCTACATGGAGGTGTCCTCCCTGCGGTCGGAGGAC ACTGCCGTGTATTACTGCGCGAGGGGCCCATACTACTACCAAAGC TACATGGACGTCTGGGGACAGGGAACCATGGTGACCGTGTCATCC GCCTCCGGTGGTGGAGGCTCCGGGGGCGGGCTTCAGGAGGCGGA GGAAGCGATATTGTGATGACCCAGACTCCGCTTAGCCTGCCCGTG ACTCCTGGAGAACCGGCCTCCATTTCCTGCCGGTCCTCGCAATCA CTCCTGCATTCCAACGGTTACAACTACCTGAATTGGTACCTCCAG AAGCCTGGCCAGTCGCCCCAGTTGCTGATCTATCTGGGCTCGAAG CGCGCCTCCGGGGTGCCTGACCGGTTTAGCGGATCTGGGAGCGGC ACGGACTTCACTCTCCACATCACCCGCGTGGGAGCGGAGGACGTG GGAGTGTACTACTGTATGCAGGCGCTGCAGACTCCGTACACATTC GGACAGGGCACCAAGCTGGAGATCAAG |
| **139100- aa VH** | 372 | QVQLVQSGAEVRKTGASVKVSCKASGYIFDNFGINWVRQAPGQGL EWMGWINPKNNNTNYAQKFQGRVTITADESTNTAYMEVSSLRSED TAVYYCARGPYYYQSYMDVWGQGTMVTVSS |
| **139100- aa VL** | 387 | DIVMTQTPLSLPVTPGEPASISCRSSQSLLHSNGYNYLNWYLQKP GQSPQLLIYLGSKRASGVPDRFSGSGSGTDFTLHITRVGAEDVGV YYCMQALQTPYTFGQGTKLEIK |

| **139101** | | |
|---|---|---|
| **139101- aa ScFv domain** | 343 | QVQLQESGGGLVQPGGSLRLSCAASGFTFSSDAMTWVRQAPGKGL EWVSVISGSGGTTYYADSVKGRFTISRDNSKNTLYLQMNSLRAED TAVYYCAKLDSSGYYYARGPRYWGQGTLVTVSSASGGGGSGGRAS GGGGSDIQLTQSPSSLSASVGDRVTITCRASQSISSYLNWYQQKP GKAPKLLIYGASTLASGVPARFSGSGSGTHFTLTINSLQSEDSAT YYCQQSYKRASFGQGTKVEIK |
| **139101- nt ScFv domain** | 358 | CAAGTGCAACTTCAAGAATCAGGCGGAGGACTCGTGCAGCCCGGA GGATCATTGCGGCTCTCGTGCGCCGCCTCGGGCTTCACCTTCTCG AGCGACGCCATGACCTGGGTCCGCCAGGCCCCGGGGAAGGGGCTG GAATGGGTGTCTGTGATTTCCGGCTCCGGGGGAACTACGTACTAC GCCGATTCCGTGAAAGGTCGCTTCACTATCTCCCGGGACAACAGC AAGAACACCCTTTATCTGCAAATGAATTCCCTCCGCGCCGAGGAC ACCGCCGTGTACTACTGCGCCAAGCTGGACTCCTCGGGCTACTAC |
| | | TATGCCCGGGTCCGAGATACTGGGACAGGAACCCTCGTGACC GTGTCCTCCGCGTCCGGCGGAGGAGGGTCGGGAGGCGGCCTCC GGCGGCGGCGGTTCGGACATCCAGCTGACCCAGTCCCCATCCTCA CTGAGCGCAAGCGTGGGCGACAGAGTCACCATTACATGCAGGGCG TCCCAGAGCATCAGCTCCTACCTGAACTGGTACCAACAGAAGCCT GGAAAGGCTCCTAAGCTGTTGATCTACGGGGCTTCGACCCTGGCA TCCGGGGTGCCCGCGAGGTTTAGCGGAAGCGGTAGCGGCACTCAC TTCACTCTGACCATTAACAGCCTCCAGTCCGAGGATTCAGCCACT TACTACTGTCAGCAGTCCTACAAGCGGGCCAGCTTCGGACAGGGC ACTAAGGTCGAGATCAAG |
| **139101- aa VH** | 373 | QVQLQESGGGLVQPGGSLRLSCAASGFTFSSDAMTWVRQAPGKGL EWVSVISGSGGTTYYADSVKGRFTISRDNSKNTLYLQMNSLRAED TAVYYCAKLDSSGYYYARGPRYWGQGTLVTVSS |
| **139101- aa VL** | 388 | DIQLTQSPSSLSASVGDRVTITCRASQSISSYLNWYQQKPGKAPK LLIYGASTLASGVPARFSGSGSGTHFTLTINSLQSEDSATYYCQQ SYKRASFGQGTKVEIK |

| **139102** | | |
|---|---|---|
| **139102- aa ScFv domain** | 344 | QVQLVQSGAEVKKPGASVKVSCKASGYTFSNYGITWVRQAPGQGL EWMGWISAYNGNTNYAQKFQGRVTMTRNTSISTAYMELSSLRSED TAVYYCARGPYYYYMDVWGKGTMVTVSSASGGGGSGGRASGGGGS EIVMTQSPLSLPVTPGEPASISCRSSQSLLYSNGYNYVDWYLQKP GQSPQLLIYLGSNRASGVPDRFSGSGSGTDFKLQISRVEAEDVGI YYCMQGRQFPYSFGQGTKVEIK |
| **139102- nt ScFv domain** | 359 | CAAGTCCAACTGGTCCAGAGCGGTGCAGAAGTGAAGAAGCCCGGA GCGAGCGTGAAAGTGTCCTGCAAGGCTTCCGGGTACACCTTCTCC AACTACGGCATCACTTGGGTGCGCCAGGCCCCGGGACAGGGCCTG GAATGGATGGGGTGGATTTCCGCGTACAACGGCAATACGAACTAC GCTCAGAAGTTCCAGGGTAGAGTGACCATGACTAGGAACACCTCC ATTTCCACCGCCTACATGGAACTGTCCTCCCTGCGGAGCGAGGAC ACCGCCGTGTACTATTGCGCCCGGGGACCATACTACTACTACATG GATGTCTGGGGGAAGGGGACTATGGTCACCGTGTCATCCGCCTCG GGAGGCGGCGGATCAGGAGGACGCGCCTCTGGTGGTGGAGGATCG GAGATCGTGATGACCCAGAGCCCTCTCTCCTTGCCCGTGACTCCT GGGGAGCCCGCATCCATTTCATGCCGGAGCTCCCAGTCACTTCTC TACTCCAACGGCTATAACTACGTGGATTGGTACCTCCAAAAGCCG GGCCAGAGCCCGCAGCTGCTGATCTACCTGGGCTCGAACAGGGCC AGCGGAGTGCCTGACCGGTTCTCCGGGTCGGAAGCGGGACCGAC TTCAAGCTGCAAATCTCGAGAGTGGAGGCCGAGGACGTGGGAATC TACTACTGTATGCAGGGCCGCCAGTTTCCGTACTCGTTCGGACAG GGCACCAAAGTGGAAATCAAG |
| **139102- aa VH** | 3 74 | QVQLVQSGAEVKKPGASVKVSCKASGYTFSNYGITWVRQAPGQGL EWMGWISAYNGNTNYAQKFQGRVTMTRNTSISTAYMELSSLRSED TAVYYCARGPYYYYMDVWGKGTMVTVSS |
| **139102- aa VL** | 389 | EIVMTQSPLSLPVTPGEPASISCRSSQSLLYSNGYNYVDWYLQKP GQSPQLLIYLGSNRASGVPDRFSGSGSGTDFKLQISRVEAEDVGI YYCMQGRQFPYSFGQGTKVEIK |

| **139104** | | |
|---|---|---|
| **139104- aa ScFv domain** | 345 | EVQLLETGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSEIV LTQSPATLSVSPGESATLSCRASQSVSSNLAWYQQKPGQAPRLLI YGASTRASGIPDRFSGSGSGTDFTLTISSLQAEDVAVYYCQQYGS SLTFGGGTKVEIK |
| **139104- nt ScFv domain** | 360 | GAAGTGCAATTGCTCGAAACTGGAGGAGGTCTGGTGCAACCTGGA GGATCACTTCGCCTGTCCTGCGCCGTGTCGGCTTTGCCCTGTCC AACCATGGAATGAGCTGGGTCCGCCGCGCGCCGGGGAAGGGCCTC GAATGGGTGTCCGGCATCGTCTACTCCGGCTCCACCTACTACGCC GCGTCCGTGAAGGGCCGGTTCACGATTTCACGGGACAACTCGCGG AACACCCTGTACCTCCAAATGAATTCCCTTCGGCCGGAGGATACT GCCATCTACTACTGCTCCGCCCACGGTGGCGAATCCGACGTCTGG GGCCAGGGAACCACCGTGACCGTGTCCAGCGCGTCCGGGGGAGGA GGAAGCGGGGGTAGAGCATCGGGTGGAGGCGGATCAGAGATCGTG CTGACCCAGTCCCCCGCCACCTTGAGCGTGTCACCAGGAGAGTCC GCCACCCTGTCATGCCGCGCCAGCCAGTCCGTGTCCTCCAACCTG GCTTGGTACCAGCAGAAGCCGGGGCAGGCCCCTAGACTCCTGATC TATGGGGCGTCGACCCGGGCATCTGGAATTCCCGATAGGTTCAGC GGATCGGGCTCGGGCACTGACTTCACTCTGACCATCTCCTCGCTG CAAGCCGAGGACGTGGCTGTGTACTACTGTCAGCAGTACGGAAGC TCCCTGACTTTCGGTGGCGGACCAAAGTCGAGATTAAG |
| **139104- aa VH** | 375 | EVQLLETGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139104- aa VL** | 390 | EIVLTQSPATLSVSPGESATLSCRASQSVSSNLAWYQQKPGQAPR LLIYGASTRASGIPDRFSGSGSGTDFTLTISSLQAEDVAVYYCQQ YGSSLTFGGGTKVEIK |

| **139106** | | |
|---|---|---|
| **139106- aa ScFv domain** | 346 | EVQLVETGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSEIV MTQSPATLSVSPGERATLSCRASQSVSSKLAWYQQKPGQAPRLLM YGASIRATGIPDRFSGSGSGTEFTLTISSLEPEDFAVYYCQQYGS SSWTFGQGTKVEIK |
| **139106- nt ScFv domain** | 361 | GAAGTGCAATTGGTGGAAACTGGAGGAGGACTTGTGCAACCTGGA GGATCATTGAGACTGAGCTGCGCAGTGTCGGGATTCGCCCTGAGC AACCATGGAATGTCCTGGGTCAGAAGGCCCCTGGAAAAGGCCTC GAATGGGTGTCAGGGATCGTGTACTCCGGTTCCACTTACTACGCC GCCTCCGTGAAGGGGCGCTTCACTATCTCACGGGATAACTCCCGC AATACCCTGTACCTCCAAATGAACAGCCTGCGGCCGGAGGATACC GCCATCTACTACTGTTCCGCCCACGGTGGAGAGTCTGACGTCTGG GGCCAGGGAACTACCGTGACCGTGTCCTCCGCGTCCGGCGGTGGA GGGAGCGGCGGCCGCGCCAGCGGCGGCGGAGGCTCCGAGATCGTG ATGACCCAGAGCCCCGCTACTCTGTCGGTGTCGCCCGGAGAAAGG GCGACCCTGTCCTGCCGGGCGTCGCAGTCCGTGAGCAGCAAGCTG GCTTGGTACCAGCAGAAGCCGGGCCAGGCACCACGCCTGCTTATG |
| | | TACGGTGCCTCCATTCGGGCCACCGGAATCCCGGACCGGTTCTCG GGGTCGGGGTCCGGTACCGAGTTCACACTGACCATTTCCTCGCTC GAGCCCGAGGACTTTGCCGTCTATTACTGCCAGCAGTACGGCTCC TCCTCATGGACGTTCGGCCAGGGGACCAAGGTCGAAATCAAG |
| **139106- aa VH** | 376 | EVQLVETGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139106- aa VL** | 391 | EIVMTQSPATLSVSPGERATLSCRASQSVSSKLAWYQQKPGQAPR LLMYGASIRATGIPDRFSGSGSGTEFTLTISSLEPEDFAVYYCQQ YGSSSWTFGQGTKVEIK |

| **139107** | | |
|---|---|---|
| **139107- aa ScFv domain** | 347 | EVQLVETGGGVVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSEIV LTQSPGTLSLSPGERATLSCRASQSVGSTNLAWYQQKPGQAPRLL IYDASNRATGIPDRFSGGGSGTDFTLTISRLEPEDFAVYYCQQYG SSPPWTFGQGTKVEIK |
| **139107- nt ScFv domain** | 362 | GAAGTGCAATTGGTGGAGACTGGAGGAGGAGTGGTGCAACCTGGA GGAAGCCTGAGACTGTCATGCGCGGTGTCGGCTTCGCCCTCTCC AACCACGGAATGTCCTGGGTCCGCCGGGCCCCTGGAAAGGACTT GAATGGGTGTCCGGCATCGTGTACTCGGGTTCCACCTACTACGCG GCCTCAGTGAAGGGCCGGTTTACTATTAGCCGCGACAACTCCAGA AACACACTGTACCTCCAAATGAACTCGCTGCGGCCGGAAGATACC GCTATCTACTACTGCTCCGCCCATGGGGGAGAGTCGGACGTCTGG GGACAGGGCACCACTGTCACTGTGTCCAGCGCTTCCGGCGGTGGT GGAAGCGGGGGACGGCCTCAGGAGGCGGTGGCAGCGAGATTGTG CTGACCCAGTCCCCCGGGACCCTGAGCCTGTCCCCGGGAGAAAGG GCCACCCTCTCCTGTCGGGCATCCCAGTCCGTGGGGTCTACTAAC CTTGCATGGTACCAGCAGAAGCCCGGCCAGGCCCCTCGCCTGCTG ATCTACGACGCGTCCAATAGAGCCACCGGCATCCCGGATCGCTTC AGCGGAGGCGGATCGGGCACCGACTTCACCCTCACCATTTCAAGG CTGGAACCGGAGGACTTCGCCGTGTACTACTGCCAGCAGTATGGT TCGTCCCCACCCTGGACGTTCGGCCAGGGGACTAAGGTCGAGATC AAG |
| **139107- aa VH** | 377 | EVQLVETGGGVVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139107- aa VL** | 392 | EIVLTQSPGTLSLSPGERATLSCRASQSVGSTNLAWYQQKPGQAP RLLIYDASNRATGIPDRFSGGGSGTDFTLTISRLEPEDFAVYYCQ QYGSSPPWTFGQGTKVEIK |

| **139108** | | |
|---|---|---|
| **139108- aa ScFv domain** | 348 | QVQLVESGGGLVKPGGSLRLSCAASGFTFSDYYMSWIRQAPGKGL EWVSYISSSGSTIYYADSVKGRFTISRDNAKNSLYLQMNSLRAED TAVYYCARESGDGMDVWGQGTTVTVSSASGGGGSGGRASGGGGSD IQMTQSPSSLSASVGDRVTITCRASQSISSYLNWYQQKPGKAPKL LIYAASSLQSGVPSRFSGSGSGTDFTLTISSLQPEDFATYYCQQS YTLAFGQGTKVDIK |
| **139108- nt ScFv domain** | 363 | CAAGTGCAACTCGTGGAATCTGGTGGAGGACTCGTGAAACCTGGA GGATCATTGAGACTGTCATGCGCGGCCTCGGATTCACGTTCTCC GATTACTACATGAGCTGGATTCGCCAGGCTCCGGGGAAGGACTG GAATGGGTGTCCTACATTTCCTCATCCGGCTCCACCATCTACTAC GCGGACTCCGTGAAGGGGAGATTCACCATTAGCCGCGATAACGCC AAGAACAGCCTGTACCTTCAGATGAACTCCCTGCGGGCTGAAGAT ACTGCCGTCTACTACTGCGCAAGGGAGAGCGGAGATGGGATGGAC GTCTGGGGACAGGGTACCACTGTGACCGTGTCGTCGGCCTCCGGC GGAGGGGGTTCGGGTGGAAGGGCCAGCGGCGGCGGAGGCAGCGAC ATCCAGATGACCCAGTCCCCCTCATCGCTGTCCGCCTCCGTGGGC GACCGCGTCACCATCACATGCCGGGCCTCACAGTCGATCTCCTCC TACCTCAATTGGTATCAGCAGAAGCCCGGAAAGGCCCCTAAGCTT CTGATCTACGCAGCGTCCTCCCTGCAATCCGGGGTCCCATCTCGG TTCTCCGGCTCGGGCAGCGGTACCGACTTCACTCTGACCATCTCG AGCCTGCAGCCGGAGGACTTCGCCACTTACTACTGTCAGCAAAGC TACACCCTCGCGTTTGGCCAGGGCACCAAAGTGGACATCAAG |
| **139108- aa VH** | 378 | QVQLVESGGGLVKPGGSLRLSCAASGFTFSDYYMSWIRQAPGKGL EWVSYISSSGSTIYYADSVKGRFTISRDNAKNSLYLQMNSLRAED TAVYYCARESGDGMDVWGQGTTVTVSS |
| **139108- aa VL** | 393 | DIQMTQSPSSLSASVGDRVTITCRASQSISSYLNWYQQKPGKAPK LLIYAASSLQSGVPSRFSGSGSGTDFTLTISSLQPEDFATYYCQQ SYTLAFGQGTKVDIK |

| **139110** | | |
|---|---|---|
| **139110- aa ScFv domain** | 350 | QVQLVQSGGGLVKPGGSLRLSCAASGFTFSDYYMSWIRQAPGKGL EWVSYISSSGNTIYYADSVKGRFTISRDNAKNSLYLQMNSLRAED TAVYYCARSTMVREDYWGQGTLVTVSSASGGGGSGGRASGGGGSD IVLTQSPLSLPVTLGQPASISCKSSESLVHNSGKTYLNWFHQRPG QSPRRLIYEVSNRDSGVPDRFTGSGSGTDFTLKISRVEAEDVGVY YCMQGTHWPGTFGQGTKLEIK |
| **139110- nt ScFv domain** | 365 | CAAGTGCAACTGGTGCAAAGCGGAGGAGGATTGGTCAAACCCGGA GGAAGCCTGAGACTGTCATGCGCGGCCTCTGGATTCACCTTCTCC GATTACTACATGTCATGGATCAGACAGGCCCCGGGAAGGGCCTC GAATGGGTGTCCTACATCTCGTCCTCCGGGAACACCATCTACTAC GCCGACAGCGTGAAGGGCCGCTTTACCATTTCCCGCGACAACGCA AAGAACTCGCTGTACCTTCAGATGAATTCCCTGCGGGCTGAAGAT ACCGCGGTGTACTATTGCGCCCGGTCCACTATGGTCCGGGAGGAC TACTGGGGACAGGGCACACTCGTGACCGTGTCCAGCGCGAGCGGG GGTGGAGGCAGCGGTGGACGCGCCTCCGGCGGCGGCGGTTCAGAC ATCGTGCTGACTCAGTCGCCCCTGTCGCTGCCGGTCACCCTGGGC CAACCGGCCTCAATTAGCTGCAAGTCCTCGGAGAGCCTGGTGCAC AACTCAGGAAAGACTTACCTGAACTGGTTCCATCAGCGGCCTGGA CAGTCCCCACGGAGGCTCATCTATGAAGTGTCCAACAGGGATTCG GGGGTGCCCGACCGCTTCACTGGCTCCGGGTCCGGCACCGACTTC ACCTTGAAAATCTCCAGAGTGGAAGCCGAGGACGTGGGCGTGTAC TACTGTATGCAGGGTACCCACTGGCCTGGAACCTTTGGACAAGGA ACTAAGCTCGAGATTAAG |
| **139110- aa VH** | 380 | QVQLVQSGGGLVKPGGSLRLSCAASGFTFSDYYMSWIRQAPGKGL |
| | | EWVSYISSSGNTIYYADSVKGRFTISRDNAKNSLYLQMNSLRAED TAVYYCARSTMVREDYWGQGTLVTVSS |
| **139110- aa VL** | 395 | DIVLTQSPLSLPVTLGQPASISCKSSESLVHNSGKTYLNWFHQRP GQSPRRLIYEVSNRDSGVPDRFTGSGSGTDFTLKISRVEAEDVGV YYCMQGTHWPGTFGQGTKLEIK |

| **139112** | | |
|---|---|---|
| **139112- aa ScFv domain** | 351 | QVQLVESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSDIR LTQSPSPLSASVGDRVTITCQASEDINKFLNWYHQTPGKAPKLLI YDASTLQTGVPSRFSGSGSGTDFTLTINSLQPEDIGTYYCQQYES LPLTFGGGTKVEIK |
| **139112- nt ScFv domain** | 366 | CAAGTGCAACTCGTGGAATCTGGTGGAGGACTCGTGCAACCCGGT GGAAGCCTTAGGCTGTCGTGCGCCGTCAGCGGGTTTGCTCTGAGC AACCATGGAATGTCCTGGGTCCGCCGGGCACCGGGAAAAGGGCTG GAATGGGTGTCCGGCATCGTGTACAGCGGGTCAACCTATTACGCC GCGTCCGTGAAGGGCAGATTCACTATCTCAAGAGACAACAGCCGG AACACCCTGTACTTGCAAATGAATTCCCTGCGCCCCGAGGACACC GCCATCTACTACTGCTCCGCCCACGGAGGAGAGTCGGACGTGTGG GGCCAGGGAACGACTGTGACTGTGTCCAGCGCATCAGGAGGGGGT GGTTCGGGCGGCCGGGCCTCGGGGGGAGGAGGTTCCGACATTCGG CTGACCCAGTCCCCGTCCCCACTGTCGGCCTCCGTCGGCGACCGC GTGACCATCACTTGTCAGGCGTCCGAGGACATTAACAAGTTCCTG AACTGGTACCACCAGACCCCTGGAAAGGCCCCCAAGCTGCTGATC TACGATGCCTCGACCCTTCAAACTGGAGTGCCTAGCCGGTTCTCC GGGTCCGGCTCCGGCACTGATTTCACTCTGACCATCAACTCATTG CAGCCGGAAGATATCGGGACCTACTATTGCCAGCAGTACGAATCC CTCCCGCTCACATTCGGCGGGGGAACCAAGGTCGAGATTAAG |
| **139112- aa VH** | 381 | QVQLVESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139112- aa VL** | 396 | DIRLTQSPSPLSASVGDRVTITCQASEDINKFLNWYHQTPGKAPK LLIYDASTLQTGVPSRFSGSGSGTDFTLTINSLQPEDIGTYYCQQ YESLPLTFGGGTKVEIK |

| **139113** | | |
|---|---|---|
| **139113- aa ScFv domain** | 352 | EVQLVETGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSETT LTQSPATLSVSPGERATLSCRASQSVGSNLAWYQQKPGQGPRLLI YGASTRATGIPARFSGSGSGTEFTLTISSLQPEDFAVYYCQQYND WLPVTFGQGTKVEIK |
| **139113- nt ScFv domain** | 367 | GAAGTGCAATTGGTGGAAACTGGAGGAGGACTTGTGCAACCTGGA GGATCATTGCGGCTCTCATGCGCTGTCTCCGGCTTCGCCCTGTCA AATCACGGGATGTCGTGGGTCAGACGGGCCCCGGGAAAGGGTCTG GAATGGGTGTCGGGGATTGTGTACAGCGGCTCCACCTACTACGCC GCTTCGGTCAAGGGCCGCTTCACTATTTCACGGGACAACAGCCGC AACACCCTCTATCTGCAAATGAACTCTCTCCGCCCGGAGGATACC |
| | | GCCATCTACTACTGCTCCGCACACGGCGGCGAATCCGACGTGTGG GGACAGGGAACCACTGTCACCGTGTCGTCCGCATCCGGTGGCGGA GGATCGGGTGGCCGGGCCTCCGGGGGCGGCGGCAGCGAGACTACC CTGACCCAGTCCCCTGCCACTCTGTCCGTGAGCCCGGGAGAGAGA GCCACCCTTAGCTGCCGGGCCAGCCAGAGCGTGGGCTCCAACCTG GCCTGGTACCAGCAGAAGCCAGGACAGGGTCCCAGGCTGCTGATC TACGGAGCCTCCACTCGCGCGACCGGCATCCCCGCGAGGTTCTCC GGGTCGGGTTCCGGGACCGAGTTCACCCTGACCATCTCCTCCCTC CAACCGGAGGACTTCGCGGTGTACTACTGTCAGCAGTACAACGAT TGGCTGCCCGTGACATTTGGACAGGGGACGAAGGTGGAAATCAAA |
| **139113- aa VH** | 382 | EVQLVETGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139113- aa VL** | 397 | ETTLTQSPATLSVSPGERATLSCRASQSVGSNLAWYQQKPGQGPR LLIYGASTRATGIPARFSGSGSGTEFTLTISSLQPEDFAVYYCQQ YNDWLPVTFGQGTKVEIK |

| **139114** | | |
|---|---|---|
| **139114- aa ScFv domain** | 353 | EVQLVESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSSASGGGGSGGRASGGGGSEIV LTQSPGTLSLSPGERATLSCRASQSIGSSSLAWYQQKPGQAPRLL MYGASSRASGIPDRFSGSGSGTDFTLTISRLEPEDFAVYYCQQYA GSPPFTFGQGTKVEIK |
| **139114- nt ScFv domain** | 368 | GAAGTGCAATTGGTGGAATCTGGTGGAGGACTTGTGCAACCTGGA GGATCACTGAGACTGTCATGCGCGGTGTCCGGTTTTGCCCTGAGC AATCATGGGATGTCGTGGGTCCGGCGCGCCCCGGAAAGGGTCTG GAATGGGTGTCGGGTATCGTCTACTCCGGGAGCACTTACTACGCC GCGAGCGTGAAGGGCCGCTTCACCATTTCCCGCGATAACTCCCGC AACACCCTGTACTTGCAAATGAACTCGCTCCGGCCTGAGGACACT GCCATCTACTACTGCTCCGCACACGGAGGAGAATCCGACGTGTGG GGCCAGGGAACTACCGTGACCGTCAGCAGCGCCTCCGGCGGCGGG GGCTCAGGCGGACGGGCTAGCGGCGGCGGTGGCTCCGAGATCGTG CTGACCCAGTCGCCTGGCACTCTCTCGCTGAGCCCCGGGAAAGG GCAACCCTGTCCTGTCGGGCCAGCCAGTCCATTGGATCATCCTCC CTCGCCTGGTATCAGCAGAAACCGGGACAGGCTCCGCGGCTGCTT ATGTATGGGGCCAGCTCAAGAGCCTCCGGCATTCCCGACCGGTTC TCCGGGTCCGGTTCCGGCACCGATTTCACCCTGACTATCTCGAGG CTGGAGCCAGAGGACTTCGCCGTGTACTACTGCCAGCAGTACGCG GGGTCCCCGCCGTTCACGTTCGGACAGGGAACCAAGGTCGAGATC AAG |
| **139114- aa VH** | 383 | EVQLVESGGGLVQPGGSLRLSCAVSGFALSNHGMSWVRRAPGKGL EWVSGIVYSGSTYYAASVKGRFTISRDNSRNTLYLQMNSLRPEDT AIYYCSAHGGESDVWGQGTTVTVSS |
| **139114- aa VL** | 398 | EIVLTQSPGTLSLSPGERATLSCRASQSIGSSSLAWYQQKPGQAP RLLMYGASSRASGIPDRFSGSGSGTDFTLTISRLEPEDFAVYYCQ QYAGSPPFTFGQGTKVEIK |

| **149362** | | |
|---|---|---|
| **149362-aa ScFv domain** | 429 | QVQLQESGPGLVKPSETLSLTCTVSGGSISSSYYYWGWIRQPPGK GLEWIGSIYYSGSAYYNPSLKSRVTISVDTSKNQFSLRLSSVTAA DTAVYYCARHWQEWPDAFDIWGQGTMVTVSSGGGGSGGGGSGGGG SETTLTQSPAFMSATPGDKVIISCKASQDIDDAMNWYQQKPGEAP LFIIQSATSPVPGIPPRFSGSGFGTDFSLTINNIESEDAAYYFCL QHDNFPLTFGQGTKLEIK |
| **149362-nt ScFv domain** | 450 | CAAGTGCAGCTTCAGGAAAGCGGACCGGGCCTGGTCAAGCCATCC GAAACTCTCTCCCTGACTTGCACTGTGTCTGGCGGTTCCATCTCA TCGTCGTACTACTACTGGGGCTGGATTAGGCAGCCGCCCGGAAAG GGACTGGAGTGGATCGGAAGCATCTACTATTCCGGCTCGGCGTAC TACAACCCTAGCCTCAAGTCGAGAGTGACCATCTCCGTGGATACC TCCAAGAACCAGTTTTCCCTGCGCCTGAGCTCCGTGACCGCCGCT GACACCGCCGTGTACTACTGTGCTCGGCATTGGCAGGAATGGCCC GATGCCTTCGACATTTGGGGCCAGGGCACTATGGTCACTGTGTCA TCCGGGGGTGGAGGCAGCGGGGGAGGAGGGTCCGGGGGGGGAGGT TCAGAGACAACCTTGACCCAGTCACCCGCATTCATGTCCGCCACT CCGGGAGACAAGGTCATCATCTCGTGCAAAGCGTCCCAGGATATC GACGATGCCATGAATTGGTACCAGCAGAAGCCTGGCGAAGCGCCG CTGTTCATTATCCAATCCGCAACCTCGCCCGTGCCTGGAATCCCA CCGCGGTTCAGCGGCAGCGGTTTCGGAACCGACTTTTCCCTGACC ATTAACAACATTGAGTCCGAGGACGCCGCCTACTACTTCTGCCTG CAACACGACAACTTCCCTCTCACGTTCGGCCAGGGAACCAAGCTG GAAATCAAG |
| **149362-aa VH** | 471 | QVQLQESGPGLVKPSETLSLTCTVSGGSISSSYYYWGWIRQPPGK GLEWIGSIYYSGSAYYNPSLKSRVTISVDTSKNQFSLRLSSVTAA DTAVYYCARHWQEWPDAFDIWGQGTMVTVSS |
| **149362-aa VL** | 492 | ETTLTQSPAFMSATPGDKVIISCKASQDIDDAMNWYQQKPGEAPL FIIQSATSPVPGIPPRFSGSGFGTDFSLTINNIESEDAAYYFCLQ HDNFPLTFGQGTKLEIK |

| **149363** | | |
|---|---|---|
| **149363-aa ScFv domain** | 430 | QVNLRESGPALVKPTQTLTLTCTFSGFSLRTSGMCVSWIRQPPGK ALEWLARIDWDEDKFYSTSLKTRLTISKDTSDNQVVLRMTNMDPA DTATYYCARSGAGGTSATAFDIWGPGTMVTVSSGGGGSGGGGSGG GGSDIQMTQSPSSLSASVGDRVTITCRASQDIYNNLAWFQLKPGS APRSLMYAANKSQSGVPSRFSGSASGTDFTLTISSLQPEDFATYY CQHYYRFPYSFGQGTKLEIK |
| **149363-nt ScFv domain** | 451 | CAAGTCAATCTGCGCGAATCCGGCCCCGCCTTGGTCAAGCCTACC CAGACCCTCACTCTGACCTGTACTTTCTCCGGCTTCTCCCTGCGG ACTTCCGGGATGTGCGTGTCCTGGATCAGACAGCCTCCGGGAAAG GCCCTGGAGTGGCTCGCTCGCATTGACTGGGATGAGGACAAGTTC TACTCCACCTCACTCAAGACCAGGCTGACCATCAGCAAAGATACC TCTGACAACCAAGTGGTGCTCCGCATGACCAACATGGACCCAGCC GACACTGCCACTTACTACTGCGCGAGGAGCGGAGCGGGCGGAACC TCCGCCACCGCCTTCGATATTTGGGGCCCGGGTACCATGGTCACC GTGTCAAGCGGAGGAGGGGGGTCCGGGGGCGGCGGTTCCGGGGGA GGCGGATCGGACATTCAGATGACTCAGTCACCATCGTCCCTGAGC GCTAGCGTGGGCGACAGAGTGACAATCACTTGCCGGGCATCCCAG GACATCTATAACAACCTTGCGTGGTTCCAGCTGAAGCCTGGTTCC |
| | | GCACCGCGGTCACTTATGTACGCCGCCAACAAGAGCCAGTCGGGA GTGCCGTCCCGGTTTTCCGGTTCGGCCTCGGGAACTGACTTCACC CTGACGATCTCCAGCCTGCAACCCGAGGATTTCGCCACCTACTAC TGCCAGCACTACTACCGCTTTCCCTACTCGTTCGGACAGGGAACC AAGCTGGAAATCAAG |
| **149363-aa VH** | 472 | QVNLRESGPALVKPTQTLTLTCTFSGFSLRTSGMCVSWIRQPPGK ALEWLARIDWDEDKFYSTSLKTRLTISKDTSDNQVVLRMTNMDPA DTATYYCARSGAGGTSATAFDIWGPGTMVTVSS |
| **149363-aa VL** | 493 | DIQMTQSPSSLSASVGDRVTITCRASQDIYNNLAWFQLKPGSAPR SLMYAANKSQSGVPSRFSGSASGTDFTLTISSLQPEDFATYYCQH YYRFPYSFGQGTKLEIK |

| **149364** | | |
|---|---|---|
| **149364-aa ScFv domain** | 431 | EVQLVESGGGLVKPGGSLRLSCAASGFTFSSYSMNWVRQAPGKGL EWVSSISSSSSYIYYADSVKGRFTISRDNAKNSLYLQMNSLRAED TAVYYCAKTIAAVYAFDIWGQGTTVTVSSGGGGSGGGGSGGGGSE IVLTQSPLSLPVTPEEPASISCRSSQSLLHSNGYNYLDWYLQKPG QSPQLLIYLGSNRASGVPDRFSGSGSGTDFTLKISRVEAEDVGVY YCMQALQTPYTFGQGTKLEIK |
| **149364-nt ScFv domain** | 452 | GAAGTGCAGCTTGTCGAATCCGGGGGGGGACTGGTCAAGCCGGGC GGATCACTGAGACTGTCCTGCGCCGCGAGCGGCTTCACGTTCTCC TCCTACTCCATGAACTGGGTCCGCCAAGCCCCCGGAAGGGACTG GAATGGGTGTCCTCTATCTCCTCGTCGTCCTACATCTACTAC GCCGACTCCGTGAAGGGAAGATTCACCATTTCCCGCGACAACGCA AAGAACTCACTGTACTTGCAAATGAACTCACTCCGGGCCGAAGAT ACTGCTGTGTACTATTGCGCCAAGACTATTGCCGCCGTCTACGCT TTCGACATCTGGGGCCAGGGAACCACCGTGACTGTGTCGTCCGGT GGTGGTGGCTCGGGCGGAGGAGGAAGCGGCGGCGGGGGTCCGAG ATTGTGCTGACCCAGTCGCCACTGAGCCTCCCTGTGACCCCCGAG GAACCCGCCAGCATCAGCTGCCGGTCCAGCCAGTCCCTGCTCCAC TCCAACGGATACAATTACCTCGATTGGTACCTTCAGAAGCCTGGA CAAAGCCCGCAGCTGCTCATCTACTTGGGATCAAACCGCGCGTCA GGAGTGCCTGACCGGTTCTCCGGCTCGGGCAGCGGTACCGATTTC ACCCTGAAAATCTCCAGGGTGGAGGCAGAGGACGTGGGAGTGTAT TACTGTATGCAGGCGCTGCAGACTCCGTACACATTTGGCAGGGC ACCAAGCTGGAGATCAAG |
| **149364-aa VH** | 473 | EVQLVESGGGLVKPGGSLRLSCAASGFTFSSYSMNWVRQAPGKGL EWVSSISSSSSYIYYADSVKGRFTISRDNAKNSLYLQMNSLRAED TAVYYCAKTIAAVYAFDIWGQGTTVTVSS |
| **149364-aa VL** | 494 | EIVLTQSPLSLPVTPEEPASISCRSSQSLLHSNGYNYLDWYLQKP GQSPQLLIYLGSNRASGVPDRFSGSGSGTDFTLKISRVEAEDVGV YYCMQALQTPYTFGQGTKLEIK |

| **149365** | | |
|---|---|---|
| **149365-aa ScFv domain** | 432 | EVQLVESGGGLVKPGGSLRLSCAASGFTFSDYYMSWIRQAPGKGL EWVSYISSSGSTIYYADSVKGRFTISRDNAKNSLYLQMNSLRAED TAVYYCARDLRGAFDIWGQGTMVTVSSGGGGSGGGGSGGGGSSYV LTQSPSVSAAPGYTATISCGGNNIGTKSVHWYQQKPGQAPLLVIR DDSVRPSKIPGRFSGSNSGNMATLTISGVQAGDEADFYCQVWDSD |
| | | SEHVVFGGGTKLTVL |
| **149365-nt ScFv domain** | 453 | GAAGTCCAGCTCGTGGAGTCCGGCGGAGGCCTTGTGAAGCCTGGA GGTTCGCTGAGACTGTCCTGCGCCGCCTCCGGCTTCACCTTCTCC GACTACTACATGTCCTGGATCAGACAGGCCCCGGGAAAGGGCCTG GAATGGGTGTCCTACATCTCGTCATCGGGCAGCACTATCTACTAC GCGGACTCAGTGAAGGGGCGGTTCACCATTTCCCGGGATAACGCG AAGAACTCGCTGTATCTGCAAATGAACTCACTGAGGGCCGAGGAC ACCGCCGTGTACTACTGCGCCCGCGATCTCCGCGGGGCATTTGAC ATCTGGGGACAGGGAACCATGGTCACAGTGTCCAGCGGAGGGGGA GGATCGGGTGGCGGAGGTTCCGGGGGTGGAGGCTCCTCCTACGTG CTGACTCAGAGCCCAAGCGTCAGCGCTGCGCCCGGTTACACGGCA ACCATCTCCTGTGGCGGAAACAACATTGGGACCAAGTCTGTGCAC TGGTATCAGCAGAAGCCGGGCCAAGCTCCCCTGTTGGTGATCCGC GATGACTCCGTGCGGCCTAGCAAAATTCCGGGACGGTTCTCCGGC TCCAACAGCGGCAATATGGCCACTCTCACCATCTCGGGAGTGCAG GCCGGAGATGAAGCCGACTTCTACTGCCAAGTCTGGGACTCAGAC TCCGAGCATGTGGTGTTCGGGGGCGGAACCAAGCTGACTGTGCTC |
| **149365-aa VH** | 474 | EVQLVESGGGLVKPGGSLRLSCAASGFTFSDYYMSWIRQAPGKGL EWVSYISSSGSTIYYADSVKGRFTISRDNAKNSLYLQMNSLRAED TAVYYCARDLRGAFDIWGQGTMVTVSS |
| **149365-aa VL** | 495 | SYVLTQSPSVSAAPGYTATISCGGNNIGTKSVHWYQQKPGQAPLL VIRDDSVRPSKIPGRFSGSNSGNMATLTISGVQAGDEADFYCQVW DSDSEHVVFGGGTKLTVL |

| **149366** | | |
|---|---|---|
| **149366-aa ScFv domain** | 433 | QVQLVQSGAEVKKPGASVKVSCKPSGYTVTSHYIHWVRRAPGQGL EWMGMINPSGGVTAYSQTLQGRVTMTSDTSSSTVYMELSSLRSED TAMYYCAREGSGSGWYFDFWGRGTLVTVSSGGGGSGGGGSGGGGS SYVLTQPPSVSVSPGQTASITCSGDGLSKKYVSWYQQKAGQSPVV LISRDKERPSGIPDRFSGSNSADTATLTISGTQAMDEADYYCQAW DDTTVVFGGGTKLTVL |
| **149366-nt ScFv domain** | 454 | CAAGTGCAGCTGGTGCAGAGCGGGGCCGAAGTCAAGAAGCCGGGA GCCTCCGTGAAAGTGTCCTGCAAGCCTTCGGGATACACCGTGACC TCCCACTACATTCATTGGGTCCGCCGCGCCCCCGGCCAAGGACTC GAGTGGATGGGCATGATCAACCCTAGCGGCGGAGTGACCGCGTAC AGCCAGACGCTGCAGGGACGCGTGACTATGACCTCGGATACCTCC TCCTCCACCGTCTATATGGAACTGTCCAGCCTGCGGTCCGAGGAT ACCGCCATGTACTACTGCGCCCGGGAAGGATCAGGCTCCGGGTGG TATTTCGACTTCTGGGGAAGAGGCACCCTCGTGACTGTGTCATCT GGGGGAGGGGGTTCCGGTGGTGGCGGATCGGGAGGAGGCGGTTCA TCCTACGTGCTGACCCAGCCACCCTCCGTGTCCGTGAGCCCCGGC CAGACTGCATCGATTACATGTAGCGGCGACGGCCTCTCCAAGAAA TACGTGTCGTGGTACCAGCAGAAGGCCGGACAGAGCCCGGTGGTG CTGATCTCAAGAGATAAGGAGCGGCCTAGCGGAATCCCGGACAGG TTCTCGGGTTCCAACTCCGCGGACACTGCTACTCTGACCATCTCG GGGACCCAGGCTATGGACGAAGCCGATTACTACTGCCAAGCCTGG GACGACACTACTGTCGTGTTTGGAGGGGGCACCAAGTTGACCGTC CTT |
| **149366-aa VH** | 475 | QVQLVQSGAEVKKPGASVKVSCKPSGYTVTSHYIHWVRRAPGQGL EWMGMINPSGGVTAYSQTLQGRVTMTSDTSSSTVYMELSSLRSED TAMYYCAREGSGSGWYFDFWGRGTLVTVSS |
| **149366-aa VL** | 496 | SYVLTQPPSVSVSPGQTASITCSGDGLSKKYVSWYQQKAGQSPVV LISRDKERPSGIPDRFSGSNSADTATLTISGTQAMDEADYYCQAW DDTTVVFGGGTKLTVL |

| **149367** | | |
|---|---|---|
| **149367-aa ScFv domain** | 434 | QVQLQESGPGLVKPSQTLSLTCTVSGGSISSGGYYWSWIRQHPGK GLEWIGYIYYSGSTYYNPSLKSRVTISVDTSKNQFSLKLSSVTAA DTAVYYCARAGIAARLRGAFDIWGQGTMVTVSSGGGGSGGGGSGG GGSDIVMTQSPSSVSASVGDRVIITCRASQGIRNWLAWYQQKPGK APNLLIYAASNLQSGVPSRFSGSGSGADFTLTISSLQPEDVATYY CQKYNSAPFTFGPGTKVDIK |
| **149367-nt ScFv domain** | 455 | CAAGTGCAGCTTCAGGAGAGCGGCCCGGGACTCGTGAAGCCGTCC CAGACCCTGTCCCTGACTTGCACCGTGTCGGGAGGAAGCATCTCG AGCGGAGGCTACTATTGGTCGTGGATTCGGCAGCACCCTGGAAAG GGCCTGGAATGGATCGGCTACATCTACTACTCCGGCTCGACCTAC TACAACCCATCGCTGAAGTCCAGAGTGACAATCTCAGTGGACACG TCCAAGAATCAGTTCAGCCTGAAGCTCTCTTCCGTGACTGCGGCC GACACCGCCGTGTACTACTGCGCACGCGCTGGAATTGCCGCCCGG CTGAGGGGTGCCTTCGACATTTGGGGACAGGGCACCATGGTCACC GTGTCCTCCGGCGGCGGAGGTTCCGGGGGTGGAGGCTCAGGAGGA GGGGGGTCCGACATCGTCATGACTCAGTCGCCCTCAAGCGTCAGC GCGTCCGTCGGGGACAGAGTGATCATCACCTGTCGGGCGTCCCAG GGAATTCGCAACTGGCTGGCCTGGTATCAGCAGAAGCCCGGAAAG GCCCCCAACCTGTTGATCTACGCCGCCTCAAACCTCCAATCCGGG GTGCCGAGCCGCTTCAGCGGCTCCGGTTCGGGTGCCGATTTCACT CTGACCATCTCCTCCCTGCAACCTGAAGATGTGGCTACCTACTAC TGCCAAAAGTACAACTCCGCACCTTTTACTTTCGGACCGGGGACC AAAGTGGACATTAAG |
| **149367-aa VH** | 476 | QVQLQESGPGLVKPSQTLSLTCTVSGGSISSGGYYWSWIRQHPGK GLEWIGYIYYSGSTYYNPSLKSRVTISVDTSKNQFSLKLSSVTAA DTAVYYCARAGIAARLRGAFDIWGQGTMVTVSS |
| **149367-aa VL** | 497 | DIVMTQSPSSVSASVGDRVIITCRASQGIRNWLAWYQQKPGKAPN LLIYAASNLQSGVPSRFSGSGSGADFTLTISSLQPEDVATYYCQK YNSAPFTFGPGTKVDIK |

| **149368** | | |
|---|---|---|
| **149368-aa ScFv domain** | 435 | QVQLVQSGAEVKKPGSSVKVSCKASGGTFSSYAISWVRQAPGQGL EWMGGIIPIFGTANYAQKFQGRVTITADESTSTAYMELSSLRSED TAVYYCARRGGYQLLRWDVGLLRSAFDIWGQGTMVTVSSGGGGSG GGGSGGGGSSYVLTQPPSVSVAPGQTARITCGGNNIGSKSVHWYQ QKPGQAPVLVLYGKNNRPSGVPDRFSGSRSGTTASLTITGAQAED EADYYCSSRDSSGDHLRVFGTGTKVTVL |
| **149368-nt ScFv domain** | 456 | CAAGTGCAGCTGGTCCAGTCGGGCGCCGAGGTCAAGAAGCCCGGG AGCTCTGTGAAAGTGTCCTGCAAGGCCTCCGGGGGCACCTTTAGC TCCTACGCCATCTCCTGGGTCCGCCAAGCACCGGGTCAAGGCCTG GAGTGGATGGGGGGAATTATCCCTATCTTCGGCACTGCCAACTAC |
| | | GCCCAGAAGTTCCAGGGACGCGTGACCATTACCGCGGACGAATCC ACCTCCACCGCTTATATGGAGCTGTCCAGCTTGCGCTCGGAAGAT ACCGCCGTGTACTACTGCGCCCGGAGGGGTGGATACCAGCTGCTG AGATGGGACGTGGGCCTCCTGCGGTCGGCGTTCGACATCTGGGGC CAGGGCACTATGGTCACTGTGTCCAGCGGAGGAGGCGGATCGGGA GGCGGCGGATCAGGGGGAGGCGGTTCCAGCTACGTGCTTACTCAA CCCCCTTCGGTGTCCGTGGCCCCGGGACAGACCGCCAGAATCACT TGCGGAGGAAACAACATTGGGTCCAAGAGCGTGCATTGGTACCAG CAGAAGCCAGGACAGGCCCCTGTGCTGGTGCTCTACGGAAGAAC AATCGGCCCAGCGGAGTGCCGGACAGGTTCTCGGGTTCACGCTCC GGTACAACCGCTTCACTGACTATCACCGGGGCCCAGGCAGAGGAT GAAGCGGACTACTACTGTTCCTCCCGGGATTCATCCGGCGACCAC CTCCGGGTGTTCGGAACCGGAACGAAGGTCACCGTGCTG |
| **149368-aa VH** | 477 | QVQLVQSGAEVKKPGSSVKVSCKASGGTFSSYAISWVRQAPGQGL EWMGGIIPIFGTANYAQKFQGRVTITADESTSTAYMELSSLRSED TAVYYCARRGGYQLLRWDVGLLRSAFDIWGQGTMVTVSS |
| **149368-aa VL** | 498 | SYVLTQPPSVSVAPGQTARITCGGNNIGSKSVHWYQQKPGQAPVL VLYGKNNRPSGVPDRFSGSRSGTTASLTITGAQAEDEADYYCSSR DSSGDHLRVFGTGTKVTVL |

| **149369** | | |
|---|---|---|
| **149369-aa ScFv domain** | 436 | EVQLQQSGPGLVKPSQTLSLTCAISGDSVSSNSAAWNWIRQSPSR GLEWLGRTYYRSKWYSFYAISLKSRIIINPDTSKNQFSLQLKSVT PEDTAVYYCARSSPEGLFLYWFDPWGQGTLVTVSSGGDGSGGGGS GGGGSSSELTQDPAVSVALGQTIRITCQGDSLGNYYATWYQQKPG QAPVLVIYGTNNRPSGIPDRFSASSSGNTASLTITGAQAEDEADY YCNSRDSSGHHLLFGTGTKVTVL |
| **149369-nt ScFv domain** | 457 | GAAGTGCAGCTCCAACAGTCAGGACCGGGCTCGTGAAGCCATCC CAGACCCTGTCCCTGACTTGTGCCATCTCGGGAGATAGCGTGTCA TCGAACTCCGCCGCCTGGAACTGGATTCGGCAGAGCCCGTCCCGC GGACTGGAGTGGCTTGGAAGGACCTACTACCGGTCCAAGTGGTAC TCTTTCTACGCGATCTCGCTGAAGTCCCGCATTATCATTAACCCT GATACCTCCAAGAATCAGTTCTCCCTCCAACTGAAATCCGTCACC CCCGAGGACACAGCAGTGTATTACTGCGCACGGAGCAGCCCCGAA GGACTGTTCCTGTATTGGTTTGACCCCTGGGGCCAGGGGACTCTT GTGACCGTGTCGAGCGGCGGAGATGGGTCCGGTGGCGGTGGTTCG GGGGGCGGCGGATCATCATCCGAACTGACCCAGGACCCGGCTGTG TCCGTGGCGCTGGGACAAACCATCCGCATTACGTGCCAGGGAGAC TCCCTGGGCAACTACTACGCCACTTGGTACCAGCAGAAGCCGGGC CAAGCCCCTGTGTTGGTCATCTACGGACCAACAACAGACCTTCC GGCATCCCCGACCGGTTCAGCGCTTCGTCCTCCGGCAACACTGCC AGCCTGACCATCACTGGAGCGCAGGCCGAAGATGAGGCCGACTAC TACTGCAACAGCAGAGACTCCTCGGGTCATCACCTCTTGTTCGGA ACTGGAACCAAGGTCACCGTGCTG |
| **149369-aa VH** | 478 | EVQLQQSGPGLVKPSQTLSLTCAISGDSVSSNSAAWNWIRQSPSR GLEWLGRTYYRSKWYSFYAISLKSRIIINPDTSKNQFSLQLKSVT PEDTAVYYCARSSPEGLFLYWFDPWGQGTLVTVSS |
| **149369-aa VL** | 499 | SSELTQDPAVSVALGQTIRITCQGDSLGNYYATWYQQKPGQAPVL |
| | | VIYGTNNRPSGIPDRFSASSSGNTASLTITGAQAEDEADYYCNSR DSSGHHLLFGTGTKVTVL |

| **BCMA_EBB-C1978-A4** | | |
|---|---|---|
| **BCMA_EB B-C1978-A4 - aa ScFv domain** | 437 | EVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKVEGSGSLDYWGQGTLVTVSSGGGGSGGGGSGGGGSEI VMTQSPGTLSLSPGERATLSCRASQSVSSAYLAWYQQKPGQPPRL LISGASTRATGIPDRFGGSGSGTDFTLTISRLEPEDFAVYYCQHY GSSFNGSSLFTFGQGTRLEIK |
| **BCMA_EB β-C1978-A4 - nt ScFv domain** | 458 | GAAGTGCAGCTCGTGGAGTCAGGAGGCGGCCTGGTCCAGCCGGGA GGGTCCCTTAGACTGTCATGCGCCGCAAGCGGATTCACTTTCTCC TCCTATGCCATGAGCTGGGTCCGCCAAGCCCCGGAAAGGACTG GAATGGGTGTCCGCCATCTCGGGGTCTGGAGGCTCAACTTACTAC GCTGACTCCGTGAAGGGACGGTTCACCATTAGCCGCGACAACTCC AAGAACACCCTCTACCTCCAAATGAACTCCCTGCGGGCCGAGGAT ACCGCCGTCTACTACTGCGCCAAAGTGGAAGGTTCAGGATCGCTG GACTACTGGGGACAGGGTACTCTCGTGACCGTGTCATCGGGCGGA GGAGGTTCCGGCGGTGGCGGCTCCGGCGGCGGAGGGTCGGAGATC GTGATGACCCAGAGCCCTGGTACTCTGAGCCTTTCGCCGGGAGAA AGGGCCACCCTGTCCTGCCGCGCTTCCCAATCCGTGTCCTCCGCG TACTTGGCGTGGTACCAGCAGAAGCCGGGACAGCCCCCTCGGCTG CTGATCAGCGGGGCCAGCACCCGGGCAACCGGAATCCCAGACAGA TTCGGGGGTTCCGGCAGCGGCACAGATTTCACCCTGACTATTTCG AGGTTGGAGCCCGAGGACTTTGCGGTGTATTACTGTCAGCACTAC GGGTCGTCCTTTAATGGCTCCAGCCTGTTCACGTTCGGACAGGGG ACCCGCCTGGAAATCAAG |
| **BCMA_EB B-C1978-A4 - aa VH** | 479 | EVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKVEGSGSLDYWGQGTLVTVSS |
| **BCMA_EB B-C1978-A4 - aa VL** | 500 | EIVMTQSPGTLSLSPGERATLSCRASQSVSSAYLAWYQQKPGQPP RLLISGASTRATGIPDRFGGSGSGTDFTLTISRLEPEDFAVYYCQ HYGSSFNGSSLFTFGQGTRLEIK |

| **BCMA_EBB-C1978-G1** | | |
|---|---|---|
| **BCMA_EB B-C1978-G1 - aa ScFv domain** | 438 | EVQLVETGGGLVQPGGSLRLSCAASGITFSRYPMSWVRQAPGKGL EWVSGISDSGVSTYYADSAKGRFTISRDNSKNTLFLQMSSLRDED TAVYYCVTRAGSEASDIWGQGTMVTVSSGGGGSGGGGSGGGGSEI VLTQSPATLSLSPGERATLSCRASQSVSNSLAWYQQKPGQAPRLL IYDASSRATGIPDRFSGSGSGTDFTLTISRLEPEDFAIYYCQQFG TSSGLTFGGGTKLEIK |
| **BCMA_EB B-C1978-G1 - nt ScFv domain** | 459 | GAAGTGCAACTGGTGGAAACCGGTGGCGGCCTGGTGCAGCCTGGA GGATCATTGAGGCTGTCATGCGCGGCCAGCGGTATTACCTTCTCC CGGTACCCCATGTCCTGGGTCAGACAGGCCCCGGGAAAGGGCTT GAATGGGTGTCCGGGATCTCGGACTCCGGTGTCAGCACTTACTAC GCCGACTCCGCCAAGGGACGCTTCACCATTTCCCGGGACAACTCG AAGAACACCCTGTTCCTCCAAATGAGCTCCCTCCGGGACGAGGAT |
| | | ACTGCAGTGTACTACTGCGTGACCCGCGCCGGGTCCGAGGCGTCT GACATTTGGGGACAGGGCACTATGGTCACCGTGTCGTCCGGCGGA GGGGGCTCGGGAGGCGGTGGCAGCGGAGGAGGAGGGTCCGAGATC GTGCTGACCCAATCCCCGGCCACCCTCTCGCTGAGCCCTGGAGAA AGGGCAACCTTGTCCTGTCGCGCGAGCCAGTCCGTGAGCAACTCC CTGGCCTGGTACCAGCAGAAGCCCGGACAGGCTCCGAGACTTCTG ATCTACGACGCTTCGAGCCGGGCCACTGGAATCCCCGACCGCTTT TCGGGGTCCGGCTCAGGAACCGATTTCACCCTGACAATCTCACGG CTGGAGCCAGAGGATTTCGCCATCTATTACTGCCAGCAGTTCGGT ACTTCCTCCGGCCTGACTTTCGGAGGCGGCACGAAGCTCGAAATC AAG |
| **BCMA_EB B-C1978-G1 - aa VH** | 480 | EVQLVETGGGLVQPGGSLRLSCAASGITFSRYPMSWVRQAPGKGL EWVSGISDSGVSTYYADSAKGRFTISRDNSKNTLFLQMSSLRDED TAVYYCVTRAGSEASDIWGQGTMVTVSS |
| **BCMA_EB B-C1978-G1 - aa VL** | 501 | EIVLTQSPATLSLSPGERATLSCRASQSVSNSLAWYQQKPGQAPR LLIYDASSRATGIPDRFSGSGSGTDFTLTISRLEPEDFAIYYCQQ FGTSSGLTFGGGTKLEIK |

| **BCMA_EBB-C1979-C1** | | |
|---|---|---|
| **BCMA_EB B-C1979-C1 - aa ScFv domain** | 439 | QVQLVESGGGLVQPGGSLRLSCAASGFTFSSYAMSWVRQAPGKGL EWVSAISGSGGSTYYADSVKGRFTISRDNAKNSLYLQMNSLRAED TAIYYCARATYKRELRYYYGMDVWGQGTMVTVSSGGGGSGGGGSG GGGSEIVMTQSPGTVSLSPGERATLSCRASQSVSSSFLAWYQQKP GQAPRLLIYGASSRATGIPDRFSGSGSGTDFTLTISRLEPEDSAV YYCQQYHSSPSWTFGQGTRLEIK |
| **BCMA_EB B-C1979-C1 - nt ScFv domain** | 460 | CAAGTGCAGCTCGTGGAATCGGGTGGCGGACTGGTGCAGCCGGGG GGCTCACTTAGACTGTCCTGCGCGGCCAGCGGATTCACTTTCTCC TCCTACGCCATGTCCTGGGTCAGACAGGCCCCTGGAAAGGGCCTG GAATGGGTGTCCGCAATCAGCGGCAGCGGCGGCTCGACCTATTAC GCGGATTCAGTGAAGGGCAGATTCACCATTTCCCGGGACAACGCC AAGAACTCCTTGTACCTTCAAATGAACTCCCTCCGCGCGGAAGAT ACCGCAATCTACTACTGCGCTCGGGCCACTTACAAGAGGGAACTG CGCTACTACTACGGGATGGACGTCTGGGGCCAGGGAACCATGGTC ACCGTGTCCAGCGGAGGAGGAGGATCGGGAGGAGGCGGTAGCGGG GGTGGAGGGTCGGAGATCGTGATGACCCAGTCCCCCGGCACTGTG TCGCTGTCCCCCGGCGAACGGGCCACCCTGTCATGTCGGGCCAGC CAGTCAGTGTCGTCAAGCTTCCTCGCCTGGTACCAGCAGAAACCG GGACAAGCTCCCCGCCTGCTGATCTACGGAGCCAGCAGCCGGGCC ACCGGTATTCCTGACCGGTTCTCCGGTTCGGGGTCCGGGACCGAC TTTACTCTGACTATCTCTCGCCTCGAGCCAGAGGACTCCGCCGTG TATTACTGCCAGCAGTACCACTCCTCCCCGTCCTGGACGTTCGGA CAGGGCACAAGGCTGGAGATTAAG |
| **BCMA_EB B-C1979-C1 - aa VH** | 481 | QVQLVESGGGLVQPGGSLRLSCAASGFTFSSYAMSWVRQAPGKGL EWVSAISGSGGSTYYADSVKGRFTISRDNAKNSLYLQMNSLRAED TAIYYCARATYKRELRYYYGMDVWGQGTMVTVSS |
| **BCMA_EB B-C1979-C1 - aa VL** | **502** | EIVMTQSPGTVSLSPGERATLSCRASQSVSSSFLAWYQQKPGQAP RLLIYGASSRATGIPDRFSGSGSGTDFTLTISRLEPEDSAVYYCQ QYHSSPSWTFGQGTRLEIK |

| **BCMA_EBB-C1978-C7** | | |
|---|---|---|
| **BCMA_EB B-C1978-C7 - aa ScFv domain** | 440 | EVQLVETGGGLVQPGGSLRLSCAASGFTFSSYAMSWVRQAPGKGL EWVSAISGSGGSTYYADSVKGRFTISRDNSKNTLYLQMNTLKAED TAVYYCARATYKRELRYYYGMDVWGQGTTVTVSSGGGGSGGGGSG GGGSEIVLTQSPSTLSLSPGESATLSCRASQSVSTTFLAWYQQKP GQAPRLLIYGSSNRATGIPDRFSGSGSGTDFTLTIRRLEPEDFAV YYCQQYHSSPSWTFGQGTKVEIK |
| **BCMA_EB B-C1978-C7 - nt ScFv domain** | 461 | GAGGTGCAGCTTGTGGAAACCGGTGGCGGACTGGTGCAGCCCGGA GGAAGCCTCAGGCTGTCCTGCGCCGCGTCCGGCTTCACCTTCTCC TCGTACGCCATGTCCTGGGTCCGCCAGGCCCCCGGAAAGGGCCTG GAATGGGTGTCCGCCATCTCTGGAAGCGGAGGTTCCACGTACTAC GCGGACAGCGTCAAGGGAAGGTTCACAATCTCCCGCGATAATTCG AAGAACACTCTGTACCTTCAAATGAACACCCTGAAGGCCGAGGAC ACTGCTGTGTACTACTGCGCACGGGCCACCTACAAGAGAGAGCTC CGGTACTACTACGGAATGGACGTCTGGGGCCAGGGAACTACTGTG ACCGTGTCCTCGGGAGGGGGTGGCTCCGGGGGGGGCGGCTCCGGC GGAGGCGGTTCCGAGATTGTGCTGACCCAGTCACCTTCAACTCTG TCGCTGTCCCCGGGAGAGAGCGCTACTCTGAGCTGCCGGGCCAGC CAGTCCGTGTCCACCACCTTCCTCGCCTGGTATCAGCAGAAGCCG GGGCAGGCACCACGGCTCTTGATCTACGGGTCAAGCAACAGAGCG ACCGGAATTCCTGACCGCTTCTCGGGGAGCGGTTCAGGCACCGAC TTCACCCTGACTATCCGGCGCCTGGAACCCGAAGATTTCGCCGTG TATTACTGTCAACAGTACCACTCCTCGCCGTCCTGGACCTTTGGC CAAGGAACCAAAGTGGAAATCAAG |
| **BCMA_EB B-C1978-C7 - aa VH** | 482 | EVQLVETGGGLVQPGGSLRLSCAASGFTFSSYAMSWVRQAPGKGL EWVSAISGSGGSTYYADSVKGRFTISRDNSKNTLYLQMNTLKAED TAVYYCARATYKRELRYYYGMDVWGQGTTVTVSS |
| **BCMA_EB B-C1978-C7 - aa VL** | 503 | EIVLTQSPSTLSLSPGESATLSCRASQSVSTTFLAWYQQKPGQAP RLLIYGSSNRATGIPDRFSGSGSGTDFTLTIRRLEPEDFAVYYCQ QYHSSPSWTFGQGTKVEIK |

| **BCMA_EBB-C1978-D10** | | |
|---|---|---|
| **BCMA_EB B-C1978-D10 - aa ScFv domain** | 441 | EVQLVETGGGLVQPGRSLRLSCAASGFTFDDYAMHWVRQAPGKGL EWVSGISWNSGSIGYADSVKGRFTISRDNAKNSLYLQMNSLRDED TAVYYCARVGKAVPDVWGQGTTVTVSSGGGGSGGGGSGGGGSDIV MTQTPSSLSASVGDRVTITCRASQSISSYLNWYQQKPGKAPKLLI YAASSLQSGVPSRFSGSGSGTDFTLTISSLQPEDFATYYCQQSYS TPYSFGQGTRLEIK |
| **BCMA_EB B-C1978-D10- nt ScFv domain** | 462 | GAAGTGCAGCTCGTGGAAACTGGAGGTGGACTCGTGCAGCCTGGA CGGTCGCTGCGGCTGAGCTGCGCTGCATCCGGCTTCACCTTCGAC GATTATGCCATGCACTGGGTCAGACAGGCGCCAGGGAAGGGACTT GAGTGGGTGTCCGGTATCAGCTGGAATAGCGGCTCAATCGGATAC |
| | | GCGGACTCCGTGAAGGGAAGGTTCACCATTTCCCGCGACAACGCC AAGAACTCCCTGTACTTGCAAATGAACAGCCTCCGGGATGAGGAC ACTGCCGTGTACTACTGCGCCCGCGTCGGAAAAGCTGTGCCCGAC GTCTGGGGCCAGGGAACCACTGTGACCGTGTCCAGCGGCGGGGGT GGATCGGGCGGTGGAGGGTCCGGTGGAGGGGGCTCAGATATTGTG ATGACCCAGACCCCCTCGTCCCTGTCCGCCTCGGTCGGCGACCGC GTGACTATCACATGTAGAGCCTCGCAGAGCATCTCCAGCTACCTG AACTGGTATCAGCAGAAGCCGGGGAAGGCCCCGAAGCTCCTGATC TACGCGGCATCATCACTGCAATCGGGAGTGCCGAGCCGGTTTTCC GGGTCCGGCTCCGGCACCGACTTCACGCTGACCATTTCTTCCCTG CAACCCGAGGACTTCGCCACTTACTACTGCCAGCAGTCCTACTCC ACCCCTTACTCCTTCGGCCAAGGAACCAGGCTGGAAATCAAG |
| **BCMA_EB B-C1978-D10 - aa VH** | 483 | EVQLVETGGGLVQPGRSLRLSCAASGFTFDDYAMHWVRQAPGKGL EWVSGISWNSGSIGYADSVKGRFTISRDNAKNSLYLQMNSLRDED TAVYYCARVGKAVPDVWGQGTTVTVSS |
| **BCMA_EB B-C1978-D10- aa VL** | 504 | DIVMTQTPSSLSASVGDRVTITCRASQSISSYLNWYQQKPGKAPK LLIYAASSLQSGVPSRFSGSGSGTDFTLTISSLQPEDFATYYCQQ SYSTPYSFGQGTRLEIK |

| **BCMA_EBB-C1979-C12** | | |
|---|---|---|
| **BCMA_EB B-C1979-C12- aa ScFv domain** | 442 | EVQLVESGGGLVQPGRSLRLSCTASGFTFDDYAMHWVRQRPGKGL EWVASINWKGNSLAYGDSVKGRFAISRDNAKNTVFLQMNSLRTED TAVYYCASHQGVAYYNYAMDVWGRGTLVTVSSGGGGSGGGGSGGG GSEIVLTQSPGTLSLSPGERATLSCRATQSIGSSFLAWYQQRPGQ APRLLIYGASQRATGIPDRFSGRGSGTDFTLTISRVEPEDSAVYY CQHYESSPSWTFGQGTKVEIK |
| **BCMA_EB B-C1979-C12 - nt ScFv domain** | 463 | GAAGTGCAGCTCGTGGAGAGCGGGGGAGGATTGGTGCAGCCCGGA AGGTCCCTGCGGCTCTCCTGCACTGCGTCTGGCTTCACCTTCGAC GACTACGCGATGCACTGGGTCAGACAGCGCCCGGGAAAGGGCCTG GAATGGGTCGCCTCAATCAACTGGAAGGGAAACTCCCTGGCCTAT GGCGACAGCGTGAAGGGCCGCTTCGCCATTTCGCGCGACAACGCC AAGAACACCGTGTTTCTGCAAATGAATTCCCTGCGGACCGAGGAT ACCGCTGTGTACTACTGCGCCAGCCACCAGGGCGTGGCATACTAT AACTACGCCATGGACGTGTGGGGAAGAGGGACGCTCGTCACCGTG TCCTCCGGGGGCGGTGGATCGGGTGGAGGAGGAAGCGGTGGCGGG GGCAGCGAAATCGTGCTGACTCAGAGCCCGGGAACTCTTTCACTG TCCCCGGGAGAACGGGCCACTCTCTCGTGCCGGGCCACCCAGTCC ATCGGCTCCTCCTTCCTTGCCTGGTACCAGCAGAGGCCAGGACAG GCGCCCCGCCTGCTGATCTACGGTGCTTCCCAACGCGCCACTGGC ATTCCTGACCGGTTCAGCGGCAGAGGGTCGGGAACCGATTTCACA CTGACCATTTCCCGGGTGGAGCCCGAAGATTCGGCAGTCTACTAC TGTCAGCATTACGAGTCCTCCCCTTCATGGACCTTCGGTCAAGGG ACCAAAGTGGAGATCAAG |
| **BCMA_EB B-C1979-C12 - aa VH** | 484 | EVQLVESGGGLVQPGRSLRLSCTASGFTFDDYAMHWVRQRPGKGL EWVASINWKGNSLAYGDSVKGRFAISRDNAKNTVFLQMNSLRTED TAVYYCASHQGVAYYNYAMDVWGRGTLVTVSS |
| | | |
| **BCMA_EB B-C1979-C12 - aa VL** | 505 | EIVLTQSPGTLSLSPGERATLSCRATQSIGSSFLAWYQQRPGQAP RLLIYGASQRATGIPDRFSGRGSGTDFTLTISRVEPEDSAVYYCQ HYESSPSWTFGQGTKVEIK |

| **BCMA_EBB-C1980-G4** | | |
|---|---|---|
| **βC111A**_**EB B- C1980-G4- aa ScFv domain** | 443 | EVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKVVRDGMDVWGQGTTVTVSSGGGGSGGGGSGGGGSEIV LTQSPATLSLSPGERATLSCRASQSVSSSYLAWYQQKPGQAPRLL IYGASSRATGIPDRFSGNGSGTDFTLTISRLEPEDFAVYYCQQYG SPPRFTFGPGTKVDIK |
| **BCMA_EB B- C1980-G4- nt ScFv domain** | 464 | GAGGTGCAGTTGGTCGAAAGCGGGGGCGGGCTTGTGCAGCCTGGC GGATCACTGCGGCTGTCCTGCGCGGCATCAGGCTTCACGTTTTCT TCCTACGCCATGTCCTGGGTGCGCCAGGCCCCTGGAAAGGGACTG GAATGGGTGTCCGCGATTTCGGGGTCCGGCGGAGCACCTACTAC GCCGATTCCGTGAAGGGCCGCTTCACTATCTCGCGGGACAACTCC AAGAACACCCTCTACCTCCAAATGAATAGCCTGCGGGCCGAGGAT ACCGCCGTCTACTATTGCGCTAAGGTCGTGCGCGACGGAATGGAC GTGTGGGGACAGGGTACCACCGTGACAGTGTCCTCGGGGGGAGGC GGTAGCGGCGGAGGAGGAAGCGGTGGTGGAGGTTCCGAGATTGTG CTGACTCAATCACCCGCGACCCTGAGCCTGTCCCCCGGCGAAAGG GCCACTCTGTCCTGTCGGGCCAGCCAATCAGTCTCCTCCTCGTAC CTGGCCTGGTACCAGCAGAAGCCAGGACAGGCTCCGAGACTCCTT ATCTATGGCGCATCCTCCCGCGCCACCGGAATCCCGGATAGGTTC TCGGGAAACGGATCGGGGACCGACTTCACTCTCACCATCTCCCGG CTGGAACCGGAGGACTTCGCCGTGTACTACTGCCAGCAGTACGGC AGCCCGCCTAGATTCACTTTCGGCCCCGGCACCAAAGTGGACATC AAG |
| **BCMA_EB B- C1980-G4- aa VH** | 485 | EVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKVVRDGMDVWGQGTTVTVSS |
| **BCMA_EB B- C1980-G4- aa VL** | 506 | EIVLTQSPATLSLSPGERATLSCRASQSVSSSYLAWYQQKPGQAP RLLIYGASSRATGIPDRFSGNGSGTDFTLTISRLEPEDFAVYYCQ QYGSPPRFTFGPGTKVDIK |

| **BCMA EBB-C1980-D2** | | |
|---|---|---|
| **BCMA_EB B- C1980-D2- aa ScFv domain** | 444 | EVQLLESGGGLVQPGGSLRLSCAASGFTFSSYAMSWVRQAPGKGL EWVSAISGSGGSTYYADSVKGRFTISRDNSKNTLYLQMNSLRAED TAVYYCAKIPQTGTFDYWGQGTLVTVSSGGGGSGGGGSGGGGSEI VLTQSPGTLSLSPGERATLSCRASQSVSSSYLAWYQQRPGQAPRL LIYGASSRATGIPDRFSGSGSGTDFTLTISRLEPEDFAVYYCQHY GSSPSWTFGQGTRLEIK |
| **BCMA_EB B- C1980-D2- nt ScFv domain** | 465 | GAAGTGCAGCTGCTGGAGTCCGGCGGTGGATTGGTGCAACCGGGG GGATCGCTCAGACTGTCCTGTGCGGCGTCAGGCTTCACCTTCTCG AGCTACGCCATGTCATGGGTCAGACAGGCCCCTGGAAAGGGTCTG |
| | | GAATGGGTGTCCGCCATTTCCGGAGCGGGGATCTACATACTAC GCCGATAGCGTGAAGGGCCGCTTCACCATTTCCCGGACAACTCC AAGAACACTCTCTATCTGCAAATGAACTCCCTCCGCGCTGAGGAC ACTGCCGTGTACTACTGCGCCAAAATCCCTCAGACCGGCACCTTC GACTACTGGGGACAGGGGACTCTGGTCACCGTCAGCAGCGGTGGC GGAGGTTCGGGGGGAGGAGGAAGCGGCGGCGGAGGGTCCGAGATT GTGCTGACCCAGTCACCCGGCACTTTGTCCCTGTCGCCTGGAGAA AGGGCCACCCTTTCCTGCCGGGCATCCCAATCCGTGTCCTCCTCG TACCTGGCCTGGTACCAGCAGAGGCCCGGACAGGCCCCACGGCTT CTGATCTACGGAGCAAGCAGCCGCGCGACCGGTATCCCGGACCGG TTTTCGGGCTCGGGCTCAGGAACTGACTTCACCCTCACCATCTCC CGCCTGGAACCCGAAGATTTCGCTGTGTATTACTGCCAGCACTAC GGCAGCTCCCCGTCCTGGACGTTCGGCCAGGGAACTCGGCTGGAG ATCAAG |
| **BCMA_EB B- C1980-D2- aa VH** | 486 | EVQLLESGGGLVQPGGSLRLSCAASGFTFSSYAMSWVRQAPGKGL EWVSAISGSGGSTYYADSVKGRFTISRDNSKNTLYLQMNSLRAED TAVYYCAKIPQTGTFDYWGQGTLVTVSS |
| **BCMA_EB B- C1980-D2- aa VL** | 507 | EIVLTQSPGTLSLSPGERATLSCRASQSVSSSYLAWYQQRPGQAP RLLIYGASSRATGIPDRFSGSGSGTDFTLTISRLEPEDFAVYYCQ HYGSSPSWTFGQGTRLEIK |

| **BCMA_EBB-C1978-A10** | | |
|---|---|---|
| **BCMA_EB B- C1978-A10- aa ScFv domain** | 445 | EVQLVETGGGLVQPGGSLRLSCAASGFTFSSYAMSWVRQAPGKGL EWVSAISGSGGSTYYADSVKGRFTMSRENDKNSVFLQMNSLRVED TGVYYCARANYKRELRYYYGMDVWGQGTMVTVSSGGGGSGGGGSG GGGSEIVMTQSPGTLSLSPGESATLSCRASQRVASNYLAWYQHKP GQAPSLLISGASSRATGVPDRFSGSGSGTDFTLAISRLEPEDSAV YYCQHYDSSPSWTFGQGTKVEIK |
| **BCMA_EB B- C1978-A10- nt ScFv domain** | 466 | GAAGTGCAACTGGTGGAAACCGGTGGAGGACTCGTGCAGCCTGGC GGCAGCCTCCGGCTGAGCTGCGCCGCTTCGGGATTCACCTTTTCC TCCTACGCGATGTCTTGGGTCAGACAGGCCCCCGGAAAGGGGCTG GAATGGGTGTCAGCCATCTCCGGCTCCGGCGGATCAACGTACTAC GCCGACTCCGTGAAAGGCCGGTTCACCATGTCGCGCGAGAATGAC AAGAACTCCGTGTTCCTGCAAATGAACTCCCTGAGGGTGGAGGAC ACCGGAGTGTACTATTGTGCGCGCCAACTACAAGAGAGAGCTG CGGTACTACTACGGAATGGACGTCTGGGGACAGGGAACTATGGTG ACCGTGTCATCCGGTGGAGGGGGAAGCGGCGGTGGAGGCAGCGGG GGCGGGGGTTCAGAAATTGTCATGACCCAGTCCCCGGGAACTCTT TCCCTCTCCCCCGGGGAATCCGCGACTTTGTCCTGCCGGGCCAGC CAGCGCGTGGCCTCGAACTACCTCGCATGGTACCAGCATAAGCCA GGCCAAGCCCCTTCCCTGCTGATTTCCGGGGCTAGCAGCCGCGCC ACTGGCGTGCCGGATAGGTTCTCGGGAAGCGGCTCGGGTACCGAT TTCACCCTGGCAATCTCGCGGCTGGAACCGGAGGATTCGGCCGTG TACTACTGCCAGCACTATGACTCATCCCCCTCCTGGACATTCGGA CAGGGCACCAAGGTCGAGATCAAG |
| **BCMA_EB B- C1978-A10- aa VH** | 487 | EVQLVETGGGLVQPGGSLRLSCAASGFTFSSYAMSWVRQAPGKGL |
| | | EWVSAISGSGGSTYYADSVKGRFTMSRENDKNSVFLQMNSLRVED TGVYYCARANYKRELRYYYGMDVWGQGTMVTVSS |
| **BCMA_EB B- C1978-A10- aa VL** | 508 | EIVMTQSPGTLSLSPGESATLSCRASQRVASNYLAWYQHKPGQAP SLLISGASSRATGVPDRFSGSGSGTDFTLAISRLEPEDSAVYYCQ HYDSSPSWTFGQGTKVEIK |

| **BCMA_EBB-C1978-D4** | | |
|---|---|---|
| **BCMA_EB B- C1978-D4- aa ScFv domain** | 446 | EVQLLETGGGLVQPGGSLRLSCAASGFSFSSYAMSWVRQAPGKGL EWVSAISGSGGSTYYADSVKGRFTISRDNSKNTLYLQMNSLRAED TAVYYCAKALVGATGAFDIWGQGTLVTVSSGGGGSGGGGSGGGGS EIVLTQSPGTLSLSPGERATLSCRASQSLSSNFLAWYQQKPGQAP GLLIYGASNWATGTPDRFSGSGSGTDFTLTITRLEPEDFAVYYCQ YYGTSPMYTFGQGTKVEIK |
| **BCMA_EB B- C1978-D4- nt ScFv domain** | 467 | GAAGTGCAGCTGCTCGAAACCGGTGGAGGCTGGTGCAGCCAGGG GGCTCCCTGAGGCTTTCATGCGCCGCTAGCGGATTCTCCTTCTCC TCTTACGCCATGTCGTGGGTCCGCCAAGCCCCTGGAAAAGGCCTG GAATGGGTGTCCGCGATTTCCGGGAGCGGAGGTTCGACCTATTAC GCCGACTCCGTGAAGGGCCGCTTTACCATCTCCCGGGATAACTCC AAGAACACTCTGTACCTCCAAATGAACTCGCTGAGAGCCGAGGAC ACCGCCGTGTATTACTGCGCGAAGGCGCTGGTCGGCGCGACTGGG GCATTCGACATCTGGGGACAGGGAACTCTTGTGACCGTGTCGAGC GGAGGCGGCGGCTCCGGCGGAGGAGGGAGCGGGGGCGGTGGTTCC GAAATCGTGTTGACTCAGTCCCCGGGAACCCTGAGCTTGTCACCC GGGGAGCGGGCCACTCTCTCCTGTCGCGCCTCCCAATCGCTCTCA TCCAATTTCCTGGCCTGGTACCAGCAGAAGCCCGGACAGGCCCCG GGCCTGCTCATCTACGGCGCTTCAAACTGGGCAACGGAACCCCT GATCGGTTCAGCGGAAGCGGATCGGGTACTGACTTTACCCTGACC ATCACCAGACTGGAACCGGAGGACTTCGCCGTGTACTACTGCCAG TACTACGGCACCTCCCCCATGTACACATTCGGACAGGGTACCAAG GTCGAGATTAAG |
| **BCMA_EB B- C1978-D4- aa VH** | 488 | EVQLLETGGGLVQPGGSLRLSCAASGFSFSSYAMSWVRQAPGKGL EWVSAISGSGGSTYYADSVKGRFTISRDNSKNTLYLQMNSLRAED TAVYYCAKALVGATGAFDIWGQGTLVTVSS |
| **BCMA_EB B- C1978-D4- aa VL** | 509 | EIVLTQSPGTLSLSPGERATLSCRASQSLSSNFLAWYQQKPGQAP GLLIYGASNWATGTPDRFSGSGSGTDFTLTITRLEPEDFAVYYCQ YYGTSPMYTFGQGTKVEIK |

| **BCMA_EBB-C1980-A2** | | |
|---|---|---|
| **BCMA_EB B- C1980-A2- aa ScFv domain** | 447 | EVQLLESGGGLVQPGGSLRLSCAASGFTFSSYAMSWVRQAPGKGL EWVSAISGSGGSTYYADSVKGRFTISRDNSKNTLYLQMNSLRAED TAVYYCVLWFGEGFDPWGQGTLVTVSSGGGGSGGGGSGGGGSDIV LTQSPLSLPVTPGEPASISCRSSQSLLHSNGYNYLDWYLQKPGQS PQLLIYLGSNRASGVPDRFSGSGSGTDFTLKISRVEAEDVGVYYC MQALQTPLTFGGGTKVDIK |
| **BCMA_EB B- C1980-A2- nt ScFv domain** | 468 | GAAGTGCAGCTGCTTGAGAGCGGTGGAGGTCTGGTGCAGCCCGGG |
| | | GGATCACTGCGCCTGTCCTGTGCCGCGTCCGGTTTCACTTTCTCC TCGTACGCCATGTCGTGGGTCAGACAGGCACCGGGAAAGGACTG GAATGGGTGTCAGCCATTTCGGGTTCGGGGGCAGCACCTACTAC GCTGACTCCGTGAAGGGCCGGTTCACCATTTCCCGCGACAACTCC AAGAACACCTTGTACCTCCAAATGAACTCCCTGCGGGCCGAAGAT ACCGCCGTGTATTACTGCGTGCTGTGGTTCGGAGAGGGATTCGAC CCGTGGGGACAAGGAACACTCGTGACTGTGTCATCCGGCGGAGGC GGCAGCGGTGGCGGCGGTTCCGGCGGCGGCGGATCTGACATCGTG TTGACCCAGTCCCCTCTGAGCCTGCCGGTCACTCCTGGCGAACCA GCCAGCATCTCCTGCCGGTCGAGCCAGTCCCTCCTGCACTCCAAT GGGTACAACTACCTCGATTGGTATCTGCAAAAGCCGGGCCAGAGC CCCCAGCTGCTGATCTACCTTGGGTCAAACCGCGCTTCCGGGGTG CCTGATAGATTCTCCGGGTCCGGGAGCGGAACCGACTTTACCCTG AAAATCTCGAGGGTGGAGGCCGAGGACGTCGGAGTGTACTACTGC ATGCAGGCGCTCCAGACTCCCCTGACCTTCGGAGGAGGAACGAAG GTCGACATCAAGA |
| **BCMA_EB B- C1980-A2- aa VH** | 489 | EVQLLESGGGLVQPGGSLRLSCAASGFTFSSYAMSWVRQAPGKGL EWVSAISGSGGSTYYADSVKGRFTISRDNSKNTLYLQMNSLRAED TAVYYCVLWFGEGFDPWGQGTLVTVSS |
| **BCMA_EB B- C1980-A2- aa VL** | 510 | DIVLTQSPLSLPVTPGEPASISCRSSQSLLHSNGYNYLDWYLQKP GQSPQLLIYLGSNRASGVPDRFSGSGSGTDFTLKISRVEAEDVGV YYCMQALQTPLTFGGGTKVDIK |

| **BCMA_EBB-C1981-C3** | | |
|---|---|---|
| **BCMA_EB B- C1981-C3- aa ScFv domain** | 448 | QVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKVGYDSSGYYRDYYGMDVWGQGTTVTVSSGGGGSGGGG SGGGGSEIVLTQSPGTLSLSPGERATLSCRASQSVSSSYLAWYQQ KPGQAPRLLIYGTSSRATGISDRFSGSGSGTDFTLTISRLEPEDF AVYYCQHYGNSPPKFTFGPGTKLEIK |
| **BCMA_EB B- C1981-C3- nt ScFv domain** | 469 | CAAGTGCAGCTCGTGGAGTCAGGCGGAGGACTGGTGCAGCCCGGG GGCTCCCTGAGACTTTCCTGCGCGGCATCGGGTTTTACCTTCTCC TCCTATGCTATGTCCTGGGTGCGCCAGGCCCCGGGAAAGGACTG GAATGGGTGTCCGCAATCAGCGGTAGCGGGGGCTCAACATACTAC GCCGACTCCGTCAAGGGTCGCTTCACTATTTCCCGGGACAACTCC AAGAATACCCTGTACCTCCAAATGAACAGCCTCAGGGCCGAGGAT ACTGCCGTGTACTACTGCGCCAAAGTCGGATACGATAGCTCCGGT TACTACCGGGACTACTACGGAATGGACGTGTGGGGACAGGGCACC ACCGTGACCGTGTCAAGCGGCGGAGGCGGTTCAGGAGGGGGAGGC TCCGGCGGTGGAGGGTCCGAAATCGTCCTGACTCAGTCGCCTGGC ACTCTGTCGTTGTCCCCGGGGGAGCGCGCTACCCTGTCGTGTCGG GCGTCGCAGTCCGTGTCGAGCTCCTACCTCGCGTGGTACCAGCAG AAGCCCGGACAGGCCCCTAGACTTCTGATCTACGGCACTTCTTCA CGCGCCACCGGGATCAGCGACAGGTTCAGCGGCTCCGGCTCCGGG ACCGACTTCACCCTGACCATTAGCCGGCTGGAGCCTGAAGATTTC GCCGTGTATTACTGCCAACACTACGGAAACTCGCCGCCAAAGTTC |
| | | ACGTTCGGACCCGGAACCAAGCTGGAAATCAAG |
| **BCMA_EB B- C1981-C3- aa VH** | 490 | QVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKVGYDSSGYYRDYYGMDVWGQGTTVTVSS |
| **BCMA_EB B- C1981-C3- aa VL** | 511 | EIVLTQSPGTLSLSPGERATLSCRASQSVSSSYLAWYQQKPGQAP RLLIYGTSSRATGISDRFSGSGSGTDFTLTISRLEPEDFAVYYCQ HYGNSPPKFTFGPGTKLEIK |

| **BCMA_EBB-C1978-G4** | | |
|---|---|---|
| **BCMA_EB B- C1978-G4- aa ScFv domain** | 449 | EVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKMGWSSGYLGAFDIWGQGTTVTVSSGGGGSGGGGSGGG GSEIVLTQSPGTLSLSPGERATLSCRASQSVASSFLAWYQQKPGQ APRLLIYGASGRATGIPDRFSGSGSGTDFTLTISRLEPEDFAVYY CQHYGGSPRLTFGGGTKVDIK |
| **BCMA_EB B- C1978-G4- nt ScFv domain** | 470 | GAAGTCCAACTGGTGGAGTCCGGGGGAGGCTCGTGCAGCCCGGA GGCAGCCTTCGGCTGTCGTGCGCCGCCTCCGGGTTCACGTTCTCA TCCTACGCGATGTCGTGGGTCAGACAGGCACCAGGAAAGGGACTG GAATGGGTGTCCGCCATTAGCGGCTCCGGCGGTAGCACCTACTAT GCCGACTCAGTGAAGGGAAGGTTCACTATCTCCCGCGACAACAGC AAGAACACCCTGTACCTCCAAATGAACTCTCTGCGGGCCGAGGAT ACCGCGGTGTACTATTGCGCCAAGATGGGTTGGTCCAGCGGATAC TTGGGAGCCTTCGACATTTGGGGACAGGGCACTACTGTGACCGTG TCCTCCGGGGGTGGCGGATCGGGAGGCGGCGGCTCGGGTGGAGGG GGTTCCGAAATCGTGTTGACCCAGTCACCGGGAACCCTCTCGCTG TCCCCGGGAGAACGGGCTACACTGTCATGTAGAGCGTCCCAGTCC GTGGCTTCCTCGTTCCTGGCCTGGTACCAGCAGAAGCCGGGACAG GCACCCCGCCTGCTCATCTACGGAGCCAGCGGCCGGGCGACCGGC ATCCCTGACCGCTTCTCCGGTTCCGGCTCGGGCACCGACTTTACT CTGACCATTAGCAGGCTTGAGCCCGAGGATTTTGCCGTGTACTAC TGCCAACACTACGGGGGGAGCCCTCGCCTGACCTTCGGAGGCGGA ACTAAGGTCGATATCAAAA |
| **BCMA_EB B- C1978-G4- aa VH** | 491 | EVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKMGWSSGYLGAFDIWGQGTTVTVSS |
| **BCMA_EB B- C1978-G4- aa VL** | 512 | EIVLTQSPGTLSLSPGERATLSCRASQSVASSFLAWYQQKPGQAP RLLIYGASGRATGIPDRFSGSGSGTDFTLTISRLEPEDFAVYYCQ HYGGSPRLTFGGGTKVDIK |

In embodiments, additional exemplary BCMA CAR constructs are generated using the VH and VL sequences from PCT Publication WO2012/0163805 .

In embodiments, additional exemplary BCMA CAR constructs are generated using the VH and VL sequences from PCT Publication WO02016/014565 .

In embodiments, additional exemplary BCMA CAR constructs are generated using the VH and VL sequences from PCT Publication WO2014/122144 .

In embodiments, additional exemplary BCMA CAR constructs are generated using the CAR molecules, and/or the VH and VL sequences from PCT Publication WO2016/014789 .

In embodiments, additional exemplary BCMA CAR constructs are generated using the CAR molecules, and/or the VH and VL sequences from PCT Publication WO2014/089335 .

In embodiments, additional exemplary BCMA CAR constructs are generated using the CAR molecules, and/or the VH and VL, sequences from PCT Publication WO2014/140248 .

In embodiments, additional exemplary BCMA CAR constructs can also be generated using the VH and VL sequences found in Table 13. The amino acid sequences of exemplary scFv domains comprising the VH and VL domains and a linker sequence, and full-length CARs are also found in Table 13.

**Table 13. Additional exemplary BCMA binding domain sequences**

| **Name** | **Sequence** | **SEQ ID NO:** |
|---|---|---|
| A7D12.2 VH | QIQLVQSGPDLKKPGETVKLSCKASGYTFTNFGMNWVKQAPGKGFKWMAWINTYTGESYFA DDFKGRFAFSVETSATTAYLQINNLKTEDTATYFCARGEIYYGYDGGFAYWGQGTLVTVSA | 555 |
| A7D12.2 VL | DVVMTQSHRFMSTSVGDRVSITCRASQDVNTAVSWYQQKPGQSPKLLIFSASYRYTGVPDR FTGSGSGADFTLTISSVQAEDLAVYYCQQHYSTPWTFGGGTKLDIK | 559 |
| A7D12.2 | QIQLVQSGPDLKKPGETVKLSCKASGYTFTNFGMNWVKQAPGKGFKWMAWINTYTGESYFA DDFKGRFAFSVETSATTAYLQINNLKTEDTATYFCARGEIYYGYDGGFAYWGQGTLVTVSA GGGGSGGGGSGGGGSDVVMTQSHRFMSTSVGDRVSITCRASQDVNTAVSWYQQKPGQSPKL LIFSASYRYTGVPDRFTGSGSGADFTLTISSVQAEDLAVYYCQQHYSTPWTFGGGTKLDIK | 563 |
| scFv domain | | |
| C11D5.3 VH | QIQLVQSGPELKKPGETVKISCKASGYTFTDYSINWVKRAPGKGLKWMGWINTETREPAYA YDFRGRFAFSLETSASTAYLQINNLKYEDTATYFCALDYSYAMDYWGQGTSVTVSS | 556 |
| | | |
| C11D5.3 VL | DIVLTQSPASLAMSLGKRATISCRASESVSVIGAHLIHWYQQKPGQPPKLLIYLASNLETG VPARFSGSGSGTDFTLTIDPVEEDDVAIYSCLQSRIFPRTFGGGTKLEIK | 560 |
| **C11D5.3** | QIQLVQSGPELKKPGETVKISCKASGYTFTDYSINWVKRAPGKGLKWMGWINTETREPAYA YDFRGRFAFSLETSASTAYLQINNLKYEDTATYFCALDYSYAMDYWGQGTSVTVSSGGGGS GGGGSGGGGSQIQLVQSGPELKKPGETVKISCKASGYTFTDYSINWVKRAPGKGLKWMGWI NTETREPAYAYDFRGRFAFSLETSASTAYLQINNLKYEDTATYFCALDYSYAMDYWGQGTS VTVSS | 564 |
| scFv domain | | |
| C12A3.2 VH | QIQLVQSGPELKKPGETVKISCKASGYTFRHYSMNWVKQAPGKGLKWMGRINTESGVPIYA DDFKGRFAFSVETSASTAYLVINNLKDEDTASYFCSNDYLYSLDFWGQGTALTVSS | 557 |
| C12A3.2 VL | DIVLTQSPPSLAMSLGKRATISCRASESVTILGSHLIYWYQQKPGQPPTLLIQLASNVQTG VPARFSGSGSRTDFTLTIDPVEEDDVAVYYCLQSRTIPRTFGGGTKLEIK | 561 |
| **C12A3.2** | QIQLVQSGPELKKPGETVKISCKASGYTFRHYSMNWVKQAPGKGLKWMGRINTESGVPIYA DDFKGRFAFSVETSASTAYLVINNLKDEDTASYFCSNDYLYSLDFWGQGTALTVSSGGGGS GGGGSGGGGSDIVLTQSPPSLAMSLGKRATISCRASESVTILGSHLIYWYQQKPGQPPTLL IQLASNVQTGVPARFSGSGSRTDFTLTIDPVEEDDVAVYYCLQSRTIPRTFGGGTKLEIK | 565 |
| scFv domain | | |
| C13F12. 1 VH | QIQLVQSGPELKKPGETVKISCKASGYTFTHYSMNWVKQAPGKGLKWMGRINTETGEPLYA DDFKGRFAFSLETSASTAYLVINNLKNEDTATFFCSNDYLYSCDYWGQGTTLTVSS | 558 |
| C13F12. 1 VL | DIVLTQSPPSLAMSLGKRATISCRASESVTILGSHLIYWYQQKPGQPPTLLIQLASNVQTG VPARFSGSGSRTDFTLTIDPVEEDDVAVYYCLQSRTIPRTFGGGTKLEIK | 562 |
| **C13F12.1** | QIQLVQSGPELKKPGETVKISCKASGYTFTHYSMNWVKQAPGKGLKWMGRINTETGEPLYA DDFKGRFAFSLETSASTAYLVINNLKNEDTATFFCSNDYLYSCDYWGQGTTLTVSSGGGGS GGGGSGGGGSDIVLTQSPPSLAMSLGKRATISCRASESVTILGSHLIYWYQQKPGQPPTLL IQLASNVQTGVPARFSGSGSRTDFTLTIDPVEEDDVAVYYCLQSRTIPRTFGGGTKLEIK | 566 |
| scFv domain | | |

The sequences of human CDR sequences of the scFv domains are shown in Table 14 for the heavy chain variable domains and in Table 15 for the light chain variable domains. "ID" stands for the respective SEQ ID NO for each CDR. The CDRs are shown according to the Kabat definition, however, the CDRs under other convention, for example, Chothia or the combined Kabat/Chothia definitions may be readily deduced based on the VH and VL sequences above.

**Table 14: Heavy Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **HCDR1** | **ID** | **HCDR2** | **ID** | **HCDR3** | **ID** |
|---|---|---|---|---|---|---|
| 139109 | NHGMS | 694 | GIVYSGSTYYAASV KG | 734 | HGGESDV | 774 |
| 139103 | NYAMS | 684 | GISRSGENTYYADS VKG | 724 | SPAHYYGGMDV | 764 |
| 139105 | DYAMH | 685 | GISWNSGSIGYADSV KG | 725 | HSFLAY | 765 |
| 139111 | NHGMS | 686 | GIVYSGSTYYAASV KG | 726 | HGGESDV | 766 |
| 139100 | NFGIN | 687 | WINPKNNNTNYAQ KFQG | 727 | GPYYYQSYMDV | 767 |
| 139101 | SDAMT | 688 | VISGSGGTTYYADS VKG | 728 | LDSSGYYYARGPRY | 768 |
| 139102 | NYGTT | 689 | WISAYNGNTNYAQ KFQG | 729 | GPYYYYMDV | 769 |
| 139104 | NHGMS | 690 | GIVYSGSTYYAASV KG | 730 | HGGESDV | 770 |
| 139106 | NHGMS | 691 | GIVYSGSTYYAASV KG | 731 | HGGESDV | 771 |
| 139107 | NHGMS | 692 | GIVYSGSTYYAASV KG | 732 | HGGESDV | 772 |
| 139108 | DYYMS | 693 | YISSSGSTIYYADSV KG | 733 | ESGDGMDV | 773 |
| 139110 | DYYMS | 695 | YISSSGNTIYYADSV KG | 735 | STMVREDY | 775 |
| 139112 | NHGMS | 696 | GIVYSGSTYYAASV KG | 736 | HGGESDV | 776 |
| 139113 | NHGMS | 697 | GIVYSGSTYYAASV KG | 737 | HGGESDV | 777 |
| 139114 | NHGMS | 698 | GIVYSGSTYYAASV KG | 738 | HGGESDV | 778 |
| 149362 | SSYYYWG | 699 | SIYYSGSAYYNPSLK S | 739 | HWQEWPDAFDI | 779 |
| 149363 | TSGMCVS | 700 | RIDWDEDKFYSTSL KT | 740 | SGAGGTSATAFDI | 780 |
| 149364 | SYSMN | 701 | SISSSSSYIYYADSVK G | 741 | TIAAVYAFDI | 781 |
| 149365 | DYYMS | 702 | YISSSGSTIYYADSV KG | 742 | DLRGAFDI | 782 |
| 149366 | SHYIH | 703 | MINPSGGVTAYSQT LQG | 743 | EGSGSGWYFDF | 783 |
| 149367 | SGGYYWS | 704 | YIYYSGSTYYNPSLK S | 744 | AGIAARLRGAFDI | 784 |
| 149368 | SYAIS | 705 | GIIPIFGTANYAQKF QG | 745 | RGGYQLLRWDVGLL RSAFDI | 785 |
| 149369 | SNSAAWN | 706 | RTYYRSKWYSFYAI SLKS | 746 | S SPEGLFLYWFDP | 786 |
| BCMA_EBB-C1978-A4 | SYAMS | 707 | AISGSGGSTYYADS VKG | 747 | VEGSGSLDY | 787 |
| BCMA_EBB-C1978-G1 | RYPMS | 708 | GISDSGVSTYYADS AKG | 748 | RAGSEASDI | 788 |
| BCMA_EBB-C1979-C1 | SYAMS | 709 | AISGSGGSTYYADS VKG | 749 | ATYKRELRYYYGM DV | 789 |
| BCMA_EBB-C1978-C7 | SYAMS | 710 | AISGSGGSTYYADS VKG | 750 | ATYKRELRYYYGM DV | 790 |
| BCMA_EBB-C1978-D10 | DYAMH | 711 | GISWNSGSIGYADSV KG | 751 | VGKAVPDV | 791 |
| BCMA_EBB-C1979-C12 | DYAMH | 712 | SINWKGNSLAYGDS | 752 | HOQGVAYYNYAMDV | 792 |
| | | | VKG | | | |
| BCMA_EBB-C1980-G4 | SYAMS | 713 | AISGSGGSTYYADS VKG | 753 | VVRDGMDV | 793 |
| BCMA_EBB-C1980-D2 | SYAMS | 714 | AISGSGGSTYYADS VKG | 754 | IPQTGTFDY | 794 |
| BCMA_EBB-C1978-A10 | SYAMS | 715 | AISGSGGSTYYADS VKG | 755 | ANYKRELRYYYGM DV | 795 |
| BCMA_EBB-C1978-D4 | SYAMS | 716 | AISGSGGSTYYADS VKG | 756 | ALVGATGAFDI | 796 |
| BCMA_EBB-C1980-A2 | SYAMS | 717 | AISGSGGSTYYADS VKG | *757* | WFGEGFDP | 797 |
| BCMA_EBB-C1981-C3 | SYAMS | 718 | AISGSGGSTYYADS VKG | 758 | VGYDSSGYYRDYYG MDV | 798 |
| BCMA_EBB-C1978-G4 | SYAMS | 719 | AISGSGGSTYYADS VKG | 739 | MGWSSGYLGAFDI | 799 |
| A7D12.2 | NFGMN | 720 | WINTYTGESYFADD FKG | 760 | GEIYYGYDGGFAY | 800 |
| C11D5.3 | DYSIN | 721 | WINTETREPAYAYD FRG | 761 | DYSYAMDY | 801 |
| C12A3.2 | HYSMN | 722 | RINTESGVPIYADDF KG | 762 | DYLYSLDF | 802 |
| C13F12.1 | HYSMN | 723 | RINTETGEPLYADDF KG | 763 | DYLYSCDY | 803 |

**Table 15: Light Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **LCDR1** | **ID** | **LCDR2** | **ID** | **LCDR3** | **ID** |
|---|---|---|---|---|---|---|
| 139109 | RASQSISSYLN | 814 | AASSLQS | 854 | QQSYSTPYT | 894 |
| 139103 | RASQSISSSFLA | 804 | GASRRAT | 844 | QQYHSSPSWT | 884 |
| 139105 | RSSQSLLHSNGYNYLD | 805 | LGSNRAS | 845 | MQALQTPYT | 885 |
| 139111 | KSSQSLLRNDGKTPLY | 806 | EVSNRFS | 846 | MONIOFPS | 886 |
| 139100 | RSSQSLLHSNGYNYLN | 807 | LGSKRAS | 84 7 | MQALQTPYT | 887 |
| 139101 | RASQSISSYLN | 808 | GASTLAS | 848 | QQSYKRAS | 888 |
| 139102 | RSSQSLLYSNGYNYVD | 809 | LGSNRAS | 849 | MOQGROQFPYS | 889 |
| 139104 | RASQSVSSNLA | 810 | GASTRAS | 850 | QQYGSSLT | 890 |
| 139106 | RASQSVSSKLA | 811 | GASIRAT | 851 | QQYGSSSWT | 891 |
| 139107 | RASQSVGSTNLA | 812 | DASNRAT | 852 | QQYGSSPPWT | 892 |
| 139108 | RASQSISSYLN | 813 | AASSLQS | 853 | QQSYTLA | 893 |
| 139110 | KSSESLVHNSGKTYLN | 815 | EVSNRDS | 855 | MQGTHWPGT | 895 |
| 139112 | QASEDINKFLN | 816 | DASTLQT | 856 | QQYESLPLT | 896 |
| 139113 | RASQSVGSNLA | 817 | GASTRAT | 857 | QQYNDWLPVT | 897 |
| 139114 | RASQSIGSSSLA | 818 | GASSRAS | 858 | QQYAGSPPFT | 898 |
| 149362 | KASQDIDDAMN | 819 | SATSPVP | 859 | LQHDNFPLT | 899 |
| 149363 | RASQDIYNNLA | 820 | AANKSQS | 860 | QHYYRFPYS | 900 |
| 149364 | RSSQSLLHSNGYNYLD | 821 | LGSNRAS | 861 | MQALQTPYT | 901 |
| 149365 | GGNNTGTKSVH | 822 | DDSVRPS | 862 | QVWDSDSEHV V | 902 |
| 149366 | SGDGLSKKYVS | 823 | RDKERPS | 863 | QAWDDTTVV | 903 |
| 149367 | RASQGIRNWLA | 824 | AASNLQS | 864 | QKYNSAPFT | 904 |
| 149368 | GGNNIGSKSVH | 825 | GKNNRPS | 865 | SSRDSSGDHLR V | 905 |
| 149369 | QGDSLGNYYAT | 826 | GTNNRP S | 866 | NSRDSSGHHLL | 906 |
| BCMA_EBB-C1978-A4 | RASQSVSSA YLA | 827 | GASTRAT | 867 | QHYGSSFNGSS LFT | 907 |
| BCMA_EBB-C1978-G1 | RASQSVSNSLA | 828 | DASSRAT | 868 | QQFGTSSGLT | 908 |
| BCMA_EBB-C1979-C1 | RASQSVSSSFLA | 829 | GASSRAT | 869 | QQYHSSPSWT | 909 |
| BCMA_EBB-C1978-C7 | RASQSVSTTFLA | 830 | GSSNRAT | 870 | QQYHSSPSWT | 910 |
| BCMA_EBB-C1978-D10 | RASQSISSYLN | 831 | AASSLQS | 871 | QQSYSTPYS | 911 |
| BCMA_EBB-C1979-C12 | RATQSIGSSFLA | 832 | GASQRAT | 872 | QHYESSPSWT | 912 |
| BCMA_EBB-C1980-G4 | RASQSVSSSYLA | 833 | GASSRAT | 873 | QQYGSPPRFT | 913 |
| BCMA_EBB-C1980-D2 | RASQSVSSSYLA | 834 | GASSRAT | 874 | QHYGSSPSWT | 914 |
| BCMA_EBB-C1978-A10 | RASQRVASNYLA | 835 | GASSRAT | 875 | QHYDSSPSWT | 915 |
| BCMA_EBB-C1978-D4 | RASQSLSSNFLA | 836 | GASNWAT | 876 | QYYGTSPMYT | 916 |
| BCMA_EBB-C1980-A2 | RSSQSLLHSNGYNYLD | 837 | LGSNRAS | 877 | MQALQTPLT | 917 |
| BCMA_EBB-C1981-C3 | RASQSVSSSYLA | 838 | GTSSRAT | 878 | QHYGNSPPKFT | 918 |
| BCMA_EBB-C1978-G4 | RASQSVASSFLA | 839 | GASGRAT | 879 | QHYGGSPRLT | 919 |
| A7D12.2 | RASQDVNTAVS | 840 | SASYRYT | 880 | QQHYSTPWT | 920 |
| C11D5.3 | RASESVS VIGAHLIH | 841 | LASNLET | 881 | LQSRIFPRT | 921 |
| C12A3.2 | RASESVTILGSHLIY | 842 | LASNVQT | 882 | LQSRTIPRT | 922 |
| C13F12.1 | RASESVTILGSHLIY | 843 | LASNVQT | 883 | LQSRTIPRT | 923 |

In one embodiment, the BCMA binding domain comprises one or more (e.g., all three) light chain complementary determining region 1 (LC CDR1), light chain complementary determining region 2 (LC CDR2), and light chain complementary determining region 3 (LC CDR3) of a BCMA binding domain described herein, e.g., provided in Table 12, 13 or 15, and/or one or more (e.g., all three) heavy chain complementary determining region 1 (HC CDR1), heavy chain complementary determining region 2 (HC CDR2), and heavy chain complementary determining region 3 (HC CDR3) of a BCMA binding domain described herein, e.g., provided in Table 12, 13 or 14. In one embodiment, the BCMA binding domain comprises one, two, or all of LC CDR1, LC CDR2, and LC CDR3 of any amino acid sequences as provided in Table 12 ; and one, two or all of HC CDR1, HC CDR2, and HC CDR3 of any amino acid sequences as provided in Table 12.

In one embodiment, the BCMA antigen binding domain comprises:
(v)
   (a) a LC CDR1 amino acid sequence of SEQ ID NO: 814, a LC CDR2 amino acid sequence of SEQ ID NO: 854, and a LC CDR3 amino acid sequence of SEQ ID NO: 894; and
   (b) a HC CDR1 amino acid sequence of SEQ ID NO: 694, a HC CDR2 amino acid sequence of SEQ ID NO: 734, and a HC CDR3 amino acid sequence of SEQ ID NO: 774
(vi)
   (a) a LC CDR1 amino acid sequence of SEQ **ID** NO: 804, a LC CDR2 amino acid sequence of SEQ ID NO: 844, and a LC CDR3 amino acid sequence of SEQ **ID** NO: 884; and
   (b) a HC CDR1 amino acid sequence of SEQ ID NO: 684, a HC CDR2 amino acid sequence of SEQ ID NO: 724, and a HC CDR3 amino acid sequence of SEQ ID NO: 764
(vii)
   (a) a LC CDR1 amino acid sequence of SEQ ID NO: 805, a LC CDR2 amino acid sequence of SEQ ID NO: 845, and a LC CDR3 amino acid sequence of SEQ ID NO: 885; and
   (b) a HC CDR1 amino acid sequence of SEQ ID NO: 685, a HC CDR2 amino acid sequence of SEQ ID NO: 725, and a HC CDR3 amino acid sequence of SEQ ID NO: 765
(viii)
   (a) a LC CDR1 amino acid sequence of SEQ ID NO: 806, a LC CDR2 amino acid sequence of SEQ ID NO: 846, and a LC CDR3 amino acid sequence of SEQ ID NO: 886; and
   (b) a HC CDR1 amino acid sequence of SEQ ID NO: 686, a HC CDR2 amino acid sequence of SEQ ID NO: 726, and a HC CDR3 amino acid sequence of SEQ ID NO: 766
(ix)
   (a) a LC CDR1 amino acid sequence of SEQ ID NO: 807, a LC CDR2 amino acid sequence of SEQ ID NO: 847, and a LC CDR3 amino acid sequence of SEQ ID NO: 887; and
   (b) a HC CDR1 amino acid sequence of SEQ ID NO: 687, a HC CDR2 amino acid sequence of SEQ ID NO: 727, and a HC CDR3 amino acid sequence of SEQ ID NO: 767
(x)
   (a) a LC CDR1 amino acid sequence of SEQ ID NO: 808, a LC CDR2 amino acid sequence of SEQ ID NO: 848, and a LC CDR3 amino acid sequence of SEQ ID NO: 888; and
   (b) a HC CDR1 amino acid sequence of SEQ ID NO: 688, a HC CDR2 amino acid sequence of SEQ ID NO: 728, and a HC CDR3 amino acid sequence of SEQ ID NO: 768
(xi)
   (a) a LC CDR1 amino acid sequence of SEQ ID NO: 809, a LC CDR2 amino acid sequence of SEQ ID NO: 849, and a LC CDR3 amino acid sequence of SEQ ID NO: 889; and
   (b) a HC CDR1 amino acid sequence of SEQ ID NO: 689, a HC CDR2 amino acid sequence of SEQ ID NO: 729, and a HC CDR3 amino acid sequence of SEQ ID NO: 769
(xii)
   (a) a LC CDR1 amino acid sequence of SEQ ID NO: 810, a LC CDR2 amino acid sequence of SEQ ID NO: 850, and a LC CDR3 amino acid sequence of SEQ ID NO: 890; and
   (b) a HC CDR1 amino acid sequence of SEQ ID NO: 690, a HC CDR2 amino acid sequence of SEQ ID NO: 730, and a HC CDR3 amino acid sequence of SEQ ID NO: 770
(xiii)
   (a) a LC CDR1 amino acid sequence of SEQ ID NO: 811, a LC CDR2 amino acid sequence of SEQ ID NO: 851, and a LC CDR3 amino acid sequence of SEQ ID NO: 891; and
   (b) a HC CDR1 amino acid sequence of SEQ ID NO: 691, a HC CDR2 amino acid sequence of SEQ ID NO: 731, and a HC CDR3 amino acid sequence of SEQ ID NO: 771
(xiv)
   (a) a LC CDR1 amino acid sequence of SEQ ID NO: 812, a LC CDR2 amino acid sequence of SEQ ID NO: 852, and a LC CDR3 amino acid sequence of SEQ ID NO: 892; and
   (b) a HC CDR1 amino acid sequence of SEQ ID NO: 692, a HC CDR2 amino acid sequence of SEQ ID NO: 732, and a HC CDR3 amino acid sequence of SEQ ID NO: 772
(xv)
   (a) a LC CDR1 amino acid sequence of SEQ ID NO: 813, a LC CDR2 amino acid sequence of SEQ ID NO: 853, and a LC CDR3 amino acid sequence of SEQ ID NO: 893; and
   (b) a HC CDR1 amino acid sequence of SEQ ID NO: 693, a HC CDR2 amino acid sequence of SEQ ID NO: 733, and a HC CDR3 amino acid sequence of SEQ ID NO: 773
(xvi)
   (a) a LC CDR1 amino acid sequence of SEQ ID NO: 815, a LC CDR2 amino acid sequence of SEQ ID NO: 855, and a LC CDR3 amino acid sequence of SEQ ID NO: 895; and
   (b) a HC CDR1 amino acid sequence of SEQ ID NO: 695, a HC CDR2 amino acid sequence of SEQ ID NO: 735, and a HC CDR3 amino acid sequence of SEQ ID NO: 775
(xvii)
   (a) a LC CDR1 amino acid sequence of SEQ ID NO: 816, a LC CDR2 amino acid sequence of SEQ ID NO: 856, and a LC CDR3 amino acid sequence of SEQ ID NO: 896; and
   (b) a HC CDR1 amino acid sequence of SEQ ID NO: 696, a HC CDR2 amino acid sequence of SEQ ID NO: 736, and a HC CDR3 amino acid sequence of SEQ ID NO: 776
(xviii)
   (a) a LC CDR1 amino acid sequence of SEQ ID NO: 817, a LC CDR2 amino acid sequence of SEQ ID NO: 857, and a LC CDR3 amino acid sequence of SEQ ID NO: 897; and
   (b) a HC CDR1 amino acid sequence of SEQ ID NO: 697, a HC CDR2 amino acid sequence of SEQ ID NO: 737, and a HC CDR3 amino acid sequence of SEQ ID NO: 777
(xix)
   (a) a LC CDR1 amino acid sequence of SEQ ID NO: 818, a LC CDR2 amino acid sequence of SEQ ID NO: 858, and a LC CDR3 amino acid sequence of SEQ ID NO: 898; and
   (b) a HC CDR1 amino acid sequence of SEQ ID NO: 698, a HC CDR2 amino acid sequence of SEQ ID NO: 738, and a HC CDR3 amino acid sequence of SEQ ID NO: 778
(xx)
   (a) a LC CDR1 amino acid sequence of SEQ ID NO: 819, a LC CDR2 amino acid sequence of SEQ ID NO: 859, and a LC CDR3 amino acid sequence of SEQ ID NO: 899; and
   (b) a HC CDR1 amino acid sequence of SEQ ID NO: 699, a HC CDR2 amino acid sequence of SEQ ID NO: 739, and a HC CDR3 amino acid sequence of SEQ ID NO: 779
(xxi)
   (a) a LC CDR1 amino acid sequence of SEQ ID NO: 820, a LC CDR2 amino acid sequence of SEQ ID NO: 860, and a LC CDR3 amino acid sequence of SEQ ID NO: 900; and
   (b) a HC CDR1 amino acid sequence of SEQ ID NO: 700, a HC CDR2 amino acid sequence of SEQ ID NO: 740, and a HC CDR3 amino acid sequence of SEQ ID NO: 780
(xxii)
   (a) a LC CDR1 amino acid sequence of SEQ ID NO: 821, a LC CDR2 amino acid sequence of SEQ ID NO: 861, and a LC CDR3 amino acid sequence of SEQ ID NO: 901; and
   (b) a HC CDR1 amino acid sequence of SEQ ID NO: 701, a HC CDR2 amino acid sequence of SEQ ID NO: 741, and a HC CDR3 amino acid sequence of SEQ ID NO: 781
(xxiii)
   (a) a LC CDR1 amino acid sequence of SEQ ID NO: 822, a LC CDR2 amino acid sequence of SEQ ID NO: 862, and a LC CDR3 amino acid sequence of SEQ ID NO: 902; and
   (b) a HC CDR1 amino acid sequence of SEQ ID NO: 702, a HC CDR2 amino acid sequence of SEQ ID NO: 742, and a HC CDR3 amino acid sequence of SEQ ID NO: 782
(xxiv)
   (a) a LC CDR1 amino acid sequence of SEQ ID NO: 823, a LC CDR2 amino acid sequence of SEQ ID NO: 863, and a LC CDR3 amino acid sequence of SEQ ID NO: 903; and
   (b) a HC CDR1 amino acid sequence of SEQ ID NO: 703, a HC CDR2 amino acid sequence of SEQ ID NO: 743, and a HC CDR3 amino acid sequence of SEQ ID NO: 783
(xxv)
   (a) a LC CDR1 amino acid sequence of SEQ ID NO: 824, a LC CDR2 amino acid sequence of SEQ ID NO: 864, and a LC CDR3 amino acid sequence of SEQ ID NO: 904; and
   (b) a HC CDR1 amino acid sequence of SEQ ID NO: 704, a HC CDR2 amino acid sequence of SEQ ID NO: 744, and a HC CDR3 amino acid sequence of SEQ ID NO: 784
(xxvi)
   (a) a LC CDR1 amino acid sequence of SEQ ID NO: 825, a LC CDR2 amino acid sequence of SEQ ID NO: 865, and a LC CDR3 amino acid sequence of SEQ ID NO: 905; and
   (b) a HC CDR1 amino acid sequence of SEQ ID NO: 705, a HC CDR2 amino acid sequence of SEQ ID NO: 745, and a HC CDR3 amino acid sequence of SEQ ID NO: 785 or
(xxvii)
   (a) a LC CDR1 amino acid sequence of SEQ ID NO: 826, a LC CDR2 amino acid sequence of SEQ ID NO: 866, and a LC CDR3 amino acid sequence of SEQ ID NO: 906; and
   (b) a HC CDR1 amino acid sequence of SEQ ID NO: 706, a HC CDR2 amino acid sequence of SEQ ID NO: 746, and a HC CDR3 amino acid sequence of SEQ ID NO: 786.

In one embodiment, the BCMA binding domain comprises a light chain variable region described herein (e.g., in Table 12 or 13) and/or a heavy chain variable region described herein (e.g., in Table 12 or 13). In one embodiment, the BCMA binding domain is a scFv comprising a light chain and a heavy chain of an amino acid sequence listed in Table 12 or 13. In an embodiment, the BCMA binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 12 or 13, or a sequence with 95-99% identity with an amino acid sequence provided in Table 12 or 13; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 12 or 13, or a sequence with 95-99% identity to an amino acid sequence provided in Table 12 or 13.

In one embodiment, the BCMA binding domain comprises an amino acid sequence selected from a group consisting of SEQ ID NO: 349; SEQ ID NO: 339, SEQ ID NO: 340; SEQ ID NO: 341; SEQ ID NO: 342; SEQ ID NO: 343; SEQ ID NO: 344, SEQ ID NO: 345, SEQ ID NO: 346, SEQ ID NO: 347, SEQ ID NO: 348, SEQ ID NO: 350, SEQ ID NO: 351, SEQ ID NO: 352, SEQ ID NO: 353, SEQ ID NO: 429, SEQ ID NO: 430, SEQ ID NO: 431, SEQ ID NO: 432, SEQ ID NO: 433, SEQ ID NO: 434, SEQ ID NO: 435, SEQ ID NO: 436, SEQ ID NO: 437, SEQ ID NO: 438, SEQ ID NO: 439, SEQ ID NO: 440, SEQ ID NO: 441, SEQ ID NO: 442, SEQ ID NO: 443, SEQ ID NO: 444, SEQ ID NO: 445, SEQ ID NO: 446, SEQ ID NO: 447, SEQ ID NO: 448, SEQ ID NO: 449, SEQ ID NO: 563, SEQ ID NO: 564, SEQ ID NO: 565 and SEQ ID NO: 566; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the BCMA binding domain is a scFv, and a light chain variable region comprising an amino acid sequence described herein, e.g., in Table 12 or 13, is attached to a heavy chain variable region comprising an amino acid sequence described herein, e.g., in Table 12 or 13, via a linker, e.g., a linker described herein. In one embodiment, the BCMA binding domain includes a (Gly4-Ser)n linker, wherein n is 1, 2, 3, 4, 5, or 6, preferably 4 (SEQ ID NO: 80). The light chain variable region and heavy chain variable region of a scFv can be, e.g., in any of the following orientations: light chain variable region-linker-heavy chain variable region or heavy chain variable region-linker-light chain variable region.

Any known BCMA CAR, e.g., the BMCA antigen binding domain of any known BCMA CAR, in the art can be used in accordance with the instant invention to construct a BCA CAR. For example, those described herein. As another example, the BCMA CAR comprises an anti-BCMA binding domain or portion thereof, e.g., CDRs, of a CAR or antigen binding domain described in, e.g., WO2016/09-4304, WO2016/014789, or US9,034,324 (e.g., C11D5 of US9,034,324) .

In one embodiment, an antigen binding domain against ROR1 is an antigen binding portion, e.g., CDRs, of an antibody described in, e.g., Hudecek et al., Clin Cancer Res 19(12):3153-3164 (2013); WO 2011159847; and US20130101607.

In one embodiment, an antigen binding domain against CD22 is an antigen binding portion, e.g., CDRs, of an antibody described in, e.g., Haso et al., Blood, 121(7): 1165-1174 (2013); Wayne et al., Clin Cancer Res 16(6): 1894-1903 (2010); Kato et al., Leuk Res 37(1):83-88 (2013); Creative BioMart (creativebiomart.net): MOM-18047-S(P). In an aspect, an antigen binding domain against CD22 is an antigen binding portion, e.g., CDRs, VL and VH, or scFV, of an antigen binding domain or CAR described in, e.g., WO2016/164731 (e.g., as described in Table 6A of WO2016/164731).

In one embodiment, an antigen binding domain against CD20 is an antigen binding portion, e.g., CDRs, of the antibody Rituximab, Ofatumumab, Ocrelizumab, Veltuzumab, or GA101, or derivatives thereof. In an aspect, an antigen binding domain against CD20 is an antigen binding portion, e.g., CDRs, VL and VH, or scFV, of an antigen binding domain or CAR described in, e.g., WO2016/164731 (e.g., as described in Table 11A or 11B of WO2016/164731).

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody that binds a tumor antigen or a B cell antigen listed above. In one embodiment, the antigen binding domain comprises a heavy chain variable region and/or a variable light chain region of an antibody that binds a tumor antigen or a B cell antigen listed above.

In one embodiment, the antigen binding domain of a CAR, e.g., a TA CAR and/or a BCA CAR, described herein is a scFv antibody fragment. In one aspect, such antibody fragments are functional in that they retain the equivalent binding affinity, e.g., they bind the same antigen with comparable efficacy, as the IgG antibody from which it is derived. In other embodiments, the antibody fragment has a lower binding affinity, e.g., it binds the same antigen with a lower binding affinity than the antibody from which it is derived, but is functional in that it provides a biological response described herein. In one embodiment, the CAR molecule comprises an antibody fragment that has a binding affinity K_{D} of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the antibody fragment has a binding affinity that is at least five-fold, 10-fold, 20-fold, 30-fold, 50-fold, 100-fold or 1,000-fold less than a reference antibody, e.g., an antibody described herein.

In one embodiment, the antigen binding domain comprises a non-human antibody or antibody fragment, e.g., a mouse antibody or antibody fragment.

In another embodiment, the antigen binding domain comprises a humanized antibody or an antibody fragment. In some aspects, a non-human antibody is humanized, where specific sequences or regions of the antibody are modified to increase similarity to an antibody naturally produced in a human or fragment thereof. In one aspect, the antigen binding domain is humanized compared to the murine sequence of the antibody or antibody fragment, e.g., scFv, from which it is derived.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089 ), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973 ), chain shuffling (see, e.g., U.S. Pat. No. 5,565,332 ), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994) . Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323 ).

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and coworkers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567, 6,331,415; 5,225,539; 5,530,101; 5,585,089; 6,548,640 ).

In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987).

Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993)).

In some embodiments, the framework region, e.g., all four framework regions, of the heavy chain variable region are derived from a VH4_4-59 germline sequence. In one embodiment, the framework region can comprise, one, two, three, four or five modifications, e.g., substitutions, e.g., from the amino acid at the corresponding murine sequence. In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., from the amino acid at the corresponding murine sequence.

In some aspects, the portion of a CAR disclosed herein, e.g., a TA CAR and/or a BCA CAR described herein, that comprises an antibody fragment is humanized with retention of high affinity for the target antigen and other favorable biological properties. According to one aspect , humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present disclosure, the ability to bind human a tumor antigen as described herein. In some embodiments, a humanized antibody or antibody fragment may have improved affinity and/or specificity of binding to a tumor antigen as described herein or a B cell antigen as described herein. In some embodiments, a humanized antibody or antibody fragment may have lower affinity and/or specificity of a tumor antigen as described herein or a B cell antigen as described herein.

In one aspect, the antigen binding domain is characterized by particular functional features or properties of an antibody or antibody fragment. For example, in one aspect, the portion of a CAR disclosed herein that comprises an antigen binding domain specifically binds a tumor antigen as described herein or a B cell antigen as described herein.

In one aspect, the antigen binding domain is a fragment, e.g., a single chain variable fragment (scFv). In one aspect, the anti- tumor antigen as described herein binding domain is a Fv, a Fab, a (Fab')2, or a bi-functional (e.g. bi-specific) hybrid antibody (e.g., Lanzavecchia et al., Eur. J. Immunol. 17, 105 (1987)). In one aspect, the antibodies and fragments thereof binds a tumor antigen as described herein protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715 .

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:22). In one embodiment, the linker can be (Gly₄Ser)₄ (SEQ ID NO:29) or (Gly₄Ser)₃(SEQ ID NO:30). Variation in the linker length may retain or enhance activity, giving rise to superior efficacy in activity studies.

In another aspect, the antigen binding domain is a T cell receptor ("TCR"), an engineered TCR, or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012) . For example, scTCR can be engineered that contains the Vα and Vβ genes from a T cell clone linked by a linker (e.g., a flexible peptide). This approach is very useful to cancer associated target that itself is intracellular, however, a fragment of such antigen (peptide) is presented on the surface of the cancer cells by MHC.

In one aspect, the antigen binding domain of the CAR comprises an amino acid sequence that is homologous to an antigen binding domain amino acid sequence described herein, and the antigen binding domain retains the desired functional properties of the antigen binding domain described herein.

In one specific aspect, the CAR composition of the invention comprises an antibody fragment. In a further aspect, the antibody fragment comprises a scFv. In a further aspect, the antibody fragment comprises a variable heavy chain (VH) only.

In various aspects, the antigen binding domain of the CAR is engineered by modifying one or more amino acids within one or both variable regions (e.g., VH and/or VL), for example within one or more CDR regions and/or within one or more framework regions. In one specific aspect, the CAR comprises an antibody fragment. In a further aspect, the antibody fragment comprises an scFv.

It will be understood by one of ordinary skill in the art that the antibody or antibody fragment may further be modified such that they vary in amino acid sequence (e.g., from wild-type), but not in desired activity. For example, additional nucleotide substitutions leading to amino acid substitutions at "non-essential" amino acid residues may be made to the protein. For example, a nonessential amino acid residue in a molecule may be replaced with another amino acid residue from the same side chain family. In another embodiment, a string of amino acids can be replaced with a structurally similar string that differs in order and/or composition of side chain family members, e.g., a conservative substitution, in which an amino acid residue is replaced with an amino acid residue having a similar side chain, may be made.

Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Percent identity in the context of two or more nucleic acids or polypeptide sequences, refers to two or more sequences that are the same. Two sequences are "substantially identical" if two sequences have a specified percentage of amino acid residues or nucleotides that are the same (e.g., 60% identity, optionally 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

In one aspect, the present disclosure contemplates modifications of the starting antibody or fragment (e.g., scFv) amino acid sequence that generate functionally equivalent molecules. For example, the VH or VL of an antigen binding domain to -a tumor antigen described herein, e.g., scFv, comprised in the CAR can be modified to retain at least about 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity of the starting VH or VL framework region of the antigen binding domain to the tumor antigen described herein, e.g., scFv. The present disclosure contemplates modifications of the entire CAR construct, e.g., modifications in one or more amino acid sequences of the various domains of the CAR construct in order to generate functionally equivalent molecules. The CAR construct can be modified to retain at least about 70%, 71%. 72%. 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity of the starting CAR construct.

### Bispecific CARs

Disclosed herein is a multispecific antibody molecule that is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an instance the first and second epitopes are on the same antigen, e.g., the same protein (or subunit of a multimeric protein). In an instance the first and second epitopes overlap. In an instance the first and second epitopes do not overlap. In an instance the first and second epitopes are on different antigens, e.g., different proteins (or different subunits of a multimeric protein). In an instance a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an instance a bispecific antibody molecule comprises a half antibody having binding specificity for a first epitope and a half antibody having binding specificity for a second epitope. In an instance a bispecific antibody molecule comprises a half antibody, or fragment thereof, having binding specificity for a first epitope and a half antibody, or fragment thereof, having binding specificity for a second epitope. In an instance a bispecific antibody molecule comprises a scFv, or fragment thereof, have binding specificity for a first epitope and a scFv, or fragment thereof, have binding specificity for a second epitope.

In certain instances, the antibody molecule is a multi-specific (*e.g.,* a bispecific or a trispecific) antibody molecule. Protocols for generating bispecific or heterodimeric antibody molecules are known in the art; including but not limited to, for example, the "knob in a hole" approach described in, *e.g.,* US 5731168; the electrostatic steering Fc pairing as described in, *e.g.,* WO 09/089004, WO 06/106905 and WO 2010/129304; Strand Exchange Engineered Domains (SEED) heterodimer formation as described in, *e.g.,* WO 07/110205; Fab arm exchange as described in, *e.g.,* WO 08/119353, WO 2011/131746, and WO 2013/060867; double antibody conjugate, *e.g.,* by antibody cross-linking to generate a bi-specific structure using a heterobifunctional reagent having an amine-reactive group and a sulfhydryl reactive group as described in, *e.g.,* US 4433059; bispecific antibody determinants generated by recombining half antibodies (heavy-light chain pairs or Fabs) from different antibodies through cycle of reduction and oxidation of disulfide bonds between the two heavy chains, as described in, *e.g.,* US 4444878; trifunctional antibodies, *e.g.,* three Fab' fragments cross-linked through sulfhdryl reactive groups, as described in, *e.g.,* US5273743; biosynthetic binding proteins, *e.g.,* pair of scFvs cross-linked through C-terminal tails preferably through disulfide or amine-reactive chemical cross-linking, as described in, *e.g.,* US5534254; bifunctional antibodies, *e.g.,* Fab fragments with different binding specificities dimerized through leucine zippers (*e.g.,* c-fos and c-jun) that have replaced the constant domain, as described in, *e.g.,* US5582996; bispecific and oligospecific mono-and oligovalent receptors, *e.g.,* VH-CH1 regions of two antibodies (two Fab fragments) linked through a polypeptide spacer between the CH1 region of one antibody and the VH region of the other antibody typically with associated light chains, as described in, *e.g.,* US5591828; bispecific DNA-antibody conjugates, *e.g.,* crosslinking of antibodies or Fab fragments through a double stranded piece of DNA, as described in, *e.g.,* US5635602; bispecific fusion proteins, *e.g.,* an expression construct containing two scFvs with a hydrophilic helical peptide linker between them and a full constant region, as described in, *e.g.,* US5637481; multivalent and multispecific binding proteins, *e.g.,* dimer of polypeptides having first domain with binding region of Ig heavy chain variable region, and second domain with binding region of Ig light chain variable region, generally termed diabodies (higher order structures are also encompassed creating for bispecifc, trispecific, or tetraspecific molecules, as described in, *e.g.,* US5837242; minibody constructs with linked VL and VH chains further connected with peptide spacers to an antibody hinge region and CH3 region, which can be dimerized to form bispecific/multivalent molecules, as described in, *e.g.,* US5837821; VH and VL domains linked with a short peptide linker (*e.g.,* 5 or 10 amino acids) or no linker at all in either orientation, which can form dimers to form bispecific diabodies; trimers and tetramers, as described in, *e.g.,* US5844094; String of VH domains (or VL domains in family members) connected by peptide linkages with crosslinkable groups at the C-terminus futher associated with VL domains to form a series of FVs (or scFvs), as described in, *e.g.,* US5864019; and single chain binding polypeptides with both a VH and a VL domain linked through a peptide linker are combined into multivalent structures through non-covalent or chemical crosslinking to form, *e.g*., homobivalent, heterobivalent, trivalent, and tetravalent structures using both scFV or diabody type format, as described in, *e.g.,* US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004-352A1, US2005069552A1, US2005079170A1, US2005100543A1, US2005136049A1, US2005136051A1, US2005163782A1, US2005260425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1.

Within each antibody or antibody fragment (e.g., scFv) of a bispecific antibody molecule, the VH can be upstream or downstream of the VL. In some instances, the upstream antibody or antibody fragment (e.g., scFv) is arranged with its VH (VH₁) upstream of its VL (VL₁) and the downstream antibody or antibody fragment (e.g., scFv) is arranged with its VL (VL₂) upstream of its VH (VH₂), such that the overall bispecific antibody molecule has the arrangement VH₁-VL₁-VL₂-VH₂. In other instances, the upstream antibody or antibody fragment (e.g., scFv) is arranged with its VL (VL₁) upstream of its VH (VH₁) and the downstream antibody or antibody fragment (e.g., scFv) is arranged with its VH (VH₂) upstream of its VL (VL₂), such that the overall bispecific antibody molecule has the arrangement VL₁-VH₁-VH₂-VL₂. Optionally, a linker is disposed between the two antibodies or antibody fragments (e.g., scFvs), e.g., between VL₁ and VL₂ if the construct is arranged as VH₁-VL₁-VL₂-VH₂, or between VH₁ and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 80). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some instances, a bispecific CAR comprises VLs, VHs, and optionally one or more linkers in an arrangement as described herein.

In one aspect, the disclosure provides a chimeric antigen receptor comprising a bispecific antigen binding domain, a transmembrane domain (e.g., as described herein), and an intracellular signaling domain (e.g., as described herein). In instances, the bispecific antigen binding domain comprises a first immunoglobulin variable domain sequence, e.g., an scFv (or comprises the light chain CDRs and/or heavy chain CDRs from a scFv described herein), which binds a B-cell antigen, e.g., as described herein, e.g., (a CD19 binding domain or BCMA binding domain described herein, e.g., in Table 6 or Table 12), and a second immunoglobulin variable domain sequence, e.g., a scFv (or comprises the light chain CDRs and/or heavy chain CDRs from a scFv described herein), which has binding specificity for one or more tumor antigens described herein, e.g., a solid tumor antigen, e.g., comprises a scFv as described herein, e.g., comprising a mesothelin binding domain or EGFRvIII binding domain (e.g., as described in Table 2 or Table 5). In instances, the bispecific antigen binding domain comprises a CD19 binding domain described herein and a mesothelin binding domain described herein. In instances, the bispecific antigen binding domain comprises a BCMA binding domain described herein and a mesothelin binding domain described herein. In instances, the bispecific antigen binding domain comprises a CD19 binding domain described herein and a EGFRvIII binding domain described herein. In instances, the bispecific antigen binding domain comprises a BCMA binding domain described herein and a EGFRvIII binding domain described herein. In another aspect, the disclosure provides a cell (e.g., a population of cells), e.g., an immune effector cell, e.g., a T cell or NK cell, e.g., as described herein, which is engineered to express (e.g., comprises) a bispecific CAR as described herein, e.g., a bispecific CAR comprising a B-cell antigen binding domain described herein and a tumor antigen (e.g., a solid tumor antigen) described herein. Without being bound by any theory, it is believed that cells expressing such bispecific CARs (e.g., comprising a B-cell antigen binding domain, e.g., as described herein, and a tumor antigen binding domain, e.g., as described herein) are useful in the methods and compositions described herein.

### Chimeric TCR

In one aspect, the antigen binding domains described herein, e.g., the antibodies and antibody fragments, e.g., provided in the Tables herein, can be grafted to one or more constant domain of a T cell receptor ("TCR") chain, for example, a TCR alpha or TCR beta chain, to create an chimeric TCR that binds specificity to a tumor antigen or B cell antigendescribed herein. Without being bound by theory, it is believed that chimeric TCRs will signal through the TCR complex upon antigen binding. For example, a mesothelin or CD19 scFv or a fragment there of, e.g., a VL domain, or VH domain, as disclosed herein, can be grafted to the constant domain, e.g., at least a portion of the extracellular constant domain, the transmembrane domain and the cytoplasmic domain, of a TCR chain, for example, the TCR alpha chain and/or the TCR beta chain. As another example, the CDRs of an antibody or antibody fragment, e.g., the CDRs of anyantibody or antibody fragment as described in Tables provided herein may be grafted into a TCR alpha and/or beta chain to create a chimeric TCR that binds specifically to a tumor antigen or a B cell antigen described herein. For example, the LCDRs disclosed herein may be grafted into the variable domain of a TCR alpha chain and the HCDRs disclosed herein may be grafted to the variable domain of a TCR beta chain, or vice versa. Such chimeric TCRs may be produced by methods known in the art (For example, Willemsen RA et al, Gene Therapy 2000; 7: 1369-1377; Zhang T et al, Cancer Gene Ther 2004; 11: 487-496; Aggen et al, Gene Ther. 2012 Apr;19(4):365-74).

### Transmembrane domain

With respect to the transmembrane domain, in various embodiments, a CAR, e.g., a TA CAR and/or a BCA CAR, can be designed to comprise a transmembrane domain that is attached to the extracellular domain of the CAR, e.g., the antigen binding domain. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR, for example, the transmembrane domain is from the same protein as the intracellular signalling domain, e.g., the costimulatory domain. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect, the amino acid sequence of the transmembrane domain may be modified or substituted so as to minimize interactions with the binding domains of the native binding partner present in the same CAR-expressing cell.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this invention may include at least the transmembrane region(s) of e.g., the alpha, beta or zeta chain of the T-cell receptor, CD28, CD27, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge, e.g., an IgG4 hinge, or a CD8a hinge. In one embodiment, the hinge or spacer comprises (e.g., consists of) the amino acid sequence of SEQ ID NO:4. In one aspect, the transmembrane domain comprises (e.g., consists of) a transmembrane domain of SEQ ID NO: 12.

In one aspect, the hinge or spacer comprises an IgG4 hinge. For example, in one embodiment, the hinge or spacer comprises a hinge of the amino acid sequence SEQ ID NO: 6. In some embodiments, the hinge or spacer comprises a hinge encoded by a nucleotide sequence of SEQ ID NO: 7. In one aspect, the hinge or spacer comprises an IgD hinge. For example, in one embodiment, the hinge or spacer comprises a hinge of the amino acid sequence SEQ ID NO: 8. In some embodiments, the hinge or spacer comprises a hinge encoded by a nucleotide sequence of SEQ ID NO: 9.

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO: 10). In some embodiments, the linker is encoded by a nucleotide sequence of GGTGGCGGAGGTTCTGGAGGTGGAGGTTCC (SEQ ID NO: 11).

In one aspect, the hinge or spacer comprises a KIR2DS2 hinge.

### Cytoplasmic domain

The cytoplasmic domain or region of the CAR, e.g., the TA CAR and/or the BCA CAR, includes an intracellular signaling domain. An intracellular signaling domain is generally responsible for activation of at least one of the normal effector functions of the immune cell in which the CAR has been introduced. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

Examples of intracellular signaling domains for use in the CAR disclosed herein include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are insufficient for full activation of the T cell and that a secondary and/or costimulatory signal is also required. Thus, T cell activation can be said to be mediated by two distinct classes of cytoplasrnic signaling sequences: those that initiate antigen-dependent primary activation through the TCR (primary intracellular signaling domains) and those that act in an antigen-independent manner to provide a secondary or costimulatory signal (secondary cytoplasmic domain, e.g., a costimulatory domain).

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the invention include those of TCR zeta, FcR gamma, FcR beta, CD3 gamma, CD3 delta , CD3 epsilon, CD5, CD22, CD79a, CD79b, CD278 (also known as "ICOS"), FcεRI, DAP10, DAP12,and CD66d. In one instance, a CAR disclosed herein comprises an intracellular signaling domain, e.g., a primary signaling domain of CD3-zeta, e.g., a CD3-zeta sequence described herein. In the invention, the intracellular domain of the second CAR comprises a primary signaling domain.

In one embodiment, a primary signaling domain comprises a modified IT AM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

The intracellular signaling domain of the CAR can comprise the CD3-zeta signaling domain by itself or it can be combined with any other desired intracellular signaling domain(s) useful in the context of a CAR disclosed herein. For example, the intracellular signaling domain of the CAR can comprise a CD3 zeta chain portion and a costimulatory signaling domain. The costimulatory signaling domain refers to a portion of the CAR comprising the intracellular domain of a costimulatory molecule. A costimulatory molecule is a cell surface molecule other than an antigen receptor or its ligands that is required for an efficient response of lymphocytes to an antigen. Examples of such molecules include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83, and the like. For example, CD27 costimulation has been demonstrated to enhance expansion, effector function, and survival of human CART cells in vitro and augments human T cell persistence and antitumor activity in vivo (Song et al. Blood. 2012; 119(3):696-706). Further examples of such costimulatory molecules include an MHC class I molecule, a TNF receptor protein, an Immunoglobulin-like protein, a cytokine receptor, an integrin, a signaling lymphocytic activation molecule (SLAM protein), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

The intracellular signaling sequences within the cytoplasmic portion of the CAR disclosed herein may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequence. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 14. In one aspect, the signaling domain of CD3-zeta is a signaling domain of SEQ ID NO: 18.

In one aspect, the intracellular signaling domain is designed to comprise the signaling domain of CD3-zeta and the signaling domain of CD27. In one aspect, the signaling domain of CD27 comprises an amino acid sequence of SEQ ID NO:16. In one aspect, the signalling domain of CD27 is encoded by a nucleic acid sequence of SEQ ID NO:17.

In one aspect, the intracellular is designed to comprise the signaling domain of CD3-zeta and the signaling domain of CD28. In one aspect, the signaling domain of CD28 comprises an amino acid sequence of SEQ ID NO: 44. In one aspect, the signaling domain of CD28 is encoded by a nucleic acid sequence of SEQ ID NO: 45.

In one aspect, the intracellular is designed to comprise the signaling domain of CD3-zeta and the signaling domain of ICOS. In one aspect, the signaling domain of ICOS comprises an amino acid sequence of SEQ ID NO: 42. In one aspect, the signaling domain of ICOS is encoded by a nucleic acid sequence of SEQ ID NO: 43.

In one aspect, the cell of the disclosure, e.g., described herein, e.g., a cell expressing both a TA CAR and a BCA CAR, includes a TA CAR that includes an antigen binding domain that binds a target tumor antigen described herein, a transmembrane domain, a primary signaling domain, and a costimulatory signaling domain, and a BCA CAR that includes an antigen binding domain that binds a target B-Cell antigen described herein, a transmembrane domain, a primary signaling domain, and a costimulatory signaling domain. In other aspects, the cell of the disclosure, e.g., described herein, e.g., a cell expressing both a TA CAR and a BCA CAR, includes a TA CAR that includes an antigen binding domain that binds a target tumor antigen described herein, a transmembrane domain, a primary signaling domain, and a costimulatory signaling domain, and a BCA CAR that includes an antigen binding domain that binds a target B-Cell antigen described herein, a transmembrane domain, and a costimulatory signaling domain, but does not include a primary signaling domain. Without being bound by theory, it is believed that providing a BCA CAR comprising a costimulatory signaling domain, but not a primary signaling domain, may allow the cell to persist and or proliferate in response to circulating B cells, but may minimize the cytotoxicity against said B cells.

In one aspect, the CAR-expressing cell described herein, e.g. a cell expressing both a TA CAR and a BCA CAR can further comprise another TA CAR, e.g., another TA CAR that includes a different antigen binding domain, e.g., to the same target or a different target (e.g., a target other than a tumor antigen described herein or a different tumor antigen described herein). For example, in an embodiment where the cell of the invention expresses a second TA CAR, the second TA CAR includes an antigen binding domain to a target expressed the same cancer cell type as the tumor antigen targeted by the first TA CAR. In one instance, the CAR-expressing cell comprises a first TA CAR that targets a first tumor antigen and includes an intracellular signaling domain having a costimulatory signaling domain but not a primary signaling domain, and a second TA CAR that targets a second, different, tumor antigen and includes an intracellular signaling domain having a primary signaling domain but not a costimulatory signaling domain. While not wishing to be bound by theory, placement of a costimulatory signaling domain, e.g., 4-1BB, CD28, CD27 or OX-40, onto the first TA CAR, and the primary signaling domain, e.g., CD3 zeta, on the second TA CAR can limit the CAR activity to cells where both targets are expressed. In one instance, the cell comprises a first tumor antigen (TA) CAR that includes an antigen binding domain that binds a target antigen described herein, a transmembrane domain and a costimulatory domain and a second TA CAR that targets a different target antigen (e.g., an antigen expressed on that same cancer cell type as the first target antigen) and includes an antigen binding domain, a transmembrane domain and a primary signaling domain. In another instance, the cell comprises (i.e., is genetically engineered to express) a first TA CAR that includes an antigen binding domain that binds a target antigen described herein, a transmembrane domain and a primary signaling domain and a second TA CAR that targets a tumor antigen other than the first target antigen (e.g., an antigen expressed on the same cancer cell type as the first target antigen) and includes an antigen binding domain to the antigen, a transmembrane domain and a costimulatory signaling domain. In another instance, the cell comprises (i.e., is genetically engineered to express) a first TA CAR that includes an antigen binding domain that binds a target antigen described herein, a transmembrane domain, a costimulatory signaling domain and a primary signaling domain, and a second TA CAR that targets a tumor antigen other than the first target antigen (e.g., an antigen expressed on the same cancer cell type as the first target antigen) and includes an antigen binding domain to the antigen, a transmembrane domain, a costimulatory signaling domain and a primary signaling domain. In instances where both the first and second TA CAR include a costimulatory signaling domain, the costimulatory signaling domain of the first TA CAR and the second TA CAR may be derived from the same protein, e.g., from a costimulatory protein described herein, e.g., 4-1BB, CD28, or ICOS. In other instances, the costimulatory signaling domain of the first TA CAR and the second TA CAR may be derived from the different proteins, e.g., the first TA CAR includes a costimulatory signaling domain described herein, e.g., of 4-1BB, and the second TA CAR includes a different costimulatory signaling domain described herein, e.g., of CD28. The cell of the invention comprises a TA CAR, wherein the antigen binding domain binds to a tumor antigen other than a B-Cell antigen, wherein the tumor antigen is a solid tumor antigen, and wherein the intracellular signaling domain of said TA CAR comprises a costimulatory signaling domain and a primary signaling domain.

In one instance, the CAR-expressing cell comprises a TA CAR described herein, a BCA CAR described herein, and an inhibitory CAR. In one instance, the inhibitory CAR comprises an antigen binding domain that binds an antigen found on normal cells but not cancer cells, e.g., normal cells that also express the tumor antigen targeted by the TA CAR. In one instance, the inhibitory CAR comprises the antigen binding domain, a transmembrane domain and an intracellular domain of an inhibitory molecule. For example, the intracellular domain of the inhibitory CAR can be an intracellular domain of PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, or TGF beta.

In one embodiment, the antigen binding domains of the different CARs (e.g., of the TA CAR and of the BCA CAR) can be such that the antigen binding domains do not interact with one another. For example, a cell expressing a first and second CAR can have an antigen binding domain of the first CAR, e.g., as a fragment, e.g., an scFv, that does not form an association with the antigen binding domain of the second CAR, e.g., the antigen binding domain of the second CAR is a VHH.

In some embodiments, the antigen binding domain comprises a single domain antigen binding (SDAB) molecules include molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies. SDAB molecules may be any of the art, or any future single domain molecules. SDAB molecules may be derived from any species including, but not limited to mouse, human, camel, llama, lamprey, fish, shark, goat, rabbit, and bovine. This term also includes naturally occurring single domain antibody molecules from species other than Camelidae and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from Camelidae species, for example in camel, llama, dromedary, alpaca and guanaco. Other species besides Camelidae may produce heavy chain molecules naturally devoid of light chain; such VHHs are within the scope of the disclosure.

The SDAB molecules can be recombinant, CDR-grafted, humanized, camelized, de-immunized and/or in vitro generated (e.g., selected by phage display).

It has also been discovered, that cells having a plurality of chimeric membrane embedded receptors comprising an antigen binding domain that interactions between the antigen binding domain of the receptors can be undesirable, e.g., because it inhibits the ability of one or more of the antigen binding domains to bind its cognate antigen. Accordingly, disclosed herein are cells having a first and a second non-naturally occurring chimeric membrane embedded receptor comprising antigen binding domains that minimize such interactions. Also disclosed herein are nucleic acids encoding a first and a second non-naturally occurring chimeric membrane embedded receptor comprising a antigen binding domains that minimize such interactions, as well as methods of making and using such cells and nucleic acids. In an embodiment the antigen binding domain of one of said first said second non-naturally occurring chimeric membrane embedded receptor, comprises an scFv, and the other comprises a single VH domain, e.g., a camelid, shark, or lamprey single VH domain, or a single VH domain derived from a human or mouse sequence.

In some embodiments, the claimed invention comprises a first and second CAR (e.g., a TA CAR and a BCA CAR), wherein the antigen binding domain of one of the first CAR and the second CAR does not comprise a variable light domain and a variable heavy domain. In some embodiments, the antigen binding domain of one of the first CAR and the second CAR is an scFv, and the other is not an scFv. In some embodiments, the antigen binding domain of one of the first CAR and the second CAR comprises a single VH domain, e.g., a camelid, shark, or lamprey single VH domain, or a single VH domain derived from a human or mouse sequence. In some embodiments, the antigen binding domain of one of the first CAR and the second CAR comprises a nanobody. In some embodiments, the antigen binding domain of one of the first CAR and the second CAR comprises a camelid VHH domain.

In some embodiments, the antigen binding domain of one of the first CAR and the second CAR comprises an scFv, and the other comprises a single VH domain, e.g., a camelid, shark, or lamprey single VH domain, or a single VH domain derived from a human or mouse sequence. In some embodiments, the antigen binding domain of one of the first CAR and the second CAR comprises an scFv, and the other comprises a nanobody. In some embodiments, the antigen binding domain of one of the first CAR and the second CAR comprises comprises an scFv, and the other comprises a camelid VHH domain.

In some embodiments, when present on the surface of a cell, binding of the antigen binding domain of the first CAR to its cognate antigen is not substantially reduced by the presence of the second CAR. In some embodiments, binding of the antigen binding domain of the first CAR to its cognate antigen in the presence of the second CAR is 85%, 90%, 95%, 96%, 97%, 98% or 99% of binding of the antigen binding domain of the first CAR to its cognate antigen in the absence of the second CAR.

In some embodiments, when present on the surface of a cell, the antigen binding domains of the first CAR and the second CAR, associate with one another less than if both were scFv antigen binding domains. In some embodiments, the antigen binding domains of said first CAR said second CAR, associate with one another 85%, 90%, 95%, 96%, 97%, 98% or 99% less than if both were scFv antigen binding domains.

In another aspect, the CAR-expressing cell described herein can further express another agent, e.g., an agent which enhances the activity of a CAR-expressing cell. For example, in one embodiment, the agent can be an agent which inhibits an inhibitory molecule. Inhibitory molecules, e.g., PD1, can, in some embodiments, decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta.

In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

In one embodiment, the agent comprises the extracellular domain (ECD) of an inhibitory molecule, e.g., Programmed Death 1 (PD1), fused to a transmembrane domain and intracellular signaling domains such as 41BB and CD3 zeta (also referred to herein as a PD1 CAR). In one embodiment, the PD1 CAR, when used incombinations with a XCAR described herein, improves the persistence of the T cell. In one embodiment, the CAR is a PD1 CAR comprising the extracellular domain of PD1 indicated as underlined in SEQ ID NO: 26. In one embodiment, the PD1 CAR comprises the amino acid sequence of SEQ ID NO:26. In one embodiment, the PD1 CAR comprises the amino acid sequence of SEQ ID NO:39).

In one embodiment, the agent comprises a nucleic acid sequence encoding the PD1 CAR, e.g., the PD1 CAR described herein. In one embodiment, the nucleic acid sequence for the PD1 CAR is shown as SEQ ID NO: 27 in Table 1, with the sequence for PD1 ECD underlined.

In another aspect, the present disclosure provides a population of CAR-expressing cells. In some embodiments, the population of CAR-expressing cells comprises a mixture of cells expressing different CARs. For example, in one embodiment, the population of CART cells can include a first cell expressing a CAR having an antigen binding domain to a tumor antigen described herein, and a second cell expressing a CAR having a different antigen binding domain, e.g., an antigen binding domain to a different tumor antigen described herein, e.g., an antigen binding domain to a tumor antigen described herein that differs from the tumor antigen bound by the antigen binding domain of the CAR expressed by the first cell. As another example, the population of CAR-expressing cells can include a first cell expressing a CAR that includes an antigen binding domain to a tumor antigen described herein, and a second cell expressing a CAR that includes an antigen binding domain to a target other than a tumor antigen as described herein. In one embodiment, the population of CAR-expressing cells includes, e.g., a first cell expressing a CAR that includes a primary intracellular signaling domain, and a second cell expressing a CAR that includes a secondary signaling domain.

In another aspect, the present disclosure provides a population of cells wherein at least one cell in the population expresses a CAR having an antigen binding domain to a tumor antigen described herein, and a second cell expressing another agent, e.g., an agent which enhances the activity of a CAR-expressing cell. For example, in one embodiment, the agent can be an agent which inhibits an inhibitory molecule. Inhibitory molecules, e.g., PD-1, can, in some embodiments, decrease the ability of a CAR-expressing cell to mount an immune effector response. Examples of inhibitory molecules include PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta, or a fragment of any of these, and a second polypeptide which is an intracellular signaling domain described herein (e.g., comprising a costimulatory domain (e.g., 41BB, CD27, OX40 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28 signaling domain described herein and/or a CD3 zeta signaling domain described herein).

In one aspect, the present disclosure provides methods comprising administering a population of CAR-expressing cells, e.g., a mixture of cells expressing different CARs, in combination with another agent, e.g., a kinase inhibitor, such as a kinase inhibitor described herein. In another aspect, the present disclosure provides methods comprising administering a population of cells wherein at least one cell in the population expresses a CAR having an antigen binding domain of a tumor antigen described herein, and a second cell expressing another agent, e.g., an agent which enhances the activity of a CAR-expressing cell, in combination with another agent, e.g., a kinase inhibitor, such as a kinase inhibitor described herein.

### Exemplary CAR Molecules

In one aspect, the BCA CAR comprises a CAR molecule comprising an antigen binding domain that binds to a B cell antigen. In one embodiment, the BCA CAR comprises a CAR molecule comprising a CD19 antigen binding domain (e.g., a murine, human or humanized antibody or antibody fragment that specifically binds to CD19), a transmembrane domain, and an intracellular signaling domain (e.g., an intracellular signaling domain comprising a costimulatory domain and/or a primary signaling domain).

Exemplary CAR molecules of a BCA CAR described herein are provided in Table 10. The CAR molecules in Table 10 comprise a CD19 antigen binding domain, e.g., an amino acid sequence of any CD19 antigen binding domain provided in Table 6.

**Table 10. Exemplary CD19 CAR molecules**

| **B cell antigen** | **Name** | **Amino Acid Sequence** | **SEQ ID NO:** |
|---|---|---|---|
| CD19 | CTL01 9 | MALPVTALLLPLALLLHAARPDIQMTQTTSSLSASLGDRVTISCRASQDISKYL NWYQQKPDGTVKLLIYHTSRLHSGVPSRFSGSGSGTDYSLTISNLEQEDIATYF CQQGNTLPYTFGGGTKLEITGGGGSGGGGSGGGGSEVKLQESGPGLVAPSQSLS VTCTVSGVSLPDYGVSWIRQPPRKGLEWLGVIWGSETTYYNSALKSRLTIIKDN SKSQVFLKMNSLQTDDTAIYYCAKHYYYGGSYAMDYWGQGTSVTVSSTTTPAPR PPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **281** |
| CD19 | CAR 1 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSQVQLQESGPGLVKPSETLS LTCTVSGVSLPDYGVSWIRQPPGKGLEWIGVIWGSETTYYSSSLKSRVTISKDN SKNQVSLKLSSVTAADTAVYYCAKHYYYGGSYAMDYWGQGTLVTVSSTTTPAPR PPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **269** |
| CD19 | CAR 2 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSQVQLQESGPGLVKPSETLS LTCTVSGVSLPDYGVSWIRQPPGKGLEWIGVIWGSETTYYQSSLKSRVTISKDN SKNQVSLKLSSVTAADTAVYYCAKHYYYGGSYAMDYWGQGTLVTVSSTTTPAPR PPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEGL | **270** |
| | | YNELQKDKMAEAYSEIGMKGERRRGKGHDGLYQGLSTATKDTYDALHMQALPPR | |
| CD19 | CAR 3 | MALPVTALLLPLALLLHAARPQVQLQESGPGLVKPSETLSLTCTVSGVSLPDYG VSWIRQPPGKGLEWIGVIWGSETTYYSSSLKSRVTISKDNSKNQVSLKLSSVTA ADTAVYYCAKHYYYGGSYAMDYWGQGTLVTVSSGGGGSGGGGSGGGGSEIVMTQ SPATLSLSPGERATLSCRASQDISKYLNWYQQKPGQAPRLLIYHTSRLHSGIPA RFSGSGSGTDYTLTISSLQPEDFAVYFCQQGNTLPYTFGQGTKLEIKTTTPAPR PPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **271** |
| CD19 | CAR 4 | MALPVTALLLPLALLLHAARPQVQLQESGPGLVKPSETLSLTCTVSGVSLPDYG VSWIRQPPGKGLEWIGVIWGSETTYYQSSLKSRVTISKDNSKNQVSLKLSSVTA ADTAVYYCAKHYYYGGSYAMDYWGQGTLVTVSSGGGGSGGGGSGGGGSEIVMTQ SPATLSLSPGERATLSCRASQDISKYLNWYQQKPGQAPRLLIYHTSRLHSGIPA RFSGSGSGTDYTLTISSLQPEDFAVYFCQQGNTLPYTFGQGTKLEIKTTTPAPR PPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **272** |
| CD19 | CAR 5 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSGGGGSQVQLQESGPGLVKP SETLSLTCTVSGVSLPDYGVSWIRQPPGKGLEWIGVIWGSETTYYSSSLKSRVT ISKDNSKNQVSLKLSSVTAADTAVYYCAKHYYYGGSYAMDYWGQGTLVTVSSTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR | **27**3 |
| CD19 | CAR 6 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSGGGGSQVQLQESGPGLVKP SETLSLTCTVSGVSLPDYGVSWIRQPPGKGLEWIGVIWGSETTYYQSSLKSRVT ISKDNSKNQVSLKLSSVTAADTAVYYCAKHYYYGGSYAMDYWGQGTLVTVSSTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR | **274** |
| CD19 | CAR 7 | MALPVTALLLPLALLLHAARPQVQLQESGPGLVKPSETLSLTCTVSGVSLPDYG VSWIRQPPGKGLEWIGVIWGSETTYYSSSLKSRVTISKDNSKNQVSLKLSSVTA ADTAVYYCAKHYYYGGSYAMDYWGQGTLVTVSSGGGGSGGGGSGGGGSGGGGSE IVMTQSPATLSLSPGERATLSCRASQDISKYLNWYQQKPGQAPRLLIYHTSRLH SGIPARFSGSGSGTDYTLTISSLQPEDFAVYFCQQGNTLPYTFGQGTKLEIKTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR | **275** |
| CD19 | CAR 8 | MALPVTALLLPLALLLHAARPQVQLQESGPGLVKPSETLSLTCTVSGVSLPDYG VSWIRQPPGKGLEWIGVIWGSETTYYQSSLKSRVTISKDNSKNQVSLKLSSVTA ADTAVYYCAKHYYYGGSYAMDYWGQGTLVTVSSGGGGSGGGGSGGGGSGGGGSE IVMTQSPATLSLSPGERATLSCRASQDISKYLNWYQQKPGQAPRLLIYHTSRLH SGIPARFSGSGSGTDYTLTISSLQPEDFAVYFCQQGNTLPYTFGQGTKLEIKTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN | **276** |
| | | PQEGLYNELQKDKMAEAYSEIGMKGERRRGKGHDGLYQGLSTATKDTYDALHMQ ALPPR | |
| CD19 | CAR 9 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSGGGGSQVQLQESGPGLVKP SETLSLTCTVSGVSLPDYGVSWIRQPPGKGLEWIGVIWGSETTYYNSSLKSRVT ISKDNSKNQVSLKLSSVTAADTAVYYCAKHYYYGGSYAMDYWGQGTLVTVSSTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR | **277** |
| CD19 | CAR 10 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSGGGGSQVQLQESGPGLVKP SETLSLTCTVSGVSLPDYGVSWIRQPPGKGLEWIGVIWGSETTYYNSSLKSRVT ISKDNSKNQVSLKLSSVTAADTAVYYCAKHYYYGGSYAMDYWGQGTLVTVSSTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR | **278** |
| CD19 | CAR 11 | MALPVTALLLPLALLLHAARPQVQLQESGPGLVKPSETLSLTCTVSGVSLPDYG VSWIRQPPGKGLEWIGVIWGSETTYYNSSLKSRVTISKDNSKNQVSLKLSSVTA ADTAVYYCAKHYYYGGSYAMDYWGQGTLVTVSSGGGGSGGGGSGGGGSGGGGSE IVMTQSPATLSLSPGERATLSCRASQDISKYLNWYQQKPGQAPRLLIYHTSRLH SGIPARFSGSGSGTDYTLTISSLQPEDFAVYFCQQGNTLPYTFGQGTKLEIKTT TPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRKN PQEGLYNELQKDKMAEAYSEIGMKGERRRGKGHDGLYQGLSTATKDTYDALHMQ ALPPR | **279** |
| CD19 | CAR 12 | MALPVTALLLPLALLLHAARPEIVMTQSPATLSLSPGERATLSCRASQDISKYL NWYQQKPGQAPRLLIYHTSRLHSGIPARFSGSGSGTDYTLTISSLQPEDFAVYF CQQGNTLPYTFGQGTKLEIKGGGGSGGGGSGGGGSQVQLQESGPGLVKPSETLS LTCTVSGVSLPDYGVSWIRQPPGKGLEWIGVIWGSETTYYNSSLKSRVTISKDN SKNQVSLKLSSVTAADTAVYYCAKHYYYGGSYAMDYWGQGTLVTVSSTTTPAPR PPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **280** |

In one embodiment, the CAR molecule of the BCA CAR comprises (e.g., consists of) an amino acid sequence as provided in Table 10, or in Table 3 of International Publication No. WO2014/153270, filed March 15, 2014; incorporated herein by reference. In one embodiment, the CAR molecule of the BCA CAR comprises (e.g., consists of) an amino acid sequence of SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, or SEQ ID NO: 281; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence of SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, or SEQ ID NO: 281; or an amino acid sequence having 85%, 90%, 95%, 96%, 97%, 98%, 99% identity to an amino acid sequence of SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, or SEQ ID NO: 281.

In one aspect, the BCA CAR comprises a CAR molecule comprising an antigen binding domain that binds to a B cell antigen. In one embodiment, the BCA CAR comprises a CAR molecule comprising a BCMA antigen binding domain (e.g., a murine, human or humanized antibody or antibody fragment that specifically binds to BCMA, e.g., human BCMA), a transmembrane domain, and an intracellular signaling domain (e.g., an intracellular signaling domain comprising a costimulatory domain and/or a primary signaling domain).

Exemplary CAR molecules of a BCA CAR described herein are provided in Table 16, or Table 1 of WO2016/014565, or as otherwise described herein. The CAR molecules in Table 16 comprise a BCMA antigen binding domain, e.g., an amino acid sequence of any BCMA antigen binding domain provided in Table 12 or 13.

**Table 16. Exemplary BCMA CAR molecules. Sequences are provided with a leader sequence.**

| **Name/ Descriptio n** | **SEQ ID NO:** | **Sequence** |
|---|---|---|
| **139109** | | |
| **139109- aa Full CAR** | 959 | MALPVTALLLPLALLLHAARPEVQLVESGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS DIQLTQSPSSLSASVGDRVTITCRASQSISSYLNWYQQKPGKAPKLLIYAA SSLQSGVPSRFSGSGSGTDFTLTISSLQPEDFATYYCQQSYSTPYTFGQGT KVEIKTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGC SCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHDG LYQGLSTATKDTYDALHMQALPPR |
| **139109- nt Full CAR** | 974 | ATGGCCCTCCCTGTCACCGCCCTGCTGCTTCCGCTGGCTCTTCTGCTCCAC GCCGCTCGGCCCGAAGTGCAATTGGTGGAATCAGGGGGAGGACTTGTGCAG CCTGGAGGATCGCTGAGACTGTCATGTGCCGTGTCCGGCTTTGCCCTGTCC AACCACGGGATGTCCTGGGTCCGCCGCGCGCCTGGAAAGGGCCTCGAATGG GTGTCGGGTATTGTGTACAGCGGTAGCACCTACTATGCCGCATCCGTGAAG |
| | | GGGAGATTCACCATCAGCCGGGACAACTCCAGGAACACTCTGTACCTCCAA ATGAATTCGCTGAGGCCAGAGGACACTGCCATCTACTACTGCTCCGCGCAT GGCGGAGAGTCCGACGTCTGGGGACAGGGGACCACCGTGACCGTGTCTAGC GCGTCCGGCGGAGGCGGCAGCGGGGGTCGGGCATCAGGGGGCGGCGGATCG GACATCCAGCTCACCCAGTCCCCGAGCTCGCTGTCCGCCTCCGTGGGAGAT CGGGTCACCATCACGTGCCGCGCCAGCCAGTCGATTTCCTCCTACCTGAAC TGGTACCAACAGAAGCCCGGAAAAGCCCCGAAGCTTCTCATCTACGCCGCC TCGAGCCTGCAGTCAGGAGTGCCCTCACGGTTCTCCGGCTCCGGTTCCGGT ACTGATTTCACCCTGACCATTTCCTCCCTGCAACCGGAGGACTTCGCTACT TACTACTGCCAGCAGTCGTACTCCACCCCCTACACTTTCGGACAAGGCACC AAGGTCGAAATCAAGACCACTACCCCAGCACCGAGGCCACCCACCCCGGCT CCTACCATCGCCTCCCAGCCTCTGTCCCTGCGTCCGGAGGCATGTAGACCC GCAGCTGGTGGGGCCGTGCATACCCGGGGTCTTGACTTCGCCTGCGATATC TACATTTGGGCCCCTCTGGCTGGTACTTGCGGGGTCCTGCTGCTTTCACTC GTGATCACTCTTTACTGTAAGCGCGGTCGGAAGAAGCTGCTGTACATCTTT AAGCAACCCTTCATGAGGCCTGTGCAGACTACTCAAGAGGAGGACGGCTGT TCATGCCGGTTCCCAGAGGAGGAGGAAGGCGGCTGCGAACTGCGCGTGAAA TTCAGCCGCAGCGCAGATGCTCCAGCCTACAAGCAGGGGCAGAACCAGCTC TACAACGAACTCAATCTTGGTCGGAGAGAGGAGTACGACGTGCTGGACAAG CGGAGAGGACGGGACCCAGAAATGGGCGGGAAGCCGCGCAGAAAGAATCCC CAAGAGGGCCTGTACAACGAGCTCCAAAAGGATAAGATGGCAGAAGCCTAT AGCGAGATTGGTATGAAAGGGGAACGCAGAAGAGGCAAAGGCCACGACGGA CTGTACCAGGGACTCAGCACCGCCACCAAGGACACCTATGACGCTCTTCAC ATGCAGGCCCTGCCGCCTCGG |

| **139103** | | |
|---|---|---|
| **139103- aa Full CAR** | 949 | MALPVTALLLPLALLLHAARPQVQLVESGGGLVQPGRSLRLSCAASGFTFS NYAMSWVRQAPGKGLGWVSGISRSGENTYYADSVKGRFTISRDNSKNTLYL QMNSLRDEDTAVYYCARSPAHYYGGMDVWGQGTTVTVSSASGGGGSGGRAS GGGGSDIVLTQSPGTLSLSPGERATLSCRASQSISSSFLAWYQQKPGQAPR LLIYGASRRATGIPDRFSGSGSGTDFTLTISRLEPEDSAVYYCQQYHSSPS WTFGQGTKLEIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **139103- nt Full CAR** | 964 | ATGGCCCTCCCTGTCACCGCCCTGCTGCTTCCGCTGGCTCTTCTGCTCCAC GCCGCTCGGCCCCAAGTGCAACTCGTGGAATCTGGTGGAGGACTCGTGCAA CCCGGAAGATCGCTTAGACTGTCGTGTGCCGCCAGCGGGTTCACTTTCTCG AACTACGCGATGTCCTGGGTCCGCCAGGCACCCGGAAAGGGACTCGGTTGG GTGTCCGGCATTTCCCGGTCCGGCGAAAATACCTACTACGCCGACTCCGTG AAGGGCCGCTTCACCATCTCAAGGGACAACAGCAAAAACACCCTGTACTTG CAAATGAACTCCCTGCGGGATGAAGATACAGCCGTGTACTATTGCGCCCGG TCGCCTGCCCATTACTACGGCGGAATGGACGTCTGGGGACAGGGAACCACT GTGACTGTCAGCAGCGCGTCGGGTGGCGGCGGCTCAGGGGGTCGGGCCTCC GGGGGGGGAGGGTCCGACATCGTGCTGACCCAGTCCCCGGGAACCCTGAGC CTGAGCCCGGGAGAGCGCGCGACCCTGTCATGCCGGGCATCCCAGAGCATT AGCTCCTCCTTTCTCGCCTGGTATCAGCAGAAGCCCGGACAGGCCCCGAGG CTGCTGATCTACGGCGCTAGCAGAAGGGCTACCGGAATCCCAGACCGGTTC TCCGGCTCCGGTTCCGGGACCGATTTCACCCTTACTATCTCGCGCCTGGAA CCTGAGGACTCCGCCGTCTACTACTGCCAGCAGTACCACTCATCCCCGTCG TGGACGTTCGGACAGGGCACCAAGCTGGAGATTAAGACCACTACCCCAGCA CCGAGGCCACCCACCCCGGCTCCTACCATCGCCTCCCAGCCTCTGTCCCTG |
| | | CGTCCGGAGGCATGTAGACCCGCAGCTGGTGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG GACACCTATGACGCTCTTCACATGCAGGCCCTGCCGCCTCGG |

| **139105** | | |
|---|---|---|
| **139105- aa Full CAR** | 950 | MALPVTALLLPLALLLHAARPQVQLVESGGGLVQPGRSLRLSCAASGFTFD DYAMHWVRQAPGKGLEWVSGISWNSGSIGYADSVKGRFTISRDNAKNSLYL QMNSLRAEDTALYYCSVHSFLAYWGQGTLVTVSSASGGGGSGGRASGGGGS DIVMTQTPLSLPVTPGEPASISCRSSQSLLHSNGYNYLDWYLQKPGQSPQL LIYLGSNRASGVPDRFSGSGSGTDFTLKISRVEAEDVGVYYCMQALQTPYT FGQGTKVEIKTTTPAPRPPTPAPTIASQPLSLRPEACRPAAGGAVHTRGLD FACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRREEY DVLDKRRGRDPEMGGKPRRKNPQEGLYNELQKDKMAEAYSEIGMKGERRRG KGHDGLYQGLSTATKDTYDALHMQALPPR |
| **139105- nt Full CAR** | 965 | ATGGCCCTCCCTGTCACCGCCCTGCTGCTTCCGCTGGCTCTTCTGCTCCAC GCCGCTCGGCCCCAAGTGCAACTCGTCGAATCCGGTGGAGGTCTGGTCCAA CCTGGTAGAAGCCTGAGACTGTCGTGTGCGGCCAGCGGATTCACCTTTGAT GACTATGCTATGCACTGGGTGCGGCAGGCCCCAGGAAAGGGCCTGGAATGG GTGTCGGGAATTAGCTGGAACTCCGGGTCCATTGGCTACGCCGACTCCGTG AAGGGCCGCTTCACCATCTCCCGCGACAACGCAAAGAACTCCCTGTACTTG CAAATGAACTCGCTCAGGGCTGAGGATACCGCGCTGTACTACTGCTCCGTG CATTCCTTCCTGGCCTACTGGGGACAGGGAACTCTGGTCACCGTGTCGAGC GCCTCCGGCGGCGGGGGCTCGGGTGGACGGGCCTCGGGCGGAGGGGGGTCC GACATCGTGATGACCCAGACCCCGCTGAGCTTGCCCGTGACTCCCGGAGAG CCTGCATCCATCTCCTGCCGGTCATCCCAGTCCCTTCTCCACTCCAACGGA TACAACTACCTCGACTGGTACCTCCAGAAGCCGGGACAGAGCCCTCAGCTT CTGATCTACCTGGGGTCAAATAGAGCCTCAGGAGTGCCGGATCGGTTCAGC GGATCTGGTTCGGGAACTGATTTCACTCTGAAGATTTCCCGCGTGGAAGCC GAGGACGTGGGCGTCTACTACTGTATGCAGGCGCTGCAGACCCCCTATACC TTCGGCCAAGGGACGAAAGTGGAGATCAAGACCACTACCCCAGCACCGAGG CCACCCACCCCGGCTCCTACCATCGCCTCCCAGCCTCTGTCCCTGCGTCCG GAGGCATGTAGACCCGCAGCTGGTGGGGCCGTGCATACCCGGGGTCTTGAC TTCGCCTGCGATATCTACATTTGGGCCCCTCTGGCTGGTACTTGCGGGGTC CTGCTGCTTTCACTCGTGATCACTCTTTACTGTAAGCGCGGTCGGAAGAAG CTGCTGTACATCTTTAAGCAACCCTTCATGAGGCCTGTGCAGACTACTCAA GAGGAGGACGGCTGTTCATGCCGGTTCCCAGAGGAGGAGGAAGGCGGCTGC GAACTGCGCGTGAAATTCAGCCGCAGCGCAGATGCTCCAGCCTACAAGCAG GGGCAGAACCAGCTCTACAACGAACTCAATCTTGGTCGGAGAGAGGAGTAC GACGTGCTGGACAAGCGGAGAGGACGGGACCCAGAAATGGGCGGAAGCCG CGCAGAAAGAATCCCCAAGAGGGCCTGTACAACGAGCTCCAAAAGGATAAG ATGGCAGAAGCCTATAGCGAGATTGGTATGAAAGGGGAACGCAGAAGAGGC AAAGGCCACGACGGACTGTACCAGGGACTCAGCACCGCCACCAAGGACACC TATGACGCTCTTCACATGCAGGCCCTGCCGCCTCGG |

| **139111** | | |
|---|---|---|
| **139111- aa Full CAR** | 951 | MALPVTALLLPLALLLHAARPEVQLLESGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS DIVMTQTPLSLSVTPGQPASISCKSSQSLLRNDGKTPLYWYLQKAGQPPQL LIYEVSNRFSGVPDRFSGSGSGTDFTLKISRVEAEDVGAYYCMQNIQFPSF GGGTKLEIKTTTPAPRPPTPAPTIASQPLSLRPEACRPAAGGAVHTRGLDF ACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYD VLDKRRGRDPEMGGKPRRKNPQEGLYNELQKDKMAEAYSEIGMKGERRRGK GHDGLYQGLSTATKDTYDALHMQALPPR |
| **139111- nt Full CAR** | 966 | ATGGCCCTCCCTGTCACCGCCCTGCTGCTTCCGCTGGCTCTTCTGCTCCAC GCCGCTCGGCCCGAAGTGCAATTGTTGGAATCTGGAGGAGGACTTGTGCAG CCTGGAGGATCACTGAGACTTTCGTGTGCGGTGTCAGGCTTCGCCCTGAGC AACCACGGCATGAGCTGGGTGCGGAGAGCCCCGGGAAGGGTCTGGAATGG GTGTCCGGGATCGTCTACTCCGGTTCAACTTACTACGCCGCAAGCGTGAAG GGTCGCTTCACCATTTCCCGCGATAACTCCCGGAACACCCTGTACCTCCAA ATGAACTCCCTGCGGCCCGAGGACACCGCCATCTACTACTGTTCCGCGCAT GGAGGAGAGTCCGATGTCTGGGGACAGGGCACTACCGTGACCGTGTCGAGC GCCTCGGGGGGAGGAGGCTCCGGCGGTCGCGCCTCCGGGGGGGGTGGCAGC GACATTGTGATGACGCAGACTCCACTCTCGCTGTCCGTGACCCCGGGACAG CCCGCGTCCATCTCGTGCAAGAGCTCCCAGAGCCTGCTGAGGAACGACGGA AAGACTCCTCTGTATTGGTACCTCCAGAAGGCTGGACAGCCCCCGCAACTG CTCATCTACGAAGTGTCAAATCGCTTCTCCGGGGTGCCGGATCGGTTTTCC GGCTCGGGATCGGGCACCGACTTCACCCTGAAAATCTCCAGGGTCGAGGCC GAGGACGTGGGAGCCTACTACTGCATGCAAAACATCCAGTTCCCTTCCTTC GGCGGCGGCACAAAGCTGGAGATTAAGACCACTACCCCAGCACCGAGGCCA CCCACCCCGGCTCCTACCATCGCCTCCCAGCCTCTGTCCCTGCGTCCGGAG GCATGTAGACCCGCAGCTGGTGGGGCCGTGCATACCCGGGGTCTTGACTTC GCCTGCGATATCTACATTTGGGCCCCTCTGGCTGGTACTTGCGGGGTCCTG CTGCTTTCACTCGTGATCACTCTTTACTGTAAGCGCGGTCGGAAGAAGCTG CTGTACATCTTTAAGCAACCCTTCATGAGGCCTGTGCAGACTACTCAAGAG GAGGACGGCTGTTCATGCCGGTTCCCAGAGGAGGAGGAAGGCGGCTGCGAA CTGCGCGTGAAATTCAGCCGCAGCGCAGATGCTCCAGCCTACAAGCAGGGG CAGAACCAGCTCTACAACGAACTCAATCTTGGTCGGAGAGAGGAGTACGAC GTGCTGGACAAGCGGAGAGGACGGGACCCAGAAATGGGCGGGAAGCCGCGC AGAAAGAATCCCCAAGAGGGCCTGTACAACGAGCTCCAAAAGGATAAGATG GCAGAAGCCTATAGCGAGATTGGTATGAAAGGGGAACGCAGAAGAGGCAAA GGCCACGACGGACTGTACCAGGGACTCAGCACCGCCACCAAGGACACCTAT GACGCTCTTCACATGCAGGCCCTGCCGCCTCGG |

| **139100** | | |
|---|---|---|
| **139100- aa Full CAR** | 952 | MALPVTALLLPLALLLHAARPQVQLVQSGAEVRKTGASVKVSCKASGYIFD NFGINWVRQAPGQGLEWMGWINPKNNNTNYAQKFQGRVTITADESTNTAYM EVSSLRSEDTAVYYCARGPYYYQSYMDVWGQGTMVTVSSASGGGGSGGRAS GGGGSDIVMTQTPLSLPVTPGEPASISCRSSQSLLHSNGYNYLNWYLQKPG QSPQLLIYLGSKRASGVPDRFSGSGSGTDFTLHITRVGAEDVGVYYCMQAL QTPYTFGQGTKLEIKTTTPAPRPPTPAPTIASQPLSLRPEACRPAAGGAVH TRGLDFACDIYIWAPLAGTCGVLLLSLVITLYCKRGRKKLLYIFKQPFMRP VQTTQEEDGCSCRFPEEEEGGCELRVKFSRSADAPAYKQGQNQLYNELNLG RREEYDVLDKRRGRDPEMGGKPRRKNPQEGLYNELQKDKMAEAYSEIGMKG ERRRGKGHDGLYQGLSTATKDTYDALHMQALPPR |
| **139100- nt Full CAR** | 967 | ATGGCCCTCCCTGTCACCGCCCTGCTGCTTCCGCTGGCTCTTCTGCTCCAC |
| | | GCCGCTCGGCCCCAAGTCCAACTCGTCCAGTCCGGCGCAGAAGTCAGAAAA ACCGGTGCTAGCGTGAAAGTGTCCTGCAAGGCCTCCGGCTACATTTTCGAT AACTTCGGAATCAACTGGGTCAGACAGGCCCCGGGCCAGGGGCTGGAATGG ATGGGATGGATCAACCCCAAGAACAACAACACCAACTACGCACAGAAGTTC CAGGGCCGCGTGACTATCACCGCCGATGAATCGACCAATACCGCCTACATG GAGGTGTCCTCCCTGCGGTCGGAGGACACTGCCGTGTATTACTGCGCGAGG GGCCCATACTACTACCAAAGCTACATGGACGTCTGGGGACAGGGAACCATG GTGACCGTGTCATCCGCCTCCGGTGGTGGAGGCTCCGGGGGCGGCTTCA GGAGGCGGAGGAAGCGATATTGTGATGACCCAGACTCCGCTTAGCCTGCCC GTGACTCCTGGAGAACCGGCCTCCATTTCCTGCCGGTCCTCGCAATCACTC CTGCATTCCAACGGTTACAACTACCTGAATTGGTACCTCCAGAAGCCTGGC CAGTCGCCCCAGTTGCTGATCTATCTGGGCTCGAAGCGCGCCTCCGGGGTG CCTGACCGGTTTAGCGGATCTGGGAGCGGCACGGACTTCACTCTCCACATC ACCCGCGTGGGAGCGGAGGACGTGGGAGTGTACTACTGTATGCAGGCGCTG CAGACTCCGTACACATTCGGACAGGGCACCAAGCTGGAGATCAAGACCACT ACCCCAGCACCGAGGCCACCCACCCCGGCTCCTACCATCGCCTCCCAGCCT CTGTCCCTGCGTCCGGAGGCATGTAGACCCGCAGCTGGTGGGGCCGTGCAT ACCCGGGGTCTTGACTTCGCCTGCGATATCTACATTTGGGCCCCTCTGGCT GGTACTTGCGGGGTCCTGCTGCTTTCACTCGTGATCACTCTTTACTGTAAG CGCGGTCGGAAGAAGCTGCTGTACATCTTTAAGCAACCCTTCATGAGGCCT GTGCAGACTACTCAAGAGGAGGACGGCTGTTCATGCCGGTTCCCAGAGGAG GAGGAAGGCGGCTGCGAACTGCGCGTGAAATTCAGCCGCAGCGCAGATGCT CCAGCCTACAAGCAGGGGCAGAACCAGCTCTACAACGAACTCAATCTTGGT CGGAGAGAGGAGTACGACGTGCTGGACAAGCGGAGAGGACGGGACCCAGAA ATGGGCGGGAAGCCGCGCAGAAAGAATCCCCAAGAGGGCCTGTACAACGAG CTCCAAAAGGATAAGATGGCAGAAGCCTATAGCGAGATTGGTATGAAAGGG GAACGCAGAAGAGGCAAAGGCCACGACGGACTGTACCAGGGACTCAGCACC GCCACCAAGGACACCTATGACGCTCTTCACATGCAGGCCCTGCCGCCTCGG |

| **139101** | | |
|---|---|---|
| **139101- aa Full CAR** | 953 | MALPVTALLLPLALLLHAARPQVQLQESGGGLVQPGGSLRLSCAASGFTFS SDAMTWVRQAPGKGLEWVSVISGSGGTTYYADSVKGRFTISRDNSKNTLYL QMNSLRAEDTAVYYCAKLDSSGYYYARGPRYWGQGTLVTVSSASGGGGSGG RASGGGGSDIQLTQSPSSLSASVGDRVTITCRASQSISSYLNWYQQKPGKA PKLLIYGASTLASGVPARFSGSGSGTHFTLTINSLQSEDSATYYCQQSYKR ASFGQGTKVEIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **139101- nt Full CAR** | 968 | ATGGCCCTCCCTGTCACCGCCCTGCTGCTTCCGCTGGCTCTTCTGCTCCAC GCCGCTCGGCCCCAAGTGCAACTTCAAGAATCAGGCGGAGGACTCGTGCAG CCCGGAGGATCATTGCGGCTCTCGTGCGCCGCCTCGGGCTTCACCTTCTCG AGCGACGCCATGACCTGGGTCCGCCAGGCCCCGGGGAAGGGGCTGGAATGG GTGTCTGTGATTTCCGGCTCCGGGGGAACTACGTACTACGCCGATTCCGTG AAAGGTCGCTTCACTATCTCCCGGGACAACAGCAAGAACACCCTTTATCTG CAAATGAATTCCCTCCGCGCCGAGGACACCGCCGTGTACTACTGCGCCAAG CTGGACTCCTCGGGCTACTACTATGCCCGGGGTCCGAGATACTGGGGACAG GGAACCCTCGTGACCGTGTCCTCCGCGTCCGGCGGAGGAGGGTCGGGAGGG CGGGCCTCCGGCGGCGGCGGTTCGGACATCCAGCTGACCCAGTCCCCATCC TCACTGAGCGCAAGCGTGGGCGACAGAGTCACCATTACATGCAGGGCGTCC CAGAGCATCAGCTCCTACCTGAACTGGTACCAACAGAAGCCTGGAAAGGCT CCTAAGCTGTTGATCTACGGGGCTTCGACCCTGGCATCCGGGGTGCCCGCG |
| | | AGGTTTAGCGGAAGCGGTAGCGGCACTCACTTCACTCTGACCATTAACAGC CTCCAGTCCGAGGATTCAGCCACTTACTACTGTCAGCAGTCCTACAAGCGG GCCAGCTTCGGACAGGGCACTAAGGTCGAGATCAAGACCACTACCCCAGCA CCGAGGCCACCCACCCCGGCTCCTACCATCGCCTCCCAGCCTCTGTCCCTG CGTCCGGAGGCATGTAGACCCGCAGCTGGTGGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG GACACCTATGACGCTCTTCACATGCAGGCCCTGCCGCCTCGG |

| **139102** | | |
|---|---|---|
| **139102- aa Full CAR** | 954 | MALPVTALLLPLALLLHAARPQVQLVQSGAEVKKPGASVKVSCKASGYTFS NYGITWVRQAPGQGLEWMGWISAYNGNTNYAQKFQGRVTMTRNTSISTAYM ELSSLRSEDTAVYYCARGPYYYYMDVWGKGTMVTVSSASGGGGSGGRASGG GGSEIVMTQSPLSLPVTPGEPASISCRSSQSLLYSNGYNYVDWYLQKPGQS PQLLIYLGSNRASGVPDRFSGSGSGTDFKLQISRVEAEDVGIYYCMQGRQF PYSFGQGTKVEIKTTTPAPRPPTPAPTIASQPLSLRPEACRPAAGGAVHTR GLDFACDIYIWAPLAGTCGVLLLSLVITLYCKRGRKKLLYIFKQPFMRPVQ TTQEEDGCSCRFPEEEEGGCELRVKFSRSADAPAYKQGQNQLYNELNLGRR EEYDVLDKRRGRDPEMGGKPRRKNPQEGLYNELQKDKMAEAYSEIGMKGER RRGKGHDGLYQGLSTATKDTYDALHMQALPPR |
| **139102- nt Full CAR** | 969 | ATGGCCCTCCCTGTCACCGCCCTGCTGCTTCCGCTGGCTCTTCTGCTCCAC GCCGCTCGGCCCCAAGTCCAACTGGTCCAGAGCGGTGCAGAAGTGAAGAAG CCCGGAGCGAGCGTGAAAGTGTCCTGCAAGGCTTCCGGGTACACCTTCTCC AACTACGGCATCACTTGGGTGCGCCAGGCCCCGGGACAGGGCCTGGAATGG ATGGGGTGGATTTCCGCGTACAACGGCAATACGAACTACGCTCAGAAGTTC CAGGGTAGAGTGACCATGACTAGGAACACCTCCATTTCCACCGCCTACATG GAACTGTCCTCCCTGCGGAGCGAGGACACCGCCGTGTACTATTGCGCCCGG GGACCATACTACTACTACATGGATGTCTGGGGGAAGGGGACTATGGTCACC GTGTCATCCGCCTCGGGAGGCGGCGGATCAGGAGGACGCGCCTCTGGTGGT GGAGGATCGGAGATCGTGATGACCCAGAGCCCTCTCTCCTTGCCCGTGACT CCTGGGGAGCCCGCATCCATTTCATGCCGGAGCTCCCAGTCACTTCTCTAC TCCAACGGCTATAACTACGTGGATTGGTACCTCCAAAAGCCGGGCCAGAGC CCGCAGCTGCTGATCTACCTGGGCTCGAACAGGGCCAGCGGAGTGCCTGAC CGGTTCTCCGGGTCGGGAAGCGGGACCGACTTCAAGCTGCAAATCTCGAGA GTGGAGGCCGAGGACGTGGGAATCTACTACTGTATGCAGGGCCGCCAGTTT CCGTACTCGTTCGGACAGGGCACCAAAGTGGAAATCAAGACCACTACCCCA GCACCGAGGCCACCCACCCCGGCTCCTACCATCGCCTCCCAGCCTCTGTCC CTGCGTCCGGAGGCATGTAGACCCGCAGCTGGTGGGGCCGTGCATACCCGG GGTCTTGACTTCGCCTGCGATATCTACATTTGGGCCCCTCTGGCTGGTACT TGCGGGGTCCTGCTGCTTTCACTCGTGATCACTCTTTACTGTAAGCGCGGT CGGAAGAAGCTGCTGTACATCTTTAAGCAACCCTTCATGAGGCCTGTGCAG ACTACTCAAGAGGAGGACGGCTGTTCATGCCGGTTCCCAGAGGAGGAGGAA GGCGGCTGCGAACTGCGCGTGAAATTCAGCCGCAGCGCAGATGCTCCAGCC TACAAGCAGGGGCAGAACCAGCTCTACAACGAACTCAATCTTGGTCGGAGA GAGGAGTACGACGTGCTGGACAAGCGGAGAGGACGGGACCCAGAAATGGGC |
| | | GGGAAGCCGCGCAGAAAGAATCCCCAAGAGGGCCTGTACAACGAGCTCCAA AAGGATAAGATGGCAGAAGCCTATAGCGAGATTGGTATGAAAGGGGAACGC AGAAGAGGCAAAGGCCACGACGGACTGTACCAGGGACTCAGCACCGCCACC AAGGACACCTATGACGCTCTTCACATGCAGGCCCTGCCGCCTCGG |

| **139104** | | |
|---|---|---|
| **139104- aa Full CAR** | 955 | MALPVTALLLPLALLLHAARPEVQLLETGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS EIVLTQSPATLSVSPGESATLSCRASQSVSSNLAWYQQKPGQAPRLLIYGA STRASGIPDRFSGSGSGTDFTLTISSLQAEDVAVYYCQQYGSSLTFGGGTK VEIKTTTPAPRPPTPAPTIASQPLSLRPEACRPAAGGAVHTRGLDFACDIY IWAPLAGTCGVLLLSLVITLYCKRGRKKLLYIFKQPFMRPVQTTQEEDGCS CRFPEEEEGGCELRVKFSRSADAPAYKQGQNQLYNELNLGRREEYDVLDKR RGRDPEMGGKPRRKNPQEGLYNELQKDKMAEAYSEIGMKGERRRGKGHDGL YQGLSTATKDTYDALHMQALPPR |
| **139104- nt Full CAR** | 970 | ATGGCCCTCCCTGTCACCGCCCTGCTGCTTCCGCTGGCTCTTCTGCTCCAC GCCGCTCGGCCCGAAGTGCAATTGCTCGAAACTGGAGGAGGTCTGGTGCAA CCTGGAGGATCACTTCGCCTGTCCTGCGCCGTGTCGGGCTTTGCCCTGTCC AACCATGGAATGAGCTGGGTCCGCCGCGCGCCGGGGAAGGGCCTCGAATGG GTGTCCGGCATCGTCTACTCCGGCTCCACCTACTACGCCGCGTCCGTGAAG GGCCGGTTCACGATTTCACGGGACAACTCGCGGAACACCCTGTACCTCCAA ATGAATTCCCTTCGGCCGGAGGATACTGCCATCTACTACTGCTCCGCCCAC GGTGGCGAATCCGACGTCTGGGGCCAGGGAACCACCGTGACCGTGTCCAGC GCGTCCGGGGGAGGAGGAAGCGGGGGTAGAGCATCGGGTGGAGGCGGATCA GAGATCGTGCTGACCCAGTCCCCCGCCACCTTGAGCGTGTCACCAGGAGAG TCCGCCACCCTGTCATGCCGCGCCAGCCAGTCCGTGTCCTCCAACCTGGCT TGGTACCAGCAGAAGCCGGGGCAGGCCCCTAGACTCCTGATCTATGGGGCG TCGACCCGGGCATCTGGAATTCCCGATAGGTTCAGCGGATCGGGCTCGGGC ACTGACTTCACTCTGACCATCTCCTCGCTGCAAGCCGAGGACGTGGCTGTG TACTACTGTCAGCAGTACGGAAGCTCCCTGACTTTCGGTGGCGGGACCAAA GTCGAGATTAAGACCACTACCCCAGCACCGAGGCCACCCACCCCGGCTCCT ACCATCGCCTCCCAGCCTCTGTCCCTGCGTCCGGAGGCATGTAGACCCGCA GCTGGTGGGGCCGTGCATACCCGGGGTCTTGACTTCGCCTGCGATATCTAC ATTTGGGCCCCTCTGGCTGGTACTTGCGGGGTCCTGCTGCTTTCACTCGTG ATCACTCTTTACTGTAAGCGCGGTCGGAAGAAGCTGCTGTACATCTTTAAG CAACCCTTCATGAGGCCTGTGCAGACTACTCAAGAGGAGGACGGCTGTTCA TGCCGGTTCCCAGAGGAGGAGGAAGGCGGCTGCGAACTGCGCGTGAAATTC AGCCGCAGCGCAGATGCTCCAGCCTACAAGCAGGGGCAGAACCAGCTCTAC AACGAACTCAATCTTGGTCGGAGAGAGGAGTACGACGTGCTGGACAAGCGG AGAGGACGGGACCCAGAAATGGGCGGAAGCCGCGCAGAAAGAATCCCCAA GAGGGCCTGTACAACGAGCTCCAAAAGGATAAGATGGCAGAAGCCTATAGC GAGATTGGTATGAAAGGGGAACGCAGAAGAGGCAAAGGCCACGACGGACTG TACCAGGGACTCAGCACCGCCACCAAGGACACCTATGACGCTCTTCACATG CAGGCCCTGCCGCCTCGG |

| **139106** | | |
|---|---|---|
| **139106- aa Full CAR** | 956 | MALPVTALLLPLALLLHAARPEVQLVETGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS EIVMTQSPATLSVSPGERATLSCRASQSVSSKLAWYQQKPGQAPRLLMYGA SIRATGIPDRFSGSGSGTEFTLTISSLEPEDFAVYYCQQYGSSSWTFGQGT KVEIKTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGC |
| | | SCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHDG LYQGLSTATKDTYDALHMQALPPR |
| **139106- nt Full CAR** | 971 | ATGGCCCTCCCTGTCACCGCCCTGCTGCTTCCGCTGGCTCTTCTGCTCCAC GCCGCTCGGCCCGAAGTGCAATTGGTGGAAACTGGAGGAGGACTTGTGCAA CCTGGAGGATCATTGAGACTGAGCTGCGCAGTGTCGGGATTCGCCCTGAGC AACCATGGAATGTCCTGGGTCAGAAGGCCCCTGGAAAAGGCCTCGAATGG GTGTCAGGGATCGTGTACTCCGGTTCCACTTACTACGCCGCCTCCGTGAAG GGGCGCTTCACTATCTCACGGGATAACTCCCGCAATACCCTGTACCTCCAA ATGAACAGCCTGCGGCCGGAGGATACCGCCATCTACTACTGTTCCGCCCAC GGTGGAGAGTCTGACGTCTGGGGCCAGGGAACTACCGTGACCGTGTCCTCC GCGTCCGGCGGTGGAGGGAGCGGCGGCCGCGCCAGCGGCGGCGGAGGCTCC GAGATCGTGATGACCCAGAGCCCCGCTACTCTGTCGGTGTCGCCCGGAGAA AGGGCGACCCTGTCCTGCCGGGCGTCGCAGTCCGTGAGCAGCAAGCTGGCT TGGTACCAGCAGAAGCCGGGCCAGGCACCACGCCTGCTTATGTACGGTGCC TCCATTCGGGCCACCGGAATCCCGGACCGGTTCTCGGGGTCGGGGTCCGGT ACCGAGTTCACACTGACCATTTCCTCGCTCGAGCCCGAGGACTTTGCCGTC TATTACTGCCAGCAGTACGGCTCCTCCTCATGGACGTTCGGCCAGGGGACC AAGGTCGAAATCAAGACCACTACCCCAGCACCGAGGCCACCCACCCCGGCT CCTACCATCGCCTCCCAGCCTCTGTCCCTGCGTCCGGAGGCATGTAGACCC GCAGCTGGTGGGGCCGTGCATACCCGGGGTCTTGACTTCGCCTGCGATATC TACATTTGGGCCCCTCTGGCTGGTACTTGCGGGGTCCTGCTGCTTTCACTC GTGATCACTCTTTACTGTAAGCGCGGTCGGAAGAAGCTGCTGTACATCTTT AAGCAACCCTTCATGAGGCCTGTGCAGACTACTCAAGAGGAGGACGGCTGT TCATGCCGGTTCCCAGAGGAGGAGGAAGGCGGCTGCGAACTGCGCGTGAAA TTCAGCCGCAGCGCAGATGCTCCAGCCTACAAGCAGGGGCAGAACCAGCTC TACAACGAACTCAATCTTGGTCGGAGAGAGGAGTACGACGTGCTGGACAAG CGGAGAGGACGGGACCCAGAAATGGGCGGGAAGCCGCGCAGAAAGAATCCC CAAGAGGGCCTGTACAACGAGCTCCAAAAGGATAAGATGGCAGAAGCCTAT AGCGAGATTGGTATGAAAGGGGAACGCAGAAGAGGCAAAGGCCACGACGGA CTGTACCAGGGACTCAGCACCGCCACCAAGGACACCTATGACGCTCTTCAC ATGCAGGCCCTGCCGCCTCGG |

| **139107** | | |
|---|---|---|
| **139107- aa Full CAR** | 957 | MALPVTALLLPLALLLHAARPEVQLVETGGGVVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS EIVLTQSPGTLSLSPGERATLSCRASQSVGSTNLAWYQQKPGQAPRLLIYD ASNRATGIPDRFSGGGSGTDFTLTISRLEPEDFAVYYCQQYGSSPPWTFGQ GTKVEIKTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGH DGLYQGLSTATKDTYDALHMQALPPR |
| **139107- nt Full CAR** | 972 | ATGGCCCTCCCTGTCACCGCCCTGCTGCTTCCGCTGGCTCTTCTGCTCCAC GCCGCTCGGCCCGAAGTGCAATTGGTGGAGACTGGAGGAGGAGTGGTGCAA CCTGGAGGAAGCCTGAGACTGTCATGCGCGGTGTCGGGCTTCGCCCTCTCC AACCACGGAATGTCCTGGGTCCGCCGGGCCCCTGGGAAAGGACTTGAATGG GTGTCCGGCATCGTGTACTCGGGTTCCACCTACTACGCGGCCTCAGTGAAG GGCCGGTTTACTATTAGCCGCGACAACTCCAGAAACACACTGTACCTCCAA ATGAACTCGCTGCGGCCGGAAGATACCGCTATCTACTACTGCTCCGCCCAT GGGGGAGAGTCGGACGTCTGGGGACAGGGCACCACTGTCACTGTGTCCAGC GCTTCCGGCGGTGGTGGAAGCGGGGGACGGGCCTCAGGAGGCGGTGGCAGC |
| | | GAGATTGTGCTGACCCAGTCCCCGGACCCTGAGCCTGTCCCCGGAGAA AGGGCCACCCTCTCCTGTCGGGCATCCCAGTCCGTGGGGTCTACTAACCTT GCATGGTACCAGCAGAAGCCCGGCCAGGCCCCTCGCCTGCTGATCTACGAC GCGTCCAATAGAGCCACCGGCATCCCGGATCGCTTCAGCGGAGGCGGATCG GGCACCGACTTCACCCTCACCATTTCAAGGCTGGAACCGGAGGACTTCGCC GTGTACTACTGCCAGCAGTATGGTTCGTCCCCACCCTGGACGTTCGGCCAG GGGACTAAGGTCGAGATCAAGACCACTACCCCAGCACCGAGGCCACCCACC CCGGCTCCTACCATCGCCTCCCAGCCTCTGTCCCTGCGTCCGGAGGCATGT AGACCCGCAGCTGGTGGGGCCGTGCATACCCGGGGTCTTGACTTCGCCTGC GATATCTACATTTGGGCCCCTCTGGCTGGTACTTGCGGGGTCCTGCTGCTT TCACTCGTGATCACTCTTTACTGTAAGCGCGGTCGGAAGAAGCTGCTGTAC ATCTTTAAGCAACCCTTCATGAGGCCTGTGCAGACTACTCAAGAGGAGGAC GGCTGTTCATGCCGGTTCCCAGAGGAGGAGGAAGGCGGCTGCGAACTGCGC GTGAAATTCAGCCGCAGCGCAGATGCTCCAGCCTACAAGCAGGGGCAGAAC CAGCTCTACAACGAACTCAATCTTGGTCGGAGAGAGGAGTACGACGTGCTG GACAAGCGGAGAGGACGGGACCCAGAAATGGGCGGGAAGCCGCGCAGAAAG AATCCCCAAGAGGGCCTGTACAACGAGCTCCAAAAGGATAAGATGGCAGAA GCCTATAGCGAGATTGGTATGAAAGGGGAACGCAGAAGAGGCAAAGGCCAC GACGGACTGTACCAGGGACTCAGCACCGCCACCAAGGACACCTATGACGCT CTTCACATGCAGGCCCTGCCGCCTCGG |

| **139108** | | |
|---|---|---|
| **139108- aa Full CAR** | 958 | MALPVTALLLPLALLLHAARPQVQLVESGGGLVKPGGSLRLSCAASGFTFS DYYMSWIRQAPGKGLEWVSYISSSGSTIYYADSVKGRFTISRDNAKNSLYL QMNSLRAEDTAVYYCARESGDGMDVWGQGTTVTVSSASGGGGSGGRASGGG GSDIQMTQSPSSLSASVGDRVTITCRASQSISSYLNWYQQKPGKAPKLLIY AASSLQSGVPSRFSGSGSGTDFTLTISSLQPEDFATYYCQQSYTLAFGQGT KVDIKTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGC SCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHDG LYQGLSTATKDTYDALHMQALPPR |
| **139108- nt Full CAR** | 973 | ATGGCCCTCCCTGTCACCGCCCTGCTGCTTCCGCTGGCTCTTCTGCTCCAC GCCGCTCGGCCCCAAGTGCAACTCGTGGAATCTGGTGGAGGACTCGTGAAA CCTGGAGGATCATTGAGACTGTCATGCGCGGCCTCGGGATTCACGTTCTCC GATTACTACATGAGCTGGATTCGCCAGGCTCCGGGGAAGGGACTGGAATGG GTGTCCTACATTTCCTCATCCGGCTCCACCATCTACTACGCGGACTCCGTG AAGGGGAGATTCACCATTAGCCGCGATAACGCCAAGAACAGCCTGTACCTT CAGATGAACTCCCTGCGGGCTGAAGATACTGCCGTCTACTACTGCGCAAGG GAGAGCGGAGATGGGATGGACGTCTGGGGACAGGGTACCACTGTGACCGTG TCGTCGGCCTCCGGCGGAGGGGGTTCGGGTGGAAGGGCCAGCGGCGGCGGA GGCAGCGACATCCAGATGACCCAGTCCCCCTCATCGCTGTCCGCCTCCGTG GGCGACCGCGTCACCATCACATGCCGGGCCTCACAGTCGATCTCCTCCTAC CTCAATTGGTATCAGCAGAAGCCCGGAAAGGCCCCTAAGCTTCTGATCTAC GCAGCGTCCTCCCTGCAATCCGGGGTCCCATCTCGGTTCTCCGGCTCGGGC AGCGGTACCGACTTCACTCTGACCATCTCGAGCCTGCAGCCGGAGGACTTC GCCACTTACTACTGTCAGCAAAGCTACACCCTCGCGTTTGGCCAGGGCACC AAAGTGGACATCAAGACCACTACCCCAGCACCGAGGCCACCCACCCCGGCT CCTACCATCGCCTCCCAGCCTCTGTCCCTGCGTCCGGAGGCATGTAGACCC GCAGCTGGTGGGGCCGTGCATACCCGGGGTCTTGACTTCGCCTGCGATATC TACATTTGGGCCCCTCTGGCTGGTACTTGCGGGGTCCTGCTGCTTTCACTC GTGATCACTCTTTACTGTAAGCGCGGTCGGAAGAAGCTGCTGTACATCTTT AAGCAACCCTTCATGAGGCCTGTGCAGACTACTCAAGAGGAGGACGGCTGT |
| | | TCATGCCGGTTCCCAGAGGAGGAGGAAGGCGGCTGCGAACTGCGCGTGAAA TTCAGCCGCAGCGCAGATGCTCCAGCCTACAAGCAGGGGCAGAACCAGCTC TACAACGAACTCAATCTTGGTCGGAGAGAGGAGTACGACGTGCTGGACAAG CGGAGAGGACGGGACCCAGAAATGGGCGGGAAGCCGCGCAGAAAGAATCCC CAAGAGGGCCTGTACAACGAGCTCCAAAAGGATAAGATGGCAGAAGCCTAT AGCGAGATTGGTATGAAAGGGGAACGCAGAAGAGGCAAAGGCCACGACGGA CTGTACCAGGGACTCAGCACCGCCACCAAGGACACCTATGACGCTCTTCAC ATGCAGGCCCTGCCGCCTCGG |

| **139110** | | |
|---|---|---|
| **139110- aa Full CAR** | 960 | MALPVTALLLPLALLLHAARPQVQLVQSGGGLVKPGGSLRLSCAASGFTFS DYYMSWIRQAPGKGLEWVSYISSSGNTIYYADSVKGRFTISRDNAKNSLYL QMNSLRAEDTAVYYCARSTMVREDYWGQGTLVTVSSASGGGGSGGRASGGG GSDIVLTQSPLSLPVTLGQPASISCKSSESLVHNSGKTYLNWFHQRPGQSP RRLIYEVSNRDSGVPDRFTGSGSGTDFTLKISRVEAEDVGVYYCMQGTHWP GTFGQGTKLEIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **139110- nt Full CAR** | 975 | ATGGCCCTCCCTGTCACCGCCCTGCTGCTTCCGCTGGCTCTTCTGCTCCAC GCCGCTCGGCCCCAAGTGCAACTGGTGCAAAGCGGAGGAGGATTGGTCAAA CCCGGAGGAAGCCTGAGACTGTCATGCGCGGCCTCTGGATTCACCTTCTCC GATTACTACATGTCATGGATCAGACAGGCCCCGGGGAAGGGCCTCGAATGG GTGTCCTACATCTCGTCCTCCGGGAACACCATCTACTACGCCGACAGCGTG AAGGGCCGCTTTACCATTTCCCGCGACAACGCAAAGAACTCGCTGTACCTT CAGATGAATTCCCTGCGGGCTGAAGATACCGCGGTGTACTATTGCGCCCGG TCCACTATGGTCCGGGAGGACTACTGGGGACAGGGCACACTCGTGACCGTG TCCAGCGCGAGCGGGGGTGGAGGCAGCGGTGGACGCGCCTCCGGCGGCGGC GGTTCAGACATCGTGCTGACTCAGTCGCCCCTGTCGCTGCCGGTCACCCTG GGCCAACCGGCCTCAATTAGCTGCAAGTCCTCGGAGAGCCTGGTGCACAAC TCAGGAAAGACTTACCTGAACTGGTTCCATCAGCGGCCTGGACAGTCCCCA CGGAGGCTCATCTATGAAGTGTCCAACAGGGATTCGGGGGTGCCCGACCGC TTCACTGGCTCCGGGTCCGGCACCGACTTCACCTTGAAAATCTCCAGAGTG GAAGCCGAGGACGTGGGCGTGTACTACTGTATGCAGGGTACCCACTGGCCT GGAACCTTTGGACAAGGAACTAAGCTCGAGATTAAGACCACTACCCCAGCA CCGAGGCCACCCACCCCGGCTCCTACCATCGCCTCCCAGCCTCTGTCCCTG CGTCCGGAGGCATGTAGACCCGCAGCTGGTGGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG GACACCTATGACGCTCTTCACATGCAGGCCCTGCCGCCTCGG |

| **139112** | | |
|---|---|---|
| **139112- aa Full CAR** | 961 | MALPVTALLLPLALLLHAARPQVQLVESGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS |
| | | DIRLTQSPSPLSASVGDRVTITCQASEDINKFLNWYHQTPGKAPKLLIYDA STLQTGVPSRFSGSGSGTDFTLTINSLQPEDIGTYYCQQYESLPLTFGGGT KVEIKTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGC SCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHDG LYQGLSTATKDTYDALHMQALPPR |
| **139112- nt Full CAR** | 976 | ATGGCCCTCCCTGTCACCGCCCTGCTGCTTCCGCTGGCTCTTCTGCTCCAC GCCGCTCGGCCCCAAGTGCAACTCGTGGAATCTGGTGGAGGACTCGTGCAA CCCGGTGGAAGCCTTAGGCTGTCGTGCGCCGTCAGCGGGTTTGCTCTGAGC AACCATGGAATGTCCTGGGTCCGCCGGGCACCGGGAAAAGGCTGGAATGG GTGTCCGGCATCGTGTACAGCGGGTCAACCTATTACGCCGCGTCCGTGAAG GGCAGATTCACTATCTCAAGAGACAACAGCCGGAACACCCTGTACTTGCAA ATGAATTCCCTGCGCCCCGAGGACACCGCCATCTACTACTGCTCCGCCCAC GGAGGAGAGTCGGACGTGTGGGGCCAGGGAACGACTGTGACTGTGTCCAGC GCATCAGGAGGGGGTGGTTCGGGCGGCCGGGCCTCGGGGGGAGGAGGTTCC GACATTCGGCTGACCCAGTCCCCGTCCCCACTGTCGGCCTCCGTCGGCGAC CGCGTGACCATCACTTGTCAGGCGTCCGAGGACATTAACAAGTTCCTGAAC TGGTACCACCAGACCCCTGGAAAGGCCCCCAAGCTGCTGATCTACGATGCC TCGACCCTTCAAACTGGAGTGCCTAGCCGGTTCTCCGGGTCCGGCTCCGGC ACTGATTTCACTCTGACCATCAACTCATTGCAGCCGGAAGATATCGGGACC TACTATTGCCAGCAGTACGAATCCCTCCCGCTCACATTCGGCGGGGGAACC AAGGTCGAGATTAAGACCACTACCCCAGCACCGAGGCCACCCACCCCGGCT CCTACCATCGCCTCCCAGCCTCTGTCCCTGCGTCCGGAGGCATGTAGACCC GCAGCTGGTGGGGCCGTGCATACCCGGGGTCTTGACTTCGCCTGCGATATC TACATTTGGGCCCCTCTGGCTGGTACTTGCGGGGTCCTGCTGCTTTCACTC GTGATCACTCTTTACTGTAAGCGCGGTCGGAAGAAGCTGCTGTACATCTTT AAGCAACCCTTCATGAGGCCTGTGCAGACTACTCAAGAGGAGGACGGCTGT TCATGCCGGTTCCCAGAGGAGGAGGAAGGCGGCTGCGAACTGCGCGTGAAA TTCAGCCGCAGCGCAGATGCTCCAGCCTACAAGCAGGGGCAGAACCAGCTC TACAACGAACTCAATCTTGGTCGGAGAGAGGAGTACGACGTGCTGGACAAG CGGAGAGGACGGGACCCAGAAATGGGCGGGAAGCCGCGCAGAAAGAATCCC CAAGAGGGCCTGTACAACGAGCTCCAAAAGGATAAGATGGCAGAAGCCTAT AGCGAGATTGGTATGAAAGGGGAACGCAGAAGAGGCAAAGGCCACGACGGA CTGTACCAGGGACTCAGCACCGCCACCAAGGACACCTATGACGCTCTTCAC ATGCAGGCCCTGCCGCCTCGG |

| **139113** | | |
|---|---|---|
| **139113- aa Full CAR** | 962 | MALPVTALLLPLALLLHAARPEVQLVETGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS ETTLTQSPATLSVSPGERATLSCRASQSVGSNLAWYQQKPGQGPRLLIYGA STRATGIPARFSGSGSGTEFTLTISSLQPEDFAVYYCQQYNDWLPVTFGQG TKVEIKTTTPAPRPPTPAPTIASQPLSLRPEACRPAAGGAVHTRGLDFACD IYIWAPLAGTCGVLLLSLVITLYCKRGRKKLLYIFKQPFMRPVQTTQEEDG CSCRFPEEEEGGCELRVKFSRSADAPAYKQGQNQLYNELNLGRREEYDVLD KRRGRDPEMGGKPRRKNPQEGLYNELQKDKMAEAYSEIGMKGERRRGKGHD GLYQGLSTATKDTYDALHMQALPPR |
| **139113- nt Full CAR** | 977 | ATGGCCCTCCCTGTCACCGCCCTGCTGCTTCCGCTGGCTCTTCTGCTCCAC GCCGCTCGGCCCGAAGTGCAATTGGTGGAAACTGGAGGAGGACTTGTGCAA CCTGGAGGATCATTGCGGCTCTCATGCGCTGTCTCCGGCTTCGCCCTGTCA AATCACGGGATGTCGTGGGTCAGACGGGCCCCGGGAAAGGGTCTGGAATGG GTGTCGGGGATTGTGTACAGCGGCTCCACCTACTACGCCGCTTCGGTCAAG |
| | | GGCCGCTTCACTATTTCACGGACAACAGCCGCAACACCCTCTATCTGCAA ATGAACTCTCTCCGCCCGGAGGATACCGCCATCTACTACTGCTCCGCACAC GGCGGCGAATCCGACGTGTGGGGACAGGGAACCACTGTCACCGTGTCGTCC GCATCCGGTGGCGGAGGATCGGGTGGCCGGGCCTCCGGGGGCGGCGGCAGC GAGACTACCCTGACCCAGTCCCCTGCCACTCTGTCCGTGAGCCCGGGAGAG AGAGCCACCCTTAGCTGCCGGGCCAGCCAGAGCGTGGGCTCCAACCTGGCC TGGTACCAGCAGAAGCCAGGACAGGGTCCCAGGCTGCTGATCTACGGAGCC TCCACTCGCGCGACCGGCATCCCCGCGAGGTTCTCCGGGTCGGGTTCCGGG ACCGAGTTCACCCTGACCATCTCCTCCCTCCAACCGGAGGACTTCGCGGTG TACTACTGTCAGCAGTACAACGATTGGCTGCCCGTGACATTTGGACAGGGG ACGAAGGTGGAAATCAAAACCACTACCCCAGCACCGAGGCCACCCACCCCG GCTCCTACCATCGCCTCCCAGCCTCTGTCCCTGCGTCCGGAGGCATGTAGA CCCGCAGCTGGTGGGGCCGTGCATACCCGGGGTCTTGACTTCGCCTGCGAT ATCTACATTTGGGCCCCTCTGGCTGGTACTTGCGGGGTCCTGCTGCTTTCA CTCGTGATCACTCTTTACTGTAAGCGCGGTCGGAAGAAGCTGCTGTACATC TTTAAGCAACCCTTCATGAGGCCTGTGCAGACTACTCAAGAGGAGGACGGC TGTTCATGCCGGTTCCCAGAGGAGGAGGAAGGCGGCTGCGAACTGCGCGTG AAATTCAGCCGCAGCGCAGATGCTCCAGCCTACAAGCAGGGGCAGAACCAG CTCTACAACGAACTCAATCTTGGTCGGAGAGAGGAGTACGACGTGCTGGAC AAGCGGAGAGGACGGGACCCAGAAATGGGCGGGAAGCCGCGCAGAAAGAAT CCCCAAGAGGGCCTGTACAACGAGCTCCAAAAGGATAAGATGGCAGAAGCC TATAGCGAGATTGGTATGAAAGGGGAACGCAGAAGAGGCAAAGGCCACGAC GGACTGTACCAGGGACTCAGCACCGCCACCAAGGACACCTATGACGCTCTT CACATGCAGGCCCTGCCGCCTCGG |

| **139114** | | |
|---|---|---|
| **139114- aa Full CAR** | 963 | MALPVTALLLPLALLLHAARPEVQLVESGGGLVQPGGSLRLSCAVSGFALS NHGMSWVRRAPGKGLEWVSGIVYSGSTYYAASVKGRFTISRDNSRNTLYLQ MNSLRPEDTAIYYCSAHGGESDVWGQGTTVTVSSASGGGGSGGRASGGGGS EIVLTQSPGTLSLSPGERATLSCRASQSIGSSSLAWYQQKPGQAPRLLMYG ASSRASGIPDRFSGSGSGTDFTLTISRLEPEDFAVYYCQQYAGSPPFTFGQ GTKVEIKTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGH DGLYQGLSTATKDTYDALHMQALPPR |
| **139114- nt Full CAR** | 978 | ATGGCCCTCCCTGTCACCGCCCTGCTGCTTCCGCTGGCTCTTCTGCTCCAC GCCGCTCGGCCCGAAGTGCAATTGGTGGAATCTGGTGGAGGACTTGTGCAA CCTGGAGGATCACTGAGACTGTCATGCGCGGTGTCCGGTTTTGCCCTGAGC AATCATGGGATGTCGTGGGTCCGGCGCGCCCCCGGAAAGGGTCTGGAATGG GTGTCGGGTATCGTCTACTCCGGGAGCACTTACTACGCCGCGAGCGTGAAG GGCCGCTTCACCATTTCCCGCGATAACTCCCGCAACACCCTGTACTTGCAA ATGAACTCGCTCCGGCCTGAGGACACTGCCATCTACTACTGCTCCGCACAC GGAGGAGAATCCGACGTGTGGGGCCAGGGAACTACCGTGACCGTCAGCAGC GCCTCCGGCGGCGGGGGCTCAGGCGGACGGGCTAGCGGCGGCGGTGGCTCC GAGATCGTGCTGACCCAGTCGCCTGGCACTCTCTCGCTGAGCCCCGGGGAA AGGGCAACCCTGTCCTGTCGGGCCAGCCAGTCCATTGGATCATCCTCCCTC GCCTGGTATCAGCAGAAACCGGGACAGGCTCCGCGGCTGCTTATGTATGGG GCCAGCTCAAGAGCCTCCGGCATTCCCGACCGGTTCTCCGGGTCCGGTTCC GGCACCGATTTCACCCTGACTATCTCGAGGCTGGAGCCAGAGGACTTCGCC GTGTACTACTGCCAGCAGTACGCGGGGTCCCCGCCGTTCACGTTCGGACAG GGAACCAAGGTCGAGATCAAGACCACTACCCCAGCACCGAGGCCACCCACC CCGGCTCCTACCATCGCCTCCCAGCCTCTGTCCCTGCGTCCGGAGGCATGT |
| | | AGACCCGCAGCTGGTGGGCCGTGCATACCCGGGGTCTTGACTTCGCCTGC GATATCTACATTTGGGCCCCTCTGGCTGGTACTTGCGGGGTCCTGCTGCTT TCACTCGTGATCACTCTTTACTGTAAGCGCGGTCGGAAGAAGCTGCTGTAC ATCTTTAAGCAACCCTTCATGAGGCCTGTGCAGACTACTCAAGAGGAGGAC GGCTGTTCATGCCGGTTCCCAGAGGAGGAGGAAGGCGGCTGCGAACTGCGC GTGAAATTCAGCCGCAGCGCAGATGCTCCAGCCTACAAGCAGGGGCAGAAC CAGCTCTACAACGAACTCAATCTTGGTCGGAGAGAGGAGTACGACGTGCTG GACAAGCGGAGAGGACGGGACCCAGAAATGGGCGGGAAGCCGCGCAGAAAG AATCCCCAAGAGGGCCTGTACAACGAGCTCCAAAAGGATAAGATGGCAGAA GCCTATAGCGAGATTGGTATGAAAGGGGAACGCAGAAGAGGCAAAGGCCAC GACGGACTGTACCAGGGACTCAGCACCGCCACCAAGGACACCTATGACGCT CTTCACATGCAGGCCCTGCCGCCTCGG |

| **149362** | | |
|---|---|---|
| **149362-aa Full CAR** | 979 | MALPVTALLLPLALLLHAARPQVQLQESGPGLVKPSETLSLTCTVSGGSIS SSYYYWGWIRQPPGKGLEWIGSIYYSGSAYYNPSLKSRVTISVDTSKNQFS LRLSSVTAADTAVYYCARHWQEWPDAFDIWGQGTMVTVSSGGGGSGGGGSG GGGSETTLTQSPAFMSATPGDKVIISCKASQDIDDAMNWYQQKPGEAPLFI IQSATSPVPGIPPRFSGSGFGTDFSLTINNIESEDAAYYFCLQHDNFPLTF GQGTKLEIKTTTPAPRPPTPAPTIASQPLSLRPEACRPAAGGAVHTRGLDF ACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYD VLDKRRGRDPEMGGKPRRKNPQEGLYNELQKDKMAEAYSEIGMKGERRRGK GHDGLYQGLSTATKDTYDALHMQALPPR |
| **149362-nt Full CAR** | 1001 | ATGGCCCTCCCTGTCACCGCCCTGCTGCTTCCGCTGGCTCTTCTGCTCCAC GCCGCTCGGCCCCAAGTGCAGCTTCAGGAAAGCGGACCGGGCCTGGTCAAG CCATCCGAAACTCTCTCCCTGACTTGCACTGTGTCTGGCGGTTCCATCTCA TCGTCGTACTACTACTGGGGCTGGATTAGGCAGCCGCCCGGAAAGGACTG GAGTGGATCGGAAGCATCTACTATTCCGGCTCGGCGTACTACAACCCTAGC CTCAAGTCGAGAGTGACCATCTCCGTGGATACCTCCAAGAACCAGTTTTCC CTGCGCCTGAGCTCCGTGACCGCCGCTGACACCGCCGTGTACTACTGTGCT CGGCATTGGCAGGAATGGCCCGATGCCTTCGACATTTGGGGCCAGGGCACT ATGGTCACTGTGTCATCCGGGGGTGGAGGCAGCGGGGGAGGAGGGTCCGGG GGGGGAGGTTCAGAGACAACCTTGACCCAGTCACCCGCATTCATGTCCGCC ACTCCGGGAGACAAGGTCATCATCTCGTGCAAAGCGTCCCAGGATATCGAC GATGCCATGAATTGGTACCAGCAGAAGCCTGGCGAAGCGCCGCTGTTCATT ATCCAATCCGCAACCTCGCCCGTGCCTGGAATCCCACCGCGGTTCAGCGGC AGCGGTTTCGGAACCGACTTTTCCCTGACCATTAACAACATTGAGTCCGAG GACGCCGCCTACTACTTCTGCCTGCAACACGACAACTTCCCTCTCACGTTC GGCCAGGGAACCAAGCTGGAAATCAAGACCACTACCCCAGCACCGAGGCCA CCCACCCCGGCTCCTACCATCGCCTCCCAGCCTCTGTCCCTGCGTCCGGAG GCATGTAGACCCGCAGCTGGTGGGGCCGTGCATACCCGGGGTCTTGACTTC GCCTGCGATATCTACATTTGGGCCCCTCTGGCTGGTACTTGCGGGGTCCTG CTGCTTTCACTCGTGATCACTCTTTACTGTAAGCGCGGTCGGAAGAAGCTG CTGTACATCTTTAAGCAACCCTTCATGAGGCCTGTGCAGACTACTCAAGAG GAGGACGGCTGTTCATGCCGGTTCCCAGAGGAGGAGGAAGGCGGCTGCGAA CTGCGCGTGAAATTCAGCCGCAGCGCAGATGCTCCAGCCTACAAGCAGGGG CAGAACCAGCTCTACAACGAACTCAATCTTGGTCGGAGAGAGGAGTACGAC GTGCTGGACAAGCGGAGAGGACGGGACCCAGAAATGGGCGGGAAGCCGCGC AGAAAGAATCCCCAAGAGGGCCTGTACAACGAGCTCCAAAAGGATAAGATG GCAGAAGCCTATAGCGAGATTGGTATGAAAGGGGAACGCAGAAGAGGCAAA GGCCACGACGGACTGTACCAGGGACTCAGCACCGCCACCAAGGACACCTAT GACGCTCTTCACATGCAGGCCCTGCCGCCTCGG |

| **149363** | | |
|---|---|---|
| **149363-aa Full CAR** | 980 | MALPVTALLLPLALLLHAARPQVNLRESGPALVKPTQTLTLTCTFSGFSLR TSGMCVSWIRQPPGKALEWLARIDWDEDKFYSTSLKTRLTISKDTSDNQVV LRMTNMDPADTATYYCARSGAGGTSATAFDIWGPGTMVTVSSGGGGSGGGG SGGGGSDIQMTQSPSSLSASVGDRVTITCRASQDIYNNLAWFQLKPGSAPR SLMYAANKSQSGVPSRFSGSASGTDFTLTISSLQPEDFATYYCQHYYRFPY SFGQGTKLEIKTTTPAPRPPTPAPTIASQPLSLRPEACRPAAGGAVHTRGL DFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERRR GKGHDGLYQGLSTATKDTYDALHMQALPPR |
| **149363-nt Full CAR** | 1002 | ATGGCCCTCCCTGTCACCGCCCTGCTGCTTCCGCTGGCTCTTCTGCTCCAC GCCGCTCGGCCCCAAGTCAATCTGCGCGAATCCGGCCCCGCCTTGGTCAAG CCTACCCAGACCCTCACTCTGACCTGTACTTTCTCCGGCTTCTCCCTGCGG ACTTCCGGGATGTGCGTGTCCTGGATCAGACAGCCTCCGGGAAAGGCCCTG GAGTGGCTCGCTCGCATTGACTGGGATGAGGACAAGTTCTACTCCACCTCA CTCAAGACCAGGCTGACCATCAGCAAAGATACCTCTGACAACCAAGTGGTG CTCCGCATGACCAACATGGACCCAGCCGACACTGCCACTTACTACTGCGCG AGGAGCGGAGCGGGCGGAACCTCCGCCACCGCCTTCGATATTTGGGGCCCG GGTACCATGGTCACCGTGTCAAGCGGAGGAGGGGGTCCGGGGGCGGCGGT TCCGGGGGAGGCGGATCGGACATTCAGATGACTCAGTCACCATCGTCCCTG AGCGCTAGCGTGGGCGACAGAGTGACAATCACTTGCCGGGCATCCCAGGAC ATCTATAACAACCTTGCGTGGTTCCAGCTGAAGCCTGGTTCCGCACCGCGG TCACTTATGTACGCCGCCAACAAGAGCCAGTCGGGAGTGCCGTCCCGGTTT TCCGGTTCGGCCTCGGGAACTGACTTCACCCTGACGATCTCCAGCCTGCAA CCCGAGGATTTCGCCACCTACTACTGCCAGCACTACTACCGCTTTCCCTAC TCGTTCGGACAGGGAACCAAGCTGGAAATCAAGACCACTACCCCAGCACCG AGGCCACCCACCCCGGCTCCTACCATCGCCTCCCAGCCTCTGTCCCTGCGT CCGGAGGCATGTAGACCCGCAGCTGGTGGGGCCGTGCATACCCGGGGTCTT GACTTCGCCTGCGATATCTACATTTGGGCCCCTCTGGCTGGTACTTGCGGG GTCCTGCTGCTTTCACTCGTGATCACTCTTTACTGTAAGCGCGGTCGGAAG AAGCTGCTGTACATCTTTAAGCAACCCTTCATGAGGCCTGTGCAGACTACT CAAGAGGAGGACGGCTGTTCATGCCGGTTCCCAGAGGAGGAGGAAGGCGGC TGCGAACTGCGCGTGAAATTCAGCCGCAGCGCAGATGCTCCAGCCTACAAG CAGGGGCAGAACCAGCTCTACAACGAACTCAATCTTGGTCGGAGAGAGGAG TACGACGTGCTGGACAAGCGGAGAGGACGGGACCCAGAAATGGGCGGAAG CCGCGCAGAAAGAATCCCCAAGAGGGCCTGTACAACGAGCTCCAAAAGGAT AAGATGGCAGAAGCCTATAGCGAGATTGGTATGAAAGGGGAACGCAGAAGA GGCAAAGGCCACGACGGACTGTACCAGGGACTCAGCACCGCCACCAAGGAC ACCTATGACGCTCTTCACATGCAGGCCCTGCCGCCTCGG |

| **149364** | | |
|---|---|---|
| **149364-aa Full CAR** | 981 | MALPVTALLLPLALLLHAARPEVQLVESGGGLVKPGGSLRLSCAASGFTFS SYSMNWVRQAPGKGLEWVSSISSSSSYIYYADSVKGRFTISRDNAKNSLYL QMNSLRAEDTAVYYCAKTIAAVYAFDIWGQGTTVTVSSGGGGSGGGGSGGG GSEIVLTQSPLSLPVTPEEPASISCRSSQSLLHSNGYNYLDWYLQKPGQSP QLLIYLGSNRASGVPDRFSGSGSGTDFTLKISRVEAEDVGVYYCMQALQTP YTFGQGTKLEIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **149364-nt Full CAR** | 1003 | ATGGCCCTCCCTGTCACCGCCCTGCTGCTTCCGCTGGCTCTTCTGCTCCAC |
| | | GCCGCTCGGCCCGAAGTGCAGCTTGTCGAATCCGGGGGGGACTGGTCAAG CCGGGCGGATCACTGAGACTGTCCTGCGCCGCGAGCGGCTTCACGTTCTCC TCCTACTCCATGAACTGGGTCCGCCAAGCCCCCGGGAAGGGACTGGAATGG GTGTCCTCTATCTCCTCGTCGTCGTCCTACATCTACTACGCCGACTCCGTG AAGGGAAGATTCACCATTTCCCGCGACAACGCAAAGAACTCACTGTACTTG CAAATGAACTCACTCCGGGCCGAAGATACTGCTGTGTACTATTGCGCCAAG ACTATTGCCGCCGTCTACGCTTTCGACATCTGGGGCCAGGGAACCACCGTG ACTGTGTCGTCCGGTGGTGGTGGCTCGGGCGGAGGAGGAAGCGGCGGCGGG GGGTCCGAGATTGTGCTGACCCAGTCGCCACTGAGCCTCCCTGTGACCCCC GAGGAACCCGCCAGCATCAGCTGCCGGTCCAGCCAGTCCCTGCTCCACTCC AACGGATACAATTACCTCGATTGGTACCTTCAGAAGCCTGGACAAAGCCCG CAGCTGCTCATCTACTTGGGATCAAACCGCGCGTCAGGAGTGCCTGACCGG TTCTCCGGCTCGGGCAGCGGTACCGATTTCACCCTGAAAATCTCCAGGGTG GAGGCAGAGGACGTGGGAGTGTATTACTGTATGCAGGCGCTGCAGACTCCG TACACATTTGGGCAGGGCACCAAGCTGGAGATCAAGACCACTACCCCAGCA CCGAGGCCACCCACCCCGGCTCCTACCATCGCCTCCCAGCCTCTGTCCCTG CGTCCGGAGGCATGTAGACCCGCAGCTGGTGGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG GACACCTATGACGCTCTTCACATGCAGGCCCTGCCGCCTCGG |

| **149365** | | |
|---|---|---|
| **149365-aa Full CAR** | 982 | MALPVTALLLPLALLLHAARPEVQLVESGGGLVKPGGSLRLSCAASGFTFS DYYMSWIRQAPGKGLEWVSYISSSGSTIYYADSVKGRFTISRDNAKNSLYL QMNSLRAEDTAVYYCARDLRGAFDIWGQGTMVTVSSGGGGSGGGGSGGGGS SYVLTQSPSVSAAPGYTATISCGGNNIGTKSVHWYQQKPGQAPLLVIRDDS VRPSKIPGRFSGSNSGNMATLTISGVQAGDEADFYCQVWDSDSEHVVFGGG TKLTVLTTTPAPRPPTPAPTIASQPLSLRPEACRPAAGGAVHTRGLDFACD IYIWAPLAGTCGVLLLSLVITLYCKRGRKKLLYIFKQPFMRPVQTTQEEDG CSCRFPEEEEGGCELRVKFSRSADAPAYKQGQNQLYNELNLGRREEYDVLD KRRGRDPEMGGKPRRKNPQEGLYNELQKDKMAEAYSEIGMKGERRRGKGHD GLYQGLSTATKDTYDALHMQALPPR |
| **149365-nt Full CAR** | 1004 | ATGGCCCTCCCTGTCACCGCCCTGCTGCTTCCGCTGGCTCTTCTGCTCCAC GCCGCTCGGCCCGAAGTCCAGCTCGTGGAGTCCGGCGGAGGCCTTGTGAAG CCTGGAGGTTCGCTGAGACTGTCCTGCGCCGCCTCCGGCTTCACCTTCTCC GACTACTACATGTCCTGGATCAGACAGGCCCCGGGAAAGGGCCTGGAATGG GTGTCCTACATCTCGTCATCGGGCAGCACTATCTACTACGCGGACTCAGTG AAGGGGCGGTTCACCATTTCCCGGGATAACGCGAAGAACTCGCTGTATCTG CAAATGAACTCACTGAGGGCCGAGGACACCGCCGTGTACTACTGCGCCCGC GATCTCCGCGGGGCATTTGACATCTGGGGACAGGGAACCATGGTCACAGTG TCCAGCGGAGGGGGAGGATCGGGTGGCGGAGGTTCCGGGGGTGGAGGCTCC TCCTACGTGCTGACTCAGAGCCCAAGCGTCAGCGCTGCGCCCGGTTACACG GCAACCATCTCCTGTGGCGGAAACAACATTGGGACCAAGTCTGTGCACTGG TATCAGCAGAAGCCGGGCCAAGCTCCCCTGTTGGTGATCCGCGATGACTCC GTGCGGCCTAGCAAAATTCCGGGACGGTTCTCCGGCTCCAACAGCGGCAAT |
| | | ATGGCCACTCTCACCATCTCGGGAGTGCAGGCCGGAGATGAAGCCGACTTC TACTGCCAAGTCTGGGACTCAGACTCCGAGCATGTGGTGTTCGGGGGCGGA ACCAAGCTGACTGTGCTCACCACTACCCCAGCACCGAGGCCACCCACCCCG GCTCCTACCATCGCCTCCCAGCCTCTGTCCCTGCGTCCGGAGGCATGTAGA CCCGCAGCTGGTGGGGCCGTGCATACCCGGGGTCTTGACTTCGCCTGCGAT ATCTACATTTGGGCCCCTCTGGCTGGTACTTGCGGGGTCCTGCTGCTTTCA CTCGTGATCACTCTTTACTGTAAGCGCGGTCGGAAGAAGCTGCTGTACATC TTTAAGCAACCCTTCATGAGGCCTGTGCAGACTACTCAAGAGGAGGACGGC TGTTCATGCCGGTTCCCAGAGGAGGAGGAAGGCGGCTGCGAACTGCGCGTG AAATTCAGCCGCAGCGCAGATGCTCCAGCCTACAAGCAGGGGCAGAACCAG CTCTACAACGAACTCAATCTTGGTCGGAGAGAGGAGTACGACGTGCTGGAC AAGCGGAGAGGACGGGACCCAGAAATGGGCGGGAAGCCGCGCAGAAAGAAT CCCCAAGAGGGCCTGTACAACGAGCTCCAAAAGGATAAGATGGCAGAAGCC TATAGCGAGATTGGTATGAAAGGGGAACGCAGAAGAGGCAAAGGCCACGAC GGACTGTACCAGGGACTCAGCACCGCCACCAAGGACACCTATGACGCTCTT CACATGCAGGCCCTGCCGCCTCGG |

| **149366** | | |
|---|---|---|
| **149366-aa Full CAR** | 983 | MALPVTALLLPLALLLHAARPQVQLVQSGAEVKKPGASVKVSCKPSGYTVT SHYIHWVRRAPGQGLEWMGMINPSGGVTAYSQTLQGRVTMTSDTSSSTVYM ELSSLRSEDTAMYYCAREGSGSGWYFDFWGRGTLVTVSSGGGGSGGGGSGG GGSSYVLTQPPSVSVSPGQTASITCSGDGLSKKYVSWYQQKAGQSPVVLIS RDKERPSGIPDRFSGSNSADTATLTISGTQAMDEADYYCQAWDDTTVVFGG GTKLTVLTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGH DGLYQGLSTATKDTYDALHMQALPPR |
| **149366-nt Full CAR** | 1005 | ATGGCCCTCCCTGTCACCGCCCTGCTGCTTCCGCTGGCTCTTCTGCTCCAC GCCGCTCGGCCCCAAGTGCAGCTGGTGCAGAGCGGGGCCGAAGTCAAGAAG CCGGGAGCCTCCGTGAAAGTGTCCTGCAAGCCTTCGGGATACACCGTGACC TCCCACTACATTCATTGGGTCCGCCGCGCCCCCGGCCAAGGACTCGAGTGG ATGGGCATGATCAACCCTAGCGGCGGAGTGACCGCGTACAGCCAGACGCTG CAGGGACGCGTGACTATGACCTCGGATACCTCCTCCACCGTCTATATG GAACTGTCCAGCCTGCGGTCCGAGGATACCGCCATGTACTACTGCGCCCGG GAAGGATCAGGCTCCGGGTGGTATTTCGACTTCTGGGGAAGAGGCACCCTC GTGACTGTGTCATCTGGGGGAGGGGGTTCCGGTGGTGGCGGATCGGGAGGA GGCGGTTCATCCTACGTGCTGACCCAGCCACCCTCCGTGTCCGTGAGCCCC GGCCAGACTGCATCGATTACATGTAGCGGCGACGGCCTCTCCAAGAAATAC GTGTCGTGGTACCAGCAGAAGGCCGGACAGAGCCCGGTGGTGCTGATCTCA AGAGATAAGGAGCGGCCTAGCGGAATCCCGGACAGGTTCTCGGGTTCCAAC TCCGCGGACACTGCTACTCTGACCATCTCGGGGACCCAGGCTATGGACGAA GCCGATTACTACTGCCAAGCCTGGGACGACACTACTGTCGTGTTTGGAGGG GGCACCAAGTTGACCGTCCTTACCACTACCCCAGCACCGAGGCCACCCACC CCGGCTCCTACCATCGCCTCCCAGCCTCTGTCCCTGCGTCCGGAGGCATGT AGACCCGCAGCTGGTGGGGCCGTGCATACCCGGGGTCTTGACTTCGCCTGC GATATCTACATTTGGGCCCCTCTGGCTGGTACTTGCGGGGTCCTGCTGCTT TCACTCGTGATCACTCTTTACTGTAAGCGCGGTCGGAAGAAGCTGCTGTAC ATCTTTAAGCAACCCTTCATGAGGCCTGTGCAGACTACTCAAGAGGAGGAC GGCTGTTCATGCCGGTTCCCAGAGGAGGAGGAAGGCGGCTGCGAACTGCGC GTGAAATTCAGCCGCAGCGCAGATGCTCCAGCCTACAAGCAGGGGCAGAAC CAGCTCTACAACGAACTCAATCTTGGTCGGAGAGAGGAGTACGACGTGCTG GACAAGCGGAGAGGACGGGACCCAGAAATGGGCGGGAAGCCGCGCAGAAAG |
| | | AATCCCCAAGAGGGCCTGTACAACGAGCTCCAAAAGGATAAGATGGCAGAA GCCTATAGCGAGATTGGTATGAAAGGGGAACGCAGAAGAGGCAAAGGCCAC GACGGACTGTACCAGGGACTCAGCACCGCCACCAAGGACACCTATGACGCT CTTCACATGCAGGCCCTGCCGCCTCGG |

| **149367** | | |
|---|---|---|
| **149367-aa Full CAR** | 984 | MALPVTALLLPLALLLHAARPQVQLQESGPGLVKPSQTLSLTCTVSGGSIS SGGYYWSWIRQHPGKGLEWIGYIYYSGSTYYNPSLKSRVTISVDTSKNQFS LKLSSVTAADTAVYYCARAGIAARLRGAFDIWGQGTMVTVSSGGGGSGGGG SGGGGSDIVMTQSPSSVSASVGDRVIITCRASQGIRNWLAWYQQKPGKAPN LLIYAASNLQSGVPSRFSGSGSGADFTLTISSLQPEDVATYYCQKYNSAPF TFGPGTKVDIKTTTPAPRPPTPAPTIASQPLSLRPEACRPAAGGAVHTRGL DFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERRR GKGHDGLYQGLSTATKDTYDALHMQALPPR |
| **149367-nt Full CAR** | 1006 | ATGGCCCTCCCTGTCACCGCCCTGCTGCTTCCGCTGGCTCTTCTGCTCCAC GCCGCTCGGCCCCAAGTGCAGCTTCAGGAGAGCGGCCCGGGACTCGTGAAG CCGTCCCAGACCCTGTCCCTGACTTGCACCGTGTCGGGAGGAAGCATCTCG AGCGGAGGCTACTATTGGTCGTGGATTCGGCAGCACCCTGGAAAGGGCCTG GAATGGATCGGCTACATCTACTACTCCGGCTCGACCTACTACAACCCATCG CTGAAGTCCAGAGTGACAATCTCAGTGGACACGTCCAAGAATCAGTTCAGC CTGAAGCTCTCTTCCGTGACTGCGGCCGACACCGCCGTGTACTACTGCGCA CGCGCTGGAATTGCCGCCCGGCTGAGGGGTGCCTTCGACATTTGGGGACAG GGCACCATGGTCACCGTGTCCTCCGGCGGCGGAGGTTCCGGGGGTGGAGGC TCAGGAGGAGGGGGGTCCGACATCGTCATGACTCAGTCGCCCTCAAGCGTC AGCGCGTCCGTCGGGGACAGAGTGATCATCACCTGTCGGGCGTCCCAGGGA ATTCGCAACTGGCTGGCCTGGTATCAGCAGAAGCCCGGAAAGGCCCCCAAC CTGTTGATCTACGCCGCCTCAAACCTCCAATCCGGGGTGCCGAGCCGCTTC AGCGGCTCCGGTTCGGGTGCCGATTTCACTCTGACCATCTCCTCCCTGCAA CCTGAAGATGTGGCTACCTACTACTGCCAAAAGTACAACTCCGCACCTTTT ACTTTCGGACCGGGGACCAAAGTGGACATTAAGACCACTACCCCAGCACCG AGGCCACCCACCCCGGCTCCTACCATCGCCTCCCAGCCTCTGTCCCTGCGT CCGGAGGCATGTAGACCCGCAGCTGGTGGGGCCGTGCATACCCGGGGTCTT GACTTCGCCTGCGATATCTACATTTGGGCCCCTCTGGCTGGTACTTGCGGG GTCCTGCTGCTTTCACTCGTGATCACTCTTTACTGTAAGCGCGGTCGGAAG AAGCTGCTGTACATCTTTAAGCAACCCTTCATGAGGCCTGTGCAGACTACT CAAGAGGAGGACGGCTGTTCATGCCGGTTCCCAGAGGAGGAGGAAGGCGGC TGCGAACTGCGCGTGAAATTCAGCCGCAGCGCAGATGCTCCAGCCTACAAG CAGGGGCAGAACCAGCTCTACAACGAACTCAATCTTGGTCGGAGAGAGGAG TACGACGTGCTGGACAAGCGGAGAGGACGGGACCCAGAAATGGGCGGAAG CCGCGCAGAAAGAATCCCCAAGAGGGCCTGTACAACGAGCTCCAAAAGGAT AAGATGGCAGAAGCCTATAGCGAGATTGGTATGAAAGGGGAACGCAGAAGA GGCAAAGGCCACGACGGACTGTACCAGGGACTCAGCACCGCCACCAAGGAC ACCTATGACGCTCTTCACATGCAGGCCCTGCCGCCTCGG |

| **149368** | | |
|---|---|---|
| **149368-aa Full CAR** | 985 | MALPVTALLLPLALLLHAARPQVQLVQSGAEVKKPGSSVKVSCKASGGTFS SYAISWVRQAPGQGLEWMGGIIPIFGTANYAQKFQGRVTITADESTSTAYM ELSSLRSEDTAVYYCARRGGYQLLRWDVGLLRSAFDIWGQGTMVTVSSGGG GSGGGGSGGGGSSYVLTQPPSVSVAPGQTARITCGGNNIGSKSVHWYQQKP GQAPVLVLYGKNNRPSGVPDRFSGSRSGTTASLTITGAQAEDEADYYCSSR DSSGDHLRVFGTGTKVTVLTTTPAPRPPTPAPTIASQPLSLRPEACRPAAG GAVHTRGLDFACDIYIWAPLAGTCGVLLLSLVITLYCKRGRKKLLYIFKQP |
| | | FMRPVQTTQEEDGCSCRFPEEEEGGCELRVKFSRSADAPAYKQGQNQLYNE LNLGRREEYDVLDKRRGRDPEMGGKPRRKNPQEGLYNELQKDKMAEAYSEI GMKGERRRGKGHDGLYQGLSTATKDTYDALHMQALPPR |
| **149368-nt Full CAR** | 1007 | ATGGCCCTCCCTGTCACCGCCCTGCTGCTTCCGCTGGCTCTTCTGCTCCAC GCCGCTCGGCCCCAAGTGCAGCTGGTCCAGTCGGGCGCCGAGGTCAAGAAG CCCGGAGCTCTGTGAAAGTGTCCTGCAAGGCCTCCGGGGGCACCTTTAGC TCCTACGCCATCTCCTGGGTCCGCCAAGCACCGGGTCAAGGCCTGGAGTGG ATGGGGGGAATTATCCCTATCTTCGGCACTGCCAACTACGCCCAGAAGTTC CAGGGACGCGTGACCATTACCGCGGACGAATCCACCTCCACCGCTTATATG GAGCTGTCCAGCTTGCGCTCGGAAGATACCGCCGTGTACTACTGCGCCCGG AGGGGTGGATACCAGCTGCTGAGATGGGACGTGGGCCTCCTGCGGTCGGCG TTCGACATCTGGGGCCAGGGCACTATGGTCACTGTGTCCAGCGGAGGAGGC GGATCGGGAGGCGGCGGATCAGGGGGAGGCGGTTCCAGCTACGTGCTTACT CAACCCCCTTCGGTGTCCGTGGCCCCGGGACAGACCGCCAGAATCACTTGC GGAGGAAACAACATTGGGTCCAAGAGCGTGCATTGGTACCAGCAGAAGCCA GGACAGGCCCCTGTGCTGGTGCTCTACGGAAGAACAATCGGCCCAGCGGA GTGCCGGACAGGTTCTCGGGTTCACGCTCCGGTACAACCGCTTCACTGACT ATCACCGGGGCCCAGGCAGAGGATGAAGCGGACTACTACTGTTCCTCCCGG GATTCATCCGGCGACCACCTCCGGGTGTTCGGAACCGGAACGAAGGTCACC GTGCTGACCACTACCCCAGCACCGAGGCCACCCACCCCGGCTCCTACCATC GCCTCCCAGCCTCTGTCCCTGCGTCCGGAGGCATGTAGACCCGCAGCTGGT GGGGCCGTGCATACCCGGGGTCTTGACTTCGCCTGCGATATCTACATTTGG GCCCCTCTGGCTGGTACTTGCGGGGTCCTGCTGCTTTCACTCGTGATCACT CTTTACTGTAAGCGCGGTCGGAAGAAGCTGCTGTACATCTTTAAGCAACCC TTCATGAGGCCTGTGCAGACTACTCAAGAGGAGGACGGCTGTTCATGCCGG TTCCCAGAGGAGGAGGAAGGCGGCTGCGAACTGCGCGTGAAATTCAGCCGC AGCGCAGATGCTCCAGCCTACAAGCAGGGGCAGAACCAGCTCTACAACGAA CTCAATCTTGGTCGGAGAGAGGAGTACGACGTGCTGGACAAGCGGAGAGGA CGGGACCCAGAAATGGGCGGGAAGCCGCGCAGAAAGAATCCCCAAGAGGGC CTGTACAACGAGCTCCAAAAGGATAAGATGGCAGAAGCCTATAGCGAGATT GGTATGAAAGGGGAACGCAGAAGAGGCAAAGGCCACGACGGACTGTACCAG GGACTCAGCACCGCCACCAAGGACACCTATGACGCTCTTCACATGCAGGCC CTGCCGCCTCGG |

| **149369** | | |
|---|---|---|
| **149369-aa Full CAR** | 986 | MALPVTALLLPLALLLHAARPEVQLQQSGPGLVKPSQTLSLTCAISGDSVS SNSAAWNWIRQSPSRGLEWLGRTYYRSKWYSFYAISLKSRIIINPDTSKNQ FSLQLKSVTPEDTAVYYCARSSPEGLFLYWFDPWGQGTLVTVSSGGDGSGG GGSGGGGSSSELTQDPAVSVALGQTIRITCQGDSLGNYYATWYQQKPGQAP VLVIYGTNNRPSGIPDRFSASSSGNTASLTITGAQAEDEADYYCNSRDSSG HHLLFGTGTKVTVLTTTPAPRPPTPAPTIASQPLSLRPEACRPAAGGAVHT RGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNLGR REEYDVLDKRRGRDPEMGGKPRRKNPQEGLYNELQKDKMAEAYSEIGMKGE RRRGKGHDGLYQGLSTATKDTYDALHMQALPPR |
| **149369-nt Full CAR** | 1008 | ATGGCCCTCCCTGTCACCGCCCTGCTGCTTCCGCTGGCTCTTCTGCTCCAC GCCGCTCGGCCCGAAGTGCAGCTCCAACAGTCAGGACCGGGGCTCGTGAAG CCATCCCAGACCCTGTCCCTGACTTGTGCCATCTCGGGAGATAGCGTGTCA TCGAACTCCGCCGCCTGGAACTGGATTCGGCAGAGCCCGTCCCGCGGACTG GAGTGGCTTGGAAGGACCTACTACCGGTCCAAGTGGTACTCTTTCTACGCG ATCTCGCTGAAGTCCCGCATTATCATTAACCCTGATACCTCCAAGAATCAG TTCTCCCTCCAACTGAAATCCGTCACCCCCGAGGACACAGCAGTGTATTAC TGCGCACGGAGCAGCCCCGAAGGACTGTTCCTGTATTGGTTTGACCCCTGG |
| | | GGCCAGGGGACTCTTGTGACCGTGTCGAGCGGCGGAGATGGGTCCGGTGGC GGTGGTTCGGGGGGCGGCGGATCATCATCCGAACTGACCCAGGACCCGGCT GTGTCCGTGGCGCTGGGACAAACCATCCGCATTACGTGCCAGGGAGACTCC CTGGGCAACTACTACGCCACTTGGTACCAGCAGAAGCCGGGCCAAGCCCCT GTGTTGGTCATCTACGGACCAACAACAGACCTTCCGGCATCCCCGACCGG TTCAGCGCTTCGTCCTCCGGCAACACTGCCAGCCTGACCATCACTGGAGCG CAGGCCGAAGATGAGGCCGACTACTACTGCAACAGCAGAGACTCCTCGGGT CATCACCTCTTGTTCGGAACTGGAACCAAGGTCACCGTGCTGACCACTACC CCAGCACCGAGGCCACCCACCCCGGCTCCTACCATCGCCTCCCAGCCTCTG TCCCTGCGTCCGGAGGCATGTAGACCCGCAGCTGGTGGGGCCGTGCATACC CGGGGTCTTGACTTCGCCTGCGATATCTACATTTGGGCCCCTCTGGCTGGT ACTTGCGGGGTCCTGCTGCTTTCACTCGTGATCACTCTTTACTGTAAGCGC GGTCGGAAGAAGCTGCTGTACATCTTTAAGCAACCCTTCATGAGGCCTGTG CAGACTACTCAAGAGGAGGACGGCTGTTCATGCCGGTTCCCAGAGGAGGAG GAAGGCGGCTGCGAACTGCGCGTGAAATTCAGCCGCAGCGCAGATGCTCCA GCCTACAAGCAGGGGCAGAACCAGCTCTACAACGAACTCAATCTTGGTCGG AGAGAGGAGTACGACGTGCTGGACAAGCGGAGAGGACGGGACCCAGAAATG GGCGGGAAGCCGCGCAGAAAGAATCCCCAAGAGGGCCTGTACAACGAGCTC CAAAAGGATAAGATGGCAGAAGCCTATAGCGAGATTGGTATGAAAGGGGAA CGCAGAAGAGGCAAAGGCCACGACGGACTGTACCAGGGACTCAGCACCGCC ACCAAGGACACCTATGACGCTCTTCACATGCAGGCCCTGCCGCCTCGG |

| **BCMA EBB-C1978-A4** | | |
|---|---|---|
| **BCMA_EBB-C1978-A4 - aa Full CART** | 987 | MALPVTALLLPLALLLHAARPEVQLVESGGGLVQPGGSLRLSCAASGFTFS SYAMSWVRQAPGKGLEWVSAISGSGGSTYYADSVKGRFTISRDNSKNTLYL QMNSLRAEDTAVYYCAKVEGSGSLDYWGQGTLVTVSSGGGGSGGGGSGGGG SEIVMTQSPGTLSLSPGERATLSCRASQSVSSAYLAWYQQKPGQPPRLLIS GASTRATGIPDRFGGSGSGTDFTLTISRLEPEDFAVYYCQHYGSSFNGSSL FTFGQGTRLEIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **BCMA_EBB-C1978-A4 - nt Full CART** | 1009 | ATGGCCCTCCCTGTCACCGCCCTGCTGCTTCCGCTGGCTCTTCTGCTCCAC GCCGCTCGGCCCGAAGTGCAGCTCGTGGAGTCAGGAGGCGGCCTGGTCCAG CCGGGAGGGTCCCTTAGACTGTCATGCGCCGCAAGCGGATTCACTTTCTCC TCCTATGCCATGAGCTGGGTCCGCCAAGCCCCCGGAAAGGACTGGAATGG GTGTCCGCCATCTCGGGGTCTGGAGGCTCAACTTACTACGCTGACTCCGTG AAGGGACGGTTCACCATTAGCCGCGACAACTCCAAGAACACCCTCTACCTC CAAATGAACTCCCTGCGGGCCGAGGATACCGCCGTCTACTACTGCGCCAAA GTGGAAGGTTCAGGATCGCTGGACTACTGGGGACAGGGTACTCTCGTGACC GTGTCATCGGGCGGAGGAGGTTCCGGCGGTGGCGGCTCCGGCGGCGGAGGG TCGGAGATCGTGATGACCCAGAGCCCTGGTACTCTGAGCCTTTCGCCGGGA GAAAGGGCCACCCTGTCCTGCCGCGCTTCCCAATCCGTGTCCTCCGCGTAC TTGGCGTGGTACCAGCAGAAGCCGGGACAGCCCCCTCGGCTGCTGATCAGC GGGGCCAGCACCCGGGCAACCGGAATCCCAGACAGATTCGGGGGTTCCGGC AGCGGCACAGATTTCACCCTGACTATTTCGAGGTTGGAGCCCGAGGACTTT GCGGTGTATTACTGTCAGCACTACGGGTCGTCCTTTAATGGCTCCAGCCTG TTCACGTTCGGACAGGGGACCCGCCTGGAAATCAAGACCACTACCCCAGCA CCGAGGCCACCCACCCCGGCTCCTACCATCGCCTCCCAGCCTCTGTCCCTG CGTCCGGAGGCATGTAGACCCGCAGCTGGTGGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG |
| | | AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG GACACCTATGACGCTCTTCACATGCAGGCCCTGCCGCCTCGG |

| **BCMA EBB-C1978-G1** | | |
|---|---|---|
| **BCMA_EBB-C1978-G1 - aa Full CART** | 988 | MALPVTALLLPLALLLHAARPEVQLVETGGGLVQPGGSLRLSCAASGITFS RYPMSWVRQAPGKGLEWVSGISDSGVSTYYADSAKGRFTISRDNSKNTLFL QMSSLRDEDTAVYYCVTRAGSEASDIWGQGTMVTVSSGGGGSGGGGSGGGG SEIVLTQSPATLSLSPGERATLSCRASQSVSNSLAWYQQKPGQAPRLLIYD ASSRATGIPDRFSGSGSGTDFTLTISRLEPEDFAIYYCQQFGTSSGLTFGG GTKLEIKTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGH DGLYQGLSTATKDTYDALHMQALPPR |
| **BCMA_EBB-C1978-G1 - nt Full CART** | 1010 | ATGGCCCTCCCTGTCACCGCCCTGCTGCTTCCGCTGGCTCTTCTGCTCCAC GCCGCTCGGCCCGAAGTGCAACTGGTGGAAACCGGTGGCGGCCTGGTGCAG CCTGGAGGATCATTGAGGCTGTCATGCGCGGCCAGCGGTATTACCTTCTCC CGGTACCCCATGTCCTGGGTCAGACAGGCCCCGGGGAAAGGGCTTGAATGG GTGTCCGGGATCTCGGACTCCGGTGTCAGCACTTACTACGCCGACTCCGCC AAGGGACGCTTCACCATTTCCCGGGACAACTCGAAGAACACCCTGTTCCTC CAAATGAGCTCCCTCCGGGACGAGGATACTGCAGTGTACTACTGCGTGACC CGCGCCGGGTCCGAGGCGTCTGACATTTGGGGACAGGGCACTATGGTCACC GTGTCGTCCGGCGGAGGGGGCTCGGGAGGCGGTGGCAGCGGAGGAGGAGGG TCCGAGATCGTGCTGACCCAATCCCCGGCCACCCTCTCGCTGAGCCCTGGA GAAAGGGCAACCTTGTCCTGTCGCGCGAGCCAGTCCGTGAGCAACTCCCTG GCCTGGTACCAGCAGAAGCCCGGACAGGCTCCGAGACTTCTGATCTACGAC GCTTCGAGCCGGGCCACTGGAATCCCCGACCGCTTTTCGGGGTCCGGCTCA GGAACCGATTTCACCCTGACAATCTCACGGCTGGAGCCAGAGGATTTCGCC ATCTATTACTGCCAGCAGTTCGGTACTTCCTCCGGCCTGACTTTCGGAGGC GGCACGAAGCTCGAAATCAAGACCACTACCCCAGCACCGAGGCCACCCACC CCGGCTCCTACCATCGCCTCCCAGCCTCTGTCCCTGCGTCCGGAGGCATGT AGACCCGCAGCTGGTGGGGCCGTGCATACCCGGGGTCTTGACTTCGCCTGC GATATCTACATTTGGGCCCCTCTGGCTGGTACTTGCGGGGTCCTGCTGCTT TCACTCGTGATCACTCTTTACTGTAAGCGCGGTCGGAAGAAGCTGCTGTAC ATCTTTAAGCAACCCTTCATGAGGCCTGTGCAGACTACTCAAGAGGAGGAC GGCTGTTCATGCCGGTTCCCAGAGGAGGAGGAAGGCGGCTGCGAACTGCGC GTGAAATTCAGCCGCAGCGCAGATGCTCCAGCCTACAAGCAGGGGCAGAAC CAGCTCTACAACGAACTCAATCTTGGTCGGAGAGAGGAGTACGACGTGCTG GACAAGCGGAGAGGACGGGACCCAGAAATGGGCGGGAAGCCGCGCAGAAAG AATCCCCAAGAGGGCCTGTACAACGAGCTCCAAAAGGATAAGATGGCAGAA GCCTATAGCGAGATTGGTATGAAAGGGGAACGCAGAAGAGGCAAAGGCCAC GACGGACTGTACCAGGGACTCAGCACCGCCACCAAGGACACCTATGACGCT CTTCACATGCAGGCCCTGCCGCCTCGG |

| **BCMA EBB-C1979-C1** | | |
|---|---|---|
| **BCMA_EBB-C1979-C1** - aa Full CART | 989 | MALPVTALLLPLALLLHAARPQVQLVESGGGLVQPGGSLRLSCAASGFTFS SYAMSWVRQAPGKGLEWVSAISGSGGSTYYADSVKGRFTISRDNAKNSLYL |
| | | QMNSLRAEDTAIYYCARATYKRELRYYYGMDVWGQGTMVTVSSGGGGSGGG GSGGGGSEIVMTQSPGTVSLSPGERATLSCRASQSVSSSFLAWYQQKPGQA PRLLIYGASSRATGIPDRFSGSGSGTDFTLTISRLEPEDSAVYYCQQYHSS PSWTFGQGTRLEIKTTTPAPRPPTPAPTIASQPLSLRPEACRPAAGGAVHT RGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNLGR REEYDVLDKRRGRDPEMGGKPRRKNPQEGLYNELQKDKMAEAYSEIGMKGE RRRGKGHDGLYQGLSTATKDTYDALHMQALPPR |
| **BCMA_EBB-C1979-C1 - nt Full CART** | 1011 | ATGGCCCTCCCTGTCACCGCCCTGCTGCTTCCGCTGGCTCTTCTGCTCCAC GCCGCTCGGCCCCAAGTGCAGCTCGTGGAATCGGGTGGCGGACTGGTGCAG CCGGGGGGCTCACTTAGACTGTCCTGCGCGGCCAGCGGATTCACTTTCTCC TCCTACGCCATGTCCTGGGTCAGACAGGCCCCTGGAAAGGGCCTGGAATGG GTGTCCGCAATCAGCGGCAGCGGCGGCTCGACCTATTACGCGGATTCAGTG AAGGGCAGATTCACCATTTCCCGGGACAACGCCAAGAACTCCTTGTACCTT CAAATGAACTCCCTCCGCGCGGAAGATACCGCAATCTACTACTGCGCTCGG GCCACTTACAAGAGGGAACTGCGCTACTACTACGGGATGGACGTCTGGGGC CAGGGAACCATGGTCACCGTGTCCAGCGGAGGAGGAGGATCGGGAGGAGGC GGTAGCGGGGGTGGAGGGTCGGAGATCGTGATGACCCAGTCCCCCGGCACT GTGTCGCTGTCCCCCGGCGAACGGGCCACCCTGTCATGTCGGGCCAGCCAG TCAGTGTCGTCAAGCTTCCTCGCCTGGTACCAGCAGAAACCGGGACAAGCT CCCCGCCTGCTGATCTACGGAGCCAGCAGCCGGGCCACCGGTATTCCTGAC CGGTTCTCCGGTTCGGGGTCCGGGACCGACTTTACTCTGACTATCTCTCGC CTCGAGCCAGAGGACTCCGCCGTGTATTACTGCCAGCAGTACCACTCCTCC CCGTCCTGGACGTTCGGACAGGGCACAAGGCTGGAGATTAAGACCACTACC CCAGCACCGAGGCCACCCACCCCGGCTCCTACCATCGCCTCCCAGCCTCTG TCCCTGCGTCCGGAGGCATGTAGACCCGCAGCTGGTGGGGCCGTGCATACC CGGGGTCTTGACTTCGCCTGCGATATCTACATTTGGGCCCCTCTGGCTGGT ACTTGCGGGGTCCTGCTGCTTTCACTCGTGATCACTCTTTACTGTAAGCGC GGTCGGAAGAAGCTGCTGTACATCTTTAAGCAACCCTTCATGAGGCCTGTG CAGACTACTCAAGAGGAGGACGGCTGTTCATGCCGGTTCCCAGAGGAGGAG GAAGGCGGCTGCGAACTGCGCGTGAAATTCAGCCGCAGCGCAGATGCTCCA GCCTACAAGCAGGGGCAGAACCAGCTCTACAACGAACTCAATCTTGGTCGG AGAGAGGAGTACGACGTGCTGGACAAGCGGAGAGGACGGGACCCAGAAATG GGCGGGAAGCCGCGCAGAAAGAATCCCCAAGAGGGCCTGTACAACGAGCTC CAAAAGGATAAGATGGCAGAAGCCTATAGCGAGATTGGTATGAAAGGGGAA CGCAGAAGAGGCAAAGGCCACGACGGACTGTACCAGGGACTCAGCACCGCC ACCAAGGACACCTATGACGCTCTTCACATGCAGGCCCTGCCGCCTCGG |

| **BCMA EBB-C1978-C7** | | |
|---|---|---|
| **BCMA_EBB-C1978-C7 - aa Full CART** | 990 | MALPVTALLLPLALLLHAARPEVQLVETGGGLVQPGGSLRLSCAASGFTFS SYAMSWVRQAPGKGLEWVSAISGSGGSTYYADSVKGRFTISRDNSKNTLYL QMNTLKAEDTAVYYCARATYKRELRYYYGMDVWGQGTTVTVSSGGGGSGGG GSGGGGSEIVLTQSPSTLSLSPGESATLSCRASQSVSTTFLAWYQQKPGQA PRLLIYGSSNRATGIPDRFSGSGSGTDFTLTIRRLEPEDFAVYYCQQYHSS PSWTFGQGTKVEIKTTTPAPRPPTPAPTIASQPLSLRPEACRPAAGGAVHT RGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNLGR REEYDVLDKRRGRDPEMGGKPRRKNPQEGLYNELQKDKMAEAYSEIGMKGE RRRGKGHDGLYQGLSTATKDTYDALHMQALPPR |
| **BCMA_EBB-C1978-C7 - nt Full CART** | 1012 | ATGGCCCTCCCTGTCACCGCCCTGCTGCTTCCGCTGGCTCTTCTGCTCCAC GCCGCTCGGCCCGAGGTGCAGCTTGTGGAAACCGGTGGCGGACTGGTGCAG CCCGGAGGAAGCCTCAGGCTGTCCTGCGCCGCGTCCGGCTTCACCTTCTCC TCGTACGCCATGTCCTGGGTCCGCCAGGCCCCCGGAAAGGGCCTGGAATGG |
| | | GTGTCCGCCATCTCTGGAAGCGGAGGTTCCACGTACTACGCGGACAGCGTC AAGGGAAGGTTCACAATCTCCCGCGATAATTCGAAGAACACTCTGTACCTT CAAATGAACACCCTGAAGGCCGAGGACACTGCTGTGTACTACTGCGCACGG GCCACCTACAAGAGAGAGCTCCGGTACTACTACGGAATGGACGTCTGGGGC CAGGGAACTACTGTGACCGTGTCCTCGGGAGGGGGTGGCTCCGGGGGGGC GGCTCCGGCGGAGGCGGTTCCGAGATTGTGCTGACCCAGTCACCTTCAACT CTGTCGCTGTCCCCGGGAGAGAGCGCTACTCTGAGCTGCCGGGCCAGCCAG TCCGTGTCCACCACCTTCCTCGCCTGGTATCAGCAGAAGCCGGGGCAGGCA CCACGGCTCTTGATCTACGGGTCAAGCAACAGAGCGACCGGAATTCCTGAC CGCTTCTCGGGGAGCGGTTCAGGCACCGACTTCACCCTGACTATCCGGCGC CTGGAACCCGAAGATTTCGCCGTGTATTACTGTCAACAGTACCACTCCTCG CCGTCCTGGACCTTTGGCCAAGGAACCAAAGTGGAAATCAAGACCACTACC CCAGCACCGAGGCCACCCACCCCGGCTCCTACCATCGCCTCCCAGCCTCTG TCCCTGCGTCCGGAGGCATGTAGACCCGCAGCTGGTGGGGCCGTGCATACC CGGGGTCTTGACTTCGCCTGCGATATCTACATTTGGGCCCCTCTGGCTGGT ACTTGCGGGGTCCTGCTGCTTTCACTCGTGATCACTCTTTACTGTAAGCGC GGTCGGAAGAAGCTGCTGTACATCTTTAAGCAACCCTTCATGAGGCCTGTG CAGACTACTCAAGAGGAGGACGGCTGTTCATGCCGGTTCCCAGAGGAGGAG GAAGGCGGCTGCGAACTGCGCGTGAAATTCAGCCGCAGCGCAGATGCTCCA GCCTACAAGCAGGGGCAGAACCAGCTCTACAACGAACTCAATCTTGGTCGG AGAGAGGAGTACGACGTGCTGGACAAGCGGAGAGGACGGGACCCAGAAATG GGCGGGAAGCCGCGCAGAAAGAATCCCCAAGAGGGCCTGTACAACGAGCTC CAAAAGGATAAGATGGCAGAAGCCTATAGCGAGATTGGTATGAAAGGGGAA CGCAGAAGAGGCAAAGGCCACGACGGACTGTACCAGGGACTCAGCACCGCC ACCAAGGACACCTATGACGCTCTTCACATGCAGGCCCTGCCGCCTCGG |

| **BCMA EBB-C1978-D10** | | |
|---|---|---|
| **BCMA_EBB-C1978-D10 - aa Full CART** | 991 | MALPVTALLLPLALLLHAARPEVQLVETGGGLVQPGRSLRLSCAASGFTFD DYAMHWVRQAPGKGLEWVSGISWNSGSIGYADSVKGRFTISRDNAKNSLYL QMNSLRDEDTAVYYCARVGKAVPDVWGQGTTVTVSSGGGGSGGGGSGGGGS DIVMTQTPSSLSASVGDRVTITCRASQSISSYLNWYQQKPGKAPKLLIYAA SSLQSGVPSRFSGSGSGTDFTLTISSLQPEDFATYYCQQSYSTPYSFGQGT RLEIKTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGC SCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHDG LYQGLSTATKDTYDALHMQALPPR |
| **BCMA_EBB-C1978-D10 - nt Full CART** | 1013 | ATGGCCCTCCCTGTCACCGCCCTGCTGCTTCCGCTGGCTCTTCTGCTCCAC GCCGCTCGGCCCGAAGTGCAGCTCGTGGAAACTGGAGGTGGACTCGTGCAG CCTGGACGGTCGCTGCGGCTGAGCTGCGCTGCATCCGGCTTCACCTTCGAC GATTATGCCATGCACTGGGTCAGACAGGCGCCAGGGAAGGGACTTGAGTGG GTGTCCGGTATCAGCTGGAATAGCGGCTCAATCGGATACGCGGACTCCGTG AAGGGAAGGTTCACCATTTCCCGCGACAACGCCAAGAACTCCCTGTACTTG CAAATGAACAGCCTCCGGGATGAGGACACTGCCGTGTACTACTGCGCCCGC GTCGGAAAAGCTGTGCCCGACGTCTGGGGCCAGGGAACCACTGTGACCGTG TCCAGCGGCGGGGGTGGATCGGGCGGTGGAGGGTCCGGTGGAGGGGGCTCA GATATTGTGATGACCCAGACCCCCTCGTCCCTGTCCGCCTCGGTCGGCGAC CGCGTGACTATCACATGTAGAGCCTCGCAGAGCATCTCCAGCTACCTGAAC TGGTATCAGCAGAAGCCGGGGAAGGCCCCGAAGCTCCTGATCTACGCGGCA TCATCACTGCAATCGGGAGTGCCGAGCCGGTTTTCCGGGTCCGGCTCCGGC ACCGACTTCACGCTGACCATTTCTTCCCTGCAACCCGAGGACTTCGCCACT TACTACTGCCAGCAGTCCTACTCCACCCCTTACTCCTTCGGCCAAGGAACC AGGCTGGAAATCAAGACCACTACCCCAGCACCGAGGCCACCCACCCCGGCT |
| | | CCTACCATCGCCTCCCAGCCTCTGTCCCTGCGTCCGGAGGCATGTAGACCC GCAGCTGGTGGGGCCGTGCATACCCGGGGTCTTGACTTCGCCTGCGATATC TACATTTGGGCCCCTCTGGCTGGTACTTGCGGGGTCCTGCTGCTTTCACTC GTGATCACTCTTTACTGTAAGCGCGGTCGGAAGAAGCTGCTGTACATCTTT AAGCAACCCTTCATGAGGCCTGTGCAGACTACTCAAGAGGAGGACGGCTGT TCATGCCGGTTCCCAGAGGAGGAGGAAGGCGGCTGCGAACTGCGCGTGAAA TTCAGCCGCAGCGCAGATGCTCCAGCCTACAAGCAGGGGCAGAACCAGCTC TACAACGAACTCAATCTTGGTCGGAGAGAGGAGTACGACGTGCTGGACAAG CGGAGAGGACGGGACCCAGAAATGGGCGGGAAGCCGCGCAGAAAGAATCCC CAAGAGGGCCTGTACAACGAGCTCCAAAAGGATAAGATGGCAGAAGCCTAT AGCGAGATTGGTATGAAAGGGGAACGCAGAAGAGGCAAAGGCCACGACGGA CTGTACCAGGGACTCAGCACCGCCACCAAGGACACCTATGACGCTCTTCAC ATGCAGGCCCTGCCGCCTCGG |

| **BCMA EBB-C1979-C12** | | |
|---|---|---|
| **BCMA_EBB-C1979-C12 - aa Full CART** | 992 | MALPVTALLLPLALLLHAARPEVQLVESGGGLVQPGRSLRLSCTASGFTFD DYAMHWVRQRPGKGLEWVASINWKGNSLAYGDSVKGRFAISRDNAKNTVFL QMNSLRTEDTAVYYCASHQGVAYYNYAMDVWGRGTLVTVSSGGGGSGGGGS GGGGSEIVLTQSPGTLSLSPGERATLSCRATQSIGSSFLAWYQQRPGQAPR LLIYGASQRATGIPDRFSGRGSGTDFTLTISRVEPEDSAVYYCQHYESSPS WTFGQGTKVEIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **BCMA_EBB-C1979-C12 - nt Full CART** | 1014 | ATGGCCCTCCCTGTCACCGCCCTGCTGCTTCCGCTGGCTCTTCTGCTCCAC GCCGCTCGGCCCGAAGTGCAGCTCGTGGAGAGCGGGGGAGGATTGGTGCAG CCCGGAAGGTCCCTGCGGCTCTCCTGCACTGCGTCTGGCTTCACCTTCGAC GACTACGCGATGCACTGGGTCAGACAGCGCCCGGGAAAGGGCCTGGAATGG GTCGCCTCAATCAACTGGAAGGGAAACTCCCTGGCCTATGGCGACAGCGTG AAGGGCCGCTTCGCCATTTCGCGCGACAACGCCAAGAACACCGTGTTTCTG CAAATGAATTCCCTGCGGACCGAGGATACCGCTGTGTACTACTGCGCCAGC CACCAGGGCGTGGCATACTATAACTACGCCATGGACGTGTGGGGAAGAGGG ACGCTCGTCACCGTGTCCTCCGGGGGCGGTGGATCGGGTGGAGGAGGAAGC GGTGGCGGGGGCAGCGAAATCGTGCTGACTCAGAGCCCGGGAACTCTTTCA CTGTCCCCGGGAGAACGGGCCACTCTCTCGTGCCGGGCCACCCAGTCCATC GGCTCCTCCTTCCTTGCCTGGTACCAGCAGAGGCCAGGACAGGCGCCCCGC CTGCTGATCTACGGTGCTTCCCAACGCGCCACTGGCATTCCTGACCGGTTC AGCGGCAGAGGGTCGGGAACCGATTTCACACTGACCATTTCCCGGGTGGAG CCCGAAGATTCGGCAGTCTACTACTGTCAGCATTACGAGTCCTCCCCTTCA TGGACCTTCGGTCAAGGGACCAAAGTGGAGATCAAGACCACTACCCCAGCA CCGAGGCCACCCACCCCGGCTCCTACCATCGCCTCCCAGCCTCTGTCCCTG CGTCCGGAGGCATGTAGACCCGCAGCTGGTGGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG |
| | | GACACCTATGACGCTCTTCACATGCAGGCCCTGCCGCCTCGG |

| **BCMA EBB-C1980-G4** | | |
|---|---|---|
| **BCMA_EBB-C1980-G4-aa Full CART** | 993 | MALPVTALLLPLALLLHAARPEVQLVESGGGLVQPGGSLRLSCAASGFTFS SYAMSWVRQAPGKGLEWVSAISGSGGSTYYADSVKGRFTISRDNSKNTLYL QMNSLRAEDTAVYYCAKVVRDGMDVWGQGTTVTVSSGGGGSGGGGSGGGGS EIVLTQSPATLSLSPGERATLSCRASQSVSSSYLAWYQQKPGQAPRLLIYG ASSRATGIPDRFSGNGSGTDFTLTISRLEPEDFAVYYCQQYGSPPRFTFGP GTKVDIKTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGH DGLYQGLSTATKDTYDALHMQALPPR |
| **BCMA_EBB-C1980-G4-nt Full CART** | 1015 | ATGGCCCTCCCTGTCACCGCCCTGCTGCTTCCGCTGGCTCTTCTGCTCCAC GCCGCTCGGCCCGAGGTGCAGTTGGTCGAAAGCGGGGGCGGGCTTGTGCAG CCTGGCGGATCACTGCGGCTGTCCTGCGCGGCATCAGGCTTCACGTTTTCT TCCTACGCCATGTCCTGGGTGCGCCAGGCCCCTGGAAAGGACTGGAATGG GTGTCCGCGATTTCGGGGTCCGGCGGGAGCACCTACTACGCCGATTCCGTG AAGGGCCGCTTCACTATCTCGCGGGACAACTCCAAGAACACCCTCTACCTC CAAATGAATAGCCTGCGGGCCGAGGATACCGCCGTCTACTATTGCGCTAAG GTCGTGCGCGACGGAATGGACGTGTGGGGACAGGGTACCACCGTGACAGTG TCCTCGGGGGGAGGCGGTAGCGGCGGAGGAGGAAGCGGTGGTGGAGGTTCC GAGATTGTGCTGACTCAATCACCCGCGACCCTGAGCCTGTCCCCCGGCGAA AGGGCCACTCTGTCCTGTCGGGCCAGCCAATCAGTCTCCTCCTCGTACCTG GCCTGGTACCAGCAGAAGCCAGGACAGGCTCCGAGACTCCTTATCTATGGC GCATCCTCCCGCGCCACCGGAATCCCGGATAGGTTCTCGGGAAACGGATCG GGGACCGACTTCACTCTCACCATCTCCCGGCTGGAACCGGAGGACTTCGCC GTGTACTACTGCCAGCAGTACGGCAGCCCGCCTAGATTCACTTTCGGCCCC GGCACCAAAGTGGACATCAAGACCACTACCCCAGCACCGAGGCCACCCACC CCGGCTCCTACCATCGCCTCCCAGCCTCTGTCCCTGCGTCCGGAGGCATGT AGACCCGCAGCTGGTGGGGCCGTGCATACCCGGGGTCTTGACTTCGCCTGC GATATCTACATTTGGGCCCCTCTGGCTGGTACTTGCGGGGTCCTGCTGCTT TCACTCGTGATCACTCTTTACTGTAAGCGCGGTCGGAAGAAGCTGCTGTAC ATCTTTAAGCAACCCTTCATGAGGCCTGTGCAGACTACTCAAGAGGAGGAC GGCTGTTCATGCCGGTTCCCAGAGGAGGAGGAAGGCGGCTGCGAACTGCGC GTGAAATTCAGCCGCAGCGCAGATGCTCCAGCCTACAAGCAGGGGCAGAAC CAGCTCTACAACGAACTCAATCTTGGTCGGAGAGAGGAGTACGACGTGCTG GACAAGCGGAGAGGACGGGACCCAGAAATGGGCGGGAAGCCGCGCAGAAAG AATCCCCAAGAGGGCCTGTACAACGAGCTCCAAAAGGATAAGATGGCAGAA GCCTATAGCGAGATTGGTATGAAAGGGGAACGCAGAAGAGGCAAAGGCCAC GACGGACTGTACCAGGGACTCAGCACCGCCACCAAGGACACCTATGACGCT CTTCACATGCAGGCCCTGCCGCCTCGG |

| **BCMA_EBB-C1980-D2** | | |
|---|---|---|
| **BCMA_EBB-C1980-D2-aa Full CART** | 994 | MALPVTALLLPLALLLHAARPEVQLLESGGGLVQPGGSLRLSCAASGFTFS SYAMSWVRQAPGKGLEWVSAISGSGGSTYYADSVKGRFTISRDNSKNTLYL QMNSLRAEDTAVYYCAKIPQTGTFDYWGQGTLVTVSSGGGGSGGGGSGGGG SEIVLTQSPGTLSLSPGERATLSCRASQSVSSSYLAWYQQRPGQAPRLLIY GASSRATGIPDRFSGSGSGTDFTLTISRLEPEDFAVYYCQHYGSSPSWTFG QGTRLEIKTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREEYDV LDKRRGRDPEMGGKPRRKNPQEGLYNELQKDKMAEAYSEIGMKGERRRGKG HDGLYQGLSTATKDTYDALHMQALPPR |
| **BCMA_EBB-C1980-D2-nt Full CART** | 1016 | ATGGCCCTCCCTGTCACCGCCCTGCTGCTTCCGCTGGCTCTTCTGCTCCAC GCCGCTCGGCCCGAAGTGCAGCTGCTGGAGTCCGGCGGTGGATTGGTGCAA CCGGGGGGATCGCTCAGACTGTCCTGTGCGGCGTCAGGCTTCACCTTCTCG AGCTACGCCATGTCATGGGTCAGACAGGCCCCTGGAAAGGGTCTGGAATGG GTGTCCGCCATTTCCGGGAGCGGGGGATCTACATACTACGCCGATAGCGTG AAGGGCCGCTTCACCATTTCCCGGGACAACTCCAAGAACACTCTCTATCTG CAAATGAACTCCCTCCGCGCTGAGGACACTGCCGTGTACTACTGCGCCAAA ATCCCTCAGACCGGCACCTTCGACTACTGGGGACAGGGGACTCTGGTCACC GTCAGCAGCGGTGGCGGAGGTTCGGGGGGAGGAGGAAGCGGCGGCGGAGGG TCCGAGATTGTGCTGACCCAGTCACCCGGCACTTTGTCCCTGTCGCCTGGA GAAAGGGCCACCCTTTCCTGCCGGGCATCCCAATCCGTGTCCTCCTCGTAC CTGGCCTGGTACCAGCAGAGGCCCGGACAGGCCCCACGGCTTCTGATCTAC GGAGCAAGCAGCCGCGCGACCGGTATCCCGGACCGGTTTTCGGGCTCGGGC TCAGGAACTGACTTCACCCTCACCATCTCCCGCCTGGAACCCGAAGATTTC GCTGTGTATTACTGCCAGCACTACGGCAGCTCCCCGTCCTGGACGTTCGGC CAGGGAACTCGGCTGGAGATCAAGACCACTACCCCAGCACCGAGGCCACCC ACCCCGGCTCCTACCATCGCCTCCCAGCCTCTGTCCCTGCGTCCGGAGGCA TGTAGACCCGCAGCTGGTGGGGCCGTGCATACCCGGGGTCTTGACTTCGCC TGCGATATCTACATTTGGGCCCCTCTGGCTGGTACTTGCGGGGTCCTGCTG CTTTCACTCGTGATCACTCTTTACTGTAAGCGCGGTCGGAAGAAGCTGCTG TACATCTTTAAGCAACCCTTCATGAGGCCTGTGCAGACTACTCAAGAGGAG GACGGCTGTTCATGCCGGTTCCCAGAGGAGGAGGAAGGCGGCTGCGAACTG CGCGTGAAATTCAGCCGCAGCGCAGATGCTCCAGCCTACAAGCAGGGGCAG AACCAGCTCTACAACGAACTCAATCTTGGTCGGAGAGAGGAGTACGACGTG CTGGACAAGCGGAGAGGACGGGACCCAGAAATGGGCGGGAAGCCGCGCAGA AAGAATCCCCAAGAGGGCCTGTACAACGAGCTCCAAAAGGATAAGATGGCA GAAGCCTATAGCGAGATTGGTATGAAAGGGGAACGCAGAAGAGGCAAAGGC CACGACGGACTGTACCAGGGACTCAGCACCGCCACCAAGGACACCTATGAC GCTCTTCACATGCAGGCCCTGCCGCCTCGG |

| **BCMA EBB-C1978-A10** | | |
|---|---|---|
| **BCMA_EBB-C1978-A10-aa Full CART** | 995 | MALPVTALLLPLALLLHAARPEVQLVETGGGLVQPGGSLRLSCAASGFTFS SYAMSWVRQAPGKGLEWVSAISGSGGSTYYADSVKGRFTMSRENDKNSVFL QMNSLRVEDTGVYYCARANYKRELRYYYGMDVWGQGTMVTVSSGGGGSGGG GSGGGGSEIVMTQSPGTLSLSPGESATLSCRASQRVASNYLAWYQHKPGQA PSLLISGASSRATGVPDRFSGSGSGTDFTLAISRLEPEDSAVYYCQHYDSS PSWTFGQGTKVEIKTTTPAPRPPTPAPTIASQPLSLRPEACRPAAGGAVHT RGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNLGR REEYDVLDKRRGRDPEMGGKPRRKNPQEGLYNELQKDKMAEAYSEIGMKGE RRRGKGHDGLYQGLSTATKDTYDALHMQALPPR |
| **BCMA_EBB-C1978-A10-nt Full CART** | 1017 | ATGGCCCTCCCTGTCACCGCCCTGCTGCTTCCGCTGGCTCTTCTGCTCCAC GCCGCTCGGCCCGAAGTGCAACTGGTGGAAACCGGTGGAGGACTCGTGCAG CCTGGCGGCAGCCTCCGGCTGAGCTGCGCCGCTTCGGGATTCACCTTTTCC TCCTACGCGATGTCTTGGGTCAGACAGGCCCCCGGAAAGGGGCTGGAATGG GTGTCAGCCATCTCCGGCTCCGGCGGATCAACGTACTACGCCGACTCCGTG AAAGGCCGGTTCACCATGTCGCGCGAGAATGACAAGAACTCCGTGTTCCTG CAAATGAACTCCCTGAGGGTGGAGGACACCGGAGTGTACTATTGTGCGCGC GCCAACTACAAGAGAGAGCTGCGGTACTACTACGGAATGGACGTCTGGGGA CAGGGAACTATGGTGACCGTGTCATCCGGTGGAGGGGGAAGCGGCGGTGGA GGCAGCGGGGGCGGGGGTTCAGAAATTGTCATGACCCAGTCCCCGGAACT CTTTCCCTCTCCCCCGGGGAATCCGCGACTTTGTCCTGCCGGGCCAGCCAG CGCGTGGCCTCGAACTACCTCGCATGGTACCAGCATAAGCCAGGCCAAGCC |
| | | CCTTCCCTGCTGATTTCCGGGCTAGCAGCCGCGCCACTGGCGTGCCGGAT AGGTTCTCGGGAAGCGGCTCGGGTACCGATTTCACCCTGGCAATCTCGCGG CTGGAACCGGAGGATTCGGCCGTGTACTACTGCCAGCACTATGACTCATCC CCCTCCTGGACATTCGGACAGGGCACCAAGGTCGAGATCAAGACCACTACC CCAGCACCGAGGCCACCCACCCCGGCTCCTACCATCGCCTCCCAGCCTCTG TCCCTGCGTCCGGAGGCATGTAGACCCGCAGCTGGTGGGGCCGTGCATACC CGGGGTCTTGACTTCGCCTGCGATATCTACATTTGGGCCCCTCTGGCTGGT ACTTGCGGGGTCCTGCTGCTTTCACTCGTGATCACTCTTTACTGTAAGCGC GGTCGGAAGAAGCTGCTGTACATCTTTAAGCAACCCTTCATGAGGCCTGTG CAGACTACTCAAGAGGAGGACGGCTGTTCATGCCGGTTCCCAGAGGAGGAG GAAGGCGGCTGCGAACTGCGCGTGAAATTCAGCCGCAGCGCAGATGCTCCA GCCTACAAGCAGGGGCAGAACCAGCTCTACAACGAACTCAATCTTGGTCGG AGAGAGGAGTACGACGTGCTGGACAAGCGGAGAGGACGGGACCCAGAAATG GGCGGGAAGCCGCGCAGAAAGAATCCCCAAGAGGGCCTGTACAACGAGCTC CAAAAGGATAAGATGGCAGAAGCCTATAGCGAGATTGGTATGAAAGGGGAA CGCAGAAGAGGCAAAGGCCACGACGGACTGTACCAGGGACTCAGCACCGCC ACCAAGGACACCTATGACGCTCTTCACATGCAGGCCCTGCCGCCTCGG |

| **BCMA EBB-C1978-D4** | | |
|---|---|---|
| **BCMA_EBB-C1978-D4-aa Full CART** | 996 | MALPVTALLLPLALLLHAARPEVQLLETGGGLVQPGGSLRLSCAASGFSFS SYAMSWVRQAPGKGLEWVSAISGSGGSTYYADSVKGRFTISRDNSKNTLYL QMNSLRAEDTAVYYCAKALVGATGAFDIWGQGTLVTVSSGGGGSGGGGSGG GGSEIVLTQSPGTLSLSPGERATLSCRASQSLSSNFLAWYQQKPGQAPGLL IYGASNWATGTPDRFSGSGSGTDFTLTITRLEPEDFAVYYCQYYGTSPMYT FGQGTKVEIKTTTPAPRPPTPAPTIASQPLSLRPEACRPAAGGAVHTRGLD FACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRREEY DVLDKRRGRDPEMGGKPRRKNPQEGLYNELQKDKMAEAYSEIGMKGERRRG KGHDGLYQGLSTATKDTYDALHMQALPPR |
| **BCMA_EBB-C1978-D4-nt Full CART** | 1018 | ATGGCCCTCCCTGTCACCGCCCTGCTGCTTCCGCTGGCTCTTCTGCTCCAC GCCGCTCGGCCCGAAGTGCAGCTGCTCGAAACCGGTGGAGGGCTGGTGCAG CCAGGGGGCTCCCTGAGGCTTTCATGCGCCGCTAGCGGATTCTCCTTCTCC TCTTACGCCATGTCGTGGGTCCGCCAAGCCCCTGGAAAAGGCCTGGAATGG GTGTCCGCGATTTCCGGGAGCGGAGGTTCGACCTATTACGCCGACTCCGTG AAGGGCCGCTTTACCATCTCCCGGGATAACTCCAAGAACACTCTGTACCTC CAAATGAACTCGCTGAGAGCCGAGGACACCGCCGTGTATTACTGCGCGAAG GCGCTGGTCGGCGCGACTGGGGCATTCGACATCTGGGGACAGGGAACTCTT GTGACCGTGTCGAGCGGAGGCGGCGGCTCCGGCGGAGGAGGGAGCGGGGGC GGTGGTTCCGAAATCGTGTTGACTCAGTCCCCGGGAACCCTGAGCTTGTCA CCCGGGGAGCGGGCCACTCTCTCCTGTCGCGCCTCCCAATCGCTCTCATCC AATTTCCTGGCCTGGTACCAGCAGAAGCCCGGACAGGCCCCGGGCCTGCTC ATCTACGGCGCTTCAAACTGGGCAACGGGAACCCCTGATCGGTTCAGCGGA AGCGGATCGGGTACTGACTTTACCCTGACCATCACCAGACTGGAACCGGAG GACTTCGCCGTGTACTACTGCCAGTACTACGGCACCTCCCCCATGTACACA TTCGGACAGGGTACCAAGGTCGAGATTAAGACCACTACCCCAGCACCGAGG CCACCCACCCCGGCTCCTACCATCGCCTCCCAGCCTCTGTCCCTGCGTCCG GAGGCATGTAGACCCGCAGCTGGTGGGGCCGTGCATACCCGGGGTCTTGAC TTCGCCTGCGATATCTACATTTGGGCCCCTCTGGCTGGTACTTGCGGGGTC CTGCTGCTTTCACTCGTGATCACTCTTTACTGTAAGCGCGGTCGGAAGAAG CTGCTGTACATCTTTAAGCAACCCTTCATGAGGCCTGTGCAGACTACTCAA GAGGAGGACGGCTGTTCATGCCGGTTCCCAGAGGAGGAGGAAGGCGGCTGC GAACTGCGCGTGAAATTCAGCCGCAGCGCAGATGCTCCAGCCTACAAGCAG GGGCAGAACCAGCTCTACAACGAACTCAATCTTGGTCGGAGAGAGGAGTAC |
| | | GACGTGCTGGACAAGCGGAGAGGACGGGACCCAGAAATGGGCGGAAGCCG CGCAGAAAGAATCCCCAAGAGGGCCTGTACAACGAGCTCCAAAAGGATAAG ATGGCAGAAGCCTATAGCGAGATTGGTATGAAAGGGGAACGCAGAAGAGGC AAAGGCCACGACGGACTGTACCAGGGACTCAGCACCGCCACCAAGGACACC TATGACGCTCTTCACATGCAGGCCCTGCCGCCTCGG |

| **BCMA EBB-C1980-A2** | | |
|---|---|---|
| **BCMA_EBB-C1980-A2-aa Full CART** | 997 | MALPVTALLLPLALLLHAARPEVQLLESGGGLVQPGGSLRLSCAASGFTFS SYAMSWVRQAPGKGLEWVSAISGSGGSTYYADSVKGRFTISRDNSKNTLYL QMNSLRAEDTAVYYCVLWFGEGFDPWGQGTLVTVSSGGGGSGGGGSGGGGS DIVLTQSPLSLPVTPGEPASISCRSSQSLLHSNGYNYLDWYLQKPGQSPQL LIYLGSNRASGVPDRFSGSGSGTDFTLKISRVEAEDVGVYYCMQALQTPLT FGGGTKVDIKTTTPAPRPPTPAPTIASQPLSLRPEACRPAAGGAVHTRGLD FACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRREEY DVLDKRRGRDPEMGGKPRRKNPQEGLYNELQKDKMAEAYSEIGMKGERRRG KGHDGLYQGLSTATKDTYDALHMQALPPR |
| **BCMA_EBB-C1980-A2-nt Full CART** | 1019 | ATGGCCCTCCCTGTCACCGCCCTGCTGCTTCCGCTGGCTCTTCTGCTCCAC GCCGCTCGGCCCGAAGTGCAGCTGCTTGAGAGCGGTGGAGGTCTGGTGCAG CCCGGGGGATCACTGCGCCTGTCCTGTGCCGCGTCCGGTTTCACTTTCTCC TCGTACGCCATGTCGTGGGTCAGACAGGCACCGGGAAAGGGACTGGAATGG GTGTCAGCCATTTCGGGTTCGGGGGGCAGCACCTACTACGCTGACTCCGTG AAGGGCCGGTTCACCATTTCCCGCGACAACTCCAAGAACACCTTGTACCTC CAAATGAACTCCCTGCGGGCCGAAGATACCGCCGTGTATTACTGCGTGCTG TGGTTCGGAGAGGGATTCGACCCGTGGGGACAAGGAACACTCGTGACTGTG TCATCCGGCGGAGGCGGCAGCGGTGGCGGCGGTTCCGGCGGCGGCGGATCT GACATCGTGTTGACCCAGTCCCCTCTGAGCCTGCCGGTCACTCCTGGCGAA CCAGCCAGCATCTCCTGCCGGTCGAGCCAGTCCCTCCTGCACTCCAATGGG TACAACTACCTCGATTGGTATCTGCAAAAGCCGGGCCAGAGCCCCCAGCTG CTGATCTACCTTGGGTCAAACCGCGCTTCCGGGGTGCCTGATAGATTCTCC GGGTCCGGGAGCGGAACCGACTTTACCCTGAAAATCTCGAGGGTGGAGGCC GAGGACGTCGGAGTGTACTACTGCATGCAGGCGCTCCAGACTCCCCTGACC TTCGGAGGAGGAACGAAGGTCGACATCAAGACCACTACCCCAGCACCGAGG CCACCCACCCCGGCTCCTACCATCGCCTCCCAGCCTCTGTCCCTGCGTCCG GAGGCATGTAGACCCGCAGCTGGTGGGGCCGTGCATACCCGGGGTCTTGAC TTCGCCTGCGATATCTACATTTGGGCCCCTCTGGCTGGTACTTGCGGGGTC CTGCTGCTTTCACTCGTGATCACTCTTTACTGTAAGCGCGGTCGGAAGAAG CTGCTGTACATCTTTAAGCAACCCTTCATGAGGCCTGTGCAGACTACTCAA GAGGAGGACGGCTGTTCATGCCGGTTCCCAGAGGAGGAGGAAGGCGGCTGC GAACTGCGCGTGAAATTCAGCCGCAGCGCAGATGCTCCAGCCTACAAGCAG GGGCAGAACCAGCTCTACAACGAACTCAATCTTGGTCGGAGAGAGGAGTAC GACGTGCTGGACAAGCGGAGAGGACGGGACCCAGAAATGGGCGGGAAGCCG CGCAGAAAGAATCCCCAAGAGGGCCTGTACAACGAGCTCCAAAAGGATAAG ATGGCAGAAGCCTATAGCGAGATTGGTATGAAAGGGGAACGCAGAAGAGGC AAAGGCCACGACGGACTGTACCAGGGACTCAGCACCGCCACCAAGGACACC TATGACGCTCTTCACATGCAGGCCCTGCCGCCTCGG |

| **BCMA EBB-C1981-C3** | | |
|---|---|---|
| **BCMA_EBB-C1981-C3-aa Full CART** | 998 | MALPVTALLLPLALLLHAARPQVQLVESGGGLVQPGGSLRLSCAASGFTFS SYAMSWVRQAPGKGLEWVSAISGSGGSTYYADSVKGRFTISRDNSKNTLYL QMNSLRAEDTAVYYCAKVGYDSSGYYRDYYGMDVWGQGTTVTVSSGGGGSG GGGSGGGGSEIVLTQSPGTLSLSPGERATLSCRASQSVSSSYLAWYQQKPG QAPRLLIYGTSSRATGISDRFSGSGSGTDFTLTISRLEPEDFAVYYCQHYG NSPPKFTFGPGTKLEIKTTTPAPRPPTPAPTIASQPLSLRPEACRPAAGGA |
| | | VHTRGLDFACDIYIWAPLAGTCGVLLLSLVITLYCKRGRKKLLYIFKQPFM RPVQTTQEEDGCSCRFPEEEEGGCELRVKFSRSADAPAYKQGQNQLYNELN LGRREEYDVLDKRRGRDPEMGGKPRRKNPQEGLYNELQKDKMAEAYSEIGM KGERRRGKGHDGLYQGLSTATKDTYDALHMQALPPR |
| **BCMA_EBB-C1981-C3-nt Full CART** | 1020 | ATGGCCCTCCCTGTCACCGCCCTGCTGCTTCCGCTGGCTCTTCTGCTCCAC GCCGCTCGGCCCCAAGTGCAGCTCGTGGAGTCAGGCGGAGGACTGGTGCAG CCCGGGGGCTCCCTGAGACTTTCCTGCGCGGCATCGGGTTTTACCTTCTCC TCCTATGCTATGTCCTGGGTGCGCCAGGCCCCGGGAAAGGACTGGAATGG GTGTCCGCAATCAGCGGTAGCGGGGGCTCAACATACTACGCCGACTCCGTC AAGGGTCGCTTCACTATTTCCCGGGACAACTCCAAGAATACCCTGTACCTC CAAATGAACAGCCTCAGGGCCGAGGATACTGCCGTGTACTACTGCGCCAAA GTCGGATACGATAGCTCCGGTTACTACCGGGACTACTACGGAATGGACGTG TGGGGACAGGGCACCACCGTGACCGTGTCAAGCGGCGGAGGCGGTTCAGGA GGGGGAGGCTCCGGCGGTGGAGGGTCCGAAATCGTCCTGACTCAGTCGCCT GGCACTCTGTCGTTGTCCCCGGGGGAGCGCGCTACCCTGTCGTGTCGGGCG TCGCAGTCCGTGTCGAGCTCCTACCTCGCGTGGTACCAGCAGAAGCCCGGA CAGGCCCCTAGACTTCTGATCTACGGCACTTCTTCACGCGCCACCGGGATC AGCGACAGGTTCAGCGGCTCCGGCTCCGGGACCGACTTCACCCTGACCATT AGCCGGCTGGAGCCTGAAGATTTCGCCGTGTATTACTGCCAACACTACGGA AACTCGCCGCCAAAGTTCACGTTCGGACCCGGAACCAAGCTGGAAATCAAG ACCACTACCCCAGCACCGAGGCCACCCACCCCGGCTCCTACCATCGCCTCC CAGCCTCTGTCCCTGCGTCCGGAGGCATGTAGACCCGCAGCTGGTGGGGCC GTGCATACCCGGGGTCTTGACTTCGCCTGCGATATCTACATTTGGGCCCCT CTGGCTGGTACTTGCGGGGTCCTGCTGCTTTCACTCGTGATCACTCTTTAC TGTAAGCGCGGTCGGAAGAAGCTGCTGTACATCTTTAAGCAACCCTTCATG AGGCCTGTGCAGACTACTCAAGAGGAGGACGGCTGTTCATGCCGGTTCCCA GAGGAGGAGGAAGGCGGCTGCGAACTGCGCGTGAAATTCAGCCGCAGCGCA GATGCTCCAGCCTACAAGCAGGGGCAGAACCAGCTCTACAACGAACTCAAT CTTGGTCGGAGAGAGGAGTACGACGTGCTGGACAAGCGGAGAGGACGGGAC CCAGAAATGGGCGGGAAGCCGCGCAGAAAGAATCCCCAAGAGGGCCTGTAC AACGAGCTCCAAAAGGATAAGATGGCAGAAGCCTATAGCGAGATTGGTATG AAAGGGGAACGCAGAAGAGGCAAAGGCCACGACGGACTGTACCAGGGACTC AGCACCGCCACCAAGGACACCTATGACGCTCTTCACATGCAGGCCCTGCCG CCTCGG |

| **BCMA EBB-C1978-G4** | | |
|---|---|---|
| **BCMA_EBB-C1978-G4-aa Full CART** | 999 | MALPVTALLLPLALLLHAARPEVQLVESGGGLVQPGGSLRLSCAASGFTFS SYAMSWVRQAPGKGLEWVSAISGSGGSTYYADSVKGRFTISRDNSKNTLYL QMNSLRAEDTAVYYCAKMGWSSGYLGAFDIWGQGTTVTVSSGGGGSGGGGS GGGGSEIVLTQSPGTLSLSPGERATLSCRASQSVASSFLAWYQQKPGQAPR LLIYGASGRATGIPDRFSGSGSGTDFTLTISRLEPEDFAVYYCQHYGGSPR LTFGGGTKVDIKTTTPAPRPPTPAPTIASQPLSLRPEACRPAAGGAVHTRG LDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERR RGKGHDGLYQGLSTATKDTYDALHMQALPPR |
| **BCMA_EBB-C1978-G4-nt Full CART** | 1021 | ATGGCCCTCCCTGTCACCGCCCTGCTGCTTCCGCTGGCTCTTCTGCTCCAC GCCGCTCGGCCCGAAGTCCAACTGGTGGAGTCCGGGGGAGGCTCGTGCAG CCCGGAGGCAGCCTTCGGCTGTCGTGCGCCGCCTCCGGGTTCACGTTCTCA TCCTACGCGATGTCGTGGGTCAGACAGGCACCAGGAAAGGGACTGGAATGG GTGTCCGCCATTAGCGGCTCCGGCGGTAGCACCTACTATGCCGACTCAGTG AAGGGAAGGTTCACTATCTCCCGCGACAACAGCAAGAACACCCTGTACCTC CAAATGAACTCTCTGCGGGCCGAGGATACCGCGGTGTACTATTGCGCCAAG |
| | | ATGGGTTGGTCCAGCGGATACTTGGAGCCTTCGACATTTGGGGACAGGGC ACTACTGTGACCGTGTCCTCCGGGGGTGGCGGATCGGGAGGCGGCGGCTCG GGTGGAGGGGGTTCCGAAATCGTGTTGACCCAGTCACCGGGAACCCTCTCG CTGTCCCCGGGAGAACGGGCTACACTGTCATGTAGAGCGTCCCAGTCCGTG GCTTCCTCGTTCCTGGCCTGGTACCAGCAGAAGCCGGGACAGGCACCCCGC CTGCTCATCTACGGAGCCAGCGGCCGGGCGACCGGCATCCCTGACCGCTTC TCCGGTTCCGGCTCGGGCACCGACTTTACTCTGACCATTAGCAGGCTTGAG CCCGAGGATTTTGCCGTGTACTACTGCCAACACTACGGGGGGAGCCCTCGC CTGACCTTCGGAGGCGGAACTAAGGTCGATATCAAAACCACTACCCCAGCA CCGAGGCCACCCACCCCGGCTCCTACCATCGCCTCCCAGCCTCTGTCCCTG CGTCCGGAGGCATGTAGACCCGCAGCTGGTGGGGCCGTGCATACCCGGGGT CTTGACTTCGCCTGCGATATCTACATTTGGGCCCCTCTGGCTGGTACTTGC GGGGTCCTGCTGCTTTCACTCGTGATCACTCTTTACTGTAAGCGCGGTCGG AAGAAGCTGCTGTACATCTTTAAGCAACCCTTCATGAGGCCTGTGCAGACT ACTCAAGAGGAGGACGGCTGTTCATGCCGGTTCCCAGAGGAGGAGGAAGGC GGCTGCGAACTGCGCGTGAAATTCAGCCGCAGCGCAGATGCTCCAGCCTAC AAGCAGGGGCAGAACCAGCTCTACAACGAACTCAATCTTGGTCGGAGAGAG GAGTACGACGTGCTGGACAAGCGGAGAGGACGGGACCCAGAAATGGGCGGG AAGCCGCGCAGAAAGAATCCCCAAGAGGGCCTGTACAACGAGCTCCAAAAG GATAAGATGGCAGAAGCCTATAGCGAGATTGGTATGAAAGGGGAACGCAGA AGAGGCAAAGGCCACGACGGACTGTACCAGGGACTCAGCACCGCCACCAAG GACACCTATGACGCTCTTCACATGCAGGCCCTGCCGCCTCGG |

In one embodiment, the CAR molecule of the BCA CAR comprises (e.g., consists of) an amino acid sequence provided in Table 16, or Table 1 of WO2016/014565, or as otherwise described herein. In one embodiment, the CAR molecule of the BCA CAR comprises (e.g., consists of) an amino acid sequence of SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, or SEQ ID NO: 999; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence of SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, or SEQ ID NO: 999; or an amino acid sequence having 85%, 90%, 95%, 96%, 97%, 98%, 99% identity to an amino acid sequence of SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, or SEQ ID NO: 999.

In one aspect, the cell of the invention (e.g., a cell comprising both a TA CAR and a BCA CAR) comprises a TA CAR molecule comprising an antigen binding domain that binds to a tumor antigen. In one embodiment, the TA CAR comprises a CAR molecule comprising a mesothelin antigen binding domain (e.g., a murine, human or humanized antibody or antibody fragment that specifically binds to mesothelin), a transmembrane domain, and an intracellular signaling domain (e.g., an intracellular signaling domain comprising a costimulatory domain and/or a primary signaling domain).

Exemplary TA CAR molecules that target mesothelin are described herein, and are provided in Table 11. The CAR molecules in Table 11 comprise a mesothelin antigen binding domain, e.g., an amino acid sequence of any mesothelin antigen binding domain provided in Table 2. The leader sequence is in bold and underlined, CDRs are underlined, and the linker sequence between the heavy and light chain of the antigen binding region is shaded in grey.

**Table 11. Exemplary mesothelin CAR molecules**

| **Name** | **Amino Acid Sequence** | **SEQ ID NO:** |
|---|---|---|
| M5 CAR | MALPVTALLLPLALLLHAARPQVQLVQSGAEVEKPGASVKVSCKASGYTFTDYYMHWVRQ APGQGLEWMGWINPNSGGTNYAQKFQGRVTMTRDTSISTAYMELSRLRSDDTAVYYCASG WDFDYWGQGTLVTVSSGGGGSGGGGSGGGGSGGGGSDIVMTQSPSSLSASVGDRVTITCR ASQSIRYYLSWYQQKPGKAPKLLIYTASILQNGVPSRFSGSGSGTDFTLTISSLQPEDFA TYYCLQTYTTPDFGPGTKVEIKTTTPAPRPPTPAPTIASQPLSLRPEACRPAAGGAVHTR GLDFACDIYIWAPLAGTCGVLLLSLVITLYCKRGRKKLLYIFKQPFMRPVQTTQEEDGCS CRFPEEEEGGCELRVKFSRSADAPAYKQGQNQLYNELNLGRREEYDVLDKRRGRDPEMGG | **286** |
| | KPRRKNPQEGLYNELQKDKMAEAYSEIGMKGERRRGKGHDGLYQGLSTATKDTYDALHMQ ALPPR | |
| M11 CAR | MALPVTALLLPLALLLHAARPQVQLQQSGAEVKKPGASVKVSCKASGYTFTGYYMHWVRQ APGQGLEWMGWINPNSGGTNYAQNFQGRVTMTRDTSISTAYMELRRLRSDDTAVYYCASG WDFDYWGQGTLVTVSSGGGGSGGGGSGGGGSGGGGSDIRMTQSPSSLSASVGDRVTITCR ASQSIRYYLSWYQQKPGKAPKLLIYTASILQNGVPSRFSGSGSGTDFTLTISSLQPEDFA TYYCLQTYTTPDFGPGTKVEIKTTTPAPRPPTPAPTIASQPLSLRPEACRPAAGGAVHTR GLDFACDIYIWAPLAGTCGVLLLSLVITLYCKRGRKKLLYIFKQPFMRPVQTTQEEDGCS CRFPEEEEGGCELRVKFSRSADAPAYKQGQNQLYNELNLGRREEYDVLDKRRGRDPEMGG KPRRKNPQEGLYNELQKDKMAEAYSEIGMKGERRRGKGHDGLYQGLSTATKDTYDALHMQ ALPPR | **292** |
| SS1 CAR | MALPVTALLLPLALLLHAARPQVQLQQSGPELEKPGASVKISCKASGYSFTGYTMNWVK QSHGKSLEWIGLITPYNGASSYNQKFRGKATLTVDKSSSTAYMDLLSLTSEDSAVYFCA RGGYDGRGFDYWGQGTTVTVSSGGGGSGGGGSGGGGSDIELTQSPAIMSASPGEKVTMT CSASSSVSYMHWYQQKSGTSPKRWIYDTSKLASGVPGRFSGSGSGNSYSLTISSVEAED DATYYCQQWSGYPLTFGAGTKLEITTTPAPRPPTPAPTIASQPLSLRPEACRPAAGGAV HTRGLDFACDIYIWAPLAGTCGVLLLSLVITLYCKRGRKKLLYIFKQPFMRPVQTTQEE DGCSCRFPEEEEGGCELRVKFSRSADAPA | **306** |
| M1 CAR | MALPVTALLLPLALLLHAARPQVQLQQSGAEVKKPGASVKVSCKASGYTFTGYYMHWVRQ APGQGLEWMGRINPNSGGTNYAQKFQGRVTMTRDTSISTAYMELSRLRSEDTAVYYCARG RYYGMDVWGQGTMVTVSSGGGGSGGGGSGGGGSGGGGSEIVLTQSPATLSLSPGERATIS CRASQSVSSNFAWYQQRPGQAPRLLIYDASNRATGIPPRFSGSGSGTDFTLTISSLEPED FAAYYCHQRSNWLYTFGQGTKVDIKTTTPAPRPPTPAPTIASQPLSLRPEACRPAAGGAV HTRGLDFACDIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVLDKRRGRDPE MGGKPRRKNPQEGLYNELQKDKMAEAYSEIGMKGERRRGKGHDGLYQGLSTATKDTYDAL HMQALPPR | **282** |
| M2 CAR | MALPVTALLLPLALLLHAARPQVQLVQSGAEVKKPGASVKVSCKASGYTFTGYYMHWVRQ APGQGLEWMGWINPNSGGTNYAQKFQGRVTMTRDTSISTAYMELSRLRSDDTAVYYCARD LRRTVVTPRAYYGMDVWGQGTTVTVSSGGGGSGGGGSGGGGSGGGGSDIQLTQSPSTLSA SVGDRVTITCQASQDISNSLNWYQQKAGKAPKLLIYDASTLETGVPSRFSGSGSGTDFSF TISSLQPEDIATYYCQQHDNLPLTFGQGTKVEIKTTTPAPRPPTPAPTIASQPLSLRPEA CRPAAGGAVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGHDGLYQGLST ATKDTYDALHMQALPPR | **283** |
| M3 CAR | MALPVTALLLPLALLLHAARPQVQLVQSGAEVKKPGAPVKVSCKASGYTFTGYYMHWVRQ APGQGLEWMGWINPNSGGTNYAQKFQGRVTMTRDTSISTAYMELSRLRSDDTAVYYCARG EWDGSYYYDYWGQGTLVTVSSGGGGSGGGGSGGGGSGGGGSDIVLTQTPSSLSASVGDRV TITCRASQSINTYLNWYQHKPGKAPKLLIYAASSLQSGVPSRFSGSGSGTDFTLTISSLQ PEDFATYYCQQSFSPLTFGGGTKLEIKTTTPAPRPPTPAPTIASQPLSLRPEACRPAAGG AVHTRGLDFACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYDVLDKRRGRD PEMGGKPRRKNPQEGLYNELQKDKMAEAYSEIGMKGERRRGKGHDGLYQGLSTATKDTYD ALHMQALPPR | **284** |
| M4 CAR | MALPVTALLLPLALLLHAARPQVQLVESGGGLVQPGGSLRLSCAASGFTFSSYWMHWVRQ VPGKGLVWVSRINTDGSTTTYADSVEGRFTISRDNAKNTLYLQMNSLRDDDTAVYYCVGG HWAVWGQGTTVTVSSGGGGSGGGGSGGGGSGGGGSDIQMTQSPSTLSASVGDRVTITCRA SQSISDRLAWYQQKPGKAPKLLIYKASSLESGVPSRFSGSGSGTEFTLTISSLQPDDFAV YYCQQYGHLPMYTFGQGTKVEIKTTTPAPRPPTPAPTIASQPLSLRPEACRPAAGGAVHT RGLDFACDIYIWAPLAGTCGVLLLSLVITLYCKRGRKKLLYIFKQPFMRPVQTTQEEDGC SCRFPEEEEGGCELRVKFSRSADAPAYKQGQNQLYNELNLGRREEYDVLDKRRGRDPEMG GKPRRKNPQEGLYNELQKDKMAEAYSEIGMKGERRRGKGHDGLYQGLSTATKDTYDALHM QALPPR | **285** |
| M6 CAR | MALPVTALLLPLALLLHAARPQVQLVQSGAEVKKPGASVKVSCKASGYTFTSYYMHWVRQ APGQGLEWMGIINPSGGSTSYAQKFQGRVTMTRDTSTSTVYMELSSLRSEDTAVYYCARY | **287** |
| | RLIAVAGDYYYYGMDVWGQGTMVTVSSGGGGSGGGGSGGGGSGGGGSDIQMTQSPSSVSA SVGDRVTITCRASQGVGRWLAWYQQKPGTAPKLLIYAASTLQSGVPSRFSGSGSGTDFTL TINNLQPEDFATYYCQQANSFPLTFGGGTRLEIKTTTPAPRPPTPAPTIASQPLSLRPEA CRPAAGGAVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGHDGLYQGLST ATKDTYDALHMQALPPR | |
| M7 CAR | MALPVTALLLPLALLLHAARPQVQLVQSGGGVVQPGRSLRLSCAASGFTFSSYAMHWVRQ APGKGLEWVAVISYDGSNKYYADSVKGRFTISRDNSKNTLYLQMNSLRAEDTAVYYCARW KVSSSSPAFDYWGQGTLVTVSSGGGGSGGGGSGGGGSGGGGSEIVLTQSPATLSLSPGER AILSCRASQSVYTKYLGWYQQKPGQAPRLLIYDASTRATGIPDRFSGSGSGTDFTLTINR LEPEDFAVYYCQHYGGSPLITFGQGTRLEIKTTTPAPRPPTPAPTIASQPLSLRPEACRP AAGGAVHTRGLDFACDIYIWAPLAGTCGVLLLSLVITLYCKRGRKKLLYIFKQPFMRPVQ TTQEEDGCSCRFPEEEEGGCELRVKFSRSADAPAYKQGQNQLYNELNLGRREEYDVLDKR RGRDPEMGGKPRRKNPQEGLYNELQKDKMAEAYSEIGMKGERRRGKGHDGLYQGLSTATK DTYDALHMQALPPR | **288** |
| M8 CAR | MALPVTALLLPLALLLHAARPQVQLQQSGAEVKKPGASVKVSCKTSGYPFTGYSLHWVRQ APGQGLEWMGWINPNSGGTNYAQKFQGRVTMTRDTSISTAYMELSRLRSDDTAVYYCARD HYGGNSLFYWGQGTLVTVSSGGGGSGGGGSGGGGSGGGGSDIQLTQSPSSISASVGDTVS ITCRASQDSGTWLAWYQQKPGKAPNLLMYDASTLEDGVPSRFSGSASGTEFTLTVNRLQP EDSATYYCQQYNSYPLTFGGGTKVDIKTTTPAPRPPTPAPTIASQPLSLRPEACRPAAGG AVHTRGLDFACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYDVLDKRRGRD PEMGGKPRRKNPQEGLYNELQKDKMAEAYSEIGMKGERRRGKGHDGLYQGLSTATKDTYD ALHMQALPPR | **289** |
| M9 CAR | MALPVTALLLPLALLLHAARPQVQLVQSGAEVKKPGASVEVSCKASGYTFTSYYMHWVRQ APGQGLEWMGIINPSGGSTGYAQKFQGRVTMTRDTSTSTVHMELSSLRSEDTAVYYCARG GYSSSSDAFDIWGQGTMVTVSSGGGGSGGGGSGGGGSGGGGSDIQMTQSPPSLSASVGDR VTITCRASQDISSALAWYQQKPGTPPKLLIYDASSLESGVPSRFSGSGSGTDFTLTISSL QPEDFATYYCQQFSSYPLTFGGGTRLEIKTTTPAPRPPTPAPTIASQPLSLRPEACRPAA GGAVHTRGLDFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRREEYDVLDKRRG RDPEMGGKPRRKNPQEGLYNELQKDKMAEAYSEIGMKGERRRGKGHDGLYQGLSTATKDT YDALHMQALPPR | **290** |
| M10 CAR | MALPVTALLLPLALLLHAARPQVQLVQSGAEVKKPGASVKVSCKASGYTFTSYGISWVRQ APGQGLEWMGWISAYNGNTNYAQKLQGRVTMTTDTSTSTAYMELRSLRSDDTAVYYCARV AGGIYYYYGMDVWGQGTTITVSSGGGGSGGGGSGGGGSGGGGSDIVMTQTPDSLAVSLGE RATISCKSSHSVLYNRNNKNYLAWYQQKPGQPPKLLFYWASTRKSGVPDRFSGSGSGTDF TLTISSLQPEDFATYFCQQTQTFPLTFGQGTRLEINTTTPAPRPPTPAPTIASQPLSLRP EACRPAAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYKQGQNQLYNELNLGRREEYD VLDKRRGRDPEMGGKPRRKNPQEGLYNELQKDKMAEAYSEIGMKGERRRGKGHDGLYQGL STATKDTYDALHMQALPPR | **291** |
| M12 CAR | MALPVTALLLPLALLLHAARPQVQLVQSGAEVKKPGASVKVSCKASGYTFTGYYMHWVRQ APGQGLEWMGRINPNSGGTNYAQKFQGRVTMTTDTSTSTAYMELRSLRSDDTAVYYCART TTSYAFDIWGQGTMVTVSSGGGGSGGGGSGGGGSGGGGSDIQLTQSPSTLSASVGDRVTI TCRASQSISTWLAWYQQKPGKAPNLLIYKASTLESGVPSRFSGSGSGTEFTLTISSLQPD DFATYYCQQYNTYSPYTFGQGTKLEIKTTTPAPRPPTPAPTIASQPLSLRPEACRPAAGG AVHTRGLDFACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYDVLDKRRGRD PEMGGKPRRKNPQEGLYNELQKDKMAEAYSEIGMKGERRRGKGHDGLYQGLSTATKDTYD ALHMQALPPR | **293** |
| M13 CAR | MALPVTALLLPLALLLHAARPQVQLVQSGGGLVKPGGSLRLSCEASGFIFSDYYMGWIRQ APGKGLEWVSYIGRSGSSMYYADSVKGRFTFSRDNAKNSLYLQMNSLRAEDTAVYYCAAS PVVAATEDFQHWGQGTLVTVSSGGGGSGGGGSGGGGSGGGGSDIVMTQTPATLSLSPGER ATLSCRASQSVTSNYLAWYQQKPGQAPRLLLFGASTRATGIPDRFSGSGSGTDFTLTINR LEPEDFAMYYCQQYGSAPVTFGQGTKLEIKTTTPAPRPPTPAPTIASQPLSLRPEACRPA | **294** |
| | AGGAVHTRGLDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR | |
| M14 CAR | MALPVTALLLPLALLLHAARPQVQLVQSGAEVRAPGASVKISCKASGFTFRGYYIHWVRQ APGQGLEWMGIINPSGGSRAYAQKFQGRVTMTRDTSTSTVYMELSSLRSDDTAMYYCART ASCGGDCYYLDYWGQGTLVTVSSGGGGSGGGGSGGGGSGGGGSDIQMTQSPPTLSASVGD RVTITCRASENVNIWLAWYQQKPGKAPKLLIYKSSSLASGVPSRFSGSGSGAEFTLTISS LQPDDFATYYCQQYQSYPLTFGGGTKVDIKTTTPAPRPPTPAPTIASQPLSLRPEACRPA AGGAVHTRGLDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR | **295** |
| M15 CAR | MALPVTALLLPLALLLHAARPQVQLVQSGGGLVQPGRSLRLSCAASGFTFDDYAMHWVRQ APGKGLEWVSGISWNSGSIGYADSVKGRFTISRDNAKNSLYLQMNSLRAEDTAVYYCAKD GSSSWSWGYFDYWGQGTLVTVSSGGGGSGGGGSGGGGSSSELTQDPAVSVALGQTVRTTC QGDALRSYYASWYQQKPGQAPMLVIYGKNNRPSGIPDRFSGSDSGDTASLTITGAQAEDE ADYYCNSRDSSGYPVFGTGTKVTVLTTTPAPRPPTPAPTIASQPLSLRPEACRPAAGGAV HTRGLDFACDIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYDVLDKRRGRDPE MGGKPRRKNPQEGLYNELQKDKMAEAYSEIGMKGERRRGKGHDGLYQGLSTATKDTYDAL HMQALPPR | **296** |
| M16 CAR | MALPVTALLLPLALLLHAARPEVQLVESGGGLVQPGRSLRLSCAASGFTFDDYAMHWVRQ APGKGLEWVSGISWNSGSTGYADSVKGRFTISRDNAKNSLYLQMNSLRAEDTALYYCAKD SSSWYGGGSAFDIWGQGTMVTVSSGGGGSGGGGSGGGGSSSELTQEPAVSVALGQTVRIT CQGDSLRSYYASWYQQKPGQAPVLVIFGRSRRPSGIPDRFSGSSSGNTASLIITGAQAED EADYYCNSRDNTANHYVFGTGTKLTVLTTTPAPRPPTPAPTIASQPLSLRPEACRPAAGG AVHTRGLDFACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYDVLDKRRGRD PEMGGKPRRKNPQEGLYNELQKDKMAEAYSEIGMKGERRRGKGHDGLYQGLSTATKDTYD ALHMQALPPR | **297** |
| M17 CAR | MALPVTALLLPLALLLHAARPEVQLVESGGGLVQPGRSLRLSCAASGFTFDDYAMHWVRQ APGKGLEWVSGISWNSGSTGYADSVKGRFTISRDNAKNSLYLQMNSLRAEDTALYYCAKD SSSWYGGGSAFDIWGQGTMVTVSSGGGGSGGGGSGGGGSSSELTQDPAVSVALGQTVRIT CQGDSLRSYYASWYQQKPGQAPVLVIYGKNNRPSGIPDRFSGSSSGNTASLTITGAQAED EADYYCNSRGSSGNHYVFGTGTKVTVLTTTPAPRPPTPAPTIASQPLSLRPEACRPAAGG AVHTRGLDFACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYDVLDKRRGRD PEMGGKPRRKNPQEGLYNELQKDKMAEAYSEIGMKGERRRGKGHDGLYQGLSTATKDTYD ALHMQALPPR | **298** |
| M18 CAR | MALPVTALLLPLALLLHAARPQVQLVQSGGGLVQPGGSLRLSCAASGFTFSSYWMHWVRQ APGKGLVWVSRINSDGSSTSYADSVKGRFTISRDNAKNTLYLQMNSLRAEDTAVYYCVRT GWVGSYYYYMDVWGKGTTVTVSSGGGGSGGGGSGGGGSGGGGSEIVLTQSPGTLSLSPGE RATLSCRASQSVSSNYLAWYQQKPGQPPRLLIYDVSTRATGIPARFSGGGSGTDFTLTIS SLEPEDFAVYYCQQRSNWPPWTFGQGTKVEIKTTTPAPRPPTPAPTIASQPLSLRPEACR PAAGGAVHTRGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHDGLYQGLSTAT KDTYDALHMQALPPR | **299** |
| M19 CAR | MALPVTALLLPLALLLHAARPQVQLVQSGGGVVQPGRSLRLSCAASGFTFSSYGMHWVRQ APGKGLEWVAVISYDGSNKYYADSVKGRFTISRDNSKNTLYLQMNSLRAEDTAVYYCAKG YSRYYYYGMDVWGQGTTVTVSSGGGGSGGGGSGGGGSGGGGSEIVMTQSPATLSLSPGER AILSCRASQSVYTKYLGWYQQKPGQAPRLLIYDASTRATGIPDRFSGSGSGTDFTLTINR LEPEDFAVYYCQHYGGSPLITFGQGTKVDIKTTTPAPRPPTPAPTIASQPLSLRPEACRP AAGGAVHTRGLDFACDIYIWAPLAGTCGVLLLSLVITLYCKRGRKKLLYIFKQPFMRPVQ TTQEEDGCSCRFPEEEEGGCELRVKFSRSADAPAYKQGQNQLYNELNLGRREEYDVLDKR RGRDPEMGGKPRRKNPQEGLYNELQKDKMAEAYSEIGMKGERRRGKGHDGLYQGLSTATK | **300** |
| | DTYDALHMQALPPR | |
| M20 CAR | MALPVTALLLPLALLLHAARPQVQLVQSGGGLVQPGGSLRLSCAASGFTFSSYAMSWVRQ APGKGLEWVSAISGSGGSTYYADSVKGRFTISRDNSKNTLYLQMNSLRAEDTAVYYCAKR EAAAGHDWYFDLWGRGTLVTVSSGGGGSGGGGSGGGGSGGGGSDIRVTQSPSSLSASVGD RVTITCRASQSISSYLNWYQQKPGKAPKLLIYAASSLQSGVPSRFSGSGSGTDFTLTISS LQPEDFATYYCQQSYSIPLTFGQGTKVEIKTTTPAPRPPTPAPTIASQPLSLRPEACRPA AGGAVHTRGLDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR | **301** |
| M21 CAR | MALPVTALLLPLALLLHAARPQVQLVQSWAEVKKPGASVKVSCKASGYTFTSYYMHWVRQ APGQGLEWMGIINPSGGSTSYAQKFQGRVTMTRDTSTSTVYMELSNLRSEDTAVYYCARS PRVTTGYFDYWGQGTLVTVSSGGGGSGGGGSGGGGSGGGGSDIQLTQSPSTLSASVGDRV TITCRASQSISSWLAWYQQKPGKAPKLLIYKASSLESGVPSRFSGSGSGTEFTLTISSLQ PDDFATYYCQQYSSYPLTFGGGTRLEIKTTTPAPRPPTPAPTIASQPLSLRPEACRPAAG GAVHTRGLDFACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRREEYDVLDKRRGR DPEMGGKPRRKNPQEGLYNELQKDKMAEAYSEIGMKGERRRGKGHDGLYQGLSTATKDTY DALHMQALPPR | **302** |
| M22 CAR | MALPVTALLLPLALLLHAARPQVQLVQSGAEVRRPGASVKISCRASGDTSTRHYIHWLRQ APGQGPEWMGVINPTTGPATGSPAYAQMLQGRVTMTRDTSTRTVYMELRSLRFEDTAVYY CARSVVGRSAPYYFDYWGQGTLVTVSSGGGGSGGGGSGGGGSGGGGSDIQMTQSPSSLSA SVGDRVTITCRASQGISDYSAWYQQKPGKAPKLLIYAASTLQSGVPSRFSGSGSGTDFTL TISYLQSEDFATYYCQQYYSYPLTFGGGTKVDIKTTTPAPRPPTPAPTIASQPLSLRPEA CRPAAGGAVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGHDGLYQGLST ATKDTYDALHMQALPPR | **303** |
| M23 CAR | MALPVTALLLPLALLLHAARPQVQLQQSGAEVKKPGASVKVSCKASGYTFTNYYMHWVRQ APGQGLEWMGIINPSGGYTTYAQKFQGRLTMTRDTSTSTVYMELSSLRSEDTAVYYCARI RSCGGDCYYFDNWGQGTLVTVSSGGGGSGGGGSGGGGSGGGGSDIQLTQSPSTLSASVGD RVTITCRASENVNIWLAWYQQKPGKAPKLLIYKSSSLASGVPSRFSGSGSGAEFTLTISS LQPDDFATYYCQQYQSYPLTFGGGTKVDIKTTTPAPRPPTPAPTIASQPLSLRPEACRPA AGGAVHTRGLDFACDIYIWAPLAGTCGVLLLSLVITLYCKRGRKKLLYIFKQPFMRPVQT TQEEDGCSCRFPEEEEGGCELRVKFSRSADAPAYKQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR | **304** |
| M24 CAR | MALPVTALLLPLALLLHAARPQITLKESGPALVKPTQTLTLTCTFSGFSLSTAGVHVGWI RQPPGKALEWLALISWADDKRYRPSLRSRLDITRVTSKDQVVLSMTNMQPEDTATYYCAL QGFDGYEANWGPGTLVTVSSGGGGSGGGGSGGGGSGGGGSDIVMTQSPSSLSASAGDRVT ITCRASRGISSALAWYQQKPGKPPKLLIYDASSLESGVPSRFSGSGSGTDFTLTIDSLEP EDFATYYCQQSYSTPWTFGQGTKVDIKTTTPAPRPPTPAPTIASQPLSLRPEACRPAAGG AVHTRGLDFACDIYIWAPLAGTCGVLLLSLVITLYCKRGRKKLLYIFKQPFMRPVQTTQE EDGCSCRFPEEEEGGCELRVKFSRSADAPAYKQGQNQLYNELNLGRREEYDVLDKRRGRD PEMGGKPRRKNPQEGLYNELQKDKMAEAYSEIGMKGERRRGKGHDGLYQGLSTATKDTYD ALHMQALPPR | **305** |

In one embodiment, the cell of the invention (e.g., a cell comprising both a TA CAR and a BCA CAR) comprises a TA CAR molecule that binds mesothelin, and comprises (e.g., consists of) an amino acid sequence as provided in Table 11 and Table 2 of International Publication No. WO2015/090230, filed December 19, 2014 .

In one embodiment, the TA CAR molecule comprises (e.g., consists of) an amino acid sequence of SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, or SEQ ID NO: 306; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence of SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, or SEQ ID NO: 306; or an amino acid sequence having 85%, 90%, 95%, 96%, 97%, 98%, 99% identity to an amino acid sequence of SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, or SEQ ID NO: 306.

In one aspect, the cell of the invention (e.g., a cell comprising both a TA CAR and a BCA CAR) comprises a TA CAR molecule comprising an antigen binding domain that binds to a tumor antigen. In one embodiment, the TA CAR comprises a CAR molecule comprising a EGFRvIII antigen binding domain (e.g., a murine, human or humanized antibody or antibody fragment that specifically binds to mesothelin), a transmembrane domain, and an intracellular signaling domain (e.g., an intracellular signaling domain comprising a costimulatory domain and/or a primary signaling domain).

Exemplary TA CAR molecules that target EGFRvIII are described herein, and are provided in Table 17, or in Table 2 of WO/2014/130657 or as described in WO2016/014789.

**Table 17. Humanized EGFRvIII CAR Constructs. Sequences are provided with a leader, and the CDRs are underlined. Nt stands for nucleic acid and aa stands for amino acid**

| **Name** | **SEQ ID NO:** | **Sequence** |
|---|---|---|
| **CAR 1** | | |
| CAR 1 - Full - nt | 1042 | atggccctccctgtcaccgccctgctgcttccgctggctcttctgctccacgccgctcggcccg agatccagctggtgcagtcgggagctgaagtcaaaaagcctggcgcaaccgtcaagatctcg tgcaaaggatcagggttcaacatcgaggactactacatccattgggtgcaacaggcacccgg aaaaggcctggagtggatggggaggattgacccagaaaatgacgaaaccaagtacggacc gatcttccaaggacgggtgaccatcacggctgacacttccactaacaccgtctacatggaact ctcgagccttcgctcggaagataccgcggtgtactactgcgcctttagaggtggagtctactgg ggacaagggactaccgtcaccgtgtcgtcaggtggcggaggatcaggcggaggcggctcc ggtggaggaggaagcggaggaggtggctccgacgtggtgatgacgcagtcaccggactcc ttggcggtgagcctgggtgaacgcgccactatcaactgcaagagctcccagagcttgctgga ctccgatggaaagacttatctcaattggctgcaacagaagcctggccagccgccaaagagac tcatctcactggtgagcaagctggatagcggagtgccagatcggttttcgggatcgggctcag gcaccgacttcaccctgactatttcctccctccaagccgaggatgtggccgtctactactgttgg caggggactcacttcccggggaccttcggtggaggcactaaggtggagatcaaaaccactac cccagcaccgaggccacccaccccggctcctaccatcgcctcccagcctctgtccctgcgtc cggaggcatgtagacccgcagctggtggggccgtgcatacccggggtcttgacttcgcctgc gatatctacatttgggcccctctggctggtacttgcggggtcctgctgctttcactcgtgatcact ctttactgtaagcgcggtcggaagaagctgctgtacatctttaagcaacccttcatgaggcctgt gcagactactcaagaggaggacggctgttcatgccggttcccagaggaggaggaaggcgg ctgcgaactgcgcgtgaaattcagccgcagcgcagatgctccagcctacaagcaggggcag aaccagctctacaacgaactcaatcttggtcggagagaggagtacgacgtgctggacaagcg gagaggacgggacccagaaatgggcgggaagccgcgcagaaagaatccccaagagggc ctgtacaacgagctccaaaaggataagatggcagaagcctatagcgagattggtatgaaagg ggaacgcagaagaggcaaaggccacgacggactgtaccagggactcagcaccgccacca aggacacctatgacgctcttcacatgcaggccctgccgcctcgg |
| CAR 1 - Full - aa | 1043 | malpvtalllplalllhaarpeiqlvqsgaevkkpgatvkisckgsgfniedyyihwvvqqap gkglewmgridpendetkygpifqgrvtitadtstntvymelsslrsedtavyycafrgg vywgqgttvtvssggggsggggsggggsggggsdvvmtqspdslavslgeratinckss qslldsdgktylnwlqqkpgqppkrlislvskldsgvpdrfsgsgsgtdftltisslqaedva vyycwqgthfpgtfgggtkveikttpaprpptpaptiasqplslrpeacrpaaggavhtrg |
| | | ldfacdiyiwaplagtcgvlllslvitlyckrgrkkllyifkqpfmrpvqttqeedgcscrfpe eeeggcelrvkfsrsadapaykqgqnqlynelnlgrreeydvldkrrgrdpemggkprrk npqeglynelqkdkmaeayseigmkgerrrgkghdglyqglstatkdtydalhmqalpp r |

| **CAR 2** | | |
|---|---|---|
| CAR 2 - Full - nt | 1048 | atggccctccctgtcaccgccctgctgcttccgctggctcttctgctccacgccgctcggcccg acgtggtcatgactcaaagcccagattccttggctgtctcccttggagaaagagcaacgatcaa ttgcaaaagctcgcagtccctgttggactccgatggaaaaacctacctcaactggctgcagca gaagccgggacaaccaccaaagcggctgatttccctcgtgtccaagctggacagcggcgtg ccggatcgcttctcgggcagcggctcgggaaccgattttactctcactatttcgtcactgcaagc ggaggacgtggcggtgtattactgctggcagggcactcacttcccgggtacttttggtggagg taccaaagtcgaaatcaagggtggaggcgggagcggaggaggcgggtcgggaggagga ggatcgggtggcggaggctcagaaatccagctggtgcagtcaggtgccgaagtgaagaag cctggggccacggtgaagatctcgtgcaaggggagcggattcaacatcgaggattactacat ccattgggtgcaacaggcccctggcaaagggctggaatggatgggaaggatcgaccccga gaatgacgagactaagtacggcccgatcttccaaggacgggtgaccatcactgcagacactt caaccaacaccgtctacatggaactctcctcgctgcgctccgaggacaccgccgtgtactact gtgctttcagaggaggagtctactggggacagggaacgaccgtgaccgtcagctcaaccact accccagcaccgaggccacccaccccggctcctaccatcgcctcccagcctctgtccctgcg tccggaggcatgtagacccgcagctggtggggccgtgcatacccggggtcttgacttcgcct gcgatatctacatttgggcccctctggctggtacttgcggggtcctgctgctttcactcgtgatca ctctttactgtaagcgcggtcggaagaagctgctgtacatctttaagcaacccttcatgaggcct gtgcagactactcaagaggaggacggctgttcatgccggttcccagaggaggaggaaggcg gctgcgaactgcgcgtgaaattcagccgcagcgcagatgctccagcctacaagcaggggca gaaccagctctacaacgaactcaatcttggtcggagagaggagtacgacgtgctggacaagc ggagaggacgggacccagaaatgggcgggaagccgcgcagaaagaatccccaagaggg cctgtacaacgagctccaaaaggataagatggcagaagcctatagcgagattggtatgaaag gggaacgcagaagaggcaaaggccacgacggactgtaccagggactcagcaccgccacc aaggacacctatgacgctcttcacatgcaggccctgccgcctcgg |
| CAR 2 - Full - aa | 1049 | malpvtalllplalllhaarpdvvmtqspdslavslgeratinckssqslldsdgktylnwlq qkpgqppkrlislvsskldsgvpdrfsgsgsgtdftltisslqaedvavyycwqgthfpgtfg |
| | | ggtkveikggggsggggsggggsggggseiqlvqsgaevkkpgatvkisckgsgfnied yyihwvqqapgkglewmgridpendetkygpifqgrvtitadtstntvymelsslrsed tavyycafrggvywgqgttvtvssttpaprpptpaptiasqplslrpeacrpaaggavhtrg ldfacdiyiwaplagtcgvlllslvitlyckrgrkkllyifkqpfmrpvqttqeedgcscrfpe eeeggcelrvkfsrsadapaykqgqnqlynelnlgrreeydvldkrrgrdpemggkprrk npqeglynelqkdkmaeayseigmkgerrrgkghdglyqglstatkdtydalhmqalpp r |

| **CAR 3** | | |
|---|---|---|
| CAR 3 **-** Full - nt | 1054 | atggccctccctgtcaccgccctgctgcttccgctggctcttctgctccacgccgctcggcccg aaatccagctggtgcaaagcggagccgaggtgaagaagcccggagaatccctgcgcatctc gtgtaagggttccggctttaacatcgaggattactacatccactgggtgagacagatgccggg caaaggtctggaatggatgggccgcatcgacccggagaacgacgaaaccaaatacggacc aatcttccaaggacatgtgactatttccgcggatacctccatcaacactgtctacttgcagtgga gctcgctcaaggcgtcggataccgccatgtactactgcgcattcagaggaggtgtgtactggg gccagggcactacggtcaccgtgtcctcgggaggtggagggtcaggaggcggaggctcgg gcggtggaggatcaggcggaggaggaagcgatgtggtcatgactcaatccccactgtcact gcctgtcactctggggcaaccggcttccatctcatgcaagtcaagccaatcgctgctcgactcc gacggaaaaacctacctcaattggcttcagcagcgcccaggccagtcgcctcggaggctgat ctcactcgtgtcgaagcttgactccggggtgccggatcggtttagcggaagcggatcgggga ccgacttcacgttgaagattagccgggtggaagccgaggacgtgggagtctattactgctggc aggggacccacttcccggggactttcggaggaggcaccaaagtcgagattaagaccactac cccagcaccgaggccacccaccccggctcctaccatcgcctcccagcctctgtccctgcgtc cggaggcatgtagacccgcagctggtggggccgtgcatacccggggtcttgacttcgcctgc gatatctacatttgggcccctctggctggtacttgcggggtcctgctgctttcactcgtgatcact ctttactgtaagcgcggtcggaagaagctgctgtacatctttaagcaacccttcatgaggcctgt gcagactactcaagaggaggacggctgttcatgccggttcccagaggaggaggaaggcgg ctgcgaactgcgcgtgaaattcagccgcagcgcagatgctccagcctacaagcaggggcag aaccagctctacaacgaactcaatcttggtcggagagaggagtacgacgtgctggacaagcg gagaggacgggacccagaaatgggcgggaagccgcgcagaaagaatccccaagagggc ctgtacaacgagctccaaaaggataagatggcagaagcctatagcgagattggtatgaaagg ggaacgcagaagaggcaaaggccacgacggactgtaccagggactcagcaccgccacca |
| | | aggacacctatgacgctcttcacatgcaggccctgccgcctcgg |
| CAR 3 - Full - aa | 1055 | aggacacctatgacgctcttcacatgcaggccctgccgcctcgg malpvtallllplalllhaarpeiqlvqsgaevkkpgeslrisckgsgfniedyyihwvrqmp gkglewmgridpendetkygpifqghvtisadtsintvylqwsslkasdtamyycafrg gyywgqgttvtvssggggsggggsggggsggggsdvvmtqsplslpvtlgqpasisscks sqsllldsdgktylnwlqqrpgqsprrlislvskldsgvpdrfsgsgsgtdftlkisrveaedv gvyycwqgthfpgtfgggtkveikttpaprpptpaptiasqplslrpeacrpaaggavhtr gldfacdiyiwaplagtcgvllllslvitlyckrgrkkllyifkqpfmrpvqttqeedgcscrfp eeeeggcelrvkfsrsadapaykqgqnqlynelnlgrreeydvldkrrgrdpemggkprr knpqeglynelqkdkmaeayseigmkgerrrgkghdglyqglstatkdtydalhmqalp pr |

| **CAR 4** | | |
|---|---|---|
| CAR 4 - Full - nt | 1060 | atggccctccctgtcaccgccctgctgcttccgctggctcttctgctccacgccgctcggcccg acgtcgtcatgacccaatcccctctctccctgccggtcaccctgggtcagccggcgtcgatctc atgcaaaagctcacagtccctgctggattcggacggaaaaacctacttgaactggctccaaca gaggccgggtcagtcccctcgcagactgatctcgctggtgagcaagctcgactcgggtgtgc cggatcggttctccgggtcaggatcgggcaccgactttacgctcaagatttcgagagtggagg ccgaggatgtgggagtgtactattgctggcagggcacgcatttccccgggacctttggaggc gggactaaggtggaaatcaagggaggtggcggatcaggcggaggaggcagcggcggag gtggatcaggaggcggagggtcagagatccagctggtccaaagcggagcagaggtgaaga agccaggcgagtcccttcgcatttcgtgcaaagggagcggcttcaacattgaagattactacat ccactgggtgcggcaaatgccaggaaagggtctggaatggatgggacggatcgacccaga aaatgatgaaactaagtacggaccgatcttccaaggacacgtcactatctccgcggacacttc gatcaacaccgtgtacctccagtggagcagcttgaaagcctccgacaccgctatgtactactgt gccttccgcggaggagtctactggggacaggggactactgtgaccgtgtcgtccaccactac cccagcaccgaggccacccaccccggctcctaccatcgcctcccagcctctgtccctgcgtc cggaggcatgtagacccgcagctggtggggccgtgcatacccggggtcttgacttcgcctgc gatatctacatttgggcccctctggctggtacttgcggggtcctgctgctttcactcgtgatcact ctttactgtaagcgcggtcggaagaagctgctgtacatctttaagcaacccttcatgaggcctgt gcagactactcaagaggaggacggctgttcatgccggttcccagaggaggaggaaggcgg ctgcgaactgcgcgtgaaattcagccgcagcgcagatgctccagcctacaagcaggggcag aaccagctctacaacgaactcaatcttggtcggagagaggagtacgacgtgctggacaagcg |
| | | gagaggacgggacccagaaatggcgggaagccgcgcagaaagaatccccaagagggc ctgtacaacgagctccaaaaggataagatggcagaagcctatagcgagattggtatgaaagg ggaacgcagaagaggcaaaggccacgacggactgtaccagggactcagcaccgccacca aggacacctatgacgctcttcacatgcaggccctgccgcctcgg |
| CAR 4 - Full - aa | 1061 | malpvtalllplalllhaarpdvvmtqsplslpvtlgqpasisckssqslldsdgktylnwlq qrpgqsprrlislvskldsgvpdrfsgsgsgtdftlkisrveaedvgvyycwqgthfpgtfg ggtkveikggggsggggsggggsggggseiqlvqsgaevkkpgeslrisckgsgfniedy yihwvrqmpgkglewmgridpendetkygpifqghvtisadtsintvylqwsslkasd tamyycafrggvywgqgttvtvsstttpaprpptpaptiasqplslrpeacrpaaggavhtr gldfacdiyiwaplagtcgvlllslvitlyckrgrkkllyifkqpfmrpvqttqeedgcscrfp eeeeggcelrvkfsrsadapaykqgqnqlynelnlgrreeydvldkrrgrdpemggkprr knpqeglynelqkdkmaeayseigmkgerrrgkghdglyqglstatkdtydalhmqalp pr |

| **CAR 5** | | |
|---|---|---|
| CAR 5 - Full - nt | 1066 | atggccctccctgtcaccgccctgctgcttccgctggctcttctgctccacgccgctcggcccg aaatccagctcgtgcagagcggagccgaggtcaagaaaccgggtgctaccgtgaagatttca tgcaagggatcgggcttcaacatcgaggattactacatccactgggtgcagcaggcaccagg aaaaggacttgaatggatgggccggatcgacccggaaaatgacgagactaagtacggccct atcttccaaggacgggtgacgatcaccgcagacactagcaccaacaccgtctatatggaactc tcgtccctgaggtccgaagatactgccgtgtactactgtgcgtttcgcggaggtgtgtactggg gacagggtaccaccgtcaccgtgtcatcgggcggtggaggctccggtggaggagggtcag gaggcggtggaagcggaggaggcggcagcgacgtggtcatgactcaatcgccgctgtcgc tgcccgtcactctgggacaacccgcgtccatcagctgcaaatcctcgcagtcactgcttgactc cgatggaaagacctacctcaactggctgcagcaacgcccaggccaatccccaagacgcctg atctcgttggtgtcaaagctggactcaggggtgccggaccggttctccgggagcgggtcggg cacggatttcactctcaagatctccagagtggaagccgaggatgtgggagtctactactgctg gcagggaacccatttccctggaacttttggcggaggaactaaggtcgagattaaaccactac cccagcaccgaggccacccaccccggctcctaccatcgcctcccagcctctgtccctgcgtc cggaggcatgtagacccgcagctggtggggccgtgcatacccggggtcttgacttcgcctgc gatatctacatttgggcccctctggctggtacttgcggggtcctgctgctttcactcgtgatcact ctttactgtaagcgcggtcggaagaagctgctgtacatctttaagcaacccttcatgaggcctgt |
| | | gcagactactcaagaggaggacggctgttcatgccggttcccagaggaggaggaaggcgg ctgcgaactgcgcgtgaaattcagccgcagcgcagatgctccagcctacaagcaggggcag aaccagctctacaacgaactcaatcttggtcggagagaggagtacgacgtgctggacaagcg gagaggacgggacccagaaatgggcgggaagccgcgcagaaagaatccccaagagggc ctgtacaacgagctccaaaaggataagatggcagaagcctatagcgagattggtatgaaagg ggaacgcagaagaggcaaaggccacgacggactgtaccagggactcagcaccgccacca aggacacctatgacgctcttcacatgcaggccctgccgcctcgg |
| CAR 5 - Full - aa | 1067 | malpvtalllplalllhaarpeiqlvqsgaevkkpgatvkisckgsgfniedyvihwvqqap gkglewmgridpendetkygpifqgrvtitadtstntvymelsslrsedtavyycafrgg vywgqgttvtvssggggsggggsggggsggggsdvvmtqsplslpvtlgqpasisckss qsllldsdgktylnwlqqrpgqsprrlislvskldsgvpdrfsgsgsgtdftlkisrveaedvg vyycwqgthfpgtfgggtkveikttpaprpptpaptiasqplslrpeacrpaaggavhtrg ldfacdiyiwaplagtcgvlllslvitlyckrgrkkllyifkqpfmrpvqttqeedgcscrfpe eeeggcelrvkfsrsadapaykqgqnqlynelnlgrreeydvldkrrgrdpemggkprrk npqeglynelqkdkmaeayseigmkgerrrgkghdglyqglstatkdtydalhmqalpp r |

| **CAR 6** | | |
|---|---|---|
| CAR6 - Full - nt | 1072 | atggccctccctgtcaccgccctgctgcttccgctggctcttctgctccacgccgctcggcccg agattcagctcgtgcaatcgggagcggaagtcaagaagccaggagagtccttgcggatctca tgcaagggtagcggctttaacatcgaggattactacatccactgggtgaggcagatgccggg gaagggactcgaatggatgggacggatcgacccagaaaacgacgaaactaagtacggtcc gatcttccaaggccatgtgactattagcgccgatacttcaatcaataccgtgtatctgcaatggtc ctcattgaaagcctcagataccgcgatgtactactgtgctttcagaggaggggtctactgggga cagggaactaccgtgactgtctcgtccggcggaggcgggtcaggaggtggcggcagcgga ggaggagggtccggcggaggtgggtccgacgtcgtgatgacccagagccctgacagcctg gcagtgagcctgggcgaaagagctaccattaactgcaaatcgtcgcagagcctgctggactc ggacggaaaaacgtacctcaattggctgcagcaaaagcctggccagccaccgaagcgcctt atctcactggtgtcgaagctggattcgggagtgcccgatcgcttctccggctcgggatcgggt actgacttcaccctcactatctcctcgcttcaagcagaggacgtggccgtctactactgctggca gggaacccactttccgggaaccttcggcggagggacgaaagtggagatcaagaccactacc ccagcaccgaggccacccaccccggctcctaccatcgcctcccagcctctgtccctgcgtcc |
| | | ggaggcatgtagacccgcagctggtggggccgtgcatacccggggtcttgacttcgcctgcg atatctacatttgggcccctctggctggtacttgcggggtcctgctgctttcactcgtgatcactct ttactgtaagcgcggtcggaagaagctgctgtacatctttaagcaacccttcatgaggcctgtg cagactactcaagaggaggacggctgttcatgccggttcccagaggaggaggaaggcggct gcgaactgcgcgtgaaattcagccgcagcgcagatgctccagcctacaagcaggggcaga accagctctacaacgaactcaatcttggtcggagagaggagtacgacgtgctggacaagcgg agaggacgggacccagaaatgggcgggaagccgcgcagaaagaatccccaagagggcct gtacaacgagctccaaaaggataagatggcagaagcctatagcgagattggtatgaaaggg gaacgcagaagaggcaaaggccacgacggactgtaccagggactcagcaccgccaccaa ggacacctatgacgctcttcacatgcaggccctgccgcctcgg |
| CAR6 - Full - aa | 1073 | malpvtalllplalllhaarpeiqlvqsgaevkkpgeslrisckgsgfniedyyihwvrqmp gkglewmgridpendetkygpifqghvtisadtsintvylqwsslkasdtamyycafrg gyywgqgttvtvssggggsggggsggggsggggsdvvmtqspdslavslgeratinckks sqslldsdgktylnwlqqkpgqppkrlislvskldsgvpdrfsgsgsgtdftltisslqaedv avyycwqgthfpgtfgggtkveikttpaprpptpaptiasqplslrpeacrpaaggavhtr gldfacdiyiwaplagtcgvlllslvitlyckrgrkkllyifkqpfmrpvqttqeedgcscrfp eeeeggcelrvkfsrsadapaykqgqnqlynelnlgrreeydvldkrrgrdpemggkprr knpqeglynelqkdkmaeayseigmkgerrrgkghdglyqglstatkdtydalhmqalp pr |

| CAR 7 | | |
|---|---|---|
| CAR 7 Full - nt | 1078 | atggccctccctgtcaccgccctgctgcttccgctggctcttctgctccacgccgctcggcccg acgtggtgatgactcagtcgcctgactcgctggctgtgtcccttggagagcgggccactatca attgcaagtcatcccagtcgctgctggattccgacgggaaaacctacctcaattggctgcagca aaaaccgggacagcctccaaagcggctcatcagcctggtgtccaagttggacagcggcgtg ccagaccgcttctccggttcgggaagcggtactgatttcacgctgaccatctcatccctccaag cggaggatgtggcagtctactactgttggcagggcacgcattttccgggcacttttggaggag ggaccaaggtcgaaatcaagggaggaggtggctcgggcggaggaggctcgggaggagg aggatcaggaggcggtggaagcgagattcaactggtccagagcggcgcagaagtcaagaa gccgggtgaatcgctcagaatctcgtgcaaaggatcgggattcaacatcgaggactactacat tcactgggtcagacaaatgccgggcaaagggctggaatggatggggaggatcgaccccga aaacgatgaaaccaagtacggaccaatcttccaagggcacgtgaccatttcggcggacacct |
| | | caatcaacactgtgtacctccagtggagctcacttaaggccagcgataccgccatgtactattg cgctttccgcggaggggtgtactggggacagggcactactgtgaccgtgtcatccaccactac cccagcaccgaggccacccaccccggctcctaccatcgcctcccagcctctgtccctgcgtc cggaggcatgtagacccgcagctggtggggccgtgcatacccggggtcttgacttcgcctgc gatatctacatttgggcccctctggctggtacttgcggggtcctgctgctttcactcgtgatcact ctttactgtaagcgcggtcggaagaagctgctgtacatctttaagcaacccttcatgaggcctgt gcagactactcaagaggaggacggctgttcatgccggttcccagaggaggaggaaggcgg ctgcgaactgcgcgtgaaattcagccgcagcgcagatgctccagcctacaagcaggggcag aaccagctctacaacgaactcaatcttggtcggagagaggagtacgacgtgctggacaagcg gagaggacgggacccagaaatgggcgggaagccgcgcagaaagaatccccaagagggc ctgtacaacgagctccaaaaggataagatggcagaagcctatagcgagattggtatgaaagg ggaacgcagaagaggcaaaggccacgacggactgtaccagggactcagcaccgccacca aggacacctatgacgctcttcacatgcaggccctgccgcctcgg malpvtalllplalllhaarpdvvmtqspdslavslgeratinckssqsllldsdgktylnwlq qkpgqppkrlislvsskldsgvpdrfsgsgsgtdftltisslqaedvavyycwqgthfpgtfg ggtkveikggggsggggsggggsggggseiqlvqsgaevkkpgeslrisckgsgfniedy yihwvrqmpgkglewmgridpendetkygpifqghvtisadtsintvylqwsslkasd tamyycafrggvywgqgttvtvsstttpaprpptpaptiasqplslrpeacrpaaggavhtr gldfacdiyiwaplagtcgvlllslvitlyckrgrkkllyifkqpfmrpvqttqeedgcscrfp eeeeggcelrvkfsrsadapaykqgqnqlynelnlgrreeydvldkrrgrdpemggkprr knpqeglynelqkdkmaeayseigmkgerrrgkghdglyqglstatkdtydalhmqalp pr |
| CAR 7 Full - aa | 1079 | malpvtalllplalllhaarpdvvmtqspdslavslgeratinckssqslldsdgktylnwlq qkpgqppkrlislvskldsgvpdrfsgsgsgtdftltisslqaedvavyycwqgthfpgtfg ggtkveikggggsggggsggggsggggseiqlvqsgaevkkpgeslrisckgsgfniedy yihwvrqmpgkglewmgridpendetkygpifqghvtisadtsintvylqwsslkasd tamyycafrggvywgqgttvtvsstttpaprpptpaptiasqplslrpeacrpaaggavhtr gldfacdiyiwaplagtcgvlllslvitlyckrgrkkllyifkqpfmrpvqttqeedgcscrfp eeeeggcelrvkfsrsadapaykqgqnqlynelnlgrreeydvldkrrgrdpemggkprr knpqeglynelqkdkmaeayseigmkgerrrgkghdglyqglstatkdtydalhmqalp pr |

| **CAR 8** | | |
|---|---|---|
| CAR 8 - Full - nt | 1084 | atggccctccctgtcaccgccctgctgcttccgctggctcttctgctccacgccgctcggcccg atgtggtcatgacgcagtcaccactgtccctccccgtgacccttggacagccagcgtcgatta gctgcaagtcatcccaatccctgctcgattcggatggaaagacctatctcaactggctgcagca aagacccggtcagagccctaggagactcatctcgttggtgtcaaagctggacagcggagtgc cggaccggttttccggttcgggatcggggacggacttcactctgaagatttcacgggtggaag ctgaggatgtgggagtgtactactgctggcagggaacccatttccctggcacttttggcggag gaactaaggtcgaaatcaagggaggaggtggctcgggaggaggcggatcgggcggaggc gggagcggcggaggagggtccgaaatccaacttgtccagtcaggagccgaagtgaagaaa |
| | | ccgggagccaccgtcaaaatcagctgtaagggatcgggattcaatatcgaggactactacatc cactgggtgcagcaagctccgggcaaaggactggagtggatggggcgcatcgacccagag aacgacgaaaccaaatacggcccgatcttccaagggcgggtgaccatcaccgcggacacct caactaacactgtgtacatggagctgagctccctgcgctccgaagatactgcagtctactactg cgccttccgcggtggtgtgtactggggacagggcaccactgtgactgtcagctcgaccactac cccagcaccgaggccacccaccccggctcctaccatcgcctcccagcctctgtccctgcgtc cggaggcatgtagacccgcagctggtggggccgtgcatacccggggtcttgacttcgcctgc gatatctacatttgggcccctctggctggtacttgcggggtcctgctgctttcactcgtgatcact ctttactgtaagcgcggtcggaagaagctgctgtacatctttaagcaacccttcatgaggcctgt gcagactactcaagaggaggacggctgttcatgccggttcccagaggaggaggaaggcgg ctgcgaactgcgcgtgaaattcagccgcagcgcagatgctccagcctacaagcaggggcag aaccagctctacaacgaactcaatcttggtcggagagaggagtacgacgtgctggacaagcg gagaggacgggacccagaaatgggcgggaagccgcgcagaaagaatccccaagagggc ctgtacaacgagctccaaaaggataagatggcagaagcctatagcgagattggtatgaaagg ggaacgcagaagaggcaaaggccacgacggactgtaccagggactcagcaccgccacca aggacacctatgacgctcttcacatgcaggccctgccgcctcgg |
| CAR 8 - Full - aa | 1085 | malpvtalllplalllhaarpdvvmtqsplslpvtlgqpasisckssqslldsdgktylnwlq qrpgqsprrlislvsskldsgvpdrfsgsgsgtdftlkisrveaedvgvyycwqgthfpgtfg ggtkveikggggsggggsggggsggggseiqlvqsgaevkkpgatvkisckgsgfnied yyihwvqqapgkglewmgridpendetkvgpifqgrvtitadtstntvymelsslrsed tavyycafrggvywgqgttvtvsstttpaprpptpaptiasqplslrpeacrpaaggavhtrg ldfacdiyiwaplagtcgvlllslvitlyckrgrkkllyifkqpfmrpvqttqeedgcscrfpe eeeggcelrvkfsrsadapaykqgqnqlynelnlgrreeydvldkrrgrdpemggkprrk npqeglynelqkdkmaeayseigmkgerrrgkghdglyqglstatkdtydalhmqalpp r |

| **CAR 9** | | Mouse anti-EGFRvIII clone 3C10 |
|---|---|---|
| CAR 9 - Full - nt | 1089 | atggccctccctgtcaccgccctgctgcttccgctggctcttctgctccacgccgctcggcccg agatccagctccaacagagcggagccgaactggtcaaaccgggagcgtcggtgaagttgtc atgcactggatcgggcttcaacatcgaggattactacatccactgggtcaagcaacgcaccga gcaggggctggaatggatcggacggatcgaccccgaaaacgatgaaaccaagtacgggcc tatcttccaaggacgggccaccattacggctgacacgtcaagcaataccgtctacctccagctt |
| | | tccagcctgacctccgaggacactgccgtgtactactgcgccttcagaggaggcgtgtactgg ggaccaggaaccactttgaccgtgtccagcggaggcggtggatcaggaggaggaggctca ggcggtggcggctcgcacatggacgtggtcatgactcagtccccgctgaccctgtcggtggc aattggacagagcgcatccatctcgtgcaagagctcacagtcgctgctggattccgacggaaa gacttatctgaactggctgctccaaagaccagggcaatcaccgaaacgccttatctccctggtg tcgaaactcgactcgggtgtgccggatcggtttaccggtagcgggtccggcacggacttcact ctccgcatttcgagggtggaagcggaggatctcgggatctactactgttggcagggaaccca cttccctgggacttttggaggcggaactaagctggaaatcaagaccactaccccagcaccga ggccacccaccccggctcctaccatcgcctcccagcctctgtccctgcgtccggaggcatgta gacccgcagctggtggggccgtgcatacccggggtcttgacttcgcctgcgatatctacatttg ggcccctctggctggtacttgcggggtcctgctgctttcactcgtgatcactctttactgtaagcg cggtcggaagaagctgctgtacatctttaagcaacccttcatgaggcctgtgcagactactcaa gaggaggacggctgttcatgccggttcccagaggaggaggaaggcggctgcgaactgcgc gtgaaattcagccgcagcgcagatgctccagcctacaagcaggggcagaaccagctctaca acgaactcaatcttggtcggagagaggagtacgacgtgctggacaagcggagaggacggg acccagaaatgggcgggaagccgcgcagaaagaatccccaagagggcctgtacaacgag ctccaaaaggataagatggcagaagcctatagcgagattggtatgaaaggggaacgcagaa gaggcaaaggccacgacggactgtaccagggactcagcaccgccaccaaggacacctatg acgctcttcacatgcaggccctgccgcctcgg |
| CAR 9 - Full - aa | 1090 | malpvtalllplalllhaarpeiqlqqsgaelvkpgasvklsctgsgfniedyyihwvkqrte qglewigridpendetkygpifqgratitadtssntvylqlssltsedtavyycafrggvyw gpgttltvssggggsggggsggggshmdvvmtqspltlsvaigqsasisckssqsllldsdg ktylnwllqrpgqspkrlislvskldsgvpdrftgsgsgtdftlrisrveaedlgiyycwqgt hfpgtfgggtkleiktttpaprpptpaptiasqplslrpeacrpaaggavhtrgldfacdiyiw aplagtcgvlllslvitlyckrgrkkllyifkqpfmrpvqttqeedgcscrfpeeeeggcelrv kfsrsadapaykqgqnqlynelnlgrreeydvldkrrgrdpemggkprrknpqeglynel qkdkmaeayseigmkgerrrgkghdglyqglstatkdtydalhmqalppr |

| **CAR10** | | Anti-EGFRvIII clone 139 |
|---|---|---|
| CAR 10 Full - nt | 1095 | atggccctccctgtcaccgccctgctgcttccgctggctcttctgctccacgccgctcggcccg atatccaaatgactcagagcccttcatccctgagcgccagcgtcggagacagggtgaccatc acgtgccgggcatcccaaggcattagaaataacttggcgtggtatcagcaaaaaccaggaaa |
| | | ggccccgaagcgcctgatctacgcggcctccaaccttcagtcaggagtgccctcgcgcttca ccgggagcggtagcggaactgagtttacccttatcgtgtcgtccctgcagccagaggacttcg cgacctactactgcctccagcatcactcgtacccgttgacttcgggaggcggaaccaaggtcg aaatcaaacgcactggctcgacgtcagggtccggtaaaccgggatcgggagaaggatcgga agtccaagtgctggagagcggaggcggactcgtgcaacctggcgggtcgctgcggctcag ctgtgccgcgtcgggttttactttcagctcgtacgctatgtcatgggtgcggcaggctccggga aaggggctggaatgggtgtccgctatttccggctcgggtggaagcaccaattacgccgactc cgtgaagggacgcttcaccatctcacgggataactccaagaatactctgtacctccagatgaa ctcgctgagagccgaggacaccgcagtgtactactgcgcagggtcaagcggctggtccgaa tactggggacagggcaccctcgtcactgtcagctccaccactaccccagcaccgaggccac ccaccccggctcctaccatcgcctcccagcctctgtccctgcgtccggaggcatgtagacccg cagctggtggggccgtgcatacccggggtcttgacttcgcctgcgatatctacatttgggcccc tctggctggtacttgcggggtcctgctgctttcactcgtgatcactctttactgtaagcgcggtcg gaagaagctgctgtacatctttaagcaacccttcatgaggcctgtgcagactactcaagagga ggacggctgttcatgccggttcccagaggaggaggaaggcggctgcgaactgcgcgtgaa attcagccgcagcgcagatgctccagcctacaagcaggggcagaaccagctctacaacgaa ctcaatcttggtcggagagaggagtacgacgtgctggacaagcggagaggacgggaccca gaaatgggcgggaagccgcgcagaaagaatccccaagagggcctgtacaacgagctccaa aaggataagatggcagaagcctatagcgagattggtatgaaaggggaacgcagaagaggc aaaggccacgacggactgtaccagggactcagcaccgccaccaaggacacctatgacgctc ttcacatgcaggccctgccgcctcgg |
| CAR 10 Full - aa | 1096 | malpvtalllplalllhaarpdiqmtqspsslsasvgdrvtitcrasqgirnnlawyqqkpgk apkrliyaasnlqsgvpsrftgsgsgteftlivsslqpedfatyyclqhhsypltsgggtkveik rtgstsgsgkpgsgegsevqvlesggglvqpggslrlscaasgftfssyamswvrqapgkg lewvsaisgsggstnyadsvkgrftisrdnskntlylqmnslraedtavyycagssgwsey wgqgtlvtvsstttpaprpptpaptiasqplslrpeacrpaaggavhtrgldfacdiyiwapla gtcgvlllslvitlyckrgrkkllyifkqpfmrpvqttqeedgcscrfpeeeeggcelrvkfsrs adapaykqgqnqlynelnlgrreeydvldkrrgrdpemggkprrknpqeglynelqkdk maeayseigmkgerrrgkghdglyqglstatkdtydalhmqalppr |

In one embodiment, the cell of the invention (e.g., a cell comprising both a TA CAR and a BCA CAR) comprises a TA CAR molecule that binds EGFRvIII that comprises (e.g., consists of) an amino acid sequence as provided in Table 17. In one embodiment, the TA CAR that binds EGFRvIII comprises (e.g., consists of) an amino acid sequence of SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090, or SEQ ID NO: 1096; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence of SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090, or SEQ ID NO: 1096; or an amino acid sequence having 85%, 90%, 95%, 96%, 97%, 98%, 99% identity to an amino acid sequence of SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090, or SEQ ID NO: 1096.

In one aspect, the cell of the disclosure (e.g., a cell comprising both a TA CAR and a BCA CAR) comprises a TA CAR molecule comprising an antigen binding domain that binds to a tumor antigen. In one instance, the TA CAR comprises a CAR molecule comprising a CD123 antigen binding domain (e.g., a murine, human or humanized antibody or antibody fragment that specifically binds to mesothelin), a transmembrane domain, and an intracellular signaling domain (e.g., an intracellular signaling domain comprising a costimulatory domain and/or a primary signaling domain).

Exemplary TA CAR molecules that target CD123 are described herein, and are provided in Tables 2, 6 and 9 of WO2016/028896. Other exemplary TA CAR molecules that target CD123 are described in WO/2014/130635 (e.g., Table 1 of WO/2014/130635). Other exemplary TA CAR molecules that target CD123 are described in WO/2014/144622.

In one aspect, the cell of the disclosure (e.g., a cell comprising both a TA CAR and a BCA CAR) comprises a TA CAR molecule comprising an antigen binding domain that binds to a tumor antigen. In one instance, the TA CAR comprises a CAR molecule comprising a CD33 antigen binding domain (e.g., a murine, human or humanized antibody or antibody fragment that specifically binds to mesothelin), a transmembrane domain, and an intracellular signaling domain (e.g., an intracellular signaling domain comprising a costimulatory domain and/or a primary signaling domain). Exemplary TA CAR molecules that target CD33 are described herein, and are provided in WO2016/014576, e.g., in Table 2 of WO2016/014576.

In one aspect, the cell of the disclosure (e.g., a cell comprising both a TA CAR and a BCA CAR) comprises a TA CAR molecule comprising an antigen binding domain that binds to a tumor antigen. In one instance, the TA CAR comprises a CAR molecule comprising a CLL-1 antigen binding domain (e.g., a murine, human or humanized antibody or antibody fragment that specifically binds to mesothelin), a transmembrane domain, and an intracellular signaling domain (e.g., an intracellular signaling domain comprising a costimulatory domain and/or a primary signaling domain). Exemplary TA CAR molecules that target CLL-1 are described herein, and are provided in WO/2016/014535, e.g., in Table 2 of WO2016/014535.

### Natural Killer Cell Receptor (NKR) CARs

In an embodiment, the CAR molecule described herein, e.g., the CAR molecule that targets a tumor antigen (TA CAR) or the CAR molecule that targets a B cell antigen (BCA CAR), comprises one or more components of a natural killer cell receptor (NKR), thereby forming an NKR-CAR. The NKR component can be a transmembrane domain, a hinge domain, or a cytoplasmic domain from any of the following natural killer cell receptors: killer cell immunoglobulin-like receptor (KIR), e.g., KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1; natural cyotoxicity receptor (NCR), e.g., NKp30, NKp44, NKp46; signaling lymphocyte activation molecule (SLAM) family of immune cell receptors, e.g., CD48, CD229, 2B4, CD84, NTB-A, CRACC, BLAME, and CD2F-10; Fc receptor (FcR), e.g., CD16, and CD64; and Ly49 receptors, e.g., LY49A, LY49C. The NKR-CAR molecules described herein may interact with an adaptor molecule or intracellular signaling domain, e.g., DAP12. Exemplary configurations and sequences of CAR molecules comprising NKR components are described in International Publication No. WO2014/145252.

### Split CAR

In some instances, the CAR-expressing cell, e.g., the cell expressing a BCA CAR and a TA CAR, described herein, uses a split CAR. The split CAR approach is described in more detail in publications WO2014/055442 and WO2014/055657.

Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens. In instances the first antigen binding domain recognizes the tumor antigen or B cell antigen described herein, e.g., comprises an antigen binding domain described herein, and the second antigen binding domain recognizes a second antigen, e.g., a second tumor antigen or a second B cell antigen described herein.

### Strategies for Regulating Chimeric Antigen Receptors

There are many ways CAR activities can be regulated. In some embodiments, a regulatable CAR (RCAR) where the CAR activity can be controlled is desirable to optimize the safety and efficacy of a CAR therapy. For example, inducing apoptosis using, e.g., a caspase fused to a dimerization domain (see, e.g., Di et al., N Engl. J. Med. 2011 Nov. 3; 365(18):1673-1683), can be used as a safety switch in the CAR therapy of the instant invention. In another example, CAR-expressing cells can also express an inducible Caspase-9 (iCaspase-9) molecule that, upon administration of a dimerizer drug (e.g., rimiducid (also called AP1903 (Bellicum Pharmaceuticals) or AP20187 (Ariad)) leads to activation of the Caspase-9 and apoptosis of the cells. The iCaspase-9 molecule contains a chemical inducer of dimerization (CID) binding domain that mediates dimerization in the presence of a CID. This results in inducible and selective depletion of CAR-expressing cells. In some cases, the iCaspase-9 molecule is encoded by a nucleic acid molecule separate from the CAR-encoding vector(s). In some cases, the iCaspase-9 molecule is encoded by the same nucleic acid molecule as the CAR-encoding vector. The iCaspase-9 can provide a safety switch to avoid any toxicity of CAR-expressing cells. See, e.g., Song et al. Cancer Gene Ther. 2008; 15(10):667-75; Clinical Trial Id. No. NCT02107963; and Di Stasi et al. N. Engl. J. Med. 2011; 365:1673-83.

Alternative strategies for regulating the CAR therapy of the instant invention include utilizing small molecules or antibodies that deactivate or turn off CAR activity, e.g., by deleting CAR-expressing cells, e.g., by inducing antibody dependent cell-mediated cytotoxicity (ADCC). For example, CAR-expressing cells described herein may also express an antigen that is recognized by molecules capable of inducing cell death, e.g., ADCC or complement-induced cell death. For example, CAR expressing cells described herein may also express a receptor capable of being targeted by an antibody or antibody fragment. Examples of such receptors include EpCAM, VEGFR, integrins (e.g., integrins αvβ3, α4, αI¾β3, α4β7, α5β1, αvβ3, αv), members of the TNF receptor superfamily (e.g., TRAIL-R1 , TRAIL-R2), PDGF Receptor, interferon receptor, folate receptor, GPNMB, ICAM-1 , HLA-DR, CEA, CA-125, MUC1 , TAG-72, IL-6 receptor, 5T4, GD2, GD3, CD2, CD3, CD4, CD5, CD1 1 , CD1 1 a/LFA-1 , CD15, CD18/ITGB2, CD19, CD20, CD22, CD23/lgE Receptor, CD25, CD28, CD30, CD33, CD38, CD40, CD41 , CD44, CD51 , CD52, CD62L, CD74, CD80, CD125, CD147/basigin, CD152/CTLA-4, CD154/CD40L, CD195/CCR5, CD319/SLAMF7, and EGFR, and truncated versions thereof (e.g., versions preserving one or more extracellular epitopes but lacking one or more regions within the cytoplasmic domain).

For example, a CAR-expressing cell described herein may also express a truncated epidermal growth factor receptor (EGFR) which lacks signaling capacity but retains the epitope that is recognized by molecules capable of inducing ADCC, e.g., cetuximab (ERBITUX^{®}), such that administration of cetuximab induces ADCC and subsequent depletion of the CAR-expressing cells (see, e.g., WO2011/056894, and Jonnalagadda et al., Gene Ther. 2013; 20(8)853-860). Another strategy includes expressing a highly compact marker/suicide gene that combines target epitopes from both CD32 and CD20 antigens in the CAR-expressing cells described herein, which binds rituximab, resulting in selective depletion of the CAR-expressing cells, e.g., by ADCC (see, e.g., Philip et al., Blood. 2014; 124(8)1277-1287). Other methods for depleting CAR-expressing cells described herein include administration of CAMPATH, a monoclonal anti-CD52 antibody that selectively binds and targets mature lymphocytes, e.g., CAR-expressing cells, for destruction, e.g., by inducing ADCC. In other embodiments, the CAR-expressing cell can be selectively targeted using a CAR ligand, e.g., an anti-idiotypic antibody. In some embodiments, the anti-idiotypic antibody can cause effector cell activity, e.g, ADCC or ADC activities, thereby reducing the number of CAR-expressing cells. In other embodiments, the CAR ligand, e.g., the anti-idiotypic antibody, can be coupled to an agent that induces cell killing, e.g., a toxin, thereby reducing the number of CAR-expressing cells. Alternatively, the CAR molecules themselves can be configured such that the activity can be regulated, e.g., turned on and off, as described below.

In other embodiments, a CAR-expressing cell described herein may also express a target protein recognized by the T cell depleting agent. In one embodiment, the target protein is CD20 and the T cell depleting agent is an anti-CD20 antibody, e.g., rituximab. In such embodiment, the T cell depleting agent is administered once it is desirable to reduce or eliminate the CAR-expressing cell, e.g., to mitigate the CAR induced toxicity. In other embodiments, the T cell depleting agent is an anti-CD52 antibody, e.g., alemtuzumab.

In other embodiments, a RCAR comprises a set of polypeptides, typically two in the simplest embodiments, in which the components of a standard CAR described herein, e.g., an antigen binding domain and an intracellular signaling domain, are partitioned on separate polypeptides or members. In some embodiments, the set of polypeptides include a dimerization switch that, upon the presence of a dimerization molecule, can couple the polypeptides to one another, e.g., can couple an antigen binding domain to an intracellular signaling domain. Additional description and exemplary configurations of such regulatable CARs are provided herein and in International Publication No. WO 2015/090229, hereby incorporated by reference in its entirety.

### Co-expression of CAR with a Chemokine Receptor

In embodiments, the CAR-expressing cell (e.g., the cell expressing both a TA CAR and a BCA CAR) described herein further comprises a chemokine receptor molecule. Transgenic expression of chemokine receptors CCR2b or CXCR2 in T cells enhances trafficking to CCL2- or CXCL1-secreting solid tumors including melanoma and neuroblastoma (Craddock et al., J Immunother. 2010 Oct; 33(8):780-8 and Kershaw et al.,Hum Gene Ther. 2002 Nov 1; 13(16): 1971-80). Thus, without wishing to be bound by theory, it is believed that chemokine receptors expressed in CAR-expressing cells (e.g., the cell expressing both a TA CAR and a BCA CAR) that recognize chemokines secreted by tumors, e.g., solid tumors, can improve homing of the CAR-expressing cell (e.g., the cell expressing both a TA CAR and a BCA CAR) to the tumor, facilitate the infiltration of the CAR-expressing cell to the tumor, and enhances antitumor efficacy of the CAR-expressing cell (e.g., the cell expressing both a TA CAR and a BCA CAR). The chemokine receptor molecule can comprise a naturally occurring or recombinant chemokine receptor or a chemokine-binding fragment thereof. A chemokine receptor molecule suitable for expression in a CAR-expressing cell (e.g., the cell expressing both a TA CAR and a BCA CAR) described herein include a CXC chemokine receptor (e.g., CXCR1, CXCR2, CXCR3, CXCR4, CXCR5, CXCR6, or CXCR7), a CC chemokine receptor (e.g., CCR1, CCR2, CCR3, CCR4, CCR5, CCR6, CCR7, CCR8, CCR9, CCR10, or CCR11), a CX3C chemokine receptor (e.g., CX3CR1), a XC chemokine receptor (e.g., XCR1), or a chemokine-binding fragment thereof. In one embodiment, the chemokine receptor molecule to be expressed with a CAR described herein is selected based on the chemokine(s) secreted by the tumor. In one embodiment, the CAR-expressing cell (e.g., the cell expressing both a TA CAR and a BCA CAR) described herein further comprises, e.g., expresses, a CCR2b receptor or a CXCR2 receptor. In an embodiment, the CAR described herein (e.g., the cell expressing both a TA CAR and a BCA CAR) and the chemokine receptor molecule are on the same vector or are on two different vectors. In embodiments where the CAR described herein and the chemokine receptor molecule are on the same vector, the CAR e.g., the cell expressing both a TA CAR and a BCA CAR) and the chemokine receptor molecule are each under control of two different promoters or are under the control of the same promoter.

### Nucleic Acid Constructs Encoding a CAR

The present disclosure also provides nucleic acid molecules encoding one or more of the CAR constructs targeting a tumor antigen and/or a B cell antigen described herein. In one aspect, the nucleic acid molecule is provided as a messenger RNA transcript. In one aspect, the nucleic acid molecule is provided as a DNA construct.

Accordingly, in one aspect, the disclosure pertains to a nucleic acid molecule encoding a chimeric antigen receptor (CAR), wherein the CAR comprises an antigen binding domain that binds to a tumor antigen described herein or a B cell antigen described herein, a transmembrane domain (e.g., a transmembrane domain described herein), and an intracellular signaling domain (e.g., an intracellular signaling domain described herein) comprising a stimulatory domain, e.g., a costimulatory signaling domain (e.g., a costimulatory signaling domain described herein) and/or a primary signaling domain (e.g., a primary signaling domain described herein, e.g., a zeta chain described herein). The invention provides a nucleic acid as defined in the claims, which encodes a first CAR and a second CAR, each of which comprises an antigen binding domain, a transmembrane domain, and an intracellular signaling domain.

In one embodiment, the transmembrane domain is transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp , NKG2D, and NKG2C.

In one embodiment, the transmembrane domain comprises a sequence of SEQ ID NO: 12, or a sequence with 95-99% identity thereof. In one embodiment, the antigen binding domain is connected to the transmembrane domain by a hinge region, e.g., a hinge described herein. In one embodiment, the hinge region comprises SEQ ID NO:4 or SEQ ID NO:6 or SEQ ID NO:8 or SEQ ID NO:10, or a sequence with 95-99% identity thereof. In one embodiment, the isolated nucleic acid molecule further comprises a sequence encoding a costimulatory domain. In one embodiment, the costimulatory domain is a functional signaling domain of a protein selected from the group consisting of OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKG2D, and NKG2C. In one embodiment, the costimulatory domain comprises a sequence of SEQ ID NO:16, or a sequence with 95-99% identity thereof. In one embodiment, the intracellular signaling domain comprises a functional signaling domain of 4-1BB and a functional signaling domain of CD3 zeta. In one embodiment, the intracellular signaling domain comprises the sequence of SEQ ID NO: 14 or SEQ ID NO: 16, 42, or 44, or a sequence with 95-99% identity thereof, and the sequence of SEQ ID NO: 18 or SEQ ID NO:20, or a sequence with 95-99% identity thereof, wherein the sequences comprising the intracellular signaling domain are expressed in the same frame and as a single polypeptide chain.

In another aspect, the disclosure pertains to an isolated nucleic acid molecule encoding a CAR construct comprising a leader sequence of SEQ ID NO: 2, a scFv domain as described herein, a hinge region of SEQ ID NO:4 or SEQ ID NO:6 or SEQ ID NO:8 or SEQ ID NO:10 (or a sequence with 95-99% identity thereof), a transmembrane domain having a sequence of SEQ ID NO: 12 (or a sequence with 95-99% identity thereof), a 4-1BB costimulatory domain having a sequence of SEQ ID NO:14, a CD27 costimulatory domain having a sequence of SEQ ID NO:16 (or a sequence with 95-99% identity thereof), a ICOS costimulatory domain having a sequence of SEQ ID NO: 42 (or a sequence with 95-99% identity thereof) or a CD28 costimulatory domain having a sequence of SEQ ID NO:44, and a CD3 zeta stimulatory domain having a sequence of SEQ ID NO: 18 or SEQ ID NO:20 (or a sequence with 95-99% identity thereof).

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present disclosure also provides vectors in which a nucleic acid of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

In another instance, the vector comprising the nucleic acid encoding the desired CAR disclosed herein is an adenoviral vector (A5/35). In another instance, the expression of nucleic acids encoding CARs can be accomplished using of transposons such as sleeping beauty, crisper, CAS9, and zinc finger nucleases. See below June et al. 2009Nature Reviews Immunology 9.10: 704-716 .

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The expression constructs of the present disclosure may also be used for nucleic acid immunization and gene therapy, using standard gene delivery protocols. Methods for gene delivery are known in the art. See, e.g., U.S. Pat. Nos. 5,399,346, 5,580,859, 5,589,466 .

In another instance, the invention provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some instances, adenovirus vectors are used. A number of adenovirus vectors are known in the art. In one instance, lentivirus vectors are used.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. Exemplary promoters include the CMV IE gene, EF-1α, ubiquitin C, or phosphoglycerokinase (PGK) promoters.

An example of a promoter that is capable of expressing a CAR encoding nucleic acid molecule in a mammalian T cell is the FF1a promoter. The native EF1a promoter drives expression of the alpha subunit of the elongation factor-1 complex, which is responsible for the enzymatic delivery of aminoacyl tRN-As to the ribosome. The EF1a promoter has been extensively used in mammalian expression plasmids and has been shown to be effective in driving CAR expression from nucleic acid molecules cloned into a lentiviral vector. See, e.g., Milone et al., Mol. Ther. 17(8): 1453-1464 (2009). In one aspect, the EF1a promoter comprises the sequence provided as SEQ ID NO:1.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α promoter, the hemoglobin promoter, and the creatine kinase promoter. Further, the disclosure should not be limited to the use of constitutive promoters. Inducible promoters are also contemplated . The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Another example of a promoter is the phosphoglycerate kinase (PGK) promoter. In embodiments, a truncated PGK promoter (e.g., a PGK promoter with one or more, e.g., 1, 2, 5, 10, 100, 200, 300, or 400, nucleotide deletions when compared to the wild-type PGK promoter sequence) may be desired. The nucleotide sequences of exemplary PGK promoters are provided below.
WT PGK Promoter 
ACCCCTCTCTCCAGCCACTAAGCCAGTTGCTCCCTCGGCTGACGGCTGCACGCGAG
GCCTCCGAACGTCTTACGCCTTGTGGCGCGCCCGTCCTTGTCCCGGGTGTGATGGC
GGGGTGTGGGGCGGAGGGCGTGGCGGGGAAGGGCCGGCGACGAGAGCCGCGCGG
GACGACTCGTCGGCGATAACCGGTGTCGGGTAGCGCCAGCCGCGCGACGGTAACG
AGGGACCGCGACAGGCAGACGCTCCCATGATCACTCTGCACGCCGAAGGCAAATA
GTGCAGGCCGTGCGGCGCTTGGCGTTCCTTGGAAGGGCTGAATCCCCGCCTCGTCC
TTCGCAGCGGCCCCCCGGGTGTTCCCATCGCCGCTTCTAGGCCCACTGCGACGCTT
GCCTGCACTTCTTACACGCTCTGGGTCCCAGCCGCGGCGACGCAAAGGGCCTTGGT
GCGGGTCTCGTCGGCGCAGGGACGCGTTTGGGTCCCGACGGAACCTTTTCCGCGTT
GGGGTTGGGGCACCATAAGCT (SEQ ID NO: 101)
Exemplary truncated PGK Promoters:
   PGK100: 
ACCCCTCTCTCCAGCCACTAAGCCAGTTGCTCCCTCGGCTGACGGCTGCACGCGAG
GCCTCCGAACGTCTTACGCCTTGTGGCGCGCCCGTCCTTGTCCCGGGTGTGATGGC
GGGGTG (SEQ ID NO: 102)
   PGK200: 
ACCCCTCTCTCCAGCCACTAAGCCAGTTGCTCCCTCGGCTGACGGCTGCACGCGAG
GCCTCCGAACGTCTTACGCCTTGTGGCGCGCCCGTCCTTGTCCCGGGTGTGATGGC
GGGGTGTGGGGCGGAGGGCGTGGCGGGGAAGGGCCGGCGACGAGAGCCGCGCGG
GACGACTCGTCGGCGATAACCGGTGTCGGGTAGCGCCAGCCGCGCGACGGTAACG
(SEQ ID NO: 103)
   PGK300: 
ACCCCTCTCTCCAGCCACTAAGCCAGTTGCTCCCTCGGCTGACGGCTGCACGCGAG
GCCTCCGAACGTCTTACGCCTTGTGGCGCGCCCGTCCTTGTCCCGGGTGTGATGGC
GGGGTGTGGGGCGGAGGGCGTGGCGGGGAAGGGCCGGCGACGAGAGCCGCGCGG
GACGACTCGTCGGCGATAACCGGTGTCGGGTAGCGCCAGCCGCGCGACGGTAACG
 
AGGGACCGCGACAGGCAGACGCTCCCATGATCACTCTGCACGCCGAAGGCAAATA
GTGCAGGCCGTGCGGCGCTTGGCGTTCCTTGGAAGGGCTGAATCCCCG (SEQ ID
NO: 104)
   PGK400:

ACCCCTCTCTCCAGCCACTAAGCCAGTTGCTCCCTCGGCTGACGGCTGCACGCGAG
GCCTCCGAACGTCTTACGCCTTGTGGCGCGCCCGTCCTTGTCCCGGGTGTGATGGC
GGGGTGTGGGGCGGAGGGCGTGGCGGGGAAGGGCCGGCGACGAGAGCCGCGCGG
GACGACTCGTCGGCGATAACCGGTGTCGGGTAGCGCCAGCCGCGCGACGGTAACG
AGGGACCGCGACAGGCAGACGCTCCCATGATCACTCTGCACGCCGAAGGCAAATA
GTGCAGGCCGTGCGGCGCTTGGCGTTCCTTGGAAGGGCTGAATCCCCGCCTCGTCC
TTCGCAGCGGCCCCCCGGGTGTTCCCATCGCCGCTTCTAGGCCCACTGCGACGCTT
GCCTGCACTTCTTACACGCTCTGGGTCCCAGCCG (SEQ ID NO: 105)

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In some instances, a vector comprising a nuclei acid sequence encoding a CAR molecules described herein, e.g., a TA CAR and/or a BCA CAR, can further comprises a second nucleic acid sequence encoding a polypeptide, e.g., an agent that increases the activity of the CAR molecule. In some instances a single nucleic acid molecule, or vector comprising said nucleic acid molecule, encodes both the TA CAR, described herein, and the BCA CAR, described herein. In some instances, the nucleic acid encoding the TA CAR is under separate regulatory control (e.g., by a promoter described herein) from the nucleic acid enciocing the BCA CAR (e.g., by a promoter described herein). In other instances, the two or more nucleic acid sequences are encoded by a single nucleic molecule in the same frame and as a single polypeptide chain. In this aspect, the two or more CARs (e.g., the TA CAR and the BCA CAR), can, e.g., be separated by one or more peptide cleavage sites. (e.g., an auto-cleavage site or a substrate for an intracellular protease). Examples of peptide cleavage sites include the following, wherein the GSG residues are optional:

| | |
|---|---|
| T2A: | (GSG) E G R G S L L T C G D V E E N P G P (SEQ ID NO: 106) |
| P2A: | (GSG) A T N F S L L K Q A G D V E E N P G P (SEQ ID NO: 107) |
| E2A: | (GSG) Q C T N Y A L L K L A G D V E S N P G P (SEQ ID NO: 108) |
| F2A: | (GSG) V K Q T L N F D L L K L A G D V E S N P G P (SEQ 10 NO: 109) |

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A preferred method for the introduction of a polynucleotide into a host cell is calcium phosphate transfection or electroporation.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about - 20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present disclosure, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure.

The present disclosure further provides a vector comprising a CAR encoding nucleic acid molecule. In one instance, the vector comprises a TA CAR encoding nucleic acid molecule, e.g., as described herein. In one instance, the vector comprises a BCA CAR encoding nucleic acid molecule, e.g., as described herein. In one instance, the vector comprises a BCA CAR encoding nucleic acid molecule and a TA CAR encoding nucleic acid molecule. In one aspect, the one or more CAR vectors (e.g., the vector comprising the TA CAR encoding nucleic acid molecule and the vector comprising the BCA CAR encoding nucleic acid molecule, or the vector comprising the TA CAR encoding nucleic acid and the BCA CAR encoding nucleic acid) can be directly transduced into a cell, *e.g.,* a T cell or a NK cell. In one aspect, the vector is a cloning or expression vector, e.g., a vector including, but not limited to, one or more plasmids (e.g., expression plasmids, cloning vectors, minicircles, minivectors, double minute chromosomes), retroviral and lentiviral vector constructs. In one aspect, the vector is capable of expressing the CAR construct in mammalian immune effector cells (e.g., T cells, NK cells).

In one embodiment, where stable expression of a TA CAR and/or a BCA CAR is desired, a vector comprising a TA CAR- and/or BCA CAR-encoding nucleic acid molecule is transduced into an immune effector cell. For example, immune effector cells with stable expression of a TA CAR and a BCA CAR can be generated using lentiviral vectors. Cells that exhibit stable expression of a TA CAR and a BCA CAR express the TA CAR and BCA CAR for at least 1 week, 2 weeks, 3 weeks, 4 weeks, 5 weeks, 6 weeks, 7 weeks, 8 weeks, 3 months, 6 months, 9 months, or 12 months after transduction.

In one embodiment, where transient expression of a TA CAR and/or a BCA CAR is desired, a TA CAR- and/or BCA CAR-encoding nucleic acid molecule is transfected into an immune effector cell. The TA CAR- and/or BCA CAR-encoding nucleic acid molecule may be a vector comprising a TA CAR- and/or BCA-CAR encoding nucleic acid molecule, or an in vitro transcribed RNA encoding TA CAR and/or BCA CAR. In vitro transcribed RNA CARs and methods for transfection into immune effector cells are further described below. Cells that exhibit transient expression of a TA CAR and/or a BCA CAR express the TA CAR and/or BCA CAR for 4, 5, 6, 7, 8, 9, 10, 11, 12, 13, 14, 15 days after transfection.

### RNA Transfection

Disclosed herein are methods for producing an in vitro transcribed RNA CAR, e.g., an in vitro transcribed RNA TA CAR and/or an in vitro transcribed RNA BCA CAR. The present disclosure also includes a CAR encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:32). RNA so produced can efficiently transfect different kinds of cells. In one aspect, the template includes sequences for the CAR.

In one aspect, a CAR of the present disclosure, e.g., a TA CAR and/or a BCA CAR, is encoded by a messenger RNA (mRNA). In one aspect, the mRNA encoding a TA CAR described herein and/or a BCA CAR described herein is introduced into a T cell or a NK cell for production of a cell that expresses both a TA CAR and a BCA CAR.

In one embodiment, the *in vitro* transcribed RNA CAR can be introduced to a cell as a form of transient transfection. The RNA is produced by *in vitro* transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired template for *in vitro* transcription is a CAR described herein. For example, the template for the RNA CAR comprises an extracellular region comprising a single chain variable domain of an antibody to a tumor antigen or B cell antigen described herein; a hinge region (e.g., a hinge region described herein), a transmembrane domain (e.g., a transmembrane domain described herein such as a transmembrane domain of CD8a); and a cytoplasmic region that includes an intracellular signaling domain, e.g., an intracellular signaling domain described herein, e.g., comprising the signaling domain of CD3-zeta and the signaling domain of 4-1BB.

In one embodiment, the DNA to be used for PCR contains an open reading frame. The DNA can be from a naturally occurring DNA sequence from the genome of an organism. In one embodiment, the nucleic acid can include some or all of the 5' and/or 3' untranslated regions (UTRs). The nucleic acid can include exons and introns. In one embodiment, the DNA to be used for PCR is a human nucleic acid sequence. In another embodiment, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one embodiment, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of poly A/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (SEQ ID NO: 35) (size can be 50-5000 T (SEQ ID NO: 265)), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines (SEQ ID NO: 82).

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides (SEQ ID NO: 38) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### Non-viral delivery methods

In some aspects, non-viral methods can be used to deliver a nucleic acid encoding a CAR described herein, e.g., a TA CAR and/or a BCA CAR, into a cell or tissue or a subject. In some embodiments, the non-viral method includes the use of a transposon (also called a transposable element). In some embodiments, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition.

Exemplary methods of nucleic acid delivery using a transposon include a Sleeping Beauty transposon system (SBTS) and a piggyBac (PB) transposon system. See, e.g., Aronovich et al. Hum. Mol. Genet. 20.R1(2011):R14-20; Singh et al. Cancer Res. 15(2008):2961-2971; Huang et al. Mol. Ther. 16(2008):580-589; Grabundzija et al. Mol. Ther. 18(2010):1200-1209; Kebriaei et al. Blood. 122.21(2013):166; Williams. Molecular Therapy 16.9(2008):1515-16; Bell et al. Nat. Protoc. 2.12(2007):3153-65; and Ding et al. Cell. 122.3(2005):473-83 .

The SBTS includes two components: 1) a transposon containing a transgene and 2) a source of transposase enzyme. The transposase can transpose the transposon from a carrier plasmid (or other donor DNA) to a target DNA, such as a host cell chromosome/genome. For example, the transposase binds to the carrier plasmid/donor DNA, cuts the transposon (including transgene(s)) out of the plasmid, and inserts it into the genome of the host cell. See, e.g., Aronovich et al. *supra.*

Exemplary transposons include a pT2-based transposon. See, e.g., Grabundzija et al. Nucleic Acids Res. 41.3(2013):1829-47; and Singh et al. Cancer Res. 68.8(2008): 2961-2971.

Exemplary transposases include a Tc1/mariner-type transposase, e.g., the SB10 transposase or the SB11 transposase (a hyperactive transposase which can be expressed, e.g., from a cytomegalovirus promoter). See, e.g., Aronovich et al.; Kebriaei et al.; and Grabundzija et al.

Use of the SBTS permits efficient integration and expression of a transgene, e.g., a nucleic acid encoding a CAR described herein. Provided herein are methods of generating a cell, e.g., T cell or NK cell, that stably expresses a CAR described herein, e.g., using a transposon system such as SBTS.

In accordance with methods described herein, in some embodiments, one or more nucleic acids, e.g., plasmids, containing the SBTS components are delivered to a cell (e.g., T or NK cell). For example, the nucleic acid(s) are delivered by standard methods of nucleic acid (e.g., plasmid DNA) delivery, e.g., methods described herein, e.g., electroporation, transfection, or lipofection. In some embodiments, the nucleic acid contains a transposon comprising a transgene, e.g., a nucleic acid encoding a CAR described herein. In some embodiments, the nucleic acid contains a transposon comprising a transgene (e.g., a nucleic acid encoding a CAR described herein) as well as a nucleic acid sequence encoding a transposase enzyme. In other embodiments, a system with two nucleic acids is provided, e.g., a dual-plasmid system, e.g., where a first plasmid contains a transposon comprising a transgene, and a second plasmid contains a nucleic acid sequence encoding a transposase enzyme. For example, the first and the second nucleic acids are co-delivered into a host cell.

In some embodiments, cells, e.g., T or NK cells, are generated that express a TA CAR and BCA CAR described herein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease re-engineered homing endonucleases).

In some embodiments, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### Sources of Cells

Prior to expansion and genetic modification, e.g., to express both a TA CAR and a BCA CAR described herein, a source of cells, e.g., T cell or NK cells, can be obtained from a subject. The term "subject" is intended to include living organisms in which an immune response can be elicited (e.g., mammals). Examples of subjects include humans, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain aspects of the present disclosure, any number of T cell lines available in the art, may be used. In certain aspects of the present disclosure, T cells can be obtained from a unit of blood collected from a subject using any number of techniques known to the skilled artisan, such as Ficoll^{™} separation. In one preferred aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and to place the cells in an appropriate buffer or media for subsequent processing steps. In one aspect , the cells are washed with phosphate buffered saline (PBS). In an alternative aspect, the wash solution lacks calcium and may lack magnesium or may lack many if not all divalent cations. Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31.

In one aspect, T cells are isolated from peripheral blood lymphocytes by lysing the red blood cells and depleting the monocytes, for example, by centrifugation through a PERCOLLTM gradient or by counterflow centrifugal elutriation. A specific subpopulation of T cells, such as CD3+, CD28+, CD4+, CD8+, CD45RA+, and CD45RO+T cells, can be further isolated by positive or negative selection techniques. For example, in one aspect, T cells are isolated by incubation with anti-CD3/anti-CD28 (e.g., 3x28)-conjugated beads, such as DYNABEADS^{®} M-450 CD3/CD28 T, for a time period sufficient for positive selection of the desired T cells. In one aspect, the time period is about 30 minutes. In a further aspect, the time period ranges from 30 minutes to 36 hours or longer and all integer values there between. In a further aspect, the time period is at least 1, 2, 3, 4, 5, or 6 hours. In yet another preferred aspect, the time period is 10 to 24 hours. In one aspect, the incubation time period is 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points. The skilled artisan would recognize that multiple rounds of selection can also be used in the context of this invention. In certain aspects, it may be desirable to perform the selection procedure and use the "unselected" cells in the activation and expansion process. "Unselected" cells can also be subjected to further rounds of selection.

Enrichment of a T cell population by negative selection can be accomplished with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CD11b, CD16, HLA-DR, and CD8. In certain aspects, it may be desirable to enrich for or positively select for regulatory T cells which typically express CD4+, CD25+, CD62Lhi, GITR+, and FoxP3+. Alternatively, in certain aspects, T regulatory cells are depleted by anti-C25 conjugated beads or other similar method of selection.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

In one embodiment, T regulatory cells, e.g., CD25+ T cells, are removed from the population using an anti-CD25 antibody, or fragment thereof, or a CD25-binding ligand, IL-2. In one embodiment, the anti-CD25 antibody, or fragment thereof, or CD25-binding ligand is conjugated to a substrate, e.g., a bead, or is otherwise coated on a substrate, e.g., a bead. In one embodiment, the anti-CD25 antibody, or fragment thereof, is conjugated to a substrate as described herein.

In one embodiment, the T regulatory cells, e.g., CD25+ T cells, are removed from the population using CD25 depletion reagent from Miltenyi^{™}. In one embodiment, the ratio of cells to CD25 depletion reagent is 1e7 cells to 20 uL, or 1e7 cells to 15 uL, or 1e7 cells to 10 uL, or 1e7 cells to 5 uL, or 1e7 cells to 2.5 uL, or 1e7 cells to 1.25 uL. In one embodiment, e.g., for T regulatory cells, e.g., CD25+ depletion, greater than 500 million cells/ml is used. In a further aspect, a concentration of cells of 600, 700, 800, or 900 million cells/ml is used.

In one embodiment, the population of immune effector cells to be depleted includes about 6 x 10⁹ CD25+ T cells. In other aspects, the population of immune effector cells to be depleted include about 1 x 10⁹ to 1x 10¹⁰ CD25+ T cell, and any integer value in between. In one embodiment, the resulting population T regulatory depleted cells has 2 x 10⁹ T regulatory cells, e.g., CD25+ cells, or less (e.g., 1 x 10⁹, 5 x 10⁸, 1 x 10⁸, 5 x 10⁷, 1 x 10⁷, or less CD25+ cells).

In one embodiment, the T regulatory cells, e.g., CD25+ cells, are removed from the population using the CliniMAC system with a depletion tubing set, such as, e.g., tubing 162-01. In one embodiment, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, and combinations thereof.

In some embodiments, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an embodiment, a subject is pre-treated with one or more therapies that reduce T_{REG} cells prior to collection of cells for CAR-expressing cell product manufacturing, thereby reducing the risk of subject relapse to CAR-expressing cell treatment. In an embodiment, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product.

In an embodiment, a subject is pre-treated with cyclophosphamide prior to collection of cells for CAR-expressing cell product manufacturing, thereby reducing the risk of subject relapse to CAR-expressing cell treatment. In an embodiment, a subject is pre-treated with an anti-GITR antibody prior to collection of cells for CAR-expressing cell product manufacturing, thereby reducing the risk of subject relapse to CAR-expressing cell treatment.

In one embodiment, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one embodiment, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one embodiment, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other embodiments, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include B7-H1, B7-1, CD160, P1H, 2B4, PD1, TIM3, CEACAM (e.g., CEACAM-1, CEACAM-3 and/or CEACAM-5), LAG3, TIGIT, CTLA-4, BTLA and LAIR1. In one embodiment, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other embodiments, the removal of T regulatory cells, e.g., CD25+ cells, and the removal of the check point inhibitor expressing cells is sequential, and can occur, e.g., in either order.

In one embodiment, a T cell population can be selected that expresses one or more of IFN-γ, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 2 billion cells/ml is used. In one aspect, a concentration of 1 billion cells/ml is used. In a further aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 X 10e6/ml. In other aspects, the concentration used can be from about 1 X 10⁵/ml to 1 X 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present disclosure.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in T cell therapy for any number of diseases or conditions that would benefit from T cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present disclosure, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present disclosure to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an embodiment, the NK cells are obtained from the subject. In another embodiment, the NK cells are an NK cell line, e.g., NK-92 cell line (Conkwest).

### Allogeneic CAR Immune Effector Cells

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class I and/or HLA class II, is downregulated.

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not expresses or expresses at low levels an inhibitory molecule, e.g. by any mehod described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VICN1), HVEM (TNFRSF14 or CD270), KIR, A2aR, MHC class I, MHC class II, GAL9, adenosine, and TGF beta), in a cell, e.g., T cell.
Expression systems for siRNA and shRNAs, and exemplary shRN-As, are described, e.g., in paragraphs 649 and 650 of International Publication WO2015/142675, filed March 13, 2015.

### CRISPR to inhibit TCR or HLA

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein.

A "CRISPR/Cas" system refers to a system derived from CRISPR and Cas which can be used to silence or mutate a TCR and/or HLA gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

The CRISPR/Cas system, and uses thereof, are described, e.g., in paragraphs 651-658 of International Publication WO2015/142675, filed March 13, 2015.

### TALEN to inhibit TCR and/or HLA

TALEN" or "TALEN to HLA and/or TCR" or "TALEN to inhibit HLA and/or TCR" refers to a transcription activator-like effector nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

TALENs, and uses thereof, are described, e.g., in paragraphs 659-665 of International Publication WO2015/142675, filed March 13, 2015.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAMS-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

ZFNs, and uses thereof, are described, e.g., in paragraphs 666-671 of International Publication WO2015/142675, filed March 13, 2015 .

### Telomerase expression

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an embodiment, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

In one aspect, the disclosure features a method of making a population of immune effector cells (e.g., T cells, NK cells). In an embodiment, the method comprises: providing a population of immune effector cells (e.g., T cells or NK cells), contacting the population of immune effector cells with a nucleic acid encoding a CAR; and contacting the population of immune effector cells with a nucleic acid encoding a telomerase subunit, e.g., hTERT, under conditions that allow for CAR and telomerase expression.

In an embodiment, the nucleic acid encoding the telomerase subunit is DNA. In an embodiment, the nucleic acid encoding the telomerase subunit comprises a promoter capable of driving expression of the telomerase subunit.

In an embodiment, hTERT has the amino acid sequence of GenBank Protein ID AAC51724.1 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795) as follows:





















 (SEQ ID NO: 110)

In an embodiment, the hTERT has a sequence at least 80%, 85%, 90%, 95%, 96^, 97%, 98%, or 99% identical to the sequence of SEQ ID NO: 110. In an embodiment, the hTERT has a sequence of SEQ ID NO: 110. In an embodiment, the hTERT comprises a deletion (e.g., of no more than 5, 10, 15, 20, or 30 amino acids) at the N-terminus, the C-terminus, or both. In an embodiment, the hTERT comprises a transgenic amino acid sequence (e.g., of no more than 5, 10, 15, 20, or 30 amino acids) at the N-terminus, the C-terminus, or both.

In an embodiment, the hTERT is encoded by the nucleic acid sequence of GenBank Accession No. AF018167 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795) as follows:

1 caggcagcgt ggtcctgctg cgcacgtggg aagccctggc cccggccacc cccgcgatgc
61 cgcgcgctcc ccgctgccga gccgtgcgct ccctgctgcg cagccactac cgcgaggtgc
121 tgccgctggc cacgttcgtg cggcgcctgg ggccccaggg ctggcggctg gtgcagcgcg
181 gggacccggc ggctttccgc gcgctggtgg cccagtgcct ggtgtgcgtg ccctgggacg

241 cacggccgcc ccccgccgcc ccctccttcc gccaggtgtc ctgcctgaag gagctggtgg
301 cccgagtgct gcagaggctg tgcgagcgcg gcgcgaagaa cgtgctggcc ttcggcttcg
361 cgctgctgga cggggcccgc gggggccccc ccgaggcctt caccaccagc gtgcgcagct
421 acctgcccaa cacggtgacc gacgcactgc gggggagcgg ggcgtggggg ctgctgttgc
481 gccgcgtggg cgacgacgtg ctggttcacc tgctggcacg ctgcgcgctc tttgtgctgg
541 tggctcccag ctgcgcctac caggtgtgcg ggccgccgct gtaccagctc ggcgctgcca
601 ctcaggcccg gcccccgcca cacgctagtg gaccccgaag gcgtctggga tgcgaacggg
661 cctggaacca tagcgtcagg gaggccgggg tccccctggg cctgccagcc ccgggtgcga
721 ggaggcgcgg gggcagtgcc agccgaagtc tgccgttgcc caagaggccc aggcgtggcg
781 ctgcccctga gccggagcgg acgcccgttg ggcaggggtc ctgggcccac ccgggcagga
841 cgcgtggacc gagtgaccgt ggtttctgtg tggtgtcacc tgccagaccc gccgaagaag
901 ccacctcttt ggagggtgcg ctctctggca cgcgccactc ccacccatcc gtgggccgcc
961 agcaccacgc gggcccccca tccacatcgc ggccaccacg tccctgggac acgccttgtc
1021 ccccggtgta cgccgagacc aagcacttcc tctactcctc aggcgacaag gagcagctgc
1081 ggccctcctt cctactcagc tctctgaggc ccagcctgac tggcgctcgg aggctcgtgg
1141 agaccatctt tctgggttcc aggccctgga tgccagggac tccccgcagg ttgccccgcc
1201 tgccccagcg ctactggcaa atgcggcccc tgtttctgga gctgcttggg aaccacgcgc
1261 agtgccccta cggggtgctc ctcaagacgc actgcccgct gcgagctgcg gtcaccccag
1321 cagccggtgt ctgtgcccgg gagaagcccc agggctctgt ggcggccccc gaggaggagg
1381 acacagaccc ccgtcgcctg gtgcagctgc tccgccagca cagcagcccc tggcaggtgt
1441 acggcttcgt gcgggcctgc ctgcgccggc tggtgccccc aggcctctgg ggctccaggc
1501 acaacgaacg ccgcttcctc aggaacacca agaagttcat ctccctgggg aagcatgcca
1561 agctctcgct gcaggagctg acgtggaaga tgagcgtgcg gggctgcgct tggctgcgca
1621 ggagcccagg ggttggctgt gttccggccg cagagcaccg tctgcgtgag gagatcctgg
1681 ccaagttcct gcactggctg atgagtgtgt acgtcgtcga gctgctcagg tctttctttt
1741 atgtcacgga gaccacgttt caaaagaaca ggctcttttt ctaccggaag agtgtctgga
1801 gcaagttgca aagcattgga atcagacagc acttgaagag ggtgcagctg cgggagctgt
1861 cggaagcaga ggtcaggcag catcgggaag ccaggcccgc cctgctgacg tccagactcc
1921 gcttcatccc caagcctgac gggctgcggc cgattgtgaa catggactac gtcgtgggag
1981 ccagaacgtt ccgcagagaa aagagggccg agcgtctcac ctcgagggtg aaggcactgt
2041 tcagcgtgct caactacgag cgggcgcggc gccccggcct cctgggcgcc tctgtgctgg
2101 gcctggacga tatccacagg gcctggcgca ccttcgtgct gcgtgtgcgg gcccaggacc

2161 cgccgcctga gctgtacttt gtcaaggtgg atgtgacggg cgcgtacgac accatccccc
2221 aggacaggct cacggaggtc atcgccagca tcatcaaacc ccagaacacg tactgcgtgc
2281 gtcggtatgc cgtggtccag aaggccgccc atgggcacgt ccgcaaggcc ttcaagagcc
2341 acgtctctac cttgacagac ctccagccgt acatgcgaca gttcgtggct cacctgcagg
2401 agaccagccc gctgagggat gccgtcgtca tcgagcagag ctcctccctg aatgaggcca
2461 gcagtggcct cttcgacgtc ttcctacgct tcatgtgcca ccacgccgtg cgcatcaggg
2521 gcaagtccta cgtccagtgc caggggatcc cgcagggctc catcctctcc acgctgctct
2581 gcagcctgtg ctacggcgac atggagaaca agctgtttgc ggggattcgg cgggacgggc
2641 tgctcctgcg tttggtggat gatttcttgt tggtgacacc tcacctcacc cacgcgaaaa
2701 ccttcctcag gaccctggtc cgaggtgtcc ctgagtatgg ctgcgtggtg aacttgcgga
2761 agacagtggt gaacttccct gtagaagacg aggccctggg tggcacggct tttgttcaga
2821 tgccggccca cggcctattc ccctggtgcg gcctgctgct ggatacccgg accctggagg
2881 tgcagagcga ctactccagc tatgcccgga cctccatcag agccagtctc accttcaacc
2941 gcggcttcaa ggctgggagg aacatgcgtc gcaaactctt tggggtcttg cggctgaagt
3001 gtcacagcct gtttctggat ttgcaggtga acagcctcca gacggtgtgc accaacatct
3061 acaagatcct cctgctgcag gcgtacaggt ttcacgcatg tgtgctgcag ctcccatttc
3121 atcagcaagt ttggaagaac cccacatttt tcctgcgcgt catctctgac acggcctccc
3181 tctgctactc catcctgaaa gccaagaacg cagggatgtc gctgggggcc aagggcgccg
3241 ccggccctct gccctccgag gccgtgcagt ggctgtgcca ccaagcattc ctgctcaagc
3301 tgactcgaca ccgtgtcacc tacgtgccac tcctggggtc actcaggaca gcccagacgc
3361 agctgagtcg gaagctcccg gggacgacgc tgactgccct ggaggccgca gccaacccgg
3421 cactgccctc agacttcaag accatcctgg actgatggcc acccgcccac agccaggccg
3481 agagcagaca ccagcagccc tgtcacgccg ggctctacgt cccagggagg gaggggcggc
3541 ccacacccag gcccgcaccg ctgggagtct gaggcctgag tgagtgtttg gccgaggcct
3601 gcatgtccgg ctgaaggctg agtgtccggc tgaggcctga gcgagtgtcc agccaagggc
3661 tgagtgtcca gcacacctgc cgtcttcact tccccacagg ctggcgctcg gctccacccc
3721 agggccagct tttcctcacc aggagcccgg cttccactcc ccacatagga atagtccatc
3781 cccagattcg ccattgttca cccctcgccc tgccctcctt tgccttccac ccccaccatc
3841 caggtggaga ccctgagaag gaccctggga gctctgggaa tttggagtga ccaaaggtgt
3901 gccctgtaca caggcgagga ccctgcacct ggatgggggt ccctgtgggt caaattgggg
3961 ggaggtgctg tgggagtaaa atactgaata tatgagtttt tcagttttga aaaaaaaaaa
4021 aaaaaaa (SEQ ID NO: 111)

In an embodiment, the hTERT is encoded by a nucleic acid having a sequence at least 80%, 85%, 90%, 95%, 96, 97%, 98%, or 99% identical to the sequence of SEQ ID NO: 111. In an embodiment, the hTERT is encoded by a nucleic acid of SEQ ID NO: 111.

### Activation and Expansion of Immune Effector Cells (e.g., T Cells)

Immune effector cells, such as T cells, may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells, e.g., T cells may be expanded by contact with a surface having attached thereto an agent that stimulates a CD3/TCR complex associated signal and a ligand that stimulates a costimulatory molecule on the surface of the immune effector cells, e.g., T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody. Examples of an anti-CD28 antibody include 9.3, B-T3, XR-CD28 (Diaclone, Besangon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then crosslinked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present disclosure.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present disclosure, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3: CD28 antibody bound to the beads ranges from 100:1 to 1:100 and all integer values there between. In one aspect of the present disclosure, more anti-CD28 antibody is bound to the particles than anti-CD3 antibody, i.e., the ratio of CD3:CD28 is less than one. In certain aspects of the disclosure, the ratio of anti CD28 antibody to anti CD3 antibody bound to the beads is greater than 2:1. In one particular aspect, a 1:100 CD3: CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one preferred aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a preferred particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present disclosure. In particular, ratios will vary depending on particle size and on cell size and type. In one aspect, the most typical ratios for use are in the neighborhood of 1:1, 2:1 and 3:1 on the first day.

In further aspects of the present disclosure, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present disclosure. In certain aspects, it may be desirable to significantly decrease the volume in which particles and cells are mixed together (i.e., increase the concentration of cells), to ensure maximum contact of cells and particles. For example, in one aspect, a concentration of about 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one embodiment, cells transduced with a nucleic acid encoding a CAR, e.g., a CAR described herein, are expanded, e.g., by a method described herein. In one embodiment, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one embodiment, the cells are expanded for a period of 4 to 9 days. In one embodiment, the cells are expanded for a period of 8 days or less, e.g., 7, 6 or 5 days. In one embodiment, the cells, e.g., a CAR-expressing cell described herein, are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one embodiment, the cells, e.g., a CAR-expressing cell described herein, expanded for 5 days show at least a one, two, three or four fold increase in cells doublings upon antigen stimulation as compared to the same cells expanded in culture for 9 days under the same culture conditions. In one embodiment, the cells, e.g., the cells expressing a CAR described herein, are expanded in culture for 5 days, and the resulting cells exhibit higher proinflammatory cytokine production, e.g., IFN-y and/or GM-CSF levels, as compared to the same cells expanded in culture for 9 days under the same culture conditions. In one embodiment, the cells, e.g., a CAR-expressing cell described herein, expanded for 5 days show at least a one, two, three, four, five, tenfold or more increase in pg/ml of proinflammatory cytokine production, e.g., IFN-γ and/or GM-CSF levels, as compared to the same cells expanded in culture for 9 days under the same culture conditions.

In one aspect of the present disclosure, the mixture may be cultured for several hours (about 3 hours) to about 14 days or any hourly integer value in between. In one aspect, the mixture may be cultured for 21 days. In one aspect the beads and the T cells are cultured together for about eight days. In one aspect, the beads and T cells are cultured together for 2-3 days. Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one embodiment, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one embodiment, the cells are expanded in the presence IL-15 and/or IL-7 (e.g., IL-15 and IL-7).

In embodiments, methods described herein, e.g., CAR-expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In embodiments, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some embodiments a CAR-expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15, during the manufacturing of the CAR-expressing cell, e.g., ex vivo. In embodiments, a CAR-expressing cell described herein is contacted with a composition comprising a IL-15 polypeptide during the manufacturing of the CAR-expressing cell, e.g., ex vivo. In embodiments, a CAR-expressing cell described herein is contacted with a composition comprising a combination of both a IL-15 polypeptide and a IL-15 Ra polypeptide during the manufacturing of the CAR-expressing cell, e.g., ex vivo. In embodiments, a CAR-expressing cell described herein is contacted with a composition comprising hetIL-15 during the manufacturing of the CAR-expressing cell, e.g., ex vivo.

In one embodiment the CAR-expressing cell described herein is contacted with a composition comprising hetIL-15 during ex vivo expansion. In an embodiment, the CAR-expressing cell described herein is contacted with a composition comprising an IL-15 polypeptide during ex vivo expansion. In an embodiment, the CAR-expressing cell described herein is contacted with a composition comprising both an IL-15 polypeptide and an IL-15Ra polypeptide during ex vivo expansion. In one embodiment the contacting results in the survival and proliferation of a lymphocyte subpopulation, e.g., CD8+ T cells.

In one embodiment, the cells are cultured (e.g., expanded, simulated, and/or transduced) in media comprising serum. The serum may be, e.g., human AB serum (hAB). In some embodiments, the hAB serum is present at about 2%, about 5%, about 2-3%, about 3-4%, about 4-5%, or about 2-5%. 2% and 5% serum are each suitable levels that allow for many fold expansion of T cells. Furthermore, as shown in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31, medium containing 2% human AB serum is suitable for *ex* vivo expansion of T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

In some embodiments, cells transduced with a nucleic acid encoding a CAR, e.g., a CAR described herein, can be selected for administration based upon, e.g., protein expression levels of one or more of CCL20, GM-CSF, IFNγ, IL-10, IL-13, IL-17a, IL-2, IL-21, IL-4, IL-5, IL-6, IL-9, TNFα and/or combinations thereof. In some embodiments, cells transduced with a nucleic acid encoding a CAR, e.g., a CAR described herein, can be selected for administration based upon, e.g., protein expression levels of CCL20, IL-17a, IL-6 and combinations thereof.

Once a TA CAR is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a TA CAR or a cell expressing both a BCA CAR and a TA CAR (e.g., a cell of the invention) are described in further detail in paragraphs 695-703 of International Publication WO2015/142675, filed March 13, 2015.

Once a BCA CAR is constructed, various assays can be used to evaluate the activity of the molecule, such as but not limited to, the ability to deplete B cells (or other preferred populations) in appropriate in vitro and animal models, and the ability to persist upon exposure to B cells. Assays to evaluate the effects of a BCA CAR or a cell expressing both a BCA CAR and a TA CAR (e.g., a cell of the invention) are described in further detail below.

For example, the cytotoxicity assay described above can be modified to evaluate the cytotoxic activity of a BCA CAR-expressing cell (e.g., a cell expressing both a BCA CAR and a TA CAR) in vitro. Cells of the invention (e.g., a cell expressing both a BCA CAR and a TA CAR) can be mixed with target cells, e.g., cells expressing the B cell antigen targeted by the BCA CAR, at varying ratios of effector to target (E:T). After sufficient incubation to allow cell-mediated cytolysis the supernatant from each ratio sample is harvested and then measured for released 51 Cr. To monitor cell-mediated persistence or proliferation, the cells of the invention can be monitored by, for example, flow cytometry.

Furthermore, animal models similar to those described above can be administered a cell of the invention (e.g., a cell expressing both a BCA CAR and a TA CAR), to evaluate the ability of the cell, including its ability to persist and/or proliferate to a greater degree or for a longer time than the same cell which does not express the BCA CAR.

### Therapeutic Application

Some methods for treating a disease associated with the expression of a tumor antigen with CAR therapy have had variable success, in part due to stimulation of the immune response of the subject to the CAR-expressing cells which can result in subsequent rejection of the CAR-expressing cells, and/or adverse response to the CAR-expressing cells.

In one aspect, the present disclosure provides methods for treating a disease associated with expression of a tumor antigen, e.g., a cancer, described herein, by administering a cell of the disclosure (e.g., a cell expressing both a BCA CAR and a TA CAR). The invention provides a cell of the invention for use in a method of treating cancer. The invention also provides a cell of the invention for use in a method of treating a disease associated with expression of a solid tumor antigen, wherein the disease is a tumor characterized as glioblastoma, ovarian cancer, lung cancer, prostate cancer, colorectal cancer, pancreatic cancer, breast carcinoma, adenocarcinoma or mesothelioma.

In some embodiments, the therapy with the cell of the invention is preceded by, or accompanied by, lymphodepleting therapy. In other embodiments, the therapy with the cell of the invention is not preceded by, or accompanied by, lymphdepleting therapy, e.g., the subject is not administered lymphodepleting therapy 1 year, 9 months, 6, months, 3 months, 2 months, 1 month, 4 weeks, 3 weeks, 2 weeks, 1 week, 6 days, 5 days, 4 days, 3 days, 2 days, 1 day, or fewer months, weeks or days than any of the foregoing, prior to administration of the cell of the invention. Suitable examples of lymphodepleting agents include, but are not limited to, fludarabine and cyclophosphamide, and are further described in the section entitled "Combination Therapies".

In another aspect, the present disclosure provides methods for treating a cancer, e.g., a solid tumor described herein, by administering an immune effector cell comprising (e.g., engineered to express) a TA CAR, e.g., a mesothelin CAR (as described herein) or EGFRvIII CAR (as described herein), and comprising (e.g., engineered to express) a BCA CAR, e.g., a CD19 CAR (as described herein) or a BCMA CAR (as described herein), as described herein. The invention provides a cell of the invention for use in a method of treating cancer, e.g. wherein the cancer is a solid tumor. In embodiments, the cell therapy may be administered with an anti-cancer therapeutic agent described herein. In one embodiment, the anti-cancer therapeutic agent is a chemotherapeutic agent, e.g., as described in the section titled "Combination Therapies". In another embodiment, the anti-cancer therapeutic agent is a therapeutic agent which treats a solid tumor.

In another aspect, the present disclosure provides methods for treating a cancer, e.g., a myeloid tumor described herein, by administering an immune effector cell comprising (e.g., engineered to express) a TA CAR, e.g., a CD123 CAR (as described herein), a CD33 CAR (as described herein) or CLL-1 CAR (as described herein), and comprising (e.g., engineered to express) a BCA CAR, e.g., a CD19 CAR (as described herein) or a BCMA CAR (as described herein), as described herein. In instances, the cell therapy may be administered with an anti-cancer therapeutic agent described herein. In one instance, the anti-cancer therapeutic agent is a chemotherapeutic agent, e.g., as described in the section titled "Combination Therapies". In another instance, the anti-cancer therapeutic agent is a therapeutic agent which treats a myeloid tumor.

Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

### Anti-Cancer Therapy

In one aspect, the present disclosure provides methods of treating a disease, e.g., cancer, by providing to the subject in need thereof a cell of the disclosure, e.g., an immune effector cell (e.g., T cells, NK cells) that comprises (e.g., is engineered to express) a TA CAR described herein, and a BCA CAR described herein, wherein the diseased cells, e.g., cancer cells, express a tumor antigen, e.g., a tumor antigen targeted by the TA CAR. The invention provides a cell of the invention for use in a method of treating cancer.

Without wishing to be bound by any particular theory, the anti-tumor immunity response elicited by the CAR-expressing cell of the invention may be an active or a passive immune response, or alternatively may be due to a direct vs indirect immune response. In one aspect, the CAR-expressing cell of the invention exhibits specific proinflammatory cytokine secretion and potent cytolytic activity in response to human cancer cells expressing the tumor antigen described herein, resist inhibition by soluble tumor antigen as described herein, mediate bystander killing and mediate regression of an established human tumor. For example, antigen-less tumor cells within a heterogeneous field of antigen-positive tumor cells may be susceptible to indirect destruction by the CAR-expressing cell of the invention that has previously reacted against the adjacent antigen-positive tumor cells.

In one instance, the present disclosure provides methods for inhibiting the proliferation or reducing the population of cancer cells expressing a tumor antigen described herein, the methods comprising contacting a tumor antigen described herein-expressing cancer cell population with a cell of the disclosure, e.g., an immune effector cell (e.g., T cells, NK cells) that comprises (e.g., is engineered to express) a TA CAR described herein, and a BCA CAR described herein, that binds to a tumor antigen described herein-expressing cell. In certain instances, a cell of the disclosure, e.g., an immune effector cell (e.g., T cells, NK cells) that comprises (e.g., is engineered to express) a TA CAR described herein, and a BCA CAR described herein, reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model of a cancer associated with the expression of a tumor antigen as described herein, relative to a negative control. In one aspect, the subject is a human.

The present disclosure also provides methods for preventing, treating and/or managing a disease associated with a tumor antigen described herein. These methods comprise administering to a subject in need thereof a cell of the disclosure, e.g., an immune effector cell (e.g., T cells, NK cells) that comprises (e.g., is engineered to express) a TA CAR described herein, and a BCA CAR described herein, that binds to a tumor antigen-expressing cell. The invention provides a cell of the invention for use in a method of treating a disease associated with expression of a solid tumor antigen, wherein the disease is a tumor characterized as glioblastoma, ovarian cancer, lung cancer, prostate cancer, colorectal cancer, pancreatic cancer, breast carcinoma, adenocarcinoma or mesothelioma.

The present disclosure provides methods for preventing relapse of a cancer associated with a tumor antigen as described herein, the methods comprising administering to a subject in need thereof a cell of the disclosure, e.g., an immune effector cell (e.g., T cells, NK cells) that comprises (e.g., is engineered to express) a TA CAR described herein, and a BCA CAR described herein, that binds to a tumor antigen-expressing cell. Without being bound by theory, it is believed that expression of a BCA CAR on the cell of the invention allows the cell to persist longer in vivo, or proliferate to a greater degree in vivo, than an identical cell which does not express the BCA CAR, thereby allowing for the generation of persisting populations of cells expressing a TA CAR, which may effectively treat a relapse without another administration of the cells fo the invention.

In one aspect, the methods comprise administering to the subject in need thereof an effective amount of a CAR-expressing cell described herein ( comprising (e.g., engineered to express) both a TA CAR and a BCA CAR) that binds to a tumor antigen-expressing cell in combination with an effective amount of another therapy, e.g., an administration of a therapy as described in the section titled "Combination Therapies."

In embodiments, administering of the cells of the invention ( comprising (e.g., engineered to express) both a TA CAR and a BCA CAR) results in partial or complete elimination of said tumor cells (e.g., targeted by the TA CAR) and, thereafter, continue to persist in said subject at a level greater than, or for a length of time longer than, otherwise identical cells that lack the BCA CAR.

A cell of the invention ( comprising (e.g., engineered to express) both a TA CAR and a BCA CAR) can stably express a TA CAR molecule that targets a tumor antigen and/or a BCA CAR molecule. Alternatively, a cell of the invention ( comprising (e.g., engineered to express) both a TA CAR and a BCA CAR) can transiently express a TA CAR molecule that targets a tumor antigen and/or a BCA CAR molecule. In embodiments where the TA CAR or the BCA CAR, or both the TA CAR and the BCA CAR are transiently expressed, multiple sequential infusions (e.g., 2, 3, 4, 5, 6, 7, 8, 9 or 10 infusions) of the cell of the invention may be required to effectively treat or manage the disease associated with expression of the tumor antigen. In some embodiments, stable expression of both the BCA CAR and the TA CAR may be preferred for long-term anti-tumor activity. In various aspects, the cell of the invention, or their progeny, persist in the subject for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the cell. In embodiments, such persistence is substantially longer than the persistence of an otherwise identical cell which does not express a BCA CAR, e.g., is at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years longer than the persistence of an otherwise identical molecule which does not express a BCA CAR.

In one aspect, the cell of the invention may be a type of vaccine for ex vivo immunization and/or in vivo therapy in a mammal. In one aspect, the mammal is a human.

With respect to ex vivo immunization, at least one of the following occurs in vitro prior to administering the cell into a mammal: i) expansion of the cells, ii) introducing a nucleic acid encoding a CAR to the cells or iii) cryopreservation of the cells.

Ex vivo procedures are well known in the art and are discussed more fully below. Briefly, cells are isolated from a mammal (e.g., a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

The procedure for ex vivo expansion of hematopoietic stem and progenitor cells is described in U.S. Pat. No. 5,199,942, can be applied to the cells of the present disclosure. Other suitable methods are known in the art, therefore the present disclosure is not limited to any particular method of ex vivo expansion of the cells. Briefly, ex vivo culture and expansion of immune effector cells (e.g., T cells, NK cells) comprises: (1) collecting CD34+ hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

Exemplary CAR-expressing cells that target a tumor antigen, e.g., expressing a solid tumor CAR or a myeloid tumor CAR, are further described herein. Exemplary cells of the disclosure express a CAR molecule that binds to a tumor antigen described herein, e.g., a solid tumor associated antigen, a myeloid tumor antigen, or a hematological tumor not of B-Cell origin, and also express a BCA CAR molecule that binds a B-Cell antigen described herein.

In one embodiment, the cell of the invention comprises (e.g., is engineered to express) a TA CAR targeting mesothelin, as described herein, and a BCA CAR targeting CD19, as described herein. In one embodiment, the cell of the invention comprises (e.g., is engineered to express) a TA CAR targeting mesothelin, as described herein, and a BCA CAR targeting BCMA, as described herein. In one embodiment, the cell of the invention comprises (e.g., is engineered to express) a TA CAR targeting EGFRvIII, as described herein, and a BCA CAR targeting CD19, as described herein. In one embodiment, the cell of the invention comprises (e.g., is engineered to express) a TA CAR targeting EGFRvIII, as described herein, and a BCA CAR targeting BCMA, as described herein. In one instance, the cell of the disclosure comprises (e.g., is engineered to express) a TA CAR targeting CD123, as described herein, and a BCA CAR targeting CD19, as described herein. In one instance, the cell of the disclosure comprises (e.g., is engineered to express) a TA CAR targeting CD123, as described herein, and a BCA CAR targeting BCMA, as described herein. In one instance, the cell of the disclosure comprises (e.g., is engineered to express) a TA CAR targeting CD33, as described herein, and a BCA CAR targeting CD19, as described herein. In one instance, the cell of the disclosure comprises (e.g., is engineered to express) a TA CAR targeting CD33, as described herein, and a BCA CAR targeting BCMA, as described herein. In one instance, the cell of the disclosure comprises (e.g., is engineered to express) a TA CAR targeting CLL-1, as described herein, and a BCA CAR targeting CD19, as described herein. In one instance, the cell of the disclosure comprises (e.g., is engineered to express) a TA CAR targeting CLL-1, as described herein, and a BCA CAR targeting BCMA, as described herein. Other instances and embodiments are described herein as well.

### Diseases associated with expression of a tumor antigen

The methods described herein relate to treating diseases associated with expression of a tumor antigen. A disease associated with expression of a tumor antigen may be a cancer or other proliferative disease, such as an atypical and/or non-classical cancer, malignancy, or precancerous condition, e.g., a hyperplasia, myelodysplasia, a myelodypslastic syndrome, or a preleukemia, associated with expression of the tumor antigen. Non-cancer related indications associated with expression of a tumor antigen as described herein include, but are not limited to, e.g., autimmune disease (e.g., lupus), inflammatory disorders (e.g., allergy and asthma), and transplantation.

Methods described herein can be used to treat any of the following cancers:
Digestive/gastrointestinal cancers such as anal cancer; bile duct cancer; extrahepatic bile duct cancer; appendix cancer; carcinoid tumor, gastrointestinal cancer; colon cancer; colorectal cancer including childhood colorectal cancer; esophageal cancer including childhood esophageal cancer; gallbladder cancer; gastric (stomach) cancer including childhood gastric (stomach) cancer; hepatocellular (liver) cancer including adult (primary) hepatocellular (liver) cancer and childhood (primary) hepatocellular (liver) cancer; pancreatic cancer including childhood pancreatic cancer; sarcoma, rhabdomyosarcoma; islet cell pancreatic cancer; rectal cancer; and small intestine cancer;
Endocrine cancers such as islet cell carcinoma (endocrine pancreas); adrenocortical carcinoma including childhood adrenocortical carcinoma; gastrointestinal carcinoid tumor; parathyroid cancer; pheochromocytoma; pituitary tumor; thyroid cancer including childhood thyroid cancer; childhood multiple endocrine neoplasia syndrome; and childhood carcinoid tumor;
Eye cancers such as intraocular melanoma; and retinoblastoma;
Musculoskeletal cancers such as Ewing's family of tumors; osteosarcoma/malignant fibrous histiocytoma of the bone; childhood rhabdomyosarcoma; soft tissue sarcoma including adult and childhood soft tissue sarcoma; clear cell sarcoma of tendon sheaths; and uterine sarcoma;
Breast cancer such as breast cancer including childhood and male breast cancer and pregnancy;
Neurologic cancers such as childhood brain stem glioma; brain tumor; childhood cerebellar astrocytoma; childhood cerebral astrocytoma/malignant glioma; childhood ependymoma; childhood medulloblastoma; childhood pineal and supratentorial primitive neuroectodermal tumors; childhood visual pathway and hypothalamic glioma; other childhood brain cancers; adrenocortical carcinoma; central nervous system lymphoma, primary; childhood cerebellar astrocytoma; neuroblastoma; craniopharyngioma; spinal cord tumors; central nervous system atypical teratoid/rhabdoid tumor; central nervous system embryonal tumors; and childhood supratentorial primitive neuroectodermal tumors and pituitary tumor;
Genitourinary cancers such as bladder cancer including childhood bladder cancer; renal cell (kidney) cancer; ovarian cancer including childhood ovarian cancer; ovarian epithelial cancer; ovarian low malignant potential tumor; penile cancer; prostate cancer; renal cell cancer including childhood renal cell cancer; renal pelvis and ureter, transitional cell cancer; testicular cancer; urethral cancer; vaginal cancer; vulvar cancer; cervical cancer; Wilms tumor and other childhood kidney tumors; endometrial cancer; and gestational trophoblastic tumor;
Germ cell cancers such as childhood extracranial germ cell tumor; extragonadal germ cell tumor; ovarian germ cell tumor; and testicular cancer;
Head and neck cancers such as lip and oral cavity cancer; oral cancer including childhood oral cancer; hypopharyngeal cancer; laryngeal cancer including childhood laryngeal cancer; metastatic squamous neck cancer with occult primary; mouth cancer; nasal cavity and paranasal sinus cancer; nasopharyngeal cancer including childhood nasopharyngeal cancer; oropharyngeal cancer; parathyroid cancer; pharyngeal cancer; salivary gland cancer including childhood salivary gland cancer; throat cancer; and thyroid cancer;
Hematological cancers such as a leukemia or a lymphoma; including, but not limited to, e.g., one or more acute leukemias including but not limited to, e.g., B-cell acute Lymphoid Leukemia ("BALL"), T-cell acute Lymphoid Leukemia ("TALL"), acute lymphoid leukemia (ALL); one or more chronic leukemias including but not limited to, e.g., chronic myelogenous leukemia (CML), Chronic Lymphoid Leukemia (CLL). Additional hematological cancers include, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like.

Lung cancer such as non-small cell lung cancer; and small cell lung cancer;
Respiratory cancers such as malignant mesothelioma, adult; malignant mesothelioma, childhood; malignant thymoma; childhood thymoma; thymic carcinoma; bronchial adenomas/carcinoids including childhood bronchial adenomas/carcinoids; pleuropulmonary blastoma; non-small cell lung cancer; and small cell lung cancer;
Skin cancers such as Kaposi's sarcoma; Merkel cell carcinoma; melanoma; and childhood skin cancer;
AIDS-related malignancies;
Other childhood cancers, unusual cancers of childhood and cancers of unknown primary site;
and metastases of the aforementioned cancers can also be treated or prevented in accordance with the methods described herein. Treatment of metastatic cancers, e.g., metastatic cancers that express PD-L1 (Iwai et al. (2005) Int. Immunol. 17:133-144) can be effected using the methods described herein. Exemplary cancers whose growth can be inhibited include cancers typically responsive to immunotherapy. Additionally, refractory or recurrent malignancies can be treated using the molecules described herein.

In one embodiment, the present invention provides therapy described herein wherein cells or compostions of the invention is administered to treat a solid tumor, e.g., to inhibit the growth of a solid tumor. In embodiments the cells comprise a TA CAR molecule that targets, e.g., binds, to a tumor antigen present on a cell or population of cells in the solid tumor. Examples of solid tumors that can be treated with methods disclosed herein include malignancies, e.g., sarcomas, adenocarcinomas, and carcinomas, of the various organ systems, such as those affecting pancreas, liver, lung, breast, ovary, lymphoid, gastrointestinal (e.g., colon), genitourinary tract (e.g., renal, urothelial cells), prostate, and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the solid tumor is a mesothelioma. Metastatic lesions of the aforementioned cancers can also be treated or prevented using the methods and compositions of the invention.

In one embodiment, the combination therapy described herein is administered to treat a CD19 negative cancer. A CD19 negative cancer can be characterized by CD19 loss *(e.g.,* an antigen loss mutation) or other CD19 alteration that reduces the level of CD19 *(e.g.,* caused by clonal selection of CD19-negative clones). It shall be understood that a CD19-negative cancer need not have 100% loss of CD19, and may retain some partial CD19 expression (e.g., retain some cancer cells that express CD19).

In one aspect, the present disclosure provides cells for use in methods of treating cancer by providing to the subject in need thereof immune effector cells (e.g., T cells, NK cells) that are engineered to express an EGFRvIIICAR, wherein the cancer cells express EGFRvIII. In one embodiment, the cancer to be treated is glioblastoma.

In one aspect, the present disclosure provides cells for use in methods of treating cancer by providing to the subject in need thereof immune effector cells (e.g., T cells, NK cells) that are engineered to express a mesothelinCAR, wherein the cancer cells express mesothelin. In one embodiment, the cancer to be treated is mesothelioma, malignant pleural mesothelioma, non-small cell lung cancer, small cell lung cancer, squamous cell lung cancer, or large cell lung cancer, pancreatic cancer, pancreatic ductal adenocarcinoma, pancreatic metatstatic, esophageal adenocarcinoma, breast cancer, ovarian cancer, colorectal cancer and bladder cancer, or any combination thereof.

In one aspect, the present disclosure provides cells for use in methods of treating cancer by providing to the subject in need thereof immune effector cells (e.g., T cells, NK cells) that are engineered to express a GD2CAR, wherein the cancer cells express GD2. In one embodiment, the cancer to be treated is neuroblastoma.

In one aspect, the present disclosure provides cells for use in methods of treating cancer by providing to the subject in need thereof immune effector cells (e.g., T cells, NK cells) that are engineered to express a TnCAR, wherein the cancer cells express Tn antigen. In one embodiment, the cancer to be treated is ovarian cancer, colon cancer, breast cancer, or pancreatic cancer.

In one aspect, the present disclosure provides cells for use in methods of treating cancer by providing to the subject in need thereof immune effector cells (e.g., T cells, NK cells) that are engineered to express a sTnCAR, wherein the cancer cells express sTn antigen. In one embodiment, the cancer to be treated is ovarian cancer, colon cancer, breast cancer, or pancreatic cancer.

In one aspect, the present disclosure provides cells for use in methods of treating cancer by providing to the subject in need thereof immune effector cells (e.g., T cells, NK cells) that are engineered to express a PSMACAR, wherein the cancer cells express PSMA. In one embodiment, the cancer to be treated is prostate cancer.

In one aspect, the present disclosure provides cells for use in methods of treating cancer by providing to the subject in need thereof immune effector cells (e.g., T cells, NK cells) that are engineered to express a TAG72CAR, wherein the cancer cells express TAG72. In one embodiment, the cancer to be treated is gastrointestinal cancer.

In one aspect, the present disclosure provides cells for use in methods of treating cancer by providing to the subject in need thereof immune effector cells (e.g., T cells, NK cells) that are engineered to express a CD44v6CAR, wherein the cancer cells express CD44v6. In one embodiment, the cancer to be treated is cervical cancer, AML, or MM.

In one aspect, the present disclosure provides cells for use in methods of treating cancer by providing to the subject in need thereof immune effector cells (e.g., T cells, NK cells) that are engineered to express an EPCAMCAR, wherein the cancer cells express EPCAM. In one embodiment, the cancer to be treated is gastrointestinal cancer.

In one aspect, the present disclosure provides cells for use in methods of treating cancer by providing to the subject in need thereof immune effector cells (e.g., T cells, NK cells) that are engineered to express a KITCAR, wherein the cancer cells express KIT. In one embodiment, the cancer to be treated is gastrointestinal cancer.

In one aspect, the present disclosure provides cells for use in methods of treating cancer by providing to the subject in need thereof immune effector cells (e.g., T cells, NK cells) that are engineered to express a IL-13Ra2CAR, wherein the cancer cells express IL-13Ra2. In one embodiment, the cancer to be treated is glioblastoma.

In one aspect, the present disclosure provides cells for use in methods of treating cancer by providing to the subject in need thereof immune effector cells (e.g., T cells, NK cells) that are engineered to express a CD171CAR, wherein the cancer cells express CD171. In one embodiment, the cancer to be treated is neuroblastoma, ovarian cancer, melanoma, breast cancer, pancreatic cancer, colon cancers, or NSCLC (non-small cell lung cancer).

In one aspect, the present disclosure provides cells for use in methods of treating cancer by providing to the subject in need thereof immune effector cells (e.g., T cells, NK cells) that are engineered to express a PSCACAR, wherein the cancer cells express PSCA. In one embodiment, the cancer to be treated is prostate cancer.

In one aspect, the present disclosure provides cells for use in methods of treating cancer by providing to the subject in need thereof immune effector cells (e.g., T cells, NK cells) that are engineered to express a LewisYCAR, wherein the cancer cells express LewisY. In one embodiment, the cancer to be treated is ovarian cancer, or AML.

In one aspect, the present disclosure provides cells for use in methods of treating cancer by providing to the subject in need thereof immune effector cells (e.g., T cells, NK cells) that are engineered to express a PDGFR-betaCAR, wherein the cancer cells express PDGFR-beta. In one embodiment, the cancer to be treated is breast cancer, prostate cancer, GIST (gastrointestinal stromal tumor), CML, DFSP (dermatofibrosarcoma protuberans), or glioma.

In one aspect, the present disclosure provides cells for use in methods of treating cancer by providing to the subject in need thereof immune effector cells (e.g., T cells, NK cells) that are engineered to express a SSEA-4CAR, wherein the cancer cells express SSEA-4. In one embodiment, the cancer to be treated is glioblastoma, breast cancer, lung cancer, or stem cell cancer.

In one aspect, the present disclosure provides cells for use in methods of treating cancer by providing to the subject in need thereof immune effector cells (e.g., T cells, NK cells) that are engineered to express a Folate receptor alphaCAR, wherein the cancer cells express folate receptor alpha. In one embodiment, the cancer to be treated is ovarian cancer, NSCLC, endometrial cancer, renal cancer, or other solid tumors.

In one aspect, the present disclosure provides cells for use in methods of treating cancer by providing to the subject in need thereof immune effector cells (e.g., T cells, NK cells) that are engineered to express an ERBB2CAR, wherein the cancer cells express ERBB2 (Her2/neu). In one embodiment, the cancer to be treated is breast cancer, gastric cancer, colorectal cancer, lung cancer, or other solid tumors.

In one aspect, the present disclosure provides cells for use in methods of treating cancer by providing to the subject in need thereof immune effector cells (e.g., T cells, NK cells) that are engineered to express a MUC1CAR, wherein the cancer cells express MUC1. In one embodiment, the cancer to be treated is breast cancer, lung cancer, or other solid tumors.

In one aspect, the present disclosure provides cells for use in methods of treating cancer by providing to the subject in need thereof immune effector cells (e.g., T cells, NK cells) that are engineered to express an EGFRCAR, wherein the cancer cells express EGFR. In one embodiment, the cancer to be treated is glioblastoma, SCLC (small cell lung cancer), SCCHN (squamous cell carcinoma of the head and neck), NSCLC, or other solid tumors.

In one aspect, the present disclosure provides cells for use in methods of treating cancer by providing to the subject in need thereof immune effector cells (e.g., T cells, NK cells) that are engineered to express a NCAMCAR, wherein the cancer cells express NCAM. In one embodiment, the cancer to be treated is neuroblastoma, or other solid tumors.

In one aspect, the present disclosure provides cells for use in methods of treating cancer by providing to the subject in need thereof immune effector cells (e.g., T cells, NK cells) that are engineered to express a CAIXCAR, wherein the cancer cells express CAIX. In one embodiment, the cancer to be treated is renal cancer, CRC, cervical cancer, or other solid tumors.

In one aspect, the present disclosure provides cells for use in methods of treating cancer by providing to the subject in need thereof immune effector cells (e.g., T cells, NK cells) that are engineered to express a HMWMAACAR, wherein the cancer cells express HMWMAA. In one embodiment, the cancer to be treated is melanoma, glioblastoma, or breast cancer.

In one aspect, the present disclosure provides cells for use in methods of treating cancer by providing to the subject in need thereof immune effector cells (e.g., T cells, NK cells) that are engineered to express an o-acetyl-GD2CAR, wherein the cancer cells express o-acetyl-GD2. In one embodiment, the cancer to be treated is neuroblastoma, or melanoma.

In one aspect, the present disclosure provides cells for use in methods of treating cancer by providing to the subject in need thereof immune effector cells (e.g., T cells, NK cells) that are engineered to express a CLDN6CAR, wherein the cancer cells express CLDN6. In one embodiment, the cancer to be treated is ovarian cancer, lung cancer, or breast cancer.

In one aspect, the present disclosure provides cells for use in methods of treating cancer by providing to the subject in need thereof immune effector cells (e.g., T cells, NK cells) that are engineered to express a TSHRCAR, wherein the cancer cells express TSHR. In one embodiment, the cancer to be treated is thyroid cancer, or multiple myeloma.

In one aspect, the present disclosure provides cells for use in methods of treating cancer by providing to the subject in need thereof immune effector cells (e.g., T cells, NK cells) that are engineered to express a CD97CAR, wherein the cancer cells express CD97. In one embodiment, the cancer to be treated is B cell malignancies, gastric cancer, pancreatic cancer, esophageal cancer, glioblastoma, breast cancer, or colorectal cancer.

In one aspect, the present disclosure provides cells for use in methods of treating cancer by providing to the subject in need thereof immune effector cells (e.g., T cells, NK cells) that are engineered to express a Plysialic acid CAR, wherein the cancer cells express Plysialic acid. In one embodiment, the cancer to be treated is small cell lung cancer.

In one aspect, the present disclosure provides cells for use in methods of treating cancer by providing to the subject in need thereof immune effector cells (e.g., T cells, NK cells) that are engineered to express a PLAC1CAR, wherein the cancer cells express PLAC1. In one embodiment, the cancer to be treated is HCC (hepatocellular carcinoma).

In one aspect, the present disclosure provides cells for use in methods of treating cancer by providing to the subject in need thereof immune effector cells (e.g., T cells, NK cells) that are engineered to express a GloboHCAR, wherein the cancer cells express GloboH. In one embodiment, the cancer to be treated is ovarian cancer, gastric cancer, prostate cancer, lung cancer, breast cancer, or pancreatic cancer.

In one aspect, the present disclosure provides cells for use in methods of treating cancer by providing to the subject in need thereof immune effector cells (e.g., T cells, NK cells) that are engineered to express a NY-BR-1CAR, wherein the cancer cells express NY-BR-1. In one embodiment, the cancer to be treated is breast cancer.

In one aspect, the present disclosure provides cells for use in methods of treating cancer by providing to the subject in need thereof immune effector cells (e.g., T cells, NK cells) that are engineered to express a MAD-CT-1CAR, wherein the cancer cells express MAD-CT-1. In one embodiment, the cancer to be treated is prostate cancer, or melanoma.

In one aspect, the present disclosure provides cells for use in methods of treating cancer by providing to the subject in need thereof immune effector cells (e.g., T cells, NK cells) that are engineered to express a MAD-CT-2CAR, wherein the cancer cells express MAD-CT-2. In one embodiment, the cancer to be treated is prostate cancer, melanoma.

In one aspect, the present disclosure provides cells for use in methods of treating cancer by providing to the subject in need thereof immune effector cells (e.g., T cells, NK cells) that are engineered to express a Fos-related antigen 1 CAR, wherein the cancer cells express Fos-related antigen 1. In one embodiment, the cancer to be treated is glioma, squamous cell cancer, or pancreatic cancer.

In one aspect, the present disclosure provides cells for use in methods of treating cancer by providing to the subject in need thereof immune effector cells (e.g., T cells, NK cells) that are engineered to express a ML-IAP CAR, wherein the cancer cells express ML-IAP. In one embodiment, the cancer to be treated is melanoma.

In one aspect, the present disclosure provides cells for use in methods of treating cancer by providing to the subject in need thereof immune effector cells (e.g., T cells, NK cells) that are engineered to express a NA17CAR, wherein the cancer cells express NA17. In one embodiment, the cancer to be treated is melanoma.

In one aspect, the present disclosure provides cells for use in methods of treating cancer by providing to the subject in need thereof immune effector cells (e.g., T cells, NK cells) that are engineered to express a TRP-2CAR, wherein the cancer cells express TRP-2. In one embodiment, the cancer to be treated is melanoma.

In one aspect, the present disclosure provides cells for use in methods of treating cancer by providing to the subject in need thereof immune effector cells (e.g., T cells, NK cells) that are engineered to express a CYP1B1CAR, wherein the cancer cells express CYP1B1. In one embodiment, the cancer to be treated is breast cancer, colon cancer, lung cancer, esophagus cancer, skin cancer, lymph node cancer, brain cancer, or testis cancer.

In one aspect, the present disclosure provides cells for use in methods of treating cancer by providing to the subject in need thereof immune effector cells (e.g., T cells, NK cells) that are engineered to express a RAGE-1CAR, wherein the cancer cells express RAGE-1. In one embodiment, the cancer to be treated is RCC (renal cell cancer), or other solid tumors

In one aspect, the present disclosure provides : cells for use in methods of treating cancer by providing to the subject in need thereof immune effector cells (e.g., T cells, NK cells) that are engineered to express a human telomerase reverse transcriptaseCAR, wherein the cancer cells express human telomerase reverse transcriptase. In one embodiment, the cancer to be treated is solid tumors.

In one aspect, the present disclosure provides cells for use in methods of treating cancer by providing to the subject in need thereof immune effector cells (e.g., T cells, NK cells) that are engineered to express an intestinal carboxyl esteraseCAR, wherein the cancer cells express intestinal carboxyl esterase. In one embodiment, the cancer to be treated is thyroid cancer, RCC, CRC (colorectal cancer), breast cancer, or other solid tumors.

In one aspect, the present disclosure provides cells for use in methods of treating cancer by providing to the subject in need thereof immune effector cells (e.g., T cells, NK cells) that are engineered to express a mut hsp70-2CAR, wherein the cancer cells express mut hsp70-2. In one embodiment, the cancer to be treated is melanoma.

### Combination Therapies

The CAR-expressing cells (e.g., a cell comprising, e.g., engineered to express, a TA CAR and a BCA CAR) described herein may be used in combination with other known agents and therapies.

The combination therapy described herein, e.g., comprising a cell of the invention, can be administered in combination with at least one additional therapeutic agent. In an instance, a CAR-expressing cell described herein, and the at least one additional therapeutic agent can be administered simultaneously, in the same or in separate compositions, or sequentially. For sequential administration, the CAR-expressing cell described herein can be administered first, and the additional agent can be administered second, or the order of administration can be reversed.

In further aspects, a CAR-expressing cell described herein may be used in a treatment regimen in combination with surgery, chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, irradiation, and peptide vaccine, such as that described in Izumoto et al. 2008 J Neurosurg 108:963-971.

In one instance, a CAR-expressing cell described herein may be used in combination with a lymphodepleting agent. An exemplary lymphodepleting agent reduces or decreases lymphocytes, e.g., B cell lymphocytes and/or T cell lymphocytes, prior to immunotherapy. Exemplary lymphodepleting agents include fludarabine, cyclophosphamide, corticosteroids, alemtuzumab, or total body irradiation (TBI), or a combination thereof. For example, a combination of fludarabine and cyclophosphamide is administered prior to or simultaneously with administration of a cell described herein.

In one instance, a CAR-expressing cell described herein may be used in combination with an agent that treats B cell aplasia. Persistent B cell aplasia leads to hypogammaglobulinemia and may increase the risk of infection. Agents for treating B cell aplasia includes intravenous immunoglobulin (IVIG), e.g., FLEBOGAMMA^{™}, GAMUNEX-C^{®}, PRIVIGEN^{®}, and GAMMAGARD^{®}.

In one instance, a CAR-expressing cell described herein can be used in combination with a chemotherapeutic agent. Exemplary chemotherapeutic agents include an anthracycline (e.g., doxorubicin (e.g., liposomal doxorubicin)). a vinca alkaloid (e.g., vinblastine, vincristine, vindesine, vinorelbine), an alkylating agent (e.g., cyclophosphamide, decarbazine, melphalan, ifosfamide, temozolomide), an immune cell antibody (e.g., alemtuzamab, gemtuzumab, rituximab, tositumomab), an antimetabolite (including, e.g., folic acid antagonists, pyrimidine analogs, purine analogs and adenosine deaminase inhibitors (e.g., fludarabine)), an mTOR inhibitor, a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide).

General Chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Exemplary alkylating agents include, without limitation, nitrogen mustards, ethylenimine derivatives, alkyl sulfonates, nitrosoureas and triazenes): uracil mustard (Aminouracil Mustard^{®}, Chlorethaminacil^{®}, Demethyldopan^{®}, Desmethyldopan^{®}, Haemanthamine^{®}, Nordopan^{®}, Uracil nitrogen mustard^{®}, Uracillost^{®}, Uracilmostaza^{®}, Uramustin^{®}, Uramustine^{®}), chlormethine (Mustargen^{®}), cyclophosphamide (Cytoxan^{®}, Neosar^{®}, Clafen^{®}, Endoxan^{®}, Procytox^{®}, Revimmune^{™}), ifosfamide (Mitoxana^{®}), melphalan (Alkeran^{®}), Chlorambucil (Leukeran^{®}), pipobroman (Amedel^{®}, Vercyte^{®}), triethylenemelamine (Hemel^{®}, Hexalen^{®}, Hexastat^{®}), triethylenethiophosphoramine, Temozolomide (Temodar^{®}), thiotepa (Thioplex^{®}), busulfan (Busilvex^{®}, Myleran^{®}), carmustine (BiCNU^{®}), lomustine (CeeNU^{®}), streptozocin (Zanosar^{®}), and Dacarbazine (DTIC-Dome^{®}). Additional exemplary alkylating agents include, without limitation, Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as CDDP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}), Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).

Exemplary mTOR inhibitors include, e.g., temsirolimus; ridaforolimus (formally known as deferolimus, (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor^{®} or RAD001); rapamycin (AY22989, Sirolimus^{®}); simapimod (CAS 164301-51-3); emsirolimus, (5-{2,4-Bis[(3*S*)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine- (SEQ ID NO: 264), inner salt (SF1126, CAS 936487-67-1), and XL765.

Exemplary immunomodulators include, e.g., afutuzumab (available from Roche^{®}); pegfilgrastim (Neulasta^{®}); lenalidomide (CC-5013, Revlimid^{®}); thalidomide (Thalomid^{®}), actimid (CC4047); and IRX-2 (mixture of human cytokines including interleukin 1, interleukin 2, and interferon γ, CAS 951209-71-5, available from IRX Therapeutics).

Exemplary anthracyclines include, e.g., doxorubicin (Adriamycin^{®} and Rubex^{®}); bleomycin (lenoxane^{®}); daunorubicin (dauorubicin hydrochloride, daunomycin, and rubidomycin hydrochloride, Cerubidine^{®}); daunorubicin liposomal (daunorubicin citrate liposome, DaunoXome^{®}); mitoxantrone (DHAD, Novantrone^{®}); epirubicin (Ellence^{™}); idarubicin (Idamycin^{®}, Idamycin PFS^{®}); mitomycin C (Mutamycin^{®}); geldanamycin; herbimycin; ravidomycin; and desacetylravidomycin.

Exemplary vinca alkaloids include, e.g., vinorelbine tartrate (Navelbine^{®}), Vincristine (Oncovin^{®}), and Vindesine (Eldisine^{®})); vinblastine (also known as vinblastine sulfate, vincaleukoblastine and VLB, Alkaban-AQ^{®} and Velban^{®}); and vinorelbine (Navelbine^{®}).

Exemplary proteosome inhibitors include bortezomib (Velcade^{®}); carfilzomib (PX-171-007, (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)-pentanamide); marizomib (NPI-0052); ixazomib citrate (MLN-9708); delanzomib (CEP-18770); and O-Methyl-N-[(2-methyl-5-thiazolyl)carbonyl]-L-seryl-O-methyl-*N*-[(1*S*)-2-[(2*R*)-2-methyl-2-oxiranyl]-2-oxo-1-(phenylmethyl)ethyl]- L-serinamide (ONX-0912).

In instances, a CAR-expressing cell described herein is administered to a subject in combination with brentuximab. Brentuximab is an antibody-drug conjugate of anti-CD30 antibody and monomethyl auristatin E. In embodiments, the subject has Hodgkin's lymphoma (HL), e.g., relapsed or refractory HL. In embodiments, the subject comprises CD30+ HL. In embodiments, the subject has undergone an autologous stem cell transplant (ASCT). In embodiments, the subject has not undergone an ASCT. In embodiments, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In instances, a CAR-expressing cell described herein is administered to a subject in combination with brentuximab and dacarbazine or in combination with brentuximab and bendamustine. Dacarbazine is an alkylating agent with a chemical name of 5-(3,3-Dimethyl-1-triazenyl)imidazole-4-carboxamide. Bendamustine is an alkylating agent with a chemical name of 4-[5-[Bis(2-chloroethyl)amino]-1-methylbenzimidazol-2-yl]butanoic acid. In embodiments, the subject has Hodgkin's lymphoma (HL). In embodiments, the subject has not previously been treated with a cancer therapy. In embodiments, the subject is at least 60 years of age, e.g., 60, 65, 70, 75, 80, 85, or older. In embodiments, dacarbazine is administered at a dosage of about 300-450 mg/m² (e.g., about 300-325, 325-350, 350-375, 375-400, 400-425, or 425-450 mg/m²), e.g., intravenously. In embodiments, bendamustine is administered at a dosage of about 75-125 mg/m2 (e.g., 75-100 or 100-125 mg/m², e.g., about 90 mg/m²), e.g., intravenously. In embodiments, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In some instances, a CAR-expressing cell described herein is administered to a subject in combination with a CD20 inhibitor, e.g., an anti-CD20 antibody (e.g., an anti-CD20 mono- or bispecific antibody) or a fragment thereof. Exemplary anti-CD20 antibodies include but are not limited to rituximab, ofatumumab, ocrelizumab, veltuzumab, obinutuzumab, TRU-015 (Trubion Pharmaceuticals), ocaratuzumab, and Pro131921 (Genentech). See, e.g., Lim et al. Haematologica. 95.1(2010):135-43.

In some embodiments, the anti-CD20 antibody comprises rituximab. Rituximab is a chimeric mouse/human monoclonal antibody IgG1 kappa that binds to CD20 and causes cytolysis of a CD20 expressing cell, e.g., as described in www.accessdata.fda.gov/drugsatfda_docs/label/2010/103705s5311lbl.pdf. In embodiments, a CAR-expressing cell described herein is administered to a subject in combination with rituximab. In embodiments, the subject has CLL or SLL.

In some embodiments, rituximab is administered intravenously, e.g., as an intravenous infusion. For example, each infusion provides about 500-2000 mg (e.g., about 500-550, 550-600, 600-650, 650-700, 700-750, 750-800, 800-850, 850-900, 900-950, 950-1000, 1000-1100, 1100-1200, 1200-1300, 1300-1400, 1400-1500, 1500-1600, 1600-1700, 1700-1800, 1800-1900, or 1900-2000 mg) of rituximab. In some embodiments, rituximab is administered at a dose of 150 mg/m² to 750 mg/m², e.g., about 150-175 mg/m², 175-200 mg/m², 200-225 mg/m², 225-250 mg/m², 250-300 mg/m², 300-325 mg/m², 325-350 mg/m², 350-375 mg/m², 375-400 mg/m², 400-425 mg/m², 425-450 mg/m², 450-475 mg/m², 475-500 mg/m², 500-525 mg/m², 525-550 mg/m², 550-575 mg/m², 575-600 mg/m², 600-625 mg/m², 625-650 mg/m², 650-675 mg/m², or 675-700 mg/m², where m² indicates the body surface area of the subject. In some embodiments, rituximab is administered at a dosing interval of at least 4 days, e.g., 4, 7, 14, 21, 28, 35 days, or more. For example, rituximab is administered at a dosing interval of at least 0.5 weeks, e.g., 0.5, 1, 2, 3, 4, 5, 6, 7, 8 weeks, or more. In some embodiments, rituximab is administered at a dose and dosing interval described herein for a period of time, e.g., at least 2 weeks, e.g., at least 2, 3, 4, 5, 6, 7, 8, 9, 10, 11, 12, 13, 14, 15, 16, 17, 18, 19, 20 weeks, or greater. For example, rituximab is administered at a dose and dosing interval described herein for a total of at least 4 doses per treatment cycle (e.g., at least 4, 5, 6, 7, 8, 9, 10, 11, 12, 13, 14, 15, 16, or more doses per treatment cycle).

In some embodiments, the anti-CD20 antibody comprises ofatumumab. Ofatumumab is an anti-CD20 IgG1κ human monoclonal antibody with a molecular weight of approximately 149 kDa. For example, ofatumumab is generated using transgenic mouse and hybridoma technology and is expressed and purified from a recombinant murine cell line (NS0). See, e.g., www.accessdata.fda.gov/drugsatfda_docs/label/2009/125326lbl.pdf; and Clinical Trial Identifier number NCT01363128, NCT01515176, NCT01626352, and NCT01397591. In embodiments, a CAR-expressing cell described herein is administered to a subject in combination with ofatumumab. In embodiments, the subject has CLL or SLL.

In some embodiments, ofatumumab is administered as an intravenous infusion. For example, each infusion provides about 150-3000 mg (e.g., about 150-200, 200-250, 250-300, 300-350, 350-400, 400-450, 450-500, 500-550, 550-600, 600-650, 650-700, 700-750, 750-800, 800-850, 850-900, 900-950, 950-1000, 1000-1200, 1200-1400, 1400-1600, 1600-1800, 1800-2000, 2000-2200, 2200-2400, 2400-2600, 2600-2800, or 2800-3000 mg) of ofatumumab. In embodiments, ofatumumab is administered at a starting dosage of about 300 mg, followed by 2000 mg, e.g., for about 11 doses, e.g., for 24 weeks. In some embodiments, ofatumumab is administered at a dosing interval of at least 4 days, e.g., 4, 7, 14, 21, 28, 35 days, or more. For example, ofatumumab is administered at a dosing interval of at least 1 week, e.g., 1, 2, 3, 4, 5, 6, 7, 8, 9, 10, 11, 12, 24, 26, 28, 20, 22, 24, 26, 28, 30 weeks, or more. In some embodiments, ofatumumab is administered at a dose and dosing interval described herein for a period of time, e.g., at least 1 week, e.g., 1, 2, 3, 4, 5, 6, 7, 8, 9, 10, 11, 12, 13, 14, 15, 16, 17, 18, 19, 20, 22, 24, 26, 28, 30, 40, 50, 60 weeks or greater, or 1, 2, 3, 4, 5, 6, 7, 8, 9, 10, 11, 12 months or greater, or 1, 2, 3, 4, 5 years or greater. For example, ofatumumab is administered at a dose and dosing interval described herein for a total of at least 2 doses per treatment cycle (e.g., at least 2, 3, 4, 5, 6, 7, 8, 9, 10, 11, 12, 13, 14, 15, 16, 18, 20, or more doses per treatment cycle).

In some cases, the anti-CD20 antibody comprises ocrelizumab. Ocrelizumab is a humanized anti-CD20 monoclonal antibody, e.g., as described in Clinical Trials Identifier Nos. NCT00077870, NCT01412333, NCT00779220, NCT00673920, NCT01194570, and Kappos et al. Lancet. 19.378(2011):1779-87.

In some cases, the anti-CD20 antibody comprises veltuzumab. Veltuzumab is a humanized monoclonal antibody against CD20. See, e.g., Clinical Trial Identifier No. NCT00547066, NCT00546793, NCT01101581, and Goldenberg et al. Leuk Lymphoma. 51(5)(2010):747-55.

In some cases, the anti-CD20 antibody comprises GA101. GA101 (also called obinutuzumab or RO5072759) is a humanized and glyco-engineered anti-CD20 monoclonal antibody. See, e.g., Robak. Curr. Opin. Investig. Drugs. 10.6(2009):588-96; Clinical Trial Identifier Numbers: NCT01995669, NCT01889797, NCT02229422, and NCT01414205; and www.accessdata.fda.gov/drugsatfda_docs/label/2013/125486s000lbl.pdf.

In some cases, the anti-CD20 antibody comprises AME-133v. AME-133v (also called LY2469298 or ocaratuzumab) is a humanized IgG1 monoclonal antibody against CD20 with increased affinity for the FcγRIIIa receptor and an enhanced antibody dependent cellular cytotoxicity (ADCC) activity compared with rituximab. See, e.g., Robak et al. BioDrugs 25.1(2011):13-25; and Forero-Torres et al. Clin Cancer Res. 18.5(2012):1395-403.

In some cases, the anti-CD20 antibody comprises PRO131921. PRO131921 is a humanized anti-CD20 monoclonal antibody engineered to have better binding to FcγRIIIa and enhanced ADCC compared with rituximab. See, e.g., Robak et al. BioDrugs 25.1(2011):13-25; and Casulo et al. Clin Immunol. 154.1 (2014): 37-46; and Clinical Trial Identifier No. NCT00452127.

In some cases, the anti-CD20 antibody comprises TRU-015. TRU-015 is an anti-CD20 fusion protein derived from domains of an antibody against CD20. TRU-015 is smaller than monoclonal antibodies, but retains Fc-mediated effector functions. See, e.g., Robak et al. BioDrugs 25.1(2011):13-25. TRU-015 contains an anti-CD20 single-chain variable fragment (scFv) linked to human IgG1 hinge, CH2, and CH3 domains but lacks CH1 and CL domains.

In some embodiments, an anti-CD20 antibody described herein is conjugated or otherwise bound to a therapeutic agent, e.g., a chemotherapeutic agent (e.g., cytoxan, fludarabine, histone deacetylase inhibitor, demethylating agent, peptide vaccine, anti-tumor antibiotic, tyrosine kinase inhibitor, alkylating agent, anti-microtubule or anti-mitotic agent), anti-allergic agent, anti-nausea agent (or anti-emetic), pain reliever, or cytoprotective agent described herein.

In instances, a CAR-expressing cell described herein is administered to a subject in combination with a B-cell lymphoma 2 (BCL-2) inhibitor (e.g., venetoclax, also called ABT-199 or GDC-0199;) and/or rituximab. In embodiments, a CAR-expressing cell described herein is administered to a subject in combination with venetoclax and rituximab. Venetoclax is a small molecule that inhibits the anti-apoptotic protein, BCL-2. The structure of venetoclax (4-(4-{[2-(4-chlorophenyl)-4,4-dimethylcyclohex-1-en-1-yl]methyl}piperazin-1-yl)-*N*-({3-nitro-4-[(tetrahydro-2*H*-pyran-4-ylmethyl)amino]phenyl}sulfonyl)-2-(1*H*-pyrrolo[2,3-b]pyridin-5-yloxy)benzamide) is shown below.

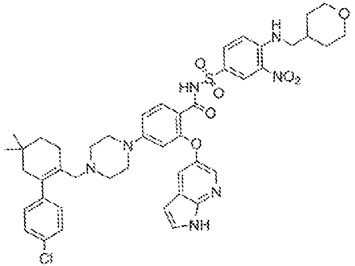

In embodiments, the subject has CLL. In embodiments, the subject has relapsed CLL, e.g., the subject has previously been administered a cancer therapy. In embodiments, venetoclax is administered at a dosage of about 15-600 mg (e.g., 15-20, 20-50, 50-75, 75-100, 100-200, 200-300, 300-400, 400-500, or 500-600 mg), e.g., daily. In embodiments, rituximab is administered at a dosage of about 350-550 mg/m2 (e.g., 350-375, 375-400, 400-425, 425-450, 450-475, or 475-500 mg/m2), e.g., intravenously, e.g., monthly.

In some instances, a CAR-expressing cell described herein is administered in combination with an oncolytic virus. In embodiments, oncolytic viruses are capable of selectively replicating in and triggering the death of or slowing the growth of a cancer cell. In some cases, oncolytic viruses have no effect or a minimal effect on non-cancer cells. An oncolytic virus includes but is not limited to an oncolytic adenovirus, oncolytic Herpes Simplex Viruses, oncolytic retrovirus, oncolytic parvovirus, oncolytic vaccinia virus, oncolytic Sinbis virus, oncolytic influenza virus, or oncolytic RNA virus (e.g., oncolytic reovirus, oncolytic Newcastle Disease Virus (NDV), oncolytic measles virus, or oncolytic vesicular stomatitis virus (VSV)).

In some embodiments, the oncolytic virus is a virus, e.g., recombinant oncolytic virus, described in US2010/0178684 A1. In some embodiments, a recombinant oncolytic virus comprises a nucleic acid sequence (e.g., heterologous nucleic acid sequence) encoding an inhibitor of an immune or inflammatory response, e.g., as described in US2010/0178684 A1 .

In embodiments, the recombinant oncolytic virus, e.g., oncolytic NDV, comprises a pro-apoptotic protein (e.g., apoptin), a cytokine (e.g., GM-CSF, interferon-gamma, interleukin-2 (IL-2), tumor necrosis factor-alpha), an immunoglobulin (e.g., an antibody against ED-B firbonectin), tumor associated antigen, a bispecific adapter protein (e.g., bispecific antibody or antibody fragment directed against NDV HN protein and a T cell co-stimulatory receptor, such as CD3 or CD28; or fusion protein between human IL-2 and single chain antibody directed against NDV HN protein). See, e.g., Zamarin et al. Future Microbiol. 7.3(2012):347-67.

In some embodiments, the oncolytic virus is a chimeric oncolytic NDV described in US 8591881 B2, US 2012/0122185 A1, or US 2014/0271677 A1 .

In some embodiments, the oncolytic virus comprises a conditionally replicative adenovirus (CRAd), which is designed to replicate exclusively in cancer cells. See, e.g., Alemany et al. Nature Biotechnol. 18(2000):723-27. In some embodiments, an oncolytic adenovirus comprises one described in Table 1 on page 725 of Alemany et al .

Exemplary oncolytic viruses include but are not limited to the following:
Group B Oncolytic Adenovirus (ColoAd1) (PsiOxus Therapeutics Ltd.) (see, e.g., Clinical Trial Identifier: NCT02053220); ONCOS-102 (previously called CGTG-102), which is an adenovirus comprising granulocyte-macrophage colony stimulating factor (GM-CSF) (Oncos Therapeutics) (see, e.g., Clinical Trial Identifier: NCT01598129); VCN-01, which is a genetically modified oncolytic human adenovirus encoding human PH20 hyaluronidase (VCN Biosciences, S.L.) (see, e.g., Clinical Trial Identifiers: NCT02045602 and NCT02045589); Conditionally Replicative Adenovirus ICOVIR-5, which is a virus derived from wild-type human adenovirus serotype 5 (Had5) that has been modified to selectively replicate in cancer cells with a deregulated retinoblastoma/E2F pathway (Institut Català d'Oncologia) (see, e.g., Clinical Trial Identifier: NCT01864759);Celyvir, which comprises bone marrow-derived autologous mesenchymal stem cells (MSCs) infected with ICOVIR5, an oncolytic adenovirus (Hospital Infantil Universitario Niño Jesús, Madrid, Spain/ Ramon Alemany) (see, e.g., Clinical Trial Identifier: NCT01844661);CG0070, which is a conditionally replicating oncolytic serotype 5 adenovirus (Ad5) in which human E2F-1 promoter drives expression of the essential E1a viral genes, thereby restricting viral replication and cytotoxicity to Rb pathway-defective tumor cells (Cold Genesys, Inc.) (see, e.g., Clinical Trial Identifier: NCT02143804); orDNX-2401 (formerly named Delta-24-RGD), which is an adenovirus that has been engineered to replicate selectively in retinoblastoma (Rb)-pathway deficient cells and to infect cells that express certain RGD-binding integrins more efficiently (Clinica Universidad de Navarra, Universidad de Navarra/ DNAtrix, Inc.) (see, e.g., Clinical Trial Identifier: NCT01956734).

In some embodiments, an oncolytic virus described herein is administering by injection, e.g., subcutaneous, intra-arterial, intravenous, intramuscular, intrathecal, or intraperitoneal injection. In embodiments, an oncolytic virus described herein is administered intratumorally, transdermally, transmucosally, orally, intranasally, or via pulmonary administration. In an embodiment, cells expressing a CAR described herein are administered to a subject in combination with a molecule that decreases the Treg cell population. Methods that decrease the number of (e.g., deplete) Treg cells are known in the art and include, e.g., CD25 depletion, cyclophosphamide administration, modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse.

In one instance, cells expressing a CAR described herein are administered to a subject in combination with a molecule targeting GITR and/or modulating GITR functions, such as a GITR agonist and/or a GITR antibody that depletes regulatory T cells (Tregs). In one embodiment, the GITR binding molecules and/or molecules modulating GITR functions (e.g., GITR agonist and/or Treg depleting GITR antibodies) are administered prior to the CAR-expressing cell. For example, in one embodiment, the GITR agonist can be administered prior to apheresis of the cells. In one embodiment, the subject has CLL. Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.:WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In one instance, a CAR expressing cell described herein is administered to a subject in combination with an mTOR inhibitor, e.g., an mTOR inhibitor described herein, e.g., a rapalog such as everolimus. In one embodiment, the mTOR inhibitor is administered prior to the CAR-expressing cell. For example, in one embodiment, the mTOR inhibitor can be administered prior to apheresis of the cells. In one embodiment, the subject has CLL.

In one instance, a CAR expressing cell described herein is administered to a subject in combination with a GITR agonist, e.g., a GITR agonist described herein. In one embodiment, the GITR agonist is administered prior to the CAR-expressing cell. For example, in one embodiment, the GITR agonist can be administered prior to apheresis of the cells. In one embodiment, the subject has CLL.

In one instance, a CAR expressing cell described herein is administered to a subject in combination with a protein tyrosine phosphatase inhibitor, e.g., a protein tyrosine phosphatase inhibitor described herein. In one embodiment, the protein tyrosine phosphatase inhibitor is an SHP-1 inhibitor, e.g., an SHP-1 inhibitor described herein, such as, e.g., sodium stibogluconate. In one embodiment, the protein tyrosine phosphatase inhibitor is an SHP-2 inhibitor.

In one instance, a CAR-expressing cell described herein can be used in combination with a kinase inhibitor. In one embodiment, the kinase inhibitor is a CDK4 inhibitor, e.g., a CDK4 inhibitor described herein, e.g., a CDK4/6 inhibitor, such as, e.g., 6-Acetyl-8-cyclopentyl-5-methyl-2-(5-piperazin-1-yl-pyridin-2-ylamino)-8*H*-pyrido[2,3-*d*]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In one embodiment, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4-*d*] pyrimidine. The MNK inhibitor can be, e.g., a MNK1a, MNK1b, MNK2a and/or MNK2b inhibitor. In one embodiment, the kinase inhibitor is a dual PI3K/mTOR inhibitor described herein, such as, e.g., PF-04695102.

In one embodiment, the kinase inhibitor is a CDK4 inhibitor selected from aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]-4*H*-1-benzopyran-4-one, hydrochloride (P276-00); 1-methyl-5-[[2-[5-(trifluoromethyl)-1*H*-imidazol-2-yl]-4-pyridinyl]oxy]-*N*-14-(trifluoromethyl)phenyl]-1*H*-benzimidazol-2-amine (RAF265); indisulam (E7070); roscovitine (CYC202); palbociclib (PD0332991); dinaciclib (SCH727965); N-[5-[[(5-*tert-*butyloxazol-2-yl)methyl]thio]thiazol-2-yl]piperidine-4-carboxamide (BMS 387032); 4-[[9-chloro-7-(2,6-difluorophenyl)-5*H*-pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1*H*-imidazol-5-yl]-*N-*[4-(methylsulfonyl)phenyl]- 2-pyrimidinamine (AZD5438); and XL281 (BMS908662).

In one embodiment, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In instances, a CAR-expressing cell described herein is administered to a subject in combination with a cyclin-dependent kinase (CDK) 4 or 6 inhibitor, e.g., a CDK4 inhibitor or a CDK6 inhibitor described herein. In instances, a CAR-expressing cell described herein is administered to a subject in combination with a CDK4/6 inhibitor (e.g., an inhibitor that targets both CDK4 and CDK6), e.g., a CDK4/6 inhibitor described herein. In an embodiment, the subject has MCL. MCL is an aggressive cancer that is poorly responsive to currently available therapies, i.e., essentially incurable. In many cases of MCL, cyclin D1 (a regulator of CDK4/6) is expressed (e.g., due to chromosomal translocation involving immunoglobulin and Cyclin D1 genes) in MCL cells. Thus, without being bound by theory, it is thought that MCL cells are highly sensitive to CDK4/6 inhibition with high specificity (i.e., minimal effect on normal immune cells). CDK4/6 inhibitors alone have had some efficacy in treating MCL, but have only achieved partial remission with a high relapse rate. An exemplary CDK4/6 inhibitor is LEE01 1 (also called ribociclib), the structure of which is shown below.

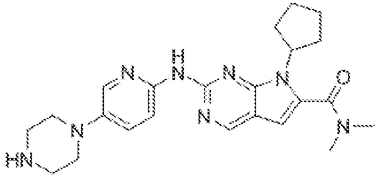

Without being bound by theory, it is believed that administration of a CAR-expressing cell described herein with a CDK4/6 inhibitor (e.g., LEE011 or other CDK4/6 inhibitor described herein) can achieve higher responsiveness, e.g., with higher remission rates and/or lower relapse rates, e.g., compared to a CDK4/6 inhibitor alone.

In one embodiment, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In a preferred embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment, the kinase inhibitor is a BTK inhibitor, e.g., ibrutinib (PCI-32765). In embodiments, a CAR-expressing cell described herein is administered to a subject in combination with a BTK inhibitor (e.g., ibrutinib). In embodiments, a CAR-expressing cell described herein is administered to a subject in combination with ibrutinib (also called PCI-32765). The structure of ibrutinib (1-[(3*R*)-3-[4-Amino-3-(4-phenoxyphenyl)-1*H-*pyrazolo[3,4-d]pyrimidin-1-yl]piperidin-1-yl]prop-2-en-1-one) is shown below.

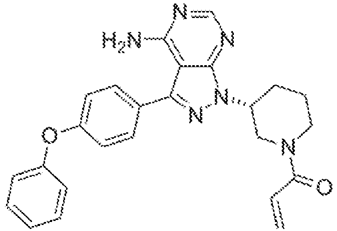

In embodiments, the subject has CLL, mantle cell lymphoma (MCL), or small lymphocytic lymphoma (SLL). For example, the subject has a deletion in the short arm of chromosome 17 (del(17p), e.g., in a leukemic cell). In other examples, the subject does not have a del(17p). In embodiments, the subject has relapsed CLL or SLL, e.g., the subject has previously been administered a cancer therapy (e.g., previously been administered one, two, three, or four prior cancer therapies). In embodiments, the subject has refractory CLL or SLL. In other embodiments, the subject has follicular lymphoma, e.g., relapse or refractory follicular lymphoma. In some embodiments, ibrutinib is administered at a dosage of about 300-600 mg/day (e.g., about 300-350, 350-400, 400-450, 450-500, 500-550, or 550-600 mg/day, e.g., about 420 mg/day or about 560 mg/day), e.g., orally. In embodiments, the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of ibrutinib are administered.

In some embodiments, ibrutinib is administered in combination with rituximab. See, e.g., Burger et al. (2013) Ibrutinib In Combination With Rituximab (iR) Is Well Tolerated and Induces a High Rate Of Durable Remissions In Patients With High-Risk Chronic Lymphocytic Leukemia (CLL): New, Updated Results Of a Phase II Trial In 40 Patients, Abstract 675 presented at 55th ASH Annual Meeting and Exposition, New Orleans, LA 7-10 Dec. Without being bound by theory, it is thought that the addition of ibrutinib enhances the T cell proliferative response and may shift T cells from a T-helper-2 (Th2) to T-helper-1 (Th1) phenotype. Th1 and Th2 are phenotypes of helper T cells, with Th1 versus Th2 directing different immune response pathways. A Th1 phenotype is associated with proinflammatory responses, e.g., for killing cells, such as intracellular pathogens/viruses or cancerous cells, or perpetuating autoimmune responses. A Th2 phenotype is associated with eosinophil accumulation and anti-inflammatory responses.

In some embodiments of the methods, uses, and compositions herein, the BTK inhibitor is a BTK inhibitor described in International Application WO/2015/079417 .

For instance, in some embodiments, the BTK inhibitor is a compound of formula (I) or a pharmaceutically acceptable salt thereof; 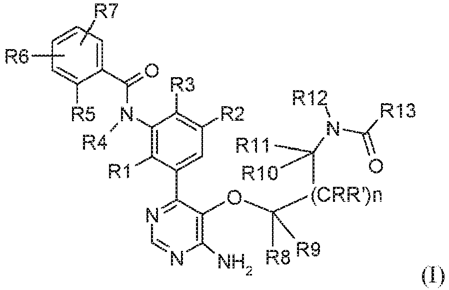 wherein,
R1 is hydrogen, C1-C6 alkyl optionally substituted by hydroxy;
R2 is hydrogen or halogen;
R3 is hydrogen or halogen;
R4 is hydrogen;
R5 is hydrogen or halogen;
or R4 and R5 are attached to each other and stand for a bond, -CH2-, -CH2-CH2- , - CH=CH-, -CH=CH-CH2-; -CH2-CH=CH-; or -CH2-CH2-CH2-;
R6 and R7 stand independently from each other for H, C1-C6 alkyl optionally substituted by hydroxyl, C3-C6 cycloalkyl optionally substituted by halogen or hydroxy, or halogen;
R8, R9, R, R', R10 and R11 independently from each other stand for H, or C1-C6 alkyl optionally substituted by C1-C6 alkoxy; or any two of R8, R9, R, R', R10 and R11 together with the carbon atom to which they are bound may form a 3 - 6 membered saturated carbocyclic ring;
R12 is hydrogen or C1-C6 alkyl optionally substituted by halogen or C1-C6 alkoxy;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl, C1-C6 alkoxy or N,N-di-C1-C6 alkyl amino; C2-C6 alkynyl optionally substituted by C1-C6 alkyl or C1-C6 alkoxy; or C2-C6 alkylenyl oxide optionally substituted by C1-C6 alkyl.

In some embodiments, the BTK inhibitor of Formula I is chosen from: N-(3-(5-((1-Acryloylazetidin-3-yl)oxy)-6-aminopyrimidin-4-yl)-5-fluoro-2-methylphenyl)-4-cyclopropyl-2-fluorobenzamide; (E)-N-(3-(6-Amino-5-((1-(but-2-enoyl)azetidin-3-yl)oxy)pyrimidin-4-yl)-5-fluoro-2-methylphenyl)-4-cyclopropyl-2-fluorobenzamide; N-(3-(6-Amino-5-((1-propioloylazetidin-3-yl)oxy)pyrimidin-4-yl)-5-fluoro-2-methylphenyl)-4-cyclopropyl-2-fluorobenzamide; N-(3-(6-Amino-5-((1-(but-2-ynoyl)azetidin-3-yl)oxy)pyrimidin-4-yl)-5-fluoro-2-methylphenyl)-4-cyclopropyl-2-fluorobenzamide; N-(3-(5-((1-Acryloylpiperidin-4-yl)oxy)-6-aminopyrimidin-4-yl)-5-fluoro-2-methylphenyl)-4-cyclopropyl-2-fluorobenzamide; N-(3-(6-Amino-5-(2-(N-methylacrylamido)ethoxy)pyrimidin-4-yl)-5-fluoro-2-methylphenyl)-4-cyclopropyl-2-fluorobenzamide; (E)-N-(3-(6-Amino-5-(2-(N-methylbut-2-enamido)ethoxy)pyrimidin-4-yl)-5-fluoro-2-methylphenyl}-4-cyclopropyl-2-fluorobenzamide; N-(3-(6-Amino-5-(2-(N-methylpropiolamido)ethoxy)pyrimidin-4-yl)-5-fluoro-2-methylphenyl)-4-cyclopropyl-2-fluorobenzamide; (E)-N-(3-(6-Amino-5-(2-(4-methoxy-N-methylbut-2-enamido)ethoxy)pyrimidin-4-yl)-5-fluoro-2-methylphenyl)-4-cyclopropyl-2-fluorobenzamide; N-(3-(6-Amino-5-(2-(N-methylbut-2-ynamido)ethoxy)pyrimidin-4-yl)-5-fluoro-2-methylphenyl)-4-cyclopropyl-2-fluorobenzamide; N-(2-((4-Amino-6-(3-(4-cyclopropyl-2-fluorobenzamido)-5-fluoro-2-methylphenyl)pyrimidin-5-yl)oxy)ethyl)-N-methyloxirane-2-carboxamide; N-(2-((4-Amino-6-(3-(6-cyclopropyl-8-fluoro-1-oxoisoquinolin-2(1H)-yl)phenyl)pyrimidin-5-yl)oxy)ethyl)-N-methylacrylamide; N-(3-(5-(2-Acrylamidoethoxy)-6-aminopyrimidin-4-yl)-5-fluoro-2-methylphenyl)-4-cyclopropyl-2-fluorobenzamide; N-(3-(6-Amino-5-(2-(N-ethylacrylamido)ethoxy)pyrimidin-4-yl)-5-fluoro-2-methylphenyl)-4-cyclopropyl-2-fluorobenzamide; N-(3-(6-Amino-5-(2-(N-(2-fluoroethyl)acrylamido)ethoxy)pyrimidin-4-yl)-5-fluoro-2-methylphenyl)-4--cyclopropyl-2-fluorobenzamide; N-(3-(5-((1-Acrylamidocyclopropyl)methoxy)-6-aminopyrimidin-4-yl)-5-fluoro-2-methylphenyl)-4-cyclopropyl-2-fluorobenzamide; (S)-N-(3-(5-(2-Acrylamidopropoxy)-6-aminopyrimidin-4-yl)-5-fluoro-2-methylphenyl)-4-cyclopropyl-2-fluorobenzamide; (S)-N-(3-(6-Amino-5-(2-(but-2-ynamido)propoxy)pyrimiclin-4-yl)-5-fluoro-2-methylphenyl)-4-cyclopropyl-2-fluorobenzamide; (S)-N-(3-(6-Amino-5-(2-(N-methylacrylamido)propoxy)pyrimidin-4-yl)-5-fluoro-2-methylphenyl)-4-cyclopropyl-2-fluorobenzamide; (S)-N-(3-(6-Amino-5-(2-(N-methylbut-2-ynamido)propoxy)pyrimidin-4-yl)-5-fluoro-2-methylphenyl)-4-cyclopropyl-2-fluorobenzamide; N-(3-(6-Amino-5-(3-(N-methylacrylamido)propoxy)pyrimidin--4-yl)-5-fluoro-2-methylphenyl)-4-cyclopropyl-2-fluorobenzamide; (S)-N-(3-(5-((1-Acryloylpyrrolidin-2-yl)methoxy)-6-aminopyrimidin-4-yl)-5-fluoro-2-methylphenyl)-4-cyclopropyl-2-fluorobenzamide; (S)-N-(3-(6-Amino-5-((1-(but-2-ynoyl)pyrrolidin-2-yl)methoxy)pyrimidin-4-yl)-5-fluoro-2-methylphenyl)-4-cyclopropyl-2-fluorobenzamide; (S)-2-(3-(5-((1-Acryloylpyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one; N-(2-((4-Amino-6-(3-(6-cyc1opropyl-l-oxo-3,4-dihydroisoquinolin-2(1H)-y1)-5-fluoro-2-(hydroxymethyl)phenyl)pyrimidin-5-yl)oxy)ethyl)-N-methylacrylamide; N-(3-(5-(((2S,4R)-1-Acryloyl-4-methoxypyrrolidin-2-yl)methoxy)-6-aminopyrimidin-4-yl)-5-fluoro-2-methylphenyl)-4-cyclopropyl-2-fluorobenzamide; N-(3-(6-Amino-5-(((2S,4R)-1-(but-2-ynoyl)-4-methoxypyrrolidin-2-yl)methoxy)pyrimidin-4-yl)-5-fluoro-2-methylphenyl)-4-cyclopropyl-2-fluorobenzamide; 2-(3-(5-(((2S,4R)-1-Acryloyl-4-methoxypyrrolidin-2-ylymethoxy)-6-aminopyrimidin-4-yl)-5-fluoro-2-(hydroxymethyl)phenyl)-6-cyclopropyl-3,4-dihydroisoquinolin-1(2H)-one; N-(3-(5-(((2S,4S)-1-Acryloyl-4-methoxypyrrolidin-2-yl)methoxy)-6-aminopyrimidin-4-yl)-5-fluoro-2-methylphenyl)-4-cyclopropyl-2-fluorobenzamide; N-(3-(6-Amino-5-(((2S,4S)-1-(but-2-ynoyl)-4-methoxypyrrolidin-2-yl)methoxy)pyrimidin-4-yl)-5-fluoro-2-methylphenyl)-4-cyclopropyl-2-fluorobenzamide; N-(3-(5-(((2S,4R)-1-Acryloyl-4-fluoropyrrolidin-2-yl)methoxy)-6-aminopyrimidin-4-yl)-5-fluoro-2-methylphenyl)-4-cyclopropyl-2-fluorobenzamide; N-(3-(6-Amino-5-(((2S,4R)-1-(but-2-ynoyl)-4-fluoropyrrolidin-2-yl)methoxy)pyrimidin-4-yl)-5-fluoro-2-methylphenyl)-4-cyclopropyl-2-fluorobenzamide; (S)-N-(3-(5-((1-Acryloylazetidin-2-yl)methoxy)-6-aminopyrimidin-4-yl)-5-fluoro-2-methylphenyl)-4-cyclopropyl-2-fluorobenzamide; (S)-N-(3-(6-Amino-5-((1-propioloylazetidin-2-yl)methoxy)pyrimidin-4-yl)-5-fluoro-2-methylphenyl)-4-cyclopropyl-2-fluorobenzamide; (S)-2-(3-(5-((1-Acryloylazetidin-2-yl)methoxy)-6-aminopyrimidin-4-yl)-5-fluoro-2-(hydroxymethyl)phenyl)-6-cyclopropyl-3,4-dihydroisoquinolin-1(2H)-one; (R)-N-(3-(5-((1-Acryloylazetidin-2-yl)methoxy)-6-aminopyrimidin-4-yl)-5-fluoro-2-methylphenyl)-4-cyclopropyl-2-fluorobenzamide; (R)-N-(3-(5-((1-Acryloylpiperidin-3-yl)methoxy)-6-aminopyrimidin-4-yl)-5-fluoro-2-methylphenyl)-4-cyclopropyl-2-fluorobenzamide; N-(3-(5-(((2R,3S)-1-Acryloyl-3-methoxypyrrolidin-2-yl)methoxy)-6-aminopyrimidin-4-yl)-5-fluoro-2-methylphenyl)-4-cyclopropyl-2-fluorobenzamide; N-(3-(5-(((2S,4R)-1-Acryloyl-4-cyanopyrrolidin-2-yl)methoxy)-6-aminopyrimidin-4-yl)-5-fluoro-2-methylphenyl)-4-cyclopropyl-2-fluorobenzamide; or N-(3-(5-(((2S,4S)-1-Acryloyl-4-cyanopyrrolidin-2-yl)methoxy)-6-aminopyrimidin-4-y1)-5-fluoro-2-methylphenyl)-4-cyclopropyl-2-fluorobenzamide.

Unless otherwise provided, the chemical terms used above in describing the BTK inhibitor of Formula I are used according to their meanings as set out in International Application WO/2015/079417, which is herein incorporated by reference in its entirety.

In one embodiment, the kinase inhibitor is an mTOR inhibitor selected from temsirolimus; ridaforolimus (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23,29,35-hexamethyl-2,3,10,14,20-pentaoxo-11,36-dioxa-4-azatricyclo[30.3.1.0^{4,9}]hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3*S*)-3-methylmorpholin-4-yl]pyrido[2,3-*d*]pyrimidin-7-yl}-2-methoxyphenyl)methanol (AZD8055); 2-amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502); and -*N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine- (SEQ ID NO: 264), inner salt (SF1126); and XL765.

In one embodiment, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4*-d*] pyrimidine.

In one embodiment, the kinase inhibitor is a dual phosphatidylinositol 3-kinase (PI3K) and mTOR inhibitor selected from 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF-04691502); *N*-[4-[[4-(Dimethylamino)-1-piperidinyl]carbonyl]phenyl]-*N'*-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In one embodiment, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-*d*] pyrimidine.

In instances, a CAR-expressing cell described herein is administered to a subject in combination with a phosphoinositide 3-kinase (PI3K) inhibitor (e.g., a PI3K inhibitor described herein, e.g., idelalisib or duvelisib) and/or rituximab. In embodiments, a CAR-expressing cell described herein is administered to a subject in combination with idelalisib and rituximab. In embodiments, a CAR-expressing cell described herein is administered to a subject in combination with duvelisib and rituximab. Idelalisib (also called GS-1101 or CAL-101; Gilead) is a small molecule that blocks the delta isoform of PI3K. The structure of idelalisib (5-Fluoro-3-phenyl-2-[(1*S*)-1-(7*H*-purin-6-ylamino)propyl]-4(3*H*)-quinazolinone) is shown below.

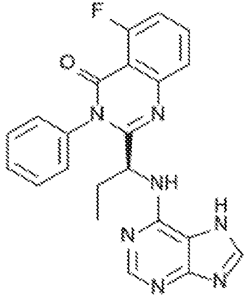

Duvelisib (also called IPI-145; Infinity Pharmaceuticals and Abbvie) is a small molecule that blocks PI3K-δ,γ. The structure of duvelisib (8-Chloro-2-phenyl-3-[(1S)-1-(9H-purin-6-ylamino)ethyl]-1(2H)-isoquinolinone) is shown below.

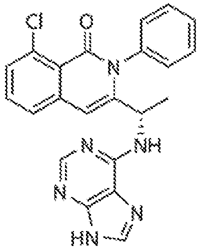

In embodiments, the subject has CLL. In embodiments, the subject has relapsed CLL, e.g., the subject has previously been administered a cancer therapy (e.g., previously been administered an anti-CD20 antibody or previously been administered ibrutinib). For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*IgV_{H}*) gene. In other embodiments, the subject does not comprise a leukemic cell comprising a mutation in the immunoglobulin heavy-chain variable-region (*IgV_{H}*) gene. In embodiments, the subject has a deletion in the long arm of chromosome 11 (del(11q)). In other embodiments, the subject does not have a del(11q). In embodiments, idelalisib is administered at a dosage of about 100-400 mg (e.g., 100-125, 125-150, 150-175, 175-200, 200-225, 225-250, 250-275, 275-300, 325-350, 350-375, or 375-400 mg), e.g., BID. In embodiments, duvelisib is administered at a dosage of about 15-100 mg (e.g., about 15-25, 25-50, 50-75, or 75-100 mg), e.g., twice a day. In embodiments, rituximab is administered at a dosage of about 350-550 mg/m² (e.g., 350-375, 375-400, 400-425, 425-450, 450-475, or 475-500 mg/m²), e.g., intravenously.

In one embodiment, the kinase inhibitor is a dual phosphatidylinositol 3-kinase (PI3K) and mTOR inhibitor selected from 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF-04691502); *N*-[4-[[4-(Dimethylamino)-1-piperidinyl]carbonyl]phenyl]-*N*'-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl) benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In instances, a CAR-expressing cell described herein is administered to a subject in combination with an anaplastic lymphoma kinase (ALK) inhibitor. Exemplary ALK kinases include but are not limited to crizotinib (Pfizer), ceritinib (Novartis), alectinib (Chugai), brigatinib (also called *AP26113*; Ariad), entrectinib (Ignyta), PF-064-63922 (Pfizer), TSR-011 (Tesaro) (see, e.g., Clinical Trial Identifier No. NCT02048488), CEP-37440 (Teva), and X-396 (Xcovery). In some embodiments, the subject has a solid cancer, e.g., a solid cancer described herein, e.g., lung cancer.

The chemical name of crizotinib is 3-[(1*R*)-1-(2,6-dichloro-3-fluorophenyl)ethoxy]-5-(1-piperidin-4-ylpyrazol-4-yl)pyridin-2-amine. The chemical name of ceritinib is 5-Chloro-*N*²-[2-isopropoxy-5-methyl-4-(4-piperidinyl)phenyl]-*N*⁴-[2-(isopropylsulfonyl)phenyl]-2,4-pyrimidinediamine. The chemical name of alectinib is 9-ethyl-6,6-dimethyl-8-(4-morpholinopiperidin-1-yl)-11-oxo-6,11-dihydro-5H-benzo[b]carbazole-3-carbonitrile. The chemical name of brigatinib is 5-Chloro-N²-{4-[4-(dimethylamino)-1-piperidinyl]-2-methoxyphenyl}-N⁴-[2-(dimethylplzosphoryl)phenyl]-2,4-pyrimidinediamine. The chemical name of entrectinib is N-(5-(3,5-difluorobenzyl)-1H-indazol-3-yl)--4-(-4-methylpiperazin-1-yl)-2-((tetralzydro-2H-pyran-4-yl)amino)benzamide. The chemical name of PF-06463922 is (10R)-7-Amino-12-fluoro-2,10,16-trimethy1-15-oxo-10,15,16,17-tetrahydro-2H-8,4-(metheno)pyrazolo[4,3-h][2,5,11]-benzoxadiazacyclotetradecine-3-carbonitrile. The chemical structure of CEP-37440 is (S)-2-((5-chloro-2-((6-(4-(2-hydroxyethyl)piperazin-1-yl)-1-methoxy-6,7,8,9-tetrahydro-5H-benzo[7]annulen-2-yl)amino)pyrimidin-4-yl)amino)-N-methylbenzamide. The chemical name of X-396 is (R)-6-amino-5-(1-(2,6-dichloro-3-fluorophenyl)ethoxy)-N-(4-(4-methylpiperazine-1-carbonyl)phenyl)pyridazine-3-carboxamide.

Drugs that inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993) can also be used. In a further aspect, the cell compositions of the present disclosure may be administered to a patient in conjunction with (e.g., before, simultaneously or following) bone marrow transplantation, T cell ablative therapy using chemotherapy agents such as, fludarabine, external-beam radiation therapy (XRT), cyclophosphamide, and/or antibodies such as OKT3 or CAMPATH. In one aspect, the cell compositions of the present disclosure are administered following B-cell ablative therapy such as agents that react with CD20, e.g., Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present disclosure. In an additional embodiment, expanded cells are administered before or following surgery.

In instances, a CAR-expressing cell described herein is administered to a subject in combination with an indoleamine 2,3-dioxygenase (IDO) inhibitor. IDO is an enzyme that catalyzes the degradation of the amino acid, L-tryptophan, to kynurenine. Many cancers overexpress IDO, e.g., prostatic, colorectal, pancreatic, cervical, gastric, ovarian, head, and lung cancer. pDCs, macrophages, and dendritic cells (DCs) can express IDO. Without being bound by theory, it is thought that a decrease in L-tryptophan (e.g., catalyzed by IDO) results in an immunosuppressive milieu by inducing T-cell anergy and apoptosis. Thus, without being bound by theory, it is thought that an IDO inhibitor can enhance the efficacy of a CAR-expressing cell described herein, e.g., by decreasing the suppression or death of a CAR-expressing immune cell. In embodiments, the subject has a solid tumor, e.g., a solid tumor described herein, e.g., prostatic, colorectal, pancreatic, cervical, gastric, ovarian, head, or lung cancer. Exemplary inhibitors of IDO include but are not limited to 1-methyl-tryptophan, indoximod (NewLink Genetics) (see, e.g., Clinical Trial Identifier Nos. NCT01191216; NCT01792050), and INCB024360 (Incyte Corp.) (see, e.g., Clinical Trial Identifier Nos. NCT01604889; NCT01685255)

In instances, a CAR-expressing cell described herein is administered to a subject in combination with a modulator of myeloid-derived suppressor cells (MDSCs). MDSCs accumulate in the periphery and at the tumor site of many solid tumors. These cells suppress T cell responses, thereby hindering the efficacy of CAR-expressing cell therapy. Without being bound by theory, it is thought that administration of a MDSC modulator enhances the efficacy of a CAR-expressing cell described herein. In an embodiment, the subject has a solid tumor, e.g., a solid tumor described herein, e.g., glioblastoma. Exemplary modulators of MDSCs include but are not limited to MCS110 and BLZ945. MCS110 is a monoclonal antibody (mAb) against macrophage colony-stimulating factor (M-CSF). See, e.g., Clinical Trial Identifier No. NCT00757757. BLZ945 is a small molecule inhibitor of colony stimulating factor 1 receptor (CSF1R). See, e.g., Pyonteck et al. Nat. Med. 19(2013):1264-72. The structure of BLZ945 is shown below.

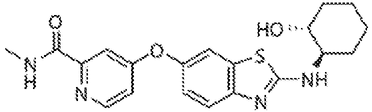

In instances, a CAR-expressing cell described herein is administered to a subject in combination with an agent that inhibits or reduces the activity of immunosuppressive plasma cells. Immunosuppressive plasma cells have been shown to impede T cell-dependent immunogenic chemotherapy, such as oxaliplatin (Shalapour et al., Nature 2015, 521:94- 101). In an embodiment, immunosuppressive plasma cells can express one or more of IgA, interleukin (IL)-10, and PD-L1. In an embodiment, the agent is a CD19 CAR-expressing cell or a BCMA CAR-expressing cell.

In some instances, a CAR-expressing cell described herein is administered to a subject in combination with a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15 (Admune Therapeutics, LLC). hetIL-15 is a heterodimeric noncovalent complex of IL-15 and IL-15Ra. hetIL-15 is described in, e.g., U.S. 8,124,084, U.S. 2012/0177598, U.S. 2009/0082299, U.S. 2012/0141413, and U.S. 2011/0081311 .

In embodiments, het-IL-15 is administered subcutaneously. In embodiments, the subject has a cancer, e.g., solid cancer, e.g., melanoma or colon cancer. In embodiments, the subject has a metastatic cancer.

In instances, a subject having a disease described herein, e.g., a hematological disorder, e.g., AML or MDS, is administered a CAR-expressing cell described herein in combination with an agent, e.g., cytotoxic or chemotherapy agent, a biologic therapy (e.g., antibody, e.g., monoclonal antibody, or cellular therapy), or an inhibitor (e.g., kinase inhibitor). In instances, the subject is administered a CAR-expressing cell described herein in combination with a cytotoxic agent, e.g., CPX-351 (Celator Pharmaceuticals), cytarabine, daunorubicin, vosaroxin (Sunesis Pharmaceuticals), sapacitabine (Cyclacel Pharmaceuticals), idarubicin, or mitoxantrone. CPX-351 is a liposomal formulation comprising cytarabine and daunorubicin at a 5:1 molar ratio. In instances, the subject is administered a CAR-expressing cell described herein in combination with a hypomethylating agent, e.g., a DNA methyltransferase inhibitor, e.g., azacitidine or decitabine. In instances, the subject is administered a CAR-expressing cell described herein in combination with a biologic therapy, e.g., an antibody or cellular therapy, e.g., 225Ac-lintuzumab (Actimab-A; Actinium Pharmaceuticals), IPH2102 (Innate Pharma/Bristol Myers Squibb), SGN-CD33A (Seattle Genetics), or gemtuzumab ozogamicin (Mylotarg; Pfizer). SGN-CD33A is an antibody-drug conjugate (ADC) comprising a pyrrolobenzodiazepine dimer that is attached to an anti-CD33 antibody. Actimab-A is an anti-CD33 antibody (lintuzumab) labeled with actinium. IPH2102 is a monoclonal antibody that targets killer immunoglobulin-like receptors (KIRs). In instances, the subject is administered a CAR-expressing cell described herein in combination a FLT3 inhibitor, e.g., sorafenib (Bayer), midostaurin (Novartis), quizartinib (Daiichi Sankyo), crenolanib (Arog Pharmaceuticals), PLX3397 (Daiichi Sankyo), AKN-028 (Akinion Pharmaceuticals), or ASP2215 (Astellas). In instances, the subject is administered a CAR-expressing cell described herein in combination with an isocitrate dehydrogenase (IDH) inhibitor, e.g., AG-221 (Celgene/Agios) or AG-120 (Agios/Celgene). In instances, the subject is administered a CAR-expressing cell described herein in combination with a cell cycle regulator, e.g., inhibitor of polo-like kinase 1 (Plk1), e.g., volasertib (Boehringer Ingelheim); or an inhibitor of cyclin-dependent kinase 9 (Cdk9), e.g., alvocidib (Tolero Pharmaceuticals/Sanofi Aventis). In instances, the subject is administered a CAR-expressing cell described herein in combination with a B cell receptor signaling network inhibitor, e.g., an inihibitor of B-cell lymphoma 2 (Bcl-2), e.g., venetoclax (Abbvie/Roche); or an inhibitor of Bruton's tyrosine kinase (Btk), e.g., ibrutinib (Pharmacyclics/Johnson & Johnson Janssen Pharmaceutical). In instances, the subject is administered a CAR-expressing cell described herein in combination with an inhibitor of M1 aminopeptidase, e.g., tosedostat (CTI BioPharma/Vernalis); an inhibitor of histone deacetylase (HDAC), e.g., pracinostat (MEI Pharma); a multi-kinase inhibitor, e.g., rigosertib (Onconova Therapeutics/Baxter/SymBio); or a peptidic CXCR4 inverse agonist, e.g., BL-8040 (BioLineRx).

In another embodiment, the subjects receive an infusion of the CAR-expressing cell of the invention, or compositions of the present invention, prior to transplantation, e.g., allogeneic stem cell transplant, of cells. In a preferred embodiment, CAR expressing cells transiently express BCA CAR and/or TA CAR, e.g., by electroporation of an mRNA encoding a BCA CAR and/or TA CAR, whereby the expression of either or both CARs is terminated prior to infusion of donor stem cells to avoid engraftment failure.

Some patients may experience allergic reactions to the compounds of the present disclosure and/or other anti-cancer agent(s) during or after administration; therefore, anti-allergic agents are often administered to minimize the risk of an allergic reaction. Suitable anti-allergic agents include corticosteroids, such as dexamethasone (e.g., Decadron^{®}), beclomethasone (e.g., Beclovent^{®}), hydrocortisone (also known as cortisone, hydrocortisone sodium succinate, hydrocortisone sodium phosphate, and sold under the tradenames Ala-Cort^{®}, hydrocortisone phosphate, Solu-Cortef^{®}, Hydrocort Acetate^{®} and Lanacort^{®}), prednisolone (sold under the tradenames Delta-Cortel^{®}, Orapred^{®}, Pediapred^{®} and Prelone^{®}), prednisone (sold under the tradenames Deltasone^{®}, Liquid Red^{®}, Meticorten^{®} and Orasone^{®}), methylprednisolone (also known as 6-methylprednisolone, methylprednisolone acetate, methylprednisolone sodium succinate, sold under the tradenames Duralone^{®}, Medralone^{®}, Medrol^{®}, M-Prednisol^{®} and Solu-Medrol^{®}); antihistamines, such as diphenhydramine (e.g., Benadryl^{®}), hydroxyzine, and cyproheptadine; and bronchodilators, such as the beta-adrenergic receptor agonists, albuterol (e.g., Proventil^{®}), and terbutaline (Brethine^{®}). Some patients may experience nausea during and after administration of the compound of the present disclosure and/or other anti-cancer agent(s); therefore, anti-emetics are used in preventing nausea (upper stomach) and vomiting. Suitable anti-emetics include aprepitant (Emend^{®}), ondansetron (Zofran^{®}), granisetron HCl (Kytril^{®}), lorazepam (Ativan^{®}. dexamethasone (Decadron^{®}), prochlorperazine (Compazine^{®}), casopitant (Rezonic^{®} and Zunrisa^{®}), and combinations thereof.

Medication to alleviate the pain experienced during the treatment period is often prescribed to make the patient more comfortable. Common over-the-counter analgesics, such Tylenol^{®}, are often used. However, opioid analgesic drugs such as hydrocodone/paracetamol or hydrocodone/acetaminophen (e.g., Vicodin^{®}), morphine (e.g., Astramorph^{®} or Avinza^{®}), oxycodone (e.g., OxyContin^{®} or Percocet^{®}), oxymorphone hydrochloride (Opana^{®}), and fentanyl (e.g., Duragesic^{®}) are also useful for moderate or severe pain.

In an effort to protect normal cells from treatment toxicity and to limit organ toxicities, cytoprotective agents (such as neuroprotectants, free-radical scavengers, cardioprotectors, anthracycline extravasation neutralizers, nutrients and the like) may be used as an adjunct therapy. Suitable cytoprotective agents include Amifostine (Ethyol^{®}), glutamine, dimesna (Tavocept^{®}), mesna (Mesnex^{®}), dexrazoxane (Zinecard^{®} or Totect^{®}), xaliproden (Xaprila^{®}), and leucovorin (also known as calcium leucovorin, citrovorum factor and folinic acid). The structure of the active compounds identified by code numbers, generic or trade names may be taken from the actual edition of the standard compendium "The Merck Index" or from databases, e.g. Patents International (e.g. IMS World Publications).

The above-mentioned compounds, which can be used in combination with a compound of the present disclosure, can be prepared and administered as described in the art, such as in the documents cited above.

In one instance, the present disclosure provides pharmaceutical compositions comprising at least one compound of the present disclosure (e.g., a compound of the present disclosure) or a pharmaceutically acceptable salt thereof together with a pharmaceutically acceptable carrier suitable for administration to a human or animal subject, either alone or together with other anti-cancer agents.

In one instance, the present disclosure provides for treating human or animal subjects suffering from a cellular proliferative disease, such as cancer. The present disclosure provides for treating a human or animal subject in need of such treatment, comprising administering to the subject a therapeutically effective amount of a compound of the present disclosure (e.g., a compound of the present disclosure) or a pharmaceutically acceptable salt thereof, either alone or in combination with other anti-cancer agents.

In particular, compositions will either be formulated together as a combination therapeutic or administered separately.

In combination therapy, the compound of the present disclosure and other anti-cancer agent(s) may be administered either simultaneously, concurrently or sequentially with no specific time limits, wherein such administration provides therapeutically effective levels of the two compounds in the body of the patient.

In a preferred embodiment, the compound of the present disclosure and the other anti-cancer agent(s) is generally administered sequentially in any order by infusion or orally. The dosing regimen may vary depending upon the stage of the disease, physical fitness of the patient, safety profiles of the individual drugs, and tolerance of the individual drugs, as well as other criteria well-known to the attending physician and medical practitioner(s) administering the combination. The compound of the present disclosure and other anti-cancer agent(s) may be administered within minutes of each other, hours, days, or even weeks apart depending upon the particular cycle being used for treatment. In addition, the cycle could include administration of one drug more often than the other during the treatment cycle and at different doses per administration of the drug.

In another aspect of the present disclosure, kits that include one or more compound of the present disclosure and a combination partner as disclosed herein are provided. Representative kits include (a) a compound of the present disclosure or a pharmaceutically acceptable salt thereof, (b) at least one combination partner, e.g., as indicated above, whereby such kit may comprise a package insert or other labeling including directions for administration.

A compound of the present disclosure may also be used to advantage in combination with known therapeutic processes, for example, the administration of hormones or especially radiation. A compound of the present disclosure may in particular be used as a radiosensitizer, especially for the treatment of tumors which exhibit poor sensitivity to radiotherapy. In one embodiment, the subject can be administered an agent which reduces or ameliorates a side effect associated with the administration of a CAR-expressing cell. Side effects associated with the administration of a CAR-expressing cell include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. CRS may include clinical constitutional signs and symptoms such as fever, fatigue, anorexia, myalgias, arthalgias, nausea, vomiting, and headache. CRS may include clinical skin signs and symptoms such as rash. CRS may include clinical gastrointestinal signs and symsptoms such as nausea, vomiting and diarrhea. CRS may include clinical respiratory signs and symptoms such as tachypnea and hypoxemia. CRS may include clinical cardiovascular signs and symptoms such as tachycardia, widened pulse pressure, hypotension, increased cardac output (early) and potentially diminished cardiac output (late). CRS may include clinical coagulation signs and symptoms such as elevated d-dimer, hypofibrinogenemia with or without bleeding. CRS may include clinical renal signs and symptoms such as azotemia. CRS may include clinical hepatic signs and symptoms such as transaminitis and hyperbilirubinemia. CRS may include clinical neurologic signs and symptoms such as headache, mental status changes, confusion, delirium, word finding difficulty or frank aphasia, hallucinations, tremor, dymetria, altered gait, and seizures.

Accordingly, the methods described herein can comprise administering a CAR-expressing cell described herein to a subject and further administering one or more agents to manage elevated levels of a soluble factor resulting from treatment with a CAR-expressing cell. In one embodiment, the soluble factor elevated in the subject is one or more of IFN-y, TNFα, IL-2 and IL-6. In an embodiment, the factor elevated in the subject is one or more of IL-1, GM-CSF, IL-10, IL-8, IL-5 and fraktalkine. Therefore, an agent administered to treat this side effect can be an agent that neutralizes one or more of these soluble factors. In one embodiment, the agent that neutralizes one or more of these soluble forms is an antibody or antigen binding fragment thereof. Examples of such agents include, but are not limited to a steroid (e.g., corticosteroid), an inhibitor of TNFα, and an inhibitor of IL-6. An example of a TNFα inhibitor is an anti-TNFα antibody molecule such as, infliximab, adalimumab, certolizumab pegol, and golimumab. Another example of a TNFα inhibitor is a fusion protein such as entanercept. Small molecule inhibitor of TNFα include, but are not limited to, xanthine derivatives (e.g. pentoxifylline) and bupropion. An example of an IL-6 inhibitor is an anti-IL-6 antibody molecule such as tocilizumab (toc), sarilumab, elsilimomab, CNTO 328, ALD518/BMS-945429, CNTO 136, CPSI-2364, CDP6038, VX30, ARGX-109, FE301, and FM101. In one embodiment, the anti-IL-6 antibody molecule is tocilizumab. An example of an IL-1R based inhibitor is anakinra.

In some embodiment, the subject is administered a corticosteroid, such as, e.g., methylprednisolone, hydrocortisone, among others.

In some embodiments, the subject is administered a vasopressor, such as, e.g., norepinephrine, dopamine, phenylephrine, epinephrine, vasopressin, or a combination thereof.

In an embodiment, the subject can be administered an antipyretic agent. In an embodiment, the subject can be administered an analgesic agent.

In one embodiment, the subject can be administered an agent which enhances the activity or fitness of a CAR-expressing cell. For example, in one embodiment, the agent can be an agent which inhibits a molecule that modulates or regulates, e.g., inhibits, T cell function. In some embodiments, the molecule that modulates or regulates T cell function is an inhibitory molecule. Inhibitory molecules, e.g., Programmed Death 1 (PD-1), can, in some embodiments, decrease the ability of a CAR-expressing cell to mount an immune effector response. Examples of inhibitory molecules include PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta. Inhibition of a molecule that modulates or regulates, e.g., inhibits, T cell function,e.g., by inhibition at the DNA, RNA or protein level, can optimize a CAR-expressing cell performance. In embodiments, an agent, e.g., an inhibitory nucleic acid, e.g., an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used to inhibit expression of an inhibitory molecule in the CAR-expressing cell. In an embodiment, the inhibitor is an shRNA.

In an embodiment, the agent that modulates or regulates, e.g., inhibits, T-cell function is inhibited within a CAR-expressing cell. In these embodiments, a dsRNA molecule that inhibits expression of a molecule that modulates or regulates, e.g., inhibits, T-cell function is linked to the nucleic acid that encodes a component, e.g., all of the components, of the CAR. In an embodiment, a nucleic acid molecule that encodes a dsRNA molecule that inhibits expression of the molecule that modulates or regulates, e.g., inhibits, T-cell function is operably linked to a promoter, e.g., a H1- or a U6-derived promoter such that the dsRNA molecule that inhibits expression of the molecule that modulates or regulates, e.g., inhibits, T-cell function is expressed, e.g., is expressed within a CAR-expressing cell. See e.g., Tiscornia. G., "Development of Lentiviral Vectors Expressing siRNA," Chapter 3, in Gene Transfer: Delivery and Expression of DNA and RNA (eds. Friedmann and Rossi). Cold Spring Harbor Laboratory Press, Cold Spring Harbor, NY, USA, 2007; Brummelkamp TR, et al. (2002) Science 296: 550-553; Miyagishi M, et al. (2002) Nat. Biotechnol. 19: 497-500. In an embodiment the nucleic acid molecule that encodes a dsRNA molecule that inhibits expression of the molecule that modulates or regulates, e.g., inhibits, T-cell function is present on the same vector, e.g., a lentiviral vector, that comprises a nucleic acid molecule that encodes a component, e.g., all of the components, of the CAR. In such an embodiment, the nucleic acid molecule that encodes a dsRNA molecule that inhibits expression of the molecule that modulates or regulates, e.g., inhibits, T-cell function is located on the vector, e.g., the lentiviral vector, 5'- or 3' - to the nucleic acid that encodes a component, e.g., all of the components, of the CAR. The nucleic acid molecule that encodes a dsRNA molecule that inhibits expression of the molecule that modulates or regulates, e.g., inhibits, T-cell function can be transcribed in the same or different direction as the nucleic acid that encodes a component, e.g., all of the components, of the CAR. In an embodiment the nucleic acid molecule that encodes a dsRNA molecule that inhibits expression of the molecule that modulates or regulates, e.g., inhibits, T-cell function is present on a vector other than the vector that comprises a nucleic acid molecule that encodes a component, e.g., all of the components, of the CAR. In an embodiment, the nucleic acid molecule that encodes a dsRNA molecule that inhibits expression of the molecule that modulates or regulates, e.g., inhibits, T-cell function it transiently expressed within a CAR-expressing cell. In an embodiment, the nucleic acid molecule that encodes a dsRNA molecule that inhibits expression of the molecule that modulates or regulates, e.g., inhibits, T-cell function is stably integrated into the genome of a CAR-expressing cell. Configurations of exemplary vectors for expressing a component, e.g., all of the components, of the CAR with a dsRNA molecule that inhibits expression of the molecule that modulates or regulates, e.g., inhibits, T-cell function, is provided, e.g., in Figure 47 of International Publication WO2015/090230, filed December 19, 2014 .
Examples of dsRNA molecules useful for inhibiting expression of a molecule that modulates or regulates, e.g., inhibits, T-cell function, wherein the molecule that modulates or regulates, e.g., inhibits, T-cell function is PD-1 include RNAi agents that target PD-1, as described, e.g., in paragraph [00489] and Tables 16 and 17 of International Publication WO2015/090230, filed December 19, 2014 .

In one embodiment, the agent that modulates or regulates, e.g., inhibits, T-cell function can be, e.g., an antibody or antibody fragment that binds to an inhibitory molecule. For example, the agent can be an antibody or antibody fragment that binds to PD-1, PD-L1, PD-L2 or CTLA4 (e.g., ipilimumab (also referred to as MDX-010 and MDX-101, and marketed as Yervoy^{®}; Bristol-Myers Squibb; Tremelimumab (IgG2 monoclonal antibody available from Pfizer, formerly known as ticilimumab, CP-675,206).). In an embodiment, the agent is an antibody or antibody fragment that binds to TIM3. In an embodiment, the agent is an antibody or antibody fragment that binds to LAG3.

PD-1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD-1, PD-L1 and PD-L2 have been shown to downregulate T cell activation upon binding to PD-1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD-1 with PD-L1. Antibodies, antibody fragments, and other inhibitors of PD-1, PD-L1 and PD-L2 are available in the art and may be used combination with a cars of the present disclosure described herein. For example, nivolumab (also referred to as BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. Pidilizumab (CT-011; Cure Tech) is a humanized IgG1k monoclonal antibody that binds to PD-1. Pidilizumab and other humanized anti-PD-1 monoclonal antibodies are disclosed in WO009/101611. Pembrolizumab (formerly known as lambrolizumab, and also referred to as MK03475; Merck) is a humanized IgG4 monoclonal antibody that binds to PD-1. Pembrolizumab and other humanized anti-PD-1 antibodies are disclosed in US 8,354,509 and WO2009/114335. MEDI4736 (Medimmune) is a human monoclonal antibody that binds to PDL1, and inhibits interaction of the ligand with PD1. MDPL3280A (Genentech / Roche) is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. Other anti-PD-L1 binding agents include YW243.55.S70 (heavy and light chain variable regions are shown in SEQ ID NOs 20 and 21 in WO2010/077634) and MDX-1 105 (also referred to as BMS-936559, and, e.g., anti-PD-L1 binding agents disclosed in WO2007/005874). AMP-224 (B7-DCIg; Amplimmune; e.g., disclosed in WO2010/027827 and WO2011/066342), is a PD-L2 Fc fusion soluble receptor that blocks the interaction between PD-1 and B7-H1. Other anti-PD-1 antibodies include AMP 514 (Amplimmune), among others, e.g., anti-PD-1 antibodies disclosed in US 8,609,089, US 2010028330, and/or US 20120114649.

In one embodiment, the anti-PD-1 antibody or fragment thereof is an anti-PD-1 antibody molecule as described in US 2015/0210769, entitled "Antibody Molecules to PD-1 and Uses Thereof," . In one embodiment, the anti-PD-1 antibody molecule includes at least one, two, three, four, five or six CDRs (or collectively all of the CDRs) from a heavy and light chain variable region from an antibody chosen from any of BAP049-hum01, BAP049-hum02, BAP049-hum03, BAP049-hum04, BAP049-hum05, BAP049-hum06, BAP049-hum07, BAP049-hum08, BAP049-hum09, BAP049-hum10, BAP049-hum11, BAP049-hum12, BAP049-hum13, BAP049-hum14, BAP049-hum15, BAP049-hum16, BAP049-Clone-A, BAP049-Clone-B, BAP049-Clone-C, BAP049-Clone-D, or BAP049-Clone-E; or as described in Table 1 of US 2015/0210769, or encoded by the nucleotide sequence in Table 1, or a sequence substantially identical (*e.g.,* at least 80%, 85%, 90%, 92%, 95%, 97%, 98%, 99% or higher identical) to any of the aforesaid sequences; or closely related CDRs, *e.g*., CDRs which are identical or which have at least one amino acid alteration, but not more than two, three or four alterations (*e.g*., substitutions, deletions, or insertions, *e.g*., conservative substitutions).

In yet another embodiment, the anti-PD-1 antibody molecule comprises at least one, two, three or four variable regions from an antibody described herein, *e.g.,* an antibody chosen from any of BAP0-49-hum01, BAP049-hum02, BAP049-hum03, BAP049-hum04, BAP049-hum05, BAP049-hum06, BAP0-49-hum07, BAP049-hum08, BAP049-hum09, BAP049-hum10, BAP049-hum11, BAP049-hum12, BAP049-hum13, BAP049-hum14, BAP049-hum15, BAP049-hum16, BAP049-Clone-A, BAP049-Clone-B, BAP049-Clone-C, BAP049-Clone-D, or BAP049-Clone-E; or as described in Table 1 of US 2015/0210769, or encoded by the nucleotide sequence in Table 1; or a sequence substantially identical (*e.g.,* at least 80%, 85%, 90%, 92%, 95%, 97%, 98%, 99% or higher identical) to any of the aforesaid sequences.

TIM3 (T cell immunoglobulin-3) also negatively regulates T cell function, particularly in IFN-g-secreting CD4+ T helper 1 and CD8+ T cytotoxic 1 cells, and plays a critical role in T cell exhaustion. Inhibition of the interaction between TIM3 and its ligands, e.g., galectin-9 (Ga19), phosphotidylserine (PS), and HMGB1, can increase immune response. Antibodies, antibody fragments, and other inhibitors of TIM3 and its ligands are available in the art and may be used combination with a CD19 CAR described herein. For example, antibodies, antibody fragments, small molecules, or peptide inhibitors that target TIM3 binds to the IgV domain of TIM3 to inhibit interaction with its ligands. Antibodies and peptides that inhibit TIM3 are disclosed in WO2013/006490 and US20100247521. Other anti-TIM3 antibodies include humanized versions of RMT3-23 (disclosed in Ngiow et al., 2011, Cancer Res, 71:3540-3551), and clone 8B.2C12 (disclosed in Monney et al., 2002, Nature, 415:536-541). Bi-specific antibodies that inhibit TIM3 and PD-1 are disclosed in US20130156774.

In one embodiment, the anti-TIM3 antibody or fragment thereof is an anti- TIM3 antibody molecule as described in US 2015/0218274, entitled "Antibody Molecules to TIM3 and Uses Thereof," . In one embodiment, the anti-TIM3 antibody molecule includes at least one, two, three, four, five or six CDRs (or collectively all of the CDRs) from a heavy and light chain variable region from an antibody chosen from any of ABTIM3, ABTIM3-hum01, ABTIM3-hum02, ABTIM3-hum03, ABTIM3-hum04, ABTIM3-hum05, ABTIM3-hum06, ABTIM3-hum07, ABTIM3-hum08, ABTIM3-hum09, ABTIM3-hum10, ABTIM3-hum11, ABTIM3-hum12, ABTIM3-hum13, ABTIM3-hum14, ABTIM3-hum15, ABTIM3-hum16, ABTIM3-hum17, ABTIM3-hum18, ABTIM3-hum19, ABTIM3-hum20, ABTIM3-hum21, ABTIM3-hum22, ABTIM3-hum23; or as described in Tables 1-4 of US 2015/0218274; or encoded by the nucleotide sequence in Tables 1-4; or a sequence substantially identical (*e.g.,* at least 80%, 85%, 90%, 92%, 95%, 97%, 98%, 99% or higher identical) to any of the aforesaid sequences, or closely related CDRs, *e.g.,* CDRs which are identical or which have at least one amino acid alteration, but not more than two, three or four alterations (*e.g.,* substitutions, deletions, or insertions, *e.g.,* conservative substitutions).

In yet another embodiment, the anti-TIM3 antibody molecule comprises at least one, two, three or four variable regions from an antibody described herein, *e.g.,* an antibody chosen from any of ABTIM3, ABTIM3-hum01, ABTIM3-hum02, ABTIM3-hum03, ABTIM3-hum04, ABTIM3-hum05, ABTIM3-hum06, ABTIM3-hum07, ABTIM3-hum08, ABTIM3-hum09, ABTIM3-hum10, ABTIM3-hum11, ABTIM3-hum12, ABTIM3-hum13, ABTIM3-hum14, ABTIM3-hum15, ABTIM3-hum16, ABTIM3-hum17, ABTIM3-hum18, ABTIM3-hum19, ABTIM3-hum20, ABTIM3-hum21, ABTIM3-hum22, ABTIM3-hum23; or as described in Tables 1-4 of US 2015/0218274; or encoded by the nucleotide sequence in Tables 1-4; or a sequence substantially identical (*e.g.,* at least 80%, 85%, 90%, 92%, 95%, 97%, 98%, 99% or higher identical) to any of the aforesaid sequences.

In other embodiments, the agent which enhances the activity of a CAR-expressing cell is a CEACAM inhibitor (*e.g*., CEACAM-1, CEACAM-3, and/or CEACAM-5 inhibitor). In one embodiment, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, *e.g.,* a monoclonal antibody 34B1, 26H7, and 5F4; or a recombinant form thereof, as described in, *e.g.,* US 2004/0047858, US 7,132,255 and WO 99/052552. In other embodiments, the anti-CEACAM antibody binds to CEACAM-5 as described in, *e.g.,* Zheng et al. PLoS One. 2010 Sep 2;5(9). pii: e12529 (DOI:10:1371/journal.pone.0021146), or crossreacts with CEACAM-1 and CEACAM-5 as described in, *e.g.,* WO 2013/054331 and US 2014/0271618.

Without wishing to be bound by theory, carcinoembryonic antigen cell adhesion molecules (CEACAM), such as CEACAM-1 and CEACAM-5, are believed to mediate, at least in part, inhibition of an anti-tumor immune response (*see e.g.,* Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun;11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion (*see e.g.,* WO 2014/022332; Huang, et al. (2014) Nature doi:10.1038/nature13848). In embodiments, co-blockade of CEACAM-1 and TIM-3 has been shown to enhance an anti-tumor immune response in xenograft colorectal cancer models (*see e.g.,* WO 2014/022332; Huang, *et al.* (2014), *supra*)*.* In other embodiments, co-blockade of CEACAM-1 and PD-1 reduce T cell tolerance as described, e.g., in WO 2014/059251. Thus, CEACAM inhibitors can be used with the other immunomodulators described herein (*e.g*., anti-PD-1 and/or anti-TIM-3 inhibitors) to enhance an immune response against a cancer, *e.g*., a melanoma, a lung cancer (e.g., NSCLC), a bladder cancer, a colon cancer an ovarian cancer, and other cancers as described herein.

LAG3 (lymphocyte activation gene-3 or CD223) is a cell surface molecule expressed on activated T cells and B cells that has been shown to play a role in CD8+ T cell exhaustion. Antibodies, antibody fragments, and other inhibitors of LAG3 and its ligands are available in the art and may be used combination with a CD19 CAR described herein. For example, BMS-986016 (Bristol-Myers Squib) is a monoclonal antibody that targets LAG3. IMP701 (Immutep) is an antagonist LAG3 antibody and IMP731 (Immutep and GlaxoSmithKline) is a depleting LAG3 antibody. Other LAG3 inhibitors include IMP321 (Immutep), which is a recombinant fusion protein of a soluble portion of LAG3 and Ig that binds to MHC class II molecules and activates antigen presenting cells (APC). Other antibodies are disclosed, e.g., in WO2010/019570.

In one embodiment, the anti-LAG3 antibody or fragment thereof is an anti-LAG3 antibody molecule as described in US 2015/0259420, entitled "Antibody Molecules to LAG3 and Uses Thereof," . In one embodiment, the anti-LAG3 antibody molecule includes at least one, two, three, four, five or six CDRs (or collectively all of the CDRs) from a heavy and light chain variable region from an antibody chosen from any of BAP050-hum01, BAP050-hum02, BAP050-hum03, BAP050-hum04, BAP050-hum05, BAP050-hum06, BAP050-hum07, BAP050-hum08, BAP050-hum09, BAP050-hum10, BAP050-hum11, BAP050-hum12, BAP050-hum13, BAP050-hum14, BAP050-hum15, BAP050-hum16, BAP050-hum17, BAP050-hum18, BAP050-hum19, BAP050-hum20, huBAP050(Ser) (*e.g*., BAP050-hum01-Ser, BAP050-hum02-Ser, BAP050-hum03-Ser, BAP050-hum04-Ser, BAP050-hum05-Ser, BAP050-hum06-Ser, BAP050-hum07-Ser, BAP050-hum08-Ser, BAP050-hum09-Ser, BAP050-hum10-Ser, BAP050-hum11-Ser, BAP050-hum12-Ser, BAP050-hum13-Ser, BAP050-hum14-Ser, BAP050-hum15-Ser, BAP050-hum18-Ser, BAP050-hum19-Ser, or BAP050-hum20-Ser), BAP050-Clone-F, BAP050-Clone-G, BAP050-Clone-H, BAP050-Clone-I, or BAP050-Clone-J; or as described in Table 1 of US 2015/0259420; or encoded by the nucleotide sequence in Table 1; or a sequence substantially identical (*e.g.*, at least 80%, 85%, 90%, 92%, 95%, 97%, 98%, 99°% or higher identical) to any of the aforesaid sequences, or closely related CDRs, *e.g.*, CDRs which are identical or which have at least one amino acid alteration, but not more than two, three or four alterations (*e.g.*, substitutions, deletions, or insertions, *e.g.*, conservative substitutions).

In yet another embodiment, the anti- LAG3 antibody molecule comprises at least one, two, three or four variable regions from an antibody described herein, *e.g*., an antibody chosen from any of BAP050-hum01, BAP050-hum02, BAP050-hum03, BAP050-hum04, BAP050-hum05, BAP050-hum06, BAP050-hum07, BAP050-hum08, BAP050-hum09, BAP050-hum10, BAP050-hum11, BAP050-hum12, BAP050-hum13, BAP050-hum14, BAP050-hum15, BAP050-hum16, BAP050-hum17, BAP050-hum18, BAP050-hum19, BAP050-hum20, huBAP050(Ser) (*e.g*., BAP050-hum01-Ser, BAP050-hum02-Ser, BAP050-hum03-Ser, BAP050-hum04-Ser, BAP050-hum05-Ser, BAP050-hum06-Ser, BAP050-hum07-Ser, BAP050-hum08-Ser, BAP050-hum09-Ser, BAP050-hum10-Ser, BAP050-hum11-Ser, BAP050-hum12-Ser, BAP050-hum13-Ser, BAP050-hum14-Ser, BAP050-hum15-Ser, BAP050-hum18-Ser, BAP050-hum19-Ser, or BAP050-hum20-Ser), BAP050-Clone-F, BAP050-Clone-G, BAP050-Clone-H, BAP050-Clone-I, or BAP050-Clone-J; or as described in Table 1 of US 2015/0259420; or encoded by the nucleotide sequence in Tables 1; or a sequence substantially identical (*e.g.*, at least 80%, 85%, 90%, 92%, 95%, 97%, 98%, 99% or higher identical) to any of the aforesaid sequences.In some embodiments, the agent which enhances the activity of a CAR-expressing cell can be, e.g., a fusion protein comprising a first domain and a second domain, wherein the first domain is an inhibitory molecule, or fragment thereof, and the second domain is a polypeptide that is associated with a positive signal, e.g., a polypeptide comrpsing an antracellular signaling domain as described herein. In some embodiments, the polypeptide that is associated with a positive signal can include a costimulatory domain of CD28, CD27, ICOS, e.g., an intracellular signaling domain of CD28, CD27 and/or ICOS, and/or a primary signaling domain, e.g., of CD3 zeta, e.g., described herein. In one embodiment, the fusion protein is expressed by the same cell that expressed the CAR. In another embodiment, the fusion protein is expressed by a cell, e.g., a T cell that does not express a CAR of the present disclosure.

In one embodiment, the agent which enhances activity of a CAR-expressing cell described herein is miR-17-92.

In one embodiment, the agent which enhances activity of a CAR-described herein is a cytokine. Cytokines have important functions related to T cell expansion, differentiation, survival, and homeostatis. Cytokines that can be administered to the subject receiving a CAR-expressing cell described herein include: IL-2, IL-4, IL-7, IL-9, IL-15, IL-18, and IL-21, or a combination thereof. In preferred embodiments, the cytokine administered is IL-7, IL-15, or IL-21, or a combination thereof. The cytokine can be administered once a day or more than once a day, e.g., twice a day, three times a day, or four times a day. The cytokine can be administered for more than one day, e.g. the cytokine is administered for 2 days, 3 days, 4 days, 5 days, 6 days, 1 week, 2 weeks, 3 weeks, or 4 weeks. For example, the cytokine is administered once a day for 7 days.

In embodiments, the cytokine is administered in combination with CAR-expressing T cells. The cytokine can be administered simultaneously or concurrently with the CAR-expressing T cells, e.g., administered on the same day. The cytokine may be prepared in the same pharmaceutical composition as the CAR-expressing T cells, or may be prepared in a separate pharmaceutical composition. Alternatively, the cytokine can be administered shortly after administration of the CAR-expressing T cells, e.g., 1 day, 2 days, 3 days, 4 days, 5 days, 6 days, or 7 days after administration of the CAR-expressing T cells. In embodiments where the cytokine is administered in a dosing regimen that occurs over more than one day, the first day of the cytokine dosing regimen can be on the same day as administration with the CAR-expressing T cells, or the first day of the cytokine dosing regimen can be 1 day, 2 days, 3 days, 4 days, 5 days, 6 days, or 7 days after administration of the CAR-expressing T cells. In one embodiment, on the first day, the CAR-expressing T cells are administered to the subject, and on the second day, a cytokine is administered once a day for the next 7 days. In a preferred embodiment, the cytokine to be administered in combination with CAR-expressing T cells is IL-7, IL-15, or IL-21.

In other embodiments, the cytokine is administered a period of time after administration of CAR-expressing cells, e.g., at least 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells. In one embodiment, the cytokine is administered after assessment of the subject's response to the CAR-expressing cells. For example, the subject is administered CAR-expressing cells according to the dosage and regimens described herein. The response of the subject to CART therapy is assessed at 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells, using any of the methods described herein, including inhibition of tumor growth, reduction of circulating tumor cells, or tumor regression. Subjects that do not exhibit a sufficient response to CART therapy can be administered a cytokine. Administration of the cytokine to the subject that has sub-optimal response to the CART therapy improves CART efficacy or anti-tumor activity. In a preferred embodiment, the cytokine administered after administration of CAR-expressing cells is IL-7.

### Combination with a low dose of an mTOR inhibitor

In one instance, the CAR-expressing cells described herein, are administered in combination with a low, immune enhancing dose of an mTOR inhibito, e.g., as described in PCT publication WO/2016/014530.

In another embodiment, administration of a low, immune enhancing, dose of an mTOR inhibitor results in increased or prolonged proliferation of CAR-expressing cells, e.g., in culture or in a subject, e.g., as compared to non-treated CAR-expressing cells or a non-treated subject. In embodiments, increased proliferation is associated with in an increase in the number of CAR-expressing cells. Methods for measuring increased or prolonged proliferation are described in Examples 4 and 5. In another embodiment, administration of a low, immune enhancing, dose of an mTOR inhibitor results in increased killing of cancer cells by CAR-expressing cells, e.g., in culture or in a subject, e.g., as compared to non-treated CAR-expressing cells or a non-treated subject. In embodiments, increased killing of cancer cells is associated with in a decrease in tumor volume.

In one instance, the cells expressing a CAR molecule, e.g., a CAR molecule described herein, are administered in combination with a low, immune enhancing dose of an mTOR inhibitor, e.g., an allosteric mTOR inhibitor, e.g., RAD001, or a catalytic mTOR inhibitor. For example, administration of the low, immune enhancing, dose of the mTOR inhibitor can be initiated prior to administration of a CAR-expressing cell described herein; completed prior to administration of a CAR-expressing cell described herein; initiated at the same time as administration of a CAR-expressing cell described herein; overlapping with administration of a CAR-expressing cell described herein; or continuing after administration of a CAR-expressing cell described herein.

Alternatively or in addition, administration of a low, immune enhancing, dose of an mTOR inhibitor can optimize immune effector cells to be engineered to express a CAR molecule described herein. In such instances, administration of a low, immune enhancing, dose of an mTOR inhibitor, e.g., an allosteric inhibitor, e.g., RAD001, or a catalytic inhibitor, is initiated or completed prior to harvest of immune effector cells, e.g., T cells or NK cells, to be engineered to express a CAR molecule described herein, from a subject.

In another instance, immune effector cells, e.g., T cells or NK cells, to be engineered to express a CAR molecule described herein, e.g., after harvest from a subject, or CAR-expressing immune effector cells, e.g., T cells or NK cells, e.g., prior to administration to a subject, can be cultured in the presence of a low, immune enhancing, dose of an mTOR inhibitor.

As used herein, the term "mTOR inhibitor" refers to a compound or ligand, or a pharmaceutically acceptable salt thereof, which inhibits the mTOR kinase in a cell. In an embodiment an mTOR inhibitor is an allosteric inhibitor. In an embodiment an mTOR inhibitor is a catalytic inhibitor.

Allosteric mTOR inhibitors include the neutral tricyclic compound rapamycin (sirolimus), rapamycin-related compounds, that is compounds having structural and functional similarity to rapamycin including, e.g., rapamycin derivatives, rapamycin analogs (also referred to as rapalogs) and other macrolide compounds that inhibit mTOR activity.

Rapamycin is a known macrolide antibiotic produced by Streptomyces hygroscopicus having the structure shown in Formula A.

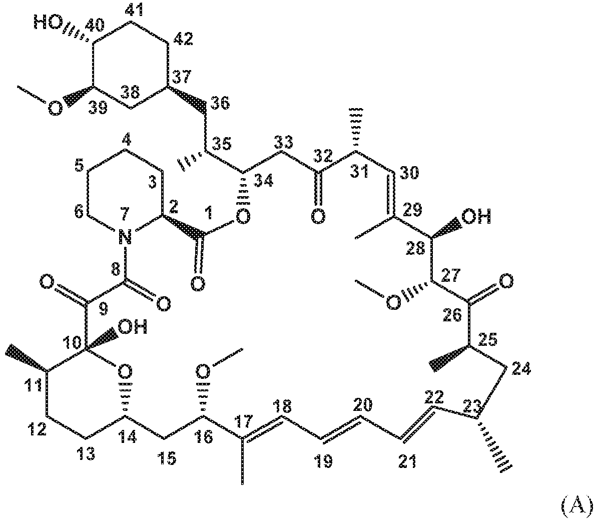

Other suitable rapamycin analogs include, but are not limited to, RAD001, otherwise known as everolimus (Afinitor^{®}), has the chemical name (1R,9S,12S,15R,16E,18R,19R,21R,23S,24E,26E,28E,30S,32S,35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone,sirolimus (rapamycin, AY-22989), 40-[3-hydroxy-2-(hydroxymethyl)-2-methylpropanoate]-rapamycin (also called temsirolimus or CCI-779) and ridaforolimus (AP-23573/MK-8669).b Other examples of allosteric mTor inhibtors include zotarolimus (ABT578) and umirolimus as described in US2005/0101624. are incorporated by reference. Other suitable mTOR inhibitors are described in paragraphs 946 to 964 of International Publication WO2015/142675, filed March 13, 2015, which is

Low, immune enhancing doses of an mTOR inhibitor, suitable levels of mTOR inhibition associated with low doses of an mTOR inhibitor, methods for detecting the level of mTOR inhibition, and suitable pharmaceutical compositions thereof are further described in paragraphs 936 to 945 and 965 to 1003 of International Publication WO2015/142675, filed March 13, 2015.

### Pharmaceutical compositions and treatments

Pharmaceutical compositions of the present disclosure may comprise a CAR-expressing cell, e.g., a plurality of CAR-expressing cells, as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are in one aspect formulated for intravenous administration.

Pharmaceutical compositions of the present disclosure may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions of the present disclosure to be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In some instances, a dose of CAR-expressing cells described herein (e.g., cells comprising, e.g., engineered to express, a TA CAR and a BCA CAR) comprises about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, 3 x 10⁸, or 5 x 10⁸ cells/kg. In some instances, a dose of CAR cells (e.g., cells comprising, e.g., engineered to express, a TA CAR and a BCA CAR) comprises at least about 1 x 10⁶, 1.1 x 10⁶, 2 x 10⁶, 3.6 x 10⁶, 5 x 10⁶, 1 x 10⁷, 1.8 x 10⁷, 2 x 10⁷, 5 x 10⁷, 1 x 10⁸, 2 x 10⁸, 3 x 10⁸, or 5 x 10⁸ cells/kg. In some instances, a dose of CAR cells (e.g., cells comprising, e.g., engineered to express, a TA CAR and a BCA CAR) comprises up to about 1 x 10⁶, 1.1 x 10⁶, 2 x 10⁶, 3.6 x 10⁶, 5 x 10⁶, 1 x 10⁷, 1.8 x 10⁷, 2 x 10⁷ 5 x 10⁷, 1 x 10⁸, 2 x 10⁸, 3 x 10⁸, or 5 x 10⁸ cells/kg. In some instances, a dose of CAR cells (e.g., cells comprising, e.g., engineered to express, a TA CAR and a BCA CAR) comprises about 1.1 x 10⁶ - 1.8 x 10⁷ cells/kg. In some instances, a dose of CAR cells (e.g., cells comprising, e.g., engineered to express, a TA CAR and a BCA CAR) comprises about 1 x 10⁷, 2 x 10⁷, 5 x 10⁷, 1 x 10⁸, 2 x 10⁸, 3 x 10⁸, 5 x 10³, 1 x 10⁹, 2 x 10⁹, or 5 x 10⁹ cells. In some instances, a dose of CAR cells (e.g., cells comprising, e.g., engineered to express, a TA CAR and a BCA CAR) comprises at least about 1 x 10⁷, 2 x 10⁷, 5 x 10⁷, 1 x 10⁸, 2 x 10⁸, 3 x 10⁸, 5 x 10⁸, 1 x 10⁹, 2 x 10⁹, or 5 x 10⁹ cells. In some instances, a dose of CAR cells (e.g., cells comprising, e.g., engineered to express, a TA CAR and a BCA CAR) comprises up to about 1 x 10⁷, 2 x 10⁷, 5 x 10⁷, 1 x 10³, 2 x 10⁸, 3 x 10⁸, 5 x 10⁸, 1 x 10⁹, 2 x 10⁹, or 5 x 10⁹ cells.

In some instances, a dose of CAR cells (e.g., cells comprising, e.g., engineered to express, a TA CAR and a BCA CAR) comprises up to about 1 x 10⁷, 1.5 x 10⁷, 2 x 10⁷, 2.5 x 10⁷,3 x 10⁷, 3.5 x 10⁷, 4 x 10⁷, 5 x 10⁷, 1 x 10⁸ , 1.5 x 10⁸, 2 x 10⁸, 2.5 x 10⁸, 3 x 10⁸, 3.5 x 10⁸, 4 x 10⁸, 5 x 10⁸, 1 x 10⁹, 2 x 10⁹, or 5 x 10⁹ cells. In some instances, a dose of CAR cells (e.g., cells comprising, e.g., engineered to express, a TA CAR and a BCA CAR) comprises up to about 1-3 x 10⁷ to 1-3 x10⁸ of cells. In some instances, the subject is administered about 1-3 x 10⁷ of the cells. In other instances, the subject is administered about 1-3 x 10⁸ of the cells.

The cells can be administered by using infusion techniques that are commonly known in immunotherapy (see, e.g., Rosenberg et al., New Eng. J. of Med. 319:1676, 1988).

In certain aspects, it may be desired to administer activated immune effector cells (e.g., T cells, NK cells) to a subject and then subsequently redraw blood (or have an apheresis performed), activate immune effector cells (e.g., T cells, NK cells) therefrom according to the present disclosure, and reinfuse the patient with these activated and expanded immune effector cells (e.g., T cells, NK cells). This process can be carried out multiple times every few weeks. In certain aspects, immune effector cells (e.g., T cells, NK cells) can be activated from blood draws of from 10cc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present disclosure are administered to a patient by intradermal or subcutaneous injection. In one aspect, the T cell compositions of the present disclosure are administered by i.v. injection. The compositions of immune effector cells (e.g., T cells, NK cells) may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs disclosed herein may be introduced, thereby creating a CAR T cell disclosed herein. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present disclosure. In an additional aspect, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In one embodiment, the CAR is introduced into immune effector cells (e.g., T cells, NK cells), e.g., using in vitro transcription, and the subject (e.g., human) receives an initial administration of CAR immune effector cells (e.g., T cells, NK cells) of the invention, and one or more subsequent administrations of the CAR immune effector cells (e.g., T cells, NK cells) of the invention, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the invention are administered to the subject (e.g., human) per week, e.g., 2, 3, or 4 administrations of the CAR immune effector cells (e.g., T cells, NK cells) of the invention are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples.

### Example 1

Human T lymphocytes are taken from a subject and are provided ex vivo, stimulated using anti-CD3/CD28, and transduced with a lentivirus vector encoding a second-generation CD19 CAR (a CAR comprising a CD19 binding domain, a transmembrane domain, and an intracellular signaling domain comprising a CD3z primary signaling domain and a 4-1BB costimulatory signaling domain) under the control of the EF1a promoter, and with a lentiviral vector encoding a second-generation EGFRvIII CAR (a CAR comprising a EGFRvIII binding domain, a transmembrane domain, and an intracellular signaling domain comprising a CD3z primary signaling domain and a 4-1BB costimulatory signaling domain) under the control of the EF1a promoter. Systems such as this, for engineering a cell to express both the CD19 CAR and the EGFRvIII CAR, are illustrated in Figure 2. As well, a single bicistronic lentivirus vector is constructed which encodes a second generation CD19 CAR and a second generation EGFRvIII CAR with an intervening P2A site, all under the control of the EF1a promoter. Such a construct, for engineering a cell to express both the CD19 CAR and the EGFRvIII CAR, is illustrated in Figure 1. Cells expressing both the second-generation CD19 CAR and the second-generation EGFRvIII CAR are provided by transducing T cells either with a mixture of the two lentiviral vectors (each encoding either the CD19 CAR or the EGFRvIII CAR; Figure 2) or with the single lentiviral vector encoding the bicistronic construct (Figure 1), and CAR T cell proliferation, cytokine release and cytotoxicity are assayed against CD19+/EGFRvIII-cells, CD19-EGFRvIII+ cells, CD19+/EGFRVIII+ cells and a population of cells comprising CD19+/EGFRvIII- cells and CD19-/EGFRvIII+ cells, using methods disclosed herein (e.g., as described in WO2014/130657), and the activities are compared to cells expressing only the second-generation EGFRvIII CAR. Cells are further assayed in vivo (proliferation, long term persistence and tumor toxicity, e.g., by methods described in WO2014/130657) by administering the cells intravenously in xenogeneic immune-compromised NOD/SCID/common-gamma chain-/- mice with established EGFRvIII U87vIII glioma tumors. Autologous B cells or immortalized B cells expressing CD19 are co-infused with the CAR T cells to enhance CAR T cell activation. For clinical application, autologous T cells are transduced to express an EGFRvIII CAR and a CD19 CAR and are administered to a patient suffering from glioblastoma multiforme (GBM). CART cell persistence, proliferation/expansion and anti-tumor efficacy are monitored. In patients, their normal B cells expressing CD19 provide a source of stimulation for the second CAR.

### Example 2

Human T lymphocytes are taken from a subject and are provided ex vivo, stimulated using anti-CD3/CD28, and transduced with a lentivirus vector encoding a second-generation BCMA CAR (a CAR comprising a BCMA binding domain, a transmembrane domain, and an intracellular signaling domain comprising a CD3z primary signaling domain and a 4-1BB costimulatory signaling domain) under the control of the EF1a promoter, and with a lentiviral vector encoding a second-generation EGFRvIII CAR (a CAR comprising a EGFRvIII binding domain, a transmembrane domain, and an intracellular signaling domain comprising a CD3z primary signaling domain and a 4-1BB costimulatory signaling domain) under the control of the EF1a promoter. Cells expressing both the second-generation BCMA CAR and the second-generation EGFRvIII CAR are provided, and CAR T cell proliferation, cytokine release and cytotoxicity are assayed against BCMA-+/EGFRvIII- cells, BCMA-/EGFRvIII+ cells, BCMA+/EGFRVIII+ cells and a population of cells comprising BCMA+/EGFRvIII- cells and BCMA-/EGFRvIII+ cells, using methods disclosed herein (e.g., as described in WO2014/130657), and the activities are compared to cells expressing only the second-generation EGFRvIII CAR. Cells are further assayed in vivo (proliferation, long term persistence and tumor toxicity, e.g., by methods described in WO2014/130657) by administering the cells intravenously in xenogeneic immune-compromised NOD/SCID/common-gamma chain-/- mice with established EGFRvIII U87vIII glioma tumors. Autologous B cells or immortalized B cells expressing BCMA are co-infused with the CAR T cells to enhance CAR T cell activation. For clinical application, autologous T cells are transduced to express an EGFRvIII CAR and a BCMA CAR and are administered to a patient suffering from glioblastoma multiforme (GBM). CART cell persistence, proliferation/expansion and anti-tumor efficacy are monitored. In patients, their normal B cells expressing BCMA provide a source of stimulation for the second CAR.

### Example 3

Human T lymphocytes are taken from a subject and are provided ex vivo, stimulated using anti-CD3/CD28, and transduced with a lentivirus vector encoding a second-generation CD19 CAR (a CAR comprising a CD19 binding domain, a transmembrane domain, and an intracellular signaling domain comprising a CD3z primary signaling domain and a 4-1BB costimulatory signaling domain) under the control of the EF1a promoter, and with a lentiviral vector encoding a second-generation Mesothelin ("Meso") CAR (a CAR comprising a Meso binding domain, a transmembrane domain, and an intracellular signaling domain comprising a CD3z primary signaling domain and a 4-1BB costimulatory signaling domain) under the control of the EF1a promoter. As well, a single bicistronic vector is constructed which encodes a second generation CD19 CAR and a second generation Mesothelin CAR with an intervening P2A site, all under the control of the EF1a promoter. Cells expressing both the second-generation CD19 CAR and the second-generation Mesothelin CAR are provided by transducing T cells either with a mixture of the two lentiviral vectors (each encoding either the CD19 CAR or the EGFRvIII CAR) or with the single lentiviral vector encoding the bicistronic construct, and CAR T cell proliferation, cytokine release and cytotoxicity are assayed against CD19+/Meso-cells, CD19-/Meso+ cells, CD19+/Meso+ cells and a population of cells comprising CD19+/Meso- cells and CD19-/Meso+ cells, using methods disclosed herein (e.g., as described in WO2015/090230), and the activities are compared to cells expressing only the second-generation Meso CAR. Cells are further assayed in vivo (proliferation, long term persistence and tumor toxicity, e.g., by methods described in WO2015/090230) by administering the cells intravenously in xenogeneic immune-compromised NOD/SCID/common-gamma chain-/- mice with established Meso-expressing ovarian adenocarcinoma (OVCAR8) xenografts. Autologous B cells or immortalized B cells expressing CD19 are co-infused with the CAR T cells to enhance CAR T cell activation. For clinical application, autologous T cells are transduced to express a Meso CAR and a CD19 CAR and are administered to patients suffering from a mesothelin-expressing cancer such as mesothelioma, pancreatic cancer, or ovarian cancer. CART cell persistence, proliferation/expansion and anti-tumor efficacy are monitored. In patients, their normal B cells expressing CD19 provide a source of stimulation for the second CAR.

### Example 4

Human T lymphocytes are taken from a subject and are provided ex vivo, stimulated using anti-CD3/CD28, and transduced with a lentivirus vector encoding a second-generation BCMA CAR (a CAR comprising a BCMA binding domain, a transmembrane domain, and an intracellular signaling domain comprising a CD3z primary signaling domain and a 4-1BB costimulatory signaling domain) under the control of the FF1a promoter, and with a lentiviral vector encoding a second-generation Mesothelin ("Meso") CAR (a CAR comprising a Meso binding domain, a transmembrane domain, and an intracellular signaling domain comprising a CD3z primary signaling domain and a 4-1BB costimulatory signaling domain) under the control of the EF1a promoter. Cells expressing both the second-generation BCMA CAR and the second-generation Meso CAR are provided, and CAR T cell proliferation, cytokine release and cytotoxicity are assayed against BCMA+/Meso- cells, BCMA-/Meso+ cells, BCMA+/Meso+ cells and a population of cells comprising BCMA+/Meso- cells and BCMA-/Meso+ cells, using methods disclosed herein (e.g., as described in WO2015/090230), and the activities are compared to cells expressing only the second-generation Meso CAR. Cells are further assayed in vivo (proliferation, long term persistence and tumor toxicity, e.g., by methods described in WO2015/090230) by administering the cells intravenously in xenogeneic immune-compromised NOD/SCID/common-gamma chain-/- mice with established Meso-expressing ovarian adenocarcinoma (OVCAR8) xenografts. Autologous B cells or immortalized B cells expressing BCMA are co-infused with the CAR T cells to enhance CAR T cell activation. For clinical application, autologous T cells are transduced to express a Meso CAR and a BCMA CAR and are administered to patients suffering from a mesothelin-expressing cancer such as mesothelioma, pancreatic cancer, or ovarian cancer. CART cell persistence, proliferation/expansion and anti-tumor efficacy are monitored. In patients, their normal B cells expressing BCMA provide a source of stimulation for the second CAR.

## Claims

1. A cell comprising a first chimeric antigen receptor (CAR) and a second CAR, each of which comprises an antigen binding domain, a transmembrane domain, and an intracellular signaling domain, wherein the antigen binding domain of said first CAR binds to a B-Cell antigen and the antigen binding domain of said second CAR binds to a tumor antigen other than a B-Cell antigen, wherein the tumor antigen other than a B-Cell antigen is a solid tumor antigen, further wherein
(i) the intracellular signaling domain of said first CAR comprises a costimulatory signaling domain, but not a primary signaling domain; and
(ii) the intracellular signaling domain of said second CAR comprises a costimulatory signaling domain and a primary signaling domain.

2. The cell of claim 1, wherein the cell is an immune effector cell and shows enhanced proliferation or persistence in vivo, compared to an otherwise identical cell that lacks said first CAR.

3. The cell of claim 1 or 2, wherein said B-Cell antigen is selected from the group consisting of CD19, CD5, CD10, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, 5, 7, IL-7/3R, IL7/4/3R, and IL4R.

4. The cell of any one of claims 1-3, wherein said B-Cell antigen is CD19, and wherein:
(i) said antigen binding domain of said first CAR comprises a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 6, Table 7 or Table 9;
(ii) said antigen binding domain of said first CAR further comprises a light chain complementary determining region 1 (LC CDR1), a light chain complementary determining region 2 (LC CDR2), and a light chain complementary determining region 3 (LC CDR3) of any light chain binding domain amino acid sequence listed in Table 6, Table 8 or Table 9;
(iii) said antigen binding domain of said first CAR comprises: the amino acid sequence of any light chain variable region listed in Table 6 or Table 9; an amino acid sequence having at least one, two or three modifications but not more than 20 or 10 modifications of the amino acid sequence of any of the light chain variable regions provided in Table 6 or Table 9; or an amino acid sequence with 95-99% identity to the amino acid sequence of any of the light chain variable regions provided in Table 6 or Table 9;
(iv) said antigen binding domain of said first CAR comprises: the amino acid sequence of any heavy chain variable region listed in Table 6 or Table 9; an amino acid sequence having at least one, two or three modifications but not more than 20 or 10 modifications of the amino acid sequence of any of the heavy chain variable regions provided in Table 6 or Table 9; or an amino acid sequence with 95-99% identity to the amino acid sequence of any of the heavy chain variable regions provided in Table 6 or Table 9;
(v) said antigen binding domain of said first CAR comprises a polypeptide having the amino acid sequence of any light chain variable region listed in Table 6 or Table 9, and the amino acid sequence of any heavy chain variable region listed in Table 6 or Table 9;
(vi) said antigen binding domain of said first CAR comprises a polypeptide having a sequence of SEQ ID NO: 83; SEQ ID NO: 84, SEQ ID NO: 85; SEQ ID NO: 86; SEQ ID NO: 87; SEQ ID NO: 88; SEQ ID NO: 89, SEQ ID NO: 90, SEQ ID NO: 91, SEQ ID NO: 92, SEQ ID NO: 93, SEQ ID NO: 94, SEQ ID NO: 95, or SEQ ID NO: 112; or
(vii) the first CAR comprises a polypeptide having an amino acid sequence selected from the group consisting of SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, and SEQ ID NO: 281.

5. The cell of any one of claims 1-4, wherein said B-Cell antigen is BCMA and wherein:
(i) said antigen binding domain of said first CAR comprises a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 12 or 13;
(ii) said antigen binding domain of said first CAR further comprises a light chain complementary determining region 1 (LC CDR1), a light chain complementary determining region 2 (LC CDR2), and a light chain complementary determining region 3 (LC CDR3) of any light chain binding domain amino acid sequence listed in Table 12 or 13;
(iii) said antigen binding domain of said first CAR comprises: the amino acid sequence of any light chain variable region listed in Table 12 or 13; an amino acid sequence having at least one, two or three modifications but not more than 20 or 10 modifications of the amino acid sequence of any of the light chain variable regions provided in Table 12 or 13; or an amino acid sequence with 95-99% identity to the amino acid sequence of any of the light chain variable regions provided in Table 12 or 13;
(iv) said antigen binding domain of said first CAR comprises: the amino acid sequence of any heavy chain variable region listed in Table 12 or 13; an amino acid sequence having at least one, two or three modifications but not more than 20 or 10 modifications of the amino acid sequence of any of the heavy chain variable regions provided in Table 12 or 13; or an amino acid sequence with 95-99% identity to the amino acid sequence of any of the heavy chain variable regions provided in Table 12 or 13;
(v) said antigen binding domain of said first CAR comprises a polypeptide having the amino acid sequence of any light chain variable region listed in Table 12 or 13, and the amino acid sequence of any heavy chain variable region listed in Table 12 or 13;
(vi) said antigen binding domain of said first CAR comprises a polypeptide having a sequence of SEQ ID NO: 349; SEQ ID NO: 339, SEQ ID NO: 340; SEQ ID NO: 341; SEQ ID NO: 342; SEQ ID NO: 343; SEQ ID NO: 344, SEQ ID NO: 345, SEQ ID NO: 346, SEQ ID NO: 347, SEQ ID NO: 348, SEQ ID NO: 350, SEQ ID NO: 351, SEQ ID NO: 352, SEQ ID NO: 353, SEQ ID NO: 429, SEQ ID NO: 430, SEQ ID NO: 431, SEQ ID NO: 432, SEQ ID NO: 433, SEQ ID NO: 434, SEQ ID NO: 435, SEQ ID NO: 436, SEQ ID NO: 437, SEQ ID NO: 438, SEQ ID NO: 439, SEQ ID NO: 440, SEQ ID NO: 441, SEQ ID NO: 442, SEQ ID NO: 443, SEQ ID NO: 444, SEQ ID NO: 445, SEQ ID NO: 446, SEQ ID NO: 447, SEQ ID NO: 448, SEQ ID NO: 449, SEQ ID NO: 563, SEQ ID NO: 564, SEQ ID NO: 565 or SEQ ID NO: 566; or
(vii) the first CAR comprises a polypeptide having an amino acid sequence selected from the group consisting of SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998, and SEQ ID NO: 999.

6. The cell of any one of claims 1-5, wherein said solid tumor antigen is selected from the group consisting of EGFRvIII, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, GFRα4, and a peptide of any of these antigens presented on MHC.

7. The cell of any one of claims 1-6, wherein said solid tumor antigen is EGFRvIII, and wherein:
(i) said antigen binding domain of said second CAR comprises a heavy chain complementary determining region 1 (HC CDR1), a heavy chain complementary determining region 2 (HC CDR2), and a heavy chain complementary determining region 3 (HC CDR3) of any anti-EGFRvIII heavy chain binding domain amino acid sequence listed in Table 5;
(ii) said antigen binding domain of said second CAR further comprises a light chain complementary determining region 1 (LC CDR1), a light chain complementary determining region 2 (LC CDR2), and a light chain complementary determining region 3 (LC CDR3) of any anti-EGFRvIII light chain binding domain amino acid sequence listed in Table 5;
(iii) said antigen binding domain of said second CAR comprises: the amino acid sequence of any anti-EGFRvIII light chain variable region listed in Table 5; an amino acid sequence having at least one, two or three modifications but not more than 20 or 10 modifications of the amino acid sequence of any of the anti-EGFRvIII light chain variable regions provided in Table 5; or an amino acid sequence with 95-99% identity to the amino acid sequence of any of the anti-EGFRvIII light chain variable regions provided in Table 5;
(iv) said antigen binding domain of said second CAR comprises: the amino acid sequence of any anti-EGFRvIII heavy chain variable region listed in Table 5; an amino acid sequence having at least one, two or three modifications but not more than 20 or 10 modifications of the amino acid sequence of any of the anti-EGFRvIII heavy chain variable regions provided in Table 5; or an amino acid sequence with 95-99% identity to the amino acid sequence of any of the anti-EGFRvIII heavy chain variable regions provided in Table 5;
(v) said antigen binding domain of said second CAR comprises a polypeptide having the amino acid sequence of any anti-EGFRvIII light chain variable region listed in Table 5, and the amino acid sequence of any anti-EGFRvIII heavy chain variable region listed in Table 5;
(vi) said antigen binding domain of said second CAR comprises a polypeptide having a sequence of any of SEQ ID NOS: 71-79; or
(vii) the second CAR comprises a polypeptide having an amino acid sequence selected from the group consisting of SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090, and SEQ ID NO: 1096.

8. The cell of any one of claims 1-6, wherein said solid tumor antigen is mesothelin and wherein:
(i) said antigen binding domain of said second CAR comprises a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 2 or 3;
(ii) said antigen binding domain of said second CAR further comprises a light chain complementary determining region 1 (LC CDR1), a light chain complementary determining region 2 (LC CDR2), and a light chain complementary determining region 3 (LC CDR3) of any light chain binding domain amino acid sequence listed in Table 2 or 4;
(iii) said antigen binding domain of said second CAR comprises: the amino acid sequence of any light chain variable region listed in Table 2; an amino acid sequence having at least one, two or three modifications but not more than 20 or 10 modifications of the amino acid sequence of any of the light chain variable regions provided in Table 2; or an amino acid sequence with 95-99% identity to the amino acid sequence of any of the light chain variable regions provided in Table 2;
(iv) said antigen binding domain of said second CAR comprises: the amino acid sequence of any heavy chain variable region listed in Table 2; an amino acid sequence having at least one, two or three modifications but not more than 20 or 10 modifications of the amino acid sequence of any of the heavy chain variable regions provided in Table 2; or an amino acid sequence with 95-99% identity to the amino acid sequence of any of the heavy chain variable regions provided in Table 2;
(v) said antigen binding domain of said second CAR comprises a polypeptide having the amino acid sequence of any light chain variable region listed in Table 2, and the amino acid sequence of any heavy chain variable region listed in Table 2;
(vi) said antigen binding domain of said second CAR comprises a polypeptide having a sequence of any one of SEQ ID NOS: 46-70; or
(vii) the second CAR comprises a polypeptide having an amino acid sequence selected from the group consisting of SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305, and SEQ ID NO: 306.

9. The cell of any one of claims 1-8, wherein said intracellular signaling domain of said second CAR comprises one or more primary signaling domains, optionally wherein the primary signaling domains comprise a CD3-zeta stimulatory domain.

10. The cell of any one of claims 1-9, wherein said intracellular signaling domain of said first or said second CAR comprises one or more costimulatory signaling domains, optionally wherein said costimulatory signaling domain is an intracellular domain of a costimulatory protein selected from the group consisting of CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, and a ligand that specifically binds with CD83.

11. The cell of any one of claims 1-10, wherein the first CAR binds to CD19 and the second CAR binds to EGFRvIII.

12. The cell of claim 11, wherein
(a) in said first CAR:
(i) said antigen binding domain comprises a heavy chain complementary determining region 1 (HC CDR1), a heavy chain complementary determining region 2 (HC CDR2), and a heavy chain complementary determining region 3 (HC CDR3) of any heavy chain binding domain amino acid sequence listed in Table 6, Table 7 or Table 9;
(ii) said antigen binding domain further comprises a light chain complementary determining region 1 (LC CDR1), a light chain complementary determining region 2 (LC CDR2), and a light chain complementary determining region 3 (LC CDR3) of any light chain binding domain amino acid sequence listed in Table 6, Table 8 or Table 9;
(iii) said antigen binding domain comprises: the amino acid sequence of any light chain variable region listed in Table 6 or Table 9; an amino acid sequence having at least one, two or three modifications but not more than 20 or 10 modifications of the amino acid sequence of any of the light chain variable regions provided in Table 6 or Table 9; or an amino acid sequence with 95-99% identity to the amino acid sequence of any of the light chain variable regions provided in Table 6 or Table 9;
(iv) said antigen binding domain comprises: the amino acid sequence of any heavy chain variable region listed in Table 6 or Table 9; an amino acid sequence having at least one, two or three modifications but not more than 20 or 10 modifications of the amino acid sequence of any of the heavy chain variable regions provided in Table 6 or Table 9; or an amino acid sequence with 95-99% identity to the amino acid sequence of any of the heavy chain variable regions provided in Table 6 or Table 9;
(v) said antigen binding domain comprises a polypeptide having the amino acid sequence of any light chain variable region listed in Table 6 or Table 9, and the amino acid sequence of any heavy chain variable region listed in Table 6 or Table 9;
(vi) said antigen binding domain comprises a polypeptide having a sequence of SEQ ID NO: 83; SEQ ID NO: 84, SEQ ID NO: 85; SEQ ID NO: 86; SEQ ID NO: 87; SEQ ID NO: 88; SEQ ID NO: 89, SEQ ID NO: 90, SEQ ID NO: 91, SEQ ID NO: 92, SEQ ID NO: 93, SEQ ID NO: 94, SEQ ID NO: 95, or SEQ ID NO: 112; or
(vii) the CAR comprises a polypeptide having an amino acid sequence selected from the group consisting of SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, and SEQ ID NO: 281; and
(b) in said second CAR:
(i) said antigen binding domain comprises a heavy chain complementary determining region 1 (HC CDR1), a heavy chain complementary determining region 2 (HC CDR2), and a heavy chain complementary determining region 3 (HC CDR3) of any anti-EGFRvIII heavy chain binding domain amino acid sequence listed in Table 5;
(ii) said antigen binding domain further comprises a light chain complementary determining region 1 (LC CDR1), a light chain complementary determining region 2 (LC CDR2), and a light chain complementary determining region 3 (LC CDR3) of any anti-EGFRvIII light chain binding domain amino acid sequence listed in Table 5;
(iii) said antigen binding domain comprises: the amino acid sequence of any anti-EGFRvIII light chain variable region listed in Table 5; an amino acid sequence having at least one, two or three modifications but not more than 20 or 10 modifications of the amino acid sequence of any of the anti-EGFRvIII light chain variable regions provided in Table 5; or an amino acid sequence with 95-99% identity to the amino acid sequence of any of the anti-EGFRvIII light chain variable regions provided in Table 5;
(iv) said antigen binding domain comprises: the amino acid sequence of any anti-EGFRvIII heavy chain variable region listed in Table 5; an amino acid sequence having at least one, two or three modifications but not more than 20 or 10 modifications of the amino acid sequence of any of the anti-EGFRvIII heavy chain variable regions provided in Table 5; or an amino acid sequence with 95-99% identity to the amino acid sequence of any of the anti-EGFRvIII heavy chain variable regions provided in Table 5;
(v) said antigen binding domain comprises a polypeptide having the amino acid sequence of any anti-EGFRvIII light chain variable region listed in Table 5, and the amino acid sequence of any anti-EGFRvIII heavy chain variable region listed in Table 5;
(vi) said antigen binding domain comprises a polypeptide having a sequence of any of SEQ ID NOS: 71-79; or
(vii) the CAR comprises a polypeptide having an amino acid sequence selected from the group consisting of SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090, and SEQ ID NO: 1096.

13. A nucleic acid encoding a first CAR and a second CAR, each of which comprises an antigen binding domain, a transmembrane domain, and an intracellular signalling domain, wherein the antigen binding domain of said first CAR binds to a B-Cell antigen and the antigen binding domain of said second CAR binds to a tumor antigen other than a B-Cell antigen, wherein the tumor antigen other than a B-Cell antigen is a solid tumor antigen, further wherein
(i) the intracellular signaling domain of said first CAR comprises a costimulatory signaling domain, but not a primary signaling domain; and
(ii) the intracellular signaling domain of said second CAR comprises a costimulatory signaling domain and a primary signaling domain.

14. The nucleic acid of claim 13, wherein said intracellular signaling domain of said second CAR comprises one or more primary signaling domains, optionally wherein the primary signaling domains comprise a CD3-zeta stimulatory domain.

15. The nucleic acid of claim 13 or 14, wherein the sequence of said first CAR and the sequence of said second CAR are separated by an independent ribosomal entry site, a promoter element, or a sequence encoding a T2A, P2A, E2A, or F2A element.

16. The cell of any one of claims 1-12 for use in a method of treating cancer.

17. The cell for use according to claim 16, wherein the cancer is a solid tumor.

18. The cell of any one of claims 1-12 for use in a method of treating a disease associated with expression of a solid tumor antigen, wherein the disease is a tumor characterized as glioblastoma, ovarian cancer, lung cancer, prostate cancer, colorectal cancer, pancreatic cancer, breast carcinoma, adenocarcinoma or mesothelioma.

## Patentansprüche

1. Zelle, umfassend einen ersten chimären Antigenrezeptor (CAR) und einen zweiten CAR, die jeweils eine Antigenbindungsdomäne, eine Transmembrandomäne und eine intrazelluläre Signalgebungsdomäne umfassen, wobei die Antigenbindungsdomäne des ersten CAR an ein B-Zell-Antigen bindet und die Antigenbindungsdomäne des zweiten CAR an ein anderes Tumorantigen als ein B-Zell-Antigen bindet, wobei es sich bei dem anderen Tumorantigen als einem B-Zell-Antigen um ein Antigen eines soliden Tumors handelt, ferner wobei
(i) die intrazelluläre Signalgebungsdomäne des ersten CAR eine costimulatorische Signalgebungsdomäne, aber keine primäre Signalgebungsdomäne umfasst; und
(ii) die intrazelluläre Signalgebungsdomäne des zweiten CAR eine costimulatorische Signalgebungsdomäne und eine primäre Signalgebungsdomäne umfasst.

2. Zelle nach Anspruch 1, wobei es sich bei der Zelle um eine Immuneffektorzelle handelt und verstärkte Proliferation oder Beständigkeit in vivo im Vergleich zu einer ansonsten identischen Zelle zeigt, der der erste CAR fehlt.

3. Zelle nach Anspruch 1 oder 2, wobei das B-Zell-Antigen aus der Gruppe bestehend aus CD19, CD5, CD10, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, 5, 7, IL-7/3R, IL7/4/3R und IL4R ausgewählt ist.

4. Zelle nach einem der Ansprüche 1-3, wobei es sich bei dem B-Zell-Antigen um CD19 handelt und wobei:
(i) die Antigenbindungsdomäne des ersten CAR eine HC CDR1 (heavy chain complementary determining region 1), eine HC CDR2 (heavy chain complementary determining region 2) und eine HC CDR3 (heavy chain complementary determining region 3) einer beliebigen in Tabelle 6, Tabelle 7 oder Tabelle 9 aufgelisteten Aminosäuresequenz einer Schwerkettenbindungsdomäne umfasst;
(ii) die Antigenbindungsdomäne des ersten CAR ferner eine LC CDR1 (light chain complementary determining region 1), eine LC CDR2 (light chain complementary determining region 2) und eine LC CDR3 (light chain complementary determining region 3) einer beliebigen in Tabelle 6, Tabelle 8 oder Tabelle 9 aufgelisteten Aminosäuresequenz einer Leichtkettenbindungsdomäne umfasst;
(iii) die Antigenbindungsdomäne des ersten CAR Folgendes umfasst: die Aminosäuresequenz einer beliebigen in Tabelle 6 oder Tabelle 9 aufgelisteten variablen Region der Leichtkette; eine Aminosäuresequenz mit mindestens einer, zwei oder drei Modifikationen, aber nicht mehr als 20 oder 10 Aminosäuresequenzmodifikationen einer beliebigen der in Tabelle 6 oder Tabelle 9 bereitgestellten variablen Regionen der Leichtkette oder eine Aminosäuresequenz mit 95-99 % Identität mit der Aminosäuresequenz einer beliebigen der in Tabelle 6 oder Tabelle 9 bereitgestellten variablen Regionen der Leichtkette;
(iv) die Antigenbindungsdomäne des ersten CAR Folgendes umfasst: die Aminosäuresequenz einer beliebigen in Tabelle 6 oder Tabelle 9 aufgelisteten variablen Region der Schwerkette; eine Aminosäuresequenz mit mindestens einer, zwei oder drei Modifikationen, aber nicht mehr als 20 oder 10 Aminosäuresequenzmodifikationen einer beliebigen der in Tabelle 6 oder Tabelle 9 bereitgestellten variablen Regionen der Schwerkette oder eine Aminosäuresequenz mit 95-99 % Identität mit der Aminosäuresequenz einer beliebigen der in Tabelle 6 oder Tabelle 9 bereitgestellten variablen Regionen der Schwerkette;
(v) die Antigenbindungsdomäne des ersten CAR ein Polypeptid mit der Aminosäuresequenz einer beliebigen in Tabelle 6 oder Tabelle 9 aufgelisteten variablen Region der Leichtkette und der Aminosäuresequenz einer beliebigen in Tabelle 6 oder Tabelle 9 aufgelisteten variablen Region der Schwerkette umfasst;
(vi) die Antigenbindungsdomäne des ersten CAR ein Polypeptid mit einer Sequenz unter SEQ ID NO: 83; SEQ ID NO: 84, SEQ ID NO: 85; SEQ ID NO: 86; SEQ ID NO: 87; SEQ ID NO: 88; SEQ ID NO: 89, SEQ ID NO: 90, SEQ ID NO: 91, SEQ ID NO: 92, SEQ ID NO: 93, SEQ ID NO: 94, SEQ ID NO: 95 oder SEQ ID NO: 112 umfasst; oder
(vii) der erste CAR ein Polypeptid mit einer Aminosäuresequenz umfasst, die aus der Gruppe bestehend aus SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280 und SEQ ID NO: 281 ausgewählt ist.

5. Zelle nach einem der Ansprüche 1-4, wobei es sich bei dem B-Zell-Antigen um BCMA handelt und wobei:
(i) die Antigenbindungsdomäne des ersten CAR eine HC CDR1 (heavy chain complementary determining region 1), eine HC CDR2 (heavy chain complementary determining region 2) und eine HC CDR3 (heavy chain complementary determining region 3) einer beliebigen in Tabelle 12 oder 13 aufgelisteten Aminosäuresequenz einer Schwerkettenbindungsdomäne umfasst;
(ii) die Antigenbindungsdomäne des ersten CAR ferner eine LC CDR1 (light chain complementary determining region 1), eine LC CDR2 (light chain complementary determining region 2) und eine LC CDR3 (light chain complementary determining region 3) einer beliebigen in Tabelle 12 oder 13 aufgelisteten Aminosäuresequenz einer Leichtkettenbindungsdomäne umfasst;
(iii) die Antigenbindungsdomäne des ersten CAR Folgendes umfasst: die Aminosäuresequenz einer beliebigen in Tabelle 12 oder 13 aufgelisteten variablen Region der Leichtkette; eine Aminosäuresequenz mit mindestens einer, zwei oder drei Modifikationen, aber nicht mehr als 20 oder 10 Aminosäuresequenzmodifikationen einer beliebigen der in Tabelle 12 oder 13 bereitgestellten variablen Regionen der Leichtkette oder eine Aminosäuresequenz mit 95-99 % Identität mit der Aminosäuresequenz einer beliebigen der in Tabelle 12 oder 13 bereitgestellten variablen Regionen der Leichtkette;
(iv) die Antigenbindungsdomäne des ersten CAR Folgendes umfasst: die Aminosäuresequenz einer beliebigen in Tabelle 12 oder 13 aufgelisteten variablen Region der Schwerkette; eine Aminosäuresequenz mit mindestens einer, zwei oder drei Modifikationen, aber nicht mehr als 20 oder 10 Aminosäuresequenzmodifikationen einer beliebigen der in Tabelle 12 oder 13 bereitgestellten variablen Regionen der Schwerkette oder eine Aminosäuresequenz mit 95-99 % Identität mit der Aminosäuresequenz einer beliebigen der in Tabelle 12 oder 13 bereitgestellten variablen Regionen der Schwerkette;
(v) die Antigenbindungsdomäne des ersten CAR ein Polypeptid mit der Aminosäuresequenz einer beliebigen in Tabelle 12 oder 13 aufgelisteten variablen Region der Leichtkette und der Aminosäuresequenz einer beliebigen in Tabelle 12 oder 13 aufgelisteten variablen Region der Schwerkette umfasst;
(vi) die Antigenbindungsdomäne des ersten CAR ein Polypeptid mit einer Sequenz unter SEQ ID NO: 349; SEQ ID NO: 339, SEQ ID NO: 340; SEQ ID NO: 341; SEQ ID NO: 342; SEQ ID NO: 343; SEQ ID NO: 344, SEQ ID NO: 345, SEQ ID NO: 346, SEQ ID NO: 347, SEQ ID NO: 348, SEQ ID NO: 350, SEQ ID NO: 351, SEQ ID NO: 352, SEQ ID NO: 353, SEQ ID NO: 429, SEQ ID NO: 430, SEQ ID NO: 431, SEQ ID NO: 432, SEQ ID NO: 433, SEQ ID NO: 434, SEQ ID NO: 435, SEQ ID NO: 436, SEQ ID NO: 437, SEQ ID NO: 438, SEQ ID NO: 439, SEQ ID NO: 440, SEQ ID NO: 441, SEQ ID NO: 442, SEQ ID NO: 443, SEQ ID NO: 444, SEQ ID NO: 445, SEQ ID NO: 446, SEQ ID NO: 447, SEQ ID NO: 448, SEQ ID NO: 449, SEQ ID NO: 563, SEQ ID NO: 564, SEQ ID NO: 565 oder SEQ ID NO: 566 umfasst; oder
(vii) der erste CAR ein Polypeptid mit einer Aminosäuresequenz umfasst, die aus der Gruppe bestehend aus SEQ ID NO: 949, SEQ ID NO: 950, SEQ ID NO: 951, SEQ ID NO: 952, SEQ ID NO: 953, SEQ ID NO: 954, SEQ ID NO: 955, SEQ ID NO: 956, SEQ ID NO: 957, SEQ ID NO: 958, SEQ ID NO: 959, SEQ ID NO: 960, SEQ ID NO: 961, SEQ ID NO: 962, SEQ ID NO: 963, SEQ ID NO: 979, SEQ ID NO: 980, SEQ ID NO: 981, SEQ ID NO: 982, SEQ ID NO: 983, SEQ ID NO: 984, SEQ ID NO: 985, SEQ ID NO: 986, SEQ ID NO: 987, SEQ ID NO: 988, SEQ ID NO: 989, SEQ ID NO: 990, SEQ ID NO: 991, SEQ ID NO: 992, SEQ ID NO: 993, SEQ ID NO: 994, SEQ ID NO: 995, SEQ ID NO: 996, SEQ ID NO: 997, SEQ ID NO: 998 und SEQ ID NO: 999 ausgewählt ist.

6. Zelle nach einem der Ansprüche 1-5, wobei das Antigen des soliden Tumors aus der Gruppe bestehend aus EGFRvIII, Mesothelin, GD2, Tn-Antigen, sTn-Antigen, Tn-O-Glykopeptiden, sTn-O-Glykopeptiden, PSMA, CD97, TAG72, CD44v6, CEA, EPCAM, KIT, IL-13Ra2, Leguman, GD3, CD171, IL-11Ra, PSCA, MAD-CT-1, MAD-CT-2, VEGFR2, LewisY, PDGFR-beta, SSEA-4, Folatrezeptor alpha, ERBBs (z.B. ERBB2), Her2/neu, MUC1, EGFR, NCAM, Ephrin B2, CAIX, LMP2, sLe, HMWMAA, o-Acetyl-GD2, Folatrezeptor beta, TEM1/CD248, TEM7R, FAP, Legumain, HPV E6 oder E7, ML-IAP, CLDN6, TSHR, GPRC5D, ALK, Polysialinsäure, Fos-bezogenes Antigen, Neutrophil-Elastase, TRP-2, CYP1B1, Spermienprotein 17, menschliches beta-Choriongonadotropin, AFP, Thyroglobulin, PLAC1, globoH, RAGE1, MN-CA IX, menschliche Telomerase-Reverse-Transkriptase, intestinale Carboxylesterase, mut hsp 70-2, NA-17, NY-BR-1, UPK2, HAVCR1, ADRB3, PANX3, NY-ESO-1, GPR20, Ly6k, OR51E2, TARP, GFRα4 und einem Peptid eines beliebigen dieser Antigene, das auf MHC präsentiert wird, ausgewählt ist.

7. Zelle nach einem der Ansprüche 1-6, wobei es sich bei dem Antigen des soliden Tumors um EGFRvIII handelt und wobei:
(i) die Antigenbindungsdomäne des zweiten CAR eine HC CDR1 (heavy chain complementary determining region 1), eine HC CDR2 (heavy chain complementary determining region 2) und eine HC CDR3 (heavy chain complementary determining region 3) einer beliebigen in Tabelle 5 aufgelisteten Aminosäuresequenz einer Anti-EGFRvIII-Schwerkettenbindungsdomäne umfasst;
(ii) die Antigenbindungsdomäne des zweiten CAR ferner eine LC CDR1 (light chain complementary determining region 1), eine LC CDR2 (light chain complementary determining region 2) und eine LC CDR3 (light chain complementary determining region 3) einer beliebigen in Tabelle 5 aufgelisteten Aminosäuresequenz einer Anti-EGFRvIII-Leichtkettenbindungsdomäne umfasst;
(iii) die Antigenbindungsdomäne des zweiten CAR Folgendes umfasst: die Aminosäuresequenz einer beliebigen in Tabelle 5 aufgelisteten variablen Region der Anti-EGFRvIII-Leichtkette; eine Aminosäuresequenz mit mindestens einer, zwei oder drei Modifikationen, aber nicht mehr als 20 oder 10 Aminosäuresequenzmodifikationen einer beliebigen der in Tabelle 5 bereitgestellten variablen Regionen der Anti-EGFRvIII-Leichtkette oder eine Aminosäuresequenz mit 95-99 % Identität mit der Aminosäuresequenz einer beliebigen der in Tabelle 5 bereitgestellten variablen Regionen der Anti-EGFRvIII-Leichtkette;
(iv) die Antigenbindungsdomäne des zweiten CAR Folgendes umfasst: die Aminosäuresequenz einer beliebigen in Tabelle 5 aufgelisteten variablen Region der Anti-EGFRvIII-Schwerkette; eine Aminosäuresequenz mit mindestens einer, zwei oder drei Modifikationen, aber nicht mehr als 20 oder 10 Aminosäuresequenzmodifikationen einer beliebigen der in Tabelle 5 bereitgestellten variablen Regionen der Anti-EGFRvIII-Schwerkette oder eine Aminosäuresequenz mit 95-99 % Identität mit der Aminosäuresequenz einer beliebigen der in Tabelle 5 bereitgestellten variablen Regionen der Anti-EGFRvIII-Schwerkette;
(v) die Antigenbindungsdomäne des zweiten CAR ein Polypeptid mit der Aminosäuresequenz einer beliebigen in Tabelle 5 aufgelisteten variablen Region der Anti-EGFRvIII-Leichtkette und der Aminosäuresequenz einer beliebigen in Tabelle 5 aufgelisteten variablen Region der Anti-EGFRvIII-Schwerkette umfasst;
(vi) die Antigenbindungsdomäne des zweiten CAR ein Polypeptid mit einer Sequenz unter einer aus SEQ ID NOS: 71-79 umfasst; oder
(vii) der zweite CAR ein Polypeptid mit einer Aminosäuresequenz umfasst, die aus der Gruppe bestehend aus SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090 und SEQ ID NO: 1096 ausgewählt ist.

8. Zelle nach einem der Ansprüche 1-6, wobei es sich bei dem Antigen des soliden Tumors um Mesothelin handelt und wobei:
(i) die Antigenbindungsdomäne des zweiten CAR eine HC CDR1 (heavy chain complementary determining region 1), eine HC CDR2 (heavy chain complementary determining region 2) und eine HC CDR3 (heavy chain complementary determining region 3) einer beliebigen in Tabelle 2 oder 3 aufgelisteten Aminosäuresequenz einer Schwerkettenbindungsdomäne umfasst;
(ii) die Antigenbindungsdomäne des zweiten CAR ferner eine LC CDR1 (light chain complementary determining region 1), eine LC CDR2 (light chain complementary determining region 2) und eine LC CDR3 (light chain complementary determining region 3) einer beliebigen in Tabelle 2 oder 4 aufgelisteten Aminosäuresequenz einer Leichtkettenbindungsdomäne umfasst;
(iii) die Antigenbindungsdomäne des zweiten CAR Folgendes umfasst: die Aminosäuresequenz einer beliebigen in Tabelle 2 aufgelisteten variablen Region der Leichtkette; eine Aminosäuresequenz mit mindestens einer, zwei oder drei Modifikationen, aber nicht mehr als 20 oder 10 Aminosäuresequenzmodifikationen einer beliebigen der in Tabelle 2 bereitgestellten variablen Regionen der Leichtkette oder eine Aminosäuresequenz mit 95-99 % Identität mit der Aminosäuresequenz einer beliebigen der in Tabelle 2 bereitgestellten variablen Regionen der Leichtkette;
(iv) die Antigenbindungsdomäne des zweiten CAR Folgendes umfasst: die Aminosäuresequenz einer beliebigen in Tabelle 2 aufgelisteten variablen Region der Schwerkette; eine Aminosäuresequenz mit mindestens einer, zwei oder drei Modifikationen, aber nicht mehr als 20 oder 10 Aminosäuresequenzmodifikationen einer beliebigen der in Tabelle 2 bereitgestellten variablen Regionen der Schwerkette oder eine Aminosäuresequenz mit 95-99 % Identität mit der Aminosäuresequenz einer beliebigen der in Tabelle 2 bereitgestellten variablen Regionen der Schwerkette;
(v) die Antigenbindungsdomäne des zweiten CAR ein Polypeptid mit der Aminosäuresequenz einer beliebigen in Tabelle 2 aufgelisteten variablen Region der Leichtkette und der Aminosäuresequenz einer beliebigen in Tabelle 2 aufgelisteten variablen Region der Schwerkette umfasst;
(vi) die Antigenbindungsdomäne des zweiten CAR ein Polypeptid mit einer Sequenz unter einer aus SEQ ID NOS: 46-70 umfasst; oder
(vii) das zweite CAR ein Polypeptid mit einer Aminosäuresequenz umfasst, die aus der Gruppe bestehend aus SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, SEQ ID NO: 300, SEQ ID NO: 301, SEQ ID NO: 302, SEQ ID NO: 303, SEQ ID NO: 304, SEQ ID NO: 305 und SEQ ID NO: 306 ausgewählt ist.

9. Zelle nach einem der Ansprüche 1-8, wobei die intrazelluläre Signalgebungsdomäne des zweiten CAR eine oder mehrere primäre Signalgebungsdomänen umfasst, gegebenenfalls wobei die primären Signalgebungsdomänen eine stimulatorische CD3-zeta-Domäne umfassen.

10. Zelle nach einem der Ansprüche 1-9, wobei die intrazelluläre Signalgebungsdomäne des ersten oder des zweiten CAR eine oder mehrere costimulatorische Signalgebungsdomänen umfasst, gegebenenfalls wobei es sich bei der costimulatorischen Signalgebungsdomäne um eine intrazelluläre Domäne eines costimulatorischen Proteins handelt, das aus der Gruppe bestehend aus CD27, CD28, 4-1BB (CD137), OX40, GITR, CD30, CD40, ICOS, BAFFR, HVEM, ICAM-1, Lymphozytenfunktion-assoziiertes Antigen-1 (LFA-1), CD2, CDS, CD7, CD287, LICHT, NKG2C, NKG2D, SLAMF7, NKp80, NKp30, NKp44, NKp46, CD160, B7-H3, und einem Liganden, der spezifisch an CD83 bindet, ausgewählt ist.

11. Zelle nach einem der Ansprüche 1-10, wobei der erste CAR an CD19 bindet und der zweite CAR an EGFRvIII bindet.

12. Zelle nach Anspruch 11, wobei
(a) bei dem ersten CAR:
(i) die Antigenbindungsdomäne eine HC CDR1 (heavy chain complementary determining region 1), eine HC CDR2 (heavy chain complementary determining region 2) und eine HC CDR3 (heavy chain complementary determining region 3) einer beliebigen in Tabelle 6, Tabelle 7 oder Tabelle 9 aufgelisteten Aminosäuresequenz einer Schwerkettenbindungsdomäne umfasst;
(ii) die Antigenbindungsdomäne ferner eine LC CDR1 (light chain complementary determining region 1), eine LC CDR2 (light chain complementary determining region 2) und eine LC CDR3 (light chain complementary determining region 3) einer beliebigen in Tabelle 6, Tabelle 8 oder Tabelle 9 aufgelisteten Aminosäuresequenz einer Leichtkettenbindungsdomäne umfasst;
(iii) die Antigenbindungsdomäne Folgendes umfasst: die Aminosäuresequenz einer beliebigen in Tabelle 6 oder Tabelle 9 aufgelisteten variablen Region der Leichtkette; eine Aminosäuresequenz mit mindestens einer, zwei oder drei Modifikationen, aber nicht mehr als 20 oder 10 Aminosäuresequenzmodifikationen einer beliebigen der in Tabelle 6 oder Tabelle 9 bereitgestellten variablen Regionen der Leichtkette oder eine Aminosäuresequenz mit 95-99 % Identität mit der Aminosäuresequenz einer beliebigen der in Tabelle 6 oder Tabelle 9 bereitgestellten variablen Regionen der Leichtkette;
(iv) die Antigenbindungsdomäne Folgendes umfasst: die Aminosäuresequenz einer beliebigen in Tabelle 6 oder Tabelle 9 aufgelisteten variablen Region der Schwerkette; eine Aminosäuresequenz mit mindestens einer, zwei oder drei Modifikationen, aber nicht mehr als 20 oder 10 Aminosäuresequenzmodifikationen einer beliebigen der in Tabelle 6 oder Tabelle 9 bereitgestellten variablen Regionen der Schwerkette oder eine Aminosäuresequenz mit 95-99 % Identität mit der Aminosäuresequenz einer beliebigen der in Tabelle 6 oder Tabelle 9 bereitgestellten variablen Regionen der Schwerkette;
(v) die Antigenbindungsdomäne ein Polypeptid mit der Aminosäuresequenz einer beliebigen in Tabelle 6 oder Tabelle 9 aufgelisteten variablen Region der Leichtkette und der Aminosäuresequenz einer beliebigen in Tabelle 6 oder Tabelle 9 aufgelisteten variablen Region der Schwerkette umfasst;
(vi) die Antigenbindungsdomäne ein Polypeptid mit einer Sequenz unter SEQ ID NO: 83; SEQ ID NO: 84, SEQ ID NO: 85; SEQ ID NO: 86; SEQ ID NO: 87; SEQ ID NO: 88; SEQ ID NO: 89, SEQ ID NO: 90, SEQ ID NO: 91, SEQ ID NO: 92, SEQ ID NO: 93, SEQ ID NO: 94, SEQ ID NO: 95 oder SEQ ID NO: 112 umfasst; oder
(vii) der CAR ein Polypeptid mit einer Aminosäuresequenz umfasst, die aus der Gruppe bestehend aus SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280 und SEQ ID NO: 281 ausgewählt ist; und
(b) bei dem zweiten CAR:
(i) die Antigenbindungsdomäne eine HC CDR1 (heavy chain complementary determining region 1), eine HC CDR2 (heavy chain complementary determining region 2) und eine HC CDR3 (heavy chain complementary determining region 3) einer beliebigen in Tabelle 5 aufgelisteten Aminosäuresequenz einer Anti-EGFRvIII-Schwerkettenbindungsdomäne umfasst;
(ii) die Antigenbindungsdomäne ferner eine LC CDR1 (light chain complementary determining region 1), eine LC CDR2 (light chain complementary determining region 2) und eine LC CDR3 (light chain complementary determining region 3) einer beliebigen in Tabelle 5 aufgelisteten Aminosäuresequenz einer Anti-EGFRvIII-Leichtkettenbindungsdomäne umfasst;
(iii) die Antigenbindungsdomäne Folgendes umfasst: die Aminosäuresequenz einer beliebigen in Tabelle 5 aufgelisteten variablen Region der Anti-EGFRvIII-Leichtkette; eine Aminosäuresequenz mit mindestens einer, zwei oder drei Modifikationen, aber nicht mehr als 20 oder 10 Aminosäuresequenzmodifikationen einer beliebigen der in Tabelle 5 bereitgestellten variablen Regionen der Anti-EGFRvIII-Leichtkette oder eine Aminosäuresequenz mit 95-99 % Identität mit der Aminosäuresequenz einer beliebigen der in Tabelle 5 bereitgestellten variablen Regionen der Anti-EGFRvIII-Leichtkette;
(iv) die Antigenbindungsdomäne Folgendes umfasst: die Aminosäuresequenz einer beliebigen in Tabelle 5 aufgelisteten variablen Region der Anti-EGFRvIII-Schwerkette; eine Aminosäuresequenz mit mindestens einer, zwei oder drei Modifikationen, aber nicht mehr als 20 oder 10 Aminosäuresequenzmodifikationen einer beliebigen der in Tabelle 5 bereitgestellten variablen Regionen der Anti-EGFRvIII-Schwerkette oder eine Aminosäuresequenz mit 95-99 % Identität mit der Aminosäuresequenz einer beliebigen der in Tabelle 5 bereitgestellten variablen Regionen der Anti-EGFRvIII-Schwerkette;
(v) die Antigenbindungsdomäne ein Polypeptid mit der Aminosäuresequenz einer beliebigen in Tabelle 5 aufgelisteten variablen Region der Anti-EGFRvIII-Leichtkette und der Aminosäuresequenz einer beliebigen in Tabelle 5 aufgelisteten variablen Region der Anti-EGFRvIII-Schwerkette umfasst;
(vi) die Antigenbindungsdomäne ein Polypeptid mit einer Sequenz unter einer aus SEQ ID NOS: 71-79 umfasst; oder
(vii) der CAR ein Polypeptid mit einer Aminosäuresequenz umfasst, die aus der Gruppe bestehend aus SEQ ID NO: 1043, SEQ ID NO: 1049, SEQ ID NO: 1055, SEQ ID NO: 1061, SEQ ID NO: 1067, SEQ ID NO: 1073, SEQ ID NO: 1079, SEQ ID NO: 1085, SEQ ID NO: 1090 und SEQ ID NO: 1096 ausgewählt ist.

13. Nukleinsäure, codierend einen ersten CAR und einen zweiten CAR, die jeweils eine Antigenbindungsdomäne, eine Transmembrandomäne und eine intrazelluläre Signalgebungsdomäne umfassen, wobei die Antigenbindungsdomäne des ersten CAR an ein B-Zell-Antigen bindet und die Antigenbindungsdomäne des zweiten CAR an ein anderes Tumorantigen als ein B-Zell-Antigen bindet, wobei es sich bei dem anderen Tumorantigen als einem B-Zell-Antigen um ein Antigen eines soliden Tumors handelt, ferner wobei
(i) die intrazelluläre Signalgebungsdomäne des ersten CAR eine costimulatorische Signalgebungsdomäne, aber keine primäre Signalgebungsdomäne umfasst; und
(ii) die intrazelluläre Signalgebungsdomäne des zweiten CAR eine costimulatorische Signalgebungsdomäne und eine primäre Signalgebungsdomäne umfasst.

14. Nukleinsäure nach Anspruch 13, wobei die intrazelluläre Signalgebungsdomäne des zweiten CAR eine oder mehrere primäre Signalgebungsdomänen umfasst, gegebenenfalls wobei die primären Signalgebungsdomänen eine stimulatorische CD3-zeta-Domäne umfassen.

15. Nukleinsäure nach Anspruch 13 oder 14, wobei die Sequenz des ersten CAR und die Sequenz des zweiten CAR durch eine unabhängige Ribosomeneintrittsstelle, ein Promotorelement oder eine Sequenz, die ein T2A-, P2A-, E2A- oder F2A-Element codiert, getrennt sind.

16. Zelle nach einem der Ansprüche 1-12 zur Verwendung bei einem Verfahren zum Behandeln von Krebs.

17. Zelle zur Verwendung gemäß Anspruch 16, wobei es sich bei dem Krebs um einen soliden Tumor handelt.

18. Zelle nach einem der Ansprüche 1-12 zur Verwendung bei einem Verfahren zum Behandeln einer Krankheit, die mit der Expression eines Antigens eines soliden Tumors assoziiert ist, wobei es sich bei der Krankheit um einen Tumor handelt, der als Glioblastom, Eierstockkrebs, Lungenkrebs, Prostatakrebs, Kolorektalkrebs, Bauchspeicheldrüsenkrebs, Brustkarzinom, Adenokarzinom oder Mesotheliom charakterisiert wird.

## Revendications

1. Cellule comprenant un premier récepteur antigénique chimérique (CAR) et un second CAR, chacun de ceux-ci comprenant un domaine de liaison à l'antigène, un domaine transmembranaire et un domaine de signalisation intracellulaire, le domaine de liaison à l'antigène dudit premier CAR se liant à un antigène de lymphocyte B et le domaine de liaison à l'antigène dudit second CAR se liant à un antigène tumoral autre qu'un antigène de lymphocyte B, l'antigène tumoral autre qu'un antigène de lymphocyte B étant un antigène associé à une tumeur solide, en outre
(i) le domaine de signalisation intracellulaire dudit premier CAR comprenant un domaine de signalisation co-stimulatrice, mais pas un domaine de signalisation primaire ; et
(ii) le domaine de signalisation intracellulaire dudit second CAR comprenant un domaine de signalisation co-stimulatrice et un domaine de signalisation primaire.

2. Cellule selon la revendication 1, la cellule étant une cellule immunitaire effectrice et présentant une prolifération ou une persistance accrues in vivo, par comparaison avec une cellule par ailleurs identique qui est dépourvue dudit premier CAR.

3. Cellule selon la revendication 1 ou 2, ledit antigène de lymphocyte B étant choisi dans le groupe constitué par CD19, CD5, CD10, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, 5, 7, IL-7/3R, IL7/4/3R et IL4R.

4. Cellule selon l'une quelconque des revendications 1 à 3, ledit antigène de lymphocyte B étant CD19 et :
(i) ledit domaine de liaison à l'antigène dudit premier CAR comprenant une région déterminant la complémentarité de chaîne lourde 1 (HC CDR1), une région déterminant la complémentarité de chaîne lourde 2 (HC CDR2) et une région déterminant la complémentarité de chaîne lourde 3 (HC CDR3) d'une quelconque séquence d'acides aminés de domaine de liaison de chaîne lourde répertoriée dans le tableau 6, le tableau 7 ou le tableau 9 ;
(ii) ledit domaine de liaison à l'antigène dudit premier CAR comprenant en outre une région déterminant la complémentarité de chaîne légère 1 (LC CDR1), une région déterminant la complémentarité de chaîne légère 2 (LC CDR2) et une région déterminant la complémentarité de chaîne légère 3 (LC CDR3) d'une quelconque séquence d'acides aminés de domaine de liaison de chaîne légère répertoriée dans le tableau 6, le tableau 8 ou le tableau 9 ;
(iii) ledit domaine de liaison à l'antigène dudit premier CAR comprenant : la séquence d'acides aminés d'une quelconque région variable de chaîne légère répertoriée dans le tableau 6 ou le tableau 9 ; une séquence d'acides aminés ayant au moins une, deux ou trois modifications mais pas plus de 20 ou 10 modifications de la séquence d'acides aminés de l'une quelconque des régions variables de chaîne légère indiquées dans le tableau 6 ou le tableau 9 ; ou une séquence d'acides aminés présentant une identité de 95 à 99 % avec la séquence d'acides aminés de l'une quelconque des régions variables de chaîne légère indiquées dans le tableau 6 ou le tableau 9 ;
(iv) ledit domaine de liaison à l'antigène dudit premier CAR comprenant : la séquence d'acides aminés d'une quelconque région variable de chaîne lourde répertoriée dans le tableau 6 ou le tableau 9 ; une séquence d'acides aminés ayant au moins une, deux ou trois modifications mais pas plus de 20 ou 10 modifications de la séquence d'acides aminés de l'une quelconque des régions variables de chaîne lourde indiquées dans le tableau 6 ou le tableau 9 ; ou une séquence d'acides aminés présentant une identité de 95 à 99 % avec la séquence d'acides aminés de l'une quelconque des régions variables de chaîne lourde indiquées dans le tableau 6 ou le tableau 9 ;
(v) ledit domaine de liaison à l'antigène dudit premier CAR comprenant un polypeptide ayant la séquence d'acides aminés d'une quelconque région variable de chaîne légère répertoriée dans le tableau 6 ou le tableau 9 et la séquence d'acides aminés d'une quelconque région variable de chaîne lourde répertoriée dans le tableau 6 ou le tableau 9 ;
(vi) ledit domaine de liaison à l'antigène dudit premier CAR comprenant un polypeptide ayant une séquence de SEQ ID N° : 83 ; SEQ ID N° : 84, SEQ ID N° : 85 ; SEQ ID N° : 86 ; SEQ ID N° : 87 ; SEQ ID N° : 88 ; SEQ ID N° : 89, SEQ ID N° : 90, SEQ ID N° : 91, SEQ ID N° : 92, SEQ ID N° : 93, SEQ ID N° : 94, SEQ ID N° : 95 ou SEQ ID N° : 112 ; ou
(vii) le premier CAR comprenant un polypeptide ayant une séquence d'acides aminés choisie dans le groupe constitué par SEQ ID N° : 269, SEQ ID N° : 270, SEQ ID N° : 271, SEQ ID N° : 272, SEQ ID N° : 273, SEQ ID N° : 274, SEQ ID N° : 275, SEQ ID N° : 276, SEQ ID N° : 277, SEQ ID N° : 278, SEQ ID N° : 279, SEQ ID N° : 280 et SEQ ID N° : 281.

5. Cellule selon l'une quelconque des revendications 1 à 4, ledit antigène de lymphocyte B étant BCMA et :
(i) ledit domaine de liaison à l'antigène dudit premier CAR comprenant une région déterminant la complémentarité de chaîne lourde 1 (HC CDR1), une région déterminant la complémentarité de chaîne lourde 2 (HC CDR2) et une région déterminant la complémentarité de chaîne lourde 3 (HC CDR3) d'une quelconque séquence d'acides aminés de domaine de liaison de chaîne lourde répertoriée dans le tableau 12 ou 13 ;
(ii) ledit domaine de liaison à l'antigène dudit premier CAR comprenant en outre une région déterminant la complémentarité de chaîne légère 1 (LC CDR1), une région déterminant la complémentarité de chaîne légère 2 (LC CDR2) et une région déterminant la complémentarité de chaîne légère 3 (LC CDR3) d'une quelconque séquence d'acides aminés de domaine de liaison de chaîne légère répertoriée dans le tableau 12 ou 13 ;
(iii) ledit domaine de liaison à l'antigène dudit premier CAR comprenant : la séquence d'acides aminés d'une quelconque région variable de chaîne légère répertoriée dans le tableau 12 ou 13 ; une séquence d'acides aminés ayant au moins une, deux ou trois modifications mais pas plus de 20 ou 10 modifications de la séquence d'acides aminés de l'une quelconque des régions variables de chaîne légère indiquées dans le tableau 12 ou 13 ; ou une séquence d'acides aminés présentant une identité de 95 à 99 % avec la séquence d'acides aminés de l'une quelconque des régions variables de chaîne légère indiquées dans le tableau 12 ou 13 ;
(iv) ledit domaine de liaison à l'antigène dudit premier CAR comprenant : la séquence d'acides aminés d'une quelconque région variable de chaîne lourde répertoriée dans le tableau 12 ou 13 ; une séquence d'acides aminés ayant au moins une, deux ou trois modifications mais pas plus de 20 ou 10 modifications de la séquence d'acides aminés de l'une quelconque des régions variables de chaîne lourde indiquées dans le tableau 12 ou 13 ; ou une séquence d'acides aminés présentant une identité de 95 à 99 % avec la séquence d'acides aminés de l'une quelconque des régions variables de chaîne lourde indiquées dans le tableau 12 ou 13 ;
(v) ledit domaine de liaison à l'antigène dudit premier CAR comprenant un polypeptide ayant la séquence d'acides aminés d'une quelconque région variable de chaîne légère répertoriée dans le tableau 12 ou 13 et la séquence d'acides aminés d'une quelconque région variable de chaîne lourde répertoriée dans le tableau 12 ou 13 ;
(vi) ledit domaine de liaison à l'antigène dudit premier CAR comprenant un polypeptide ayant une séquence de SEQ ID N° : 349 ; SEQ ID N° : 339, SEQ ID N° : 340 ; SEQ ID N° : 341 ; SEQ ID N° : 342 ; SEQ ID N° : 343 ; SEQ ID N° : 344, SEQ ID N° : 345, SEQ ID N° : 346, SEQ ID N° : 347, SEQ ID N° : 348, SEQ ID N° : 350, SEQ ID N° : 351, SEQ ID N° : 352, SEQ ID N° : 353, SEQ ID N° : 429, SEQ ID N° : 430, SEQ ID N° : 431, SEQ ID N° : 432, SEQ ID N° : 433, SEQ ID N° : 434, SEQ ID N° : 435, SEQ ID N° : 436, SEQ ID N° : 437, SEQ ID N° : 438, SEQ ID N° : 439, SEQ ID N° : 440, SEQ ID N° : 441, SEQ ID N° : 442, SEQ ID N° : 443, SEQ ID N° : 444, SEQ ID N° : 445, SEQ ID N° : 446, SEQ ID N° : 447, SEQ ID N° : 448, SEQ ID N° : 449, SEQ ID N° : 563, SEQ ID N° : 564, SEQ ID N° : 565 ou SEQ ID N° : 566 ; ou
(vii) le premier CAR comprenant un polypeptide ayant une séquence d'acides aminés choisie dans le groupe constitué par SEQ ID N° : 949, SEQ ID N° : 950, SEQ ID N° : 951, SEQ ID N° : 952, SEQ ID N° : 953, SEQ ID N° : 954, SEQ ID N° : 955, SEQ ID N° : 956, SEQ ID N° : 957, SEQ ID N° : 958, SEQ ID N° : 959, SEQ ID N° : 960, SEQ ID N° : 961, SEQ ID N° : 962, SEQ ID N° : 963, SEQ ID N° : 979, SEQ ID N° : 980, SEQ ID N° : 981, SEQ ID N° : 982, SEQ ID N° : 983, SEQ ID N° : 984, SEQ ID N° : 985, SEQ ID N° : 986, SEQ ID N° : 987, SEQ ID N° : 988, SEQ ID N° : 989, SEQ ID N° : 990, SEQ ID N° : 991, SEQ ID N° : 992, SEQ ID N° : 993, SEQ ID N° : 994, SEQ ID N° : 995, SEQ ID N° : 996, SEQ ID N° : 997, SEQ ID N° : 998 et SEQ ID N° : 999.

6. Cellule selon l'une quelconque des revendications 1 à 5, ledit antigène associé à une tumeur solide étant choisi dans le groupe constitué par EGFRvIII, la mésothéline, GD2, l'antigène Tn, l'antigène sTn, les Tn-O-glycopeptides, les sTn-O-glycopeptides, PSMA, CD97, TAG72, CD44v6, CEA, EPCAM, KIT, IL-13Ra2, la legumain, GD3, CD171, IL-11Ra, PSCA, MAD-CT-1, MAD-CT-2, VEGFR2, LewisY, PDGFR-bêta, SSEA-4, le récepteur alpha du folate, les ERBB (par exemple, ERBB2), Her2/neu, MUC1, EGFR, NCAM, l'éphrine B2, CAIX, LMP2, sLe, HMWMAA, o-acétyl-GD2, le récepteur bêta du folate, TEM1/CD248, TEM7R, FAP, la legumain, HPV E6 ou E7, ML-IAP, CLDN6, TSHR, GPRC5D, ALK, l'acide polysialique, l'antigène apparenté à Fos, l'élastase des neutrophiles, TRP-2, CYP1B1, la protéine 17 du sperme, la bêta-hormone chorionique gonadotrope humaine, AFP, la thyroglobuline, PLAC1, globoH, RAGE1, MN-CA IX, la télomérase transcriptase inverse humaine, la carboxylestérase intestinale, mut hsp 70-2, NA-17, NY-BR-1, UPK2, HAVCR1, ADRB3, PANX3, NY-ESO-1, GPR20, Ly6k, OR51E2, TARP, GFRα4 et un peptide de l'un quelconque de ces antigènes présentés sur le CMH.

7. Cellule selon l'une quelconque des revendications 1 à 6, ledit antigène associé à une tumeur solide étant EGFRvIII et :
(i) ledit domaine de liaison à l'antigène dudit second CAR comprenant une région déterminant la complémentarité de chaîne lourde 1 (HC CDR1), une région déterminant la complémentarité de chaîne lourde 2 (HC CDR2) et une région déterminant la complémentarité de chaîne lourde 3 (HC CDR3) d'une quelconque séquence d'acides aminés de domaine de liaison de chaîne lourde anti-EGFRvIII répertoriée dans le tableau 5 ;
(ii) ledit domaine de liaison à l'antigène dudit second CAR comprenant en outre une région déterminant la complémentarité de chaîne légère 1 (LC CDR1), une région déterminant la complémentarité de chaîne légère 2 (LC CDR2) et une région déterminant la complémentarité de chaîne légère 3 (LC CDR3) d'une quelconque séquence d'acides aminés de domaine de liaison de chaîne légère anti-EGFRvIII répertoriée dans le tableau 5 ;
(iii) ledit domaine de liaison à l'antigène dudit second CAR comprenant : la séquence d'acides aminés d'une quelconque région variable de chaîne légère anti-EGFRvIII répertoriée dans le tableau 5 ; une séquence d'acides aminés ayant au moins une, deux ou trois modifications mais pas plus de 20 ou 10 modifications de la séquence d'acides aminés de l'une quelconque des régions variables de chaîne légère anti-EGFRvIII indiquées dans le tableau 5 ; ou une séquence d'acides aminés présentant une identité de 95 à 99 % avec la séquence d'acides aminés de l'une quelconque des régions variables de chaîne légère anti-EGFRvIII indiquées dans le tableau 5 ;
(iv) ledit domaine de liaison à l'antigène dudit second CAR comprenant : la séquence d'acides aminés d'une quelconque région variable de chaîne lourde anti-EGFRvIII répertoriée dans le tableau 5 ; une séquence d'acides aminés ayant au moins une, deux ou trois modifications mais pas plus de 20 ou 10 modifications de la séquence d'acides aminés de l'une quelconque des régions variables de chaîne lourde anti-EGFRvIII indiquées dans le tableau 5 ; ou une séquence d'acides aminés présentant une identité de 95 à 99 % avec la séquence d'acides aminés de l'une quelconque des régions variables de chaîne lourde anti-EGFRvIII indiquées dans le tableau 5 ;
(v) ledit domaine de liaison à l'antigène dudit second CAR comprenant un polypeptide ayant la séquence d'acides aminés d'une quelconque région variable de chaîne légère anti-EGFRvIII répertoriée dans le tableau 5 et la séquence d'acides aminés d'une quelconque région variable de chaîne lourde anti-EGFRvIII répertoriée dans le tableau 5 ;
(vi) ledit domaine de liaison à l'antigène dudit second CAR comprenant un polypeptide ayant une séquence de l'une quelconque des SEQ ID N^{os} : 71 à 79 ; ou
(vii) le second CAR comprenant un polypeptide ayant une séquence d'acides aminés choisie dans le groupe constitué par SEQ ID N° : 1043, SEQ ID N° : 1049, SEQ ID N° : 1055, SEQ ID N° : 1061, SEQ ID N° : 1067, SEQ ID N° : 1073, SEQ ID N° : 1079, SEQ ID N° : 1085, SEQ ID N° : 1090 et SEQ ID N° : 1096.

8. Cellule selon l'une quelconque des revendications 1 à 6, ledit antigène associé à une tumeur solide étant la mésothéline et :
(i) ledit domaine de liaison à l'antigène dudit second CAR comprenant une région déterminant la complémentarité de chaîne lourde 1 (HC CDR1), une région déterminant la complémentarité de chaîne lourde 2 (HC CDR2) et une région déterminant la complémentarité de chaîne lourde 3 (HC CDR3) d'une quelconque séquence d'acides aminés de domaine de liaison de chaîne lourde répertoriée dans le tableau 2 ou 3 ;
(ii) ledit domaine de liaison à l'antigène dudit second CAR comprenant en outre une région déterminant la complémentarité de chaîne légère 1 (LC CDR1), une région déterminant la complémentarité de chaîne légère 2 (LC CDR2) et une région déterminant la complémentarité de chaîne légère 3 (LC CDR3) d'une quelconque séquence d'acides aminés de domaine de liaison de chaîne légère répertoriée dans le tableau 2 ou 4 ;
(iii) ledit domaine de liaison à l'antigène dudit second CAR comprenant : la séquence d'acides aminés d'une quelconque région variable de chaîne légère répertoriée dans le tableau 2 ; une séquence d'acides aminés ayant au moins une, deux ou trois modifications mais pas plus de 20 ou 10 modifications de la séquence d'acides aminés de l'une quelconque des régions variables de chaîne légère indiquées dans le tableau 2 ; ou une séquence d'acides aminés présentant une identité de 95 à 99 % avec la séquence d'acides aminés de l'une quelconque des régions variables de chaîne légère indiquées dans le tableau 2 ;
(iv) ledit domaine de liaison à l'antigène dudit second CAR comprenant : la séquence d'acides aminés d'une quelconque région variable de chaîne lourde répertoriée dans le tableau 2 ; une séquence d'acides aminés ayant au moins une, deux ou trois modifications mais pas plus de 20 ou 10 modifications de la séquence d'acides aminés de l'une quelconque des régions variables de chaîne lourde indiquées dans le tableau 2 ; ou une séquence d'acides aminés présentant une identité de 95 à 99 % avec la séquence d'acides aminés de l'une quelconque des régions variables de chaîne lourde indiquées dans le tableau 2 ;
(v) ledit domaine de liaison à l'antigène dudit second CAR comprenant un polypeptide ayant la séquence d'acides aminés d'une quelconque région variable de chaîne légère répertoriée dans le tableau 2 et la séquence d'acides aminés d'une quelconque région variable de chaîne lourde répertoriée dans le tableau 2 ;
(vi) ledit domaine de liaison à l'antigène dudit second CAR comprenant un polypeptide ayant une séquence de l'une quelconque des SEQ ID N^{os} : 46 à 70 ; ou
(vii) le second CAR comprenant un polypeptide ayant une séquence d'acides aminés choisie dans le groupe constitué par SEQ ID N° : 282, SEQ ID N° : 283, SEQ ID N° : 284, SEQ ID N° : 285, SEQ ID N° : 286, SEQ ID N° : 287, SEQ ID N° : 288, SEQ ID N° : 289, SEQ ID N° : 290, SEQ ID N° : 291, SEQ ID N° : 292, SEQ ID N° : 293, SEQ ID N° : 294, SEQ ID N° : 295, SEQ ID N° : 296, SEQ ID N° : 297, SEQ ID N° : 298, SEQ ID N° : 299, SEQ ID N° : 300, SEQ ID N° : 301, SEQ ID N° : 302, SEQ ID N° : 303, SEQ ID N° : 304, SEQ ID N° : 305 et SEQ ID N° : 306.

9. Cellule selon l'une quelconque des revendications 1 à 8, ledit domaine de signalisation intracellulaire dudit second CAR comprenant un ou plusieurs domaines de signalisation primaire, éventuellement les domaines de signalisation primaire comprenant un domaine de stimulation CD3-zêta.

10. Cellule selon l'une quelconque des revendications 1 à 9, ledit domaine de signalisation intracellulaire dudit premier CAR ou dudit second CAR comprenant un ou plusieurs domaines de signalisation co-stimulatrice, éventuellement ledit domaine de signalisation co-stimulatrice étant un domaine intracellulaire d'une protéine co-stimulatrice choisie dans le groupe constitué par CD27, CD28, 4-1BB (CD137), OX40, GITR, CD30, CD40, ICOS, BAFFR, HVEM, ICAM-1, l'antigène-1 associé à la fonction lymphocytaire (LFA-1), CD2, CDS, CD7, CD287, LIGHT, NKG2C, NKG2D, SLAMF7, NKp80, NKp30, NKp44, NKp46, CD160, B7-H3 et un ligand qui se lie spécifiquement à CD83.

11. Cellule selon l'une quelconque des revendications 1 à 10, le premier CAR se liant à CD19 et le second CAR se liant à EGFRvIII.

12. Cellule selon la revendication 11,
(a) dans ledit premier CAR :
(i) ledit domaine de liaison à l'antigène comprenant une région déterminant la complémentarité de chaîne lourde 1 (HC CDR1), une région déterminant la complémentarité de chaîne lourde 2 (HC CDR2) et une région déterminant la complémentarité de chaîne lourde 3 (HC CDR3) d'une quelconque séquence d'acides aminés de domaine de liaison de chaîne lourde répertoriée dans le tableau 6, le tableau 7 ou le tableau 9 ;
(ii) ledit domaine de liaison à l'antigène comprenant en outre une région déterminant la complémentarité de chaîne légère 1 (LC CDR1), une région déterminant la complémentarité de chaîne légère 2 (LC CDR2) et une région déterminant la complémentarité de chaîne légère 3 (LC CDR3) d'une quelconque séquence d'acides aminés de domaine de liaison de chaîne légère répertoriée dans le tableau 6, le tableau 8 ou le tableau 9 ;
(iii) ledit domaine de liaison à l'antigène comprenant : la séquence d'acides aminés d'une quelconque région variable de chaîne légère répertoriée dans le tableau 6 ou le tableau 9 ; une séquence d'acides aminés ayant au moins une, deux ou trois modifications mais pas plus de 20 ou 10 modifications de la séquence d'acides aminés de l'une quelconque des régions variables de chaîne légère indiquées dans le tableau 6 ou le tableau 9 ; ou une séquence d'acides aminés présentant une identité de 95 à 99 % avec la séquence d'acides aminés de l'une quelconque des régions variables de chaîne légère indiquées dans le tableau 6 ou le tableau 9 ;
(iv) ledit domaine de liaison à l'antigène comprenant : la séquence d'acides aminés d'une quelconque région variable de chaîne lourde répertoriée dans le tableau 6 ou le tableau 9 ; une séquence d'acides aminés ayant au moins une, deux ou trois modifications mais pas plus de 20 ou 10 modifications de la séquence d'acides aminés de l'une quelconque des régions variables de chaîne lourde indiquées dans le tableau 6 ou le tableau 9 ; ou une séquence d'acides aminés présentant une identité de 95 à 99 % avec la séquence d'acides aminés de l'une quelconque des régions variables de chaîne lourde indiquées dans le tableau 6 ou le tableau 9 ;
(v) ledit domaine de liaison à l'antigène comprenant un polypeptide ayant la séquence d'acides aminés d'une quelconque région variable de chaîne légère répertoriée dans le tableau 6 ou le tableau 9 et la séquence d'acides aminés d'une quelconque région variable de chaîne lourde répertoriée dans le tableau 6 ou le tableau 9 ;
(vi) ledit domaine de liaison à l'antigène comprenant un polypeptide ayant une séquence de SEQ ID N° : 83 ; SEQ ID N° : 84, SEQ ID N° : 85 ; SEQ ID N° : 86 ; SEQ ID N° : 87 ; SEQ ID N° : 88 ; SEQ ID N° : 89, SEQ ID N° : 90, SEQ ID N° : 91, SEQ ID N° : 92, SEQ ID N° : 93, SEQ ID N° : 94, SEQ ID N° : 95 ou SEQ ID N° : 112 ; ou
(vii) le CAR comprenant un polypeptide ayant une séquence d'acides aminés choisie dans le groupe constitué par SEQ ID N° : 269, SEQ ID N° : 270, SEQ ID N° : 271, SEQ ID N° : 272, SEQ ID N° : 273, SEQ ID N° : 274, SEQ ID N° : 275, SEQ ID N° : 276, SEQ ID N° : 277, SEQ ID N° : 278, SEQ ID N° : 279, SEQ ID N° : 280 et SEQ ID N° : 281 ; et
(b) dans ledit second CAR :
(i) ledit domaine de liaison à l'antigène comprenant une région déterminant la complémentarité de chaîne lourde 1 (HC CDR1), une région déterminant la complémentarité de chaîne lourde 2 (HC CDR2) et une région déterminant la complémentarité de chaîne lourde 3 (HC CDR3) d'une quelconque séquence d'acides aminés de domaine de liaison de chaîne lourde anti-EGFRvIII répertoriée dans le tableau 5 ;
(ii) ledit domaine de liaison à l'antigène comprenant en outre une région déterminant la complémentarité de chaîne légère 1 (LC CDR1), une région déterminant la complémentarité de chaîne légère 2 (LC CDR2) et une région déterminant la complémentarité de chaîne légère 3 (LC CDR3) d'une quelconque séquence d'acides aminés de domaine de liaison de chaîne légère anti-EGFRvIII répertoriée dans le tableau 5 ;
(iii) ledit domaine de liaison à l'antigène comprenant : la séquence d'acides aminés d'une quelconque région variable de chaîne légère anti-EGFRvIII répertoriée dans le tableau 5 ; une séquence d'acides aminés ayant au moins une, deux ou trois modifications mais pas plus de 20 ou 10 modifications de la séquence d'acides aminés de l'une quelconque des régions variables de chaîne légère anti-EGFRvIII indiquées dans le tableau 5 ; ou une séquence d'acides aminés présentant une identité de 95 à 99 % avec la séquence d'acides aminés de l'une quelconque des régions variables de chaîne légère anti-EGFRvIII indiquées dans le tableau 5 ;
(iv) ledit domaine de liaison à l'antigène comprenant : la séquence d'acides aminés d'une quelconque région variable de chaîne lourde anti-EGFRvIII répertoriée dans le tableau 5 ; une séquence d'acides aminés ayant au moins une, deux ou trois modifications mais pas plus de 20 ou 10 modifications de la séquence d'acides aminés de l'une quelconque des régions variables de chaîne lourde anti-EGFRvIII indiquées dans le tableau 5 ; ou une séquence d'acides aminés présentant une identité de 95 à 99 % avec la séquence d'acides aminés de l'une quelconque des régions variables de chaîne lourde anti-EGFRvIII indiquées dans le tableau 5 ;
(v) ledit domaine de liaison à l'antigène comprenant un polypeptide ayant la séquence d'acides aminés d'une quelconque région variable de chaîne légère anti-EGFRvIII répertoriée dans le tableau 5 et la séquence d'acides aminés d'une quelconque région variable de chaîne lourde anti-EGFRvIII répertoriée dans le tableau 5 ;
(vi) ledit domaine de liaison à l'antigène comprenant un polypeptide ayant une séquence de l'une quelconque des SEQ ID N^{os} : 71 à 79 ; ou
(vii) le CAR comprenant un polypeptide ayant une séquence d'acides aminés choisie dans le groupe constitué par SEQ ID N° : 1043, SEQ ID N° : 1049, SEQ ID N° : 1055, SEQ ID N° : 1061, SEQ ID N° : 1067, SEQ ID N° : 1073, SEQ ID N° : 1079, SEQ ID N° : 1085, SEQ ID N° : 1090 et SEQ ID N° : 1096.

13. Acide nucléique codant pour un premier CAR et un second CAR, chacun de ceux-ci comprenant un domaine de liaison à l'antigène, un domaine transmembranaire et un domaine de signalisation intracellulaire, le domaine de liaison à l'antigène dudit premier CAR se liant à un antigène de lymphocyte B et le domaine de liaison à l'antigène dudit second CAR se liant à un antigène tumoral autre qu'un antigène de lymphocyte B, l'antigène tumoral autre qu'un antigène de lymphocyte B étant un antigène associé à une tumeur solide, en outre
(i) le domaine de signalisation intracellulaire dudit premier CAR comprenant un domaine de signalisation co-stimulatrice, mais pas un domaine de signalisation primaire ; et
(ii) le domaine de signalisation intracellulaire dudit second CAR comprenant un domaine de signalisation co-stimulatrice et un domaine de signalisation primaire.

14. Acide nucléique selon la revendication 13, ledit domaine de signalisation intracellulaire dudit second CAR comprenant un ou plusieurs domaines de signalisation primaire, éventuellement les domaines de signalisation primaire comprenant un domaine de stimulation CD3-zêta.

15. Acide nucléique selon la revendication 13 ou 14, la séquence dudit premier CAR et la séquence dudit second CAR étant séparées par un site d'entrée de ribosome indépendant, un élément promoteur ou une séquence codant pour un élément T2A, P2A, E2A ou F2A.

16. Cellule selon l'une quelconque des revendications 1 à 12 destinée à être utilisée dans une méthode de traitement de cancer.

17. Cellule destinée à être utilisée selon la revendication 16, le cancer étant une tumeur solide.

18. Cellule selon l'une quelconque des revendications 1 à 12 destinée à être utilisée dans une méthode de traitement d'une maladie associée à l'expression d'un antigène associé à une tumeur solide, la maladie étant une tumeur caractérisée comme étant un glioblastome, un cancer de l'ovaire, un cancer du poumon, un cancer de la prostate, un cancer colorectal, un cancer du pancréas, un carcinome du sein, un adénocarcinome ou un mésothéliome.
